# EUROPEAN PATENT APPLICATION

(11) **EP 1 394 267 A1**
(43) Date of publication of application: **03.03.2004**
(21) Application number: 02018158.2
(22) Date of filing: 19.08.2002
(51) Int. Cl.: C12Q 1/68, G01N 33/50, C07K 14/47, C12N 15/63, A61K 48/00

(54) **Single nucleotide polymorphisms predictive for cardiovascular disease, adverse drug reactions, and drug efficacy**

(71) Applicant: Bayer HealthCare AG, 51368 Leverkusen (DE)
(72) Inventor: Schwers, Stephan, Dr., 51145 Köln (DE); Kallabis, Harald, Dr., 51061 Köln (DE); Stropp, Udo, Dr., 42781 Haan (DE)

(57) **Abstract**

The invention provides diagnostic methods and kits including oligo and/or polynucleotides or derivatives, including as well antibodies determining whether a human subject is at risk of getting adverse drug reaction after statin therapy or whether the human subject is a high or low responder or a good a or bad metabolizer of statins. The invention provides further diagnostic methods and kits including antibodies determining whether a human subject is at risk for a cardiovascular disease. Still further the invention provides polymorphic sequences and other genes.

The present invention further relates to isolated polynucleotides encoding a phenotype associated (PA) gene polypeptide useful in methods to identify therapeutic agents and useful for preparation of a medicament to treat cardiovascular disease or influence drug response, the polynucleotide is selected from the group comprising:
SEQ ID 1-168 with allelic variation as indicated in the sequences section contained in a functional surrounding like full length cDNA for PA gene polypeptide and with or without the PA gene promoter sequence.

Sequences:

SEQUENCE LISTING

<110>  Bayer AG

<120>  Single Nucleotide Polymorphisms sensitively predicting ADR and Drug
Efficacy

<130>  LeA

<160>  168

<170>  PatentIn version 3.1

<210>  1

<211>  414

<212>  DNA

<213>  Homo Sapiens

<220>

<221>  variation

<222>  (137)..(137)

<223>  baySNP28_C137T

<220>

<221>  misc_feature

<222>  (229)..(229)

<223>  Unsure

<220>

<221>  misc_feature

<222>  (233)..(233)

<223>  Unsure

<400>  1
ggatcttaat tgcatgctcc ccagtgtcca gtgttaggaa aaagccgaaa gtaactccaa      60

caaaggagtc ttacactgaa gaaatagtgt ctgaagcaga atctcatgtt tcaggtattt     120

ctagaattgt gcttccyaca gaaaaaacta caggagccag aagaagtaag gctaaatctc     180

tgacagatcc aagccaagaa tctcatacag aagccacatc tgatgctgng acntaaagct     240

cagacatttc attctctgga attgcaacta gaagaaccag gagtatgcag aggaaattaa     300

aggcacaaac tgaaaagaaa gatagtaaga ttgtaccagg aaatgagaaa cagatcgtgg     360

gtacacctgt gattcagagg attcagaaac cagacaaact tcccatttac aagc          414

<210>  2

<211>  641

<212>  DNA

<213>  Homo Sapiens

<220>

<221>  variation

<222>  (464)..(464)

<223>  baySNP29_A464G

<400>  2
caaaaccgtg tgtgcaagag cgcgacctaa ggggacattc ttgtcgacgg tacaggaggg      60

tgggcagagt tagggcagga agtatctaca cacattctct acggggcagg tgacgcagtc     120

agatctcagt gttcagtttc ctggaggggg caaagtcttc acgcctaacc caaatgactt     180

cctcgcttgc gctctccagg cctccattga tccccgacag ggcagcttgc ttcttgagct     240

gtgccatgcg ggacgcgtga ctatccaggg ttttccggcc tctttcccgc tgactgatgg     300

aggtggcctg aagccgctcc tgcaggtctc tgtccactga ggcacccttc acggactcac     360

agaactcatc atcgtcactg aggatgtctg tctccttgat gtcatcttgc tcctcatact     420

gagcaagatc aaactgctcc tctacccatc ggtcagtgtc cccrccaccg gggggctgct     480

gggactcttt ctccgggctg cttgcggaga cggcatcaga atcaatggta aacctgtttc     540

gagccagccg ccgcctcctc ctcccaagag acttgcttgg ggcagacact gcacacacac 600

acaaaaatat aaaaataaaa cccccacatg ctttacgtga g 641

<210> 3

<211> 606

<212> DNA

<213> Homo Sapiens

<220>

<221> variation

<222> (397)..(397)

<223> baySNP_C397G

<220>

<221> misc_feature

<222> (513)..(513)

<223> Unsure

<400> 3
ttgaggccag gagtttgaga ccagcctgga caacatagca agatcccacc tgtagtccta 60

gatacttggg agagtgagga gggagggtta cttgagccca ggagattaag gctataatag 120

tgagggatga ttgcaccact gcactccagc ctgggcaaca gagtgagacc ctgtctctaa 180

aaaaaaaaaa aaattattaa aaaaaaaaaa gttttcttaa gagtccagac ttgtgaattg 240

ccagattagt gtaattttta aaatatgttt ctattataaa ttacccatac tcataaaaat 300

ataaatcaat ttattacacc ctctagaatt cactattaat tttcaacatt tttttcattc 360

tttttccatg catatttttt cacaattcta tgcatasttt tgcattataa aatatttctc 420

aatataaaat cttcttcaag gccaggcgca gtggctcatg cctgcaatcc cagcacttta 480

gaaggccaag gcgagcagat cacttgaggt cangaattca agaccagcct gaccaacatg 540

gtgaaacccc ttctctacta aaaatacaaa aattagccgg gcatggtggt gcgcgcctgt 600

aatccc 606

<210> 4

<211> 746

<212> DNA

<213> Homo Sapiens

<220>

<221> variation

<222> (429)..(429)

<223> baySNP56_A429G

<400> 4
tgattagcca ccttagttcc atctgcaact ttagttccca ctggctgtgt aacctaacat 60

agtcacaggc tctggggact gtcacgtgga catctttggg aggccgttat tctgcccacc 120

gcaccctccg ttcatcccct gccctgccgg gcacctcgct ctaccccagg aaaatgtgag 180

ctcgttttcc tgctcggcat gtgctccccc taaggctctg ctcctccctg ggcctgaaag 240

ttccttctca gcctgagagg gggcccttcg atctcaggca tgactcagcc cggctgatgc 300

ctctgcagtg ctgagtcagg atttggggcc ggctctcttg ggtctgtccc cttttcccag 360

gtactgcctt acaaagctgt ggccaggaag tggccggtat aaaggatgcc caaggtcttt 420

gtacgtgtrt aggagttagc gtgtttgata ttggtaatat aataataatt attttttaga 480

gtactgcttt tgtatgtatg ttgaacagga tccaggtttt tatagcttga tataaaacag 540

aattcaaagt ggctgtggag tgatttattt tgggagctgc acaatgatgg ggggcccagc 600

caggcagcct tagcagcttt ctgagcctct aggggtaccg gcaaacctac ctaaggtctt 660

tgtaatcccc agactgtcca agcccagagc acctcatgtg gttcagcagg agattgccca 720

catcctcact ggagcagagc ttttga 746

<210> 5

<211> 1936

<212> DNA

<213> Homo Sapiens

<220>

<221> variation

<222>  (159)..(159)

<223>  baySNP89_A159G

<400>  5
tagttcaagg gtaaaggatc agaaaccaca gtgggaagga gtctcaccgg aggaccctcg      60

tcacctttct ctccaacttc accctgtgaa acatgagagt cagccctggt tccaagaacc     120

cccatgatgc tggcaacagc cctactccct tacccgccrt gactcccccg actccagcct     180

cttacatctc gtcctcgggg gccaacaggt cctggggggc caggccgccc agggtctccc     240

ttctctccag gaggccctga gtcaccctgt ggaggaggca agagggaggt gatgcaggac     300

gctcgaagca agcagttctc aaggggaggg gaccccagaa gccttgaggt tgcccagggt     360

aacgggtact cactgggggt cctgctctgc cagtaaggcc aatgggaccc tgaaggggca     420

cagaaggggg gcaggactta gtcagggtcc caccacctca tctccctctg tcacactccc     480

cattcccaca ttgattcacc cggtctccag ggtctccctt ggggccaggg tctccaggaa     540

gaaccaagcc gggtgggccc tgtggatgga aggataagaa gtcaggaaga caaccttcac     600

caactgcccc ctaaagagtc tgcttcactt acccgttccc cttggactcc ggtagctcct     660

ctgggcccag cgggccccac atctccctgg aggtgacaaa gaccatcagt gctagtccca     720

ggctccagtt aaccccctgg cccagcaagt ctgtagcccc ccaagcccat agataggcct     780

atgaccatag cctcaaccct gtagaaacct cccctgcccc ataccaggct taccttttct     840

cctttgggtc cagcaacagc aggtccctga aaacaaacag ggacagatac agcttggagc     900

tgccttgggt gtatggatca aagtcttctt ggaatggagg tcacatggaa ttggaagtca     960

tacagctcag tcccctccca gaagtcacag agtcaccgct gacagcaggg aaggggttat    1020

acaaaatgga ctgacacaaa gagctcatga cctgcatggc ggtcttgggg tcacaggatc 1080

atagccaaga gtctgggggc aggttctagc agaaggagag gccagagctc ccgacctgct 1140

cacgggccca gcactgtgga atcacagccc agatagtgtt ctataggagg gtcactgctc 1200

aagggctgtg ctgtgctcag agcgccatcc tcccgtcact caccaccact gcagggtccc 1260

cagggcgacc aggctccccc tgtggagaga ggataggagc agggacaggt cagggagtca 1320

cctaggagcc caaaatccca gtgtcccatc tgccacatcc ctagtaccag acagtgccac 1380

ccccagccga ggacccacct tctctccttg gcggccagtg ggtcctggtg gcccctgaat 1440

gtagagaaag tgtgagccca ggaggggaag ggaaagggga gggacacaca aaagtcccac 1500

tcctggtccc accacagtca cagactcact tcaggaccct tggctccagg acgtccagca 1560

acccctggca gcccctggag gagaggaagg gaagagctgt gcaggtgggt ggggacgggg 1620

gatatggaca gacagggcta gaggctgggg tgggggctgg agaggggtca gggaagattg 1680

ggatttaggt tggcaggggt aagacttggc aggagaacag agaccaggtc agtgaaaatc 1740

aaggtcagtt cagcagggtc agaggttcaa agccaggtcc ctggggctga gggttagagc 1800

ctgtatcagc agggataagt ggtcattgag gaggggtgag gagcaggggt agcaggggtc 1860

aagggcaaga agtcagaacc agaaagggca cagccaggtc agctggtatg agcattgcag 1920

ccagtgggtt tacccg 1936

<210> 6

<211> 578

<212> DNA

<213> Homo Sapiens

<220>

<221>  variation

<222>  (154)..(154)

<223>  baySNP90_C154T

<400>  6
tgtgttgctg ggcctggtgc tgctggtggt gctgctgctg cttctgaggc actggggctg      60

gggcctgtgc cttatcggct ggaacgagtt catcctgcag cccatccaca acctgctcat     120

gggtgacacc aaggagcagc gcatcctgaa ccaygtgctg cagcatgcgg agagcccggg     180

aacgcacaaa gcgtgctgga ggccattgac acctactgcg agcagaagga gtgggccatg     240

aacgtgggcg acaagaaagg tggggtccgg gccagcaggt gctcagctct gggacaggga     300

cccaggacca ggcatcaaag cccttacagg agaagctgtt atcaccccgt ttccaggggg     360

ctgggaaccc tgggatatgc ccagataggg ctggggggct cctctggagt cccagggtgc     420

cagggtccct gatgacccct gcaggccctg ctgcctgctg ccccaagaca acaggccccc     480

acactcacag ggtctgacgg tggtgcagtt ccccttgaac tctgttctgg gcaccatgga     540

acctgcttgg ggaccagtca gacaggttct cctgggcc                             578

<210>  7

<211>  224

<212>  DNA

<213>  Homo Sapiens

<220>

<221>  variation

<222>  (58)..(58)

<223>  baySNP99_C58T

<400>  7
agcctgcctg gtcactgact gcttactcta ccttagctgg acctcgtctc ccagggayta      60

gagaaaagag caggagtcct gggtcttccc agttgagact gctggagctg agacacagta     120

ctctcttaaa gaaggtgggg agctgcccag ggcagaaccc actggtgttc atgactaccc     180

ctggctcctc tcaccttgtc cctccactgc caacagaaaa caaa                      224

<210>  8

<211>  630

<212>  DNA

<213>  Homo Sapiens

<220>

<221>  variation

<222>  (468)..(468)

<223>  baySNP140_C468T

<400>  8
ggatcttcac atcaggataa atggtgcttt ctttttgtag atgatgtaaa cttcaccctg      60

acatatttcc ttttttacac tgactgccat aaagcttagg acaaaatttg aagacagcct     120

tacagggtca catggtatct acttatctgt ggctttattt tctttgtccc catattctat     180

cccaattaca tagactcctt gttttatgcc tttataactt gagaaactgt ctcagatcct     240

ttgtattact gagtaagctg taaataaata caaatactaa ataaaaacta aaagttgcat     300

ttgaatttaa aattatatga gcatcttttt cttttaaaat taaaaaataa ccaggtactc     360

cataatattt tactatgtaa tttctcccat gattctgtat ttgtgttact tactttgagt     420

gtgtctctga cctgggctct gataatagga cccaaaatcc catcttcytt catattggga     480

ttcactgtat gtttggtgaa ggactcatct tcgtactgtg tgtacataac tttcttataa     540

tgttttccaa tttggtttga gaaattatcc aaatgctgag acctgtattt tcttaaagtg     600

aagtaaaaaa aaattaaacc actttctcaa                                      630

<210>  9

<211>  968

<212>  DNA

<213>  Homo Sapiens

<220>

<221>  variation

<222>  (587)..(587)

<223>  baySNP152_A587G

<400>  9
tttccaggac ccaagacaga tgctgaagaa gacagcaggg accggagccc agcaaactgg      60

atgagagcag caaggccttc atgcatcttc aggctccatc agcagaggga gagaatgcat     120

tagacgggag tttcctgact cctattttct aatgtggcag tcctaatgag aagagggttt     180

gaagcgctaa gatgtgctga cagtcctaat ggtttgttat gagtttcctt agaaaaatta     240

aacactgaaa aacaaaactg cctccctccc tccctttgtg gttgctgaca tcagaggtca     300

ggaatataac aggcctgctc gtcagccaag ctctccacga gagccctctg ttccagaaga     360

gcaccaaggc agtgacctca acatacttgg gcaacattcc ctttcttggt aacccaggct     420

gagaaggtag aaagaatgtc tccaggttat gcaggaagaa agctagaagg gggaggggtg     480

gatattaaag tttcagttcc cagggaaacc ggatgttaca caaaaagggc aaaaatagcc     540

aagacccagg ggaagggcct gagcacagag gtgggaggtt ccagccrgcg ccctgcgagc     600

tggctgaatt acctccctct cctgggaggg tgacacacgc tcttggtacc attctccatt     660

aatcccattt tggaaattgg gaaagtacaa catgaatagt tgtcattatc ctgcctattt     720

cacagggttg gtcaggaagt aaaagaagac tgtgaatgtg aaatgctttc aaaatataaa     780

aatctcaaca taagttaact atactgaaat ccattaggac agaggttccc aaccttttag     840

gcaccaggga ctggtttcag ggaagacaat ttttccatgg acagggaggt ggggatggtt     900

tcagaatgaa actgttccac cccagatcat caggcattag tttgatcctc ataaggagtg     960

aacaacct                                                              968

<210> 10

<211> 709

<212> DNA

<213> Homo Sapiens

<220>

<221> variation

<222> (209)..(209)

<223> baySNP214_A209G

<400> 10
tgttgtggaa tgtcccccct gcccccataa ggtattcccg gcgtttccac aagactctaa 60

ggcgcttggt gccagactcc gatgtgcgct tcctcctctc ggagagtggc agcggcaagg 120

gggctgccat ggtgacggcg gtggcctacc gcttggccga gcagcaccgg cagatagagg 180

agaccctggc tcatttccac ctcaccaarg acatgctgct ggaggtgaag aagaggatgc 240

gggccgagat ggagctgggg ctgaggaagc agacgcacaa caatgccgtg gttaagatgc 300

tgccctcctt cgtccggaga actcccgacg ggaccggtga gggcctgctg ggggctgaca 360

tgcctgtcct gctcctgcca ggaactgagc cgcagtgggg gaggttggat tcgccagtgg 420

atgccttcac actggtgatg cacgattttg ccagagctag aaactccctc acatctctgt 480

tttccatttt tattaactga tttgtcatgt gtgtgactca gggcagttta attttcctgg 540

tcagacctcc caagcaagtg ctatctctaa aaccacagcc ctgctcagaa cagtgctctt 600

agctgggcaa ggtgacacac gcctgtcatc cagctactta aaaggctgag gtgggaggac 660

tgcttgagcc caagagttca agaacagtct gggcacatag caagaccct 709

<210> 11

<211> 674

<212> DNA

<213> Homo Sapiens

<220>

<221> variation

<222> (339)..(339)

<223> baySNP221_C339G

<400> 11
aaaataaaat ttagattaat attccacaaa acagttttaa agtattaaaa tttaaaatac 60

tatagtatag ctatagcagt gtactatata gtacaatgaa atatgttaca aaaacacagg 120

cttttaacaa agcacctaaa atctttttaa tgccataatc ttttgacaaa atgccacctg 180

atgatattcc agcttctgag cctccagttg atcatgctga cgttgtagct cttctttttg 240

tttctgaagc tcatctgcct tcttcttaag ttcattttct tgtaaagcaa taatattttc 300

atattctttt agttcttcct ctgtgaagaa ctgttgctsa tctaatgtct aaaagaaaaa 360

aaatgaggaa agaataatct caaaactaca acaataaagt ctgatttagc tttaaaattc 420

tatggttagc aaagtataaa agagtatcta taggattacg cctttggagg taagaaggaa 480

ggggaaataa gtaatatata tttatgtact catttgaaca aaaggaaaaa caggaagaaa 540

aactcaagag attctcttct gggattgaca actaaaggca aagagtgaga actgggtaga 600

agaggcaaag agaacgtggg aagagaaata aaatttgtca ttccccccaa ccctcactga 660

aacatgaaat ttta 674

<210> 12

<211> 811

<212> DNA

<213> Homo Sapiens

<220>

<221> variation

<222> (189)..(189)

<223> baySNP224_C189T

<400> 12
ctgccagaga agcatcggag ggaattgagg tctgctcggc cgtcttcact cgcccccggg 60

tttggcgggc caaggactgc cgaccgaggc tggagctggc gtctgtcttc aagggcttac 120

acgtggagga atgctccccc atcctcccct tccctgcaaa catggggttg gctgggccca 180

gaaggttgyg atgaagaaaa gcgggccagt gtgggaatgc ggcaagaagg aattgacttc 240

gactgtgacc tgtggggatt tctcccagct ctagacaacc ctgcaaagga ctgttttttc 300

ctgagcttgg ccagaagggg gccatgaggc ctcagtggac tttccacccc ctccctggcc 360

tgttctgttt tgcctgaagt tggaatgagt gtggctcccc tctatttagc atgacaagcc 420

ccaggcaggc tgtgcgctga caaccaccgc tccccagccc agggttcccc cagccctgtg 480

gaagggacta ggagcactgt agtaaatggc aattctttga cctcaacctg tgatgagggg 540

aggaaactca cctgctggcc cctcacctgg gcacctgggg agtgggacag agtctgggtg 600

tatttatttt cctccccaca ggtggggagg gggtttgggg ggcttgcaag tatgttttag 660

catgtgtttg gttctggggc ccctttttac tccccttgag ctgaaaggaa ccccttttgg 720

cccccgagct gggggccatg agctcagacc cccacaaccc tcctataacc tcccctcctt 780

gccttctgtg taatcatttc ttgggccctc c 811

<210> 13

<211> 776

<212> DNA

<213> Homo Sapiens

<220>

<221> variation

<222> (465)..(465)

<223> baySNP294_C465T

<400> 13

tccatttgat tcttctgagc aggagcctct gccagacccc agcttttccc tacctcccct 60

gaagtcagaa aaggaatgga ttacaggatt actcacctgt cgtgcggcca gaggcataga 120

gggagagcac agcttgaatg gcgacgtaca tggcagggac attgaaggtt tcaaacatga 180

tcttaaagga caatgaagaa taaaagatta tttggcaaaa atcacagcac ttcttggcct 240

gtagaatggt ttgtaatctc attaagggca ggaactatgc cagtttgata gttcattatt 300

ccacacccac tcccccaggg cctggcatgg accacagaca tagcaggtcc tcagagagtt 360

ctgttgcact gatgtttgaa ggggagaaag tatctggcca agcatccatg ttgcattctt 420

atgccagtgt tcaagtcttc ctggcttctt acctgggtca tcttytccct gttggccttg 480

ggatttaggg gagcctctgt gagcagggtg gggtgctctt caggtgctac acgcagctca 540

ttgtagaagg agtggtgcca gatctagtgg agacaaaagc catatgagcc tggcagactc 600

ccaatggctg gaaaattctc attgacattc ccatgctggg agagtgtggc accatgttac 660

acaggatggg atggatctgg agaggagaat ggtcagccct ggaaatttct gattgacatt 720

taaagggtat actccttctc acagggcccc agtaggactc agactggaac agtatt 776

<210> 14

<211> 1920

<212> DNA

<213> Homo Sapiens

<220>

<221> variation

<222>  (215)..(215)

<223>  baySNP307_C215T

<400>  14
   atcccagtgt ctctgccagg gccaggctct gccccattgg gatcggaaat ctgggcagat      60
   ttgggatcta gagcagggaa gtctcagggt tgaggcctga agtctagcat ggcacacagc     120
   agggctgaga gcaaaaccca gggtcttgtc tggattccca ggccagttac tgcctctctg     180
   accccagacg gctcacctgt cgaatgggta cattygggaa cagcacccac tctacgaagc     240
   caccatgaag acgaaagaga aaatcgtcta catgggcagt gtagaacggg cgcccggtga     300
   gtgctcaggg atgaccctcc tctgtagctg cccagaggcc aacaccgccc ataccatagc     360
   cactgtcccc aagtcagcct ggagggaaga gagcaggtca cactcacctg attctgatga     420
   atcagctggc ctgggttatg cctctcaggg agaaaacctt tgagtccaca aaaccgcttg     480
   tgaataccac tgcgtgtgtg taactgccgt agaacacaga cgcagtctgg agagcggata     540
   ggtgctaagc accagtgaca ttctgaggtc atggcacgca tcacaatggg gccttgcccg     600
   ggtcagcagg gcccagagtc agggtcctcc gctgcctgag gcgtcaacat gcctgcctgc     660
   aatgtgtttg tgcacatgcg tgcacatgtg tatgtggata aacatgtctg tgcacgtgtg     720
   tgttgcttct ctggccaggc ccggctgccc cactcatgtg tgcacccagt tcctcatcac     780
   tgtcaccccc ggagcccagg accagcttca gacctccctg acctcagccc tccccgccca     840
   gggtggtcct gggatacaca taggagtgga gggaagtgac tgctgctgtt gaatctcaga     900
   atacaaaagc taatactatt acttaatggt atttttagta tctctaatcg tctttttagt     960
   gtctctaatg gtatcacttt ttcatttctg atattttaac tgggtatttc tctccatgac    1020
   ccttggatat tctagctaga ggatcatgtg cggaaatgcc aggcacacag taggggccca 1080
   ctctagacat gttatctaaa cctggctcag ctgtcccacc cagggcctag gggatgccaa 1140
   attccagggt ccagaagagc ttgggataaa atgaacatcc aaggggaggg ctttgacctg 1200
   ggttagtctg cctgtgccat ccaaacggag tctcaagtcc tgagacaggg cgtccagatg 1260
   cccagtgcag ggtccccctg atcaacacct gctcccctgt actcattagc aacctcaccc 1320
   accctactct caaagcactt ggccctcgta tctgggagct ctgcaactgt ggattcagcc 1380
   aacagcagat ggaaaatatt cagaaaacaa atcggatggt tgtctctact gaacatgtgc 1440
   agacgttgtt cttgtcatca ttccctaaat acagtatcac aaccatttat atagcatctg 1500
   cattgtatta cacataatga ataatctaga gatggtctaa tgtatacggg agaatgtgca 1560
   tagcttatat gtaaatacta ggtcatgtta tgtcagcgac ttgagcatcc atggattttg 1620
   gcatccccgg ggaccctaga actaatcctc catggatacc aagggatgac tatataaact 1680
   cactcaggaa ggcttctcac tggaggaagg gcccagttca ggacacacag ggacatctcc 1740
   ctggactgct gtccattcat ccatccattc atccattgtc tcccccgact ccccatctca 1800
   gactgtccca atgacagccc tagcaagaag agacaagaaa caagacaagt tcacgttgtc 1860
   cagttttgag gtcttggaag aagttgcacc agtatgagaa tagtgggtca gttttctcca 1920

<210> 15

<211> 587

<212> DNA

<213> Homo Sapiens

<220>

<221>  variation

<222>  (369)..(369)

<223>  baySNP411_A397T

<400>  15
tggagcctcc ccataggcac tcggggcctc ccctgggatg agactccagc tttgctccct      60

gccttcctcc cccaggcatg gctggggact ggaagaccac cttcaccgtg gcgcagaatg     120

agcgcttcga tgcggactat gcggagaaga tggcaggctg cagcctcagc ttccgctctg     180

agctgtgaga ggggttcctg gagtcactgc agagggagtg tgcgaatcaa gcctgaccaa     240

gaggctccag aataaagtat gatttgtgtt caatgcagag tctctattcc aagccaagag     300

aaaccctgag ctgaaagagt gatcgcccac tggggccaaa tacggccacc tccccgctcc     360

agctcctcwa cttgccctgt ttggagaggg gagagggtct ggagaagtaa aacccaggag     420

acgagtagag ggggaatgtg tttaatccca gcacgtcctc tgctgtcctg ccctgtgtcg     480

ttgggggatg gcgagtctgc caggcggcat cactttttct tgggttcctt acaagccacc     540

acgtatctct gagccacatt gaggggaggg gaatagccat ctgcata                   587

<210>  16

<211>  478

<212>  DNA

<213>  Homo Sapiens

<220>

<221>  variation

<222>  (323)..(323)

<223>  baySNP449_C323G

<400>  16
gaattcttcc acgatcattg ctatctacca caaaaataag accctgggta ttctggaagt      60

aatgcttcca gagaggccta attctgtctt gaccaccaac atcccgtact gtgaaacaag     120

tgttcttata ctctactgtt tccacgttaa aaccagtggt aggaatggtg gtgactatct     180

cccctaactt cagtttatac agaatggtca tcttgccagc agcatccaat ccaaatagca     240

aaatgtgcat ttacttcttg ccaaagaagc aggagaagag ggaggagatg gtgaggccca     300

tggtggtagt gccacttgca atsagcagaa gcagaagcgg tttggggcag ccccctgctt     360

tctccttacg tgctccaggc aaactgaaca agagagaaga gaaagagcgg aggaagaaag     420

agggaggcag gaacatctca ctggccgctg tgctaatgaa gctccaggac acgaattc       478

<210>  17

<211>  844

<212>  DNA

<213>  Homo Sapiens

<220>

<221>  variation

<222>  (123)..(123)

<223>  baySNP466_C123T

<400>  17
ctggccccgc tcctgggggg cctgcccagc ctccacccac ccctcaaccc tccatggctg      60

atcttcagtt ctctcagctt ctggggaacc tgctagggcc tgcagggcca ggggctggag     120

ggyctggtgt ggcttctccc accatcactg tggcgatgcc tggtgtccct gcctttctcc     180

aaggcatgac tgacttcttg caggtgagtg gctggcttgc cattcacctc atcttcctgt     240

cccccggccc agccaggcag tggtaccttc ttgccattac ataaccactg ggtaagagcc     300

cctagtgaca tgttagggga aggggccctg ggaattcatt gactctgaac ctttcatttt     360

aaagatgaag aaaatgaggt ttagaaaaag aaagtatagt aattagtgtc actgctttct     420

gacaacctgt tcagaggaag aggtaggtaa gttgtgctgc ctgactctgt taaataccta     480

ccatacttct gctttaagca gtgaagatca gaatgtaact ccccatcttc ccagacccgt     540

tggtttgtgc cctcttgatg ccgtattatt tccgtgtgtc ttcattctca cctcactttc     600

tgttctttgt tctttcttta tttgccaggc accaaaaaca ggccctttca ccaaccccaa     660

cctttttcac ccccaccacc tgcccccaga gcagcagaac catgccccca ccaggctccc     720

ctttttggtg gcgcagggga gtcctggagg cctgggtctt gaaaacctgt caccggagtt     780

ttttacttca gtggtgcaag ggtggcctca acttcctgct gggctccctg ggggcccggg     840

ctgg                                                                  844

<210> 18

<211> 561

<212> DNA

<213> Homo Sapiens

<220>

<221> variation

<222> (497)..(497)

<223> baySNP472_A497G

<400> 18
ccaagaaaca agcatcaccg agggagcagg acgaggaggg aaggccatgc agacggcatg 60

gggctggagg ggctgttagg tgaacagatg cagacaggta tggaaggcca ggcaggaagc 120

aagtgtaggg ccaggaagca taagtgggta actcagaaaa gctgtggcca ctgacactgg 180

ggctgcggcc aactgacgtc tgctctgctc ttgcatctta catcctttgt tttggagttc 240

cctgcatctt acatcctttg ttttggagct ccccactggg tatgggagtc tcagaaccca 300

cagggattaa ggagacaagt tttctctcca acttacaagg gatctcactt acccatcata 360

cttctttttc tggggtccgc caatatgggg cctccctaca gaacgctttt gatgtgctgg 420

gcttcacttc agaggagaaa aactccatgt ataagctgac aggcgccatc atgcactttg 480

gaaacatgaa gttcaarctg aagcagcggg aggagcaggc ggagccagac ggcactgaag 540

gtgggaggca ggattcttgg g 561

<210> 19

<211> 832

<212> DNA

<213> Homo Sapiens

<220>

<221> variation

<222> (402)..(402)

<223> baysNP542_A402G

<400> 19
tgaggatcaa gtaacttgcc aaggtcacag agttcactta ctactcagtg agcttgggta 60

aatggctgaa ttttgctgtt tcagtttctc cataataaaa ggattattat aataatacgt 120

aaagtgttgc tgacaaggtg aaatacataa tttcactttc tgagtactta agaacagtta 180

gctgttatta tcaagcaaat ttgtatcaag tgttcccaca cattcagcca tgttgctgtg 240

gtactgcctg gcttgtatag gctgctggac aggcagaggc tccttcttcc taaaagccct 300

tggcagtcac attatattta cctgtcttct ggtattaagc cgtaatttgc atgattgagc 360

cagttgttta tctttcgctc catcaaccaa gtcacaattg grgttgggag ggaatttctc 420

aacatgttct gaaagcgtgt catgaacacc atgtcccatg ggtagcccga gtcaaagatt 480

cggctgatca cccatccccc tccggtggtg ctgaggaaca cctggaagca atcaaagaca 540

tcccttcatt tgacactgtg aacacggctg gctggttctg ccagagattc aagaagtgtc 600

atgaatggaa aggcactgaa aagtctgcac tctcaaaata cacccctgac aagatagtct 660

tggttcttct aacttctatt ttaaagttaa tccttttgag attaggattc agagcatcta 720

gagtattaaa atgccttaag acatattatt gatacttatt atctgccagc ctttagtaaa 780

ccatagggac cttaagtcta cgtaagaaac aatgaacagt gattgctact tg 832

<210> 20

<211> 988

<212> DNA

<213> Homo Sapiens

<220>

<221> variation

<222> (87)..(87)

<223> baySNP739_C87G

<400> 20
ctgacggtcg accttggtat aatcctttcc tgaaaggcac ctcctccatg agcaccccaa 60

ccgccataag tgtccacaat gattttscgt ccagtcaaac cagcatcacc ctgaaattac 120

aggatttaag ttactttacg cctagtgctt tccaagtgga ttaggttgcc tattctgaat 180

tcattcagca aatatcctcc gatgattcac atgtaggttt cagaaagcct gaaaaaaatg 240

tagtagccaa aattttaagt aaccatcaaa aatccagaaa aatgcaacaa aatgacatta 300

cctgaggccc accaataaca aaatctgcca cttggctgta ggtggtagat tgtatcctca 360

tcaaggtatt tcgcaggcac aactgctttg atgactttct cctttagggc atccctcatt 420

tcatcaagac aaacctcttc atcatgctga acagatataa caattgtgtg gactctgatg 480

ggaagcacag cacctcgatc ctgcatatac tgcacagtaa ccttgaaggc aacacaggtc 540

atcagaacat cacaccaaac aataactttg tatttgtttc tctacctgaa gttacaatat 600

aggaaaacaa atgctatttt ttccagttag ctattatcaa aattatggat cttctgtgct 660

gatgtaataa atgagacaac cagctttatg atcatcactt acttgagttt tagaatcagg 720

gcgtacccaa ggcaaagtgc attacggcgt aattctgcca gtttggcatt tagcttgtgt 780

gccaagacat ggtaaaagca tacactcctc agttcatcag tggcatagcc caacattaag 840

cctaagaaag aagtcaccat gtgatcaagc tcgatttatg ttgagagatt aaacagcttt 900

acacaagaac tgcttcagac catgtctctt attttgtcag atgaccctgg gcaattttgt 960

gatgtggtcc aggtaccacg tacagctg 988

<210> 21

<211> 851

<212> DNA

<213> Homo Sapiens

<220>

<221> variation

<222>  (140)..(140)

<223>  baySNP821_A140C

<400>  21
tgtttctaga atttcaatca tttaagtaca cccacaaaac aagaatatgg agatcttctt      60

ttccctccaa tcttcttttc ccctcgactt taatttgccc agttatacct cagtgttgta     120

acagtgctgt gatacctggm acagtgcttt aatctcatga taccctctgt actgacctga     180

aggagaccta agagtccttt ctctttttga gtttgaatca tagccttgat gtggtctctc     240

ttttatgtcc ttgttcctaa tgtgaaagtg cttaactgct tcttccttgt attaggtagc     300

actgggataa gattttaact gggtattctt gaattacttt tacaataaac caagtctata     360

atattaaaaa taaaaaagaa agaaaacaaa gccttattgc tggtatggag aaagtttaag     420

tggtctggac agaagaccag gccaaccaca acattccctt aagccaaagc ctaatccgta     480

gcaaagccct aattctcttc aattttatga aggccgagag agataaggaa gctacagaag     540

aaaagtctga agttagcata ggttggttca tgaggtttaa gaaagaagac atctctatga     600

tataatagca caagatgaag ctgcaagttc tgatgtagaa gcttcagcaa gttatccaga     660

aaatctggct tagatcattg atgaaagtgg ctatgctgaa caatggactg tcaatgtagg     720

tgaaacagcc ttctattgga aaaagatgcc atctggactt tggatagaga ggataagtca     780

tgcctggctt aaagctcaaa ggaggcagta cagtggctca tgcctgttat ccacactttg     840

aagatgaagc t                                                          851

<210>  22

<211>  880

<212>  DNA

<213>  Homo Sapiens

<220>

<221>  variation

<222>  (257)..(257)

<223>  baySNP1005_A257G

<400>  22
tgaatggagc tgactccatc cttgaaatgt gtgagcctct cccctccccc actctaccag      60

cccttgcctc ttcttgccct gggcagcttt tgtgtcttgg gagccaacca gaactcaggc     120

taggtgctaa ttaaaagcta cctacctcct ttctgcagag gctgagcccc tccatttgca     180

cacagccctt gtctaccatt gctagcctca ggcaaatgac tccacttctc tatgcctcag     240

tttcttcatc tgtaaarcag ggctaacatt tcctgtcttg cctcccttct ggggtgtcac     300

aaggtttaaa ggacacatgc tgtgtatatg tagggctgca gacatgagga gtcctggacg     360

atgggcaata gctgccctcc tcagaggctg tggggcacta tcctgggatg ggtagaagtt     420

gacaatacat agctggggac agcttggatg gatgggggca gaacgaggag aggaatgggg     480

ggagcatgtg ggcccaagtg tctgttttgc tgattgctcc ttttgctttc aaggagatta     540

aactattttt agtctgcgct actgctggtg agacgccgga ggaagccttt ccatcgctga     600

gatcttctgg aagctgccaa gtgtggtctt cagctcgatt ctgggagcct cccagagcag     660

gcgggagggg tgtcaccatc tgggggctgt ggggacaggc tggtgtgtgc ttgctccctg 720

aggtcctttt gtgcgctggc ctccctgagc cgtgtggccc tggcctgcag agaacagaat 780

ctgatcaccc gatgttggac gctgtgttaa aaaaagaatt atttctaata ttccttgctg 840

cataccggca tcgcaagtgt cgctattaat attatctctc 880

<210> 23

<211> 550

<212> DNA

<213> Homo Sapiens

<220>

<221> variation

<222> (287)..(287)

<223> baySNP1055_A287T

<400> 23
tgcaaggagg attcatccgg caaccagttg aggctagatt ctcagcagct ttattgaata 60

ttgagaggtg gtctcacctc ccactggaca tgtgtcctca agttcatacc agaacttctt 120

tgggacagac agacagacag gtggcagggc agggcaggtg tccacgaggg tggggccagt 180

gcaccagggg cagctcagct ctccaaaggg gccagcatct gcacctgctc ctgctgctgc 240

tcctgctgct gttcctgctg ttgctccagc tcagggaggg agagagwctt gtcctggctc 300

tctttctcct tgaaggtgct gaagaaggag ttgaccttgt ccctcaggtc cttctccagg 360

aagctcaagt ggccttccac gtcccccgca tgggggccca gtttctgcct gagctgttcc 420

atctgctgca ccagggcttt gttgaagttt tccccgtagg gctccacccg gcgtcggaac 480

tcctccacct gctggtccag gtgcccaccc agctctgcca gtgacttctg cagcccctcg 540

gtgttgcccc 550

<210> 24

<211> 668

<212> DNA

<213> Homo Sapiens

<220>

<221> variation

<222> (354)..(354)

<223> baySNP1056_A354G

<400> 24
agcctgtctg tcttgtctga atgtgggaaa ggacaaggga ccacctaggg taggactcac 60

aggcttgttc aggccgagag ggggatccag ggtaacagca tcagtccctg ctgtgtccca 120

gattccaggt ctgaggcagc gagtaccact gggatccata gctccggctg gaagaggcag 180

gcccccatca caggacttct gcaccagcca tgcggggctc agtccaggca gtcagcacct 240

accctgggca ggcaggggtg ggtgccagcg tctccaccct gtgggctgac ctgcatgtgt 300

gtctatgtgt ctgtgtgtat gcacgtgcgt gatctgcccc ctgcagccaa acarcaggac 360

ggggcagccg ccatggagat gcagcccctc aagagtgccg agggcggcga cgctgacgac 420

aggaagaagg ccagcatgca caagaaggag aagtccgtgc tgcagggcaa gctcaccaag 480

ctggctgtgc agatcgggaa ggcgggtgag tgcagcatgt gggaggcagg ggacagggct 540

cacaggggga ccccaggcag agccctgccc catgctcggc ctcagttacc tcatctgcaa 600

agtggggggc ttgaatgagt gcctgaagcc cacctttttt taccacagca cccagtctga 660

gtcctgtg 668

<210> 25

<211> 789

<212> DNA

<213> Homo Sapiens

<220>

<221> variation

<222> (257)..(257)

<223> baySNP1085_A257G

<400> 25
ctggtggtgg ccgacaatca ctgtagtctt ggtgcccata cgaacagaat agatggggcc 60

atattttttc tgcaggcttg aagaagttgt tatgcatatg gccatgtctg gggaggaatg 120

gcaggctgcc caccaggggc agggacagga ggctcttggg gtacttggca ccagggcacc 180

ttctcttggg ccaaaacaaa taagctaggg taagcagcaa gagagccacg agctcccaca 240

tggtggctgg gtgccggcag gcaagataga cagcrgtgga gtagaagagc tgtggcaact 300

ctagggcaca aggaggcctt ttaaagggct accctgatct tcaccttgac tttgtgttat 360

ctcttgcctt gtggaaagat tctcctggag cccagccagg cctgagctca tatccagaag 420

ggagagaggc ggtgggagtg aaggcctcct caagggctgg ctcaactcca gggcaaacct 480

ccggaggagg agctaggtaa gggaggtcag ttgatcaccc tctgaggagc tccccatgct 540

tgaatgactc cagagtgcga atggtatctg ggctcaagag tcaaggcttg gaactttcca 600

tgctgcaaaa tcaaaatcac tggacagatg acagattcag gagggtcaca agtagcaggg 660

actgttaaag gacttttatg cttctttttt tttttttcag agtctgctcc atcaccaggc 720

ttgtgtgcag tggtgtgact gtccatgcag ccctgaccct ccagcccatt gtggttttgc 780

agcgataag 789

<210> 26

<211> 789

<212> DNA

<213> Homo Sapiens

<220>

<221> variation

<222> (104)..(104)

<223> baySNP1086_A104G

<400>  26
ctggtggtgg ccgacaatca ctgtagtctt ggtgcccata cgaacagaat agatggggcc      60

atattttttc tgcaggcttg aagaagttgt tatgcatatg gccrtgtctg gggaggaatg     120

gcaggctgcc caccaggggc agggacagga ggctcttggg gtacttggca ccagggcacc     180

ttctcttggg ccaaaacaaa taagctaggg taagcagcaa gagagccacg agctcccaca     240

tggtggctgg gtgccggcag gcaagataga cagcagtgga gtagaagagc tgtggcaact     300

ctagggcaca aggaggcctt ttaaagggct accctgatct tcaccttgac tttgtgttat     360

ctcttgcctt gtggaaagat tctcctggag cccagccagg cctgagctca tatccagaag     420

ggagagaggc ggtgggagtg aaggcctcct caagggctgg ctcaactcca gggcaaacct     480

ccggaggagg agctaggtaa gggaggtcag ttgatcaccc tctgaggagc tccccatgct     540

tgaatgactc cagagtgcga atggtatctg ggctcaagag tcaaggcttg gaactttcca     600

tgctgcaaaa tcaaaatcac tggacagatg acagattcag gagggtcaca agtagcaggg     660

actgttaaag gacttttatg cttctttttt tttttttcag agtctgctcc atcaccaggc     720

ttgtgtgcag tggtgtgact gtccatgcag ccctgaccct ccagcccatt gtggttttgc     780

agcgataag                                                            789

<210>  27

<211>  1756

<212>  DNA

<213>  Homo Sapiens

<220>

<221>  variation

<222>  (1687)..(1687)

<223>  baySNP1092_C1687G

<400>  27
tgagggaatg ggggagctga gacgaaaccc ccatttctat tcagaagatg agctatgagt      60

ctgggcttgg gctgatagaa gccttggccc ctggcctggt gggagctctg ggcagctggc     120

ctacagacgt tccttagtgc tggcgggtag gtttgaatca tcacgcaggc cctggcctcc     180

acccgccccc accagccccc tggcctcagt tccctggcaa catctggggt tgggggggca     240

gcaggaacaa gggcctctgt ctgcccagct gcctccccct ttgggttttg ccagactcca     300

cagtgcatac gtgggctcca acaggtcctc ttccctccca gtcactgact aaccccggaa     360

ccacacagct tcccgttctc agctccacaa acttggtgcc aaattcttct cccctgggaa     420

gcatccctgg acacttccca aaggacccca gtcactccag cctgttggct gccgctcact     480

ttgatgtctg caggccagat gagggctcca gatggcacat tgtcagaggg acacactgtg     540

gcccctgtgc ccagccctgg gctctctgta catgaagcaa ctccagtccc aaatatgtag     600

ctgtttggga ggtcagaaat agggggtcca ggagcaaact ccccccaccc cctttccaaa     660

gcccattccc tctttagcca gagccggggt gtgcagacgg cagtcactag ggggcgctcg     720

gccaccacag ggaagctggg tgaatggagc gagcagcgtc ttcgagagtg aggacgtgtg     780

tgtctgtgtg ggtgagtgag tgtgtgcgtg tggggttgag ggtgttggag cggggagaag     840

gccaggggtc actccaggat tccaacagat ctgtgtgtcc ctctccccac ccgtccctgt 900

ccggctctcc gccttcccct gcccccttca atattcctag caaagaggga acggctctca 960

ggccctgtcc gcacgtaacc tcactttcct gctccctcct cgccaatgcc ccgcgggcgc 1020

gtgtctctgg acagagtttc cgggggcgga tgggtaattt tcaggctgtg aaccttggtg 1080

ggggtcgagc ttccccttca ttgcggcggg ctgcgggcca ggcttcactg ggcgtccgca 1140

gagcccgggc ccgagccgcg tgtggagggg ctgaggctcg cctgtccccg ccccccgggg 1200

cgggccgggg gcggggtccc ggcggggcgg agccatgcgc cccccccttt tttttttaaa 1260

agtcggctgg tagcggggag gatcgcggag gcttggggca gccgggtagc tcggaggtcg 1320

tggcgctggg ggctagcacc agcgctctgt cgggaggcgc agcggttagg tggaccggtc 1380

agcggactca ccggccaggg cgctcggtgc tggaatttga tattcattga tccgggtttt 1440

atccctcttc ttttttctta aacatttttt tttaaaactg tattgtttct cgttttaatt 1500

tatttttgct tgccattccc cacttgaatc gggccgacgg cttggggaga ttgctctact 1560

tccccaaatc actgtggatt ttggaaacca gcagaaagag gaaagaggta gcaagagctc 1620

cagagagaag tcgaggaaga gagagacggg gtcagagaga gcgcgcgggc gtgcgagcag 1680

cgaaagsgac aggggcaaag tgagtgacct gcttttgggg gtgaccgccg gagcgcggcg 1740

tgagccctcc cccttg 1756

<210> 28

<211> 565

<212> DNA

<213> Homo Sapiens

<220>

<221>  variation

<222>  (454)..(454)

<223>  baySNP1096_G454T

<400>  28
cagctggaga ctgaccgagc ccggcccttc cctccagccc caattcctgc acaagccctg      60

ctattcctcc tgatggcaag aggacccggt ggactcttgg ccatgatgac tctgttcctg     120

taccagtctg ctccgttcct gaggacccta acttttctca gcggaagcag gaagacctga     180

ccatcttgtc ctattgctga ggaggagcag ctcctcctgt aagaccccag gtgggcaccg     240

agtgggggaa agcccgcgtc cggatgactg gtcttatgag aggggagagg tttttcagtc     300

atcaccgtgc ctcctcccgg ccttttcctc ctagcccaca gctcagttac atctgagaga     360

gacaaggccg agaaggagct gagggggccc ccggcttacc ttctgctgta gtacccagaa     420

caacggcagc ttcttccccc ggccgggtca cgakgcccta tttatagctg aggggtgggg     480

atggagctgt tcccaggctg cctgtgcaca ggctggagag gagggttaca tcacttggcc     540

agaccacagg ctggccagaa ggaca                                           565

<210>  29

<211>  842

<212>  DNA

<213>  Homo Sapiens

<220>

<221>  variation

<222>  (249)..(249)

<223>  baySNP1101_C249T

<400>  29
ctgctgcatc ttctcattgc atcctcattc catttcaggt taaattttgg tgttggactc      60

cttagaattc tacctacagc atctctgaca aaagcccgcc aggctccgct tgactctctg     120

cccggtgcgc tgtcagatgg cgccaagccc aagctctttc ctttgcttcc tgtagaagcc     180

cgtcctagag ccctgggctg tgaggaggag cagccccaca gggtcacact gtccttttcc     240

cttgcagayc ttggcacccg ccatcaactg gttgcctttt ctcaacacca tcttctaccc     300

cgtggagatc aatgaatccg agcctattgt ggtctatgac aaggaatacc ttgagcagat     360

ctccactctc atcaacacca ccgacagatg gtaagaggac ccccagtggc ctggggcacg     420

cctctcacag acagccctct tcttgggcag gtgggcaggg gtggggatga cagtctgact     480

caggagagtg ggacctgggc gggcttttac gagaggcagc aggacatcct ggttacaggc     540

acaggtttaa atcccagcct ctttcaaact accagctttg tagccttggg caaatttcgt     600

aacctttctt aagttcattc tcctccttca taaaatggtg atacaaatat cacctgtggc     660

cgggcatgaa cgcccgtaat gcgggacttt tgggagacag aggcaggagg atcccttgag     720

tcccctgcgt ttgagaccca gcctgggcaa catggcgaga cgtgtctcta caaaaaataa     780

aaaatagcca gacatggtgt cgcgtgcctg tcacccagct cttgagagag gaagctgaag 840

tg 842

<210> 30

<211> 656

<212> DNA

<213> Homo Sapiens

<220>

<221> variation

<222> (307)..(307)

<223> baySNP1204_A307G

<400> 30
gtgccaaggt acacgactat gcggtacagc tccaccagct tggccttctc cgtgccgttg 60

gcgtggaagg gcgggaagtc cgtcacgttg gggccacata gcttgtccag gttgttgggg 120

aacggctccc cctgggctgt gtactgaggg gcagaaggga ggtgacgtgg gagtcagggg 180

tcagtgtccc agccctgccg ccaacccttt gggcaagctc ttgcgtctgt ttccccatct 240

agcgcatgag gacccaactc cttgccctgt aagcatctgg aattgtcatg agagccaaaa 300

ctaattrtaa tgtgagtgcc cttgctaaag atcaaagact gagccatgca cgcagtcatc 360

attatcatca tcatcatcat caccacccta aggggacaga agggaaaact cggtgtctag 420

ccctagctgg ggcaccacac acaagtactt ccatccctgc actcacaatg ttccgggacg 480

cccctccatg cccagcaccc acagcccact gcctctcaac cgcatctccc tggtgcctca 540

cgcccatttc ccctccatcc ctcgctccct gcagcaggac aatcacaaga taagaagtgc 600

caggtcccca cctttgcact cagttctccc cttgctaact gggcaccctg gggaag 656

<210> 31

<211> 615

<212> DNA

<213> Homo Sapiens

<220>

<221> variation

<222> (180)..(180)

<223> baySNP1504_C180T

<400> 31
atgggcaaaa ctcggggtca ctggtctcat gctccaaggc cccgtgagca ggccaggtgg 60

ggggatccct gggggaagcc cctcaccctt ctccaacccc cagccttcgg ggagcccctg 120

ccctccaggc cctgtgctca gaggtggggg gaggacggct cagtgacttt tggttcccay 180

gaccttgaca gagacaggga cctcgacgca tcctccatac ccgctgcggg gaaatggacc 240

ccaccagcca gcacggggtg agttgggggc cagtccctgc agagtgtggg tgtccacaga 300

gggtctcttc tgcagtggcg gatcgggcgc cagcagggtc ccggcctcag tgctgcctgt 360

gcgggcgagg gtggcctgtg ggcagggctg gagctgcccc acagccgcgg gctctgcctc 420

acgcccagtg ggtgccccgt gatgtggcag ggctggcctt gagtttcctg cttctcacga 480

gcctggcacc gccaagccca ggcccctctg gcccatctcc acaacaccct gtgtggccgc 540

cacttggggc aggccctgtg ggccccaccc agcctggctg aggggcggtg gagagtgtcc 600

gcctggacgg tgagg 615

<210> 32

<211> 527

<212> DNA

<213> Homo Sapiens

<220>

<221> variation

<222> (153)..(153)

<223> baySNP1511_G153T

<400> 32
cagaaactgt agctggcggg cctcgggtct gtggacacca gttctccctc ctcaccaggc 60

actgggggtc ggggagcatc ttcggtgaaa cttgctccga ggtccaaggg gaaaccctgc 120

tcttggatat tcattttggg actctgcgtc aakgaacaat gagcgctgca ccctaataca 180

tcagtactta cagctccaaa gtgaatcaat ctgtcctgtt gaaaccatta agaggacaag 240

gattaggaaa ggtgggtcca tgaaacacga ccagaggttt tctcaagaca agactcacga 300

tgacgccctc ccgctcaaac tcaaagcaag aaactcagtt taaatgttca atcaagatat 360

attttggcca caagcatagt tctactttaa agcaatttct ggaaactttt acaactacag 420

gaatgcctct catgctaaga agggatgccc aggacatggg aatgccactc cccacaagtc 480

tcactgcaca aacatcacgg aggctggcag cttgagcacc tcccacc 527

<210> 33

<211> 663

<212> DNA

<213> Homo Sapiens

<220>

<221> variation

<222> (284)..(284)

<223> baySNP1524_A284C

<400> 33
ggtgtggtgg cacatgcctg taatctcagc tattcaggag gctgaggcag gagaattgct 60

tgagcctggg agacagaggt tgcagtgagc agagatcgcg ccactgcact ccagcctggc 120

cgacagagcg agactctgtc tcaaaaaaaa aaaaaaaaaa aaaaaaaaaa agaaaagaaa 180

agaaaaagaa aaagaaaagg aaaaagaaaa ccagaagtag gagcagcctg aagaaatgac 240

agggagttga tttcccactg ggagccctct caaagcccac acamccgcct gcctggggta 300

acagtatctc ctcggacatc ctgcaccctc ccacgctccc ccctccctac agtagtgaaa 360

gacctaggca ggatgacccc agctcctctg tgagaatttc acaccctagt gtgaagtcat 420

agccttgtaa ctttcccttt aagaactgtc agagctgggg aggctggcca agctcaggct 480

ggaggtgggg acagagggaa gaaagaaaaa aaaaaaagag agagaggcag gaaaagtttt 540

ttcagaggaa aatgcagggt ttgtccttca ccctgacgtc agatcttgct ttataaaaac 600

ccccaaggct gcggagaagc atatctggtg ctcctgatgg gcggccagtc tgggcccagc 660

tcc 663

<210> 34

<211> 609

<212> DNA

<213> Homo Sapiens

<220>

<221> variation

<222> (367)..(367)

<223> baySNP1556_C367G

<400> 34
   ctttagatca gagtattcag aagggttata actccgtcag tcatttacat gttagtagta 60
   taaattatct gagcttcctg ttctaacttt tagctcttct agcataaact cttctgtgca 120
   atttagctgc accgaggaaa cgggagtttt tctggaaggg acttttgatc tctttagact 180
   gagggaacgt cctttgggag tagaggggca gggagcatac gcaagggatt ccaggtgcag 240
   gtaaaaggtg gcactagttc aaggttttgc tgactcagtc tggtagtcag agtctgcagg 300
   agaagacagt tcaaggcagg gcctggagga ttggatcagt ttagggacag gtcaaaggct 360
   ggcttasaga ccttagaggc aggttgcttg ggtcgttgaa tgctagtctg gtgctgagag 420
   cccttttctc tggcaactgt ggactcagag ctaaccaatt gtagttggca gtgggggtga 480
   agggtgatcc agaggcctga gctgcagagg gcacaagaga gaaaagatgt cttagaaaga 540
   gctttgagaa catgccttgg ctgctggcag ggaccttgga tggggtagtc tacacccgga 600
   agtgcctgc 609

<210> 35

<211> 740

<212> DNA

<213> Homo Sapiens

<220>

<221> variation

<222> (699)..(699)

<223> baySNP1561_A699C

<400> 35
tcaagcacct gcctctacag gtacctttct tgggaccaat ttacaatctc tgggatcccc 60

aactatagaa cctggaagct agtggggaca gaaagacggg gagcctgggc taggtgtagg 120

ggtcctgagt tccgggcttt gctacccagc tcttgacttc tgtttcccga ttttaaatga 180

gcagtttgga ctaagccatt tttaaggaga gcgatgggga gggcttcccc cttagcacaa 240

gggcagccct ggccctggct gaagcccaac cccaacctcc aagactgtga gaggatgggg 300

actcatccct ggaggaggtg cccctcctgg tattgataaa gaatgccctg gggagggggc 360

atcacaggct atttgaacca gccctgggac cttggccacc tcagtgtcac tgggtagggg 420

gaactcctgg tcccttgggt atatggaagg tatcagcaga aagccagcac tggcagggac 480

tctttggtac aatacccagc atgcatgctg tgccaggggc tgacaagggt gctgtccttg 540

gcttccccat tttggagtgg tcacttgcct ctactccagc cccagaagtg gaaactgaga 600

tgatgtgtgg aggagagagc cagcgttcat gttgggaatc ttgaggctcc tttccagctc 660

tcagattctg tgatgctcaa agggtgagct ctgtgggcmc aggacgcatg gtagatggag 720

cttagtcttt ctggtatcca 740

<210> 36

<211> 1181

<212> DNA

<213> Homo Sapiens

<220>

<221> variation

<222> (389)..(389)

<223>  baySNP1582_C389T

<400>  36
ataaccacac ttttctgaaa gggagtagaa ttcaaggtct gcattttcta ggtatgaaca     60

ctgtgcatga tgaagtcttt ccaagccaca ccagtggttc catgtgtgtg cacttccggt    120

ttgagtgcta gtgagatact tctgtggttc tgaattgcct gactatttgg ggttgtgata    180

ttttcataaa gattgatcaa catgttcgaa tttcctcccc aacagtcttc cattaccaag    240

taaagattca tttttctggg actgagagtg aaacccatac caatcaggcc tttgagattt    300

ctctgtatgg caccgtggcc gagagtgaga acatcccatt cactctgtga gtagcacagg    360

ggggcggtca tcatggcacc agtccctcyc ctgccataac ccttggtctg agcagcagaa    420

gcagagagcg atgcctagaa aacaagtctt tagttaaaaa aatcagaatt tcaaaattga    480

ggtctttcct ctatttgata ttgagaaaaa aatgcttcaa attggccatt ttattttcac    540

ttactagtta tattttttta tttatcatct tatatctgtt tatttctttt ataaagctgc    600

tgttaaacaa tataattaaa ctatctcaaa aggtttgaca ttaaagaaaa tgagcaatgg    660

taacaggaaa ccactctata gatgtacata taatatgtac agaaaatata agtagtaaga    720

agtccatgac aaagtgttag ctcttttttt tttttttttt tttttttttt ttgagatgga    780

gtctctctcc tattgcccag gctggagtgc agtgattcga tctcagctca ctgcaacctc    840

tacctcccga gttcaaacaa ttcttctgtc tcagcctccc gagtagctgg ggctgcaggt    900

gcccaccacc atgcccagct aatttttgta tttttagtag cgacagggtc tcaccatgtt    960

ggccaagctg gtcttgaatt cctgatctca ggtgatccac ccgcctcggc ctcccaaagt   1020

gctgggatta caggtgtgag ccaccatgcc cagcctaccc tttactacta atcaaagaaa   1080

taaaagtaag gcaacttgat acttttacaa ttactagatg aacaaatctt taaaaatagc 1140

cagtgcagac aaggtggtga agcagaacat gcgaacctac c 1181

<210> 37

<211> 531

<212> DNA

<213> Homo Sapiens

<220>

<221> variation

<222> (443)..(443)

<223> baySNP1638_A443G

<400> 37
aactcggtgc cctgcaacac accccagttc tagacagcgt ggtggaggag acgctgcggc 60

tgagggctgc acccaccctc ctcaggttgg ttcatgaaga ctataccctg aagatgtcca 120

gtgggcagga gtatctgttc cgccatggag acatcctggc cctctttccc tacctctcag 180

tgcacatgga ccctgacatc caccctgagc ccaccgtctt caagtacgat cgcttcctca 240

accctaatgg cagccggaaa gtggacttct tcaagacagg caagaagatc caccactaca 300

ccatgccctg ggggttcggc gtttccatct gccctgggag gttctttgca ctcagtgagg 360

tgaagctctt tatcctgctt atggtcacac actttgactt agagttggtg gaccctgaca 420

caccactacc ccatgttgac ccrcagcgct ggggttttgg caccatgcag cccagccacg 480

atgtgcgctt ccgctaccgc ctgcatccta cagagtgagc ttggccaagc c 531

<210> 38

<211> 711

<212> DNA

<213> Homo Sapiens

<220>

<221> variation

<222> (648)..(648)

<223> baySNP1653_G648T

<400> 38
ttcagatttc accaattgag aattagtaag taatttctct gatacaggcc tgaagtttac 60

cttagtaaac actttacttc catatggtaa aaattagatt ttgggaggaa tgcttacctc 120

ctaaatatat tcaatctaat atttgaggac acatgggaat atatttatga ttcatctgct 180

ttttaaacat aagcctttgt taactgtaag ttcttgaact ttataaggct gctgttattt 240

aaatgagcac agctcctgat ctgcaaacag cagagcgcag ggctacagct tgggggatgc 300

cagccgactc agggtggtcc tgtggactga acaatctctt gctgctgtac tggagggcct 360

gggagctttt ccatcagcct cggcctgagg tgtgcactct tctcctgccc accccaggaa 420

taaatgagat tcctggttaa aaaggaccag agcagtcatt ttacagttga ggaaactgtt 480

gctctgagaa gtgagggatt tattcatgac tacactgatg gtgagtgccc atgtcaggtc 540

tggaaccaaa gtctacccag tatccacaca ccaccatccc tcaggtggct ctgccacagt 600

ctgatgggag gctccaaagc gggaggaaga aggaaagtct tgcccackgc atctcctcag 660

ttggccttcc tctctgcctg ttttccctcc ctacagttag catcttaagc a 711

<210> 39

<211> 2438

<212> DNA

<213> Homo Sapiens

<220>

<221> variation

<222> (398)..(398)

<223> baySNP1662_C398T

<400> 39
catgcccatg accctgccag ctgacaattc taagcatgcg caaactggcc ccaaaaattc 60

ctcccacatt tccgaagaac tatttggccc tttatgtgaa gtacctggtt tttccatttt 120

ctgttttacc ataggcctca gttcggtgtg tggcgtattt attctacatt taacaatttg 180

aagatcattc tattagatta aaaaaaaaga atacaatgga agccaagtga ttaagctttc 240

cttatgctta tattaagttg tagcatatgc atttaccgat agttaaccgt attaacctac 300

agaaaatgtc cagggaaatg gtctatttct tattctattt ttgacctaaa gaaaatcttt 360

aaaatgtctt agcattttcc ccagtctcca tccacttycc tcagctttgg cctgaagcta 420

tctttaaagg taccctgtac agctcttgcc ctgtacagct agctacagag attcaatcct 480

ttctgttcga ttaggacaca tctcagtggc agataacatg caaagttatt atatgtatga 540

accagaactt gtttttcctt aggggccagg atgttacact aaggtcttaa gactatagta 600

atatcttcac ttgaaaaagc cctctattat tcctatctca gatgataaaa attcaattaa 660

gagaaataag aacgtgacat gtgtaatcgc acctggctct acaaagctag tctggacaga 720

catttaaaca attatcctct aagattattt gatgaaatgc atttcaatga ctagttaacc 780

attaaaaacc aaagtgagca tcccatctgt tcccagtcaa atgacctaga gcaaaggact 840

aggcaaacca catctgtggg catagcaagc tgtacatcac aaacaaatga atttgctttg 900

tatatgagtg agagcaaaca ctctttattg tacaacttgg gtgggtaagt agggagaata 960

atggttttac tgaaatcgca ggtaacggtt acgttggagt taaaggttag gaagaaaacc 1020

aaagggtaag agctgttgtt ctgggctggc attgtcaatg aagagcataa attcagatgt 1080

gaatgtatat tttgtagaag catgtgtgtt gttggttttt gtgtatgtgt gagtctgaaa 1140

gagggaaaac aggctcccat tagactatga ctaacaaaaa tgtttgacag attataactc 1200

agatgtctta ctcagagcat atgccttccc attttcccca ttattcccca acatgatgtc 1260

tttaagaact tgtccttgac cgagcagaca tctcataccc caaatagcta atattttgat 1320

agctatgatc ctgaacggcc aaacattcca aaaccaagta gtttgtaata tctttaaatg 1380

caaatatatt ttaggccttt tccttggcaa ggatgtttgg tcaggggttg gcaaaaataa 1440

tgctcttcag acttaaaaga acacaaccat atttcttagc catccaccag aaagtagtag 1500

aacgctccag gaagcaagtc tttgtcagga gtcagactag ctacatcata atctctctgc 1560

ccaggggctg tggatgtcat ccatcctggc ctaactagcc tactgagctg agagatgtcc 1620

aatttccccc caatacacta accagaggag aaggaccgtg atatcattgc atgtgaattc 1680

ttaattccaa ttgcttaaac aaatatgttc agttgtaact atcaatacca gtatataaca 1740

gtgttggcca agttttattg atgctgacaa tcaattggag ttacagccag acacatggtc 1800

ttatgaccgg cgtacttacg cagggctttg cactgagaca ggtcgtgcat ctgaggttta 1860

ctgctttgca tttttgtttt gtaactgaag tctgatgaga cagccagagc atgtgctacc 1920

tagggacttg aatccctgca gccccatttc acttctcacc accttccggg gtggtttctc 1980

gacctcccac tcccctacca cctggtgcct tagccagccc tggctctccc tccaaacacc 2040

tgcccaatga gcactgccac cccatggtgc ccagacatgc tctccctcct catccctacc 2100

tagctaccat tgccactccc ctcccccagc ggggacatgg gcataggagc agggagagtt 2160

aaggttggtc aggtgcacgt gccctatgct atcttggaag ggggcttggc catgtggcat 2220

ctctggacca agaatgcgcc acagcacatt tggagggtga atggtggggg cacacccctt 2280

gtccacctct atttcaggca tggaacacat cctggcatga aagttgcagt cccttgggaa 2340

tcacctctcc accttgattg ccacagtagg ccagtgacaa gggaagattg acacatcatc 2400

ccctgctggg gcccagtgtc ctgtggctgg caggcagg 2438

<210> 40

<211> 794

<212> DNA

<213> Homo Sapiens

<220>

<221>  variation

<222>  (376)..(376)

<223>  baySNP1714_A376G

<400>  40
tcggtacccc tgtttaactt ccaccgaggc agggggcccg agcagggaca gaagaggccg      60

gggtgaacat caccctttct acagtggctc gatgccttgc ttcacactcc agttttaatg     120

ttcgtgtgat acttgctatt ctctttctag aacattgccg ttaggagagg gtcccaccct     180

ttgtgttcgc tccctccctg ccctcatctg ctctgttgac aaacgttttc tgccagatga     240

agtgtttctg ctgtgcctgc ctctctggga gggaacgtgg agcagctccc tgagcattca     300

ggctccgtgg ggaagtcgga ctgattttaa atagatcccg gttttccagg cttggagctg     360

aacggcatga cggggrcgga cgtggccgat gtcccacccc ctctgcctct caaagcagcg     420

tggcagatta cgggaatttg atggaaaacc aggacttgct gggctcgcca acaccttcac     480

ctccccctcc acaccagagg gtaagtcggc aatctgaaac acaggctttc attgcttcgc     540

ctccacaaag taggacgagt ccctcattcc agatactagc aggtttactt cgggctagtg     600

agcacatgag gaaaaccatg tatgatgggc cccgggtttg attttgcaaa aagatatcga     660

agcagtaagg actccagtgg gtaagatctg tggctctgcg tcattatcaa taactgcatc     720

aaaaagcaca ttttgatatc atgcctcaga acgaggaaga ggatagacat ttccaggttg     780

tcatcggtta tcat                                                       794

<210> 41

<211> 813

<212> DNA

<213> Homo Sapiens

<220>

<221> variation

<222> (89)..(89)

<223> baySNP1722_C89T

<400> 41
tgtttatcct cctcatgtcc cctccccacc caagttccag cttgcctggg gcttgttctg 60

ggaccctaag aaccccagga tgcccacayc gccaggccag cagggccaat ggtcatgcag 120

ggactgcggg acaggttccc atcatctcct gtgttagccc attccacctg ggaaaccaag 180

cgactgccgt ggctggctta tttctccctc tgccaacccc catctacaat gttctgggga 240

aaacaatgag aggtggaaaa ggtaacatac aaccaccttc cacccacatc acttccagtg 300

aggcagctaa gtccaccaac agagcgtggg agagtctttc atccaaaaat cccaacacgt 360

cgcatgcagc gacaggaggc tgtgctgggg aaacccaagg cctggctcag cctccatact 420

ctaagggcct cgggccagaa cgaccacgag aggggagcac ccaaaggcac gcagaggggc 480

ccacctgggt gcactgcaga ggggacagag gggctgtgag tgggaggtgg gccagtgctg 540

ttcactgccg cctgctcagg accaggggct gctgcagacc cagcccggtc tatgcccacc 600

cccgcaggtg cagcccagct tctgcacatc agatggagtg acgctgacta aaggacctgg 660

acagcgccgg cagatactcc caacactgtc cttctctttg ggagccaaag agaagccacg 720

cagatactcc caacactgtc cttttttttg ggagccaaaa gaaaaccccg gccaggcagc 780

caggcgagcc atttctccta caccaggcag ggg 813

<210> 42

<211> 524

<212> DNA

<213> Homo Sapiens

<220>

<221> variation

<222> (210)..(210)

<223> baySNP1757_A210G

<400> 42
ttctggaatg cctctcttgg cacccctgcc ccaacaggct gctgggatct gcacgtggaa 60

tcacagggct ggttgcatgc aacggcaaag ggcatgctct tctgcctggg ggagggcctc 120

ttgtccctcc cagcaggtcc cgggacaggg ctggcagcca ccccagcacc caaaataaga 180

tgacaaacaa gaggaaacaa accaaaatgr aggagagcca tccgggctgg aggagggcag 240

aggctcccca aggggatctg ggagggcttc caacttatga atgcatcagg ccttggaaga 300

cgtggatgga agaggcgagg gcaaaaggaa gagatgaggg gcatggagag agactcaggg 360

aagaaggaga aagagcaatc atgcagcttg ggacaaatct tttgttgctt tatcagattc 420

tcagtcaatc aattggtgtt tgctagaacc ggggtacgca ggggagtgtt gaagagagag 480

tgcgcgcgcg agaagagaga gatcaagaga acgggcatcg ccag 524

<210> 43

<211> 1279

<212> DNA

<213> Homo Sapiens

<220>

<221> variation

<222> (240)..(240)

<223> baySNP1765_A240G

<400> 43
gtatctgtgt gtctgtgtct gtgtgtgtgt gagtgtgtgt ccttgaccac gtgttctcca 60

taggcgggga gtcctgcagt tactcatgga tgagtacctg tgtttgtgtc tcagcaggca 120

aatttgtaaa tgtacaagcc ttcaagcctg cacaagcata aatgtcactg tgtgggggtg 180

cttgagagtc tgtgagcctg cccttaggag ctgtgaggta tgggcttcac ggaggaagar 240

gtcatagctg caggaagata tactgtcttg ggaccttcag ctgcatcttg acatctctgg 300

ctccccatgg aaagggcatc cctagctgag atgcaggacc tctgagaaga ggcaggggtt 360

aaaggatcag ataagccctc catttccccc atccaagtga agagagaagg aggggaggta 420

gccccctacc ctgctccatc ttagagcaag gtagacccag ctcaggaggt cttgcttggg 480

aagtgatgag accttgactt ttccagtctg tctttttccc ctagcacccc caaactcccc 540

ttaatcacca tcctaagttg ctgtgggtga tgcaatagca agatgaggag cagatctggg 600

ctgtttaaac taagaggctg ggtgaggtgg gggtatttag ggcctggagc ttagagttca 660

acctaccaac gacccctgaa gagggaaggc atctgacacc cacaacctgt tctagggatg 720

atttttcctc caatcccttc tcccctgcat ctccactgca gaggcaggct tcactgtccc 780

cccattaccc agtggctgtg aagggcagcg tgggagttgg gggaagggac gacactggtg 840

ggagggagcc cagcctgctc cagctaccac ggagaggctg agatgggggg agcgttggcg 900

gattcccagc tgcccccact ctgtcccagc ctctggcttt ctcaaaaagg actctctgtt 960

ctccttcagc attcaagacc cagagagggg gacttgtggt tgggggaggg aggagtggag 1020

ggaggttggg ggggtccttg cttcctctct ttctttcttg cctgggcagc cgctggcccc 1080

aaatctctgc aggctcctgg ctgcagagcc tgagatcttt gccaggacag gaggaggggg 1140

aaggggcagt gtgtctcaag ctctaagcct gctggagagc agggcgggag cttgggaaaa 1200

ggaggcactg cgtggagctg cttagctcag ccacaatcca gcatgccaaa gtgcatggac 1260

cagcaagttt ttaaaaagc 1279

<210> 44

<211> 359

<212> DNA

<213> Homo Sapiens

<220>

<221>  variation

<222>  (200)..(200)

<223>  baySNP1776_A200G

<400>  44
taaggaggca atctgtaaaa ctagcacagg tctcccgctg ttccactggc tcacccacat      60

gattagcaga gtgcacgaaa aaataaaact tctattaaag aatcatgctg agcacaagat     120

cagagaggtt gtgacattgc aaactcgata gatgcagggg gcctgggaga ctggcgttct     180

ccaaagggct cccaacaccr tctctcctct gatttctgtg acaaatgtgg aaagctactt     240

gcttggaggt actgggggaa ctgatggggg aactttcatc ctgttaggaa ctccgctttc     300

cattcctgcg tctctgtctt gctccctgga gatggatgga tcacggaggg ggccacagt      359

<210>  45

<211>  437

<212>  DNA

<213>  Homo Sapiens

<220>

<221>  variation

<222>  (127)..(127)

<223>  baySNP1799_A127G

<400>  45
taaagcagga ataggttaga ggagggagag agctggctgt gtcaccatgg aaaagtgaaa      60

gccaagtcca cctacttact ctgcggaaac tttggtgggt tgtcattgac atcggtcagt     120

gtgatcrtca ctttggttgt ccctgagagt ccgcccatat gtccacccat gtccttggcc     180

tggatcacca cgtggtactc ctccttggcc tccctgtcca tgttgggtag ggctgttctg     240

atgatacctg gacaggtaag caggcaacat cacagatatt cgtttataac attatgacaa     300

caagaaagtt ctgagcactg aggaagcaat gctgaataaa atatcccatg ccctcagcaa     360

gaatcatttc aacatggtaa ttacaaccaa aggcagccat actgaagcat cattatgtct     420

tttcaagcaa atcataa                                                    437

<210>  46

<211>  752

<212>  DNA

<213>  Homo Sapiens

<220>

<221>  variation

<222>  (201)..(201)

<223>  baySNP1806_A201G

<400> 46
ctgggggttt gggacagaac acacgcagct tcagtcagtt ggtaaacggg tccctttcct 60

ctggggcaga aacgctttgg ggtttgactc aaatcatgga ctccttgggg gcctattctt 120

cgggctaact ctttgcatgt tctgcaggga gccaaacaag ccgctcctac acttcgctgg 180

tcctgggcgt cctggtgggc rgtttcatca ttgctggtgt ggctgtaggc atcttcctgt 240

gcacaggtgg acggcgatgt taattactct ccagccccgt cagaaggggc tggattgatg 300

gaggctggca agggaaagtt tcagctcact gtgaagccag actccccaac tgaaacacca 360

gaaggtttgg agtgacagct cctttcttct cccacatctg cccactgaag atttgaggga 420

ggggagatgg agaggagagg tggacaaagt acttggtttg ctaagaacct aagaacgtgt 480

atgctttgct gaattagtct gataagtgaa tgtttatcta tctttgtgga aaacagataa 540

tggagttggg gcaggaagcc tatggcccat cctccaaaga cagacagaat cacctgagcg 600

ttcaaaagat ataaccaaat aaacaagtca tccacaatca aaatacaaca ttcaatactt 660

ccaggtgtgt cagacttggg atgggacgct gatataatag ggtagaaagg agtaacacga 720

agaagtggtg gaatgtaaaa tccagtcata tg 752

<210> 47

<211> 527

<212> DNA

<213> Homo Sapiens

<220>

<221>  variation

<222>  (413)..(413)

<223>  baySNP1837_C413T

<400>  47
ctcaccagtc ccccttctga gagcccgtat gagcagggag ccggccccta ctccttctgg      60

cagacccagc taaggttcta ccttaggggc cacgccacct ccccagggag gggtccacag     120

gcatggggac ctggggtgcc cctcacagga cacttccttg caggaacaga ggtgccatgc     180

agccccgggt actccttgtt gttgccctcc tggcgctcct ggcctctgcc cgtaagcact     240

tggtgggact gggctggggg cagggtggag gcaacttggg gatcccagtc ccaatgggtg     300

gtcaagcagg agcccagggc tcgtccagag gccgatccac cccactcagc cctgctcttt     360

cctcaggagc ttcagaggcc gaggatgcct cccttctcag cttcatgcag ggytacatga     420

agcacgccac caagaccgcc aaggatgcac tgagcagcgt gcaggagtcc caggtggccc     480

agcaggccag gtacacccgc tggcctccct ccccatcccc cctgcca                   527

<210>  48

<211>  600

<212>  DNA

<213>  Homo Sapiens

<220>

<221>  variation

<222>  (323)..(323)

<223>  baySNP1870_C323T

<400>  48
aacagctatt cacgcttgtt gagtgggcga agaggatccc acacttttcc tccttgcctc      60

tggatgatca ggtcatattg ctgcgggcag gtcagtgacc ttggatccct ttgacttctt     120

gacatttgac ccctctttga cttcccgatc tttagtgacc ccagtggcct taccttgcgt     180

acccagggag ccaaacttgc tgacctcgcc acctcttttc tccttctctt ccactgatgt     240

gctttgaatc ccttggcctg atttctggct cctgaccctt gctgccccac ccaggctgga     300

atgaactcct cattgcctcc ttytcacacc gatccattga tgttcgagat ggcatcctcc     360

ttgccacagg tcttcacgtg caccgcaact cagcccattc agcaggagta ggagccatct     420

ttgatcggtc agtggccctc ggctaggctg gcatgtagat agagggggtg gggctatagg     480

ctggtccgtg tccaaggctg gctgagctgt gacctttgag tgacctgcag gtccctctcc     540

agggtgctga cagagctagt gtccaaaatg cgtgacatga ggatggacaa gacagagctt     600

<210>  49

<211>  725

<212>  DNA

<213>  Homo Sapiens

<220>

<221>  variation

<222>  (115)..(115)

<223>  baySNP1882_C115T

<400>  49
tgatcctgag acctgtaccc tttaccccta gggcctgggg cctcacctgc tggcattggg      60

aggaaccaag ccgcgaattc tcagcaccgt gccagggagg atgccctcgg gcagygagga     120

cttgtggggg acgttctgca agagtggggt gcaggggcca ctgtgagccg gtgagaccga     180

aggagggagg cagaaggtgg gtcacccctc agaggagccg accagggctg agactgacag     240

aaatcagtgg aagaggaaag ggtggcggag aggcaggaaa gagaccagaa acagacaggg     300

aaacagatgg ggagagcaac aaagacaaac agaaagatcc tcaaagaggg ccccagagga     360

agccacaaag aggtagaggg gacagtcggg atgggggtta cccggaggcc acagcccagg     420

ccagtccgtg gcacactcac tgcccacttg ctcctttgaa agaggagccc ccagccccct     480

ccctgcttgg ccccgccccg ggcccctgga gcactcacgg acatggctgg gaccgggttg     540

ggcagccgtg gtggtggggc ctgctgggga ccttaaaata aaagcagggc ggggctggcc     600

ctgatgactt accccaccct ttccacccac cacccaaacc tggcacgacc ctggccctgg     660

ggcaagcctg gcgaaacggg agtgggactt ttttgggggc ctgatccccc atcacccagg     720

cccgg                                                                 725

<210>  50

<211>  493

<212>  DNA

<213>  Homo Sapiens

<220>

<221>  variation

<222>  (214)..(214)

<223>  baySNP1988_C214T

<400>  50
tgctctatag ctgggccctg aagaatggag caaagacctg tggcaaggat aaatcaaggt      60

ctgcagccgg gggtggctgt gggctgtgat tgtgtttggg ggcggccatg tgtctctgac     120

cctgtgtttt gccccaacta cagcacactg ggatgagaaa ttccaccaca agatggtgga     180

caaccgtggc ttcatggtga ctcggtccta tacygtgggt gtcatgatga tgcaccggac     240

aggtaggtgc tgtgaggagc agggtgtcaa ggtgggtggg ggtccaaggg tagttggtct     300

gacccacccc tgcacacagg gtgcccagtg tcctttccgc tcctctccca ggcctctaca     360

actactacga cgacgagaag gaaaagctgc aaatcgtgga gatgcccctg gcccacaagc     420

tctccagcct catcatcctc atgccccatc acgtggagcc tctcgagcgc cttgaaaagc     480

tgctaaccaa aga                                                        493

<210>  51

<211>  1161

<212>  DNA

<213>  Homo Sapiens

<220>

<221>  variation

<222>  (349)..(349)

<223>  baySNP2000_C349T

<400>  51
aaccacccta accctgactt ccctaattcc ccccatcctt accaccctcg ttaaccctaa      60

caaaaaaaac tcataccccc attatgtaaa atccattgtc gcatccacct ttattatcag     120

tctcttcccc acaacaatat tcatgtgcct agaccaagaa gttattatct cgaactgaca     180

ctgagccaca acccaaacaa cccagctctc cctaagcttc aaactagact acttctccat     240

aatattcatc cctgtagcat tgttcgttac atggtccatc atagaattct cactgtgata     300

tataaactca gacccaaaca ttaatcagtt cttcaaatat ctactcatyt tcctaattac     360

catactaatc ttagttaccg ctaacaacct attccaactg ttcatcggct gagagggcgt     420

aggaattata tccttcttgc tcatcagttg atgatacgcc cgagcagatg ccaacacagc     480

agccattcaa gcaatcctat acaaccgtat cggcgatatc ggtttcatcc tcgccttagc     540

atgatttatc ctacactcca actcatgaga cccacaacaa atagcccttc taaacgctaa     600

tccaagcctc accccactac taggcctcct cctagcagca gcaggcaaat cagcccaatt 660

aggtctccac ccctgactcc cctcagccat agaaggcccc accccagtct cagccctact 720

ccactcaagc actatagttg tagcaggaat cttcttactc atccgcttcc accccctagc 780

agaaaatagc ccactaatcc aaactctaac actatgctta ggcgctatca ccactctgtt 840

cgcagcagtc tgcgccctta cacaaaatga catcaaaaaa atcgtagcct tctccacttc 900

aagtcaacta ggactcataa tagttacaat cggcatcaac caaccacacc tagcattcct 960

gcacatctgt acccacgcct tcttcaaagc catactattt atgtgctccg ggtccatcat 1020

ccacaacctt aacaatgaac aagatattcg aaaaatagga ggactactca aaaccatacc 1080

tctcacttca acctccctca ccattggcag cctagcatta gcaggaatac ctttcctcac 1140

aggtttctac tccaaagacc a 1161

<210> 52

<211> 2915

<212> DNA

<213> Homo Sapiens

<220>

<221> variation

<222> (338)..(338)

<223> baySNP2071_A338G

<400> 52

aacattctat atggctaact ctgaatatga tcttagattt taatattttt cacacggcat 60

gccaatcccc ccaaaatttc tcctgctgtg accttcaatg acctacattt gttacacttg 120

agcaaaatca agttcattgc tattctcagg ctataatgaa aattaaggct atttctgttt 180

ccttcacatg ccccaaaatt acacaattag aattaggact tctattattg aatgttagtt 240

gtagtaatgt tcagcatacc atgcaaattt tgctgaagta tacttaattt gactgctaaa 300

atgtgtgata tccctagaca ggatttacat tatgaaartc acaggaaaca atttttatcg 360

aaagttgaaa ctaaaaatcc tttgcaggac tgtcaagcag agaatgggta ctcacgtttc 420

ctttaaccac ataattagaa tcattcttga tgtctctggc tagaccaaaa tcacaaatct 480

ttgtgatccg accatgagta aggaggatat ttctggctgc caagtctctg tgaatacact 540

attaggttgg aggagaaaag aaaaccattt aaattcattt taacttgtaa agagtgaaaa 600

ctaacttttt aatactatag tgttgcttat attataaaaa atccaatact tttgttttaa 660

agtattcagt acaccaaaaa taaaaatcaa aagtacgcct tgaatgatgt tcacataatc 720

aagagtctaa ttattataat ctcttttaaa agcaattcta ataaatgctg ttttcttctc 780

aggactgctt tcattcatag ggaaatacat aagaaatagt catatcatat tgtttaaact 840

gaaaattcat tttgcatgca tctctttgaa tgtcctttca cagagttcct aatatgcctt 900

tgcaaattca atctgacatc tcccaaggac acacggaaga aggtctttat ttcagccatc 960

ttatatcctt tcttagtttg gtaattgttg ggaaatgtca cccatgacat ttaagggtag 1020

gactggtaac tcattctgtt cggttaatct tacttcctaa ggaactcctt tcacccttcc 1080

acagaccatg ctccttgttt tggttccatc attctttcca ttcctaacct tctcccaagt 1140

gtttattact ccctctgacc ccttatttcc tgacacttca taagccacac atataatatt 1200

tggatctaac ttcaataaaa ctcaagtcta tgcttcaccc caagccttgt aactatgagg 1260

tgccctctgc ttttactgat aaaatgattc tgttctctag gaacatatcc tatatcctaa 1320

aagccaaaag ttttaaaaga atttcactgc atataaaaga atattcatag aaaaactcat 1380

ttagacaggc ttgtacttta cagcttataa attagaacag gaattccaaa gagacagcag 1440

ttggaacatg aagaaactaa atttcctctg cttttggtta ccacacttca aaatgacatt 1500

ctgcatttta actatggctc taaaatgctc tgttctcaaa aaaaaaaaaa aaaaaaaaaa 1560

aaatcaaaaa aacaaaacac aaaactcttt agagaatcac tcccacttac attcttggag 1620

gcgaggaaag ccatgccctt tgccacctgg taagaaaagc tcagcaagtc ttctaagtct 1680

agggccaact cgtcatcctc catgatggcg ggagtcacat ctctttctat gtatgagcct 1740

gtgaggaaga gaggattttt cttagcaatt acaaccataa aggtcaggaa aggaaaagac 1800

agtggcaatg acataccaaa ggctggtaat gaatgaacta atgtgaactt gcaggcagat 1860

cacttccctg gtgtccagga aaggtagatg ctgaatcaca aagttaatat ttaacaatcc 1920

taggctgggt tacaaaatcc atagaggaga gggatggcga attaaagcaa agatcccata 1980

tctatagaaa gatactcagg cacaaaaaca tatgctctga ccccaaacac ccacttgcaa 2040

ccctaactgc cattgaccat tggcactgct accataaagc agaactggtc tgcatcattg 2100

ctactgggaa tgatgttata ttccacatta gcatgatata catactctct gtaaagttac 2160

tcttggttgc ttgataggta ggtactcacc tattctcaca gatctccttt tgtcggcctt 2220

ggttgggaca acataagaaa ctccaggttt catgtccatg tactcattag tactatcgct 2280

gctgggagag atgaaacaag tcatgactga atcaagtggg acatgtagaa gggcactcat 2340

gggtcatgct ctacctcctc atcccctttg ctacataata gactggacca atttgaggaa 2400

agtcatgtaa agtttgaata aagagtttga aaatcctgtt ctgtaagaaa tgagttttta 2460

ataatcgata ttggggtcca tgcccagaaa ctaggtgtcc tttttgtgta taatcatata 2520

gaggactcag gttagaaatt tcccgtctct tgacacaatg aatttaatgg gagacagaga 2580

gccaaatatg gtatcccgta ttacctcaaa ctctctaaat gagccccagg agacaagatg 2640

aaagaaagac aaagaagagc ttccaagacg tgttatgcag gaggcagaga tcgttaacat 2700

atagtgactg atggaggaag gtttaaaaaa aaatcaagtt ggttcagata aacaccaata 2760

atttggatga ttttctgaac catctggacc tcttcaatct gcaacatttg gctggagact 2820

cttcaagatt tctagtactt cctgcttctg attagcatag aaacaccatg acaacaatct 2880

ccttcagagt gtgctggtgg ttccaatccc atatg 2915

<210> 53

<211> 1557

<212> DNA

<213> Homo Sapiens

<220>

<221> variation

<222> (876)..(876)

<223> baySNP2078_G876T

<400> 53
tacctcccca caaagccttg tacacaaatg cttataatgc ctatattcat tatagtaaac 60

aattggaaac catccagtct acatccatgg gtgaatcgat acattgtggt acatagatac 120

aataaacact acttagcaat gaaaagaaaa aacggatgca cgcaatgaca tggatgcttc 180

tcaaagtctt catgctaagt gaaagaagcc agatatgaac aagtacatct ggtatgattc 240

catttacaat atataagtcc tcaaaacatg atttcttagg taaacagcaa catataggga 300

tcaaaatcag atgagtggtt gtcaaggagc agaggtgagg ggtggagcaa aggggtacta 360

ggaaaatgct tggttgttgg aaatggtctt tgtctttttt taaagtgtta gaaataatgt 420

tttattgtca cccagatggt atttgagttt atagtcttca tgctttatat ttttttgtaa 480

attaaaaaaa ttacaagttt taaatagcca atggctggtt atgttttcag aaaacacgat 540

tagactaatt catgaatggt ggcttcaagc ttttccttat gggctccaga aaattcaccc 600

accttttgtc ccttcttaaa acactggaat gttggcatgc attcggcttc acactctgaa 660

gcaacatcct gacagtcatc cacatctact tcaaggaata ccacgctgga atactttcca 720

gagaggggat aaagaaaggc ttgatcattt tgcaaggccc acaccacgtg gccgagaagt 780

caactattac aagtttatcg cctgtggcat ccaaggcttc ctaaaaagca agtttgctct 840

cgatctcctt caccatcttg gctgctgagg tctgakgagc ggcttttttt tttttaatag 900

ggaactgacc gtattcattt gtagcctgtt accccataat tctttaggat gttcaaagct 960

ggagtgacca tatgcccgtc cctgttcctc ttgacactca gtgagaggca gccatctgag 1020

taagtggaag tgtgatattc gctccgggca ttatgtcatg agccttaaat ccagaataca 1080

ctggattgag tctttcagcc caatagtttg ccgccgttgc cagatttggg agcatccgag 1140

gagagacctc aaatcataat ggggtggtgc agggctcccc catactcaaa aaactttccg 1200

tgtgttttcc aggcaagtgt ctttctcctc gaatacaaca atcttggtta caactgacag 1260

tagtgcaata gatcggtcag tacactgatc caggaacatc ttgatggtat agcatgttgt 1320

cattcgcact ctaccagcca ctagtctaac ctggtcgagt agctttttcc agggactcaa 1380

attctccatt atcaaagcac agacaagtgg tccgacccac ggtctcgaat taaaccagtt 1440

agtcaccagc cctgcgttgt cattgagtga caggttggca ccatccctgt gtgttggtcg 1500

ttggagagac accatcaggg tcttgaatca tttttgagcc tagagactat tagcatg 1557

<210> 54

<211> 503

<212> DNA

<213> Homo Sapiens

<220>

<221> variation

<222> (415)..(415)

<223> baySNP2085_G415T

<400> 54
gggcagtccg tatggtggtg gttatggatc tggtggtgga agtggtggat atggtagcag 60

aaggttctaa aaacagcaga agagggctac agttcttaag aggggagaga gcaagaagtt 120

gttaggaaag ctgcaggtta ctttgagaca gtcgtcccaa atgcattaga ggaattgtaa 180

aaatctgcca cagaaggaac gatgatcgct agtcataaaa gttactgcag cttaaacggg 240

aaacccttct tgttcaggat tgccatagcc acagtttgca aaaagtgcag ctattgatta 300

atgcaatgta atatcaatta gatgtacatt tctgaggtct tttatctgtt gtagcttttt 360

cttttcatta catcaggtat attgcactgt aaattgtggt agtgttacca gaaakaaaaa 420

attaaagaat ttttaacttt tcaaaaaaaa gaaaagaaat aagatttctt taagttcttc 480

agtgtctctg attctaaaag agg 503

<210> 55

<211> 553

<212> DNA

<213> Homo Sapiens

<220>

<221> variation

<222> (183)..(183)

<223> baySNP2095_C183T

<400> 55
ggtaaatacc tctgtattga gaaatattca ttcttgcaga tctatgtcgg tcttccaccc 60

attgggttac aatatcccaa acagagagct agagattgag aaaattggtt gagataaacg 120

atgagttatt tgggtgtttc tcctcctctc tatattttgt gtaggtgtgc accgggatat 180

ttygggaatc cccagaaatt cggaggtagc tgccaaccat gcagttgtaa cagcaatggc 240

cagctgggca gctgtcatcc cctgactgga ggtaaggccg acccacaccc ctgctaactt 300

gcatttataa agctggtata acttaacagc ctaagaaccc aagcacacat cttcaggatg 360

cataatacac aattccagcc agcaagctgt ccccaggatg cataatacac aattccagcc 420

agcaagctgt ccccttgttt tgacaaacct gggaatgctt agatagtatt ttcagctttg 480

gtgaataacc tgtttcaatg cacttttttt ttttaaaaag tgaattgttt ttgaaactta 540

cctaattata acc 553

<210> 56

<211> 275

<212> DNA

<213> Homo Sapiens

<220>

<221> variation

<222> (67)..(67)

<223> baySNP2119_A67G

<400> 56
gtcagccatg atgtagggct ggaagtggtt tttgttcagg ctttcgtcca gcacttctgg 60

agccatrtag cgtttggtgc ccaccctggt attcaagggc acatcaactt catttgtgtc 120

actgttgaat ttaacagcaa ggcccaggtc agcaatgcag caactcccat ttttcttgat 180

gaggatgttt ttgctcttta ggtctcgatg agcaattgcg ggctttcctt gggtgccata 240

aatttctgtg tgcaggtggc acagaccaca ggcag 275

<210> 57

<211> 393

<212> DNA

<213> Homo Sapiens

<220>

<221> variation

<222> (176)..(176)

<223> baySNP2141_A176G

<400> 57
gaatggccat caggacctat aaaggctgag gaagaaaggt ttggtctgca ctacccctac 60

ctgtgaccac aagctccagg gggtcgctgg gggctgacca caggtatggg tccctgctgg 120

agaagctgta gcatcggtag gttccgctgt gggcggcggt caccgtgatg atgggraaac 180

tagccctgta ccatctctcg ggattcttgt agggcgcagg gtccccttcc ttgtacagag 240

caaattggtc aaagccatac cgagtctgac actgtagggt tacgtcccct cctgacgaca 300

ccgccgggcc gggctgggct gagagcgagg gtttggcaaa aactcctggg agaaaaagaa 360

agtctgatgt tgaaggcagg agccagcatc tca 393

<210> 58

<211> 423

<212>  DNA

<213>  Homo Sapiens

<220>

<221>  variation

<222>  (318)..(318)

<223>  baySNP2182_A318G

<400>  58
gaattcccat tcaggaccca gacctgaaac ccagggaatc cacccctcag accccttatt      60

tttgggagaa tgggttcccc cagcttcctg ccccctgcag cgtgtctcct ttctctctcc     120

ccttctgtca tgtttccata tggccctagc ccctcaggtg tgttctcacc agtcagctcc     180

attctggccg gggtgtctgg ctggcgtggc tccctgtttg gggcctctcc cctgaatcct     240

tcctggggcc atggaggcgg cttaggctct tgcacttctg ggcagagtag ggtggggcaa     300

aggcgggcca agggtgarga atctatcctg aaagtagcaa gaagagtgaa catttaacca     360

agtttctagg aagagactgc ctggcagggt gaacagatgc tggggagggc ttcagatgct     420

gac                                                                   423

<210>  59

<211>  542

<212>  DNA

<213>  Homo Sapiens

<220>

<221>  variation

<222>  (296)..(296)

<223>  baySNP2234_G296T

<400>  59
cccccagtct accccacctt cctggggtct ggcccagcca ggccccccgc accgtctatg      60

ggtgtccggg ccacaggagg agcttccctg ctgtgtcgga gcatgctggg tgcaggggca     120

gggttgttcc agggcgctat ttcagaggca gcatggggac acagaaacaa ggacagggtg     180

ggccacaagg actgtcttgc ccactgctcc agggggcaca atatctgcca ggaacagtgc     240

gcctcacaac acaatgctgg ggcgcccaag aacagtgtga accagccccc tggaakcaag     300

acagaaaggc acccggcctc tccacaaatt ggcccagccc ctgcagcctg gaccctgaca     360

ccctaaagca agtcacagta ggggatgggg gggggtggag caaggccccc cactcccact     420

caggcctccc cattctctca gatccgaccc ttctctgagc ttcacccgta aggcttattc     480

cacttgtaac attgtcgact tccagaccag gccctgctaa gccctggccc tggcgcttgt     540

gt                                                                    542

<210>  60

<211>  859

<212>  DNA

<213>  Homo Sapiens

<220>

<221>  variation

<222>  (227)..(227)

<223>  baySNP2281_A227C

<400>  60
aatctgtgag gctcaccagt caggcacacc aactcaagac tgaacaacaa aattagtgct      60

gcaaagaagc agagcgtctc cctggctgag ccgtggagcc acagtgccca gtgctgggta     120

ttcagttgga aggtgccagt cacaggggct gcagtgtcaa acaggtgttc agagcactga     180

cctcaggctg cattggagag gactgatcct gtttgcatac cacaaamcca ggtctccaag     240

cctgctcagc aatgcatgag tcacctggaa ttctttgaat atccttttca atatccattc     300

tttgaacatc cgtttcttta taagttaacc ggagctggtt tatgttacct gcaactaaga     360

accttgcctt atatagaagt tggtgtcagg agtggttaca ggcagtggat gttggaggaa     420

gctcggagaa gctggaattg gttatctgga ctgcttgggg ctgaagtcag taaaaattca     480

gcacatcata agggctcaga tctggaagcc atggcatgca ggggaagcag aatgtcagat     540

ttgccacctg gagtcaccgg gaaggaagag cccgttgaaa acaaagcttc attgagaatt     600

cattgcctta gaaaagaatg aaaggaatga ggaattagac tgaatgggct ctacagcact     660

gcactgttaa agatgcagaa gcccgtctcc taaattccta aattcttggc tcagacccag     720

atcaaaaact gagacttctg ggccaggtgc agtacctcat gcctgtaatc ccggtacttt 780

gcgaggccga gcaggaggat cacttttgct cagagtccga gaccacctgg gcacatatga 840

gaccccatct ttactagta 859

<210> 61

<211> 371

<212> DNA

<213> Homo Sapiens

<220>

<221> variation

<222> (77)..(77)

<223> baySNP2298_A77C

<400> 61
gccctccgag agcccagtga ccccacaggc gctggtacct gctagtgttt tctggttgca 60

tatttctatc atgggcmttt caagtgcttt tagatcaagt ctacacggtg cccagaaacc 120

taacaaagca gatatgaaaa caattcaata tctaaatatt ccctaatatc tggatttatg 180

gcatcaggaa ggtaaaactt acaatctgtt gaatggaatt ttcatttcga tgttggggga 240

tgagtatact caccccacca cctatcagtt acagtgtgca tacggcttca ggcctctctc 300

ctctaaaaga tagcaaagct gagctcaagt caggtgttca cggactggcc ctgccactca 360

ctggctgcaa a 371

<210> 62

<211> 825

<212> DNA

<213> Homo Sapiens

<220>

<221> variation

<222> (286)..(286)

<223> baySNP2341_C286T

<400> 62
tgttgtggtg actcatgtcc caacctagct gcctctccca ggagactttc ccctggggac 60

aagggggagg gaatggcatg gaggaggccc acatcaagcg gggccaggaa cccacggtgg 120

caggagctgg gctggtgacc tacccagggc agaagggccc gggactcatc cagaggggaa 180

ggaaggggtc ttcaggaaga ccacggagat gccacaggca gaattggctt cccatctggg 240

agataggtgg ggagaccctg gcattttgac agccagaacc tggggygctg agcagaatct 300

tcatgcctgg cctggccgct ccttcggagg gaagctggag ggtgggctgc gagaggagtg 360

gggtcagagc ccctacatcc gcaggacccc aaatcggctg ggccccaagg cccggactgc 420

gctccccggt ggccccggcg gccctccgcg aatgcgtcct gcccctcccc tgcccaagcc 480

ctctgccctc acccgggtcc ggcgccgccc ccgaagtggc gggaacaacc cgaacccgaa 540

ccttctgtcc tcgggagccc ccagataagc ggctgggaaa cccgcggggc ccgcagggga 600

ggcccgctgt tccgcccgct aagtgcatta gcacagctca ccttccttat cgcgcctgcc 660

atcggacggg cagtgccgcg ccctgctttt gggcccccgg agcgaccaca gcggagccgg 720

aacggactgt ccttttgggg cggggaaggg agggggtgtc cttggagggc ccggtggcat 780

agcaacgacc aaaaagcctt cagagggcgg gagggggaat ttttt 825

<210> 63

<211> 565

<212> DNA

<213> Homo Sapiens

<220>

<221> variation

<222> (165)..(165)

<223> baySNP2357_A165G

<400> 63
   aacaactcag atctcaagtt tgttactgat ttctcttttt ttttctttta aggaatgtgt 60
   ttactgtgaa aacaaggaaa aaggtaatat ttgcatacca tatgaggaag atattccttc 120
   tctgggactc agcgaagtgt cggacaccaa agaagacgaa aatgratccc ccttgaatca 180
   caggatcgaa gagcagacgt aacccctgcc ccacctcagc ccggcagcca gcgaggtaca 240
   ccaggtggtg cttggaagag atgaaagatc ttcatggctg tttccactga aatggacaca 300
   tatgctcatg ttgctttttt tgttttagaa aaaaaaacaa catagttttc tgaaggggcg 360
   acttaaaact gtggagagtg gggagagttc ggaaagaaat atgtttttat atataaaata 420
   tatatgtgga gttttgtggg atggggaaga gattttagtt gttatttaac ttgagaaaga 480
   ctaagcgcct cttagtgtca gggaagttgc ctcagtgctc ccagaagtcc tgtgactgtg 540
   acgagacctc tgtctgctgc accag 565

<210> 64

<211> 549

<212> DNA

<213> Homo Sapiens

<220>

<221> variation

<222> (50)..(50)

<223> baySNP2366_G50T

<400> 64
gcctcttctc ccggctgctc cgaaacacgg tccagagcac cagaccgttk cccgtggtgc 60

ccaggaggaa gaccaacatg tagatggcag ggatgagggc ccccgaggat ttccagtctg 120

tgtactcaca ctcagactgg ttgtctgccc catagtagtt gtcaaaatca ccaccttcct 180

ccatgctggg gagtggagag aagacgggca gagggtccca gggacaccag ctccgtggct 240

ctgtcaccca ctctgcaagc agcctgaatt tctggcttga gcctcagaga gtaagaaggg 300

ctgaagatgc ccagagtcct tcccagcact caggaggagc tgcctctggg ttctccagac 360

cctggaggaa gcctgtctcc tgcagaattt cctccaccct ctcttgcctt acccccgtct 420

cagttaagac acttccttgc accctcctgc gtccctgtct cctccttgac tcgtcactcc 480

ctcctcccac ctccagacca gcccctctct tgtgagtcaa aatctttagt gacagcccac 540

ttttttttt 549

<210> 65

<211> 609

<212> DNA

<213> Homo Sapiens

<220>

<221> variation

<222> (170)..(170)

<223> baySNP2423_A170G

<400> 65
cagtaaggaa gagcccaaca ctgctggaag tgagcatgcc ccatttcatg agaaccaaca 60

gcttcgccga ggacctggac ctggaagggg agactctgct gacacccatc acccacatct 120

cacagtgagt gcctacatgt gcgtgaaggg ctgggctgga ggggactggr gctcaaggag 180

tcagacttgg tgctggggaa gccttgctct ctggccagag tgctatctac tcgcctagtg 240

cccaccactt gccgtctgcc tggccccaac acggaggcac caggcaagag aggagagcaa 300

gggcacttcc cactgagcct gggaacatga tcctcttgtt ttgacttatg gaaaaccaga 360

ctgatcattt tccacttgtt ttcatgcttt gagagtctga gattgttttt ctctccccca 420

gccccctccc ctgcccccca aaaaagagac gactttgttt tttagcattt ccccatggaa 480

ccctggccaa agccatgggg cagatgccgg gtgcagtctg ccagttgctg ctgctgtcct 540

caggggctcc ttcaggtgga ggaaatggct gattttccac gccttccagt tggccttccc 600

ctgaggcac 609

<210> 66

<211> 636

<212> DNA

<213> Homo Sapiens

<220>

<221> variation

<222> (140)..(140)

<223> baySNP2708_C140T

<400> 66
caccatttta tgaaggagga gaatgaactt aagaaaggtt acgaaatttg cccaaggcta 60

caaagctggt agtttgaaag aggctgggat ttaaacctgt gcctgtaacc aggatgtcct 120

gctgcctctc gtaaaagccy gcccaggtcc cactctcctg agtcagactg tcatccccac 180

ccctgcccac ctgcccaaga agagggctgt ctgtgagagg cgtgcccagg ccactggggg 240

tcctcttacc atctgtcggt ggtgttgatg agagtggaga tctgctcaag gtattccttg 300

tcatagacca caataggctc ggattcattg atctccacgg ggtagaagat ggtgttgaga 360

aaaggcaacc agttgatggc gggtgccaag gtctgcaagg gaaaaggaca gtgtgaccct 420

gtggggctgc tcctcctcac agcccagggc tctaggacgg gcttctacag gaagcaaagg 480

aaagagcttg ggcttggcgc catctgacag cgcaccgggc agagagtcaa gcggagcctg 540

gcgggctttt gtcagagatg ctgtaggtag aattctaagg agtccaacac caaaatttaa 600

cctgaaatgg aatgaggatg caatgagaag atgcag 636

<210> 67

<211> 620

<212> DNA

<213> Homo Sapiens

<220>

<221> variation

<222> (299)..(299)

<223> baySNP2995_A299C

<400> 67
ctgtgctgca gaggggagca gcatcagcag gcgtgtggag tgagtgagcc ccgtcctccc 60

cctcctcctg tcattgactc tcattgccta acccccttgt gtcttggccc caggttgatt 120

gtccggggct accgccagcc cctggagggc agtgacctct ggtccttaaa caaggaggac 180

acgtcggaac aagtcgtgcc tgttttggta aagaactgga agaaggaatg cgccaagact 240

aggaagtaag tgtgagtttc cttgtcctcc aggatgccct ggtcacctcc tttccactmc 300

tgtggcctca atccaggatg gggccctggc agtgctgcct gttactagct ttgtggttct 360

gggcaagatg cctcacttct cctcctggcc tcagtttgct tttctgccaa atggggtagg 420

atctcacatg tcattaaagc aactgggcag agaaaagaag aaagaagctg ggtgcggtgg 480

ctctcgtgtc taatcccagc actttgggag gccaaggcag gcggatcact tggtcgggag 540

tttgagacca gcctggccaa catggtgaaa ccctgtctct actaaaaata caaaattagc 600

taggcatggt ggcaggcgcc 620

<210> 68

<211> 934

<212> DNA

<213> Homo Sapiens

<220>

<221> variation

<222> (777)..(777)

<223> baySNP3360_G777T

<400>  68
ttgaattatg attcaaagat atgatagtaa tagaatgagc aaaactcagg aagagataaa      60

agttgaacac aatgatacta atataatagt aagagaaatg atcaggcttg ctaactatga     120

accagctact gtactacttt ctacaattat ctaccaacct cacaattatc ttgcaaatta     180

ggcattatta aatccatttt tcatatatga aactaaagtc tggaaaaatt gaataagaac     240

tcatattcca taagtagtga ctaccttata acctgtatct agcttgacag atgaaggtca     300

gcctgatttt taaaaaagtc aaagagagaa gcctatgttt atgtacaaca acatgtaaaa     360

ttttcatccc tcacccagtg gacaatagag ggacgatttt cttttctcca gagaagagac     420

attgaccaga gttaagaatc taactgttta tgagttccag tgacatttac aatcaattct     480

ggtttggctt ggtttctctc attccctctt aaccctgact tgtgatattg ccgtgaaggt     540

gatggagaag ctcttccata tcccagggtg agccctcaat tctggaataa ccacaggaat     600

gttggttggc ttggaatgga tgaagggaaa tggaaccggg ccagtaaaga gaaggcttgg     660

atctgttcta ggcctggctg atcctgaatt agttcaattc ccaaagatct gggtctcacc     720

atgggttcat taccttgact gactttgtta cttaaacctc caactatata ggagcckagt     780

gtgactcgga ctatggattg ttattcttaa ttataatgtt ttccctctca tttttctggt     840

aggtatcaga aacatttaga aaatgccaaa gagattggaa ttaaaaaagc tatttcagca     900

aacatttcca tgggtattgc cttcctgtta atat                                 934

<210>  69

<211>  372

<212>  DNA

<213>  Homo Sapiens

<220>

<221>  variation

<222>  (110)..(110)

<223>  baySNP3464_A110G

<400>  69
gttgctgcac ctgaagaacg agctgaaccg aggagatttt ttcatgaccc ttcggaatca      60

gcccatggcc ctcagtttgt accgacaggt gtgtgtagtg ggcagggttr tggtgtagcc     120

ttctgagcac ttgagttggc cttgctgact gattgcctgc ctgtggcccc agttctgtaa     180

gcatcaggag ctagagacgc tgaaggacct ttacaatcag gatgacaatc accaggaatt     240

gggcagcttc cacatccgag ccagctatgc tgcagaagaa gtctgagatc catggggcgt     300

gtggggcgtg tggggcatgt gggctggggc tgttggtccg gttcctcagg aatctaggcc     360

ttctgttggg tg                                                         372

<210>  70

<211>  652

<212>  DNA

<213>  Homo Sapiens

<220>

<221>  variation

<222>  (298)..(298)

<223>  baySNP3689_C298G

<400>  70
cttattttaa acctttattt ttggtcaatg gatttacact aatattcatc tcgaaaaata      60

aaaagaaaga aaactttaaa aagtagtagc atgtatcact gactaaagga aatggagaag     120

aagagagaaa aagagcactg accatggagt tgtaaggaaa tcaaaaacaa attcatccat     180

ttcacaattc acaattctgt gtaggctgac cctgaccttc atactcaaag caacacatgc     240

accattagcg tttctcattt tttaaatata tccaagcaaa taatgcccat ggagatgsca     300

ccaacctgat ctgtttgtgg cttcacttgg ttctacaaca aactgtagag aggcttcttt     360

cttctttgaa gaaactggaa ctgactttgg atgtgatttc tggcactgag cacagaagaa     420

atacaacatt agaactattt tattaactta ccaattcaat gaaatatatt tgttttttaa     480

atggggggaa aaacaagtat tctaaaacaa caaaccagct gttaagagtt ttatactact     540

ttttatttta tttctaaagt aatagctact ttggaaaaca atctggcatt attcagtaaa     600

gctgaaattt catacaacct atcacagaaa caatattact ctaaacagca ac             652

<210>  71

<211>  442

<212>  DNA

<213>  Homo Sapiens

<220>

<221>  variation

<222>  (65)..(65)

<223>  baysNP3975_A65C

<400>  71
gtctccttct agaaaagaac cctggtgaag agcaggaggg caggcagtct gtattgactg      60

ggctmtccct gactcccatg cgtggcaata acttgaaagt gatgaataca taattctggc     120

cctgcctggg atctgctgtg gggccttagt cctcatttcc tccatctgca caatgaggga     180

gcaagacaag ctctggattt agtgatgcag acattcatgc ccaaaccagc cagcctgggc     240

agtgacaaac agtgacacag gcagggacag ctccttccat ctcttttatc agggttgggg     300

gtcacagttc ttgtaccaaa gcccaaatcc cattatgcag aggtttgggt cttcttagtg     360

aggggaggaa gagccagttg taagatgctt acttgcacag agtggttaga aactgagaca     420

gtactccatt ctcccctgag ct                                              442

<210>  72

<211>  442

<212>  DNA

<213>  Homo Sapiens

<220>

<221>  variation

<222>  (239)..(239)

<223>  baySNP3976_A239G

<400>  72
gtctccttct agaaaagaac cctggtgaag agcaggaggg caggcagtct gtattgactg      60

ggctctccct gactcccatg cgtggcaata acttgaaagt gatgaataca taattctggc     120

cctgcctggg atctgctgtg gggccttagt cctcatttcc tccatctgca caatgaggga     180

gcaagacaag ctctggattt agtgatgcag acattcatgc ccaaaccagc cagcctggrc     240

agtgacaaac agtgacacag gcagggacag ctccttccat ctcttttatc agggttgggg     300

gtcacagttc ttgtaccaaa gcccaaatcc cattatgcag aggtttgggt cttcttagtg     360

aggggaggaa gagccagttg taagatgctt acttgcacag agtggttaga aactgagaca     420

gtactccatt ctcccctgag ct                                              442

<210>  73

<211>  486

<212>  DNA

<213>  Homo Sapiens

<220>

<221>  variation

<222>  (304)..(304)

<223>  baySNP4206_A304T

<400>  73
aattctggtt ctaccacttt ctgtgcagcc ttggcccagg tagttaatct ttcagtgcaa      60

gcagtgttgt cattcataaa atggaggtga tgcctaccat gaagtgttgc tatggaaatt     120

aactgatctg tgatattaaa gcatagtcac tggtacttgg ggttacctaa tctaaatgcc     180

agaggagtga tttaacaagg gaggcaagac cctttctttt tagccaagtg gagttagcaa     240

agtatgtttt gtatgtggaa agtctacttt tgaattgtct tcgtttctgc cttcctacta     300

atgwttttct ctctgcctgg tctaggatta acacagattc ctcagatcca gaagactgaa     360

atttcctttc gtcctaatga tcccaagagc tatgaggcat atgtactgaa catagttagg     420

ttcctggaaa agtacaaaga ttcagcccag agggatgaca tgatttttga agattgtggc     480

ggtaag                                                                486

<210>  74

<211>  801

<212>  DNA

<213>  Homo Sapiens

<220>

<221>  variation

<222>  (583)..(583)

<223>  baySNP4838_A583G

<400>  74
tttaaccaga accaagtgtt caccccccac agaatgtaca tgaaacacta gaggactgca      60

tgtttttccc tgagagaagc gtaagacaaa cagaagtcaa aaagtagtca ctgggagcgc     120

catccttcta agcaaatcct ccctttccct tttggaggat ttgcccgaac tacgtagcca     180

gtcagcactt agaccacctg cctcctcctc cccctataaa cccaccactc ccctcctcct     240

ttcccaaacc acttggggtg tcctaagccc tcactgcccc aagcccaaaa tatcaactaa     300

gatccttgtc tgtatttcca cagtcatacc taatgaattg ggaagtgggg cccctaaaaa     360

ccaattcaca tctatgcact tgtttccact ggatttggca gacaggcttt tttagttacc     420

gtaaccagat cttaagatta attaaaaact acataaagtg cttttaggtc cttagagata     480

catgatataa ataagcggct tatgatcaag aacgataaat acataggtaa aaatagctct     540

agcaaagatg accttatggc tctgagatgg gcaggcaggg carttcttcc ccattacatt     600

cttagtcatc ttattcatac ctatttttaa atggagtcca cagactaaag gtcatgttcc     660

gagactgaag ctttcaaatg accctagttc caatatagac actttcttca gtttatcagt     720

agcttttaaa ctctgaggtt aacacagctc actctttctg taatttagtg agcatactcc     780

tatacaaggg aaacttagag a                                               801

<210>  75

<211>  921

<212>  DNA

<213>  Homo Sapiens

<220>

<221>  variation

<222>  (74)..(74)

<223>  baySNP4912_A74G

<400>  75
cccttcattg cggcgggctg cgggccaggc ttcactgagc gtccgcagag cccgggcccg      60

agccgcgtgt ggargggctg aggctcgcct gtccccgccc cccggggcgg gccgggggcg     120

gggtcccggc ggggcggagc catgcgcccc cccctttttt ttttaaaagt cggctggtag     180

cggggaggat cgcggaggct tggggcagcc gggtagctcg gaggtcgtgg cgctgggggc     240

tagcaccagc gctctgtcgg gaggcgcagc ggttaggtgg accggtcagc ggactcaccg     300

gccagggcgc tcggtgctgg aatttgatat tcattgatcc gggttttatc cctcttcttt     360

tttcttaaac attttttttt aaaactgtat tgtttctcgt tttaatttat ttttgcttgc     420

cattccccac ttgaatcggg ccgacggctt ggggagattg ctctacttcc ccaaatcact     480

gtggattttg gaaaccagca gaaagaggaa agaggtagca agagctccag agagaagtcg     540

aggaagagag agacggggtc agagagagcg cgcgggcgtg cgagcagcga aagcgacagg     600

ggcaaagtga gtgacctgct tttgggggtg accgccggag cgcggcgtga gccctccccc 660

ttgggatccc gcagctgacc agtcgcgctg acggacagac agacagacac cgcccccagc 720

cccagctacc acctcctccc cggccggcgg cggacagtgg acgcggcggc gagccgcggg 780

caggggccgg agcccgcgcc cggaggcggg gtggaggggg tcggggctcg cggcgtcgca 840

ctgaaacttt tcgtccaact tctgggctgt tctcgcttcg gaggagccgt ggtccgcgcg 900

ggggaagccg agccgagcgg a 921

<210> 76

<211> 2679

<212> DNA

<213> Homo Sapiens

<220>

<221> variation

<222> (2613)..(2613)

<223> baySNP4925_A2613C

<400> 76
   ccccagctct gcgatgatgg cagaagagca cacagatctc gaggcccaga tcgtcaagga 60
   tatccactgc aaggagattg acctggtgaa ccgagacccc aagaacatta acgaggacat 120
   agtcaaggta ggctctgcag gcctgcctcg gcgggcggag agtgtcaggt ttgcgagacg 180
   tgggcgcttg gcaggggagg gtatctgctg cagacacagt ttccctgaaa agagacttgt 240
   tgctctgttg tctctgtatc acccccccaa ccccacccca cccccaaaaa aaggcttcta 300
   aatcaccgaa ggatctttag caaaatcaag agcaaaaaat tagcctcatc tccatgacct 360
   aagtgctaat cagggcagga ggagcgagtc acaaagaaaa tccctcctta ctactgtgac 420
   gctcctgcca ctcactcctg gctctcaccc tgggaggaac aggaacgggc ggccaagttg 480
   ggctcctttc tgtgccaagg ggcatttctg cggtctctcc agtgggtgca gggttaatcc 540
   ccagagggca gcttccagca gggccggcag cccctcgagg tacactcact cccccagagc 600
   actgcccaat atctgtcccc aaggccgagg gccatgccca gccctcccct agctaccccc 660
   tcaccccagg tctagtggcc aggacagggc agacggcacc cgggaccctc gaagagaaag 720
   ctcggggttg tctagccctc agggagtctc tcagcgtctg aagttgggcc tcttccctcg 780
   cctcctggtg acactagcgg ccctgctgcc tccaggccca caccttcaat ccaacttcca 840
   gccagtaccc agagggatct ttcttttttc tttttgagac agtctctctc tgtccccaag 900
   gctggagtgc agtcgcacac tcacgcctca ctgcagcctc aacctcccag gctcaagtga 960
   ttctccctcc tccacctcct gattagctgg gaccacaggc gagtgccacc atgcccagct 1020
   aatttttgta tttttagtag agactgggtt tcattatgtt gaccaagctg gtatcaaact 1080
   cttgggctca agtgatcctc ctgcctccca aagtgctggg attacaggtg caagactcca 1140
   caactggctc agaggtacct ttctaaagca gaattcctcc aagaagggtc cccagccacc 1200
   agcatctgag tcactggagg ggctgttaag aggccagtgt ctgaggcctc tcagatccta 1260
   ctgaaagcat tggaatctca ggccagtgct gtccaatagg gatactgtta ccacaaggaa 1320
   tttaaaattt tctagcagcc acattttaaa atgcgaaagc aaacaggtga aattaatgtt 1380
   cctgatattt cttacttaac cctatatatc taaaatagta ttatttcaac atgtaagcaa 1440
   tataaaacaa ttatcaatga gctattgtac agtcttcaag tttgaaatcc agtgtgcttc 1500
   ctatacccac agtatatttc agttcagacc ccacattttc atccgaaatg cttgttctgc 1560
   atttagattt cataaaatgt acatgtgaaa atcgattcgc atgcctgtac tgtttcaaac 1620
   attctgcaaa aatttttcaa taatgaaatc ggggtttaca ttttaattaa agttaaacaa 1680
   aatctagaat tcttcagttt cactagccac atttcaagcg ctcaaagctc ctggccactg 1740
   gatcagacag gcctgtattg cggtctttca gtggggcacc tcccatgctg gagaagccta 1800
   gcctctccac gtggctgtcc aaacccaaca caatctggcc ccaacctgct tcttctgcct 1860
   ggttggctcc cacctgccag cccacccacc agagtacatt cctgtgccat tgccagaagt 1920
   gcctgtcctc tcttctccag gtggtgaatg cctcactcag ccttcaaggc ccaactcaaa 1980
   tgcaacctcc tccagaaagc cttccctgac tcctccaggc aattagttgc tcccacttct 2040
   gtccccctgc ttcactcagg gcattcctcc accacagctc ttcttatact ggatattata 2100
   tttctataca tcgttagtga tggagataga gtatacagta tagcacagtg tgaaagctga 2160
   aatgaggaaa attaggaaac aaatttcaca aactgtaaag cactatatac atgtaaataa 2220
   aattgtacct gtaaaatatt attagttatg gtttccatca gtgtcatcta gcaggaatag 2280
   tcctggactt gactttaata gacttgattt caaatccagt ccattttacc taaaaatgat 2340
   aacactggac aaaccaacta ggttttctga gactcagttt cccctgtaaa gtgggaataa 2400
   tatcatttcc caatttacct ccaccaggta aacgaatctc aaagcagcac tgtgcgttag 2460
   aaatatagca tgaggattga atgagatgaa gggtttgaaa gtgtctatca cagtccctgg 2520
   cacccagcaa ggatgagaca gtgtgagtca aagaagggtc tcaaagagct tgcagtctag 2580
   agggcaagag agctaagcca ggagcctgcg tcmtgcccag gtagccatct ggtacaccca 2640
   ggtgggctgc agggagatgg gaaagaggcc ccccccccc 2679

<210> 77

<211> 1025

<212> DNA

<213> Homo Sapiens

<220>

<221> variation

<222> (807)..(807)

<223> baySNP4966_A807G

<400> 77
aggtctcacc taatatcatc agaattctgt ctttaactct tattctgtaa atgtgttttg 60

tagagtgttg acttatgctt gggtgttgca gaacatgttg gaccatgtag gagccatgtc 120

aagacatgct gggtgtgctg taccatgtca gggtgtatca ttgctctata gtgttgttgc 180

agcctagtcc ggtccccttt atgcccccca ccctcctttc ttcttctgcc catggcctcc 240

ttcctgctat gctgggcttg gagaaaagac gtccaaacct ctggctggga gccaccctcc 300

atacccaaag tccctcctct attcctctcc atggaccctg atggtcctca ttcatgtcag 360

atgccattac agacaaggac atagtcttct acaagaccct gaagccctgg ctgggtaagt 420

aactgtaggt ggacgggacg gggaccaact tttgtggcca ggggaagatt ctgcccttgc 480

ccacagcctt tggctgccgt actaggggat gggctcttgt taagtgttgg tgacaagtgg 540

agacaccacc gtcgcttgtg acgcctgcct tccatttcaa catcctgaag ccctatataa 600

agattttcag caagagtgca aacatcatgc atgtgagtgc cttgaactca gcatcccagc 660

tgcagccttg gggtggaggg atcacataca attgggtctg gaatgttggc tctcctgggt 720

gggtttgggg ccattgctct tcctctctgt gccttgattt ccccatgagg ctaataatcc 780

tcactaaaag gtggtaggag catgtartgg actcatgtct ctgacactta gtaggtggtc 840

agaaggagtc aatttccaca tttttctcac agaagccctg taaactcaag aaaggaatga 900

tgacacgtgc atagtagtca ttgctgagta cagatcactt caaaactaat tggtttagag 960

caataagggt ttatatttgc taacaaatct gtgggactgt tggccagccc ttctgcatgt 1020

ggata 1025

<210> 78

<211> 2246

<212> DNA

<213> Homo Sapiens

<220>

<221> variation

<222> (2057)..(2057)

<223> baySNP5014_A2057G

<400> 78

tcactggaaa aaggaattct gattgttcaa aagcacagga tatattaagg gcctcatata 60

atgcctggca cataagagac ctcagcaaat tacggcattt catattatgt ttatacagtg 120

aaggcatcaa ggttataagc attctctttt ttcttttggg tagactgaag ctcagagagg 180

ttgagtggct tacttaaagc tgcacagcta ttagtaggca gatcaagatt agagttcaga 240

acttctcact ccctgcccag tttctgcttt ctttaccctt tgcctctttc aagttgtggg 300

tctgccggcc aggtgggagg tgctgtctgc aaagggcttc cctttctctt tggccactat 360

ctggctgggg agaggcctca cctagatgtt gttgaaggcc tgttacagcc gctttattgg 420

ggattttctg gcgatgagaa cgtgtgaatg tcctggcctt tagtcaactc cctacacctc 480

tgagagtgtc agacaagaga gcccatcaca ctggtgggat tgcaatcttt gcctctacca 540

ctgcttagct gcctttcctt agggcaagtt acttaatggt tctgtgactc agtttccctg 600

tttgtgaaaa gtgaaggtta atagtaccca ccatataggg ctgttagaat ggagtggaat 660

aattcatgta gaataagtat gtataacagt ggctaaaaca tagtcaggct gggcgcggtg 720

gctcacgcct gtaatcccag cactttggga ggccaaggca tgtggatcac gtgaggtcag 780

gagctcaaga ccagcctggc caacatggtg aaaccccgtc tccactgaaa atacaaaaat 840

tagctgggct tggtggcggg tgcctgtaat cccagctact cgggaggctg aggcaggaga 900

atcacttgaa cccaggaggc agaggttgca gttagccaag atcacaccac tgcactccag 960

cctgggcaac agagtgagac tccgtctcaa aaaaaaaaaa aaaaaaaata gccagttgcc 1020

tagaatagaa ccaactaaca gtggttttat ttttactgca aaaaataaaa ataaaaatag 1080

gagtagtgca agcactgggc cacatcacta caaaacaagt gtatctcagc atctcccacg 1140

agaataccac tcaggtcaaa acatgatata gtgaagtggg gatgaaaagg atccaaccat 1200

gggcagaacc tggggtctgg tgccagtgga gacagcccca gtgtctagca tgagacacgg 1260

ggaatgttcc gttggagggt gggtatgatg actctcctga aagcttccct ccctccagtc 1320

cagatggccc ctccatccct caacgtgacc aaggatggag acagctacag cctgcgctgg 1380

gaaacaatga aaatgcgata cgaacacata gaccacacat ttgagatcca gtacaggaaa 1440

gacacggcca cgtggaaggt gagggccttt gcccagggag gggagaaaca ctggggaggg 1500

cgggagaagg gaaagcaacc agaggcattc cacctgcaag gcgtcgggcc cttggcaggt 1560

gaccagtgag aggtagccac tgggacgtgg tgatcactag gctgtgtggt cagcaggtca 1620

ctgtcctgtc tcttggtgaa gtaactgagg tttggaaaag tggcgtggct tggccaacgt 1680

gaacagctga ccctgagtcc ccaggcaaca gaagaccctc tgggcaggga ggggttgaaa 1740

ggccactggg aagaaggttt tcaaaagtca tgaaagtttg gggttatttc ctcagaggaa 1800

tctcatctgg acacacatgg aggctcagac agagctgctt ctaatgagtc gggggtgcgc 1860

ccaggccagg gctcggtccc ctgcctccac agagcccaga acagaaacca cagaaccaac 1920

cccacacctt cagtctagaa atggggcaac tgaggctagg agggaggtgg gccagtggtg 1980

gagccaggag cgggccctgg ggtcctgaac ccccattctc agggtccaga gtccagtcgg 2040

cctgcactgc gttcctraaa aggccacaat atgggtgcaa gctgccccag aagggctggg 2100

agctgagaag gctcaaaata gggtgggaca ggtggcttca gggttctggg cctcagtgtt 2160

gtcaatgtca ggggctgcac tgacaggtgg agtccccggt gccatccgaa gtgctgtccg 2220

tgggtgggcc ctcagggagg atccac 2246

<210> 79

<211> 486

<212>  DNA

<213>  Homo Sapiens

<220>

<221>  variation

<222>  (366)..(366)

<223>  baySNP5296_A366G

<400>  79
tgttggtgcc acacacacct taacacctgc acgctggcaa caaaaccgtc cgctctgcag      60

cacagctggg gtcacctgac ctttctcctg tcccccccac ttgagctcag tggctgggca     120

gcaggggatg catggccact ggccggccag gtgcagctct cagctggggt gctcagagga     180

cgcctgtgtc ctcccctccc ccatccctct gtcacccttg gaggcagaga actttgcccg     240

tcagtcccat ggggaatgtc aacaggcagg ggcagcactg cagagatttc atcatggtct     300

cccaggccct caggctcctc tgccttctgc ttgggcttca gggctgcctg gctgcaggtg     360

cgtccrggga ggttttctcc ataaacttgg tggaagggca gtgggcaaat ccaggagcca     420

gcccgggctt cccaaacccc gcccttgctc cggacacccc catccaccag gagggttttc     480

tggcgg                                                                486

<210>  80

<211>  486

<212>  DNA

<213>  Homo Sapiens

<220>

<221>  variation

<222>  (172)..(172)

<223>  baySNP5298_C172T

<400>  80
tgttggtgcc acacacacct taacacctgc acgctggcaa caaaaccgtc cgctctgcag      60

cacagctggg gtcacctgac ctttctcctg tcccccccac ttgagctcag tggctgggca     120

gcaggggatg catggccact ggccggccag gtgcagctct cagctggggt gytcagagga     180

cgcctgtgtc ctcccctccc ccatccctct gtcacccttg gaggcagaga actttgcccg     240

tcagtcccat ggggaatgtc aacaggcagg ggcagcactg cagagatttc atcatggtct     300

cccaggccct caggctcctc tgccttctgc ttgggcttca gggctgcctg gctgcaggtg     360

cgtccaggga ggttttctcc ataaacttgg tggaagggca gtgggcaaat ccaggagcca     420

gcccgggctt cccaaacccc gcccttgctc cggacacccc catccaccag gagggttttc     480

tggcgg                                                                486

<210>  81

<211>  458

<212>  DNA

<213>  Homo Sapiens

<220>

<221>  variation

<222>  (101)..(101)

<223>  baySNP5320_A101G

<400>  81
tgttgctagg tgctcgggat atatagtaga aaaacaagcc tgtctttttt aatgtatgca      60

ggaaatctct ggagaagtaa gagaggttaa ggaaagctgg rtatgtgcct gctttacatg     120

gagtagtagt gattaagttg gccagtgccc agaagtgctt actgggtgcc aggattgttg     180

ctgtggaata tcgagtacca ctaactttta aattcttcaa agagggctgt gctgaagttt     240

gctgctgcca ctggagccac tccaattgct ggccgcttca ctcctggaac cttcactaac     300

cagatccagg cagccttccg ggagccacgg cttcttgtgg ttactgaccc agggctgacc     360

accagcctct cacggaggca tcttatgtta acctacctac cattgcgctg tgtaacacag     420

attctcctct gcgctatgtg gacattgcca tcccatgc                             458

<210>  82

<211>  1975

<212>  DNA

<213>  Homo Sapiens

<220>

<221>  variation

<222>  (1604)..(1604)

<223>  baySNP5361_A1604C

<400>  82
catgaagcgg actagtccca gaggacaaac agaatctttg ccctcctgtc tgctagtcca      60

gtcttttccc tgctggatca ttgtgtgcct gtgccatgag gtttctgcca tctaacaaac     120

agtgccagcc aagcaaagcg ggcatgcagg cctgcaagtg gttctcatgg aagctgttcc     180

tccccacagt ttgggacact gaccaacagt cctttagcag gaggtcgtct tagtccattt     240

ggtctgctat aacaaactac catagactgg gtggctcata aaaacaggac tttatttctc     300

acagttctgg aggctgggaa gtccaagatc aaggtgccag cagattcagt gtctggtgag     360

ggcccttgcc ttggtttata gagggaacct tctcactgtg tgctgatatg gtgtaaggag     420

caagggaatg ctctggggtc tctttcataa gggaattaat cccactcttg agtgctccac     480

cctcatgccc taatcacctc caaatacctt cacattgggg attaggtttc agcatatgaa     540

tttgggagaa gggacagaca cagtcagtct atagcagagg tcatcttttt tcagtgtcga     600

aaaatgttgt tgagtcccac acaaaggaag ttctatttta ttttatttta ttttatttta     660

ttttatttta ttttatttta ttttatttta tttctggaga tagggtatct ctatgttgcc     720

caggcaggtc tcaaactcct ggcttcaagc aatccttcca cctcaacctc ccaaagtgtt     780

gggattactg gcgtgagcca ccacactggg cctattttca atatctgtta ttttaaaaca 840

tttttaatag tcaacagcca ctagaaaacg taatgacact taaaaagatt gttattttac 900

ttaaccaata acatctgcat gcaagtgagc gatgaacata cacaatggga aatggattaa 960

gaggttctca gataaggttt gctagtttag accaaatgag atcaggaggt tctagaacac 1020

aagggaggaa agcagcatct gtccacccca agccaagtta ctgccaaccc aaggcaatgc 1080

ttagtggata aataaaaaac tgaacaaaat gcagtacttc atgtgagagg tggtttctgt 1140

ggacttggat tcttaccgta tttcctctaa tatctgttca ttacaagcac catgtaaacc 1200

tgttgcctta aaaactcttg aaaaggtgtc ctttggggga atgtgcttgg gggaagcaac 1260

ccttaagcat ggctgtctgc tgcccagccc cactcccaca ccttgctcag aagttcctcc 1320

accatcaaca gcaccactga gcttgtgtcc agaaggccct caggaggtcg aggtaagagg 1380

atgctttgta tcagtggggg cagggatggg agaggtaata agagccggga tgggggccag 1440

gggagtgact gggttctggt gagcatacag aagctttgct gttattcagg aaggaatgca 1500

ggttttgcag aaagcacatt gggcatttca tcacgtagaa agctactgaa actgacacta 1560

cacgccttca gccagggagt cccgctaagg gattatgaaa ggcmggctga ttccaaaact 1620

acaaatccca ggctcccccg ggggatggac acttgtgaga ggaagtgggt tagccagtca 1680

aacctccagg gtttcagcca gttgagaaag gatagggagg gaagttttaa aaggaatgtg 1740

ggcccacaag cctgggatct gtctctctct ctcagtctga tgcggcctcc tgtctaattg 1800

tttcagtctg tccgagctgg taaatcccta agcacatagt caatttacac tcagcttaaa 1860

agctctagag ggagacagga aaaatttcca gtgagaccac tcactcacct acaactgcta 1920

gattaacctt ctgcatttta actgagttaa ctcatatgtt ttcttagtgg gagga 1975

<210> 83

<211> 527

<212> DNA

<213> Homo Sapiens

<220>

<221> variation

<222> (134)..(134)

<223> baySNP5457_A134G

<400> 83
tgaggctgac atgttgcagc agaatgggca atagaaatca attactaaga ttaaaataca 60

gcttttacaa cagtgagtgg aaaggactga ccttctaaat taacagcagc ctaaatggaa 120

agcctacttt cttrtgttgc ttaattcaca tctggggacc tgatttaaac atgtcttgcg 180

ctgttgttgc catggttgct ttgcccatcc catcaacctc atcctgtcac ttttctctct 240

gacttctttc tctgcccaca tctctccctc cctgctgctc ccgtctcctg tcttcttctg 300

gccatttgag atgttccagc agaagatgac ccttcccctt gtgcgctgga aacgggccac 360

gggcggcagt gccagaacgg cacggtgtgc aagcccggct gggatggtcc caagcacggc 420

atcaccaact ttgacaactt tgccttcgcc atgctcacgg tgttccagtg catcaccatg 480

gagggctgga cggacgtgct gtactgggta cgtagcatga gtgggca 527

<210> 84

<211> 562

<212> DNA

<213> Homo Sapiens

<220>

<221> variation

<222> (61)..(61)

<223> baySNP5704_C61T

<400> 84
actctgacag ccataacagg agtgccactt catggtgcga agtgaacact gtagtcttgt 60

ygttttccca aagagaactc cgtatgttct cttaggttga gtaacccact ctgaattctg 120

gttacatgtg tttttctctc cctccttaaa taaagagagg ggttaaacat gccctctaaa 180

agtaggtggt tttgaagaga ataaattcat cagataacct caagtcacat gagaatctta 240

gtccatttac attgccttgg ctagtaaaag ccatctatgt atatgtctta cctcatctcc 300

taaaaggcag agtacaaagt aagccatgta tctcaggaag gtaacttcat tttgtctatt 360

tgctgttgat tgtaccaagg gatggaagaa gtaaatatag ctcaggtagc actttatact 420

caggcagatc tcagccctct actgagtccc ttagccaagc agtttctttc aaagaagcca 480

gcaggcgaaa agcagggact gccactgcat ttcatatcac actgttaaaa gttgtgtttt 540

gaaattttat gtttagttgc ac 562

<210> 85

<211> 2246

<212> DNA

<213> Homo Sapiens

<220>

<221> variation

<222> (1960)..(1960)

<223> baySNP5717_A1960G

<400> 85
tcactggaaa aaggaattct gattgttcaa aagcacagga tatattaagg gcctcatata 60

atgcctggca cataagagac ctcagcaaat tacgggcatt catattatgt ttatacagtg 120

aaggcatcaa ggttataagc attctctttt ttcttttggg tagactgaag ctcagagagg 180

ttgagtggct tacttaaagc tgcacagcta ttagtaggca gatcaagatt agagttcaga 240

acttctcact ccctgcccag tttctgcttt ctttaccctt tgcctctttc aagttgtggg 300

tctgccggcc aggtgggagg tgctgtctgc aaagggcttc cctttctctt tggccactat 360

ctggctgggg agaggcctca cctagatgtt gttgaaggcc tgttacagcc gctttattgg 420

ggattttctg gcgatgagaa cgtgtgaatg tcctggcctt tagtcaactc cctacacctc 480

tgagagtgtc agacaagaga gcccatcaca ctggtgggat tgcaatcttt gcctctacca 540

ctgcttagct gcctttcctt agggcaagtt acttaatggt tctgtgactc agtttccctg 600

tttgtgaaaa gtgaaggtta atagtaccca ccatataggg ctgttagaat ggagtggaat 660

aattcatgta gaataagtat gtataacagt ggctaaaaca tagtcaggct gggcgcggtg 720

gctcacgcct gtaatcccag cactttggga ggccaaggca tgtggatcac gtgaggtcag 780

gagctcaaga ccagcctggc caacatggtg aaaccccgtc tccactgaaa atacaaaaat 840

tagctgggct tggtggcggg tgcctgtaat cccagctact cgggaggctg aggcaggaga 900

atcacttgaa cccaggaggc agaggttgca gttagccaag atcacaccac tgcactccag 960

cctgggcaac agagtgagac tccgtctcaa aaaaaaaaaa aaaaaaaata gccagttgcc 1020

tagaatagaa ccaactaaca gtggttttat ttttactgca aaaaataaaa ataaaaatag 1080

gagtagtgca agcactgggc cacatcacta caaaacaagt gtatctcagc atctcccacg 1140

agaataccac tcaggtcaaa acatgatata gtgaagtggg gatgaaaagg atccaaccat 1200

gggcagaacc tggggtctgg tgccagtgga gacagcccca gtgtctagca tgagacacgg 1260

ggaatgttcc gttggagggt gggtatgatg actctcctga aagcttccct ccctccagtc 1320

cagatggccc ctccatccct caacgtgacc aaggatggag acagctacag cctgcgctgg 1380

gaaacaatga aaatgcgata cgaacacata gaccacacat ttgagatcca gtacaggaaa 1440

gacacggcca cgtggaaggt gagggccttt gcccagggag gggagaaaca ctggggaggg 1500

cgggagaagg gaaagcaacc agaggcattc cacctgcaag gcgtcgggcc cttggcaggt 1560

gaccagtgag aggtagccac tgggacgtgg tgatcactag gctgtgtggt cagcaggtca 1620

ctgtcctgtc tcttggtgaa gtaactgagg tttggaaaag tggcgtggct tggccaacgt 1680

gaacagctga ccctgagtcc ccaggcaaca gaagaccctc tgggcaggga ggggttgaaa 1740

ggccactggg aagaaggttt tcaaaagtca tgaaagtttg gggttatttc ctcagaggaa 1800

tctcatctgg acacacatgg aggctcagac agagctgctt ctaatgagtc gggggtgcgc 1860

ccaggccagg gctcggtccc ctgcctccac agagcccaga acagaaacca cagaaccaac 1920

cccacacctt cagtctagaa atggggcaac tgaggctagr agggaggtgg gccagtggtg 1980

gagccaggag cgggccctgg ggtcctgaac ccccattctc agggtccaga gtccagtcgg 2040

cctgcactgc gttcctaaaa aggccacaat atgggtgcaa gctgccccag aagggctggg 2100

agctgagaag gctcaaaata gggtgggaca ggtggcttca gggttctggg cctcagtgtt 2160

gtcaatgtca ggggctgcac tgacaggtgg agtccccggt gccatccgaa gtgctgtccg 2220

tgggtgggcc ctcagggagg atccac 2246

<210> 86

<211> 564

<212> DNA

<213> Homo Sapiens

<220>

<221> variation

<222> (71)..(71)

<223> baysNP5959_A71G

<400> 86
taaaccacaa accaagagcc aaaccatgcc tgacttaacc agaaacaaat gccaaccact 60

gtcgaatgtg rctgcccagc ctcttaacac catcttctca ggtggctgac atcccttggt 120

ttcacactgg gggccttgcc ctggattatt tactttcttt tatttagctc aagttagaag 180

caattttaca cctgaagttg aatgacttcc aaatggattt tagcatagac ccagggaaga 240

gtgtaagaaa gaggacagga aacaattctc ttgacattat tttcaaggca ggagagaaaa 300

gaccatctca tggcctttgg gtacaaaccc ttggcctatg taaatagagc cccccacttc 360

tgccctctga atctcatacc ttgcttccac tgattctgga tttttttacg gtatgatgtg 420

taacatcgat ccaaggcccg caagtagcac tggatggaaa gcttcccatc cactattggg 480

tactccgagg ccaaatttgg tttgtagaag tcatacacat tctccatatg ggttcccctc 540

agccctggaa aggcacacaa agtg 564

<210> 87

<211> 494

<212> DNA

<213> Homo Sapiens

<220>

<221> variation

<222> (340)..(340)

<223> baySNP6162-C340G

<400> 87
gaaggtccag ctgttacaaa atgaagacag acaacacaac atttactctg tggagatatc 60

ctactcatac tatgcacgtg ctgtgatttt gaacataact cgtcccaaaa acttgtcacg 120

atcatcctga ctttttaggt tggctgatcc atcaatcttg cactcaactg ttacttcttt 180

ccccagtgtt gttaggagca aagctgacct gaacagcaac caatggctgt agatacccaa 240

cctggcaata aaaggaaaat atattaattc aaactctatg gaaacttaat acttctctct 300

accaggaatt ggcttattct gtaaattttc tttttgagas tgttgcccag gctgcagtgc 360

agtggtgcaa ttatggctca ttgaagcctc aaccaatcct cccacctcag tttcctaagt 420

agctgggact ataggcacat gccgccacag cccactaatt tttaaatttt tttgtagaga 480

cagggtctcc ctgc 494

<210> 88

<211> 1898

<212> DNA

<213> Homo Sapiens

<220>

<221> variation

<222> (906)..(906)

<223> baySNP6236_C906T

<400> 88
gagccctcag aaataacacc gcatatctac aactatctga tcttcggcaa catctgccgc 60

acacacaagg caaattggtg cgaaatggat ttccctatta taactaaatg gtgctggaga 120

aacactggtc tagaccacta tgtacgcaaa gctgaaactg gatccttcct tacacctata 180

caaaaatcaa ttcaagatgg attaaagact taaacgttag acctaaaacc ataaaaaccc 240

tagaagaaaa cctaggcatt accattcagg acataggcat gggcaaggac ttcatgtata 300

aaacaccaaa agcaatggca acaaaagcca aaattgacaa atggggtcta attaaactaa 360

agagcttctg cacggcaaaa gaaactacca tcagagtgaa caggcaacct acaaaatggg 420

agaaaatttt cgcaacctac tcttcggaca aagggctaat atccagaatc taaaaaacac 480

attaataaaa gctttgaatc cacatagatc actctcaaca gaaagatgtc tccagatgga 540

agatgcagct ggagatcaac agtgaacatc aaaggacata ttcagtgacc tccaatcttc 600

cctctgatcg ttcctaggaa tgggaggggc acagccagca tccccatatg cagaaaagtg 660

ataagagatc ccctgattta ctacgttcac ttccccagag atgactacag catttctgca 720

acctgtgtta actttcttcg gccatttgga caggaaaatg gaaagatact aaaaaatacc 780

gtaatgaaat actggccagt gtaatttttg aaattttctc caacctgagc atctcaaata 840

cctttatctg aaattctact cttctataat atgcctagga tagatgtacc ccatcctact 900

cagcayttta aatagaagta ggttattcca tcaaaagctc aaatgtgtcc aagatacaaa 960

ataaatagtg accatatgaa ctctctattc tatcataaca gttcctttca aggtttcaga 1020

caaacatatt atttccttgt tttttcttcc tacagtcact ttaggaaggg cctgagcata 1080

aagagtttgc tagcatgcat accacaacta ctttattttc catataagca taatcagcaa 1140

gtataactgt ttctcaattt gttaaagcct accataaaat cagttcaagt aactaacctt 1200

gactagtaca ccatatgtgg attacaccat tttttccttc tttttattgt agtttctcta 1260

cgtgatttta aatgctagtg gatgcccaga tttagctaga aatttcacct atttatgaaa 1320

atgaatgagt gctttttctc acatagaggt gattttatac ttatttgccc aatgatccac 1380

ccaaaatccc acgtggaaag agggatcctg gtataatctg gttaagagtg tttcttacct 1440

ctcttggctt actggcaatg gcaacactgc catctttgtt gtatttgatg ggcgatcctc 1500

cttcttgtac cagggtccca taccctgttg gggtgtaaaa tgcaggaact ccagccccgc 1560

ctgcacggat cctctctgca agtgtgccct gcaagtgagc aaccaacacc ccataagttc 1620

actaagcaca cttctctatg tggacacaaa catttagacg tggttcctac tatctagagg 1680

cttataattc taaaaagtag ctagcattgt acattagcat acttcactaa tattgtatgc 1740

tagaagatca attttaagaa gttactgccc ctctagaacc attctaaatc cacattcaaa 1800

atgtaaccgc ttatttgtat agtcatgcat tgagcttcca tacctctcaa tgatctcttg 1860

acacatttaa atgtgtctat ttttgatatt ctaagtca 1898

<210> 89

<211> 1351

<212> DNA

<213> Homo Sapiens

<220>

<221> variation

<222> (151)..(151)

<223> baySNP6482_A151G

<400> 89

aaacctgatc atatcacaac agtataaatg caagaaaagc tctatgcttt gtagttagcc 60

ctgaatggtt gtttgacaga tacagaattt tttaaacctc attctctcat agtttaggat 120

aaacaaaagg gattcaaaca gatctggtct rcctctgggt tgtggcgttt ccatgacaag 180

cttttacact atgcacctac tttcattcgg gaaacggtct tattgatctc ttctacatcc 240

ccaacagtga caatgttgga cttcagcatc tggtcgatta ataccagcca gtcggctagt 300

tctgttatag ttttatcaag atcagcagga attgaaattt ccgaagtacg atgagactga 360

acaggaatat ctgacgcaga tgatactagc accacctttt ggacattagg atgagctgaa 420

atgaaaaatg tagcaaaaga gaaattaatc aacaggatgt tttaattatg gtggtgtcat 480

ggtgacacgt gtatgtgtat ttaaataaaa tttagtttaa gtaaaaaaaa ttctaagagg 540

ttattttttc atactgaaat ggctatggta ttacctgtaa tatccatatg tctttggtag 600

ctatcaagcc aacaaaacag ttttgcaaca ttctataaca taatcagcac tgacactgag 660

aaaaattaaa agtacaaatg tgggagaact ggaagtatta ttttattttg gcaagatgat 720

tttaattata aagccaactt attcataatt ttttattttt ttcatttaac tttataatgt 780

gagcatattt tattcttagt aaacacaatt atgaatacct atgtaataac ctatacccat 840

attcactttt atttaaccct tggatattta gattgctcct gaaattttat tttaattttt 900

taaaaaactt gcaaagaata tcttttcatg tagagtattt tgcacatttc tgatctctat 960

cttagcatag atttcccaag tagaattact gagtcagaaa atacaaaaaa aagtatgtat 1020

ttaatattca ttgccaaatt gattggtttt gatacaataa gtagttgaat tagtaattaa 1080

aactatctgt aacctacttc aggtatgata tattagatga ggcttaaata attttctaaa 1140

atgtatagcc aatctgtaaa ttcagcacaa tcctaataaa aatcccaaca ggcttgaatg 1200

taacaaaatc attaattcaa atttctggtt ataaaattaa tattgaagag aaaaggacaa 1260

aaaacagtca aattattttt tgatagagga atatgccctc ccagatatta agttatatta 1320

tttagctaat aattattaac tcagtatatt a 1351

<210> 90

<211> 851

<212> DNA

<213> Homo Sapiens

<220>

<221> variation

<222> (289)..(289)

<223> baySNP6498_A289G

<400> 90
tgcagatgga ggttttgttt gggggctttg tggatctttc tgcggtgtaa tagatcctat 60

tactgtttct ggagcaacag ttatgggaca ctgaatatta tcagtggctg cctttcttct 120

gtcttttttt tttttttttt tttttgagag acagggtcta cctctgtcac ccgagctaga 180

gtgcagtggt gcagtcttgg ctcactacag cctcgaactc ctggtctcaa gcagtcctcc 240

tgtcttagcc tcccaaagtg ctgggattac agttgtgaga ttgtgcccrg cccttctgct 300

ttaattttca ctctgggctc ctcaacatgg tccttgtccc aagcccaggg actggcttct 360

ctttctctga gtgtagttat gtatctgggt tctaaccagt aaggttataa ctgtggctaa 420

ttcacataac ttctctgagt ctgaatttcc tgcctacaaa atggagaaat aggaccttcc 480

acacagggtg catgtgagga tgaaaggagt gaatgtataa agacaccttt cccgataacc 540

catactaccc tcattatgac tcttaacatt agagagggct aaacaagggc agtgagaaga 600

ctggcaggca gcagaaatca ttttcttttt gatctcacac accagggcat tccccctaag 660

aggcagtgag atgtagcctg gccctggaac tagaaacaga ggtggctgct gtgctagtgc 720

ctgagatgca cgatgctggg gtcaaagact tgtgtcccca ccttcagctg ccagatgcgt 780

gtgtgccgtg acagggctcc cgatcatgcc tcaggcgaca gcaggctgac ccacattcag 840

gggtggcttc t 851

<210> 91

<211> 2958

<212> DNA

<213> Homo Sapiens

<220>

<221> variation

<222> (348)..(348)

<223> baySNP6744_C384T

<400> 91
cacacctcag acatgggcct cggggagcag gcactcggta ctccggggat acagagtccc 60

tcctaggcgc ttggccacac cagacagtcc ccactcaccc caggccccaa acactgagta 120

ctgggtgttg gcatgagagg cacacagcca atggagaagt tgggagctgg tgaggaggga 180

ggcatccagg tggcttgggg gagacagggc catcctctct tagaaacaca gagatgcctc 240

aggatatata tccaggtgaa gaaagcaaaa tactgaactc ggtctcagat gctacttttt 300

gcataagaaa gggagaatga aatatatatt tggtgtgtgt tttgtttytt tttttgtttt 360

ttggtttttg gtttttgttt tgagacagag tctcactcta tcgtccaggc tggagtgcag 420

tagcgtgatc ttggctcact gtaacctccg cctcccgggt tcaagcagtt cttgtgcctc 480

agcttcctga gtagctgaga ctacaggtgt gcaccaccat gcccagctaa tttatgtatt 540

tttagtagag atggggtttt gccatgttgg ccaggctggt cttgaactcc tgacctcaag 600

tgatctgccc acttcagtta ggattacagg tgtgagcctc cacgcccagc tcaaatatgt 660

attagtaaaa cagaagtatt agaactgtaa gaacaccatg acagtgcttt gacatctcca 720

ttgcctcatg gaatctcatt ctgagcttcc ttggttacaa gtgagaattt ttcacatggg 780

tattgctgta ggtatgtatg ttgagtaaat gacacaggtc tcactgtgtc ccccaggctg 840

aagtgatctt cccacctcag cctcccagta gctgagacca caggtgcaca cccacacacc 900

cagctagtga gagatggggt ctcactgtgt tgcccaagct ggtcttgaac ttctgggttt 960

aagtgatccc ctgagctgtg attgtgcgac tgcactccag cctgggcatc agagtaaggc 1020

cctgtataaa acaaaaaata aaccgagggc tgggccaggc acggtggcta acgctgtaat 1080

cccagcactt tgagtggctg aggtgggcag atcacttgag gtcagcagtt cgagaccagc 1140

ctgtccaaca tggggaaacc ccgtctctac taaaaaaaat taaaaactcg actggttgca 1200

gtgactcatg cctgtaagcc cagcattttg ggaggctgag gtgggcggag cacgaagtca 1260

ggagatcgag accatcctcg ctaacacggt gaaaccccat ctctactaaa aatacaaaaa 1320

attagctggg catggaggct ggcacctgta gtcccagcta ctcgggagac tgaggcagga 1380

gaattgcttg aacctgggag gtggagggtg cagtgagccg aaatcgcacc actctactcc 1440

ggcttggcaa cagagcaaga ctccctctca aaaaaaaagc actaacaaca actacaacag 1500

aatcagctgg gcatggtggg agatgcctgt aatcccagct acctgggagg ctgaggcagg 1560

agactcgctt gaacccggga ggcagagatt gcagtgagcc caggtggtgc cactgcactc 1620

cagcctgggc ggcagagcaa gactgtctca caacaaacga accaaaaaac gaaggaccaa 1680

tgctcaggcc atccagttcg gaatgcacta aattcagttt tatgaaattc aaagtaactt 1740

tttccttcat cccttatttt gtatggatta catttatatg ttataatttc tgttttcaga 1800

ttttgtacag cagcaatttc ttactctctc tgcaagtttt cttcccagtc acgagatact 1860

ttgtgcagct gcttatctcc aggccttatt aaaaaggtag gttcactctt ttcctatttt 1920

tccattctgc catacgtttc aggtttacgc agctttcgga ccggaaggcc gaggtctgct 1980

gccgctttgt gcccatctca ggggcagctc ctgggtcagg ctggctgtca tggggcagct 2040

cctgagtcag gctggctgtc atggccacct ctgctgtctg cttaggcagc ctgactgctg 2100

agtgctgcct ttccttgtgt tctctgagtg taaagtgaag ttgtatgaag acaactacaa 2160

gtctgtaatt gtaatgaaat agatgttggc atttatagga tagatgaatt aaaagatcag 2220

tattcataat aagtatcttt ccctggaaag aagaattctt attcctctca tctagtaaat 2280

gttccttaca ttgttcagac aaaggaattt tttttttttt ttgagatgga gtcttgctct 2340

gtcgcccagg ctggagtgca gtggtgcgat ctcggcccac tgcaacctcc acctcccggg 2400

ttcaggcagt tctcctgcct tagcttccca agttgctggg attacaggca cgtgccacca 2460

tacccggcta attgttttgt atgtttttta gtagagatag ggtttcacca tgttggccag 2520

gctgttttcg aactcctgac ctcaagtgat ccacccgcct tggcctccca aagtgctggg 2580

attacaggcg tgagccgctg gtgcccggcc cagacaaagg aatttaaaga tgagcattgg 2640

aaaccctgaa tgtcaaaagt aaaggtgaaa tggcactgca aaccagtgtt gtccttttat 2700

aggattttaa tgaacgtctc gtgtataaaa tagtggtttt caggctgggc gtggtggctc 2760

atgcctgtaa tcccaacact ttgggaggcc gaggcgggca gatcatgagg tcaagagatc 2820

gagaccagcc tggccaacat ggtgaaacct ggtctctact aaaaatacaa aaattagcca 2880

ggcgtggtgg cgcacgcctg taatcccagc tacttgggag gctgaggcag gagaatcgct 2940

tgaaccaggg aggcaaag 2958

<210> 92

<211> 689

<212> DNA

<213> Homo Sapiens

<220>

<221> variation

<222> (63)..(63)

<223> baysNP7133_C63G

<400> 92
tgtccagctt tgagacccta cagagccatg ttcacctagc acgtatcccg tctgcggtca 60

cgstcatttc ttaccttatt ccagggcttt cacctcagct tgccaggctg gagccaaggg 120

ccaacgcagc cgcgccttgt tcgcgatggt agcttcccag gagcccccta tggttccgga 180

acgcgctgcc ggccccatcc tgtttgctac ctcctaaagc caaaggcact ggcgggccgg 240

gccagcttct aaagtcgcgc aaggttagaa ggttccggac aggaacggcg tgaggccaat 300

ggaaggaggt acttcagttt ccctccagat gcccagcgat gggctcagag ctccttgaga 360

actcgggaaa ggaagcaggg tctctgaaga aatacttcag gagtagaaag aggaagctag 420

agggttaaat gcactacaca ggaacagaaa tgagtttttc ttagagttag tatatgtcta 480

gaggtgtagt aaactaaaac aagtcttgaa ttgcataccg ccacgtaggg aagaaatgaa 540

aacctttgaa tattagtgaa aaaagggaaa ctgcaacgcc tgtattacta gatagctttc 600

atcaacagct caaaaccgac agatttaaag aagcaacacc gcattttggc tttctaaagc 660

tttaatttgg tttggatccc atgcccatg 689

<210> 93

<211> 2184

<212> DNA

<213> Homo Sapiens

<220>

<221> variation

<222> (1603)..(1603)

<223> baySNP8021_A1603G

<400> 93
atttctgagg ccccacaggg agagctttct ctggaacaaa gagaggaaaa ccttgggctg 60
tccctgcagc cactactgtg gcttagggga catcactagg ggcctgagag ggccttagac 120
tctagcctgc atgggacagc agggtcaaat tcagactgcg gggtctgtct tggctccatg 180
ggctgggtga cctctgactc ctgggttcaa gtgattctcc tgcctcagcc tcctgagtag 240
ctgggattac aggcgcccgc catcatgccc ggctaatttt tgtattttca gtagagatgg 300
ggtttcacta tgttggccag gctggtctca aactcctgac ctcaagtgat ctgcccacct 360
cggtctccca aagtagtggg attacaggcg tgagccaccg cacccggcct ggaacaggcc 420
ttcttggcag gtgagggtgc ctgtgccagg tcaggtttgc catggatggt cacctggacg 480
tggttttctc ccaagctggg cagtgggacc ttcagagggc cagttccttg gcctgagagg 540
agcatcagca ctgtccaatt ggctgttttg ctgtgaaggt tgtttagagg aagggccaca 600
aactggcagc ctgtgggctg gatggggcct taaatttgtt gtgtggacca tgaggtgttt 660
cagtgagaag tgaacaagta gggccgggct cacggctgta atcccagcac tttgggaggc 720
tgaggtgggt ggatcacctg aaggtcagga gttcgagacc attctgacca acatggagaa 780
accctgtctc tactaaaaat aaaaaattag ctgggcatgg tgggggcgag cctgtgatcc 840
cagctactag ggaggctgag gcaggagaat cgcttcaacc cgggaggcag aggttgcggt 900
gagctgagat cgcaccactg cacttcagcc tgggcaacaa gagcgaaacc catctcaaga 960
aaaaacaagt agggctgggt gtggtggctc acctctataa tcccagaact ttgggaggcc 1020
aaggcaggca gattgcttga gctcaagagt tcaagaccag cttgggcaac ctagtgagac 1080
cctgtctcta caaaaaataa gatgtcaaaa aatcaaaaaa ttagccatgt ggaggggcag 1140

gcgcctgcag ccctagctac tcgggaggct gaggcaagag attggcttga acctgggagg 1200

cggaggttgc agtaggccca gattgtgcca ttacactcca gtctaggtga cagaccaagg 1260

ccccatctca aaaaaagaaa aaagaaaaac agaaaactga gcaagttggc agtgtttctt 1320

ctgtctgtga agcagttgtt tggtcgttga ttcaaggaag ggtgattgag ggccttctat 1380

gtactaggcg ctgggaagcc ggcagggcga gatgctcatc aaagaaagca ataaagaaac 1440

acacaccaaa aagtctgata gggctgaggt gctgcagaaa gagttgaatt tggccgggcg 1500

tggtggctta cacctgtaat cccagcacta tggaagaccg aggcagcagg atcacttgag 1560

accaggagtt tgagaccagc ctgggcaaca cagggagacc ccrgctctaa aaaaatacaa 1620

aaattagcta ggtgtggcgc cgtgctactc ccagctactc cagaggctga ggtgggatga 1680

tcgcttgagt ccgggagggc caggctgcag tgagtcgaga ccacaccact gcactccagc 1740

ccgggtgaca gaatgagacc ctgcctgaaa aaattaaaat aaatattaaa aaagattgat 1800

gcctccagaa agagcgtttt ctgcaggtct ttagaggaca tggagacact cctggacttg 1860

ggcaaaccta gaagggtgtc ccggggttgg acacagtccg ggcaaaggga gagtgatggg 1920

caagtaggga atcagtttgg ggctggtgtt gcagatgccc ccaaaggggc agaaggtggg 1980

ctgggagtct gctggtagat gacacctttt caagtgaggg gagaggtggg tgggtacatg 2040

gggagtttgt ggggacccct gcaaacacag gaaaccgagg ctcagaaagt atcacgcagt 2100

gggcaagtgg cgggtttagg atttgaatcc tggactgact ggcctacagc cccggccctg 2160

gcctctctgt gcatggccaa aatg 2184

<210> 94

<211> 607

<212>  DNA

<213>  Homo Sapiens

<220>

<221>  variation

<222>  (118)..(118)

<223>  baySNP8060_A118G

<400>  94
ctactcaact tatggtttcc atgctcagaa cctcatgaga tacattgcac gttactgtag      60

tacctgaatc cttcattgct attgaatgga tgtgccttat tttccactcc cacctggrga     120

atccaaacat atgctgatgc catgcaaact catgcaaagc gttttgagtg gaatagattt     180

tccacagttg ggatggggaa aggtacaatt aaagaaggga agctgacatt gctgtaatgt     240

aaaaaaatat tttatatgga aatttagcat ctcagacttt ggcttcagaa cataaggaac     300

aagatttacc ctttttctca acaactagac aggcagacaa aacatataaa acagaagttt     360

tcagacatcg aatgacagac aactcaagac agtaaaagaa gagtaacaaa ccagatgtgc     420

cctatgagca cctaaactta ctgcttgaag agtgtttcca gggaacagca caagcaggga     480

gaacccagac agagtctcta tgggttgagg aaatagagtt tggacttcgg agaagctcaa     540

atggctagaa tttgtgattt tagggcagag tcctgcagag gacagtataa tcacagagaa     600

agagctt                                                               607

<210> 95

<211> 1151

<212> DNA

<213> Homo Sapiens

<220>

<221> variation

<222> (255)..(255)

<223> bySNP8210_A255G

<400> 95
tggatccccc atcagttgac ccaatgcaag aatctcagac acacatgacg caccagttac 60

aggggttgtt tttaaagaaa aaaacaatcc cagcaggagg gattgctggg attgtttttt 120

ctttaaagaa gaatgttaat attttacttt tacagtcatt ttcctacatc ggaatccaag 180

ctaatttcta atggccttcc ataataattc tgctttagat gtgtatacag aaaatgaaag 240

aaactagggt ccatrtgagg gaaaacccaa tgtcaagtgg cagctcagcc accactcagt 300

gcttctctgt gcaggagcca gtcctgatta atatgtggga attagtgaga catcagggag 360

taagtgacac tttgaactcc tcaaggacag agaactgtct ttcatttttg aaccctcggt 420

gtacacagag gcgggtctgt aacaggcaat caacaaacgt ttcttgagct agaccaaggt 480

cagatttgaa aagaacagaa ggactgaaga ccagctgtgt ttcttaacta aatttgtctt 540

tcaagtgaaa ccagcttcct tcatctctaa ggctaaggat agggaaaggg tggatgctct 600

caggctgagg gaggcagaaa gggaaagtat tagcatgagc tttccagtta gggctgttga 660

tttatgcttt aacttcagag tgagtgtagg ggtggtgatg ctaccattac tgtgaggacc 720

taccagtgtg gctggagcag ggactctctc ccaggccttt tactcctcag cacctccctg 780

catactgatt gttgttttta gtttctgtga aattatattc atgaaatgaa aatagcgcat 840

tttactttgc tgtagtttca taaggtttta tacaaaaaag caagtaaata tggcagaaaa 900

gcactcattt gcccctgctc cctcaaaaca ccacagaatg acatagaact aaaggcggca 960

ggaatctaca agaatgaaga aaacacagtg atgctacctg caaaatcttg ggagccagaa 1020

agcaaatgga caattgataa tagagttaca agatgagaga aaacaaaaat gtaacctgtt 1080

agttggggga gcctagaaac atcctgtttt gtaccacaga cccctagaaa gtttcaagat 1140

gtaaaaacac t 1151

<210> 96

<211> 518

<212> DNA

<213> Homo Sapiens

<220>

<221> variation

<222> (46)..(46)

<223> baySNP8592_C46T

<400>  96
accagcctct tccctgagac accatggtga aggtgaaggc cagagycaac ggattggact      60

atattgggca cccggtcact agggctgctt ttaattctgg taaagtggat attgtcacca     120

tcaatgaccc cttcattgac ctcaacaaca tagtctacat gatctagtat gattccaccc     180

atggcaaatt ccatggtaca gtcaaggctg agaatggaaa gctcgtcttc aatggaaatc     240

ccatcatcat cttccaggac caagatccta ccaaaatcca atggggtgat gttggcacta     300

aatgtgttgt ggagtccgcc agtgtcttca ccgccatgga gaaggctggg gctcatttgc     360

agggggagcc aaaaaggtca tcatctctgc cgcctctagt gatgaccccg tgtttgtgat     420

gggcgtgaac tatgagaagt atgacaacag cctcaagatt gtcagcaata cctcctgcac     480

caccaactgc ttagcacccc tggccaaggt catccatg                             518

<210>  97

<211>  538

<212>  DNA

<213>  Homo Sapiens

<220>

<221>  variation

<222>  (181)..(181)

<223>  baysNP8816_C181G

<400>  97
   gaaaagggtg tagcacctgg gaaaggcgat caactcccag tcagggagcc cacggtgata 60
   acaccagtca gacgatcaca ccatggaagg gtccaatgag atccaatgga aagacttaga 120
   acatctaagg ttgggggagg tgcaaccttc tgctattcag cccctctgct ccaagcggaa 180
   scttttttct caggaggtaa tctctaataa caagcagagt gccctctgga gcctcctcgc 240
   tggtatctca gtgcctggac agagggggac acaccacagc acaaacacgt ggcacagact 300
   caatcccaac acacagccag tcaacgagcc tctggcccct tcctctgggt cctgatacag 360
   agctggcagc gagggcctct gaagaggtta cagggagccc aagggagtgt caggtagagg 420
   cagcaccagg tcccggaggg acagatgagg gatccctagt aaacagctcg tggacgcact 480
   tgactagcag ttcgaaagca aaaagagatt cggattacaa gagacttttc ccttgcaa 538

<210> 98

<211> 524

<212> DNA

<213> Homo Sapiens

<220>

<221> variation

<222> (196)..(196)

<223> baySNP8846_A196G

<400> 98
tggctgcccc tcggccgccg actggacgcg cgggccttgc cagcgagcac cctcatcggg 60

ctgtcgcctg acagcatacc tcaaaaggcc ctagctaata aacctgtaag cccagcattt 120

gagagaaggt agggtgtgta tatataaaag gttgtgtaca actccacgag gtgaaaaata 180

ttcagtaact tgtttrcata gcatttgtgt aaagactatg atctcatccc aataaaatga 240

tatattaaac cttcagatta atgactggct acagagtaac aaaaaataaa gaatttaatg 300

tacagtaaat tctctcccat acaaaggtct agtctgatgt tttgtgtaca aactcacatc 360

tccaattaac agtatttatt gagggtgact ttgtattgca ctaacgtcta ttgctattac 420

ctgttgtgat tgataagtaa agccactcat tgaaaaaccc aattccaaac accacagttt 480

gtgacacatg aagtaatgaa tgactcttgg tatgaaaacg tggc 524

<210> 99

<211> 1150

<212> DNA

<213> Homo Sapiens

<220>

<221> variation

<222> (351)..(351)

<223> baySNP8943_A351C

<400> 99
ttctgcttca ccaccttgtc tgcactggct atttttaaag atttgttcat ctagtaattg 60

taaaagtatc aagttgcctt acttttattt ctttgattag tagtaaaggg taggctgggc 120

atggtggctc acacctgtaa tcccagcact ttgggaggcc gaggcaggtg gatcacctga 180

gatcaggaat tcaagaccag cttggccaac atggtgagac cctgtcgcta ctaaaaatac 240

aaaaattagc tgggcatggt ggtgggcacc tgcagcccca gctactcggg aggctgagac 300

agaagaattg cttgaactcg ggaggtagag gttgcagtga gctgagatcg matcactgca 360

ctccagcctg ggcaatagag agagactcca tctcaaaaaa aaaaaaaaaa aaaaaaaaaa 420

gagctaacag tttgtcatgg acttcttact acttatattt tctgtacata ttatatgtac 480

atctatagag tggtttcctg ttaccattgc tcattttctt taatgtcaaa ccttttgaga 540

tagtttaatt atattgttta acagcagctt tataaaagaa ataaacagat ataagatgat 600

aaataaaaaa atataactag taagtgaaaa taaaatggcc aatttgaagc atttttttct 660

caatatcaaa tagaggaaag acctcaattt tgaaattctg atttttttaa ctaaagactt 720

gttttctagg catcgctctc tgcttctgct gctcagacca agggttatgg caggggaggg 780

actggtgcca tgatgaccgc ccccctgtgc tactcacaga gtgaatggga tgttctcact 840

ctcggccacg gtgccataca gagaaatctc aaaggcctga ttggtatggg tttcactctc 900

agtcccagaa aaatgaatct ttacttggta atggaagact gttggggagg aaattcgaac 960

atgttgatca atctttatga aaatatcaca accccaaata gtcaggcaat tcagaaccac 1020

agaagtatct cactagcact caaaccggaa gtgcacacac atggaaccac tggtgtggct 1080

tggaaagact tcatcatgca cagtgttcat acctagaaaa tgcagacctt gaattctact 1140

ccctttcaga 1150

<210> 100

<211>  783

<212>  DNA

<213>  Homo Sapiens

<220>

<221>  variation

<222>  (88)..(88)

<223>  baySNP9193_C88G

<400>  100
ctgcggcggc gggtgcagta gcatcagcgg gctcggagac acgctgttga gtggggggaa      60

accccccagg ccgttggagc cgaacgcsgc agcctcagac ccggggccgt aggggaggcc     120

ggtctgaccg tagacctgcg cgttggcggc ggccgcggcg ttgaactcgt aggcggcgcc     180

ctcggggtag ttgtacacgg cgggcttgct gctgtccagg tacacctcgc ccaggggccg     240

ctccaggggg atcttgagct gcggacggtt caggggctcc agctcgttcc cttggatctg     300

atgcagtagg gccatcccag atgctttggt gtggagggtc atggtcatgg tccgtggccg     360

cgggcagggt gcagaccgtg tccccgcagg gcagaaggct cagaaaccgg cgggccacct     420

ggaaaaagag cacagcccga ggttagaggc gacgcatcgc atgtcccgcc gacacgcgag     480

ctctggcccc ggccctgccc cgggagcctg cgggtccggt gaagccgggc gacccgacgg     540

gagcaagtgc agtcccagga cgaacgccct ccgtcagctc ctgggctccc gggcctccaa     600

ctttaagtac tggtctcccg agctcatatg cattacaaag gtgctggagg acggccaggg     660

actgttgcct tgccctgaca ttggcttaaa catcactcca agcacaactc gatttggagc 720

gatcccaaag agcagctttc ctgaacctta ctttacttgt ctttccctgc tggatagagg 780

ctg 783

<210> 101

<211> 611

<212> DNA

<213> Homo Sapiens

<220>

<221> variation

<222> (329)..(329)

<223> baySNP9443_C329T

<220>

<221> misc_feature

<222> (391)..(391)

<223> Unsure

<400> 101
tctagaacaa tcctcctgcc acaactttca ccctaccccc acccgacccc acccttttat 60

acacatcacc attgccagta ttgccttggt ttagccaatc aagatattac catcctcaaa 120

atatacgatg aacaaggaaa aaggtaattt tggagcaggt gagactctta agacagtctc 180

caccctcctt cctttccctt caagttcact tttgtcactc aaaactgcct gtcatcctgg 240

agaatacatt tagagattcc aaatgtaaaa catgcatttt tggagaaaac aaaaataaat 300

tcataatcag gattatgaag cagaattgyt tcaaaacagc aagctttcat tttcaatgca 360

atgccaaggt cattcactaa tttgtaagag ntaacctgag cagtacctga ctctgatcag 420

tccagatcga ggggagatct catttctaaa agaatttcca atctgggcag cagcaaaagg 480

caactttcct tggttgaact ccaaaagtcg tttgaaattc aagaaaatcc cctgtgcagt 540

ttctggtctc aagtacctat aaatttaagt aagtcagtct gttacaaaga aagaaattat 600

gttttccaac a 611

<210> 102

<211> 589

<212> DNA

<213> Homo Sapiens

<220>

<221> variation

<222> (187)..(187)

<223> baySNP9516_A187G

<400> 102

ccagagactt tccagatatc tgaagaagtc ctgatgtcac tgccccggtc cttccccagg 60

tagagcaaca ctcctcgccg caacccaact ggctcccctt accttctaca cacacacaca 120

cacacacaca cacacacaca cacacacaca caaatccaag acaacactac taaggcttct 180

ttgggarggg gaagtaggga taggtaagag gaaagtaagg gacctcctat ccagcctcca 240

tggaatcctg acttcttttc cttgttattt caacttcttc caccccatct tttaaacttt 300

agactccagc cacagaagct tacaactaaa agaaactcta aggccaattt aatccaaggt 360

ttcattctat gtgctggaga tggtgtacag tagggtgagg aaaccaaatt ctcagttggc 420

actggtgtac ccttgtacag gtgatgtaat atctctgtgc ctcagtttgc tcactataaa 480

atagagacgg taggggtcat ggtgagcact acctgactag catataagaa gctttcagca 540

agtgcagact actcttaccc acttccccca agcacagttg gggtggggg 589

<210> 103

<211> 1584

<212> DNA

<213> Homo Sapiens

<220>

<221> variation

<222> (1546)..(1546)

<223> baySNP9698_A1546G

<400> 103

gcccagttgc ccctggtccc caagcagcag ccccctctgc ctccagggtg ggcctggagc 60

aggagggggg cctctgcccg ggggccgggc tctctggggc cgccgtcgcg ccgccccctc 120

cccgcaccgc ataccttgcc cacagccgag cagctgggag gctatttata aaggcgggtg 180

agatcagcgg ccggccaagg ctataaattc gcaggccgcg gccgggcccc acaggagcag 240

ccgcccgggg caccggagct gcgggctgcg tggccgggat gagcgccagc acgggcggtg 300

gtggggacag cggcggcagc ggcggcagta gcagcaggta gggctcggct ggggcacccg 360

gagcccctgg cgtctctcat gcccactgcc actcacccca cccgcagctc acaggcctcc 420

tgcgggcccg agtcctcggg ctccgaacta gccctggcca caccggtgcc tcagatgctg 480

cagggccttc tgggctccga cgacgaggaa caggaagacc ccaaagacta ctgcaagggt 540

gagacttggc cttggggaca tgcggcctca cggccactgc agggacccag ggcagtcctg 600

ggcccacatg ggccagatgg tcaatggggc cgaggtgttc gggggcccag gggagtgaga 660

accccctcca ccccaatcta catctcccct gggcaggcgg ctaccaccct gtgaagatcg 720

gcgacgtgtt caatgggcgg taccacgtgg tgcgcaaact gggctggggc cacttctcca 780

ccgtctggct ctgctgggac atccagtgag tgcctccttc gcctccgggg cccagcactg 840

gctgggatcc tgtccctggg cttgcttggg gccaccctga tccccgccgt gggtcctgcc 900

agggccacag cctacaaggg tctcggtatt gcaggcgcaa gcgctttgtg gccctcaaag 960

tggtgaagag tgcggggcat tacacggaga cagctgtgga tgagatcaag ctcctgaaat 1020

gtgtgaggca cctccctacc ccactcccag ctcccctgga gctgcctggg gcctggcaat 1080

gcgggtgcaa ggcctgccgg ggctctgtgg ggcagggcgg ggctccctga ggggcagcct 1140

ccagctggct gtgcccaagg gggaggatct ggaggaacag gcgagggaca ggaggggttg 1200

gcggcctttc ttccagcagg gcccagctgg agcaggagaa gggtacactg aagggagctg 1260

tgggcttcag ggcagggtgg aaccatctgt ggccccttgg cttttgctcc aggtccggga 1320

cagcgacccc agtgacccca aaagagagac cattgtccag ctcattgatg acttcaggat 1380

ctcaggagtc aatggagtcc gtatcctttg caggaagagc aaagcagtgt ggcagccaag 1440

ggccggcaaa tgggggggcc ctcgctgcta gagcctgtct gcagacccgc acaatgggct 1500

tgcatccctc ccagtaacgg gagcctctgg cacgaccccg cccccrggta actgtgttta 1560

cgtggaggca gtaacaagct agcg 1584

<210> 104

<211> 744

<212> DNA

<213> Homo Sapiens

<220>

<221> variation

<222> (277)..(277)

<223> baysNP9849_C277T

<400> 104
gggctagagg ctgagcacct ttggaaacaa catttaaggg aatgtgagca caatgcataa 60

tgtctttaaa aagcatgttg tgatgtacac attttgttat tacctttttt gttgttttgt 120

agcaaccatt tgtaaaacat tccaaataat tccacagccc tgaagcagca atcgaatccc 180

tttctcactt ttggaaggtg acttttcacc ttaatgcata ttcccctctc catagaggag 240

aggaaaaggt ataggcctgc cttactgaga gccaaayaga gcccaggaga ctccactatg 300

ggaaacctca ttgctctgta caaagtacta gctaaaccag aaaggtgatt ccaggaggag 360

ctagccaaac aacaacaaaa acaagaaatg ttctgttcaa gttttcagct ttaagatatc 420

tttggataat gttatttcta ttttttattt ttttcattag aagtgaccaa attaagatgg 480

taagacctct gagaccaaaa ttttgtccca tctctacccc ctcccacctg cttacagaat 540

ggatcatgtc ccccttatgt tgaggtgacc acttaattgc tttcctgcct ccttgaaaga 600

aacaaagatc gtgtttttgc cactgattta gccatgtgaa actcatctca ttaccctttt 660

ctgggtttga agctgctgtc tctagaagtg ccatctcatt gtgctttgta tcagtcagtg 720

ctggagaaat cttgaatagc ttat 744

<210> 105

<211> 560

<212> DNA

<213> Homo Sapiens

<220>

<221> variation

<222> (249)..(249)

<223> baySNP9883_A249G

<220>

<221>  misc_feature

<222>  (506)..(506)

<223>  Unsure

<400>  105
   gatctgactg tcgtgtgttt tgttcctttt tttcttttac actaagtatt ccattaattc      60
   tcagagaaca tttccacaac ctcaaaacca cagagaggga ggctaggggt tagaactcac     120
   ctggcagtga agagcctgcc tagacctgag ggtgccagag cgcaggccac agcgcaaatg     180
   ctccggagag tagccctcag ccagccactg tgagggtgcc caggacagag gagacgggaa     240
   aggggaggrg tgagccacgg ccacggccac catggcgcct ccctgttcac cagggtccct     300
   tcgatgggct cggtggggtg gggtggcttg tccctgtgct gctgagcttg caggactgtg     360
   ccttttttta aaggattagg agcctgccgc cccttggttc tggttactaa ttcttactct     420
   cccctccccc tgcctttttc aaaattcaag gtgaaatctg tgcactgtca agctggagac     480
   acagttggag aaggggatct gctcgnggag ctggaatgaa ggatttataa cctttcagtc     540
   atcacccaat ttaattagcc                                                 560

<210>  106

<211>  555

<212>  DNA

<213>  Homo Sapiens

<220>

<221>  variation

<222>  (249)..(249)

<223>  baySNP10079_A249G

<400>  106
gtaccgaggc aggccgcagc tcccaccatt gcgtacagcc ctggcgtgac acagtctgca      60

ccgggtctgc accagttcct gaagatgatc cagtcatggt gatggtaggc cagctgctcc     120

acgccaattc ccaccatcct gcccgctatc gcgcccacag ccatgctggg gatgaagagg     180

cccgacggga tcttgaggag acacagagaa aagcaacatg agggaaaaag accaagtccc     240

cgcttcacra cccccaagaa acacattccc cctcgacggg cttgcattta gcttcagtct     300

tcagaggagg tgcaactctg caaaataggt cagctgggga aaggtgggac aaataatttg     360

acctgcctag tcagtcatat ctactgcaac ttagagagga cgcaatttta tactttgatt     420

ccctctcctt caaaagctgg ctgggtttgc ctgctgtgga ttaaagaatg ataagcccca     480

ctgcaaataa cattcaaagt gagcatctgt cagttgagac accactttct ctcgcccctt     540

gccatgtcat tcaga                                                      555

<210>  107

<211>  609

<212>  DNA

<213>  Homo Sapiens

<220>

<221>  variation

<222>  (107)..(107)

<223>  baySNP10481_A107T

<400>  107
gaactgcggg ggactgggcc gcgggcctcg ggggagggcg gccgccggcc catccagagg      60

tggcccacgt agcgggacag cgctgtcggc ccggcgcgcc tcggagwgtc acggcgcctc     120

gtccaagtgg agccccgaac ccctgaaggc gcggcaggct ctggagagcg gggtcttgtg     180

cgcctgggcc aggtctgggg gctcttgcca aactgcacgt ggcctgtact gctccagggc     240

cccttggggc tcgtccccga gcggggactg cgggggggtc ccccgagcag catgttttcc      300

acagcgcgtt atgtttggag cgggccctgc gccgcctgtc gccatggaaa caaaacaggg     360

gcggtggcgg cggccggagc ggaggccggg ctggggcttg ggtgggggag gggaagagag     420

gctcgcaggc tgtcgcttag gtgacgggaa ctcaggcgcc cctctgcttc atccgggtca     480

cggcccgtcc gctagtaccc acagtgttcc acagtctggt ccttggctcc tcgcctgtac     540

ccctggtctt ctgcgcctgt ccctggtgtc cctttcctct tttttggttt cttcactctg     600

acctcactg                                                             609

<210> 108

<211> 2388

<212> DNA

<213> Homo Sapiens

<220>

<221> variation

<222> (367)..(367)

<223> baySNP10542_C367T

<400> 108
ctctaacttg ctgtgtgatg agtgaattcc tttcggaagg ctccagtttt ctcctgtccc 60

cctttctggg ctgctgctgt cgagatgttc agggcagact caagtcgcca agatggtcct 120

ttgataaatc tcatggtgtg gagtggagcc aggtcatggg gagacactgg gggagcaggg 180

cagcctctct ccccttccag actctgcact taggctggct cccctgtgtc cctgtctcca 240

gtctctcctg gcttcagctg tgtgactttg agtaagtcac ttaacctctc tgagcctcag 300

catcctcagg tgtaaaatac actgggtcct gcttcctctc tgggcttcct tctcttccct 360

ccccttytct gggctcccct gacccccagc ttcatcctca cctgtgtgtc cacccacttg 420

gcaccctggc tggtgtgctc cacatggccg tagaagtgca caggtagcgt gaactcgggc 480

cgggccttct cccaggggtt gccgtagcga agccagtcat cggcctcctc catctgcacc 540

caaggcaggt cagggagaaa ggccagcagt atcagtacag gcactcacag tgcacggtgg 600

ggcagggtgg gggctgtggg ccggtgtacc ctacaccaag tataagtcag gagctctgag 660

gtgggcccct ggtctgctgg gctatcgagt gggacacgga ccacctgttt caggggcacc 720

agctggcttt gggtcggggg gtggggagca gcaaggatgc tttccacaga gcttgcgggg 780

ctgtttcgga cctttccaga gatgataaac aagtgggggt cttccccatc ctgactagac 840

cccacaagtt agagccaagg ctgctcacct gccagccccc ggagatcttc tggttaaaaa 900

tcccaaactc atagcgaatc ccgtagccat aggcggccag gcccagtgtt gccatggagt 960

caagaaagca ggctgggggt gtgcagggag gtggctgtca gggacccagc aaggaggacc 1020

ccatcggccc actccaccct cacggccctg tcttcttacc tgccagccgg cccaggcccc 1080

cgttgcccag ccccgcatcc tcctcaattt cctccagctc ctccatgtcc aggcccagct 1140

ggaggagtga gggtgacagt ggtcagggtc aagtgtcagc agtggaatcc cccagtcccc 1200

acggcttgcc ccaccccaca cacacctggt aggtggcctc gtcacaggca ttctctaagg 1260

ccaggttcac catggtgttc tgtagcgtcc gtcccatata gaactctaaa gacaggtagt 1320

agatcctctg cccagagaga cggatgggca gggaatgggg tcagggccac acaccaacac 1380

tcagccaggc ccagccacgc ctggacctct ggcccgcctg ccctgctcag cagtcccatc 1440

cctgtcccca atttgtttct ccctgtctca ggctttgggg cccagcggtt aaggttctgg 1500

tcccagcatt cctctgactt tggacaaatc actctctttg gcctcagtct cctcatctgg 1560

gaaatagcgc tggccatacc cacttcaggg actgaggtac tcgggagaat gggcacccct 1620

tccccctgca cacacccccc cgcacagccc agcaggatgc caggacatgt ggaagcgcct 1680

ccaagagctc cccaggaggg caaggccaag gcccagagaa aaaagtcatt tgctcaaggt 1740

cacacagtgg ctagagactg ggctagagac tgtcacctcc catccttgca ctgtccagtt 1800

ccccttggcc ctcaagcctc aatcccaagg agacccagag gcaccagaaa ctaggatgct 1860

gccagtgcct ggtctcccag tttcctgggt cccctcgtgg gccgtctgcc cgcccctccc 1920

ccactcccca ccgaggcgtg tcaggttgct gacatttacc agtgacccca ccggacactt 1980

gatctctgcc gccccagctg ccccagcccc gcctgctgca caggcacacc gcgcatacat 2040

gcagcaactg atgtgtcctt ccacctgggc ccagccaggc cacaggcgcc gtcctgccca 2100

ccagcaccca ctccaacctt ctccgagtgg gcactgaccc tttgcagcca gcccacctgc 2160

tctgagctcc ctgaccccca ctcctgcccc aggtttctcc ctcagattgt cccagcaccc 2220

cttgtgcctg gcacccctgg attctccacc cccaagaacc tagagtgacg aggggcagcc 2280

acttaagtca agatcgccag ctccctggca gcgccttcag cccatacccc caccccaggc 2340

tccccagcag caccttgggg tccttctcat agtagtgctg ctgcgtgc 2388

<210> 109

<211> 597

<212> DNA

<213> Homo Sapiens

<220>

<221> variation

<222> (510)..(510)

<223> baySNP10600_A510G

<400> 109

gggaagggaa aggacctgcc agtgggcagg gtccttaagg aggccatctg ttttggcagg      60

atacgtacta tcagccccgt gccctggaga aacatgctga cagcatcctg gcactggtat     120

gtctaccctg gcatctggga ctctgcatct ttcagaccca cctgtcctct tgaacttagc     180

cctccctaac ttgggagcaa cagtgccctg gggttggggg atgccctggg ctctgcagca     240

gacctctcca acacagacac acaggcacac tctaaatgtg catacttggc actccccttt     300

ggtatgtagc actgctttct ctgggggcaa cctgagccgt aaggaaaaca tgtttactct     360

tgggaccatc tgcaggtgct aagtcctgca gttccccagt gacctgtcct ccctggcaac     420

taacctcttt ggctgcaggg acagctgttc tttctggcac agttcctgtg gtgacgtttg     480

cgcatctctt ggggaccagg ccaggggctr tcctgagcag ggccaattga aaaggcttga     540

ttaacttgag tagacagaga gactgccttt aaatgtgaga gaattatgtc tggagat        597

<210>  110

<211>  569

<212>  DNA

<213>  Homo Sapiens

<220>

<221>  variation

<222>  (402)..(402)

<223>  baySNP10621_C402T

<400> 110
acatgtatgt ccgtgttgca aaaataatac atgcctcaaa aacctgccta ggggagccct 60

agtgcctggg tgctgtggcc tgaggtagca ggtgggaagt tagggatgtc acagaaatgt 120

ctgtgtctga atccaggatt ggggtgggtg ttggagaggg ctttcagctc ccctcctccc 180

aggggggcct ctttttttaa cggctgccgt gcccttcctg gcccagccct aaacctaaat 240

tcaaatctcc tccatgcctt tgcgcaaagg acctccctct tgcactctaa gccttagttt 300

cctcctctaa aaaaaggggg tctctaaaca ggagctacct catagggttg ttgaggatta 360

agtgaaccaa tacatataca gtgcttagca cttaataagt aytcccccct gcgacaccta 420

gctgaactat ggtttggtgt ctgatcttga gaggttgatg taacctttta aaggcctcag 480

ttcgctcacc tgtgaaatgg gtctaagaat agcactgatc tcacagggtt gtgatgcaga 540

ttaaaggaga tggcatgtgt aatgtatac 569

<210> 111

<211> 631

<212> DNA

<213> Homo Sapiens

<220>

<221> variation

<222> (431)..(431)

<223> baysNP10745_A431G

<400>  111
aggcatctac ctgggtcttc atggtgttct gtgtagacac aaagagctcc atctcccctg      60

cctcgccttt tggaacagca atggtgcagt gagtctccag gtagaagtgc tcttggccac     120

cgatgtatat ctcccctggg gaagcagtga gatccctcac agtgagccca atgctgcaga     180

aggggccagc ttgcctaagg agagagtata ctctgccaag tggtctgggc aggaagcaat     240

ggctacctga ggggtaacct caaggtttcc cacttggggt ggaaaaggat cccaaagtct     300

tagctatctg ggagctgagt gctccagcct catccccacc cccacaacaa agaaagaacg     360

acactgaaat gcatccagtc caaagttctc ttctcactgc agcttatgga aatcaaggat     420

ttcaaaaacc rtcattgtaa aattttggaa aaagtaaagc agggattaat agatgtgatt     480

gaaacactga aaaccagcgc atgttcccga acctaagcta ccaggcctgg tggagagtcg     540

gcttcatacc aggcttgttg gagagttggc ttcatagtga ctttgcaaag cacaacagcc     600

aatgtaaaaa acagttccaa aagaggtagt c                                    631

<210>  112

<211>  2956

<212>  DNA

<213>  Homo Sapiens

<220>

<221>  variation

<222>  (2831)..(2831)

<223>  baySNP10747_C2831T

<400>  112
taatacatgc acatccaggg acggggagct cactcctcta cacaggcagc ccattctcag      60

ttacctcaat tgtgataatg gctggtagtt cttcataggt gattttcacc ccttgggcag     120

ctctctgtgt gtgttccggg gtgtcagcaa ccacagcacc aatgatatgc ccaacacaag     180

taacctagta gcagagacag actgttaaag aacgcaacca ggctcatggt gaaacaaatt     240

atttttggat tgttcaaaaa gccaaaagaa gtccctgccc tcacaatcaa agcacacttt     300

aggaatgccc tagggcctct taatttcctt atcctactga ttgaggatca ctaggatcaa     360

gatggcaaac acatttcatc tcaagggcca attctgagta tttagtactg gcagactatt     420

gagaagctga accagataga atgtggcaat tgattccttg tttgccatga gcagaaaggg     480

ggatatggca ccaggaagtg catttgtcaa ttcggattca ctccaacacc attggtcttc     540

ctatggagac tcagaaggca aggccacatt tggctagtta ctattgacag atcatgacta     600

accttaggcc ctgaccccca gttttatttt cttttcacct caccactctc ttttatgttc     660

tatttcccag atttatacca aaagcttctc aaaggctgta aaggctgtaa gcatcaatat     720

aaggcctgta agacacgtac ttacaagaca tgatacttta agtatcatgt cttatacttc     780

aactaatacc caaaagcagt agctctcaaa tactggttct tagacttgat gacccataaa     840

aatattttta aaaatttata tgataatgat tgttgtttta ttttgtttaa taattcagtt     900

tcataaaagc ttaatggatc tccttcagag ataattgtca tccaacatcc ccgaaaaagt     960

aaacaacaaa aaatttttgt acaacacatc actttagaag atgaagtttc tgacaatagg    1020

aaccaacaat tagtatgcat gtctctgata attatctctt atggcccaaa taagctattg 1080

actcagttgg ttagtgcaat ggtggttaaa tctggctgtt ggttattgca atggtggtta 1140

aatctggctg actttcacta gccataatgc tagcactgct ctgagaagct cttaaagtat 1200

gtaaagcaat ttgttcttct atgaaatgct ctcctcatgg agtctttgtt ctcatatttt 1260

tagttttttg ttatctcctt cacttttaca tcaataataa gatgaattca ctcttcagaa 1320

gtacccactc atgtctaatt tgcaacattg gatgtctatt ggaattacaa aatgactgtc 1380

gagaactatc ctatgagtaa aatataagga cttttgtagc agctgtctta aacatttgaa 1440

aaattttgtt tgttgttgaa gaattccaca tagttgaaat caatagataa actttcatgt 1500

aagtgaatta ccaattcacg tagtggtggt attgtgattt ctagttagtg tactaaatgt 1560

attcttattt tattttaatt aaagagctta ctgttgtccc ttaagaaccg aattgttagt 1620

tcaaatgcaa tcaccagtgt tgttttactg ccactttggc atagagacat cctgccatgt 1680

acaaacagca gccatcaatt aaataaacaa gtaacatatt ctatcttttt ggattgtcct 1740

gaatatgggc caactggatg atggccataa tccatgcaaa tgtataacct ttccagatca 1800

ggagtttgga gcaatgtaca caaattaaaa cttcaatact tattcaattt acttcatata 1860

aaaaaatcca agacttacct tatccttcgc aaagactgtc tcatcattac aaattccagt 1920

tatgttactc ccaggaacat catcagcgga aatgaaacaa acaaaccctg gaaccttctt 1980

agcttctgat gtatctatgg acctgcaaga atgagtggtg tgaggggcca ggtcagcaag 2040

ctggagcagg ggacccatca cctggttgga caaccctgtt cttattttga ttggcttggc 2100

tccatcctaa ttcctcgatt ctctgcttct tttctattag attctaattc aattatatcc 2160

tattctgcaa atgttgatta aatcctcacc atgttcaggc cactatgctg aatgttgctg 2220

gggatataaa gtgaataagc cagccaggca cagtggctca tgcctataat cccagcacct 2280

tggcaggtca aggtgggagg atcacttgag cccaggagtt taagaccagc ctgggcaaca 2340

tagcaagatc ctgtctccat ttaaaaataa ataaagtgaa gaaggcaggc caggcctctt 2400

tggatgttac gtgatgctct gtctggtctc caagtacaag caagccatat cagaacgaac 2460

agcacttact ctggagcgct ctggctcatg cacacatcac acctaactcc ctcccaaaat 2520

cagcacatgg agcccggctg tgccactgat ccaatggccc tgaacagctt cataaagctg 2580

gatttgtctc cccacattca ttgtgctatc ccagcatgac agggcatcct accataatta 2640

ggcaacacga accgcaaagg gagaatgccc gactatatga tcaaggataa cagtctcaac 2700

tatgtactca tcacactggc acatgccccc atggggaaat agggcaacac atttcaggga 2760

aaggtatgta aactgccctg aagttctaac catcttccaa atgcttaatc ctcacaagaa 2820

accctgaaag yacttatcct aattcccatt ttatgtttgg ggaaactcag actcaaggaa 2880

gttaggtagt ttttccaaag tcactaagcc agaaagcagt gtgactggga ttattggaac 2940

tgaggttaag ttagga 2956

<210> 113

<211> 557

<212> DNA

<213> Homo Sapiens

<220>

<221> variation

<222>  (64)..(64)

<223>  baySNP10771_C64G

<400>  113
ggaggttagg gtccccaggg aagagcagaa cctgggccca ccgagttact gaaggcaagg      60

aagsgtggcc tgtgtcaccc tggcggccct gcctgggtct gctcctcgct gtgcacactg     120

catcacgccc cgctggctgc ctccctctgc ctcttcccgt acagacgcac actcacagag     180

atcatgcaca cgacacgggg cggggacgga gggcccatgg cagagaaggg agcatggggg     240

ccgggaggga gggtgctctg agccagaggc gggggttggg agggcagccc agccaggtgg     300

cagcagccag ggcctccttg gagtggccca cctggagtgg agtggcagtt tgggtggctt     360

ctaggcgaag atgcccccaa cagtggaggc gatgacgatg cccaggatca cacagcagat     420

gatgatcatg attctcttct gcatgggaag cgggcaagag aggcctcata cagtgttggc     480

ggcacgtggg gtgggactat tgcaaaggct gatgactgga cagtcggagg ggatccaagc     540

aagggaaggt gactgcc                                                    557

<210>  114

<211>  544

<212>  DNA

<213>  Homo Sapiens

<220>

<221>  variation

<222>  (339)..(339)

<223>  baySNP10870_A339G

<400>  114
ggcagttctc gatattcacg atcacggctt tgaagtcccg gtcaacctgc ccctctctgt      60

ccccggcatt cattgacacg ggctttccct ggcctgggat cccatcccaa cacacacgac     120

agaaaacagt gcaaaataat aacacaaatg ccaaatgtac acagtgtaca actctgaact     180

gagaaagtgc aaggagacca cgggaatgga agtgggtagg ggtcgggagg atgggcacca     240

ggctggtgtc ctgacagcca cacctgggtg caggccacgt gtcctcacgg ccaaggtaac     300

cgggtgtctc aggcacttaa taaatattaa gggtgaccrg tgactcaggc tctgcctctg     360

ggaagtggca tcatttggtg aatgagtttg gtctcggtgc caccacggat tcagccagat     420

catttccgac gccatgaatc cctttcctgg cttaaggaag gtacaaaccc acctcgaaag     480

acactgggtg ggtcatgatt cgggggtgac agggcaaagg ctaacctggc tgtctagcaa     540

ggct                                                                  544

<210>  115

<211>  1357

<212>  DNA

<213>  Homo Sapiens

<220>

<221>  variation

<222>  (1290)..(1290)

<223>  baySNP10877_A1290C

<400>  115
ggtaagaatc aatacatata acacagggga ttttaggata gagaaactat tccatgtgat      60

attcgaatat ttgccaaggt cacagagtta gctgggccaa gacttaaact caagtctctg     120

actcccaagt tcatgctctt tccattaatc taagttgctt cttttgatat ggaatataac     180

aaagactaca ttgactatga aaatataaac ttaggggagg aaaaggcaac tgggaaagaa     240

aaaatatatc cttagtctgt caatcagaga aaactgttag cttgagcagc ttcaacttgg     300

ttaatcagta tccctctctt ctaccattaa gaaagaatac gtacttgcaa aagaattaag     360

gaatctatcc tctcttccaa agatgtccga cggctttacg ataatggctt ccggaaatgc     420

atctctcact actttctctc caacagccta aacaataacc aaacaaacct gttttgagtt     480

tagagacaag tgaataaagc atccattctt tccactatta agacatgact tgtatttcaa     540

aggcgatatt ttcctccaga taaaggtaat catgtaattc ataaaactca gttacacaga     600

acttttaaaa aatataatcc acaaaaatgt cctgcacctt acagattggg ttgggtctct     660

tccaattttt ttattttaag ggaagcttac attgaagaag gagaaaaatc aaagtagaat     720

ttcttcagcc tccatttttc cttctattta cagattctct ggtttgtgtc aaccatctag     780

cagcctgtta gaaaaaggac agtatggtgg cagcaggctg caataatgga agtgtcacca     840

gtacctacat atccaaagga ggttggggat agagtcacta atcctcctaa tctcaattgc     900

tgtaccatcc ttacttgtta tcatctacta gcctcctagg caagtcttct tgttcgatga 960

agatatcagc acctaattca gtcttccttt ctacttccac caccactgga ggtgacatga 1020

tgtccaagtg ggcttggact tctcaggttc ttgagctgct caactccaac gacctcctct 1080

tccttccacc tctaccactc ttaacttggt tacaccacct actgtatcaa catctggaat 1140

tgctcttctt ctgaggtatt aaatgaagac atcctgctct tggatgtcca tttcctacaa 1200

tttccagcct gcttgttctt tgttcatctg ctcctgggat acctgtttct caaccttctc 1260

aagaactcta gtgcactgat tctgcttccm ctgtatcaac tcttccgact gttcctgtcc 1320

ttccctctga attccagatt aggttccata gaccatc 1357

<210> 116

<211> 623

<212> DNA

<213> Homo Sapiens

<220>

<221> variation

<222> (140)..(140)

<223> baySNP10948_G140T

<400> 116
gagtccagat ccccagcccc ctctccagat tacattcatc caggcacagg aaaggacagg 60

gtcaggaaag gaggactctg ggcggcagcc tccacattcc ccttccacgc ttggccccca 120

gaatggagga gggtgtctgk attactgggc gaggtgtcct cccttcctgg ggactgtggg 180

gggtggtcaa aagacctcta tgccccacct ccttcctccc tctgccctgc tgtgcctggg 240

gcagggggag aacagcccac ctcgtgactg ggggctggcc cagcccgccc tatccctggg 300

ggagggggcg ggacaggggg agccctataa ttggacaagt ctgggatcct tgagtcctac 360

tcagccccag cggaggtgaa ggacgtcctt ccccaggagc cggtgagaag cgcagtcggg 420

ggcacgggga tgagctcagg ggcctctaga aagagctggg accctgggaa cccctggcct 480

ccaggtagtc tcaggagagc tactcggggt cgggcttggg gagaggagga gcgggggtga 540

ggcaagcagc aggggactgg acctgggaag ggctgggcag cagagacgac ccgacccgct 600

agaaggtggg gtggggagag cag 623

<210> 117

<211> 555

<212> DNA

<213> Homo Sapiens

<220>

<221> variation

<222> (286)..(286)

<223> baySNP11001_C286T

<400> 117

gggccaagcc tgcaaacttc ttgatattcc ctcgatatca caaagaccct cagaaaacat 60

cctcggcgac cacacaggca cgaggatgac gctgcacaga gaaggagcca ctgggcaccg 120

ggcggccccc ttcccaaaga cccacagtgt gggggcacac accacgatca tcatgtagcg 180

ccggccgccc tggtgcagct cctgcaccat ggccgggaag tcccggaagc catccttgtt 240

gaacgtgaag tccctccggg agtccatgta gtccaggtcg ttccaytgga cgtcctgcag 300

ccacaacacg ggaccgtctg ctggggcctg aggagacgcg tcccggccag ccccttgcct 360

cccctgccac caccccaact caccaggggg aagtgggccc tggtcatgtt ctccaccacc 420

tggcgggtga tagcggtgga ggagtagccc cagcggcaca ggtggaagcc caggccccag 480

tatggcggca tgaacgggta tcctgcaggc caacgccgac ttcatgaggg agggaggagg 540

gagccttggg gcggg 555

<210> 118

<211> 1051

<212> DNA

<213> Homo Sapiens

<220>

<221> variation

<222> (215)..(215)

<223> baySNP11073_C215G

<400> 118
gctgcgtccg ggcccccggc tgtcggcgcc ctcgcggctc agatcatccg ggcgttggag 60

agggaggcag agagcgggtc ccggagaagg aggaggaaga gccgaggact catcgctttc 120

gcgcctcagc tgtctagagt ccccccaccc ccccctcccc aaccacccgt tccggagcaa 180

cagccgcctg aggacccgaa gctgcgggcc ggacsagcgc cgagaccact gctctctagc 240

tcccgcgcgc gctcccgtcc ctagcgccgc caccgccgcc gccgccgcct ctagcgcggc 300

cccgcgcagg cgcgctcacg accgcgtcgc ccctccccca cccctccgct cgccccgccc 360

cgctggctcc gccgccgcta cagctgcggc gcggcgcgca gctacagaga cccggatgtc 420

gttgggcagg aactgcggtg agggctggcg ggcgggggag gggccgccac agatgacgtc 480

actgccttac ccagcttctt tctctgccgc tgctgctgcc gccgcctttg ccttacggcc 540

tggagcacca ggccgaagcc gaagggagcg tccggatccc ggatagcagc caggacccgg 600

gccttcacgt tccggcgcca gggactggac agaatgtgcg gctattaggt gcccagtaga 660

tcgacagccc ctccgtgccg gcccttggcc aagtagggac tcggaagcta cccctacggc 720

gtccgcgccc ggagccctgg ctcttctacc tgcctgcaga tacaaacccc actccctgcg 780

aagatggtgt acacgcagcc ccgagcacag acccgaagct gtccaccctt cttgggcgcc 840

gtcgcggtct ggccgctgcc cttcgaaccc atggtgggtc ccaggcagct gccagacccc 900

cctaccccag agtcccactc ctccagagcc ttcctctccc agtgacccca ggaagcccaa 960

gcaggtgcca cggccacaca cctcgcgacc caaagggcag agacacaagc acgctaccac 1020

cgcccaccaa tgtgcgagtg caactgattc a 1051

<210> 119

<211>  478

<212>  DNA

<213>  Homo Sapiens

<220>

<221>  variation

<222>  (116)..(116)

<223>  baySNP11153_C116T

<400>  119
ccactaccct acaggccctg cgagtgggaa gaaaagtaga aactgcttag ctaatgattg      60

acctcagccc ttcttctact gctttgggct tagatggaga ggtcaaagct ctcaayggcc     120

tctaccctat cttgggcgct atgcccagta attctaggca ggcagtcatt cttagaggag     180

cagcccccag cccccacgaa cacagcccag cagctattgg gaagttggaa tgcccagatt     240

tagttcctcc ttccaaagct gggccagagc tgagtcttga attgagctgc aacaacttta     300

ccattcttgt tcccttattc tgccccgagt tgggtcagcg ggctggtctc cctgaagtcc     360

tgttatcttt cagcagctta tgttaaggca gccagcattc tcatcgtagg aatggaaagc     420

ctgggaaaat accctcctca gctctcagta agtagtgctg gcttcatttc taagtaga       478

<210>  120

<211>  1197

<212>  DNA

<213>  Homo Sapiens

<220>

<221>  variation

<222>  (194)..(194)

<223>  baysNP11210_C194T

<400>  120
ttaaccagaa ggcagtacgg agaggaaaaa gtgtaaaggc tctctcagta gcagaagaga      60

atgaaaaagg gagaaagttt ggaacagagg agtgagggaa agtaaggctt aagacgaggc     120

aggagaaagg agcaggagtc ttggccaaaa taaagatttg ggttttgttc cctaagccag     180

gaaatgacat gatyagattt gcttttaagg atccttccaa actgctaaag acatgtagat     240

caagtattca ttggctgggc gcggtagccc acgcctgtaa tcccaccact ttgggaggct     300

gaggcgggaa gatcccttga ggccaatagt tctagaccag cctgggcaac acagtgagat     360

cccatctctc tccatttatt tattttaaaa agagactcag gccgagcgcg gtggctcacg     420

ccagtaatcc cagcactttg ggaggctcac gaggtgagga gttcgagacc agcctgacca     480

acatggtgaa actgcagtct ctactaaaaa taccaaaatt agccagtcgt ggtggcgcgc     540

gcctgtaatc tcagctacgc aggaggccga ggcaggaaaa tcggttgaac ccgggaggcg     600

gaggttgcag tgagccaaga tcgcgccact gcactccagc ctgggcgaca gcccaagact     660

ccgtctcaaa taaacatata aaaagaaagt caaggttatt cattgcctcc cttggagact     720

gtactccccc ccaccatgtc aatcaacaaa tatctgaaca cctatttttt tttttttttt 780

tttttttttt gagacggagt ctcgctctgt cgcccaggcc ggactgcgga ctgcagtggc 840

gcaatctcgg ctcactgcaa gctccgcttc ccgggttcac gccattctcc tgcctcagcc 900

tcccgagtag ctgggactac aggcgcccgc caccgcgccc ggctaatttt ttgtattttt 960

agtagagacg gggtttcacc ttgttagcca ggatggtctc gatctcctga cctcatgatc 1020

cacccgcctc ggcctcccaa agtgctggga ttacaggcgt gagccaccgc gcccggcctc 1080

tgaacaccta ttacatacgg ggatacagct aagaactttt cacagagacc cccgccccta 1140

acaaaacttt acctaaaagg gttgggagca tgtcctactc tagtttcatt ccccgtt 1197

<210> 121

<211> 500

<212> DNA

<213> Homo Sapiens

<220>

<221> variation

<222> (225)..(225)

<223> baySNP11248_C225T

<400> 121
acgcccctgg cctcccaccc tccaacccca aaaagctctg gaagccgggg gagggacgga 60

gcgaagcccc ggctccctga gttgaacagc acttggcgac agccggtgcg cgccctgcca 120

cttcttgctt gctatagtta gaaaaaaata tatatatatg catctacccg gcgcccaata 180

actagccaga cccggctgaa ggaaagctaa ctcccctgac gcgaygccaa gcgaaagaat 240

cagcagtcga gtgagccggt cgctttggta agattttccc tcccttaccc ttcaactcct 300

cccgctgcca ggagagctgc caggtaggac ggcgggcaaa gcgcttcttc aagttcctgc 360

gctggagtca ccacccgaga ggcatcgccg tctggcgttg gtgtctgagc agaagccgca 420

ggaaccagcg ggagcagcag gaggagctgc gggactcgtt tgccgaggct cgtgttctgc 480

tcaaaagttt gtagacgcgc 500

<210> 122

<211> 2955

<212> DNA

<213> Homo Sapiens

<220>

<221> variation

<222> (1110)..(1110)

<223> baySNP11372_A1110G

<400> 122
   tcccaccctg ccctctaaag tgctggatta caaacatgag ccacgacacc cggcctgaat 60
   aaatgctgat ctttatatag aggaaagttc ttcacattct gctcctgatt atcttaccct 120
   gggaggtgag ggaaaatagc aaatgttaat catattaatc atctttaggc actgttttaa 180
   gaagacagaa gagcatttat caacctcccc tcctgagatc aaactacagc catagtcaag 240
   gcacttacag gtataatggc acttaatgtt aatgtgggca aacattggtg gattttgcac 300
   cactaattgc tctaagatgt tataaatagt aactcacaga actcttccaa gaacctagga 360
   agtgtgtacc attggtattt ccattttcag gtaagaaaac tgaaacagat gttcagtgac 420
   ttgcccaagc tcagaatcta ctacgtggta aagactgggt tcccacccag acagacggac 480
   tcaagaactc acgcactgcc tctgcaccct ctgctgccaa tgaaaattta aatgagggca 540
   acaggagatc agagatgcca agagatgttg gaaaacagaa ctggctgaag aaatgacaac 600
   tgaataaaga ggggtgaagg gagctgtgaa ccacagcaat gccaagaggg catccagcca 660
   ggcagagtgg tctactaggc agagcctcag agaggggagg agttcaccag gcccacgtgc 720
   catatgggat tcatgccagc cttgagtcta caccagtgaa aaaaatcaga gagttcatga 780
   ctgaagcaat atgccccaga aataatatct ttgtattctg acacttcagt gggttcccat 840
   tcaatcaccc tacagggaag actgccagta gacctgcccc ccaccccgtt caatatgaat 900
   agatctagga tcagaaattg gagaaaggtg aaagtgggga aacagaccaa gatgaccaaa 960
   tacaaaagga gtccagaaca aacaagtaat tttttaaatc tcaaacccat gtcctcaggg 1020
   agattaaaaa atattttatt cataaactta aaacaagata tatagaaaag aagaaaaatc 1080
   aatgaaacaa acaaaaaaag agattttctr gaaactaaaa ggtgatgatc aaataaacaa 1140
   gtctagagag accatgtgaa agaaagtcac ggaaatcctc cagaaaataa accaaaaaga 1200
   tgacagagga aaaggagcta tatggggaat agaaaagtta ccaggcacag ggtcacaggg 1260
   tgttctaaga aagctacttg agaatgagcc accgggagga agaaagggtg ggtgggtggg 1320
   tgtgtgtgtg tgtgtgtgtg tgtgtgtgtg tatgtgttgt gcatccatgg agggcaggtg 1380
   gggataacca agaaagaaaa agacatggga tccagaaaac aggaaaatca acccagagag 1440
   aagggaatct gaggatcaca gtgggacatg cagtaggcct acaaaacgac cagtccagga 1500
   tggaataaaa agggaatggt tctgtgggaa aaagggactc aatagaatac ctcaaaggat 1560
   tcacagtttg agagaaactg aggaaatgaa gaaggcaagc agtacaaggg aggaaaaaaa 1620
   acaaaggcaa ttagaaacat caggaaaaag taaagctgtg ccacaaaaca tgtgcaaaca 1680
   tggtgtacaa ctcagctcca cagtaaatag tatttaaaac atcacaaaat gtaaacactc 1740
   ttgattgatt tttaactttg agaacagaac agagcatata aatatgagaa ctaaatatac 1800
   ttgttaccga ttagtacaaa cttaaagcac aaaggtagca gttgggatgt gaagcactga 1860
   acgaaggaga aggaggatta atattgtcat cttttaaaga ggggagtcaa gaaatactag 1920
   gtaaaactga gagtataaga aagaaagttg taagtaaatt gtttacagct acaaagttgc 1980
   taatggagga cataaaactg gtgatacaat attaggaaaa ggtggagagg agagatagag 2040
   ggagtgtaca aacaagatga aaatgactaa taatgacact ataagtaata atgataatac 2100
   cgattcaggc ccatcccagt taggggcggc tccaaaatca ccattgttct tttctcacct 2160
   tgtgtctctg cgcactcccc ggcacaccca cttccagcag ctggtctttg tttgcagact 2220
   ttattttaac agctcagtgg ttcccgggca tcctccagct cttatacaaa agccccctcc 2280
   tcaaaagcca ctcgcctccc tctttccact tcccacctct gtttcagggt cgaggcatcc 2340
   agatatctgt cttctggggc ccctacgcag caagactaaa agggggaccc agtcctgggc 2400
   agcagttcac caggggagta tgcagagggg catctggaca cttcttgctg taagggacgg 2460
   ggtgacggga gggggtcttc ccttcccctt cttcctgctt tgttcactct gtgacaccct 2520
   aggccagggg agctgtctca acgccatctc atgagccatg agaaccagga ggaccaggag 2580
   ggagaagagg agcaggaggg cagacagagc tggggcctcg gacctgccca atacttgtga 2640
   ggagctgcat ccaggatccc atcctcctgg gtcattgttg gcctggctcc cacctttgtc 2700
   tggagcctct gctgggggtt gaggctggat actgcccctc aggcttgact aggacacagt 2760
   gtgtactcca tgggtgagcc tgcaggctgg tcaattccgg tgtccaactg agcgacctgg 2820
   aagaggagcc ctccctccca cctcagctct gagccaagct ctccagatgc agaggacagg 2880
   gactctcagc aaagcatctc cctccaagtg gttgtggggc atcctccttg ggcagggaaa 2940
   gaggagtcga aatga 2955

<210> 123

<211> 1004

<212> DNA

<213> Homo Sapiens

<220>

<221> variation

<222> (486)..(486)

<223> baySNP11449_C486G

<400> 123
   taaataaata aaataattct tggaatgttt tttcttctat atcccactat ccttggtcag 60
   atttttttct tatttgtgtt actgtctatt aaatgtattt tctcttaagc tgagacacag 120
   atctcttaag actgatggtt tagaatatag cagaccatat ttttatgctg ctttagactc 180
   ttgtccaggt gaaaaaattt ttaataaatg ttgtgaactt ctgataacaa tctctatttt 240
   gtttagatac atctcttttt ataattaaaa agtagtcaga tactaagcaa aaacagaaga 300
   acaacaacaa aaccccacag ctactaaaag atgaagagac attgaattga gaattaagca 360
   aaacccttgc aggcagcagg acaggccaga gctgaagaag ctgggagtgg tgaggtgccc 420
   ctaggggaca ctgacagagc cttgaaccca ctggtgcagg aggcagggat gagtgaagcc 480
   tgtctstgtc ttcaagatta tggtcccaca gtcctctctt tacctcatac gcattgcctg 540
   attttgaaga gttaaggttt ccttgaagca caggtgcctc aacctctata ccacgaaaga 600
   cacctctaca gattctaaga atcaccttac acatgggtgc catatgtttt acctggggcc 660
   actgttcttt aatatcatcc cttccctcag gtttccatct ggattccaca aaagccaccc 720
   ttcctgaagt tggtccctct caccggattt atccgaagac ctcctctctc attttccgac 780
   cattccttta ggtctgcctc taaacaagag gccaagcttg aaattcaagt gtgaaacaaa 840
   ctcctggcaa aatggcagta tcttagaagg tgtgagatat tcttgccttc caggaatagt 900
   tgcatttaaa aagacagatg aggccaagga gggcagatca cctgaggtca gcagttcgag 960
   accagcctgg tcaacacggt gaaaccccct ctctactaaa aata 1004

<210> 124

<211> 1004

<212> DNA

<213> Homo Sapiens

<220>

<221>  variation

<222>  (489)..(489)

<223>  baySNP11450_A489T

<400>  124
taaataaata aaataattct tggaatgttt tttcttctat atcccactat ccttggtcag      60

atttttttct tatttgtgtt actgtctatt aaatgtattt tctcttaagc tgagacacag     120

atctcttaag actgatggtt tagaatatag cagaccatat ttttatgctg ctttagactc     180

ttgtccaggt gaaaaaattt ttaataaatg ttgtgaactt ctgataacaa tctctatttt     240

gtttagatac atctcttttt ataattaaaa agtagtcaga tactaagcaa aaacagaaga     300

acaacaacaa aaccccacag ctactaaaag atgaagagac attgaattga gaattaagca     360

aaacccttgc aggcagcagg acaggccaga gctgaagaag ctgggagtgg tgaggtgccc     420

ctaggggaca ctgacagagc cttgaaccca ctggtgcagg aggcagggat gagtgaagcc     480

tgtctctgwc ttcaagatta tggtcccaca gtcctctctt tacctcatac gcattgcctg     540

attttgaaga gttaaggttt ccttgaagca caggtgcctc aacctctata ccacgaaaga     600

cacctctaca gattctaaga atcaccttac acatgggtgc catatgtttt acctggggcc     660

actgttcttt aatatcatcc cttccctcag gtttccatct ggattccaca aaagccaccc     720

ttcctgaagt tggtccctct caccggattt atccgaagac ctcctctctc attttccgac     780

cattccttta ggtctgcctc taaacaagag gccaagcttg aaattcaagt gtgaaacaaa     840

ctcctggcaa aatggcagta tcttagaagg tgtgagatat tcttgccttc caggaatagt     900

tgcatttaaa aagacagatg aggccaagga gggcagatca cctgaggtca gcagttcgag 960

accagcctgg tcaacacggt gaaaccccct ctctactaaa aata 1004

<210> 125

<211> 540

<212> DNA

<213> Homo Sapiens

<220>

<221> variation

<222> (511)..(511)

<223> baySNP11470_C511T

<400> 125
ccattcttgt gcgggtccat ttatatatgt tcctgaacag ctaatatttc atttagtttc 60

caattaaact tgctcaaata catttttgaa gtgcattgct taaaatttac attcaaggca 120

agaaaacgag tttgtcagta aatgtagtaa ttaaggaagt cccaagtctg tatttttaag 180

atactagagg taattgtaac taatcacttt aacttaaaga aatagaattg gaaatacccc 240

cctccccccg ccacccccta ccaaaaccat accttagtca ctttggaatt ataaccgtct 300

tttacgtcta tcaatataaa tgtattgcat ttggcattca aattactttc aaagggataa 360

atttcgaatt tttttttttc attgtagctt ctgatcttta aatgtactga acaggaggat 420

ttggggagct tgtcttgtct gataggtgtt aagtatctat gacgacaaat ttgagctgaa 480

gcaatgtgca atatggacta gtaatatctt yacaagacta ttttggaaat tctcacgttg    540

<210>  126

<211>  641

<212>  DNA

<213>  Homo Sapiens

<220>

<221>  variation

<222>  (556)..(556)

<223>  baySNP11472_A556T

<400>  126
aaacacaaaa agtaagactt ttcaagacaa cctcattcac actgtttact tgaattattt     60

actcttgttt tattattaat ttttatgctg ttcaattgac tacaattcaa aacccagtgt    120

attcatacca tgtgctaaga tatgctcctt cctatatgtg ttcctctaaa gaaccatctg    180

gatcttgtgg gggatatttt cctttgtggt atcaaagcca ggagctcgtg gttttccttt    240

attttttaag ggaacactat catgtagcat ctgggaccag aaagaaatct tgatgttata    300

tattttacag atattttctt tttggatttt ttttcctcta agagaaaagc ttttgttaat    360

gtgtcatgta aaaatacatt aactagtaac ttgtcaagaa gtgctagatt ccattcttgt    420

gcgggtccat ttatatatgt tcctgaacag ctaatatttc atttagtttc caattaaact    480

tgctcaaata catttttgaa gtgcattgct taaaatttac attcaaggca agaaaacgag 540

tttgtcagta aatgtwgtaa ttaaggaagt cccaagtctg tatttttaag atactagagg 600

taattgtaac taatcacttt aacttaaaga aatagaattg g 641

<210> 127

<211> 1547

<212> DNA

<213> Homo Sapiens

<220>

<221> variation

<222> (485)..(485)

<223> baySNP11487_A485T

<220>

<221> misc_feature

<222> (848)..(947)

<223> N_region

<400> 127
gccaggcact gtgcaaagca ctttgtatga actagctcat ttaattctca ttcaatcagc 60

atttaatgta taatttttca attttgcaga taaggaaatt gagatacaga tagattttta 120

aaaatttacc caaagccata tagctaataa atggtagtca agatttagaa tcaagtaatt 180

gggtgcttaa caatatgctg tatagcctct tattctgaag agtggttacc accaagaata 240

tccagattgc atctcctaaa atgacagtat ttacttcata gggctggtgt aagaattaca 300

tgagatgtgg caaaaatctt agcagagttc ctgacgtaca gcatgtgctc cacaggtgtc 360

agctggtagt attactattt ttactgtctg ttcaagagag cagctagact gagactagac 420

tcttagtatt gatttcaagt tatctttgaa gggattcaga ttggcaagca caagagtcag 480

acccwatcct gagccctcaa ctgtttgcag gaaggaataa tctcttgtgt cagatgcagc 540

ttgctggggc ttcaccttgt gaattaggga caggggagaa agtgttggaa ggcagcctac 600

catctcaagt gatgcaaatt ataatctatc aaaggaatga atgaacgttg gtctggcaac 660

aaatatcacc atcccatttt atttactaaa cttactaaac cactttagca agttaaaagt 720

agcactgaag gcagatttac atattctgag ctctgaagtg aggcttttct tttatgggct 780

atattgatgg tagctttaaa actacaaata tcagaaaaac taaatttaca gtggattaag 840

gaaagtgnnn nnnnnnnnnn nnnnnnnnnn nnnnnnnnnn nnnnnnnnnn nnnnnnnnnn 900

nnnnnnnnnn nnnnnnnnnn nnnnnnnnnn nnnnnnnnnn nnnnnnncta acccgcttta 960

gcaagtttag agtagccctg aaggcagata cactattgtg agctctgaag gagggctttt 1020

cttttatggg ctatatgatg ggtagcttta aaactacaaa tatcagaaaa aactaaattc 1080

ccagtggatt aaggaaagtg ggtttatttt tttctcccat aaccataagt ctggagacag 1140

ggctggcatc tatgaggatc tcttagccac tttctcaagg ttgcaaggga gacaggtgct 1200

gggaatgact gttagaaggt cagctatgtg agcagataag tatttgactt caaaagaaac 1260

ataacactta gtggaactat gttctttgca gagccctacc taatccattc atctaaaagt 1320

gttgcaacat aggtaggaga atacgttgtc tgggaaacca caaattacag tactatgtgc 1380

atcccctcat aatttcacct taacaatttc gttacagagg aaggtgcatc cagttcctca 1440

tgctcggaaa cctactgtgg actttatcct gagtcagaac cagaagtgaa ggcagtggct 1500

agtttcttga gaagaaatat caaccagatt aaagcataca tcagcat 1547

<210> 128

<211> 1547

<212> DNA

<213> Homo Sapiens

<220>

<221> variation

<222> (533)..(533)

<223> baySNP11488_C533G

<220>

<221> misc_feature

<222> (848)..(947)

<223> Unsure

<400> 128
gccaggcact gtgcaaagca ctttgtatga actagctcat ttaattctca ttcaatcagc 60

atttaatgta taatttttca attttgcaga taaggaaatt gagatacaga tagattttta 120

aaaatttacc caaagccata tagctaataa atggtagtca agatttagaa tcaagtaatt 180

gggtgcttaa caatatgctg tatagcctct tattctgaag agtggttacc accaagaata 240

tccagattgc atctcctaaa atgacagtat ttacttcata gggctggtgt aagaattaca 300

tgagatgtgg caaaaatctt agcagagttc ctgacgtaca gcatgtgctc cacaggtgtc 360

agctggtagt attactattt ttactgtctg ttcaagagag cagctagact gagactagac 420

tcttagtatt gatttcaagt tatctttgaa gggattcaga ttggcaagca caagagtcag 480

acccaatcct gagccctcaa ctgtttgcag gaaggaataa tctcttgtgt casatgcagc 540

ttgctggggc ttcaccttgt gaattaggga caggggagaa agtgttggaa ggcagcctac 600

catctcaagt gatgcaaatt ataatctatc aaaggaatga atgaacgttg gtctggcaac 660

aaatatcacc atcccatttt atttactaaa cttactaaac cactttagca agttaaaagt 720

agcactgaag gcagatttac atattctgag ctctgaagtg aggcttttct tttatgggct 780

atattgatgg tagctttaaa actacaaata tcagaaaaac taaatttaca gtggattaag 840

gaaagtgnnn nnnnnnnnnn nnnnnnnnnn nnnnnnnnnn nnnnnnnnnn nnnnnnnnnn 900

nnnnnnnnnn nnnnnnnnnn nnnnnnnnnn nnnnnnnnnn nnnnnnncta acccgcttta 960

gcaagtttag agtagccctg aaggcagata cactattgtg agctctgaag gagggctttt 1020

cttttatggg ctatatgatg ggtagcttta aaactacaaa tatcagaaaa aactaaattc 1080

ccagtggatt aaggaaagtg gggtttattt tttctcccat aaccataagt ctggagacag 1140

ggctggcatc tatgaggatc tcttagccac tttctcaagg ttgcaaggga gacaggtgct 1200

gggaatgact gttagaaggt cagctatgtg agcagataag tatttgactt caaaagaaac 1260

ataacactta gtggaactat gttctttgca gagccctacc taatccattc atctaaaagt 1320

gttgcaacat aggtaggaga atacgttgtc tgggaaacca caaattacag tactatgtgc 1380

atcccctcat aatttcacct taacaatttc gttacagagg aaggtgcatc cagttcctca 1440

tgctcggaaa cctactgtgg actttatcct gagtcagaac cagaagtgaa ggcagtggct 1500

agtttcttga gaagaaatat caaccagatt aaagcataca tcagcat 1547

<210> 129

<211> 799

<212> DNA

<213> Homo Sapiens

<220>

<221> variation

<222> (171)..(171)

<223> baySNP11493_A171G

<400> 129
   agcaccccct gggactccat tctctcagct tgctgccaaa agccttttgt gttttgtttt 60
   gtatcattat gaagtcatgc gtttaatcac attcgagtgt ttcagtgctt cgcagatgtc 120
   cttgatgctc atattgttcc ctattttgcc agtgggaact cctaaatcaa rttggcttct 180
   aatcaaagct tttaaaccct attggtaaag aatggaaggt ggagaagctc cctgaagtaa 240
   gcaaagactt tcctcttagt cgagccaagt taagaatgtt cttatgttgc ccagtgtgtt 300
   tctgatctga tgcaagcaag aaacactggg cttctagaac caggcaactt gggaactaga 360
   ctcccaagct ggactatggc tctactttca ggccacatgg ctaaagaagg tttcagaaag 420
   aagtggggac agagcagaac tttcaccttc atatatttgt atgatcctaa tgaatgcata 480
   aaatgttaag ttgatggtga tgaaatgtaa atactgtttt taacaactat gatttggaaa 540
   ataaatcaat gctataacta tgttgataaa agatttaaaa acaactggct gtttttttac 600
   actgtggtgt ggaagattgt gttggttcac aacttttcac ttcttcccct gtgtgattac 660
   acacacctgc cccttgtggt gtgacttgca gtgcgcccta caggccacac accccatgcc 720
   ctccaccact ggctctgctg ctggaatgtg agcagaaggg acatctgcct tattcaagca 780
   aagcctttgc ttagcccca 799

<210> 130

<211> 535

<212> DNA

<213> Homo Sapiens

<220>

<221> variation

<222> (455)..(455)

<223> baySNP11502_C455T

<400> 130

ccttggcagc agcaccccca tggcgtctcc tgacctcctt ccagaggacg tgagggagca 60

cctcgaaggc ttgatgctct cgctcttcga gcagagtctg agctgccagg cccagggggg 120

ctgcagtaga cccgccatgg tcctcacaga cccacacacg ccctacgagg aggagcagcg 180

gcagtcagtg cagtctgacc agggctacat ctccaggagc tccccgcagc cccccgaggg 240

actcacggaa atggaggaag aggaggaaga ggagcaggac ccagggaagc cggccctgcc 300

actctctccc gaggacctgg agagcctgag gagcctccag cggcagctgc ttttccgcca 360

gctgcagaag aactcgggct gggacacgat ggggtcagag tcagaggggc ccagtgcatg 420

agggcggctc cccagggacc gcccagatcc cagcyttgag agaggagtgt gtgtgcacgt 480

attcatctgt gtgtacatgt ctgcatgtgt atatgttcgt gtgtgaaatg taggc 535

<210> 131

<211> 630

<212> DNA

<213> Homo Sapiens

<220>

<221> variation

<222> (258)..(258)

<223> baySNP11534_G258T

<400> 131
tgggacctaa ccagggactg ggagagaccg ataggagtgg aggcaggaag gctgtcagtc 60

gggccatcgt ctttccctat aggctcttga gtgtgtggga tgggaggaag gcaagtgctc 120

tgtggtatcc ctgggggaac ctccctgaag agtgccctgg tcacagcacc cttgaagaca 180

gccattggcc atgggtcagt gttgagtttg gttggccctg tgacacgctg gagctctctt 240

tccagtgcgg tatccttkgc cctggtgggt ggagggggtc tcaggcttgt gtgtggatga 300

tgctggagcg aggaagggag ttttgctctg gagaagtgag aaggcctggc tgtctccccg 360

gttgctggat gaagccactt gttgctgggg gaaaccagtc agggctgctt gggcctctgg 420

gcctctgagc ctctgagccc accaggcctc tgcgccccct ggagagagaa ggagggagga 480

gggaggttca gcaactggac tgcactgact cacaggagag ctgggggcag ggtggctgag 540

cctggggaaa ggccgatttc ggaggcgatt aaaaatattt gtacaactga ttccagggaa 600

gtgaagcaaa aggccctttt gtccagaaaa 630

<210> 132

<211> 1854

<212> DNA

<213> Homo Sapiens

<220>

<221> variation

<222> (274)..(274)

<223> baySNP11537_A274G

<400> 132

ccactgcact gggctgtgcc tcctctgccc cgtgtcccct gccagtcctc tcctcacctg 60

tgtagctgct ggaaggaagt gcatgctgtg gtcccccatt gagctctggg gctccttctg 120

cctctgccac ttcctctttc tcctcctctt cccggacctc aggggcttcc tcctgtggct 180

gctccatggc tgatctcccc tcggcgccac acctgcaggc ccagtcatag ctctggcatc 240

gtctgggtct gaacgcagat ggacctctac aggrtggttc ccatcagcct gggcagagga 300

gaagacagga agaggtcagg ggaggcagtg acaaggaaga ggatggtgcg agtggggcac 360

gcttgggggc tgctgtcatg gagggggttc ttctgctcct gctccttccc tagttcctgc 420

agtgctatac catggtctct gctccttgta tcacaagtca cctgccctat cacccttccc 480

acctggctct tgtagtgggt ttgtggggtt cggagcaggc tccataaact agagaggtga 540

cacatcactg tgccaccctt ccctgcctcc tctgctgcct tctgttctga gtctccagga 600

agcacattgg aatgacttga ggggcttttc ccaactctcc agcccacctg gagatccaga 660

tatgcatcac gcctcccatc cccgaccctg tccctatcag cactcaggcc caagaatcac 720

ggctctgctg tgctactgtt cctgaagggg tgagtcccat cctttcagtg gttgggtgtg 780

gaaaaaagac ataaagccct gctgttcttg gctcatgttg acagcagatc tgcaaccatg 840

gcctcaatag cagagacatc agaaaaagga agatgtgctc catcctggag tctttccaag 900

gtgacaactt actgggtggt gatgccacgt ccacatcacc caggaggaga cagttccaac 960

ccttagcaag taaaacatta tccacaaatg ccacagtcag agatgataag atcatgaggt 1020

actgtggaga gtctcccagc ggggaaatgt ctgcaccaga ccttgtggaa ggatcaggag 1080

aagaaaagat ctgctttaag atgttaagca tctgtgggac cccaactctc agatatttta 1140

ccgacagctc aaactcaatc tatccaaaca gaacccatca ttttccccaa acccattcgt 1200

cctctagtaa tctttacttt agcaacccac ctgcctgtct acccactcat cagaaccaga 1260

acctggagtg atcagagact ccttctgctc tctctccctg ctctagacct gccaactcgg 1320

ctgtacactg tagtcactgg ggagttttta aaaagacaga tgcctggtcc catccccaga 1380

gattctcctt tgattggttt ggggagcagc ttggacattg ggtttcttat tttttaattg 1440

tcctggtgat tgtaatgtac agccaagttt gtacaaaaca tgttaaggct acacattcat 1500

ttccaccagg ccttcccttg cttggactta acaataacct cggggcctac ttgagggtgg 1560

aggctgggag gaggagagga gcagaaaaaa taactatcgg gtactagtct tagtacctgg 1620

gtgatgaaat aatctgtata ataaacgcct gtgacacaag ttcacccaca taacaaacct 1680

gcatctgtac cctgaaccta aaataaaaca ccaccacaaa actcttagct gttttctgat 1740

gggttttttt gttgttgttg ttattgtttt ttgagacagt ctcactctgt cgcctaggct 1800

ggagtgcagt ggcgcaatca tggctcactg cagcctttat ctcccgtgct caag 1854

<210> 133

<211> 588

<212> DNA

<213> Homo Sapiens

<220>

<221> variation

<222> (185)..(185)

<223>  baySNP11560_A185G

<400>  133
tgtgggcctc agtttcccta tctgtagtaa gaaggctaaa ctataaggcc aaaagcacga     60

ggctctgatc ttgcaggtag aagagtttca atgaggatga tgacagtggt gatggtggtg    120

ctgccaagcc cccctcctcc aactccttta cccctagggc cttctttctt gaggccagaa    180

cccgractag ggaggggaca ggggaacagg gacaatccca tggctaagta agacaaccca    240

gaaggtgact tggctgtggc tgggtgggat ctcagggtct gggaacgacc cctcaggatg    300

cagtgagaag tatttgccac ttggtcctgt gcacttcaga ggtgccaggc actgtgctca    360

ctaagccctt tcagaccttc ataccctttt atcatgtaaa ctttttcaga ctcacaaagc    420

agatgtataa ttagctccac tttacacttg ataagctaag gctcagagaa gttcagtcat    480

ttgtccagcg ccacacagcc tggaggttgt ggagccaata ctcaaggcgg agccgcttcc    540

ttcctgaaat tatactccag tgggaaggag agaggaacaa cgaggcag                 588

<210>  134

<211>  620

<212>  DNA

<213>  Homo Sapiens

<220>

<221>  variation

<222>  (353)..(353)

<223>  baySNP11578_C353T

<400>  134
cacctatgtc tgtgtttctg tggcatgtta tctaaagagt aaaggtacca tgcctttgcc      60

tttctgggcc ttgaaatctg ttgtactcag acaactccct catctcaaac ctccctgttg     120

cgtgagggca tccgacttgc ctcacagagc caactaatta tcatcggtgg caatcctggg     180

caattatgtc tggctagata tgccataaag ctacaaatta attaccactt acagctggga     240

agatctctct cggcgtgctt ggtaataaca ttggaagaaa cacgtgcgtg ggtccatgca     300

ggctcggcct ctgaagcaag ggcgggggct ggcaagttgt tccatcccac ctyggtctcc     360

acctggggct gtgggcaggc cagggcttgt ttgagattcc tccagagttc ggctccgtgg     420

ctgcctcctc cacggaggag gcacgagagg cagcgttttc cagattgtgg aggacactgt     480

ccctgcgggt gcttgggatg gcttcagaag gtccaaggag gtgagagtga gcttctgggt     540

ccatgctctt ttcctccttg ccatcacctg caggagaagg tctcttggtg ttgagaggac     600

ttgaagagcc ttccacactc                                                 620

<210>  135

<211>  501

<212>  DNA

<213>  Homo Sapiens

<220>

<221>  variation

<222>  (328)..(328)

<223>  baySNP11594_C328T

<400>  135
tgagtgagag ggtggtgtga ggaggcacag gcaggtcctt actaatcaca cagcatcaga      60

gctgccaact cgagtgtcga cagcctttcc tgggcacacc ttcctgaact cactctgaag     120

ggagggaggc tattaggaaa gaaatataag ctgaaaatgc ccccgaaagg tccactcaca     180

gaaaaccaag agctggccac caccctgcca gcagtggtga cgggaatcat gccagtgact     240

caggcagcag ccacagctcc aaagtcctgg agccggcccc gcccaacaca ccggatgcca     300

caaagcacca cctacctgag catactgytc cttgagtgga ccagagagcc ggaggacagc     360

acagacatca gctccaagtc tgcaggacag ggagatcaga atccagttag cctagaattt     420

ccaggagctt ccatgtccca gaaagggagt caagaggcct gtgttgaagt cccatttcta     480

ctactggtat tatgtaactt t                                               501

<210>  136

<211>  1395

<212>  DNA

<213>  Homo Sapiens

<220>

<221>  variation

<222>  (912)..(912)

<223>  baySNP11624_C912T

<400>  136
atgctggtgt gctgcaccca ttaactcgtc atttagcatt aggtgccatt atccaaataa      60

caagtaccag aaacactgat tctttatgct ccaccagatt gaaagcctgg agatggagga     120

ggtaggggag ggtgagaagg ggccacggga ggcctcagaa gggatgagtc ccaagagaag     180

gagcagcacg ggtgacaagg gtcagatggt gggacccccg agagccacag cgcagtggcg     240

gcagagccca gggtgagtgg gatgaggcaa tgagaacaca ctctgttttt ggacccatgt     300

ggcgtgggtg tcagtggaca cggggcaatg cacaccaggc tgtggtgatg agagccagga     360

agaggaggac gtgaagatgt ggggtccccg cttagcaggc agtaacagag gaggaaaagc     420

tacaatactg cctggtatat atacttgaca acacagcatc tgagcctctg gtgaccctgg     480

ggaagacctg atggagctcc ctgtcaggac caaatcctgg agggagggga gggagctgac     540

tgagctccca ctatgcgtca cgctcccact caagcctcgc cagcccccat taaggcctta     600

taggcccccc ttgcagatga caaaagtgac aggcctgagg atcccaacag ggcatctcag     660

aacatgctag aggacgaagc agaaaacggc agagcttcgg acacagcagg actcgagccg     720

ggccaggccc tgacagctgt gtgcactggg ccgtgttact cagcagagcc tcagctccca     780

ctcccacata tgagctggta gtgggttttt ttcaccagaa ccggtaaaac tgttctcagt     840

ttcgggaggt gtaagtaagt ggttgcctct tagactcaga tgcagttctg gactctggct     900

agagttctga ayttgggacc agggtctcta gttcccacgc tgacatctct ccctagcctg 960

gaattgtctc ttctgactgt ggcctctcaa ggacaccagt gaagaggaag gggagctgta 1020

gcctggctgt tctaaaacct acccacgtcc cttgggctgc tcctatttag attgaactgc 1080

tgtgctgcag cccatccctg cattttgcca gaagcctgcc attttcatat ggcctccttg 1140

ctccatctac agctgaggtc tgtcatctac cctgagccaa ggttgctccc tgttctcagc 1200

ttgcccagcc aggagcaacc ttgaccccta ggctctctcc cctgacccac tggacctagg 1260

cccactgtct agattttctc aggtagctgc caccccaccc accacaattc ctgcaatgaa 1320

ccctattctc ctcttccttt cttaagccct ggcactaaag atagcacagg ctgggctggg 1380

catggctcat gcctg 1395

<210> 137

<211> 572

<212> DNA

<213> Homo Sapiens

<220>

<221> variation

<222> (277)..(277)

<223> baySNP11627_C277T

<400> 137
tcacacatca cttaatcgac ccagtgagat gagtgaaggc agggctattt catttatcac 60

tacaccccct actctgaggg aagctctgac tggctcttgc tcgcctgtga atgctctagg    120

tgaggggacg aaggctggtg gtaaggagaa gggcagccac tgagtgggtg gccagcaggc    180

cccatgagag gcaatgaaac tctgactggc ccaggagaaa tgggacgtgc aattgcagcg    240

gggaggcagg aatagggatc tgcagggcag agagacytct caaccttccc agggagagag    300

gagacatgga aaaatcccac agttaagctg tgattggtcg gtctgtccag gagaacccag    360

ggatctcaag ttcaagtctc acctaaaagc gttcacgttc tttgacccta tacttcccct    420

tataggaaaa cactgagctg ttcactgcaa tgttatttat aataacaaaa aatcagaaat    480

ggcccaatgc ccaataatag aggaatggtt aaatatatta gggcacaccc agacaactga    540

gtattaccta gtcattaaac tcatgttttc at                                  572

<210>  138

<211>  558

<212>  DNA

<213>  Homo Sapiens

<220>

<221>  variation

<222>  (419)..(419)

<223>  baySNP11644_A419G

<400>  138

tgtactgcct tgcaacactg tgccattttc tgcataaacc tgtgtcactt taggttcttc 60

tgttagaatg ttctggattc gaagcacctg tggaagaaat aacattgggt taatattttt 120

gttaagtcac ttaaaacaac cttatgcata atagggtcat cctccataag aagccatata 180

atcttataca atgagataaa tcctattttg ttccaaaatt acacttaaca cacctgtttc 240

ttcatttggg aacaggtggg tactgatctc aagtgatctt tagcagaggt ggagagtatc 300

tagacctaca gggatgaggc aggaaatgca aatgagcagc gcatcctgga tgtgggacat 360

tagggagcag tggggatttc agtaaatgtg gagggaggaa gctgttactc agtatgatrg 420

ttaatactga gagtcaactt gattggattg aaggatgcaa agtattgatc ctgggtgtct 480

gtgagcgtgt tgccaaagga gattaacatt tgagtcagtg ggctgcaaaa ggcagaccca 540

cccttaatct gggtgggc 558

<210> 139

<211> 597

<212> DNA

<213> Homo Sapiens

<220>

<221> variation

<222> (146)..(146)

<223> baySNP11650_A146G

<400>  139
agcagtgccc cgggaggatt ccgatggaag tggtgtgggg tgggggtggg gggcttccac      60

tgtctgtttg ggtcttcgta tatccgtccc ctcggagcct ccctccaccc ctggtacctg     120

cctcacgctg gccatgctct gggggrcttt tagacatttg gccagggggt ggcagaacta     180

gatcaacccg taccaggtct taaccacgcc cacctaggga catcgtggga atgtgcctgc     240

tccagtggac agagaacaac gtccaactct gtcttgggga tgttctgagc tggtctcaga     300

gaaaatagac gcagctactg agcgcacatc ctgagggcag cagacaggac tccccagcag     360

ggagctgggc cctctcccca ttctctcctg cggtctgagc ccttaggggt ctgtcctggc     420

cttgtggtgt ggtaaggtaa gacaccttac ccacagagtg acagcatcat ccctcctttc     480

tcccctctcc tgctacggag tgactgagac atgaattggc gatctttaac atgtgctaat     540

gagtgttagc tgaaccacct tccaagagga gagtgaaagg caaggaggta agtgaga        597

<210>  140

<211>  501

<212>  DNA

<213>  Homo Sapiens

<220>

<221>  variation

<222>  (402)..(402)

<223>  baySNP11654_A402G

<400>  140
ggggattgga aatgctgctc aagagaggga aaataggata tcacgattgg ctcaacaatt      60

ccctgtgtta agagcggtcc ttttctattt ggtaggttgt ggggcaaata catagctagc     120

tcaggtgatg aaatctttca tctctttagt ttgtgtactt ttaaccaagg actgcgtatc     180

tcttgccttt cttgtggttt tcacctgcaa cttaataatt ataccattgt acaccttatc     240

ctcttttccc atattcaaga aagattatct ccaactctta ctgttttaaa actcatgtgc     300

ttacttcaaa tatttctgca ctaaagctgt aatattacaa gtttgttttc ctattagagg     360

tttccatttc catccattta tcttttactt gaaaggacac crtatttttc acctaatccc     420

tcaattttat tggtggggaa ggcataagag aagttcactg catacaatct aaggtctaag     480

atcccccaaa atttccagca g                                               501

<210>  141

<211>  501

<212>  DNA

<213>  Homo Sapiens

<220>

<221>  variation

<222>  (331)..(331)

<223>  baySNP11655_A331C

<400>  141
ggggattgga aatgctgctc aagagaggga aaataggata tcacgattgg ctcaacaatt      60

ccctgtgtta agagcggtcc ttttctattt ggtaggttgt ggggcaaata catagctagc     120

tcaggtgatg aaatctttca tctctttagt ttgtgtactt ttaaccaagg actgcgtatc     180

tcttgccttt cttgtggttt tcacctgcaa cttaataatt ataccattgt acaccttatc     240

ctcttttccc atattcaaga aagattatct ccaactctta ctgttttaaa actcatgtgc     300

ttacttcaaa tatttctgca ctaaagctgt matattacaa gtttgttttc ctattagagg     360

tttccatttc catccattta tcttttactt gaaaggacac cgtatttttc acctaatccc     420

tcaattttat tggtggggaa ggcataagag aagttcactg catacaatct aaggtctaag     480

atcccccaaa atttccagca g                                               501

<210>  142

<211>  1224

<212>  DNA

<213>  Homo Sapiens

<220>

<221>  variation

<222>  (897)..(897)

<223>  baySNP11656_C897T

<400> 142

tgatctcgaa ctcctgacct caggtgatcc acccgcctca gccccgcaaa gcgctgcgat 60

tacaggcatg agccaccgtg cccggccgaa atgggaccat ttttattatc ccgtcatgac 120

tctctctgag ataagacctt ctcttggcct tgtaagtttt attttaacag tgacaaaaca 180

caggaatata aaaggaccag tcttgttggc aacatgtggt ttcaaggagt ctgacttttc 240

ccggcctggg tctctggaac tgctgcagac tctaagagcc cactccccag aacgaccctc 300

catgccctgc gtcctgatga ctttgactcc tgaccctcaa ggttgtccag tcttggctgg 360

cttggctctt catgagaact tcaattcttt atttttagtt gtattttttc tacccatgtg 420

atggaaaata attcccgtcc aatgatgggg atggaagggt tgaggaggtg ggtaggagga 480

agaactttcc aaagaaaaac aaacggaatt gattttccaa ggtctgtgcc tacatgggtg 540

tttttccctc taaaaacata gcaggctgag ttgattgact ttaattatat tgacttacag 600

ttcttgattg ttgttactct ttccctggca tctttgggag aaattatagc ttgcttttac 660

tctgaaataa agatgtttcc ctttttgcat ggtctggtct aaatgcttaa tcatatggga 720

agaaaaagag aagccagtgg attggcctta tggaaatgaa cgttagagga taaacagtta 780

ggggcttgat gaaatgttca aaggcttttc ttgctgattt tcatgattgt ctttattcac 840

gctgttgtca tcctgccagc ccaagtacag ctgcgtctgt gatgctgggt ggatgtyttc 900

acccaacagc cctgcctgca cgctggacag agacgagtgc agcttccagc ccgggccttg 960

ctccacactt gtgcagtgtt tcaacactca aggctctttc tactgtgggg cctgtccaac 1020

aggtacattc tcagggccac aagccaggct ctgcatgcca ggttggcgag gtgccagaac 1080

caatttcctt tcactattga tgagtaagaa gacaattctt aggaggtgtt caaatccagt 1140

tttgtgtcct ggttctgctc gacgatctca ctgctatcac aattccacgt caaaaggggg 1200

aggcttgtcc aaaactgtgc taga 1224

<210> 143

<211> 608

<212> DNA

<213> Homo Sapiens

<220>

<221> variation

<222> (277)..(277)

<223> baySNP11825_A277G

<400> 143
tgtgtgttct catgtattga gatcattact acggctatca ggaaggtttt ttgtagtttc 60

atattttgct tgagaaatac taagataaag cctaaaatgt ttatgtcata tatgtcataa 120

atataacacc aattgtggaa tgcaccacaa tgtcagcatt gtcctctgac ttgacatgaa 180

cttaaacttg taatgtgacc ttggaaataa tggaggtcaa tatataaata ttcctacaaa 240

tcattaagga aaatgctaac aacccagcaa aaatggrcaa aagatataaa taaaaagttc 300

acaagaaaga aaatgcaaat ggttcttaat gtcttaaaac aatctcaata tcactcagag 360

tacaaataca aattaaaact actataagat acaattttca cttatcaggc tggcaaaggc 420

aattttcgtc cctgtctatt caaagtaaca ccattttctt tattagttcc agtgggcaag 480

taagagcaag gagtccacat agcgttggcc aaccaaagtg atgaaaggaa ggcacatttt 540

taaaaattca tagatccaaa tccacgaaaa aaattctcag tgtattaggc catagaattt 600

attaaaaa 608

<210> 144

<211> 574

<212> DNA

<213> Homo Sapiens

<220>

<221> variation

<222> (246)..(246)

<223> baySNP11914_A246T

<400> 144
agaaaaacta aaatagactt ttgaatcaag acacttgagc ttaaaaaatg tcatttctaa 60

ctaaaaaaag tgacctccat tgcatgaagg tttctgattt attgtattca taaaatagtg 120

tagtaagtat atgaatataa cttattttaa atgtgatgct gaggagtggg gatggggaag 180

cattttttat tggaagtgag ataagatagg tgattaaatg ttccaaagat aattaaaatt 240

tttttwcctc tgatgtctgt tttctttcat ttacttgtgt cagcatagtt tcccgatgct 300

acacattatc tttcttcatt tttaaagagt tttttctggc atttttacaa ggcgttacta 360

gttgttgcct tgttaacctc tcattctcac cgtgattctg acattgagat gttcaaccag 420

ttgaggaatc tttcccagag cttataaatt agtgtgttgc caagaataat gtatgcgtac 480

attaaaatca tttatcctcc ctttattaag gttactgttt aaaaaggtca aggccctgct 540

gggagggaat gtccgcatga tgctgtctgg aggg 574

<210> 145

<211> 569

<212> DNA

<213> Homo Sapiens

<220>

<221> variation

<222> (449)..(449)

<223> baySNP12008_C449T

<400> 145
ttgttccctc caaccaaaga agttttacca caggtgcccg accaagccct cacgccccat 60

gccagtactt ccagcctctg ccttttagta tgctgctttg ccctgagatt cagagggggt 120

actgaagact tggacatgga ggaaatacct agttgttggt aaagtatcta ttggtgttag 180

aattattact taaggaacta atttgggaag cagaagctct gcccaggaag cagcatagat 240

ataagcacat tacatcatcc tgcggtacgg tgtctttcat accggtgatc attgtggtcg 300

gggcgaaaag aatcatctta actgagaatt ataaagaacc gaagaccaag acgcaggact 360

ttttaaagca gagcatcctc cacacagttc tctccccagg agaagtctgc tgtaaaacat 420

cagtcataaa caagaaattc tgtttatayc atagagcttt attttccata aaaaaatttc 480

cagatattcc attttcattt taaattcaga gccttgtttt tatggaagac attttcactg 540

caattttgga tataatgtct gtatgacaa 569

<210> 146

<211> 1619

<212> DNA

<213> Homo Sapiens

<220>

<221> variation

<222> (411)..(411)

<223> baySNP12097_A411G

<400> 146
tgcatgggag ccttgaaaga tatgatactc aaagaatggc ctgatgattg aagcttaaat 60

agcattctga gctataaaaa tgtagtatct tctgatgggg agtgggtgac acaagacatg 120

agagagtgag ggaagaaaaa gcatgataaa caaaagtcat cttattataa tgtctgtcag 180

gcatcagtgc tccaaagaac aagtattatc aaccttagtc tccctttctg tgagctaata 240

ttattttttt cttttttttt tcttttagac taagttttgc tcttgttgtt caggctagag 300

tgcaatggcg caatcttggc tcactgcaac ctctgcctcc agggttcagg cgattctcct 360

gcctcagcct cccgagtagc tgggattgca ggcacctgcc accacaccca rctaattttt 420

gtatatttag tagagacagg gtttcatcat gttgaccagg ctggtcttga acttctgacc 480

tcacgtgatc cacccacctc ggctttccaa agtgctggaa ttacaggtat gagccaccat 540

gcctggccct gctaagattc tttttcatga gattataggg agggggctca agaccattgc 600

ttttttttag gaggatcttc ctttaggtat gagaacttca gagaaagccc cttcctatat 660

tttgggagaa atagcagggt tgggagacag gagaacataa gagttagctt ggttctgaag 720

actttctgag acctttcaat ttcctttggt tcaaagtcct caacatgctt aagtgccata 780

ttttggggtt tcatttttgg agccccaact ttcccctgtc ataaacttcc ctagaaaatg 840

tacatactaa agctgagttg atgcttgtgg agaggacaat tgaattggta gctgagtcgc 900

aaggaatcac attaaaccag tataaaccag tctcacattc ctagcaatgt caatccagtt 960

aaacagctgt gcctcatttc aggaggtgat gttgcaggtc tgctgtcatg aaagataggt 1020

ctgtatatgg tgcagttaaa caggtactta ataagaggtc attctaccaa agcaaaagga 1080

gaccaaaagt taatcattta gcagactagt ttctgagttc acagggcagc cagacaaaaa 1140

gatttctaca tgttggtttt gaaacagctc cagttggagt agaggtaggc aatggtgatc 1200

tgacagattt tttttctctc ctatagctcc ttagggccag atctgaaaga ctttaagttg 1260

ttgcaatttc tctggagtct gctctatcac aaagaattct gcaaaaatca catttcctgc 1320

ctttgtggac caaaccaatg tatttcttaa atgtatttga tatctcatgc ctccctaaaa 1380

gaataaaacc aagctgcacc ctgaccacct tgggtacatg ttcttaagac ctcctgaagg 1440

ctgtgtcatg ggccatggtc actcatattt ggcccagaat aaatctcttc aagtattgta 1500

cagagtttgg ctctttttgt tggcagcatg tattaactca ttctctatta caattcccct 1560

gtcttgatga atgggctctg ttgaggcagg gggcaaggtg aatctcttgg gtggttaca    1619

<210>  147

<211>  482

<212>  DNA

<213>  Homo Sapiens

<220>

<221>  variation

<222>  (412)..(412)

<223>  baySNP12366_A412G

<400>  147
ctccccaacc cggccaggac cccttggcct cactggaacg cacagacggc acccagcagg      60

taggcatgtt ctcaggggtg gccagcaagg gctctctcag ggtacagctg ggagaagcgg     120

ccgagggccc agatggggaa gatgttcctg tagctcgtgt aggagatggc acaggacttg     180

ttgaagaccc cagcaatgtt ttcctaaaag aacacagaga aataaacaca aaggcttcac     240

cagtcagctg agggctagtc cctgaactgg agccctggct gttggcttgg ctggcttgtg     300

ggtgagtcag cctggaaagg gctaattaag caagagcata ggaaagcaag tagtccatcc     360

cgcatcagtc atggcaccat cagcccagct cagactcccc gccctgcagc araccctaag     420

ccagggtcac cagcccctca caaccacatc agcttccacc aactcagcct catcccaggg     480

ga                                                                    482

<210>  148

<211>  624

<212>  DNA

<213>  Homo Sapiens

<220>

<221>  variation

<222>  (25)..(25)

<223>  baySNP12619_A25G

<400>  148
tcttgtttgt gtcacagtgc tctgrgggca gaagagactc ctggggactg aatacagatg      60

gcttctgaat ttgtttacat tttctatcag aactgacatt tacttctggt cacacgatgt     120

ttttcttcta tcacctttac cctcttttgt gttctctttt ctgtctttta tcctttatca     180

gtcaaagtcc agccagaaaa acagaaacca tgctaaatat ttcaaacaca gggggactac     240

tagatagaac atgttacaaa agcactggga gggctgagag agccacaacg aggagagggg     300

aggaagatga gctaccacag agattggaaa ttccaggaag ctgtgatttt ccctaggact     360

gaaggaacca aacaaggggg ttctagagcc cagtagcaca gtggtggctg accctgctgg     420

aggctgcagc tgctattcaa agaaatcact gctgcactgt agatagcaac aagaggccat     480

tggaagctgc agctacctgc tgctgctgct gctgctgctg ctgctgctgc tgctgctgct     540

gctgctgctg ctgctgctgg gtctagagcc atctctgggc cctgtaggta ttggccactt 600

ctggccatca cccacagcag ccac 624

<210> 149

<211> 648

<212> DNA

<213> Homo Sapiens

<220>

<221> variation

<222> (585)..(585)

<223> baySNP13025_A585C

<400> 149
gtggaaaaat taaaaaagga atggaatgtt atttgtatta ctgatgttgt ctacaatcat 60

actggtatga gcttcattga ctgccttcat taattttgat gagaatttat gcacacacac 120

atatacacac aaataagaga aaggaagaaa gaaagagaaa ggaaaggggc taacataaat 180

tttaaaaaca caaatgatga ttaggtgtcc ttctgtttaa aaaattaata tttttccttt 240

caaagaaaac attcttaatt gaggattaag tcaatatata agtatcttgc atctgttgct 300

tataaataca ggagagtgat catagaagga acataatgat cacatatttc aacttacagt 360

acagtagtcc ccccttattc gcagttttgc tttctgaggt ttcagttacc tgcagtcaat 420

cacatttcaa aaatattgaa tattccagaa atcagcaatt cataagtttt aattttcatg 480

ctgttttaag taacatgaca aactctcatg ccatcctgct ctatcccacc tggaatgtga 540

ctcatccttt tgtccagtgc atccatgctg taaatactac ctgtmcacca gttacttaat 600

agcctgctca gttatcagat aaaaaaacac aaaatatcaa aatatata 648

<210> 150

<211> 625

<212> DNA

<213> Homo Sapiens

<220>

<221> variation

<222> (504)..(504)

<223> baySNP13191_A504G

<400> 150
aagctgggaa ttacgtctca aagtaggaac tattggcaaa aggatgggat gaagatttca 60

atggaggaag gctatgttta ctgtaggaaa atgttgtact cttataataa aagtcttaat 120

agacttttat taaggcctta agtgctagat tcaagatggc tgcccctctt gttctgtggg 180

tccagtgttc tatttggtgg actaagggtg accttgcagc cccttacagc ccagccaaga 240

gagcttcact gtgaaggggc agacatcttc attactattt tctcttccaa aaactcatat 300

aactctttgt gagtactgcc tcttctcctc attccacagt gaatttctcc ccacctggtg 360

acacaaacag ccttttccca ggtacttggt acctggagcg agtggacgag cagcatcgcc 420

gaaagtatgc ccggcgtccc gtctaaaggt ggtgagtgag agtttgcaga gttggtggca 480

taaaacccta atgtcttcct ctgrgtaaca acacagagag agaaggtggg gacaggtgca 540

gggagaagaa agtttaatgg aagaggattg gggtgacagg agaaatggga gaattatctg 600

tggaattttt aaaaggaaaa gcaag 625

<210> 151

<211> 581

<212> DNA

<213> Homo Sapiens

<220>

<221> variation

<222> (47)..(47)

<223> baySNP13937_A47C

<400> 151
cctccttgta atacatcatg cacttaatga cagctcccat tggaagmcgc tgaattaact 60

ggtttctctc tgctggaagc tctggtctga agtgaatctt ggcagtcaag gtcggaggga 120

tcgcattaat tacgtatttg cactaaaaca cacacaagca aaatattacc tcaaatgtga 180

gctgcagtct ttgtggcagg cgtgaaaaat catctttatc acttctaaac aacattgagc 240

agtaaaaaca tacaaaatga gaaaatgagg caactgtgag gcaacaaaac agatcaaggt 300

aggtcaacca aggctggtaa tttggaagac tgaggcggaa acatgtcagc attaattatg 360

ctactgcttg aattatatct ggttagaaac tgcaaaagac acaaggccta tttactatat 420

gctccttttg actaaactga gttacctcat aatgttcatg gttcagcgtc tctatgatga 480

tgttgtcact tgactggtca acgtgagtga caggatggtt cagcttcact tggtctccga 540

ggaggtccat tatccgttcg ctcacttgac cagatccacc t 581

<210> 152

<211> 751

<212> DNA

<213> Homo Sapiens

<220>

<221> variation

<222> (371)..(371)

<223> baySNP900002_G371T

<400> 152
   tctcaggagc tgaggcagga gaattgctcg aacctgggag tcagaggctg tagtgagcaa 60
   agattgcacc actgcactcc agcctgggtg acagagtgat aaaaaccaga ttcctagatc 120
   cctctttgaa tattcgcctt aagtttctct aggccgaagc ctggaatccg tagcagtttg 180
   accagcaacc cttgggaatt tttatagaaa taggaaccac tgccttagaa cattgggcag 240
   ggataagaga aaagtgtgga gattttagac catcttcctt ttttaacatc tgacttttgt 300
   acaacccaca cccatgttca ttacccaccg acactgctaa gctgctttca cacagcagtt 360
   cacgcagacc kgcagaacat accgcattct gtgattcatc agggtgacac aactgctctg 420
   taagcaggtc ttgtgcagtt aagtgaaaat gatgtatttt atccttgata ttttaaaatg 480
   actgaaaaat gggagacaac caaaaggagt atctcccgat gataagaccc ctaagtcctt 540
   aaccccactg gacaaacagg ggtatcagta ggtaggtgaa cagtgatgaa gtgatccctt 600
   caatcatact tattggcaaa cttaagctga ttaaaattgg gaagggaaaa aaataagccc 660
   tggaggaaaa gttccacatg tatttttgag atatatttaa aggggagggg gactgatatt 720
   agggggattt taactttttg aaagggactt g 751

<210> 153

<211> 511

<212> DNA

<213> Homo Sapiens

<220>

<221> variation

<222> (322)..(322)

<223> baySNP900025_G322T

<400> 153

ggacttgctt gtaaatatgt taatatttga tcttaataag ctctgtcata ctaacactgt 60

ttattaaact ttgtggtaga attactaccc ataactaaag aatgaaatgc atatatttgc 120

aaattgttaa aaatttataa atcgatagaa aagttgaatg aatagtgaac acccttgtgt 180

ctttcactaa ctcagtggct cacttgctaa cgtttgcctc atttgctttc tctgtttata 240

aagacatata tacttttttt attcccctga gccatttgaa agtaagctgc aaacatcttg 300

acatttaact tctaaacatt tkagcacgtg tatcctaaga gcaaggttgt cttcctgaag 360

agttacatta tcatacttaa gagatatagt attaatacaa tattatctaa catgtgatcc 420

ctatttaaat ttcatttatt ttcttcccca gaacagttct ttatagatgt tttaattttt 480

gatccagaaa ccaagggaaa aaatatatct a 511

<210> 154

<211> 598

<212> DNA

<213> Homo Sapiens

<220>

<221> variation

<222> (261)..(261)

<223> baySNP900032_C261T

<400> 154
tccctgtgca ccctggtgga gtgcctttac ttcgtcagca tgtacggaag cgtcttcacc 60

atctgcttca tcagcatgga ccggttcttg gccatccgtt acccgctact ggtgagccac 120

ctccggtccc ccaggaagat ctttgggatc tgctgcacca tctgggtcct ggtgtggacc 180

ggaagcatcc ctatctacag tttccatggg aaagtggaaa aatacatgtg cttccacaac 240

atgtctgatg atacctggag ygccaaggtc ttcttcccgc tggaggtgtt tggcttcctc 300

cttcccatgg gcatcatggg cttctgctgc tccaggagca tccacatcct gctgggccgc 360

cgagaccaca cccaggactg ggtgcagcag aaagcctgca tctacagcat cgcagccagc 420

ctggctgtct tcgtggtctc cttcctccca gtccacctgg ggttcttcct gcagttcctg 480

gtgagaaaca gctttatcgt agagtgcaga gccaagcaga gcatcagctt cttcttgcaa 540

ttgtccatgt gtttctccaa cgtcaactgc tgcctggatg ttttctgcta ctactttg 598

<210> 155

<211> 1086

<212> DNA

<213> Homo Sapiens

<220>

<221> variation

<222> (116)..(116)

<223> baySNP900045_C116T

<220>

<221>  misc_feature

<222>  (986)..(1085)

<223>  N_region

<220>

<221>  misc_feature

<222>  (986)..(1085)

<223>  Unsure

<400>  155
tggccatctc ctgatccaag tccagggtga cgtagcacag cttctccttg atgccatgca      60

cgatttccca ctgggcggtg gtggtgaagc tgtagcggcg ctccacgagg atcttyatga     120

ggtaatcagt caggtcctgg tcagccaggt ccagaaacag ggtggcctgg aggagggcct     180

acccctcgta gatgggcaca gtgtgggtga ccctgtcgct ggagtccatc acgatgtggc     240

cagaagcgta cagggacagc atggcctgga tggccatgta catggctggg gtgttgaagg     300

tctcaaacat gatatgggtc atcttctcgt gattggcctt ggagttcagg ggggcctcag     360

tcggcactac gggtgctcct tggagccaca cgcggctcgt tgtagaagtt gtggtgccag     420

atcttctcca tgtcatccca gttggtgatg atgctgtgct ctatggggta cttcagggtc     480

aggatacctc tcttgctctg ggcctcatca ccctcgtagg agtccttttg acccatgccc     540

accatcacac agatgtggaa gggcagcgga gctctgtgct cgcggggtgg acgcggtctt     600

agcagtctgt tctacattct taaccttttt tttttatgga cctcttggct ttctggtaga 660

gtctgggacc cccttttcaa agtaatgctt ctatgtgcat aaaacaaata catgggatta 720

caaggcaaac caactatatt gaaatacagt tgcctccttg gatcctatgg aggtccaagt 780

actcaagtga aaaatttcca taagagctgg gtgcgttggc tcacacctgt aatcctagca 840

ttttgggagg ccaaggcagg cgtactgcct gagttcagga gtctgagaac agctcaggca 900

acacagtgaa accctgtctc tactaaaata caaaaaatta gcttggcatg gcagcatgcg 960

cctgtagtct cagctacttg ggaggnnnnn nnnnnnnnnn nnnnnnnnnn nnnnnnnnnn 1020

nnnnnnnnnn nnnnnnnnnn nnnnnnnnnn nnnnnnnnnn nnnnnnnnnn nnnnnnnnnn 1080

nnnnnc 1086

<210> 156

<211> 1864

<212> DNA

<213> Homo Sapiens

<220>

<221> variation

<222> (877)..(877)

<223> baysNP900065_A877C

<400> 156
tagatgacgg gttgataggt gcagcaaacc accatggcac atgtatacct atgtaacaaa 60

cctacgcatt ctacacacat atcccaaaac ttataagtaa aacagaaaaa aaaagaatta 120

gttttataaa aatcaataaa ttatactcag tgggtttttt tttttttttt tgagacggag 180

tctcgctctg tcgcccaggc tggagtgcag tggcgggatc tcggctcact gcaagctccg 240

cctcccgggt tcacgccatt ctcctgcctc agcctcccaa gtagctggga ctacaggcgc 300

ccgccactac gcccggctaa ttttttgtat ttttagtaga gacggggttt caccgtttta 360

gccgggatgg tctcgatctc ctgacctcgt gatccgcccg cctcggcctc ccaaagtgct 420

gggattacag gcgtgagcca ccgcgcccgg ccctcagtgg gttttaataa aatattttag 480

ggccttttta gcttatttat ttaaaatttc aagatcatat agtcttgaag tgaaccaaaa 540

aaatcagaaa gagttaccca ctttacctat ggtatgtgag gcaagctttg aatgagtaaa 600

taaggagtga atctgtgtaa tcgtcagctt ccattttggt ataattctgc ttcttattcc 660

caatctgggt ttacgtttta tctttgcaat tatattcatt ctttatttat ttttatttat 720

tttttttgag atggcatctc acctctgtca cttaagctgg agtgcagtgg tgcaatctca 780

gttcactgca acctccacct cccgggttca agcgattctc ctgcctcagc ctcctgagta 840

gctaggatca caggtgcgtg ccaccatgcc cagctamttt ttgtattttt agtagagatg 900

gggtttcgcc atgttggcca ggctggtctc aaactccgga cctcaggtga tccacccgct 960

tcagcctccc aaagtgctgg gactacaggt gtgagccacc gtgccaggcc aactttctta 1020

aatctcgaac aaagcattta cttactagtt agtggaaaca agattcacat tgcatcatag 1080

gtattccaga tactttcagt gatgtggaaa tcagaaaccc ataaaattaa gtatgccaga 1140

aagcagtttt gtacacatct aagtaaattc tctcttaaac aagagaaagg aataaataga 1200

taccaataaa catgcatctt tatctcaata atattttaat gattatttca atgatattta 1260

aaaaatcatc atgacttgat acaattgttg gtacacaaaa tgacgccact atggtcttca 1320

tggaaagtgt gacctcatta acgactgaac aatctgacag attcattcaa atatgtctaa 1380

cagaccctgg gtgagtctgg aagctttggc agtgaaacca gaaggactca gcctgtgagg 1440

gaaggttggt ttgtctccaa ctcgaactgg gaaatacctt ctaataagat gacactgttc 1500

ttgggaattc cagaatcatg aaaaggcagt ggaaatcact gggtgtagga atcagcagct 1560

gtgggcttct ggaaataacg tcctggcact tacacgacct tgttccaatt cagagttaag 1620

gctgctctac actgtgtgca ttcgtgtccg taaagaaagt agcaaaataa aaatcatgac 1680

tggtttccat tttaggcctc actctttgca gtgtccttct tcattcccaa atcttccatg 1740

ttgggaaaat taaggtaaca agaaaatctc ctctgttctt tttccactaa atccaaaatc 1800

atgacacaga actctgttga aatacatcat aaaggtagaa ttttaaaaca aaaggctgaa 1860

taaa 1864

<210> 157

<211> 535

<212> DNA

<213> Homo Sapiens

<220>

<221> variation

<222> (375)..(375)

<223> baySNP900082_A375G

<400>  157
gcaaacatgt tccaccagat catcagtcag aggatctgaa cttgatcccc cttttttgta     60

tttactggag ctggaggtac tagagcagca ctgtatctct tgtgtctggc attgttcaat    120

ccagatgtta gttgggacag aaagaataac ccagagccct ggaacaaact gggtcccaat    180

gatctataca agttctactc agtgaatgtg gattacagcc aactgaagaa agaaggtcca    240

gatttctaaa tgaaatgttt cactataaag ctgctttaga atgaaggtct tccagaagca    300

atccacacaa ttttccactt aaccaggaaa tatttctcct ctaaatgcat gaaatcatgt    360

tgatgtaatc tactrgggag attacactga ttaataaata actgaaactt gaaaaaaaaa    420

gttattatcc aacttgatag aaaactggag tctggaaatc tgaaatatca ccttcctctt    480

ttcatgcggc tcttcttcct gcttgtcaat ggaattcact ttccgtggta ccatg         535

<210>  158

<211>  708

<212>  DNA

<213>  Homo Sapiens

<220>

<221>  variation

<222>  (136)..(136)

<223>  baySNP900096_A136G

<400>  158
agtggccctg tgctcgtcac aggctggtgt cctctgctct cctcccctca ctcctggggc      60

aagcaacagt ggtgttccat gtgtgagctg gacctaccac tagtgttggc ttgtttaaat     120

tctctgacag agacaractg tcccgggggg cggggaaggc agtgtggagc tcaccccctg     180

agggagcagc attgtctctg tgggctttgg ctgggaatgt ttctgaagac gccctaatcc     240

cttgccctgc ctagcctcaa agtctctatc actcagagga gtactgggag ggctcagtgt     300

gagccatcag aaccttccag ggtatcttcc cttgcttgtt ctctttgcgc agtccacttg     360

gtttgctaaa ctcctgctct tccatcaaga cccaactcaa ggccaggcac ggtggctcac     420

gcctgtaatc ccagcactct gggaggcaga ggtgggccga tcacttgagg tcaggagttc     480

gagaccagcc tgggcaacat ggtgaaacac catctctact aaaaacacaa aaattagcca     540

ggtgtggtgg caggcacctg cagtcccagc tactccggag ctgaggcagg agaattgctc     600

gaacctggga ggcaggggtt gcagtgagcc gacatggcgc cactgcactc cagtctgggc     660

gacagagtga gaccctatct caaaaaaaaa aaaaaaaaaa aaaagacc                  708

<210>  159

<211>  790

<212>  DNA

<213>  Homo Sapiens

<220>

<221>  variation

<222>  (426)..(426)

<223>  baySNP900107_C426T

<400>  159
cccacatgtg gctcccgaga tgggagctgc tccttgcata ccctgtgccc tccggagggt      60

gcacctggag tgcttcctca cacatggctt tctcccctcc ccgcccttcc cctcccttcg     120

gtctgtttct cttcccatgg tctaagcatt tattttatgc cattctttat gtaacaacca     180

ccccagaagg cggatgtgaa cacttggatt tgggattttc aaattcctta tctttcactg     240

agtcattttc cattttattc aagagcacat ggagaaacca agggatatct taatggaagg     300

agacctttcc tgggtagaac aggaagactg aagatgaagg attcctctgg gatataaatg     360

gctcccttga gactaattac aaggcctttg ggtgggccac cagcccacac tctgaacctg     420

ggggayaggt caagctgagg ccctgagacg ttacccagtg ggtcagtggt ggcacgtggg     480

tgaaggctga tgggtaactc tacctgaaaa agcccttgca tccttcacat gtcatgacat     540

tgaagtgata gccagtggcc ttgtccccac atacacggca gatttgggga cctccgactt     600

cctcatctgc gttgacactg ggctttccag gaacagactc tgtgtcctca cagtgtacaa     660

agtcagcatg gttccagctt tctttgggtc tcacctccag gtttgcttct gggcctcttg     720

gactagaaat gagaaaacca cagagaatga aaagactgga cagggatgtg tagaggcctc     780

agaggaaaca                                                            790

<210>  160

<211>  558

<212>  DNA

<213>  Homo Sapiens

<220>

<221>  variation

<222>  (263)..(263)

<223>  baySNP900121_G263T

<220>

<221>  variation

<222>  (263)..(263)

<223>

<400>  160
atctttacct atttttcctg cctacagaat cttcctaaaa ggactgtgtc actgtggtct      60

tacataaaca gccagctgga agacttcact aatcctctct atgggagcta ttccaatcat     120

gtcctttatc cagtagccag catgcgccac ctagagctct gggtgggata ttacataagg     180

tggaatccac ggatgaaacc acaggtatgt gccttacaat atacaatggg agttttcatt     240

catgcagtgc aactttgtct ccktaaaagc agctggaaag attatctgtg catatttaaa     300

actttactcc ttacccattg atagttaaca attgtatagt agaaaaatac tacactgggg     360

cagggtaatc agtatacctg gttcttagga ctgcttgtat gacattggac aaatcactgc 420

tctcttttct tgcatcttcc ctcctctgtc tggataacct ctagggggtc ttgttttaat 480

aagctgaaga aagtagcttt aggaaagtta aaagctgaac aaaattctgg tctactgtga 540

atatgtttat ggaatatt 558

<210> 161

<211> 101

<212> DNA

<213> Homo Sapiens

<220>

<221> variation

<222> (51)..(51)

<223> baySNP900115_A51G

<400> 161
tccatctctg agcccgtgtc acagatccag aagatgaaag aaggaagtga rcatcctttt 60

gctctgtcct ccccaccccg atagtgacac atcttcaggc a 101

<210> 162

<211> 1345

<212> DNA

<213>  Homo Sapiens

<220>

<221>  variation

<222>  (816)..(816)

<223>  baySNP900078_A816G

<400>  162
ctgcagtgac cactgcccca tcattgctgg ctgaggtggt tggggtccat ctggctatct      60

gggcagctgt tctcttctct cctttctctc ctgtttccag acatgcagta tttccagaga     120

gaaggggcca ctctttggca aagaacctgt ctaacttgct atctatggca ggacctttga     180

agggttcaca ggaagcagca caaattgata ctattccacc aagccatcag ctccatctca     240

tccatgccct gtctctcctt taggggtccc cttgccaaca gaatcacaga ggaccagcct     300

gaaagtgcag agacagcagc tgaggcacag ccaagagctc tggctgtatt aatgacctaa     360

gaagtcacca gaaagtcaga aggatgcata gcagaggccc agcaatctca gctaagtcaa     420

ctccaccagc ctttctagtt gcccactgtg tgtacagcac cctggtaggg accagagcca     480

tgacagggaa taagactaga ctatgccctt gaggagctca cctctgttca gggaaacagg     540

cgtggaaaca caatggtggt aaagaggaaa gaggacaata ggattgcatg aaggggatgg     600

aaagtgccca ggggaggaaa tggttacatc tgtgtgagga gtttggtgag gaaagactct     660

aagagaaggc tctgtctgtc tgggtttgga aggatgtgta ggagtcttct agggggcaca     720

ggcacactcc aggcataggt aaagatctgt aggtgtggct tgttgggatg aatttcaagt     780

attttggaat gaggacagcc atagagacaa gggcargaga gaggcgattt aatagatttt 840

atgccaatgg ctccacttga gtttctgata agaacccaga acccttggac tccccagtaa 900

cattgattga gttgtttatg atacctcata gaatatgaac tcaaaggagg tcagtgagtg 960

gtgtgtgtgt gattctttgc caacttccaa ggtggagaag cctcttccaa ctgcaggcag 1020

agcacaggtg gccctgctac tggctgcagc tccagccctg cctccttctc tagcatataa 1080

acaatccaac agcctcactg aatcactgct gtgcagggca ggaaagctcc atgcacatag 1140

cccagcaaag agcaacacag agctgaaagg aagactcaga ggagagagat aagtaaggaa 1200

agtagtgatg gctctcatcc cagacttggc catggaaacc tggcttctcc tggctgtcag 1260

cctggtgctc ctctatctgt gagtaactgt tcaggctcct cttctctgtt tcttggactt 1320

ggggtcgtaa tcaggcctct ctttt 1345

<210> 163

<211> 204

<212> DNA

<213> Homo Sapiens

<220>

<221> variation

<222> (103)..(103)

<223> baySNP10000002_A103G

<400> 163
aattcatttt ccaactaata gaagataaca ttaaacagct gaaagagatg aaatttactt 60

atcttattaa ttatatccaa gatgagatca acacaatctt cartgattat atcccatatg 120

tttttaaatt gttgaaagaa aacctatgcc ttaatcttca taagttcaat gaatttattc 180

aaaacgagct tcaggaagct tctc 204

<210> 164

<211> 210

<212> DNA

<213> Homo Sapiens

<220>

<221> variation

<222> (107)..(107)

<223> baySNP10000006_G107A

<400> 164
cgagtcttcc actcgctggc caaggtagct ttccaggatg gccgcctcat gctcagcctg 60

atgggagacg agttcaaggt gagtgggtgg ggctgggctg ctagggratc cagatggcat 120

gtggtatgtg tgtgtgtgca cacgcatggg gaggagggag gaaactcgga aacttggtgg 180

tgggcaaaag aactaagctg gagcaatagc 210

<210>  165

<211>  399

<212>  DNA

<213>  Homo Sapiens

<220>

<221>  variation

<222>  (153)..(153)

<223>  baySNP10000014_A153C

<400>  165
ttcagttaaa gtctatggca ctctgtagga ctgctgatgt ctctgttgca cactgttgat      60

tctaaaatca aaggcactca gtataagcac agtaatagtc ctcctcatca tgctttgtat     120

tttccgtagc tgcctgtacc aatacatcct gcmgtggcca cggtgaatgt gtagagacca     180

tcaataatta cacttgcaag tgtgaccctg gcttcagtgg actcaagtgt gagcaaagta     240

agtctggttc ttgcctcttt cttcacttga gatggtagca ccatctcacg tcctagctgg     300

cattagagtc aggtctgcat gccttccctt ccctggtgca gatggtgtca tatggtgatc     360

gtgagctgag actatgaagt cagaccctgc tgggtttga                            399

<210>  166

<211>  587

<212>  DNA

<213>  Homo Sapiens

<220>

<221>  variation

<222>  (291)..(291)

<223>  baySNP10000025_C291T

<400>  166
   tcttccttct tttggtctct cctgtcagaa tcagcggctc ctgcctcacc ccttctctgg      60
   cgcagagctt gtccctcatc acagggcctg gggcttttta cagaatggag gaagggatcc     120
   tctctgtctg gttatcttgt catcgccacg ggggtgccct gcagaccaca gctctgtgca     180
   gacctccggc ctggcaggac ctgccaatat actgtccttg tctgatgtcc cctccctgcc     240
   cctcttctag gtgccccctt gtttctctcc catcctcact tcctcaacgc ygacccggtt     300
   ctggcagaag cggtgactgg cctgcaccct aaccaggagg cacactcctt gttcctggac     360
   atccacccgg tgagcccctg ccatcctctg tggggggtgg gtgattcctg gttggagcac     420
   acctggctgc ctcctctctc cccaggcaga gagctgctgt gggctggggt ggtgggaagc     480
   ctggcttcta gaatctcgag ccaccaaagt tccttacttc accccgactc catagttcaa     540
   ggtagttcaa gggttttatg atccctgtac tggtttctat aaatggg                   587

<210>  167

<211>  3877

<212>  DNA

<213>  Homo sapiens

<220>

<221>  variation

<222>  (2503)..(2503)

<223>  baySNP900173_G2503T

<400>  167
gaattcaagg tctgcatttt ctaggtatga acactgtgca tgatgaagtc tttccaagcc      60

acaccagtgg ttccatgtgt gtgcacttcc ggtttgagtg ctagtgagat acttctgtgg     120

ttctgaattg cctgactatt tggggttgtg atattttcat aaagattgat caacatgttc     180

gaatttcctc cccaacagtc ttccattacc aagtaaagat tcatttttct gggactgaga     240

gtgaaaccca taccaatcag gcctttgaga tttctctgta tggcaccgtg gccgagagtg     300

agaacatccc attcactctg tgagtagcac aggggggcgg tcatcatggc accagtccct     360

ctcctgccat aacccttggt ctgagcagca gaagcagaga gcgatgccta gaaaacaagt     420

ctttagttaa aaaaatcaga atttcaaaat tgaggtcttt cctctatttg atattgagaa     480

aaaaatgctt caaattggcc attttatttt cacttactag ttatattttt ttatttatca     540

tcttatatct gtttatttct tttataaagc tgctgttaaa caatataatt aaactatctc     600

aaaaggtttg acattaaaga aaatgagcaa tggtaacagg aaaccactct atagatgtac     660

atataatatg tacagaaaat ataagtagta agaagtccat gacaaagtgt tagctctttt 720

tttttttttt tttttttttt tttttgagat ggagtctctc tctattgccc aggctggagt 780

gcagtgattc gatctcagct cactgcaacc tctacctccc gagttcaaac aattcttctg 840

tctcagcctc ccgagtagct ggggctgcag gtgcccacca ccatgcccag ctaatttttg 900

tatttttagt agcgacaggg tctcaccatg ttggccaagc tggtcttgaa ttcctgatct 960

caggtgatcc acccgcctcg gcctcccaaa gtgctgggat tacaggtgtg agccaccatg 1020

cccagcctac cctttactac taatcaaaga aataaaagta aggcaacttg atacttttac 1080

aattactaga tgaacaaatc tttaaaaata gccagtgcag acaaggtggt gaagcagaac 1140

atgcgaacct accatgcatc attcacggct agaaccctcc aggtgcggaa ggtagtattt 1200

taataacttt ccatagctac aaaatattat tacatagaag ggagtgattt ttttctaata 1260

tttatcctaa agaaatagtc aacaaacatt tttaaaaaca tcaattacag tcgtacctat 1320

actagcataa attagaaacc cagtatccaa cattgaggca gtgggtaaat gaatcgtggt 1380

ttatcaagtc attaaaatca atctagcctt taaaaactat aattgtagga aacccaggaa 1440

aacatagtaa aaaatggaat ataaaatctg aagagaataa agaatagaga atcgtatgtg 1500

tgctatgatt gtagctaaat aatgttcaag tatcaacaca aattgaaaag gaatacatga 1560

aaatgaaaat tatatttctg aatgattgac ttcaggattt tcttttagaa ttgtattaaa 1620

tagttcatgt cattaggata aatgctggaa tgtggatata atttaaaata tactaaatgc 1680

catcgacctt cattttgagt tctttgttgg acatttttgt gcatttttaa aatatcccct 1740

aaataataaa gctatttata tttggagagg agaaaaaaaa gtggggggca gggagagctg 1800

atctctataa ctaaccaaat ttattgcttt tttgtttagg cctgaagttt ccacaaataa 1860

gacctactcc ttcctaattt acacagaggt agatattgga gaactactca tgttgaagct 1920

caaatggaag agtgattcat actttagctg gtcagactgg tggagcagtc ccggcttcgc 1980

cattcagaag atcagagtaa aagcaggaga gactcagaaa aagtaattaa atgtattttt 2040

cttccttcac tttagacccc cacctgatgt caggacctag gggctgtatt tcaggggcct 2100

tcacaattca gggagagctt taggaaacct tgtatttatt actgtatgat gtagattttc 2160

tttaggagtc ttcttttatt ttcttatttt tggggggcgg ggggggaagt gacagtattt 2220

ttgtatttca tgtaaggaaa acataagccc tgaatcgctc acagttattc agtgagagct 2280

gggattagaa gtcaggaatc tcagcttctc atttggcact gtttcttgta agtacaaaat 2340

agttagggaa caaacctccg agatgctacc tggataatca aagattcaaa ccaacctctt 2400

ccagaagggt gagattccaa gataatctca acctgtctcc gcagccccac ccatgtgtac 2460

ccataaaatg aattacacag agatcgctat aggatttaaa gcktttatac taaatgtgct 2520

gggattttgc aaactatagt gtgctgttat tgttaattta aaaaaactct aagttaggat 2580

tgacaaatta tttctcttta gtcatttgct tgtatcacca aagaagcaaa caaacaaaca 2640

aaaaaaaaaa gaaaaagatc ttggggatgg aaatgttata aagaatcttt tttacactag 2700

caatgtctag ctgaaggcag atgccctaat tccttaatgc agatgctaag agatggcaga 2760

gttgatcttt tatcatctct tggtgaaagc ccagtaacat aagactgctc taggctgtct 2820

gcatgcctgt ctatctaaat taactagctt ggttgctgaa caccaggtta ggctctcaaa 2880

ttaccctctg attctgatgt ggcctgagtg tgacagttaa ttattgggaa tatcaaaaca 2940

attacccagc atgatcatgt attatttaaa cagtcctgac agaactgtac ctttgtgaac 3000

agtgcttttg attgttctac atggcatatt cacatccatt ttcttccaca gggtgatctt 3060

ctgttctagg gagaaagtgt ctcatttgca gaaaggaaag gcacctgcgg tatttgtgaa 3120

atgccatgac aagtctctga ataagaagtc aggctggtga gcattctggg ctaaagctga 3180

ctgggcatcc tgagcttgca ccctaaggga ggcagcttca tgcattcctc ttcaccccat 3240

caccagcagc ttgccctgac tcatgtgatc aaagcattca atcagtcttt cttagtcctt 3300

ctgcatatgt atcaaatggg tctgttgctt tatgcaatac ttcctctttt tttctttctc 3360

ctcttgtttc tcccagcccg gaccttcaac ccaggcacac attttaggtt ttattttact 3420

ccttgaacta cccctgaatc ttcacttctc cttttttctc tactgcgtct ctgctgactt 3480

tgcagatgcc atctgcagag catgtaacac aagtttagta gttgccgttc tggctgtggg 3540

tgcagctctt cccaggatgt attcagggaa gtaaaaagat ctcactgcat cacctgcagc 3600

cacatagttc ttgattctcc aagtgccagc atactccggg acacacagcc aacagggctg 3660

ccccaagcac ccattctcaa aaccctcaaa gctgccaagc aaacagaatg agagttatag 3720

gaaactgttc tctcttctat ctccaaacaa ctctgtgcct ctttcctacc tgacctttag 3780

ggctaatcca tgtggcagct gttagctgca tctttccaga gcgtcagtac tgagaggaca 3840

ctaagcatgt gaccttcact actcctgttc tgaattc 3877

<210> 168

<211> 620

<212> DNA

<213> Homo sapiens

<220>

<221> variation

<222>  (309)..(309)

<223>  baySNP900013_C309G

<400>  168
ctgtgctgca gaggggagca gcatcagcag gcgtgtggag tgagtgagcc ccgtcctccc      60

cctcctcctg tcattgactc tcattgccta acccccttgt gtcttggccc caggttgatt     120

gtccggggct accgccagcc cctggagggc agtgacctct ggtccttaaa caaggaggac     180

acgtcggaac aagtcgtgcc tgttttggta aagaactgga agaaggaatg cgccaagact     240

aggaagtaag tgtgagtttc cttgtcctcc aggatgccct ggtcacctcc tttccactmc     300

tgtggcctsa atccaggatg gggccctggc agtgctgcct gttactagct ttgtggttct     360

gggcaagatg cctcacttct cctcctggcc tcagtttgct tttctgccaa atggggtagg     420

atctcacatg tcattaaagc aactgggcag agaaaagaag aaagaagctg ggtgcggtgg     480

ctctcgtgtc taatcccagc actttgggag gccaaggcag gcggatcact tggtcgggag     540

tttgagacca gcctggccaa catggtgaaa ccctgtctct actaaaaata caaaattagc     600

taggcatggt ggcaggcgcc                                                  620

## Description

### Technical Field

This invention relates to genetic polymorphisms useful for assessing cardiovascular risks in humans, including, but not limited to, atherosclerosis, ischemia/reperfusion, hypertension, restenosis, arterial inflammation, myocardial infarction, and stroke. In addition it relates to genetic polymorphisms useful for assessing the response to lipid lowering drug therapy. Specifically, the present invention identifies and describes gene variations which are individually present in humans with cardiovascular disease states, rela to humans with normal, or non-cardiovascular disease states, and/or in response to medications relevant to cardiovascular disease. Further, the present invention provides methods for the identification and therapeutic use of compounds as treatments of cardiovascular disease. Moreover, the present invention provides methods for the diagnostic monitoring of patients undergoing clinical evaluation for the treatment of cardiovascular disease, and for monitoring the efficacy of compounds in clinical trials. Still further, the present invention provides methods to use gene variations to predict personal medication schemes omitting adverse drug reactions and allowing an adjustment of the drug dose to achieve maximum benefit for the patient. Additionally, the present invention describes methods for the diagnostic evaluation and prognosis of various cardiovascular diseases, and for the identification of subjects exhibiting a predisposition to such conditions.

### Background of the Invention

Cardiovascular disease is a major health risk throughout the industrialized world.

Cardiovascular diseases include but are not limited by the following disorders of the heart and the vascular system: congestive heart failure, myocardial infarction, atherosclerosis, ischemic diseases of the heart, coronary heart disease, all kinds of atrial and ventricular arrhythmias, hypertensive vascular diseases and peripheral vascular diseases.

Heart failure is defined as a pathophysiologic state in which an abnormality of cardiac function is responsible for the failure of the heart to pump blood at a rate commensurate with the requirement of the metabolizing tissue. It includes all forms of pumping failure such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in an perfusion which is inadequate to meet the myocardial requirement for oxygen. This group of diseases include stable angina, unstable angina and asymptomatic ischemia.

Arrhythmias include all forms of atrial and ventricular tachyarrhythmias (atrial tachycardia, atrial flutter, atrial fibrillation, atrio-ventricular reentrant tachycardia, preexitation syndrome, ventricular tachycardia, ventricular flutter, ventricular fibrillation) as well as bradycardic forms of arrhythmias.

Hypertensive vascular diseases include primary as well as all kinds of secondary arterial hypertension (renal, endocrine, neurogenic, others).

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis, the most prevalent of vascular diseases, is the principal cause of heart attack, stroke, and gangrene of the extremities, and thereby the principal cause of death. Atherosclerosis is a complex disease involving many cell types and molecular factors (for a detailed review, see Ross, 1993, Nature 362: 801-809 and Lusis, A. J., Nature 407, 233-241 (2000)). The process, in normal circumstances a protective response to insults to the endothelium and smooth muscle cells (SMCs) of the wall of the artery, consists of the formation of fibrofatty and fibrous lesions or plaques, preceded and accompanied by inflammation. The advanced lesions of atherosclerosis may occlude the artery concerned, and result from an excessive inflammatory-fibroproliferative response to numerous different forms of insult. For example, shear stresses are thought to be responsible for the frequent occurrence of atherosclerotic plaques in regions of the circulatory system where turbulent blood flow occurs, such as branch points and irregular structures.

The first observable event in the formation of an atherosclerotic plaque occurs when blood-borne monocytes adhere to the vascular endothelial layer and transmigrate through to the sub-endothelial space. Adjacent endothelial cells at the same time produce oxidized low density lipoprotein (LDL). These oxidized LDLs are then taken up in large amounts by the monocytes through scavenger receptors expressed on their surfaces. In contrast to the regulated pathway by which native LDL (nLDL) is taken up by nLDL specific receptors, the scavenger pathway of uptake is not regulated by the monocytes.

These lipid-filled monocytes are called foam cells, and are the major constituent of the fatty streak. Interactions between foam cells and the endothelial and SMCs which surround them lead to a state of chronic local inflammation which can eventually lead to smooth muscle cell proliferation and migration, and the formation of a fibrous plaque. Such plaques occlude the blood vessel concerned and thus restrict the flow of blood, resulting in ischemia.

Ischemia is a condition characterized by a lack of oxygen supply in tissues of organs due to inadequate perfusion. Such inadequate perfusion can have number of natural causes, including atherosclerotic or restenotic lesions, anemia, or stroke, to name a few. Many medical interventions, such as the interruption of the flow of blood during bypass surgery, for example, also lead to ischemia. In addition to sometimes being caused by diseased cardiovascular tissue, ischemia may sometimes affect cardiovascular tissue, such as in ischemic heart disease. Ischemia may occur in any organ, however, that is suffering a lack of oxygen supply.

The most common cause of ischemia in the heart is atherosclerotic disease of epicardial coronary arteries. By reducing the lumen of these vessels, atherosclerosis causes an absolute decrease in myocardial perfusion in the basal state or limits appropriate increases in perfusion when the demand for flow is augmented. Coronary blood flow can also be limited by arterial thrombi, spasm, and, rarely, coronary emboli, as well as by ostial narrowing due to luetic aortitis. Congenital abnormalities, such as anomalous origin of the left anterior descending coronary artery from the pulmonary artery, may cause myocardial ischemia and infarction in infancy, but this cause is very rare in adults. Myocardial ischemia can also occur if myocardial oxygen demands are abnormally increased, as in severe ventricular hypertrophy due to hypertension or aortic stenosis. The latter can be present with angina that is indistinguishable from that caused by coronary atherosclerosis. A reduction in the oxygen-carrying capacity of the blood, as in extremely severe anemia or in the presence of carboxy-hemoglobin, is a rare cause of myocardial ischemia. Not infrequently, two or more causes of ischemia will coexist, such as an increase in oxygen demand due to left ventricular hypertrophy and a reduction in oxygen supply secondary to coronary atherosclerosis.

The foregoing studies are aimed at defining the role of particular gene variations presumed to be involved in the misleading of normal cellular function leading to cardiovascular disease. However, such approaches cannot identify the full panoply of gene variations that are involved in the disease process.

At present, the only available treatments for cardiovascular disorders are pharmaceutical based medications that are not targeted to an individual's actual defect; examples include angiotensin converting enzyme (ACE) inhibitors and diuretics for hypertension, insulin supplementation for non-insulin dependent diabetes mellitus (NIDDM), cholesterol reduction strategies for dyslipidaemia, anti-coagulants, β blockers for cardiovascular disorders and weight reduction strategies for obesity. If targeted treatment strategies were available it might be possible to predict the response to a particular regime of therapy and could markedly increase the effectiveness of such treatment. Although targeted therapy requires accurate diagnostic tests for disease susceptibility, once these tests are developed the opportunity to utilize targeted therapy will become widespread. Such diagnostic tests could initially serve to identify individuals at most risk of hypertension and could allow them to make changes in lifestyle or diet that would serve as preventative measures. The benefits associated by coupling the diagnostic tests with a system of targeted therapy could include the reduction in dosage of administered drugs and thus the amount of unpleasant side effects suffered by an individual. In more severe cases a diagnostic test may suggest that earlier surgical intervention would be useful in preventing a further deterioration in condition.

It is an object of the invention to provide genetic diagnosis of predisposition or susceptibility for cardiovascular diseases. Another related object is to provide treatment to reduce or prevent or delay the onset of disease in those predisposed or susceptible to this disease. A further object is to provide means for carrying out this diagnosis.

Accordingly, a first aspect of the invention provides a method of diagnosis of disease in an individual, said method comprising determining one, various or all genotypes in said individual of the genes listed in the Examples.

In another aspect, the invention provides a method of identifying an individual predisposed or susceptible to a disease, said method comprising determining one, various or all genotypes in said individual of the genes listed in the Examples.

The invention is of advantage in that it enables diagnosis of a disease or of certain disease states via genetic analysis which can yield useable results before onset of disease symptoms, or before onset of severe symptoms. The invention is further of advantage in that it enables diagnosis of predisposition or susceptibility to a disease or of certain disease states via genetic analysis.

The invention may also be of use in confirming or corroborating the results of other diagnostic methods. The diagnosis of the invention may thus suitably be used either as an isolated technique or in combination with other methods and apparatus for diagnosis, in which latter case the invention provides a further test on which a diagnosis may be assessed.

The present invention stems from using allelic association as a method for genotyping individuals; allowing the investigation of the molecular genetic basis for cardiovascular diseases. In a specific embodiment the invention tests for the polymorphisms in the sequences of the listed genes in the Examples. The invention demonstrates a link between this polymorphisms and predispositions to cardiovascular diseases by showing that allele frequencies significantly differ when individuals with "bad" serum lipids are compared to individuals with "good" serum levels. The meaning of "good and bad" serum lipid levels is defined in Table 1a.

Certain disease states would benefit, that is to say the suffering of the patient may be reduced or prevented or delayed, by administration of treatment or therapy in advance of disease appearance; this can be more reliably carried out if advance diagnosis of predisposition or susceptibility to disease can be diagnosed.

### Pharmacogenomics and adverse drug reactions

Adverse drug reactions (ADRs) remain a major clinical problem. A recent meta-analysis suggested that in the USA in 1994, ADRs were responsible for 100 000 deaths, making them between the fourth and sixth commonest cause of death (Lazarou 1998, J. Am. Med. Assoc. 279:1200). Although these figures have been heavily criticized, they emphasize the importance of ADRs. Indeed, there is good evidence that ADRs account for 5% of all hospital admissions and increase the length of stay in hospital by two days at an increased cost of ∼$2500 per patient. ADRs are also one of the commonest causes of drug withdrawal, which has enormous financial implications for the pharmaceutical industry. ADRs, perhaps fortunately, only affect a minority of those taking a particular drug. Although factors that determine susceptibility are unclear in most cases, there is increasing interest in the role of genetic factors. Indeed, the role of inheritable variations in predisposing patients to ADRs has been appreciated since the late 1950s and early 1960s through the discovery of deficiencies in enzymes such as pseudocholinesterase (butyrylcholinesterase) and glucose-6-phosphate dehydrogenase (G6PD). More recently, with the first draft of the human genome just completed, there has been renewed interest in this area with the introduction of terms such as pharmacogenomics and toxicogenomics . Essentially, the aim of pharmacogenomics is to produce personalized medicines, whereby administration of the drug class and dosage is tailored to an individual genotype. Thus, the term pharmacogenomics embraces both efficacy and toxicity.

The 3-hydroxy-3-methylglutaryl coenzyme A (HMG-CoA) reductase inhibitors ("statins") specifically inhibit the enzyme HMG-CoA reductase which catalyzes the rate limiting step in cholesterol biosynthesis. These drugs are effective in reducing the primary and secondary risk of coronary artery disease and coronary events, such as heart attack, in middle-aged and older men and women, in both diabetic and non-diabetic patients, and are often prescribed for patients with hyperlipidemia. Statins used in secondary prevention of coronary artery or heart disease significantly reduce the risk of stroke, total mortality and morbidity and attacks of myocardial ischemia; the use of statins is also associated with improvements in endothelial and fibrinolytic functions and decreased platelet thrombus formation.

The tolerability of these drugs during long term administration is an important issue. Adverse reactions involving skeletal muscle are not uncommon, and sometimes serious adverse reactions involving skeletal muscle such as myopathy and rhabdomyolysis may occur, requiring discontinuation of the drug. In addition an increase in serum creatine kinase (CK) may be a sign of a statin related adverse event. The extend of such adverse events can be read from the extend of the CK level increase (as compared to the upper limit of normal [ULN]).

Occasionally arthralgia, alone or in association with myalgia, has been reported. Also an elevation of liver transaminases has been associated with statin administration.

It was shown that the drug response to statin therapy is a class effects, i.e. all known and presumably also all so far undiscovered statins share the same benefical and harmful effects (Ucar, M. et al., Drug Safety 2000, 22:441). It follows that the discovery of diagnostic tools to predict the drug response to a single statin will also be of aid to guide therapy with other statins.

The present invention provides diagnostic tests to predict the patient's individual response to statin therapy. Such responses include, but are not limited by the extent of adverse drug reactions, the level of lipid lowering or the drug's influence on disease states. Those diagnostic tests may predict the response to statin therapy either alone or in combination with another diagnostic test or another drug regimen.

### Detailed Description of the Invention

The present invention is based at least in part on the discovery that a specific allele of a polymorphic region of a so called "candidate gene" (as defined below) is associated with CVD or drug response.

For the present invention the following candidate genes were analyzed:
- Genes found to be expressed in cardiac tissue (Hwang et al., Circulation 1997, 96:4146-4203).
- Genes from the following metabolic pathways and their regulatory elements:

### Lipid metabolism

Numerous studies have shown a connection between serum lipid levels and cardiovascular diseases. Candidate genes falling into this group include but are not limited by genes of the cholesterol pathway, apolipoproteins and their modifiying factors.

### Coagulation

Ischemic diseases of the heart and in particular myocardial infarction may be caused by a thrombotic occlusion. Genes falling into this group include all genes of the coagulation cascade and their regulatory elements.

### Inflammation

Complications of atherosclerosis are the most common causes of death in Western societies. In broad outline atherosclerosis can be considered to be a form of chronic inflammation resulting from interaction modified lipoproteins, monocyte-derived macrophages,T cells, and the normal cellular elements of the arterial wall. This inflammatory process can ultimately lead to the development of complex lesions, or plaques, that protrude into the arterial lumen. Finally plaque rupture and thrombosis result in the acute clinical complications of myocardial infarction and stroke (Glass et al., Cell 2001, 104:503-516).

It follows that all genes related to inflammatory processes, including but not limited by cytokines, cytokine receptors and cell adhesion molecules are candidate genes for CVD.

### Glucose and energy metabolism

As glucose and energy metabolism is interdependent with the metabolism of lipids (see above) also the former pathways contain candidate genes. Energy metabolism in general also relates to obesity, which is an independent risk factor for CVD (Melanson et al., Cardiol Rev 2001 9:202-207). In addition high blood glucose levels are associated with many microvascular and macrovascular complications and may therefore affect an individuals disposition to CVD (Duckworth, Curr Atheroscler Rep 2001, 3:383-391).

### Hypertension

As hypertension is an independent risk factor for CVD, also genes that are involved in the regulation of systolic and diastolic blood pressure affect an individuals risk for CVD (Safar, Curr Opin Cardiol 2000, 15:258-263). Interestingly hypertension and diabetes (see above) appear to be interdependent, since hypertension is approximately twice as frequent in patients with diabetes compared with patients without the disease. Conversely, recent data suggest that hypertensive persons are more predisposed to the development of diabetes than are normotensive persons (Sowers et al., Hypertension 2001, 37:1053-1059).

### Genes related to drug response

Those genes include metabolic pathways involved in the absorption, distribution, metabolism, excretion and toxicity (ADMET) of drugs. Prominent members of this group are the cytochrome P450 proteins which catalyze many reactions involved in drug metabolism.

### Unclassified genes

As stated above, the mechanisms that lead to cardiovascular diseases or define the patient's individual response to drugs are not completely elucidated. Hence also candidate genes were analysed, which could not be assigned to the above listed categories. The present invention is based at least in part on the discovery of polymorphisms, that lie in genomic regions of unknown physiological function.

### Results

After conducting an association study, we surprisingly found polymorphic sites in a number of candidate genes which show a strong correlation with the following phenotypes of the patients analysed: "Healthy" as used herein refers to individuals that neither suffer from existing CVD, nor exhibit an increased risk for CVD through their serum lipid level profile. "CVD prone" as used herein refers to individuals with existing CVD and/or a serum lipid profile that confers a high risk to get CVD (see Table 1a for definitions of healthy and CVD prone serum lipid levels). "High responder" as used herein refers to patients who benefit from relatively small amounts of a given drug. "Low responder" as used herein refers to patients who need relatively high doses in order to obtain benefit from the medication. "Tolerant patient" refers to individuals who can tolerate high doses of a medicament without exhibiting adverse drug reactions. "ADR patient" as used herein refers to individuals who suffer from ADR or show clinical symptoms (like creatine kinase elevation in blood) even after receiving only minor doses of a medicament (see Table 1b for a detailed definition of drug response phenotypes).

Polymorphic sites in candidate genes that were found to be significantly associated with either of the above mentioned phenotypes will be referred to as "phenotype associated SNPs" (PA SNPs). The respective genomic loci that harbour PA SNPs will be referred to as "phenotype associated genes" (PA genes), irrespective of the actual function of this gene locus.

In particular we surprisingly found PA SNPs associated with CVD, drug efficacy (EFF) or adverse drug reactions (ADR) in the following genes.

### ABCB11: ATP-binding cassette, sub-family B (MDR/TAP), member 11

The membrane-associated protein encoded by this gene is a member of the superfamily of ATP-binding cassette (ABC) transporters. ABC proteins transport various molecules across extra- and intra-cellular membranes. ABC genes are divided into seven distinct subfamilies (ABC1, MDR/TAP, MRP, ALD, OABP, GCN20, White). This protein is a member of the MDR/TAP subfamily. Members of the MDR/TAP subfamily are involved in multidrug resistance. The protein encoded by this gene is the major canalicular bile salt export pump in man. Mutations in this gene cause a form of progressive familial intrahepatic cholestases which are a group of inherited disorders with severe cholestatic liver disease from early infancy.

### ABCB4: ATP-binding cassette, sub-family B (MDR/TAP), member 4

The membrane-associated protein encoded by this gene is a member of the superfamily of ATP-binding cassette (ABC) transporters. ABC proteins transport various molecules across extra- and intra-cellular membranes. ABC genes are divided into seven distinct subfamilies (ABC1, MDR/TAP, MRP, ALD, OABP, GCN20, White). This protein is a member of the MDR/TAP subfamily. Members of the MDR/TAP subfamily are involved in multidrug resistance as well as antigen presentation. This gene encodes a full transporter and member of the p-glycoprotein family of membrane proteins with phosphatidylcholine as its substrate. The function of this protein has not yet been determined; however, it may involve transport of phospholipids from liver hepatocytes into bile. Alternative splicing of this gene results in several products of undetermined function.

### ABCC1: ATP-binding cassette, sub-family C (CFTR/MRP), member 1

The protein encoded by this gene is a member of the superfamily of ATP-binding cassette (ABC) transporters. ABC proteins transport various molecules across extra- and intra-cellular membranes. ABC genes are divided into seven distinct subfamilies (ABC1, MDR/TAP, MRP, ALD, OABP, GCN20, White). This full transporter is a member of the MRP subfamily which is involved in multi-drug resistance. This protein functions as a multispecific organic anion transporter, with oxidized glutatione, cysteinyl leukotrienes, and activated aflatoxin B1 as substrates. This protein also transports glucuronides and sulfate conjugates of steroid hormones and bile salts. Alternative splicing by exon deletion results in several splice variants but maintains the original open reading frame in all forms.

### ACTB mRNA for mutant beta-actin

Beta actin is one of six different actin isoforms which have been identified. ACTB is one of the two nonmuscle cytoskeletal actins. Actins are highly conserved proteins that are involved in cell motility, structure and integrity. Alpha actins are a major constituent of the contractile apparatus.

### ACTIN, ALPHA SKELETAL MUSCLE (ALPHA-ACTIN 1).

Actin alpha 1 which is expressed in skeletal muscle is one of six different actin isoforms which have been identified. Actins are highly conserved proteins that are involved in cell motility, structure and integrity. Alpha actins are a major constituent of the contractile apparatus.

### ADCYAP1: adenylate cyclase activating polypeptide 1 (pituitary)

This gene encodes adenylate cyclase activating polypeptide 1. Mediated by adenylate cyclase activating polypeptide 1 receptors, this polypeptide stimulates adenylate cyclase and subsequently increases the cAMP level in target cells. Adenylate cyclase activating polypeptide 1 is not only a hypophysiotropic hormone, but also functions as a neurotransmitter and neuromodulator. In addition, it plays a role in paracrine and autocrine regulation of certain types of cells. This gene is composed of five exons. Exons 1 and 2 encode the 5' UTR and signal peptide, respectively; exon 4 encodes an adenylate cyclase activating polypeptide 1-related peptide; and exon 5 encodes the mature peptide and 3' UTR. This gene encodes three different mature peptides, including two isotypes: a shorter form and a longer form.

### ADRB3: adrenergic, beta-3-, receptor

The ADRB3 gene product, beta-3-adrenergic receptor, is located mainly in adipose tissue and is involved in the regulation of lipolysis and thermogenesis. Beta adrenergic receptors are involved in the epenephrine and norepinephrine-induced activation of adenylate cyclase through the action of G proteins.

### AGL: amylo-1, 6-glucosidase, 4-alpha-glucanotransferase (glycogen debranching enzyme, glycogen storage disease type III)

Glycogen debranching enzyme is involved in glycogen degradation and has two independent catalytic activities: a 4-alpha-glucotransferase activity (EC 2.4.1.25) and a amylo-1,6-glucosidase activity (EC 3.4.1.33). Both activities occur at different sites on the single polypeptide chain. Mutations in this gene cause glycogen storage disease. A wide range of clinical and enzymatic variability occurs in glycogen debrancher deficiency, some of which may be due to tissue-specific alternative splicing. Six splice varients that differ in the 5' end have been identified in liver and muscle tissue. Variants 1, 5, and 6 are present in both liver and muscle, whereas variants 2, 3, and 4 occur in muscle. Variants 1 through 4 encode identical proteins (isoform 1) that include 27 N-terminal amino acids not found in splice variants 5 and 6. Variants 5 and 6 encode different amino-terminal ends of 10 and 11 amino acids in protein isoforms 2 and 3, respectively, with the remainder of the peptide identical to that of isoforms 1..

### AKAP1: A kinase (PRKA) anchor protein 1

Anchors cAMP-dependent protein kinase near its physiological substrates, interacts with both the type I and type II regulatory subunits.

### Angiotensinogen gene

The protein encoded by this gene, pre-angiotensinogen or angiotensinogen precursor, is expressed in the liver and is cleaved by the enzyme renin in response to lowered blood pressure. The resulting product, angiotensin I is then cleaved by angiotensin converting enzyme (ACE) to generate the physiologically active enzyme angiotensin II. The protein is involved in maintaining blood pressure and in the pathogenesis of essential hypertension and preeclampsia.

### ANXA6: annexin A6

Annexin VI belongs to a family of calcium-dependent membrane and phospholipid binding proteins. Although their functions are still not clearly defined, several members of the annexin family have been implicated in membrane-related events along exocytotic and endocytotic pathways. The annexin VI gene is approximately 60 kbp long and contains 26 exons. It encodes a protein of about 68 kDa that consists of eight 68-amino acid repeats separated by linking sequences of variable lengths. It is highly similar to human annexins I and II sequences, each of which contain four such repeats. Exon 21 of annexin VI is alternatively spliced, giving rise to two isoforms that differ by a 6-amino acid insertion at the start of the seventh repeat. Annexin VI has been implicated in mediating the endosome aggregation and vesicle fusion in secreting epithelia during exocytosis.

### AP2B1: adaptor-related protein complex 2, beta 1 subunit

The beta adaptin subunit is part of the clathrin coat assembly complex which links clathrin to receptors in coated pits and vesicles. These vesicles are involved in endocytosis and Golgi processing. The beta 1 subunit is one of the assembly proteins which binds to clathrin and initiates coat formation.

### APOA1: apolipoprotein A-I

APOA1 promotes cholesterol efflux from tissues to the liver for excretion. Apolipoprotein A-I is the major protein component of high density lipoprotein (HDL) in the plasma. Synthesized in the liver and small intestine, it consists of two identical chains of 77 amino acids; an 18-amino acid signal peptide is removed co-translationally and a 6-amino acid propeptide is cleaved post-translationally. Variation in the latter step, in addition to modifications leading to so-called isoforms, is responsible for some of the polymorphism observed. APOA1 is a cofactor for lecithin cholesterolacyltransferase (LCAT) which is responsible for the formation of most plasma cholesteryl esters. The APOA1, APOC3 and APOA4 genes are closely linked in both rat and human genomes. The A-I and A-IV genes are transcribed from the same strand, while the C-III gene is transcribed convergently in relation to A-I. Defects in the apolipoprotein A-1 gene are associated with HDL deficiency and Tangier disease.

### APOA4: apolipoprotein A-IV

Apoliprotein (apo) A-IV gene contains 3 exons separated by two introns. A sequence polymorphism has been identified in the 3'UTR of the third exon. The primary translation product is a 396-residue preprotein which after proteolytic processing is secreted its primary site of synthesis, the intestine, in association with chylomicron particles. Although its precise function is not known, apo A-IV is a potent activator of lecithin-cholesterol acyltransferase in vitro.

### APOB: apolipoprotein B

Apolipoprotein B (ApoB) is the main apolipoprotein of chylomicrons and low density lipoproteins (LDL). The protein occurs in the plasma in 2 main isoforms, apoB-48 and apoB-100. The first is synthesized exclusively by the gut, the second by the liver. The intestinal (B-48) and hepatic (B-100) forms of apoB are coded by a single gene and by a single mRNA transcript larger than 16 kb. The 2 proteins share a common amino terminal sequence. In the ApoB-100 isoform the precursor has 4,563 amino acids, and the mature apoB-100 has 4,536 amino acid residues. Mature, circulating B-48 is homologous over its entire length (estimated to be between 2,130 and 2,144 amino acid residues) with the amino-terminal portion of B-100 and contains no sequence from the carboxyl end of B-100. From structural studies, it is thought that apoB-48 represents the amino-terminal 47% of apoB-100 and that the carboxyl terminus of apoB-48 is in the vicinity of residue 2151 of apoB-100. Apolipoprotein B-48 may be the product of an intestinal mRNA with an in-frame UAA stop codon resulting from a C-to-U change in the codon CAA encoding Gln(2153) in apoB-100 mRNA. Since only the sequence that codes B-100 is present in genomic DNA, this presents the possibility of an organ-specific introduction of a stop codon to an mRNA and the change from CAA to UAA of codon 2153 of the message as a unique RNA editing process..

### APOD: apolipoprotein D

Apolipoprotein D (Apo-D) is a component of high density lipoprotein that has no marked similarity to other apolipoprotein sequences. It has a high degree of homology to plasma retinol-binding protein and other members of the alpha 2 microglobulin protein superfamily of carrier proteins, also known as lipocalins. It is a glycoprotein of estimated molecular weight 33 KDa. Apo-D is closely associated with the enzyme lecithin:cholesterol acyltransferase - an enzyme involved in lipoprotein metabolism.

### Apolipoprotein B

Apolipoprotein B (ApoB) is the main apolipoprotein of chylomicrons and low density lipoproteins (LDL). The protein occurs in the plasma in 2 main isoforms, apoB-48 and apoB-100. The first is synthesized exclusively by the gut, the second by the liver. The intestinal (B-48) and hepatic (B-100) forms of apoB are coded by a single gene and by a single mRNA transcript larger than 16 kb. The 2 proteins share a common amino terminal sequence. In the ApoB-100 isoform the precursor has 4,563 amino acids, and the mature apoB-100 has 4,536 amino acid residues. Mature, circulating B-48 is homologous over its entire length (estimated to be between 2,130 and 2,144 amino acid residues) with the amino-terminal portion of B-100 and contains no sequence from the carboxyl end of B-100. From structural studies, it is thought that apoB-48 represents the amino-terminal 47% of apoB-100 and that the carboxyl terminus of apoB-48 is in the vicinity of residue 2151 of apoB-100. Apolipoprotein B-48 may be the product of an intestinal mRNA with an in-frame UAA stop codon resulting from a C-to-U change in the codon CAA encoding Gln(2153) in apoB-100 mRNA. Since only the sequence that codes B-100 is present in genomic DNA, this presents the possibility of an organ-specific introduction of a stop codon to an mRNA and the change from CAA to UAA of codon 2153 of the message as a unique RNA editing process..

### APXL: apical protein-like (Xenopus laevis)

The protein encoded by this gene shares significant similarities with the apical protein from Xenopus laevis which is implicated in amiloride-sensitive sodium channel activity. This gene is a strong candidate gene for ocular albinism type 1 syndrome.

### ARF4: ADP-ribosylation factor 4

ADP-ribosylation factor 4 (ARF4) is a member of the human ARF gene family. These genes encode small guanine nucleotide-binding proteins that stimulate the ADP-ribosyltransferase activity of cholera toxin and play a role in vesicular trafficking and as activators of phospholipase D. The gene products include 6 ARF proteins and 11 ARF-like proteins and constitute 1 family of the RAS superfamily. The ARF proteins are categorized as class I (ARF1, ARF2,and ARF3), class II (ARF4 and ARF5) and class III (ARF6). The members of each class share a common gene organization. The ARF4 gene spans approximately 12kb and contains six exons and five introns. The ARF4 is the most divergent member of the human ARFs. Conflicting Map positions at 3p14 or 3p21 have been reported for this gene.

### ATP1A2: ATPase, Na+/K+ transporting, alpha 2 (+) polypeptide

Alpha 2 subunit of the sodium- and potassium-transporting ATPase; required for Na+ and K+ gradient maintenance across plasma membrane.

### ATP1B1: ATPase, Na+/K+ transporting, beta 1 polypeptide

Beta 1 subunit of Na+/K+-ATPase.

### ATP1B3: ATPase, Na+/K+ transporting, beta 3 polypeptide

Beta 3 subunit of the Na+/K+ -ATPase.

### ATP2A2: ATPase, Ca++ transporting, cardiac muscle, slow twitch 2

Slow twitch cardiac muscle Ca2+-ATPase; pumps calcium, may have a role in calcium signaling pathways.

### ATP5G1: ATP synthase, H+ transporting, mitochondrial F0 complex, subunit c (subunit 9), isoform 1

Isoform 1 (P1) of subunit c, H+-translocating subunit of F0 ATP synthase; catalyzes the synthesis of ATP during oxidative phosphorylation.

### ATP6V1E: ATPase, H+ transporting, lysosomal 31kD, V1 subunit E

This gene encodes a component of vacuolar ATPase (V-ATPase), a multisubunit enzyme that mediates acidification of eukaryotic intracellular organelles. V-ATPase dependent organelle acidification is necessary for such intracellular processes as protein sorting, zymogen activation, and receptor-mediated endocytosis. V-ATPase is comprised of a cytosolic V1 domain and a transmembrane V0 domain. The V1 domain consists of a hexamer of three A and three B subunits plus the C, D, and E subunits. It contains the ATP catalytic site. The encoded protein is known as the E subunit and is found ubiquitously. Pseudogenes for this gene have been found in the genome.

### ATPase, Ca++ transporting, cardiac muscle, fast twitch 1

Fast-twitch skeletal muscle sarcoplasmic reticulum Ca2+-ATPase; pumps calcium.

### AXIN1: axin

Strongly similar to murine Axin; may regulate embryonic axis formation.

### BMPR1A: bone morphogenetic protein receptor, type IA

The bone morphogenetic protein (BMP) receptors are a family of transmembrane serine/threonine kinases that include the type I receptors BMPR1A and BMPR1B and the type II receptor BMPR2. These receptors are also closely related to the activin receptors, ACVR1 and ACVR2. The ligands of these receptors are members of the TGF-beta superfamily. TGF-betas and activins transduce their signals through the formation of heteromeric complexes with 2 different types of serine (threonine) kinase receptors: type I receptors of about 50-55 kD and type II receptors of about 70-80 kD. Type II receptors bind ligands in the absence of type I receptors, but they require their respective type I receptors for signaling, whereas type I receptors require their respective type II receptors for ligand binding.

### BRD3: bromodomain containing 3

This gene was identified based on its homology to the gene encoding the RING3 protein, a serine/threonine kinase. The gene localizes to 9q34, a region which contains several major histocompatibility complex (MHC) genes. The function of the encoded protein is not known.

### CACNA1C: calcium channel, voltage-dependent, L type, alpha 1C subunit

Alpha 1C subunit of the voltage-dependent calcium channel; channel is of the L type and is expressed in the heart.

### CALB2: calbindin 2, (29kD, calretinin)

Calbindin 2 (calretinin), closely related to calbindin 1, is an intracellular calcium-binding protein belonging to the troponin C superfamily. Calbindin 1 is known to be involved in the vitamin-D-dependent calcium absorption through intestinal and renal epithelia, while the function of neuronal calbindin 1 and calbindin 2 is poorly understood. The sequence of the calbindin 2 cDNA reveals an open reading frame of 271 codons coding for a protein of 31,520 Da, and shares 58% identical residues with human calbindin 1. Calbindin 2 contains five presumably active and one presumably inactive calcium-binding domains. Comparison with the partial sequences available for chick and guinea pig calbindin 2 reveals that the protein is highly conserved in evolution. The calbindin 2 message was detected in the brain, while absent from heart muscle, kidney, liver, lung, spleen, stomach and thyroid gland. There are two additional forms of alternatively spliced calbindin 2 mRNAs encoding C-terminally truncated proteins. Exon 7 can splice to exon 9, resulting in a frame shift and a translational stop at the second codon of exon 9, and encoding calretinin-20k. Exon 7 can also splice to exon 10, resulting in a frame shift and a translational stop at codon 15 of exon 10, and encoding calretinin-22k. The truncated proteins are able to bind calcium..

### CALCIUM-TRANSPORTING ATPASE PLASMA MEMBRANE, ISOFORMS 3A/3B (EC 3.6.1.38) (CALCIUM PUMP) (PMCA3).

Plasma membrane Ca2+-ATPase 3; pumps calcium.

### CALM3: calmodulin 3 (phosphorylase kinase, delta)

Calmodulin 3; binds calcium.

### CAV1: caveolin 1, caveolae protein, 22kD

The scaffolding protein encoded by this gene is the main component of the caveolae plasma membranes found in most cell types. The protein links integrin subunits to the tyrosine kinase FYN, an initiating step in coupling integrins to the Ras-ERK pathway and promoting cell cycle progression. The gene is a tumor suppressor gene candidate and a negative regulator of the Ras-p42/44 MAP kinase cascade. CAV1 and CAV2 are located next to each other on chromosome 7 and express colocalizing proteins that form a stable hetero-oligomeric complex. By using alternative initiation codons in the same reading frame, two isoforms (alpha and beta) are encoded by a single transcript from this gene.

### CAV3: caveolin 3

This gene encodes a caveolin family member, which functions as a component of the caveolae plasma membranes found in most cell types. Caveolin proteins are proposed to be scaffolding proteins for organizing and concentrating certain caveolin-interacting molecules. Mutations identified in this gene lead to interference with protein oligomerization or intra-cellular routing, disrupting caveolae formation and resulting in Limb-Girdle muscular dystrophy type-1C (LGMD-1C), hyperCKemia or rippling muscle disease (RMD). Alternative splicing has been identified for this locus, with inclusion or exclusion of a differentially spliced intron. In addition, transcripts utilize multiple polyA sites and contain two potential translation initiation sites.

### CCR2: chemokine (C-C motif) receptor 2

This gene encodes two isoforms of a receptor for monocyte chemoattractant protein-1, a chemokine which specifically mediates monocyte chemotaxis. Monocyte chemoattractant protein-1 is involved in monocyte infiltration in inflammatory diseases such as rheumatoid arthritis as well as in the inflammatory response against tumors. The receptors encoded by this gene mediate agonist-dependent calcium mobilization and inhibition of adenylyl cyclase. This gene is located in the chemokine receptor gene cluster region. Two alternatively spliced transcript variants are expressed by the gene.

### CDH1: cadherin 1, type 1, E-cadherin (epithelial)

This gene is a classical cadherin from the cadherin superfamily. The encoded protein is a calcium dependent cell-cell adhesion glycoprotein comprised of five extracellular cadherin repeats, a transmembrane region and a highly conserved cytoplasmic tail. Mutations in this gene are correlated with gastric, breast, colorectal, thyroid and ovarian cancer. Loss of function is thought to contribute to progression in cancer by increasing proliferation, invasion, and/or metastasis. The ectodomain of this protein mediates bacterial adhesion to mammalian cells and the cytoplasmic domain is required for internalization. Identified transcript variants arise from mutation at consensus splice sites.

### CDH11: cadherin 11, type 2, OB-cadherin (osteoblast)

This gene encodes a type II classical cadherin from the cadherin superfamily, integral membrane proteins that mediate calcium-dependent cell-cell adhesion. Mature cadherin proteins are composed of a large N-terminal extracellular domain, a single membrane-spanning domain, and a small, highly conserved C-terminal cytoplasmic domain. Type II (atypical) cadherins are defined based on their lack of a HAV cell adhesion recognition sequence specific to type I cadherins. Expression of this particular cadherin in osteoblastic cell lines, and its upregulation during differentiation, suggests a specific function in bone development and maintenance. Two splice variants have been identified, one of which encodes an isoform with a truncated cytoplasmic domain.

### CDH13: cadherin 13, H-cadherin (heart)

This gene is a member of the cadherin superfamily. The encoded protein is a calcium dependent cell-cell adhesion glycoprotein comprised of five extracellular cadherin repeats, a transmembrane region but, unlike the typical cadherin superfamily member, lacks the highly conserved cytoplasmic region. This particular cadherin is a putative mediator of cell-cell interaction in the heart and may act as a negative regulator of neural cell growth. The gene locus is hypermethylated or deleted in breast, ovarian and lung cancers. Two major mRNA transcripts encoding identical proteins are found, products of alternative polyadenylation sites.

### CENPC1: centromere protein C 1

Centromere protein C 1 is a centromere autoantigen and a component of the inner kinetochore plate. The protein is required for maintaining proper kinetochore size and a timely transition to anaphase. A putative psuedogene exists on chromosome 12.

### Cholesteryl ester transfer protein (CETP)

Cholestery ester transfer protein (CETP) transfers cholesteryl esters between lipoproteins. CETP may effect susceptibility to atherosclerosis.

### CLCN4: chloride channel 4

The CLCN family of voltage-dependent chloride channel genes comprises nine members (CLCN1-7, Ka and Kb) which demonstrate quite diverse functional characteristics while sharing significant sequence homology. Chloride channel 4 has an evolutionary conserved CpG island and is conserved in both mouse and hamster. This gene is mapped in close proximity to APXL (Apical protein Xenopus laevis-like) and OA1 (Ocular albinism type I), which are both located on the human X chromosome at band p22.3. The physiological role of chloride channel 4 remains unknown but may contribute to the pathogenesis of neuronal disorders.

### CLCNKA: chloride channel Ka

Putative chloride channel; member of the CLC family of voltage-gated chloride channels.

### COL6A3: collagen, type VI, alpha 3

This gene encodes the alpha 3 chain, one of the three alpha chains of type VI collagen, a beaded filament collagen found in most connective tissues. The alpha 3 chain of type VI collagen is much larger than the alpha 1 and 2 chains. This difference in size is largely due to an increase in the number of subdomains, similar to von Willebrand Factor type A domains, found in the amino terminal globular domain of all the alpha chains. These domains have been shown to bind extracellular matrix proteins, an interaction that explains the importance of this collagen in organizing matrix components. Mutations in the type VI collagen genes are associated with Bethlem myopathy. In addition to the full length transcript, four transcript variants have been identified that encode proteins with N-terminal globular domains of varying sizes.

### COL7A1: collagen, type VII, alpha 1 (epidermolysis bullosa, dystrophic, dominant and recessive)

This gene encodes the alpha chain of type VII collagen. The type VII collagen fibril, composed of three identical alpha collagen chains, is restricted to the basement zone beneath stratified squamous epithelia. It functions as an anchoring fibril between the external epithelia and the underlying stroma. Mutations in this gene are associated with all forms of dystrophic epidermolysis bullosa. In the absence of mutations, however, an acquired form of this disease can result from an autoimmune response made to type VII collagen.

### COL9A3: collagen, type IX, alpha 3

This gene encodes one of the three alpha chains of type IX collagen, the major collagen component of hyaline cartilage. Type IX collagen, a heterotrimeric molecule, is usually found in tissues containing type II collagen, a fibrillar collagen. Mutations in this gene are associated with multiple epiphyseal dysplasia.

### COMT: catechol-O-methyltransferase

Catechol-O-methyltransferase catalyzes the transfer of a methyl group from S-adenosylmethionine to catecholamines, including the neurotransmitters dopamine, epinephrine, and norepinephrine. This O-methylation results in one of the major degradative pathways of the catecholamine transmitters. In addition to its role in the metabolism of endogenous substances, COMT is important in the metabolism of catechol drugs used in the treatment of hypertension, asthma, and Parkinson disease. COMT is found in two forms in tissues, a soluble form (S-COMT) and a membrane-bound form (MB-COMT). The differences between S-COMT and MB-COMT reside within the N-termini. The transcript variants are formed through the use of alternative translation initiation sites and promoters.

### COX10: COX10 homolog, cytochrome c oxidase assembly protein, heme A: farnesyltransferase (yeast)

Cytochrome c oxidase (COX), the terminal component of the mitochondrial respiratory chain, catalyzes the electron transfer from reduced cytochrome c to oxygen. This component is a heteromeric complex consisting of 3 catalytic subunits encoded by mitochondrial genes and multiple structural subunits encoded by nuclear genes. The mitochondrially-encoded subunits function in electron transfer, and the nuclear-encoded subunits may function in the regulation and assembly of the complex. This nuclear gene encodes heme A:farnesyltransferase, which is not a structural subunit but required for the expression of functional COX and functions in the maturation of the heme A prosthetic group of COX. This protein is predicted to contain 7-9 transmembrane domains localized in the mitochondrial inner membrane. A gene mutation, which results in the substitution of a lysine for an asparagine (N204K), is identified to be responsible for cytochrome c oxidase deficiency. In addition, this gene is disrupted in patients with CMT1A (Charcot-Marie-Tooth type 1A) duplication and with HNPP (hereditary neuropathy with liability to pressure palsies) deletion..

### CPB2: carboxypeptidase B2 (plasma, carboxypeptidase U)

Carboxypeptidases are enzymes that hydrolyze C-terminal peptide bonds. The carboxypeptidase family includes metallo-, serine, and cysteine carboxypeptidases. According to their substrate specificity, these enzymes are referred to as carboxypeptidase A (cleaving aliphatic residues) or carboxypeptidase B (cleaving basic amino residues). The protein encoded by this gene is activated by trypsin and acts on carboxypeptidase B substrates. After thrombin activation, the mature protein downregulates fibrinolysis. Polymorphisms have been described for this gene and its promoter region. Available sequence data analyses indicate splice variants that encode different isoforms.

### CPO: coproporphyrinogen oxidase (coproporphyria, harderoporphyria)

Coproporphyrinogen; catalyzes oxidative decarboxylation in sixth step of heme biosynthesis.

### CRYAB: crystallin, alpha B

Crystallins are separated into two classes: taxon-specific, or enzyme, and ubiquitous. The latter class constitutes the major proteins of vertebrate eye lens and maintains the transparency and refractive index of the lens. Since lens central fiber cells lose their nuclei during development, these crystallins are made and then retained throughout life, making them extremely stable proteins. Mammalian lens crystallins are divided into alpha, beta, and gamma families; beta and gamma crystallins are also considered as a superfamily. Alpha and beta families are further divided into acidic and basic groups. Seven protein regions exist in crystallins: four homologous motifs, a connecting peptide, and N- and C-terminal extensions. Alpha crystallins are composed of two gene products: alpha-A and alpha-B, for acidic and basic, respectively. Alpha crystallins can be induced by heat shock and are members of the small heat shock protein (sHSP also known as the HSP20) family. They act as molecular chaperones although they do not renature proteins and release them in the fashion of a true chaperone; instead they hold them in large soluble aggregates. Post-translational modifications decrease the ability to chaperone. These heterogeneous aggregates consist of 30-40 subunits; the alpha-A and alpha-B subunits have a 3:1 ratio, respectively. Two additional functions of alpha crystallins are an autokinase activity and participation in the intracellular architecture. Alpha-A and alpha-B gene products are differentially expressed; alpha-A is preferentially restricted to the lens and alpha-B is expressed widely in many tissues and organs. Elevated expression of alpha-B crystallin occurs in many neurological diseases; a missense mutation cosegregated in a family with a desmin-related myopathy..

### CSF2RB: colony stimulating factor 2 receptor, beta, low-affinity (granulocyte-macrophage)

CSF2RB is a common beta chain of the high affinity receptor for IL-3, IL-5 and CSF. Defective CSF2RB has been reported to be associated with protein alveolar proteinosis.

### CUBN: cubilin (intrinsic factor-cobalamin receptor)

Cubilin (CUBN) acts as a receptor for intrinsic factor-vitamin B12 complexes. The role of receptor is supported by the presence of 27 CUB domains. Cubulin is located within the epithelium of intestine and kidney. Mutations in CUBN may play a role in autosomal recessive megaloblastic anemia.

### CXorf6: chromosome X open reading frame 6

### CYP17: cytochrome P450, subfamily XVII (steroid 17-alpha-hydroxylase), adrenal hyperplasia

This gene encodes a member of the cytochrome P450 superfamily of enzymes. The cytochrome P450 proteins are monooxygenases which catalyze many reactions involved in drug metabolism and synthesis of cholesterol, steroids and other lipids. This protein localizes to the endoplasmic reticulum. It has both 17alpha-hydroxylase and 17,20-lyase activities and is a key enzyme in the steroidogenic pathway that produces progestins, mineralocorticoids, glucocorticoids, androgens, and estrogens. Mutations in this gene are associated with isolated steroid-17 alpha-hydroxylase deficiency, 17-alpha-hydroxylase/17,20-lyase deficiency, pseudohermaphroditism, and adrenal hyperplasia.

### CYP2C8: cytochrome P450, subfamily IIC (mephenytoin 4-hydroxylase), polypeptide 8

This gene encodes a member of the cytochrome P450 superfamily of enzymes. The cytochrome P450 proteins are monooxygenases which catalyze many reactions involved in drug metabolism and synthesis of cholesterol, steroids and other lipids. This protein localizes to the endoplasmic reticulum and its expression is induced by phenobarbital. The enzyme is known to metabolize many xenobiotics, including the anticonvulsive drug mephenytoin, benzo(a)pyrene, 7-ethyoxycoumarin, and the anticancer drug taxol. Two transcript variants for this gene have been described; it is thought that the longer form does not encode an active cytochrome P450 since its protein product lacks the heme binding site. This gene is located within a cluster of cytochrome P450 genes on chromosome 10q24.

### CYP2E: cytochrome P450, subfamily IIE (ethanol-inducible)

This gene encodes a member of the cytochrome P450 superfamily of enzymes. The cytochrome P450 proteins are monooxygenases which catalyze many reactions involved in drug metabolism and synthesis of cholesterol, steroids and other lipids. This protein localizes to the endoplasmic reticulum and is induced by ethanol, the diabetic state, and starvation. The enzyme metabolizes both endogenous substrates, such as ethanol, acetone, and acetal, as well as exogenous substrates including benzene, carbon tetrachloride, ethylene glycol, and nitrosamines which are premutagens found in cigarette smoke. Due to its many substrates, this enzyme may be involved in such varied processes as gluconeogenesis, hepatic cirrhosis, diabetes, and cancer.

### CYP3A4

This gene, CYP3A4, encodes a member of the cytochrome P450 superfamily of enzymes. The cytochrome P450 proteins are monooxygenases which catalyze many reactions involved in drug metabolism and synthesis of cholesterol, steroids and other lipids. This protein localizes to the endoplasmic reticulum and its expression is induced by glucocorticoids and some pharmacological agents. This enzyme is involved in the metabolism of approximately half the drugs which are are used today, including acetaminophen, codeine, cyclosporin A, diazepam and erythromycin. The enzyme also metabolizes some steroids and carcinogens. This gene is part of a cluster of cytochrome P450 genes on chromosome 7q21.1. Previously another CYP3A gene, CYP3A3, was thought to exist; however, it is now thought that this sequence represents a transcript variant of CYP3A4.

### CYP4F8: cytochrome P450, subfamily IVF, polypeptide 8

This gene, CYP4F8, encodes a member of the cytochrome P450 superfamily of enzymes. The cytochrome P450 proteins are monooxygenases which catalyze many reactions involved in drug metabolism and synthesis of cholesterol, steroids and other lipids. This protein localizes to the endoplasmic reticulum and functions as a 19-hydroxylase of prostaglandins in seminal vesicles. This gene is part of a cluster of cytochrome P450 genes on chromosome 19. Another member of this family, CYP4F3, is approximately 18 kb away.

### CYP8B1: cytochrome P450, subfamily VIIIB (sterol 12-alpha-hydroxylase), polypeptide 1

This gene encodes a member of the cytochrome P450 superfamily of enzymes. The cytochrome P450 proteins are monooxygenases which catalyze many reactions involved in drug metabolism and synthesis of cholesterol, steroids and other lipids. This endoplasmic reticulum membrane protein catalyzes the conversion of 7 alpha-hydroxy-4-cholesten-3-one into 7-alpha,12-alpha-dihydroxy-4-cholesten-3-one. The balance between these two steroids determines the relative amounts of cholic acid and chenodeoxycholic acid both of which are secreted in the bile and affect the solubility of cholesterol. This gene is unique among the cytochrome P450 genes in that it is intronless.

### DBI: diazepam binding inhibitor (GABA receptor modulator, acyl-Coenzyme A binding protein)

Diazepam binding inhibitor (acyl-CoA-binding protein); binds and induces medium-chain acyl-CoA ester synthesis.

### DEFA6: defensin, alpha 6, Paneth cell-specific

Defensins are a family of microbicidal and cytotoxic peptides thought to be involved in host defense. They are abundant in the granules of neutrophils and also found in the epithelia of mucosal surfaces such as those of the intestine, respiratory tract, urinary tract, and vagina. Members of the defensin family are highly similar in protein sequence and distinguished by a conserved cysteine motif. Several alpha defensin genes appear to be clustered on chromosome 8. The protein encoded by this gene, defensin, alpha 6, is highly expressed in the secretory granules of Paneth cells of the small intestine, and likely plays a role in host defense of human bowel.

### DEK: DEK oncogene (DNA binding)

Site-specific DNA binding protein; involved in transcriptional regulation and signal transduction.

### DFNA5: deafness, autosomal dominant 5

Hearing impairment is a heterogeneous condition with over 40 loci described. The protein encoded by this gene is expressed in fetal cochlea, however, its function is not known. Nonsyndromic hearing impairment is associated with a mutation in this gene.

### DGKD: diacylglycerol kinase, delta (130kD)

Diacylglycerol kinase delta; phosphorylates the arachidonoyl type of diacylglycerol; contains a pleckstrin homology domain and an EPH domain.

### DOCK1: dedicator of cyto-kinesis 1

Dedicator of cyto-kinesis 1 binds to the SH3 domain of CRK protein. It may regulate cell surface extension and may have a role in the cell surface extension of an engulfing cell around a dying cell during apoptosis.

### ECE1: endothelin converting enzyme 1

Endothelin converting enzyme; metalloprotease that regulates a peptide involved in vasocontriction.

### E-Selectin (CD62E)

The endothelial leukocyte adhesion molecule-1 is expressed by cytokine-stimulated endothelial cells. It is thought to be responsible for the accumulation of blood leukocytes at sites of inflammation by mediating the adhesion of cells to the vascular lining. It exhibits structural features such as the presence of lectin- and EGF-like domains followed by short consensus repeat (SCR) domains that contain 6 conserved cysteine residues. These proteins are part of the selectin family of cell adhesion molecules. This gene is present in single copy in the human genome and contains 14 exons spanning about 13 kb of DNA. Adhesion molecules participate in the interaction between leukocytes and the endothelium and appear to be involved in the pathogenesis of atherosclerosis.

### ESR1: estrogen receptor 1

Estrogen receptor; nuclear receptor transcription factor activated by ligand-binding, involved in hormone-mediated inhibition of gene expression.

### ESR2: estrogen receptor 2 (ER beta)

Estrogen receptor beta 2; transcriptional activator involved in regulation of reproduction; exists in five isoforms.

### F2: coagulation factor II (thrombin)

Coagulation factor II is proteolytically cleaved to form thrombin in the first step of the coagulation cascade which ultimately results in the stemming of blood loss. F2 also plays a role in maintaining vascular integrity during development and postnatal life. Mutations in F2 leads to various forms of thrombosis and dysprothrombinemia.

### F3: coagulation factor III (thromboplastin, tissue factor)

This gene encodes coagulation factor III which is a cell surface glycoprotein. This factor enables cells to initiate the blood coagulation cascades, and it functions as the high-affinity receptor for the coagulation factor VII. The resulting complex provides a catalytic event that is responsible for initiation of the coagulation protease cascades by specific limited proteolysis. Unlike the other cofactors of these protease cascades, which circulate as nonfunctional precursors, this factor is a potent initiator that is fully functional when expressed on cell surfaces. There are 3 distinct domains of this factor: extracellular, transmembrane, and cytoplasmic. This protein is the only one in the coagulation pathway for which a congenital deficiency has not been described.

### F5: coagulation factor V (proaccelerin, labile factor)

This gene encodes coagulation factor V which is an essential factor of the blood coagulation cascade. This factor circulates in plasma, and is converted to the active form by the release of the activation peptide by thrombin during coagulation. This generates a heavy chain and a light chain which are held together by calcium ions. The active factor V is a cofactor that participates with activated coagulation factor X to activate prothrombin to thrombin. Defects in this gene result in either an autosomal recessive hemorrhagic diathesis or an autosomal dominant form of thrombophilia, which is known as activated protein C resistance.

### F7: coagulation factor VII (serum prothrombin conversion accelerator)

This gene encodes coagulation factor VII which is a vitamin K-dependent factor essential for hemostasis. This factor circulates in the blood in a zymogen form, and is converted to an active form by either factor IXa, factor Xa, factor XIIa, or thrombin by minor proteolysis. Upon activation of the factor VII, a heavy chain containing a catalytic domain and a light chain containing 2 EGF-like domains are generated, and two chains are held together by a disulfide bond. In the presence of factor III and calcium ions, the activated factor then further activates the coagulation cascade by converting factor IX to factor IXa and/or factor X to factor Xa. Alternative splicing of this gene results in 2 transcripts. Defects in this gene can cause coagulopathy.

### F9: coagulation factor IX (plasma thromboplastic component, Christmas disease, hemophilia B)

This gene encodes vitamin K-dependent coagulation factor IX that circulates in the blood as an inactive zymogen. This factor is converted to an active form by factor XIa, which excises the activation peptide and thus generates a heavy chain and a light chain held together by one or more disulfide bonds. The role of this activated factor IX in the blood coagulation cascade is to activate factor X to its active form through interactions with Ca+2 ions, membrane phospholipids, and factor VIII. Alterations of this gene, including point mutations, insertions and deletions, cause factor IX deficiency, which is a recessive X-linked disorder, also called hemophilia B or Christmas disease.

### FABP3: fatty acid binding protein 3, muscle and heart (mammary-derived growth inhibitor)

The intracellular fatty acid-binding proteins (FABPs) belongs to a multigene family. FABPs are divided into at least three distinct types, namely the hepatic-, intestinal- and cardiac-type. They form 14-15 kDa proteins and are thought to participate in the uptake, intracellular metabolism and/or transport of long-chain fatty acids. They may also be responsible in the modulation of cell growth and proliferation. Fatty acid-binding protein 3 gene contains four exons and its function is to arrest growth of mammary epithelial cells. This gene is a candidate tumor suppressor gene for human breast cancer.

### FACL3: fatty-acid-Coenzyme A ligase, long-chain 3

The protein encoded by this gene is an isozyme of the long-chain fatty-acid-coenzyme A ligase family. Although differing in substrate specificity, subcellular localization, and tissue distribution, all isozymes of this family convert free long-chain fatty acids into fatty acyl-CoA esters, and thereby play a key role in lipid biosynthesis and fatty acid degradation. This isozyme is highly expressed in brain, and preferentially utilizes myristate, arachidonate, and eicosapentaenoate as substrates. The amino acid sequence of this isozyme is 92% identical to that of rat homolog.

### FACL4: fatty-acid-Coenzyme A ligase, long-chain 4

The protein encoded by this gene is an isozyme of the long-chain fatty-acid-coenzyme A ligase family. Although differing in substrate specificity, subcellular localization, and tissue distribution, all isozymes of this family convert free long-chain fatty acids into fatty acyl-CoA esters, and thereby play a key role in lipid biosynthesis and fatty acid degradation. This isozyme preferentially utilizes arachidonate as substrate. The absence of this enzyme may contribute to the mental retardation or Alport syndrome. Alternative splicing of this gene generates 2 transcript variants.

### FMO1: flavin containing monooxygenase 1

Metabolic N-oxidation of the diet-derived amino-trimethylamine (TMA) is mediated by flavin-containing monooxygenase and is subject to an inherited FMO3 polymorphism in man resulting in a small subpopulation with reduced TMA N-oxidation capacity resulting in fish odor syndrome Trimethylaminuria. Three forms of the enzyme, FMO1 found in fetal liver, FMO2 found in adult liver, and FMO3 are encoded by genes clustered in the 1q23-q25 region. Flavin-containing monooxygenases are NADPH-dependent flavoenzymes that catalyzes the oxidation of soft nucleophilic heteroatom centers in drugs, pesticides, and xenobiotics.

### GAA: glucosidase, alpha; acid (Pompe disease, glycogen storage disease type II)

This gene encodes acid alpha-glucosidase, which is essential for the degradation of glycogen to glucose in lysosomes. Different forms of acid alpha-glucosidase are obtained by proteolytic processing. Defects in this gene are the cause of glycogen storage disease II, also known as Pompe's disease, which is an autosomal recessive disorder with a broad clinical spectrum.

### GAPD: glyceraldehyde-3-phosphate dehydrogenase

Glyceraldehyde-3-phosphate dehydrogenase catalyzes an important energy-yielding step in carbohydrate metabolism, the reversible oxidative phosphorylation of glyceraldehyde-3-phosphate in the presence of inorganic phosphate and nicotinamide adenine dinucleotide (NAD). The enzyme exists as a tetramer of identical chains. A GAPD pseudogene has been mapped to Xp21-p11 and 15 GAPD-like loci have been identified.

### GARS: glycyl-tRNA synthetase

Aminoacyl-tRNA synthetases are a class of enzymes that charge tRNAs with their cognate amino acids. Glycyl-tRNA synthetase is an (alpha)2 dimer which belongs to the class II family of tRNA synthetases. It has been shown to be a target of autoantibodies in the human autoimmune diseases, polymyositis or dermatomyositis.

### GBE1: glucan (1,4-alpha-), branching enzyme 1 (glycogen branching enzyme, Andersen disease, glycogen storage disease type IV)

This monomeric enzyme functions in glycogen symthesis by catalyzing the formation of alpha 1,6- glucosidic linkages. It is most highly expressed in liver and muscle. Deficiency can result in glycogen storage disease IV (Andersen's disease).

### GP6: glycoprotein VI (platelet)

Platelet glycoprotein VI; member of the paired Ig-like receptor family.

### GPR-55

Member of the G protein-coupled receptor family.

### GPRC5C: G protein-coupled receptor, family C, group 5, member C

The protein encoded by this gene is a member of the type 3 G protein-coupled receptor family. Members of this superfamily are characterized by a signature 7-transmembrane domain motif. The specific function of this protein is unknown; however, this protein may mediate the cellular effects of retinoic acid on the G protein signal transduction cascade. Alternative splicing in the 5' UTR of this gene results in two transcript variants.

### 3-hydroxy-3-methylglutaryl coenzyme A synthase

3-hydroxy-3-methylglutaryl-Coenzyme A synthase; functions in the first step in ketogenesis.

### HK1: hexokinase 1

Hexokinases phosphorylate glucose to produce glucose-6-phosphate, thus committing glucose to the glycolytic pathway. This gene encodes a ubiquitous form of hexokinase which localizes to the outer membrane of mitochondria. Mutations in this gene have been associated with hemolytic anemia due to hexokinase deficiency. Alternative splicing of this gene results in five transcript variants which encode different isoforms, some of which are tissue-specific. Each isoform has a distinct N-terminus; the remainder of the protein is identical among all the isoforms. A sixth transcript variant has been described, but due to the presence of several stop codons, it is not thought to encode a protein.

### HLA-B associated transcript 3 (BAT3)

A cluster of genes, BAT1-BAT5, has been localized in the vicinity of the genes for TNF alpha and TNF beta. These genes are all within the human major histocompatibility complex class III region. The protein encoded by this gene is a nuclear protein. It has been implicated in the control of apoptosis and regulating heat shock protein. There are three alternatively spliced transcript variants described for this gene.

### HMGCL: 3-hydroxymethyl-3-methylglutaryl-Coenzyme A lyase (hydroxymethylglutaricaciduria)

3-Hydroxy-3-methylglutaryl coenzyme A lyase; cleaves 3-OH-3-methylglutaryl CoA to acetoacetic acid and acetyl CoA.

### HNF4A: hepatocyte nuclear factor 4, alpha

Nuclear hormone receptor transcription factor; regulates liver specific gene expression.

### Chromosome 12 BAC RP11-13J12

### Cathepsin B

Cathepsin B; lysosomal cysteine (thiol) protease that cleaves APP.

### Chromosome 5 clone CTD-2235C13

### Chromosome 7 clone RP11-351B12

### Cytochrome P450 3A locus

The CYP3A locus includes all the known members of the 3A subfamily of the cytochrome P450 superfamily of genes. These genes encode monooxygenases which catalyze many reactions involved in drug metabolism and synthesis of cholesterol, steroids and other lipids. The CYP3A cluster consists of four genes, CYP3A43, CYP3A4, CYP3A7 and CYP3A5. The region also contains two pseudogenes, CYP3A5P1 and CYP3A5P2, as well as several extra exons which may or may not be included in transcripts produced from this region. Previously another CYP3A member, CYP3A3, was thought to exist; however, it is now thought that this sequence represents a transcript variant of CYP3A4.

### ITGB3

The ITGB3 protein product is the integrin beta chain beta 3. Integrins are integral cell-surface proteins composed of an alpha chain and a beta chain. A given chain may combine with multiple partners resulting in different integrins. Integrin beta 3 is found along with the alpha IIb chain in platelets. Integrins are known to participate in cell adhesion as well as cell-surface mediated signalling.

### Methionine adenosyltransferase alpha subunit gene fragment.

MAT1A encodes methionine adenosyltransferase I (alpha isoform). MATIA catalyzes the formation of S-adenosylmethionine from methionine and ATP. Both the beta and alpha isoforms may be encoded by MATIA. Methionine adenosyltransferase deficiency is known to be caused by recessive as well as dominant mutations, the latter identified in autosomal dominant persistant hyper-methioninemia.

### Homo sapiens PAC clone RP1-102K2 from 22q12.1-qter

### Homo sapiens partial ZNF202 gene for zinc finger protein homolog, exon 4

Zinc-finger protein 202 may repress genes involved in lipid metabolism; contains zinc fingers.

### Homo sapiens vHNF1-C mRNA

Hepatocyte Nuclear Factor 1.

### Human 2.5 kb mRNA for cytoskeletal tropomyosin TM30(nm)

### Human c-kit gene.

KIT encodes the human homolog of the proto-oncogene c-kit. C-kit was first identified as the cellular homolog of the feline sarcoma viral oncogene v-kit. KIT is a type 3 transmembrane receptor for MGF (mast cell growth factor, also known as stem cell factor). Mutations in KIT are associated with gastrointestinal stromal tumors, mast cell disease, acute myelogenous lukemia, and piebaldism.

### Human coagulation factor VII (F7) gene exon 1 and factor X (F10) gene, exon 1.

This gene encodes coagulation factor VII which is a vitamin K-dependent factor essential for hemostasis. This factor circulates in the blood in a zymogen form, and is converted to an active form by either factor IXa, factor Xa, factor XIIa, or thrombin by minor proteolysis. Upon activation of the factor VII, a heavy chain containing a catalytic domain and a light chain containing 2 EGF-like domains are generated, and two chains are held together by a disulfide bond. In the presence of factor III and calcium ions, the activated factor then further activates the coagulation cascade by converting factor IX to factor IXa and/or factor X to factor Xa. Alternative splicing of this gene results in 2 transcripts. Defects in this gene can cause coagulopathy.

### Human cytochrome P450 (CYP1A2) gene, exons 1 and 2.

This gene, CYP1A2, encodes a member of the cytochrome P450 superfamily of enzymes. The cytochrome P450 proteins are monooxygenases which catalyze many reactions involved in drug metabolism and synthesis of cholesterol, steroids and other lipids. The protein encoded by this gene localizes to the endoplasmic reticulum and its expression is induced by some polycyclic aromatic hydrocarbons (PAHs), some of which are found in cigarette smoke. The enzyme's endogenous substrate is unknown; however, it is able to metabolize some PAHs to carcinogenic intermediates. Other xenobiotic substrates for this enzyme include caffeine, aflatoxin B1, and acetaminophen. The transcript from this gene contains four Alu sequences flanked by direct repeats in the 3' untranslated region. A related family member, CYP1A1, is located approximately 25 kb away from CYP1A2 on chromosome .

### Human multidrug resistance-associated protein mRNA

See ABCC1.

### Human succinyl CoA:3-oxoacid CoA transferase precursor (OXCT) mRNA

The mitochondrial matrix enzyme 3-oxoacid CoA transferase is homodimeric. It is a key enzyme in the extrahepatic utilization of ketone bodies, catalyzing the reversible transfer of coenzyme A from succinyl-CoA to acetoacetate, a necessary step in ketolytic energy production. Deficiencies can result in intermittent ketoacidosis.

### Human T-lymphoma invasion and metastasis inducing TIAM1 protein (TIAM1) mRNA

Member of the GDP-GTP exchange factor family of proteins; modulates the activity of Rho-like proteins; has a Dbl homology and pleckstrin homology domains.

### IL10: interleukin 10

Interleukin 10 (cytokine synthesis inhibitory factor); functions as a specific chemotactic factor for CD8+T cells.

### IL17R: interleukin 17 receptor

Highly similar to murine I117r; may play a role in T cell activation and induction of IL-2 (I12).

### IL3: interleukin 3 (colony-stimulating factor, multiple)

Interleukin-3 (colony-stimulating factor); plays a role in hematopoeisis; member of a family of growth factors.

### IL6: interleukin 6 (interferon, beta 2)

Interleukin 6 (interferon-beta 2); induces the maturation of B cells into immunoglobulin-secreting cells.

### IL8RA: interleukin 8 receptor, alpha

Interleukin 8 receptor alpha; G protein-coupled receptor that mediates neutrophil chemotaxis and binds interleukin 8 (IL8).

### INHBC: inhibin, beta C

This gene encodes the beta C chain of inhibin, a member of the TGF-beta superfamily. This subunit forms heterodimers with beta A and beta B subunits. Inhibins and activins, also members of the TGF-beta superfamily, are hormones with opposing actions and are involved in hypothalamic, pituitary, and gonadal hormone secretion, as well as growth and differentiation of various cell types.

### ITGAL: integrin, alpha L (antigen CD11A (p180), lymphocyte function-associated antigen 1; alpha polypeptide)

ITGAL encodes the integrin alpha L chain. Integrins are heterodimeric integral membrane proteins composed of an alpha chain and a beta chain. This I-domain containing alpha integrin combines with the beta 2 chain (ITGB2) to form the integrin lymphocyte function-associated antigen-1 (LFA-1), which is expressed on all leukocytes. LFA-1 plays a central role in leukocyte intercellular adhesion through interactions with its ligands, ICAMs 1-3 (intercellular adhesion molecules 1 through 3), and also functions in lymphocyte costimulatory signaling.

### ITGB2: integrin, beta 2 (antigen CD18 (p95), lymphocyte function-associated antigen 1; macrophage antigen 1 (mac-1) beta subunit)

The ITGB2 protein product is the integrin beta chain beta 2. Integrins are integral cell-surface proteins composed of an alpha chain and a beta chain. A given chain may combine with multiple partners resulting in different integrins. For example, beta 2 combines with the alpha L chain to form the integrin LFA-1, and combines with the alpha M chain to form the integrin Mac-1. Integrins are known to participate in cell adhesion as well as cell-surface mediated signalling.

### KCNQ1: potassium voltage-gated channel, KQT-like subfamily, member 1

KCNQ1 encodes the K+ channel subunit responsible for the delayed-rectifier K+ current in cardiac myocytes. The delayed-rectifier channel is completed by the protein encoded by KCNE1. Mutations in KCNQ1 cause inherited long-QT syndrome.

### LAMA3: laminin, alpha 3 (nicein (150kD), kalinin (165kD), BM600 (150kD), epilegrin)

Laminins are basement membrane components thought to mediate the attachment, migration and organization of cells into tissues during embryonic development by interacting with other extracellular matrix components. The protein encoded by this gene is the alpha-3 chain of laminin 5, which is a complex glycoprotein composed of three subunits (alpha, beta, and gamma). Laminin 5 is thought to be involved in cell adhesion, signal transduction and differentiation of keratinocytes. Mutations in this gene have been identified as the cause of Herlitz type junctional epidermolysis bullosa. Alternative splicing has been observed at this locus but the full-length nature of these variants has not been determined.

### LAMR1: laminin receptor 1 (67kD, ribosomal protein SA)

Laminins, a family of extracellular matrix glycoproteins, are the major non-collagenous constituent of basement membranes. They have been implicated in a wide variety of biological processes including cell adhesion, differentiation, migration, signaling, neurite outgrowth and metastasis. Many of the effects of laminin are mediated through interactions with cell surface receptors. These receptors include members of the integrin family, as well as non-integrin laminin-binding proteins. This gene encodes a high-affinity, non-integrin family, laminin receptor 1. This receptor has been variously called 67 kD laminin receptor, 37 kD laminin receptor precursor (37LRP) and p40 ribosome-associated protein. The amino acid sequence of laminin receptor 1 is highly conserved through evolution, suggesting a key biological function. It has been observed that the level of the laminin receptor transcript is higher in colon carcinoma tissue and lung cancer cell line than their normal counterparts. Also, there is a correlation between the upregulation of this polypeptide in cancer cells and their invasive and metastatic phenotype. Multiple copies of this gene exist, however, most of them are pseudogenes thought to have arisen from retropositional events..

### LDLR: low density lipoprotein receptor (familial hypercholesterolemia)

The low density lipoprotein receptor (LDLR) gene family consists of cell surface proteins involved in receptor-mediated endocytosis of specific ligands. Low density lipoprotein (LDL) is normally bound at the cell membrane and taken into the cell ending up in lysosomes where the protein is degraded and the cholesterol is made available for repression of microsomal enzyme 3-hydroxy-3-methylglutaryl coenzyme A (HMG CoA) reductase, the rate-limiting step in cholesterol synthesis. At the same time, a reciprocal stimulation of cholesterol ester synthesis takes place. Mutations in the LDL receptor (LDLR) gene cause the autosomal dominant disorder, familial hypercholesterolemia.

### LGALS7: lectin, galactoside-binding, soluble, 7 (galectin 7)

The galectins are a family of beta-galactoside-binding proteins implicated in modulating cell-cell and cell-matrix interactions. Differential and in situ hybridizations indicate that this lectin is specifically expressed in keratinocytes. It is expressed at all stages of epidermal differentiation (i.e., in basal and suprabasal layers). It is moderately repressed by retinoic acid. The protein was found mainly in stratified squamous epithelium. The antigen localized to basal keratinocytes, although it was also found, albeit at lower levels, in the suprabasal layers where it concentrated to areas of cell-to-cell contact. The cellular localization and its striking down-regulation in cultured keratinocytes imply a role in cell-cell and/or cell-matrix interactions necessary for normal growth control.

### LIMK1: LIM domain kinase 1

There are approximately 40 known eukaryotic LIM proteins, so named for the LIM domains they contain. LIM domains are highly conserved cysteine-rich structures containing 2 zinc fingers. Although zinc fingers usually function by binding to DNA or RNA, the LIM motif probably mediates protein-protein interactions. LIM kinase-1 and LIM kinase-2 belong to a small subfamily with a unique combination of 2 N-terminal LIM motifs and a C-terminal protein kinase domain. LIMK1 is likely to be a component of an intracellular signaling pathway and may be involved in brain development. LIMK1 hemizygosity is implicated in the impaired visuospatial constructive cognition of Williams syndrome. Two splice variant have been identified.

### LMNB2: lamin B2

Lamin B2; member of a family of structural nuclear envelope proteins.

### LPL: lipoprotein lipase

LPL encodes lipoprotein lipase, which is expressed in heart, muscle, and adipose tissue. LPL functions as a homodimer, and has the dual functions of triglyceride hydrolase and ligand/bridging factor for receptor-mediated lipoprotein uptake. Severe mutations that cause LPL deficiency result in type I hyperlipoproteinemia, while less extreme mutations in LPL are linked to many disorders of lipoprotein metabolism.

### LRP8: low density lipoprotein receptor-related protein 8, apolipoprotein e receptor

This gene encodes an apolipoprotein E receptor, a member of the low density lipoprotein receptor (LDLR) family. Apolipoprotein E is a small lipophilic plasma protein and a component of lipoproteins such as chylomicron remnants, very low density lipoprotein (VLDL), and high density lipoprotein (HDL). The apolipoprotein E receptor is involved in cellular recognition and internalization of these lipoproteins. Alternative splicing generates three transcript variants for this gene; additional variants have been described, but their full length nature has not been determined.

### LSS: lanosterol synthase (2,3-oxidosqualene-lanosterol cyclase)

Lanosterol synthase ((S)-2,3-epoxysqualene mutase); catalyzes the cyclization of (S)-2,3-oxidosqualene; forms lanosterol during sterol biosynthesis.

### LTA: lymphotoxin alpha (TNF superfamily, member 1)

Lymphotoxin alpha, a member of the tumor necrosis factor family, is a cytokine produced by lymphocytes. LTA is highly inducible, secreted, and exists as homotrimeric molecule. LTA forms heterotrimers with lymphotoxin-beta which anchors lymphotoxin-alpha to the cell surface. LTA mediates a large variety of inflammatory, immunostimulatory, and antiviral responses. LTA is also involved in the formation of secondary lymphoid organs during development and plays a role in apoptosis.

### MAOA: monoamine oxidase A

MAOA encodes monoamine oxidase A, an enzyme that degrades amine neurotransmitters, such as dopamine, norepinephrine, and serotonin. Deficiency of this enzyme results in Brunner syndrome.

### MARCKS: myristoylated alanine-rich protein kinase C substrate

The protein encoded by this gene is a substrate for protein kinase C. It is localized to the plasma membrane and is an actin filament crosslinking protein. Phosphorylation by protein kinase C or binding to calcium-calmodulin inhibits its association with actin and with the plasma membrane, leading to its presence in the cytoplasm. The protein is thought to be involved in cell motility, phagocytosis, membrane trafficking and mitogenesis.

### MCL1: myeloid cell leukemia sequence 1 (BCL2-related)

Similar to BCL2.

### MCP: membrane cofactor protein (CD46, trophoblast-lymphocyte cross- reactive antigen)

Membrane cofactor protein; acts as the receptor for the measles virus, may be involved in the regulation of complement activation; contains SCRs.

### METTL1: methyltransferase-like 1

This gene is an ortholog of the S. cerevisiae YDL201w gene, which is predicted to encode a methyltransferase. The gene product contains a conserved S-adenosyl-methionine-binding motif, which is typical of a methyltransferase. Alternative splice variants encoding different protein isoforms and transcript variants utilizing alternative polyA sites have been described in the literature.

### MLLT3: myeloid/lymphoid or mixed-lineage leukemia (trithorax homolog, Drosophila);

Serine and proline rich protein, has a nuclear targeting sequence.

### MTHFD1: methylenetetrahydrofolate dehydrogenase (NADP+ dependent), methenyltetrahydrofolate cyclohydrolase, formyltetrahydrofolate synthetase

This gene encodes a protein that possesses three distinct enzymatic activities, 5,10-methylenetetrahydrofolate dehydrogenase, 5,10-methenyltetrahydrofolate cyclohydrolase and 10-formyltetrahydrofolate synthetase. Each of these activities catalyzes one of three sequential reactions in the interconversion of 1-carbon derivatives of tetrahydrofolate, which are substrates for methionine, thymidylate, and de novo purine syntheses. The trifunctional enzymatic activities are conferred by two major domains, an aminoterminal portion containing the dehydrogenase and cyclohydrolase activities and a larger synthetase domain.

### MTMR2 myotubularin related protein 2 (MTMR2)

This gene is a member of the myotubularin family and encodes a putative tyrosine phosphatase. Mutations in this gene are a cause of Charcot-Marie-Tooth disease type 4B, an autosomal recessive demyelinating neuropathy. This gene utilizes multiple polyA signals, only one of which has been determined.

### Muscle specific serine kinase (MSSK1; serine/threonine kinase 23, STK23),

Highly similar to SRPK2; may be protein kinase for SR family of RNA splicing factors; contains a kinase domain.

### MVD: mevalonate (diphospho) decarboxylase

The enzyme mevalonate pyrophosphate decarboxylase catalyzes the conversion of mevalonate pyrophosphate into isopentenyl pyrophosphate in one of the early steps in cholesterol biosynthesis. It decarboxylates and dehydrates its substrate while hydrolyzing ATP.

### MYH11: myosin, heavy polypeptide 11, smooth muscle

The protein encoded by this gene is a smooth muscle myosin belonging to the myosin heavy chain family. The gene product is a subunit of a hexameric protein that consists of 2 heavy chain subunits and 2 pairs of non-identical light chain subunits. It functions as a major contractile protein, converting chemical energy into mechanical energy through the hydrolysis of ATP. The gene encoding a human ortholog of rat NUDE1 is transcribed from the reverse strand of MYH11 gene, and its 3' end overlaps with that of the latter. The pericentric inversion of chromosome 16 [inv(16)(p13q22)] produces a chimeric transcript consisting of the first 165 residues from the N terminus of core-binding factor beta in a fusion with the C-terminal portion of the smooth muscle myosin heavy chain. This chromosomal rearrangement is associated with acute myeloid leukemia of the M4Eo subtype. Alternative splicing generates isoforms that are differentially expressed, with ratios changing during muscle cell maturation. Additional splice variants have been described but their full-length nature has not been determined..

### MYH7: myosin, heavy polypeptide 7, cardiac muscle, beta

MYH7 encodes the cardiac muscle beta (or slow) isoform of myosin. Changes in the relative abundance of MYH7 and MYH6 (the alpha, or fast, isoform of cardiac myosin heavy chain) correlate with the contractile velocity of cardiac muscle. Mutations in MYH7 are associated with familial hypertrophic cardiomyopathy.

### NADH dehydrogenase (ubiquinone) 1, alpha subcomplex, 4 (9kD, MLRQ), NDUFA4

Subunit of NADH-ubiquinone oxidoreductase (complex I); transports electrons from NADH to ubiquinone.

### NADH-UBIQUINONE OXIDOREDUCTASE CHAIN 5 (EC 1.6.5.3).

Subunit of NADH-ubiquinone oxidoreductase (complex I); transports electrons from NADH to ubiquinone.

### NDUFA9: NADH dehydrogenase (ubiquinone) 1 alpha subcomplex, 9 (39kD)

### NGFB: nerve growth factor, beta polypeptide

Nerve growth factor beta; has roles in neuronal differentiation and survival.

### NGFR: nerve growth factor receptor (TNFR superfamily, member 16)

Nerve growth factor receptor contains an extracellular domain containing four 40-amino acid repeats with 6 cysteine residues at conserved positions followed by a serine/threonine-rich region, a single transmembrane domain, and a 155-amino acid cytoplasmic domain. The cysteine-rich region contains the nerve growth factor binding domain.

### NID2: nidogen 2

Nidogen-2; basement membrane protein,.

### HSU15552: acidic 82 kDa protein mRNA

### Nonmuscle type myosin heavy chain 9 (MYH9)

Non-muscle myosin heavy chain 9; motor protein that provides force for muscle contraction, cytokinesis and phagocytosis; contains an ATPase head domain and a rod-like tail domain.

### NPC1: Niemann-Pick disease, type C1

NPC1 was identified as the gene that when mutated, results in Niemann-Pick C disease. NPC1 encodes a putative integral membrane protein containing motifs consistent with a role in intracellular transport of cholesterol to post-lysosomal destinations.

### Nth endonuclease III-like 1 (NTHL1)

Endonuclease; excises damaged pyrimidines.

### NUCB2: nucleobindin 2

Nucleobindin 2; may bind DNA and calcium; has DNA-binding and EF-hand domains, and a leucine-zipper.

### nuclear receptor subfamily 1, group I, member 2 (NR1I2)

The gene product belongs to the nuclear receptor superfamily, members of which are transcription factors characterized by a ligand-binding domain and a DNA-binding domain. The encoded protein is a transcriptional regulator of the cytochrome P450 gene CYP3A4, binding to the response element of the CYP3A4 promoter as a heterodimer with the 9-cis retinoic acid receptor RXR. It is activated by a range of compounds that induce CYP3A4, including dexamethasone and rifampicin. The gene product contains a zinc finger domain. Three alternatively spliced transcripts that encode different isoforms have been described, one of which encodes two products through the use of alternative translation initiation codons. Additional transcript variants derived from alternative promoter usage, alternative splicing, and/or alternative polyadenylation exist, but they have not been fully described.

### OGDH: oxoglutarate (alpha-ketoglutarate) dehydrogenase (lipoamide)

Alpha-ketoglutarate or 2-oxoglutarate dehydrogenase; helps convert a-ketoglutarate to succinyl coenzyme A in Krebs cycle.

### OXCT: 3-oxoacid CoA transferase

The mitochondrial matrix enzyme 3-oxoacid CoA transferase is homodimeric. It is a key enzyme in the extrahepatic utilization of ketone bodies, catalyzing the reversible transfer of coenzyme A from succinyl-CoA to acetoacetate, a necessary step in ketolytic energy production. Deficiencies can result in intermittent ketoacidosis.

### P2RY1: purinergic receptor P2Y, G-protein coupled, 1

Purinergic receptor P2Y1, a G protein-coupled receptor; mediates responses to ATP and increases inositol phosphate levels.

### PCCA: propionyl Coenzyme A carboxylase, alpha polypeptide

PCCA encodes the alpha subunit of the heterodimeric mitochondrial enzyme Propionyl-CoA carboxylase. PCCA encodes the biotin-binding region of this enzyme. Mutations in either PCCA or PCCB (encoding the beta subunit) lead to an enzyme deficiency result in propionic acidemia.

### PDGFB: platelet-derived growth factor beta polypeptide (simian sarcoma viral (v-sis) oncogene homolog)

The protein encoded by this gene is a member of the platelet-derived growth factor family. The four members of this family are mitogenic factors for cells of mesenchymal origin and are characterized by a motif of eight cysteines. This gene product can exist either as a homodimer or as a heterodimer with the platelet-derived growth factor alpha polypeptide, where the dimers are connected by disulfide bonds. Mutations in this gene are associated with meningioma. Reciprocal translocations between chromosomes 22 and 7, at sites where this gene and that for COL1A1 are located, are associated with a particular type of skin tumor called dermatofibro-sarcoma protuberans resulting from unregulated expression of growth factor. Two splice variants have been identified for this gene.

### PERIOD CIRCADIAN PROTEIN 2 (KIAA0347)

This gene is a member of the Period family of genes and is expressed in a circadian pattern in the suprachiasmatic nucleus, the primary circadian pacemaker in the mammalian brain. Genes in this family encode components of the circadian rhythms of locomotor activity, metabolism, and behavior. Circadian expression in the suprachiasmatic nucleus continues in constant darkness, and a shift in the light/dark cycle evokes a proportional shift of gene expression in the suprachiasmatic nucleus. The specific function of this gene is not yet known.

### Peroxisome proliferative activated receptor, delta (PPARD)

Peroxisome proliferator-activated receptor delta is a member of the steroid hormone receptor superfamily.

### PGM5: phosphoglucomutase 5

Phosphoglucomutase-related (aciculin) putative structural protein; interacts with the cytoskeletal proteins dystrophin and utrophin.

### PLA2G3: phospholipase A2, group III

Group III secreted phospholipase A2; calcium-dependent, displays a preference for phosphatidylglycerol over phosphatidylcholine.

### PLA2G4C: phospholipase A2, group IVC (cytosolic, calcium-independent)

Group IVC calcium-independent phospholipase a2; hydrolyzes the phospholipid sn-2 ester bond; member of the phospholipase family.

### PLA2G6: phospholipase A2, group VI (cytosolic, calcium-independent)

Cytosolic calcium-independent phospholipase_a2; hydrolyzes the phospholipid sn-2 ester bond; member of the phospholipase family.

### PMVK: phosphomevalonate kinase

Phosphomevalonate kinase; converts mevalonate-5-phosphate to mevalonate-5-diphosphate.

### PNMT: phenylethanolamine N-methyltransferase

Phenylethanolamine N-methyltransferase; converts norepinephrine to epinephrine.

### PON1: paraoxonase 1

### PON2: paraoxonase 2

Paraoxonase/arylesterase 2; possibly functions in protecting low density lipoprotein against oxidative modification; member of a family that hydrolyzes toxic organo-phosphates.

### PPARA: peroxisome proliferative activated receptor, alpha

Peroxisome proliferators are a diverse group of chemicals which include hypolipidemic drugs, herbicides, leukotriene antagonists, and plasticizers, and are so called because they induce an increase in the size and number of peroxisomes. Peroxisomes are subcellular organelles found in plants and animals, and contain enzymes for respiration, cholesterol and lipid metabolism. Infact, the fibrate class of hypolipidemic drugs is used to reduce triglycerides and cholesterol in patients with hyperlipidemia, a major risk factor for coronary heart disease. The action of peroxisome proliferators is thought to be mediated via specific receptors belonging to the steroid hormone receptor superfamily, called PPARs. Thus far, four closely related subtypes, alpha, beta, gamma and delta, have been identified. The subtype PPAR-alpha, encoded by PPARA, is a nuclear transcription factor. Upon activation by peroxisome proliferators, it modulates the expression of target genes involved in lipid metabolism, suggesting a role for PPAR-alpha in lipid homeostasis..

### PPARG: peroxisome proliferative activated receptor, gamma

The protein encoded by this gene is a member of the peroxisome proliferator-activated receptor (PPAR) subfamily of nuclear receptors. PPARs form heterodimers with retinoid X receptors (RXRs) and these heterodimers regulate transcription of various genes. Three subtypes of PPARs are known: PPAR-alpha, PPAR-delta, and PPAR-gamma. The protein encoded by this gene is PPAR-gamma and is a regulator of adipocyte differentiation. Additionally, PPAR-gamma has been implicated in the pathology of numerous diseases including obesity, diabetes, atherosclerosis and cancer. Multiple transcript variants that use alternate promoters and splicing have been identified for this gene. At least three of these variants encode the same isoform.

### PPM1A: protein phosphatase 1A (formerly 2C), magnesium-dependent, alpha isoform

Magnesium- or manganese-dependent alpha protein phosphatase 1A; regulates cell stress responses.

### PROBABLE G PROTEIN-COUPLED RECEPTOR APJ.

### PTPRA: protein tyrosine phosphatase, receptor type, A

The protein encoded by this gene is a member of the protein tyrosine phosphatase (PTP) family. PTPs are known to be signaling molecules that regulate a variety of cellular processes including cell growth, differentiation, mitotic cycle, and oncogenic transformation. This PTP contains an extracellular domain, a single transmembrane segment and two tandem intracytoplasmic catalytic domains, and thus represents a receptor-type PTP. This PTP has been shown to dephosphorylate and activate Src family tyrosine kinases, and is implicated in the regulation of integrin signaling, cell adhesion and proliferation. Three alternatively spliced variants of this gene, which encode two distinct isoforms, have been reported.

### PYGM: phosphorylase, glycogen; muscle (McArdle syndrome, glycogen storage disease type V)

Muscle glycogen phosphorylase.

### RTN1: reticulon 1

### RXRA: retinoid X receptor, alpha

Retinoid X receptors (RXRs) and retinoic acid receptors (RARs), are nuclear receptors that mediate the biological effects of retinoids by their involvement in retinoic acid-mediated gene activation. These receptors exert their action by binding, as homodimers or heterodimers, to specific sequences in the promoters of target genes and regulating their transcription. The protein encoded by this gene is a member of the steroid and thyroid hormone receptor superfamily of transcriptional regulators.

### RXRB: retinoid X receptor, beta

Retinoid X receptor beta; binds to and serves as transcriptional coactivator for retinoic acid.

### SCA1: spinocerebellar ataxia 1 (olivopontocerebellar ataxia 1, autosomal dominant, ataxin 1)

The autosomal dominant cerebellar ataxias (ADCA) are a heterogeneous group of neurodegenerative disorders characterized by progressive degeneration of the cerebellum, brain stem and spinal cord. Clinically, ADCA has been divided into three groups: ADCA types I-III. ADCAI is genetically heterogeneous, with five genetic loci, designated spinocerebellar ataxia (SCA) 1, 2, 3, 4 and 6, being assigned to five different chromosomes. ADCAII, which always presents with retinal degeneration (SCA7), and ADCAIII often referred to as the 'pure' cerebellar syndrome (SCA5), are most likely homogeneous disorders. Several SCA genes have been cloned and shown to contain CAG repeats in their coding regions. ADCA is caused by the expansion of the CAG repeats, producing an elongated polyglutamine tract in the corresponding protein. The expanded repeats are variable in size and unstable, usually increasing in size when transmitted to successive generations. The function of the ataxins is not known. The SCA1 locus has been mapped to chromosome 6, and it has been determined that the diseased allele contains 41-81 CAG repeats, compared to 6-39 in the normal allele. Several transcript variants of SCA1 in the 5' UTR have been described; however, their full-length nature is not known..

### SDF1: stromal cell-derived factor 1

Stromal cell-derived factor 1; lymphocyte chemoattractant that signals through the receptor CXCR4.

### SERPINA5: serine (or cysteine) proteinase inhibitor, clade A (alpha-1 antiproteinase, antitrypsin), member 5

Protein C inhibitor (plasminogen activator inhibitor III); may be a serine protease inhibitor; member of the serpin family of serine protease inhibitors.

### SERPINH1: serine (or cysteine) proteinase inhibitor, clade H (heat shock protein 47), member 1, (collagen binding protein 1)

Colligin; collagen-binding protein; Similar to HSPs and to serpin family serine protease inhibitors.

### SLC21A6: solute carrier family 21 (organic anion transporter), member 6

Organic anion transporter.

### SLC27A1: solute carrier family 27 (fatty acid transporter), member 1

### SULT1A2: sulfotransferase family, cytosolic, 1A, phenol-preferring, member 2

Phenol-metabolizing sulfotransferase 2; sulfonates simple planar phenols.

### THBS3: Thrombospondin 3

Thrombospondin 3 binds heparin and calcium; similar to murine Thbs3

### TBP: TATA box binding protein

TATA box binding protein, component of the TFIID complex; functions in the initiation of mRNA synthesis and basal transcription.

### TBXA2R: thromboxane A2 receptor

Thromboxane A2 receptor (prostaglandin H2 receptor); G protein-coupled receptor, activates Ca2+-activated chloride channels; stimulates platelet aggregation and smooth muscle constriction.

### TCF2: transcription factor 2, hepatic; LF-B3; variant hepatic nuclear factor

TCF2 encodes transcription factor 2, a liver-specific factor of the homeobox-containing basic helix-turn-helix family. The TCF2 protein is believed to form heterodimers with another liver-specific member of this transcription factor family, TCF1; depending on the TCF2 isoform, the result may be to activate or inhibit transcription of target genes. Mutation of TCF2 that disrupts normal function has been identified as the cause of MODY5 (Maturity-Onset of Diabetes, Type 5). A third human transcript variant is believed to exist based on such a variant in the rat: however, to date such an mRNA species has not been isolated.

### TETRAN: tetracycline transporter-like protein

Similar to E. coli tetracycline resistance efflux protein.

### TGFB1: transforming growth factor, beta 1 (Camurati-Engelmann disease)

Transforming growth factor-beta 1; regulates cell proliferation, differentiation, and apoptosis.

### TGFB2: transforming growth factor, beta 2

Transforming growth factor-beta 2 (glioblastoma-derived T cell suppressor factor); suppresses IL2 - dependent growth of T cells; member of a family of cytokines that transmits signals through transmembrane serine/threonine kinases.

### TGFB3: transforming growth factor, beta 3

Transforming growth factor-beta 3; transmits signals through transmembrane serine/threonine kinases, may be required for normal development of the lung and palate; member of family of cytokines, very strongly similar to murine Tgfb3.

### THPO: thrombopoietin (myeloproliferative leukemia virus oncogene ligand, megakaryocyte growth and development factor)

Thrombopoietin; binds to c-Mpl receptor and regulates megakaryocyte development.

### TNFAIP2: tumor necrosis factor, alpha-induced protein 2

Secreted by vascular endothelium, expression is induced by tumor necrosis factor alpha, interleukin-1 beta, and lipopolysaccharide.

### TRAP1: heat shock protein 75

Heat shock protein 75; binds and refolds denatured RB1 during M phase and after heat shock; member of the HSP90 family of molecular chaperones.

### TRIP10: thyroid hormone receptor interactor 10

Similar to the non-kinase domains of FER and Fes/Fps tyrosine kinases; binds to activated Cdc42 and may regulate actin cytoskeleton; contains an SH3 domain.

### TXN: thioredoxin

Thioredoxin; has dithiol-disulfide oxidoreductase activity.

### USP6: ubiquitin specific protease 6 (Tre-2 oncogene)

Ubiquitin specific protease 6 (Tre-2 oncogene); cleaves ubiquitin from proteins, has predicted nucleic acid-binding properties.

### UTRN: utrophin (homologous to dystrophin)

This gene shares both structural and functional similarities with the dystrophin gene. It contains an actin-binding N-terminus, a triple coiled-coil repeat central region, and a C-terminus that consists of protein-protein interaction motifs which interact with dystroglycan protein components. The protein encoded by this gene is located at the neuromuscular synapse and myotendinous junctions, where it participates in post-synaptic membrane maintenance and acetylcholine receptor clustering. Mouse studies suggest that this gene may serve as a functional substitute for the dystrophin gene and therefore, may serve as a potential therapeutic alternative to muscular dystrophy which caused by mutations in the dystrophin gene. Alternative splicing of the utrophin gene has been described; however, the full-length nature of these variants has not yet been determined.

### VEGF: vascular endothelial growth factor

Vascular endothelial growth factor; induces endothelial cell proliferation and vascular permeability.

### VEGFB: vascular endothelial growth factor B

Vascular endothelial growth factor B; involved in angiogenesis and endothelial cell growth.

### WISP1: WNT1 inducible signaling pathway protein 1

This gene encodes a member of the WNT1 inducible signaling pathway (WISP) protein subfamily, which belongs to the connective tissue growth factor (CTGF) family. WNT1 is a member of a family of cysteine-rich, glycosylated signaling proteins that mediate diverse developmental processes. The CTGF family members are characterized by four conserved cysteine-rich domains: insulin-like growth factor-binding domain, von Willebrand factor type C module, thrombospondin domain and C-terminal cystine knot-like domain. This gene may be downstream in the WNT1 signaling pathway that is relevant to malignant transformation. It is expressed at a high level in fibroblast cells, and overexpressed in colon tumors. The encoded protein binds to decorin and biglycan, two members of a family of small leucine-rich proteoglycans present in the extracellular matrix of connective tissue, and possibly prevents the inhibitory activity of decorin and biglycan in tumor cell proliferation. It also attenuates p53-mediated apoptosis in response to DNA damage through activation of the Akt kinase. It is 83% identical to the mouse protein at the amino acid level. Alternative splicing of this gene generates 2 transcript variants..

### XDH: xanthene dehydrogenase

Xanthine dehydrogenase belongs to the group of molybdenum-containing hydroxylases involved in the oxidative metabolism of purines. The enzyme is a homodimer. Xanthine dehydrogenase can be converted to xanthine oxidase by reversible sulfhydryl oxidation or by irreversible proteolytic modification. Defects in xanthine dehydrogenase cause xanthinuria, may contribute to adult respiratory stress syndrome, and may potentiate influenza infection through an oxygen metabolite-dependent mechanism..

### YAP1: Yes-associated protein 1, 65 kD

Yes-associated protein; binds to the proto-oncoprotein Yes; has a WW domain.

### PROCR: protein C receptor, endothelial (EPCR)

Endothelial Protein C receptor; binds protein C in a calcium-dependent manner; member of the CD1/major histocompatibility complex superfamily.

### STX1A: syntaxin 1A (brain)

Syntaxin 1A (brain); involved in intracellular transport and neurotransmitter release.

As SNPs are linked to other SNPs in neighboring genes on a chromosome (Linkage Disequilibrium) those SNPs could also be used as marker SNPs. In a recent publication it was shown that SNPs are linked over 100 kb in some cases more than 150 kb (Reich D.E. et al. Nature 411, 199-204, 2001). Hence SNPs lying in regions neighbouring PA SNPs could be linked to the latter and by this being a diagnostic marker. These associations could be performed as described for the gene polymorphism in methods.

### Definitions

For convenience, the meaning of certain terms and phrases employed in the specification, examples, and appended claims are provided below. Moreover, the definitions by itself are intended to explain a further background of the invention.

The term "allele", which is used interchangeably herein with "allelic variant" refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides. An allele of a gene can also be a form of a gene containing a mutation.

The term "allelic variant of a polymorphic region of a gene" refers to a region of a gene having one of several nucleotide sequences found in that region of the gene in other individuals.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, though preferably less than 25% identity, with one of the sequences of the present invention.

The term "a homologue of a nucleic acid" refers to a nucleic acid having a nucleotide sequence having a certain degree of homology with the nucleotide sequence of the nucleic acid or complement thereof. A homologue of a double stranded nucleic acid having SEQ ID NO. X is intended to include nucleic acids having a nucleotide sequence which has a certain degree of homology with SEQ ID NO. X or with the complement thereof. Preferred homologous of nucleic acids are capable of hybridizing to the nucleic acid or complement thereof.

The term "interact" as used herein is meant to include detectable interactions between molecules, such as can be detected using, for example, a hybridization assay.

The term interact is also meant to include "binding" interactions between molecules. Interactions may be, for example, protein-protein, protein-nucleic acid, protein-small molecule or small molecule-nucleic acid in nature.

The term "intronic sequence" or "intronic nucleotide sequence" refers to the nucleotide sequence of an intron or portion thereof.

The term "isolated" as used herein with respect to nucleic acids, such as DNA or RNA, refers to molecules separated from other DNAs or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized.

Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

The term "lipid" shall refer to a fat or fat-like substance that is insoluble in polar solvents such as water. The term "lipid" is intended to include true fats (e.g. esters of fatty acids and glycerol); lipids (phospholipids, cerebrosides, waxes); sterols (cholesterol, ergosterol) and lipoproteins (e.g. HDL, LDL and VLDL).

The term "locus" refers to a specific position in a chromosome. For example, a locus of a gene refers to the chromosomal position of the gene.

The term "modulation" as used herein refers to both up-regulation, (i.e., activation or stimulation), for example by agonizing, and down-regulation (i.e. inhibition or suppression), for example by antagonizing of a bioactivity (e.g. expression of a gene).

The term "molecular structure" of a gene or a portion thereof refers to the structure as defined by the nucleotide content (including deletions, substitutions, additions of one or more nucleotides), the nucleotide sequence, the state of methylation, and/or any other modification of the gene or portion thereof.

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous (for that gene) subject, the mutation is said to be co-dominant.

As used herein, the term "nucleic acid" refers to polynucleotides such as deoxyribonucleic acid (DNA), and, where appropriate, ribonucleic acid (RNA). The term should also be understood to include, as equivalents, derivatives, variants and analogs of either RNA or DNA made from nucleotide analogs, including peptide nucleic acids (PNA), morpholino oligonucleotides (J. Summerton and D. Weller, Antisense and Nucleic Acid Drug Development 7:187 (1997)) and, as applicable to the embodiment being described, single (sense or antisense) and double-stranded polynucleotides. Deoxyribonucleotides include deoxyadenosine, deoxycytidine, deoxyguanosine, and deoxythymidine. For purposes of clarity, when referring herein to a nucleotide of a nucleic acid, which can be DNA or an RNA, the term "adenosine", "cytidine", "guanosine", and "thymidine" are used. It is understood that if the nucleic acid is RNA, a nucleotide having a uracil base is uridine.

The term "nucleotide sequence complementary to the nucleotide sequence set forth in SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a non-coding strand. When referring to double stranded nucleic acids, the complement of a nucleic acid having SEQ ID NO. x refers to the complementary strand of the strand having SEQ ID NO. x or to any nucleic acid having the nucleotide sequence of the complementary strand of SEQ ID NO. x. When referring to a single stranded nucleic acid having the nucleotide sequence SEQ ID NO. x, the complement of this nucleic acid is a nucleic acid having a nucleotide sequence which is complementary to that of SEQ ID NO. x. The nucleotide sequences and complementary sequences thereof are always given in the 5' to 3' direction. The term "complement" and "reverse complement" are used interchangeably herein.

The term "operably linked" is intended to mean that the promoter is associated with the nucleic acid in such a manner as to facilitate transcription of the nucleic acid.

The term "polymorphism" refers to the coexistence of more than one form of a gene or portion thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

To describe a "polymorphic site" in a nucleotide sequence often there is used an "ambiguity code" that stands for the possible variations of nucleotides in one site. The list of ambiguity codes is summarized in the following table:

| Ambiguity | Codes |
|---|---|
| (IUPAC Nomenclature) | |
| B | c/g/t |
| D | a/g/t |
| H | a/c/t |
| K | g/t |
| M | a/c |
| N | a/c/g/t |
| R | a/g |
| S | c/g |
| V | a/c/g |
| W | a/t |
| Y | c/t |

So, for example, a "R" in a nucleotide sequence means that either an "a" or a "g" could be at that position.

The terms "protein", "polypeptide" and "peptide" are used interchangeably herein when referring to a gene product.

A "regulatory element", also termed herein "regulatory sequence is intended to include elements which are capable of modulating transcription from a basic promoter and include elements such as enhancers and silencers. The term "enhancer", also referred to herein as "enhancer element", is intended to include regulatory elements capable of increasing, stimulating, or enhancing transcription from a basic promoter. The term "silencer", also referred to herein as "silencer element" is intended to include regulatory elements capable of decreasing, inhibiting, or repressing transcription from a basic promoter. Regulatory elements are typically present in 5' flanking regions of genes. However, regulatory elements have also been shown to be present in other regions of a gene, in particular in introns. Thus, it is possible that genes have regulatory elements located in introns, exons, coding regions, and 3' flanking sequences. Such regulatory elements are also intended to be encompassed by the present invention and can be identified by any of the assays that can be used to identify regulatory elements in 5' flanking regions of genes.

The term "regulatory element" further encompasses "tissue specific" regulatory elements, i.e., regulatory elements which effect expression of the selected DNA sequence preferentially in specific cells (e.g., cells of a specific tissue). gene expression occurs preferentially in a specific cell if expression in this cell type is significantly higher than expression in other cell types. The term "regulatory element" also encompasses non-tissue specific regulatory elements, i.e., regulatory elements which are active in most cell types. Furthermore, a regulatory element can be a constitutive regulatory element, i.e., a regulatory element which constitutively regulates transcription, as opposed to a regulatory element which is inducible, i.e., a regulatory element which is active primarily in response to a stimulus. A stimulus can be, e.g., a molecule, such as a hormone, cytokine, heavy metal, phorbol ester, cyclic AMP (cAMP), or retinoic acid.

Regulatory elements are typically bound by proteins, e.g., transcription factors. The term "transcription factor" is intended to include proteins or modified forms thereof, which interact preferentially with specific nucleic acid sequences, i.e., regulatory elements, and which in appropriate conditions stimulate or repress transcription. Some transcription factors are active when they are in the form of a monomer. Alternatively, other transcription factors are active in the form of a dimer consisting of two identical proteins or different proteins (heterodimer). Modified forms of transcription factors are intended to refer to transcription factors having a post-translational modification, such as the attachment of a phosphate group. The activity of a transcription factor is frequently modulated by a post-translational modification. For example, certain transcription factors are active only if they are phosphorylated on specific residues. Alternatively, transcription factors can be active in the absence of phosphorylated residues and become inactivated by phosphorylation. A list of known transcription factors and their DNA binding site can be found, e.g., in public databases, e.g., TFMATRIX Transcription Factor Binding Site Profile database.

As used herein, the term "specifically hybridizes" or "specifically detects" refers to the ability of a nucleic acid molecule of the invention to hybridize to at least approximately 6, 12, 20, 30, 40, 50, 60, 70, 80, 90, 100, 110, 120, 130 or 140 consecutive nucleotides of either strand of a gene.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

"Adverse drug reaction" (ADR) as used herein refers to an appreciably harmful or unpleasant reaction, resulting from an intervention related to the use of a medicinal product, which predicts hazard from future administration and warrants prevention or specific treatment, or alteration of the dosage regimen, or withdrawal of the product. In it's most severe form an ADR might lead to the death of an individual.

The term "Drug Response" is intended to mean any response that a patient exhibits upon drug administration. Specifically drug response includes beneficial, i.e. desired drug effects, ADR or no detectable reaction at all. More specifically the term drug response could also have a qualitative meaning, i.e. it embraces low or high beneficial effects, respectively and mild or severe ADR, respectively. The term "Statin Response" as used herein refers to drug response after statin administration. An individual drug response includes also a good or bad metabolizing of the drug, meaning that "bad metabolizers" accumulate the drug in the body and by this could show side effects of the drug due to accumulative overdoses.

"Candidate gene" as used herein includes genes that can be assigned to either normal cardiovascular function or to metabolic pathways that are related to onset and/or progression of cardiovascular diseases.

With regard to drug response the term "candidate gene" includes genes that can be assigned to distinct phenotypes regarding the patient's response to drug administration. Those phenotypes may include patients who benefit from relatively small amounts of a given drug (high responders) or patients who need relatively high doses in order to obtain the same benefit (low responders). In addition those phenotypes may include patients who can tolerate high doses of a medicament without exhibiting ADR, or patients who suffer from ADR even after receiving only low doses of a medicament.

As neither the development of cardiovascular diseases nor the patient's response to drug administration is completely understood, the term "candidate gene" may also comprise genes with presently unknown function.

"PA SNP" (phenotype associated SNP) refers to a polymorphic site which shows a significant association with a patients phenotype (healthy, diseased, low or high responder, drug tolerant, ADR prone, etc.)

"PA gene" (phenotype associated gene) refers to a genomic locus harbouring a PA SNP, irrespective of the actual function of this gene locus.

PA gene polypeptide refers to a polypeptide encoded at least in part by a PA gene.

The term "Haplotype" as used herein refers to a group of two or more SNPs that are functionally and/or spatially linked. I.e. haplotypes define groups of SNPs that lie inside genes belonging to identical (or related metabolic) pathways and/or lie on the same chromosome. Haplotypes are expected to give better predictive/diagnostic information than a single SNP

The term "statin" is intended to embrace all inhibitors of the enzyme 3-hydroxy-3-methylglutaryl coenzyme A (HMG-CoA) reductase. Statins specifically inhibit the enzyme HMG-CoA reductase which catalyzes the rate limiting step in cholesterol biosynthesis. Known statins are Atorvastatin, Cerivastatin, Fluvastatin, Lovastatin, Pravastatin and Simvastatin.

### Methods for Assessing Cardiovascular Status

The present invention provides diagnostic methods for assessing cardiovascular status in a human individual. Cardiovascular status as used herein refers to the physiological status of an individual's cardiovascular system as reflected in one or more markers or indicators. Status markers include without limitation clinical measurements such as, e.g., blood pressure, electrocardiographic profile, and differentiated blood flow analysis as well as measurements of LDL- and HDL-Cholesterol levels, other lipids and other well established clinical parameters that are standard in the art. Status markers according to the invention include diagnoses of one or more cardiovascular syndromes, such as, e.g., hypertension, acute myocardial infarction, silent myocardial infarction, stroke, and atherosclerosis. It will be understood that a diagnosis of a cardiovascular syndrome made by a medical practitioner encompasses clinical measurements and medical judgement. Status markers according to the invention are assessed using conventional methods well known in the art. Also included in the evaluation of cardiovascular status are quantitative or qualitative changes in status markers with time, such as would be used, e.g., in the determination of an individual's response to a particular therapeutic regimen.

The methods are carried out by the steps of:
(i) determining the sequence of one or more polymorphic positions within one, several or all of the genes listed in Examples or other genes mentioned in this file in the individual to establish a polymorphic pattern for the individual; and
(ii) comparing the polymorphic pattern established in (i) with the polymorphic patterns of humans exhibiting different markers of cardiovascular status. The polymorphic pattern of the individual is, preferably, highly similar and, most preferably, identical to the polymorphic pattern of individuals who exhibit particular status markers, cardiovascular syndromes, and/or particular patterns of response to therapeutic interventions. Polymorphic patterns may also include polymorphic positions in other genes which are shown, in combination with one or more polymorphic positions in the genes listed in the Examples, to correlate with the presence of particular status markers. In one embodiment, the method involves comparing an individual's polymorphic pattern with polymorphic patterns of individuals who have been shown to respond positively or negatively to a particular therapeutic regimen. Therapeutic regimen as used herein refers to treatments aimed at the elimination or amelioration of symptoms and events associated cardiovascular disease. Such treatments include without limitation one or more of alteration in diet, lifestyle, and exercise regimen; invasive and noninvasive surgical techniques such as atherectomy, angioplasty, and coronary bypass surgery; and pharmaceutical interventions, such as administration of ACE inhibitors, angiotensin II receptor antagonists, diuretics, alpha-adrenoreceptor antagonists, cardiac glycosides, phosphodiesterase inhibitors, beta-adrenoreceptor antagonists, calcium channel blockers, HMG-CoA reductase inhibitors, imidazoline receptor blockers, endothelin receptor blockers, organic nitrites, and modulators of protein function of genes listed in the Examples. Interventions with pharmaceutical agents not yet known whose activity correlates with particular polymorphic patterns associated with cardiovascular disease are also encompassed. It is contemplated, for example, that patients who are candidates for a particular therapeutic regimen will be screened for polymorphic patterns that correlate with responsivity to that particular regimen.

In a preferred embodiment, the method involves comparing an individual's polymorphic pattern with polymorphic patterns of individuals who exhibit or have exhibited one or more markers of cardiovascular disease, such as, e.g., elevated LDL-Cholesterol levels, high blood pressure, abnormal electrocardiographic profile, myocardial infarction, stroke, or atherosclerosis.

In another embodiement, the method involves comparing an individual's polymorphic pattern with polymorphic patterns of individuals who exhibit or have exhibited one or more drug related phenotypes, such as, e.g., low or high drug response, or adverse drug reactions.

In practicing the methods of the invention, an individual's polymorphic pattern can be established by obtaining DNA from the individual and determining the sequence at predetermined polymorphic positions in the genes such as those described in this file.

The DNA may be obtained from any cell source. Non-limiting examples of cell sources available in clinical practice include blood cells, buccal cells, cervicovaginal cells, epithelial cells from urine, fetal cells, or any cells present in tissue obtained by biopsy. Cells may also be obtained from body fluids, including without limitation blood, saliva, sweat, urine, cerebrospinal fluid, feces, and tissue exudates at the site of infection or inflammation. DNA is extracted from the cell source or body fluid using any of the numerous methods that are standard in the art. It will be understood that the particular method used to extract DNA will depend on the nature of the source.

### Diagnostic and Prognostic Assays

The present invention provides methods for determining the molecular structure of at least one polymorphic region of a gene, specific allelic variants of said polymorphic region being associated with cardiovascular disease. In one embodiment, determining the molecular structure of a polymorphic region of a gene comprises determining the identity of the allelic variant. A polymorphic region of a gene, of which specific alleles are associated with cardiovascular disease can be located in an exon, an intron, at an intron/exon border, or in the promoter of the gene.

The invention provides methods for determining whether a subject has, or is at risk, of developing a cardiovascular disease. Such disorders can be associated with an aberrant gene activity, e.g., abnormal binding to a form of a lipid, or an aberrant gene protein level. An aberrant gene protein level can result from an aberrant transcription or post-transcriptional regulation. Thus, allelic differences in specific regions of a gene can result in differences of gene protein due to differences in regulation of expression. In particular, some of the identified polymorphisms in the human gene may be associated with differences in the level of transcription, RNA maturation, splicing, or translation of the gene or transcription product.

In preferred embodiments, the methods of the invention can be characterized as comprising detecting, in a sample of cells from the subject, the presence or absence of a specific allelic variant of one or more polymorphic regions of a gene. The allelic differences can be: (i) a difference in the identity of at least one nucleotide or (ii) a difference in the number of nucleotides, which difference can be a single nucleotide or several nucleotides.

A preferred detection method is allele specific hybridization using probes overlapping the polymorphic site and having about 5, 10, 20, 25, or 30 nucleotides around the polymorphic region. Examples of probes for detecting specific allelic variants of the polymorphic region located in intron X are probes comprising a nucleotide sequence set forth in any of SEQ ID NO. X. In a preferred embodiment of the invention, several probes capable of hybridizing specifically to allelic variants are attached to a solid phase support, e.g., a "chip". Oligonucleotides can be bound to a solid support by a variety of processes, including lithography. For example a chip can hold up to 250,000 oligonucleotides (GeneChip, Affymetrix). Mutation detection analysis using these chips comprising oligonucleotides, also termed "DNA probe arrays" is described e.g., in Cronin et al. (1996) Human Mutation 7:244 and in Kozal et al. (1996) Nature Medicine 2:753. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment. For example, the identity of the allelic variant of the nucleotide polymorphism of nucleotide A or G at position 33 of Seq ID 1 (baySNP179) and that of other possible polymorphic regions can be determined in a single hybridization experiment.

In other detection methods, it is necessary to first amplify at least a portion of a gene prior to identifying the allelic variant. Amplification can be performed, e.g., by PCR and/or LCR, according to methods known in the art. In one embodiment, genomic DNA of a cell is exposed to two PCR primers and amplification for a number of cycles sufficient to produce the required amount of amplified DNA. In preferred embodiments, the primers are located between 40 and 350 base pairs apart. Preferred primers for amplifying gene fragments of genes of this file are listed in Table 2 in the Examples.

Alternative amplification methods include: self sustained sequence replication (Guatelli, J. C. et al., 1990, Proc. Natl. Acad. Sci. U.S.A. 87:1874-1878), transcriptional amplification system (Kwoh, D. Y. et al., 1989, Proc. Natl. Acad. Sci. U.S.A. 86:1173-1177), Q-Beta Replicase (Lizardi, P. M. et al., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In one embodiment, any of a variety of sequencing reactions known in the art can be used to directly sequence at least a portion of a gene and detect allelic variants, e.g., mutations, by comparing the sequence of the sample sequence with the corresponding wild-type (control) sequence. Exemplary sequencing reactions include those based on techniques developed by Maxam and Gilbert (Proc. Natl Acad Sci USA (1977) 74:560) or Sanger (Sanger et al (1977) Proc. Nat. Acad. Sci 74:5463). It is also contemplated that any of a variety of automated sequencing procedures may be utilized when performing the subject assays (Biotechniques (1995) 19:448), including sequencing by mass spectrometry (see, for example, U.S. Pat. No. 5,547,835 and international patent application Publication Number WO 94/16101, entitled DNA Sequencing by Mass Spectrometry by H. Koster; U.S. Pat. No. 5,547,835 and international patent application Publication Number WO 94/21822 entitled "DNA Sequencing by Mass Spectrometry Via Exonuclease Degradation" by H. Koster), and U.S. Pat. No. 5,605,798 and International Patent Application No. PCT/US96/03651 entitled DNA Diagnostics Based on Mass Spectrometry by H. Koster; Cohen et al. (1996) Adv Chromatogr 36:127-162; and Griffin et al. (1993) Appl Biochem Biotechnol 38:147-159). It will be evident to one skilled in the art that, for certain embodiments, the occurrence of only one, two or three of the nucleic acid bases need be determined in the sequencing reaction. For instance, A-track or the like, e.g., where only one nucleotide is detected, can be carried out.

Yet other sequencing methods are disclosed, e.g., in U.S. Pat. No. 5,580,732 entitled "Method of DNA sequencing employing a mixed DNA-polymer chain probe" and U.S. Pat. No. 5,571,676 entitled "Method for mismatch-directed in vitro DNA sequencing".

In some cases, the presence of a specific allele of a gene in DNA from a subject can be shown by restriction enzyme analysis. For example, a specific nucleotide polymorphism can result in a nucleotide sequence comprising a restriction site which is absent from the nucleotide sequence of another allelic variant.

In other embodiments, alterations in electrophoretic mobility is used to identify the type of gene allelic variant. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control nucleic acids are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In another preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the identity of an allelic variant of a polymorphic region is obtained by analyzing the movement of a nucleic acid comprising the polymorphic region in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:1275).

Examples of techniques for detecting differences of at least one nucleotide between 2 nucleic acids include, but are not limited to, selective oligonucleotide hybridization, selective amplification, or selective primer extension. For example, oligonucleotide probes may be prepared in which the known polymorphic nucleotide is placed centrally (allele-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace et al. (1979) Nucl. Acids Res. 6:3543). Such allele specific oligonucleotide hybridization techniques may be used for the simultaneous detection of several nucleotide changes in different polymorphic regions of gene. For example, oligonucleotides having nucleotide sequences of specific allelic variants are attached to a hybridizing membrane and this membrane is then hybridized with labeled sample nucleic acid. Analysis of the hybridization signal will then reveal the identity of the nucleotides of the sample nucleic acid.

Alternatively, allele specific amplification technology which depends on selective PCR amplification may be used. Oligonucleotides used as primers for specific amplification may carry the allelic variant of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238; Newton et al. (1989) Nucl. Acids Res. 17:2503). This technique is also termed "PROBE" for Probe Oligo Base Extension. In addition it may be desirable to introduce a novel restriction site in the region of the mutation to create cleavage-based detection (Gasparini et al (1992) Mol. Cell Probes 6:1).

In another embodiment, identification of the allelic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990). In this method, PCR is used to achieve the exponential amplification of target DNA, which is then detected using OLA.

Several techniques based on this OLA method have been developed and can be used to detect specific allelic variants of a polymorphic region of a gene. For example, U.S. Pat. No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996)Nucleic Acids Res 24: 3728), OLA combined with PCR permits typing of two alleles in a single microtiter well. By marking each of the allele-specific primers with a unique hapten, i.e. digoxigenin and fluorescein, each LA reaction can be detected by using hapten specific antibodies that are labeled with different enzyme reporters, alkaline phosphatase or horseradish peroxidase. This system permits the detection of the two alleles using a high throughput format that leads to the production of two different colors.

The invention further provides methods for detecting single nucleotide polymorphisms in a gene. Because single nucleotide polymorphisms constitute sites of variation flanked by regions of invariant sequence, their analysis requires no more than the determination of the identity of the single nucleotide present at the site of variation and it is unnecessary to determine a complete gene sequence for each patient. Several methods have been developed to facilitate the analysis of such single nucleotide polymorphisms.

In one embodiment, the single base polymorphism can be detected by using a specialized exonuclease-resistant nucleotide, as disclosed, e.g., in Mundy, C. R. (U.S. Pat. No. 4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA TM is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990), Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA TM in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

For determining the identity of the allelic variant of a polymorphic region located in the coding region of a gene, yet other methods than those described above can be used. For example, identification of an allelic variant which encodes a mutated gene protein can be performed by using an antibody specifically recognizing the mutant protein in, e.g., immunohistochemistry or immunoprecipitation. Antibodies to wild-type gene protein are described, e.g., in Acton et al. (1999) Science 271:518 (antimouse gene antibody cross-reactive with human gene). Other antibodies to wild-type gene or mutated forms of gene proteins can be prepared according to methods known in the art. Alternatively, one can also measure an activity of an gene protein, such as binding to a lipid or lipoprotein. Binding assays are known in the art and involve, e.g., obtaining cells from a subject, and performing binding experiments with a labeled lipid, to determine whether binding to the mutated form of the receptor differs from binding to the wild-type of the receptor.

If a polymorphic region is located in an exon, either in a coding or non-coding region of the gene, the identity of the allelic variant can be determined by determining the molecular structure of the mRNA, pre-mRNA, or cDNA. The molecular structure can be determined using any of the above described methods for determining the molecular structure of the genomic DNA, e.g., sequencing and SSCP.

The methods described herein may be performed, for example, by utilizing pre-packaged diagnostic kits, such as those described above, comprising at least one probe or primer nucleic acid described herein, which may be conveniently used, e.g., to determine whether a subject has or is at risk of developing a disease associated with a specific gene allelic variant.

Sample nucleic acid for using in the above-described diagnostic and prognostic methods can be obtained from any cell type or tissue of a subject. For example, a subject's bodily fluid (e.g. blood) can be obtained by known techniques (e.g. venipuncture) or from human tissues like heart (biopsies, transplanted organs). Alternatively, nucleic acid tests can be performed on dry samples (e.g. hair or skin). Fetal nucleic acid samples for prenatal diagnostics can be obtained from maternal blood as described in International Patent Application No.WO91/07660 to Bianchi. Alternatively, amniocytes or chorionic villi may be obtained for performing prenatal testing.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, PCR in situ hybridization: protocols and applications, Raven Press, New York).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

In practicing the present invention, the distribution of polymorphic patterns in a large number of individuals exhibiting particular markers of cardiovascular status or drug response is determined by any of the methods described above, and compared with the distribution of polymorphic patterns in patients that have been matched for age, ethnic origin, and/or any other statistically or medically relevant parameters, who exhibit quantitatively or qualitatively different status markers. Correlations are achieved using any method known in the art, including nominal logistic regression, chi square tests or standard least squares regression analysis. In this manner, it is possible to establish statistically significant correlations between particular polymorphic patterns and particular cardiovascular statuses (given in p values). It is further possible to establish statistically significant correlations between particular polymorphic patterns and changes in cardiovascular status or drug response such as, would result, e.g., from particular treatment regimens. In this manner, it is possible to correlate polymorphic patterns with responsivity to particular treatments.

In another embodiment of the present invention two or more polymorphic regions are combined to define so called 'haplotypes' . Haplotypes are groups of two or more SNPs that are functionally and/or spatially linked. It is possible to combine SNPs that are disclosed in the present invention either with each other or with additional polymorphic regions to form a haplotype. Haplotypes are expected to give better predictive/diagnostic information than a single SNP.
In a preferred embodiment of the present invention a panel of SNPs/haplotypes is defined that predicts the risk for CVD or drug response. This predictive panel is then used for genotyping of patients on a platform that can genotype multiple SNPs at the same time (Multiplexing). Preferred platforms are e.g. gene chips (Affymetrix) or the Luminex LabMAP reader. The subsequent identification and evaluation of a patient's haplotype can then help to guide specific and individualized therapy.

For example the present invention can identify patients exhibiting genetic polymorphisms or haplotypes which indicate an increased risk for adverse drug reactions. In that case the drug dose should be lowered in a way that the risk for ADR is diminished. Also if the patient's response to drug administration is particularly high (or the patient is badly metabolizing the drug), the drug dose should be lowered to avoid the risk of ADR.
In turn if the patient's response to drug administration is low (or the patient is a particularly high metabolizer of the drug), and there is no evident risk of ADR, the drug dose should be raised to an efficacious level.
It is self evident that the ability to predict a patient's individual drug response should affect the formulation of a drug, i.e. drug formulations should be tailored in a way that they suit the different patient classes (low/high responder, poor/good metabolizer, ADR prone patients). Those different drug formulations may encompass different doses of the drug, i.e. the medicinal products contains low or high amounts of the active substance. In another embodiement of the invention the drug formulation may contain additional substances that facilitate the beneficial effects and/or diminish the risk for ADR (Folkers et al. 1991, US Pat. 5,316,765).

### Isolated Polymorphic Nucleic Acids, Probes, and Vectors

The present invention provides isolated nucleic acids comprising the polymorphic positions described herein for human genes; vectors comprising the nucleic acids; and transformed host cells comprising the vectors. The invention also provides probes which are useful for detecting these polymorphisms.

In practicing the present invention, many conventional techniques in molecular biology, microbiology, and recombinant DNA, are used. Such techniques are well known and are explained fully in, for example, Sambrook et al., 1989, Molecular Cloning: A Laboratory Manual, Second Edition, Cold Spring Harbor Laboratory Press, Cold Spring Harbor, New York; DNA Cloning: A Practical Approach, Volumes I and II, 1985 (D. N. Glover ed.); Oligonucleotide Synthesis, 1984, (M. L.Gait ed.); Nucleic Acid Hybridization, 1985, (Hames and Higgins); Ausubel et al., Current Protocols in Molecular Biology, 1997, (John Wiley and Sons); and Methods in Enzymology Vol. 154 and Vol. 155 (Wu and Grossman, and Wu, eds., respectively).

Insertion of nucleic acids (typically DNAs) comprising the sequences in a functional surrounding like full length cDNA of the present invention into a vector is easily accomplished when the termini of both the DNAs and the vector comprise compatible restriction sites. If this cannot be done, it may be necessary to modify the termini of the DNAs and/or vector by digesting back single-stranded DNA overhangs generated by restriction endonuclease cleavage to produce blunt ends, or to achieve the same result by filling in the single-stranded termini with an appropriate DNA polymerase.

Alternatively, any site desired may be produced, e.g., by ligating nucleotide sequences (linkers) onto the termini. Such linkers may comprise specific oligonucleotide sequences that define desired restriction sites. Restriction sites can also be generated by the use of the polymerase chain reaction (PCR). See, e.g., Saiki et al., 1988, Science 239:48. The cleaved vector and the DNA fragments may also be modified if required by homopolymeric tailing.

The nucleic acids may be isolated directly from cells or may be chemically synthesized using known methods. Alternatively, the polymerase chain reaction (PCR) method can be used to produce the nucleic acids of the invention, using either chemically synthesized strands or genomic material as templates. Primers used for PCR can be synthesized using the sequence information provided herein and can further be designed to introduce appropriate new restriction sites, if desirable, to facilitate incorporation into a given vector for recombinant expression.

The nucleic acids of the present invention may be flanked by native gene sequences, or may be associated with heterologous sequences, including promoters, enhancers, response elements, signal sequences, polyadenylation sequences, introns, 5'- and 3'-noncoding regions, and the like. The nucleic acids may also be modified by many means known in the art. Non-limiting examples of such modifications include methylation, "caps", substitution of one or more of the naturally occurring nucleotides with an analog, internucleotide modifications such as, for example, those with uncharged linkages (e.g., methyl phosphonates, phosphotriesters, phosphoro-amidates, carbamates, morpholines etc.) and with charged linkages (e.g., phosphorothioates, phosphorodithioates, etc.). Nucleic acids may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. PNAs are also included. The nucleic acid may be derivatized by formation of a methyl or ethyl phosphotriester or an alkyl phosphoramidate linkage. Furthermore, the nucleic acid sequences of the present invention may also be modified with a label capable of providing a detectable signal, either directly or indirectly. Exemplary labels include radioisotopes, fluorescent molecules, biotin, and the like.

The invention also provides nucleic acid vectors comprising the gene sequences or derivatives or fragments thereof of genes described in the Examles. A large number of vectors, including plasmid and fungal vectors, have been described for replication and/or expression in a variety of eukaryotic and prokaryotic hosts, and may be used for gene therapy as well as for simple cloning or protein expression. Non-limiting examples of suitable vectors include without limitation pUC plasmids, pET plasmids (Novagen, Inc., Madison, Wis.), or pRSET or pREP (Invitrogen, San Diego, Calif.), and many appropriate host cells, using methods disclosed or cited herein or otherwise known to those skilled in the relevant art. The particular choice of vector/host is not critical to the practice of the invention.

Suitable host cells may be transformed/transfected/infected as appropriate by any suitable method including electroporation, CaCl₂ mediated DNA uptake, fungal or viral infection, microinjection, microprojectile, or other established methods. Appropriate host cells included bacteria, archebacteria, fungi, especially yeast, and plant and animal cells, especially mammalian cells. A large number of transcription initiation and termination regulatory regions have been isolated and shown to be effective in the transcription and translation of heterologous proteins in the various hosts. Examples of these regions, methods of isolation, manner of manipulation, etc. are known in the art. Under appropriate expression conditions, host cells can be used as a source of recombinantly produced peptides and polypeptides encoded by genes of the Examples. Nucleic acids encoding peptides or polypeptides from gene sequences of the Examples may also be introduced into cells by recombination events. For example, such a sequence can be introduced into a cell and thereby effect homologous recombination at the site of an endogenous gene or a sequence with substantial identity to the gene. Other recombination-based methods such as non-homologous recombinations or deletion of endogenous genes by homologous recombination may also be used.

In case of proteins that form heterodimers or other multimers, both or all subunits have to be expressed in one system or cell.

The nucleic acids of the present invention find use as probes for the detection of genetic polymorphisms and as templates for the recombinant production of normal or variant peptides or polypeptides encoded by genes listed in the Examples.

Probes in accordance with the present invention comprise without limitation isolated nucleic acids of about 10-100 bp, preferably 15-75 bp and most preferably 17-25 bp in length, which hybridize at high stringency to one or more of the polymorphic sequences disclosed herein or to a sequence immediately adjacent to a polymorphic position. Furthermore, in some embodiments a full-length gene sequence may be used as a probe. In one series of embodiments, the probes span the polymorphic positions in genes disclosed herein. In another series of embodiments, the probes correspond to sequences immediately adjacent to the polymorphic positions.

### Polymorphic Polypeptides and Polymorphism-Specific Antibodies

The present invention encompasses isolated peptides and polypeptides encoded by genes listed in the Examples comprising polymorphic positions disclosed herein. In one preferred embodiment, the peptides and polypeptides are useful screening targets to identify cardiovascular drugs. In another preferred embodiments, the peptides and polypeptides are capable of eliciting antibodies in a suitable host animal that react specifically with a polypeptide comprising the polymorphic position and distinguish it from other polypeptides having a different sequence at that position.

Polypeptides according to the invention are preferably at least five or more residues in length, preferably at least fifteen residues. Methods for obtaining these polypeptides are described below. Many conventional techniques in protein biochemistry and immunology are used. Such techniques are well known and are explained in Immunochemical Methods in Cell and Molecular Biology, 1987 (Mayer and Waler, eds; Academic Press, London); Scopes, 1987, Protein Purification: Principles and Practice, Second Edition (Springer-Verlag, N.Y.) and Handbook of Experimental Immunology, 1986, Volumes I-IV (Weir and Blackwell eds.).

Nucleic acids comprising protein-coding sequences can be used to direct the ITT recombinant expression of polypeptides encoded by genes disclosed herein in intact cells or in cell-free translation systems. The known genetic code, tailored if desired for more efficient expression in a given host organism, can be used to synthesize oligonucleotides encoding the desired amino acid sequences. The polypeptides may be isolated from human cells, or from heterologous organisms or cells (including, but not limited to, bacteria, fungi, insect, plant, and mammalian cells) into which an appropriate protein-coding sequence has been introduced and expressed. Furthermore, the polypeptides may be part of recombinant fusion proteins.

Peptides and polypeptides may be chemically synthesized by commercially available automated procedures, including, without limitation, exclusive solid phase synthesis, partial solid phase methods, fragment condensation or classical solution synthesis. The polypeptides are preferably prepared by solid phase peptide synthesis as described by Merrifield, 1963, J. Am. Chem. Soc. 85:2149.

Methods for polypeptide purification are well-known in the art, including, without limitation, preparative disc-gel electrophoresis, isoelectric focusing, HPLC, reversed-phase HPLC, gel filtration, ion exchange and partition chromatography, and countercurrent distribution. For some purposes, it is preferable to produce the polypeptide in a recombinant system in which the protein contains an additional sequence tag that facilitates purification, such as, but not limited to, a polyhistidine sequence. The polypeptide can then be purified from a crude lysate of the host cell by chromatography on an appropriate solid-phase matrix. Alternatively, antibodies produced against peptides encoded by genes disclosed herein, can be used as purification reagents. Other purification methods are possible.

The present invention also encompasses derivatives and homologues of the polypeptides. For some purposes, nucleic acid sequences encoding the peptides may be altered by substitutions, additions, or deletions that provide for functionally equivalent molecules, i.e., function-conservative variants. For example, one or more amino acid residues within the sequence can be substituted by another amino acid of similar properties, such as, for example, positively charged amino acids (arginine, lysine, and histidine); negatively charged amino acids (aspartate and glutamate); polar neutral amino acids; and non-polar amino acids.

The isolated polypeptides may be modified by, for example, phosphorylation, sulfation, acylation, or other protein modifications. They may also be modified with a label capable of providing a detectable signal, either directly or indirectly, including, but not limited to, radioisotopes and fluorescent compounds.

The present invention also encompasses antibodies that specifically recognize the polymorphic positions of the invention and distinguish a peptide or polypeptide containing a particular polymorphism from one that contains a different sequence at that position. Such polymorphic position-specific antibodies according to the present invention include polyclonal and monoclonal antibodies. The antibodies may be elicited in an animal host by immunization with peptides encoded by genes disclosed herein or may be formed by in vitro immunization of immune cells. The immunogenic components used to elicit the antibodies may be isolated from human cells or produced in recombinant systems. The antibodies may also be produced in recombinant systems programmed with appropriate antibody-encoding DNA. Alternatively, the antibodies may be constructed by biochemical reconstitution of purified heavy and light chains. The antibodies include hybrid antibodies (i.e., containing two sets of heavy chain/light chain combinations, each of which recognizes a different antigen), chimeric antibodies (i.e., in which either the heavy chains, light chains, or both, are fusion proteins), and univalent antibodies (i.e., comprised of a heavy chain/light chain complex bound to the constant region of a second heavy chain). Also included are Fab fragments, including Fab' and F(ab).sub.2 fragments of antibodies. Methods for the production of all of the above types of antibodies and derivatives are well-known in the art and are discussed in more detail below. For example, techniques for producing and processing polyclonal antisera are disclosed in Mayer and Walker, 1987, Immunochemical Methods in Cell and Molecular Biology, (Academic Press, London). The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., Schreier et al., 1980, Hybridoma Techniques; U.S. Pat. Nos. 4,341,761; 4,399,121; 4,427,783; 4,444,887; 4,466,917; 4,472,500; 4,491,632; and 4,493,890. Panels of monoclonal antibodies produced against peptides encoded by genes disclosed herein can be screened for various properties; i.e. for isotype, epitope affinity, etc.

The antibodies of this invention can be purified by standard methods, including but not limited to preparative disc-gel electrophoresis, isoelectric focusing, HPLC, reversed-phase HPLC, gel filtration, ion exchange and partition chromatography, and countercurrent distribution. Purification methods for antibodies are disclosed, e.g., in The Art of Antibody Purification, 1989, Amicon Division, W. R. Grace & Co. General protein purification methods are described in Protein Purification: Principles and Practice, R. K. Scopes, Ed., 1987, Springer-Verlag, New York, N.Y.

Methods for determining the immunogenic capability of the disclosed sequences and the characteristics of the resulting sequence-specific antibodies and immune cells are well-known in the art. For example, antibodies elicited in response to a peptide comprising a particular polymorphic sequence can be tested for their ability to specifically recognize that polymorphic sequence, i.e., to bind differentially to a peptide or polypeptide comprising the polymorphic sequence and thus distinguish it from a similar peptide or polypeptide containing a different sequence at the same position.

### Kits

As set forth herein, the invention provides diagnostic methods, e.g., for determining the identity of the allelic variants of polymorphic regions present in the gene loci of genes disclosed herein, wherein specific allelic variants of the polymorphic region are associated with cardiovascular diseases. In a preferred embodiment, the diagnostic kit can be used to determine whether a subject is at risk of developing a cardiovascular disease. This information could then be used, e.g., to optimize treatment of such individuals.

In preferred embodiments, the kit comprises a probe or primer which is capable of hybridizing to a gene and thereby identifying whether the gene contains an allelic variant of a polymorphic region which is associated with a risk for cardiovascular disease. The kit preferably further comprises instructions for use in diagnosing a subject as having, or having a predisposition, towards developing a cardiovascular disease. The probe or primers of the kit can be any of the probes or primers described in this file.

Preferred kits for amplifying a region of a gene comprising a polymorphic region of interest comprise one, two or more primers.

### Antibody-based diagnostic methods and kits:

The invention also provides antibody-based methods for detecting polymorphic patterns in a biological sample. The methods comprise the steps of: (i) contacting a sample with one or more antibody preparations, wherein each of the antibody preparations is specific for a particular polymorphic form of the proteins encoded by genes disclosed herein, under conditions in which a stable antigen-antibody complex can form between the antibody and antigenic components in the sample; and (ii) detecting any antigen-antibody complex formed in step (i) using any suitable means known in the art, wherein the detection of a complex indicates the presence of the particular polymorphic form in the sample.

Typically, immunoassays use either a labelled antibody or a labelled antigenic component (e.g., that competes with the antigen in the sample for binding to the antibody). Suitable labels include without limitation enzyme-based, fluorescent, chemiluminescent, radioactive, or dye molecules. Assays that amplify the signals from the probe are also known, such as, for example, those that utilize biotin and avidin, and enzyme-labelled immunoassays, such as ELISA assays.

The present invention also provides kits suitable for antibody-based diagnostic applications. Diagnostic kits typically include one or more of the following components:
(i) Polymorphism-specific antibodies. The antibodies may be pre-labelled; alternatively, the antibody may be unlabelled and the ingredients for labelling may be included in the kit in separate containers, or a secondary, labelled antibody is provided; and
(ii) Reaction components: The kit may also contain other suitably packaged reagents and materials needed for the particular immunoassay protocol, including solid-phase matrices, if applicable, and standards.

The kits referred to above may include instructions for conducting the test. Furthermore, in preferred embodiments, the diagnostic kits are adaptable to high-throughput and/or automated operation.

### Drug Targets and Screening Methods

According to the present invention, nucleotide sequences derived from genes disclosed herein and peptide sequences encoded by genes disclosed herein, particularly those that contain one or more polymorphic sequences, comprise useful targets to identify cardiovascular drugs, i.e., compounds that are effective in treating one or more clinical symptoms of cardiovascular disease. Furthermore, especially when a protein is a multimeric protein that are build of two or more subunits, is a combination of different polymorphic subunits very useful.

Drug targets include without limitation (i) isolated nucleic acids derived from the genes disclosed herein, and (ii) isolated peptides and polypeptides encoded by genes disclosed herein, each of which comprises one or more polymorphic positions.

### In vitro screening methods:

In one series of embodiments, an isolated nucleic acid comprising one or more polymorphic positions is tested in vitro for its ability to bind test compounds in a sequence-specific manner. The methods comprise:
(i) providing a first nucleic acid containing a particular sequence at a polymorphic position and a second nucleic acid whose sequence is identical to that of the first nucleic acid except for a different sequence at the same polymorphic position;
(ii) contacting the nucleic acids with a multiplicity of test compounds under conditions appropriate for binding; and
(iii) identifying those compounds that bind selectively to either the first or second nucleic acid sequence.

Selective binding as used herein refers to any measurable difference in any parameter of binding, such as, e.g., binding affinity, binding capacity, etc.

In another series of embodiments, an isolated peptide or polypeptide comprising one or more polymorphic positions is tested in vitro for its ability to bind test compounds in a sequence-specific manner. The screening methods involve:
(i) providing a first peptide or polypeptide containing a particular sequence at a polymorphic position and a second peptide or polypeptide whose sequence is identical to the first peptide or polypeptide except for a different sequence at the same polymorphic position;
(ii) contacting the polypeptides with a multiplicity of test compounds under conditions appropriate for binding; and
(iii) identifying those compounds that bind selectively to one of the nucleic acid sequences.

In preferred embodiments, high-throughput screening protocols are used to survey a large number of test compounds for their ability to bind the genes or peptides disclosed above in a sequence-specific manner.

Test compounds are screened from large libraries of synthetic or natural compounds. Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means.

### In vivo screening methods:

Intact cells or whole animals expressing polymorphic variants of genes disclosed herein can be used in screening methods to identify candidate cardiovascular drugs.

In one series of embodiments, a permanent cell line is established from an individual exhibiting a particular polymorphic pattern. Alternatively, cells (including without limitation mammalian, insect, yeast, or bacterial cells) are programmed to express a gene comprising one or more polymorphic sequences by introduction of appropriate DNA. Identification of candidate compounds can be achieved using any suitable assay, including without limitation (i) assays that measure selective binding of test compounds to particular polymorphic variants of proteins encoded by genes disclosed herein; (ii) assays that measure the ability of a test compound to modify (i.e., inhibit or enhance) a measurable activity or function of proteins encoded by genes disclosed herein; and (iii) assays that measure the ability of a compound to modify (i.e., inhibit or enhance) the transcriptional activity of sequences derived from the promoter (i.e., regulatory) regions of genes disclosed herein.

In another series of embodiments, transgenic animals are created in which (i) one or more human genes disclosed herein, having different sequences at particular polymorphic positions are stably inserted into the genome of the transgenic animal; and/or (ii) the endogenous genes disclosed herein are inactivated and replaced with human genes disclosed herein, having different sequences at particular polymorphic positions. See, e.g., Coffman, Semin. Nephrol. 17:404, 1997; Esther et al., Lab. Invest. 74:953, 1996; Murakami et al., Blood Press. Suppl. 2:36, 1996. Such animals can be treated with candidate compounds and monitored for one or more clinical markers of cardiovascular status.

The following are intended as non-limiting examples of the invention.

### Material and Methods

Genotyping of patient DNA with the Pyrosequencing™ Method as described in the patent application WO 9813523:

First a PCR is set up to amplify the flanking regions around a SNP. Therefor 2 ng of genomic DNA (patient sample) are mixed with a primerset (20 - 40 pmol) producing a 75 to 320 bp PCR fragment with 0,3 to 1 U Qiagens Hot Star Taq Polymerase™ in a total volume of 20 µL. One primer is biotinylated depending on the direction of the sequencing primer. To force the biotinylated primer to be incorporated it is used 0,8 fold.

For primer design, programms like Oligo 6™ (Molecular Biology Insights) or Primer Select™ (DNAStar) are used. PCR setup is performed by a BioRobot 3000 ™ from Qiagen. PCR takes place in T1 or Tgradient Thermocyclers ™ from Biometra. The whole PCR reaction is transferred into a PSQ plate ™ (Pyrosequencing) and prepared using the Sample Prep Tool ™ and SNP Reagent Kit ™ from Pyrosequencing according to their instructions.

### Preparation of template for Pyrosequencing™:

Sample preparation using PSQ 96 Sample Prep Tool:
1. Mount the PSQ 96 Sample Prep Tool Cover onto the PSQ 96 Sample Prep Tool as follows: Place the cover on the desk, retract the 4 attachment rods by separating the handle from the magnetic rod holder, fit the magnetic rods into the holes of the cover plate, push the handle downward until a click is heard. The PSQ 96 Sample Prep Tool is now ready for use.
2. To transfer beads from one plate to another, place the covered tool into the PSQ 96 Plate containing the samples and lower the magnetic rods by separating the handle from the magnetic rod holder. Move the tool up and down a few times then wait for 30-60 seconds. Transfer the beads into a new PSQ 96 plate containing the solution of choice.
3. Release the beads by lifting the magnetic rod holder, bringing it together with the handle. Move the tool up and down a few times to make sure that the beads are released.

All steps are performed at room temperature unless otherwise stated.

### Immobilization of PCR product:

Biotinylated PCR products are immobilized on streptavidin-coated Dynabeads™ M-280 Streptavidin. Parallel immobilization of several samples are performed in the PSQ 96 Plate.
1. Mix PCR product, 20 µl of a well optimized PCR, with 25 µl 2X BW-buffer II. Add 60-150 µg Dynabeads. It is also possible to add a mix of Dynabeads and 2X BW-buffer II to the PCR product yielding a final BW-buffer II concentration of approximately 1x.
2. Incubate at 65°C for 15 min agitation constantly to keep the beads dispersed. For optimal immobilization of fragments longer than 300 bp use 30 min incubation time.

### Strand separation:

4. For strand separation, use the PSQ 96 Sample Prep Tool to transfer the beads with the immobilized sample to a PSQ 96 Plate containing 50 µl 0.50 M NaOH per well. Release the beads.
5. After approximately 1 min, transfer the beads with the immobilized strand to a PSQ 96 Plate containing 99 µl 1x Annealing buffer per well and mix thoroughly.
6. Transfer the beads to a PSQ 96 Plate containing 45 µl of a mix of 1x Annealing buffer and 3-15 pmoles sequencing primer per well.
7. Heat at 80°C for 2 minutes in the PSQ 96 Sample Prep Thermoplate and move to room temperature.
8. After reaching room temperature, continue with the sequencing reaction.

Sequencing reaction:
1. Choose the method to be used ("SNP Method") and enter relevant information in the PSQ 96 Instrument Control software.
2. Place the cartridge and PSQ 96 Plate in the PSQ 96 Instrument.
3. Start the run.

### Genotyping using the ABI 7700/7900 instrument (TaqMan)

SNP genotypisation using the TaqMan (Applied Biosystems/Perkin Elmer) was performed according to the manufacturer's instructions. The TaqMan assay is discussed by Lee et al., Nucleic Acids Research 1993, 21: 3761-3766.

### Genotyping with a service contractor:

Qiagen Genomics, formerly Rapigene, is a service contractor for genotyping SNPs in patient samples. Their method is based on a primer extension method where two complementary primers are designed for each genotype that are labeled with different tags. Depending on the genotype only one primer will be elongated together with a certain tag. This tag can be detected with mass spectrometry and is a measure for the respective genotype. The method is described in the following patent: "Detection and identification of nucleic acid molecules - using tags which may be detected by non-fluorescent spectrometry or potentiometry" (WO 9727325).

### Examples

To exemplify the present invention and it's utility baySNP 28 will be used in the following:

baySNP 28 is a C to T polymorphism and presumably resides in the gene of the human acidic 82 kDa protein (information taken from table 3). baySNP 28 was genotyped in various patient cohorts using the primers from table 2. As a result the following number of patients carrying different genotypes were found (information combined from tables 3 and 5a):

| **baySNP** | **Cohort** | **Total** | **Genotype 11 "CC"** | **Genotype 12 "CT"** | **Genotype 22 "TT"** |
|---|---|---|---|---|---|
| 28 | HELD_FEM_HIRESP | 12 | 1 | 2 | 9 |
| 28 | HELD_FEM_LORESP | 22 | 3 | 12 | 7 |

When comparing the number of female patients exhibiting a high response to statin therapy (HELD_FEM_HIRESP) with the control cohort (HELD_FEM_LORESP) it appears that the number of low responders carrying the CT genotype is increased. This points to a lower statin response among female individuals with the CT genotype. Applying statistical tests on those findings the following p-values were obtained (data taken from table 5b):

| **BAYSNP** | **COMPARISON** | **GTYPE CPVAL** | **GTYPE XPVAL** | **GTYPE LRPVAL** |
|---|---|---|---|---|
| 28 | HELD_FEM_EFF | 0,0506 | 0,0508 | 0,0442 |

As at least one of the GTYPE p values is below 0,05 the association of genotype and statin response phenotype is regarded as statistically significant. I.e. the analysis of a patient's genotype can predict the response to statin therapy. In more detail one can calculate the relative risk to exhibit a certain statin response phenotype when carrying a certain genotype (data taken from table 6a):

| **BAYSNP** | **COMPARISON** | **GTYPE1** | **GTYPE2** | **GTYPE3** | **RR1** | **RR2** | **RR3** |
|---|---|---|---|---|---|---|---|
| 28 | HELD_FEM_EFF | CC | CT | TT | 0,68 | 0,29 | 3,38 |

In case of baySNP 28 the risk to exhibit a high responder phenotype is 3,38 times higher when carrying the TT genotype. This indicates that a TT polymorphism in baySNP 28 is an independent risk factor for high statin response in females. On the other hand carriers of a CT or CC genotype have a reduced risk of being a high responder.

In addition statistical associations can be calculated on the basis on alleles. This calculation would identify risk alleles instead of risk genotypes.

In case of baySNP 28 the following allele counts were obtained (data combined from tables 3 and 5a):

| **baySNP** | **Cohort** | **Total** | **Allele 1 "C"** | **Allele 2 "T"** |
|---|---|---|---|---|
| 28 | HELD_FEM_HIRESP | 12 | 4 | 20 |
| 28 | HELD_FEM_LORESP | 22 | 18 | 26 |

When comparing the number of female patients with high statin response (HELD_FEM_HIRESP) with the control cohort (HELD_FEM_LORESP) it appears that the number of high responders carrying the T allele is increased, whereas the number of high responders carrying the C allele is diminished. This points to a higher statin response among female individuals with the T allele. Applying statistical tests on those findings the following p-values were obtained (data taken from table 5b):

| **BAYSNP** | **COMPARISON** | **ALLELE CPVAL** | **ALLELE XPVAL** | **ALLELE LRPVAL** |
|---|---|---|---|---|
| 28 | HELD_FEM_EFF | 0,0411 | 0,0579 | 0,0349 |

As at least one of the ALLELE p values is below 0,05 the association of allele and statin response phenotype is regarded as statistically significant (in this example significant p values were obtained from two statistical tests). I.e. also the analysis of a patient's alleles from baySNP 28 can predict the extend of statin response. In more detail one can calculate the relative risk to exhibit a certain statin response phenotype when carrying a certain allele (data taken from table 6b):

| **baySNP** | **Allele 1** | **Allele 2** | **COMPARISON** | **RR1** | **RR2** |
|---|---|---|---|---|---|
| 28 | C | T | HELD_FEM_EFF | 0,42 | 2,39 |

In case of baySNP 28 the risk to exhibit a high responder phenotype is 2,39 times higher when carrying the T allele. This indicates that the T allele of baySNP28 is an independent risk factor for a high statin response in females. In other words those patients should receive lower doses of statins in order to avoid ADR. However due to their 'high responder' phenotype they will still benefit from the drug. In turn carriers of the C allele should receive higher drug doses in order to experience a benefical therapeutic effect.

Another example is baySNP 29, which is taken to exemplify polymorphisms relevant for adverse drug reactions. baySNP 29 was found significant when comparing male patients with severe ADR to the respective controls (as defined in table 1b).

The relative risk ratios for the genotypes AA, AG and GG were as follows (data taken from table 6a):

| **BAYSNP** | **COMPARISON** | **GTYPE1** | **GTYPE2** | **GTYPE3** | **RR1** | **RR2** | **RR3** |
|---|---|---|---|---|---|---|---|
| 29 | HELD_MAL_ADR5ULN | AA | AG | GG | 3,15 | 0,66 | 0,32 |

In this case male patients carrying the AA genotype have a 3,15 times higher risk to suffer from ADR. In other words those patients should either receive lower doses of statins or switch to an alternative therapy in order to avoid ADR. On the other hand male patients with AG or GG genotypes appear to be more resistant to ADR and hence better tolerate statin therapy.

As can be seen from the following tables some of the associations that are disclosed in the present invention are indicative for more than one phenotype. baySNP 1837 is for example linked to ADR, but also to the risk to suffer from CVD (table 6).

**Table 1a**

| Definition of "good" and "bad" serum lipid levels | | |
|---|---|---|
| | "Good" | "Bad" |
| LDL-Cholesterol [mg/dL] | 125 -150 | 170 - 200 |
| Cholesterol [mg/dL] | 190 - 240 | 265 - 315 |
| HDL-Cholesterol [mg/dL] | 60 -105 | 30 - 55 |
| Triglycerides [mg/dL] | 45 - 115 | 170 - 450 |

**Table 1b**

| Definition of drug response phenotypes | |
|---|---|
| Low responder | Decrease of serum LDL of at least 10% and at most 50% upon administration of 0.8 mg Cerivastatin (female patients) |
| High responder | Decrease of serum LDL of at least 50% upon administration of 0.4 mg Cerivastatin (female patients) |
| Very low responder | Decrease of serum LDL of at least 10% and at most 35% upon administration of 0.8 mg Cerivastatin (female patients) |
| Very high responder | Decrease of serum LDL of at least 55% upon administration of 0.4 mg Cerivastatin (female patients) |
| Ultra low responder | Decrease of serum LDL of at least 10% and at most 25% upon administration of 0.8 mg Cerivastatin (female patients) |
| Ultra high responder | Decrease of serum LDL of at least 60% upon administration of 0.4 mg Cerivastatin (female patients) |
| Tolerant patient | No diagnosis of muscle cramps, muscle pain, muscle weakness, myalgia or myopathy AND serum CK levels below 70 mg/dl in women and below 80 mg/dl in men. |
| ADR patient (CK increase at least 2×ULN) | Diagnosis of muscle cramps, muscle pain, muscle weakness, myalgia or myopathy OR serum CK levels higher than 140 mg/dl in women and 160 mg/dl in men. |
| Advanced ADR patient [ADR3] (advanced CK increase, at least 3×ULN)* | Serum CK levels higher than 210 mg/dl in women and 240 mg/dl in men |
| Severe ADR patient [ADR5] (severe CK increase, at least 5×ULN)* | Serum CK levels higher than 350 mg/dl in women and 400 mg/dl in men |

| | |
|---|---|
| *: When assembling the cohorts for advanced and severe ADR we focused on the CK serum levels as those provide a more independent measure of statin related ADR. | |

**Table 1c**

| Definition of "high" and "low" serum HDL cholesterol levels | | |
|---|---|---|
| | Male individuals | Female individuals |
| ,High' HDL-Cholesterol [mg/dL] | >=80 | >=104 |
| ,Low' HDL-Cholesterol [mg/dL] | <=35 | <=37 |

An informed consent was signed by the patients and control people. Blood was taken by a physician according to medical standard procedures.
Samples were collected anonymous and labeled with a patient number.
DNA was extracted using kits from Qiagen.

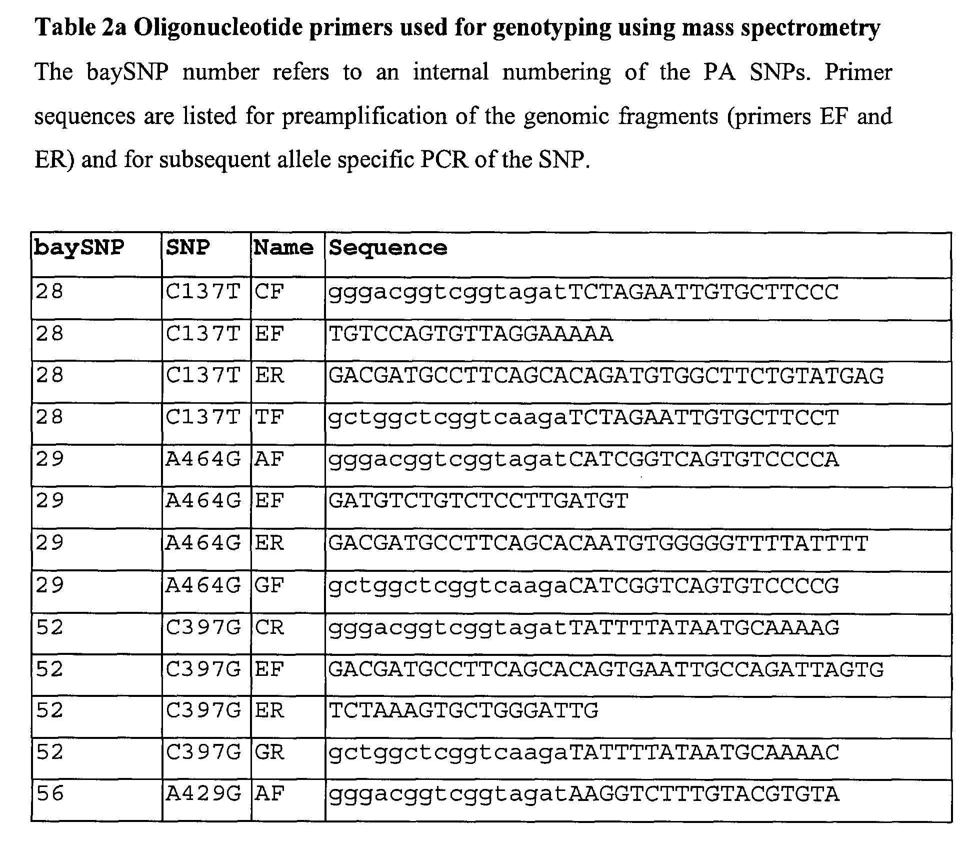

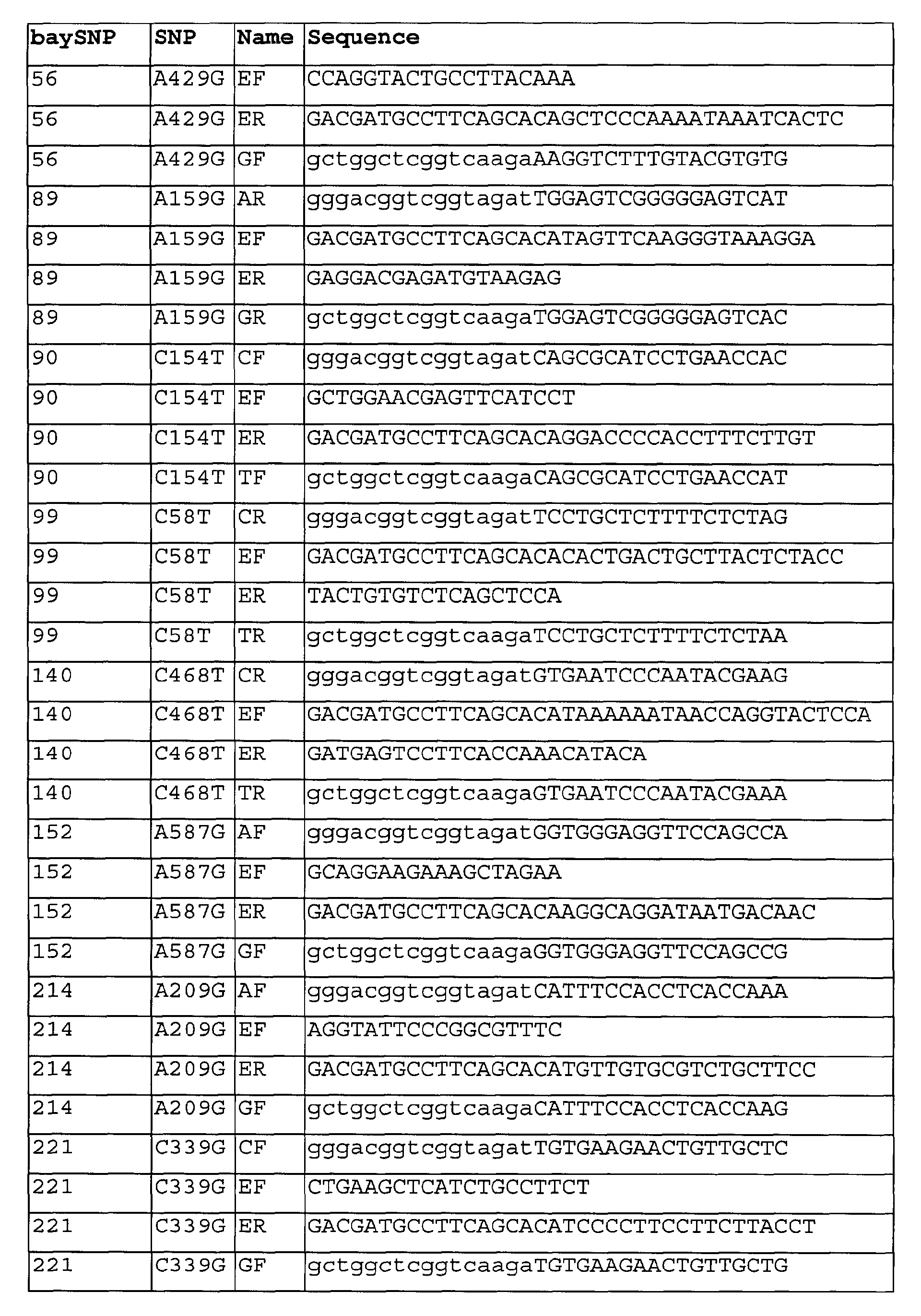

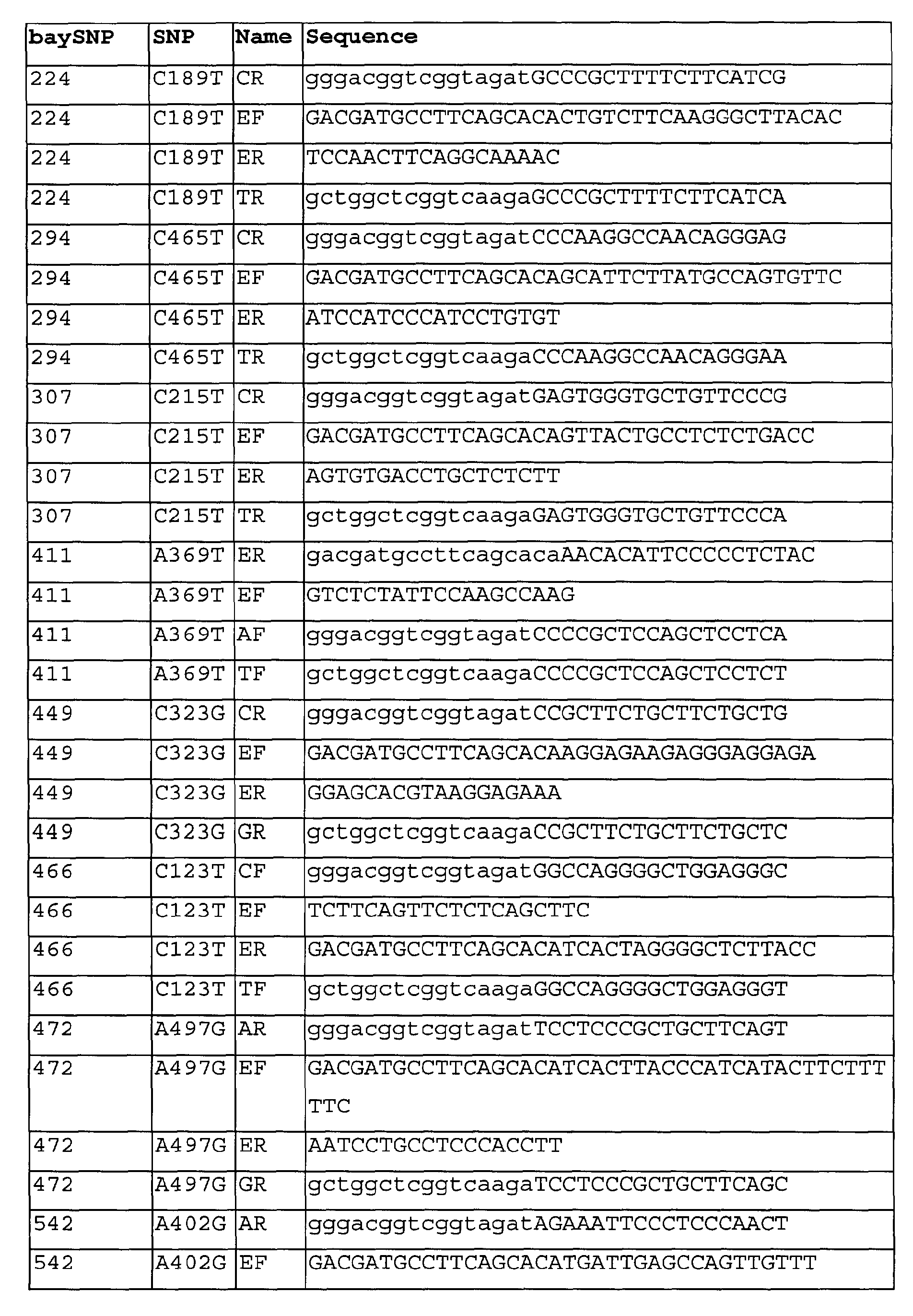

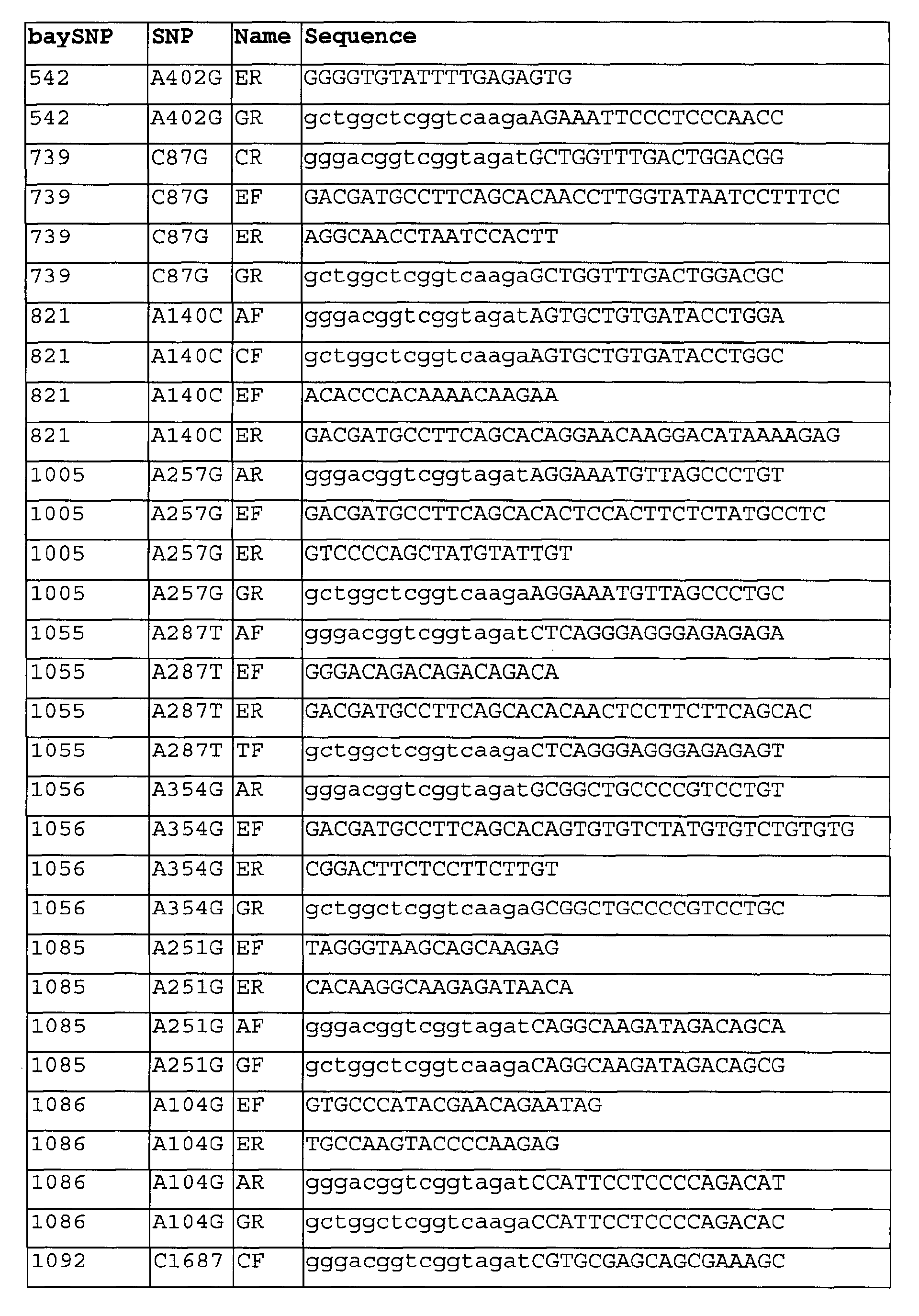

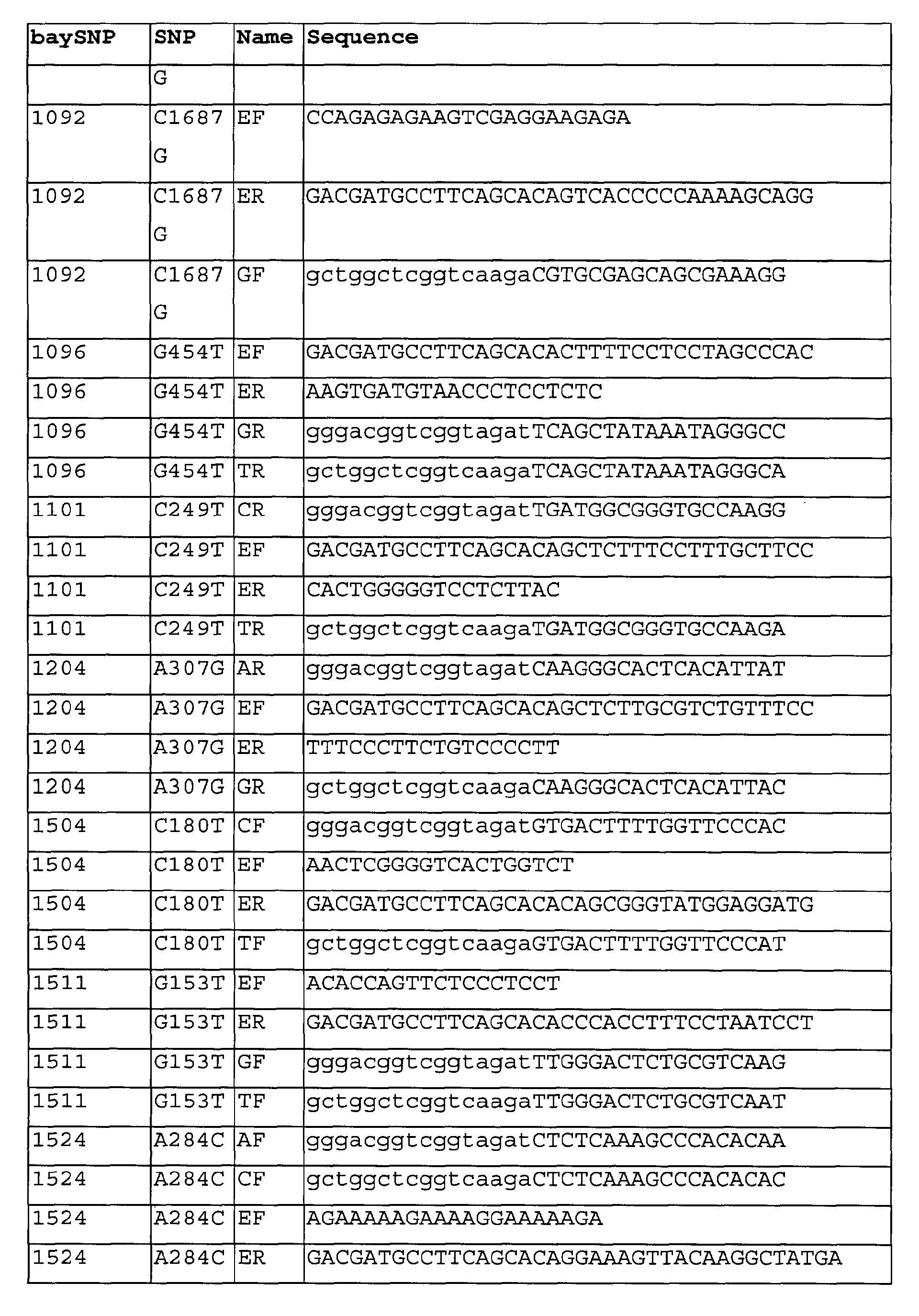

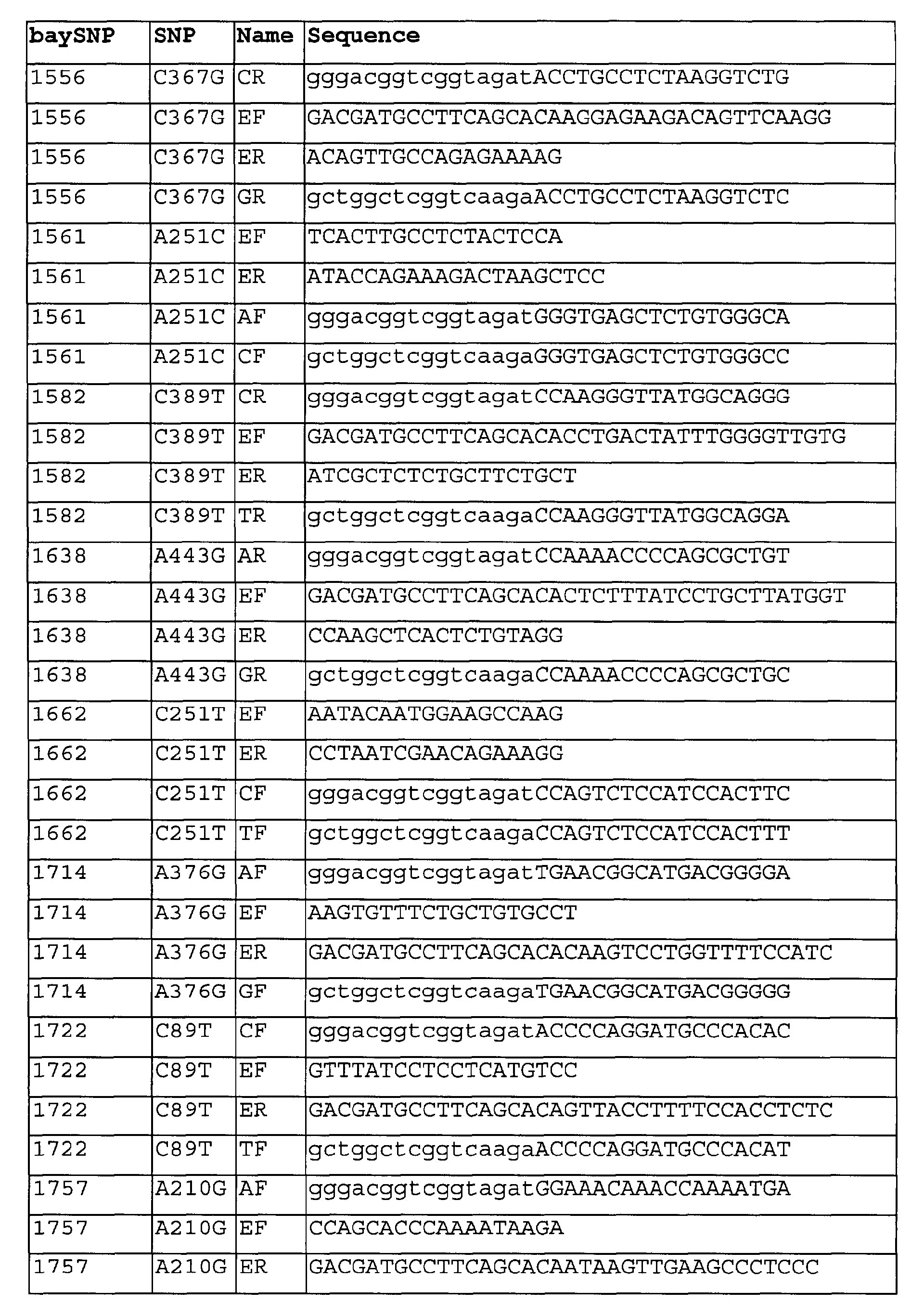

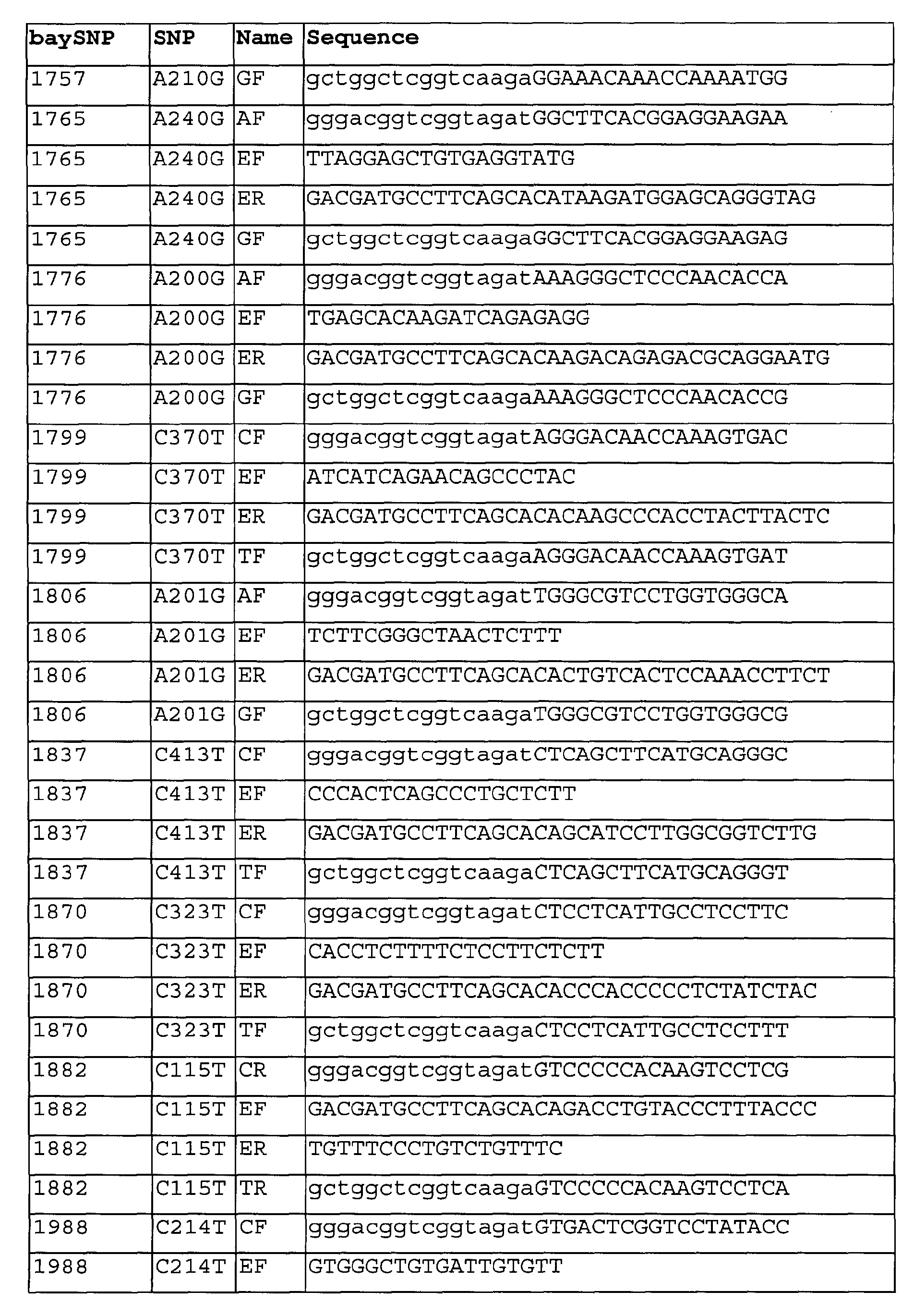

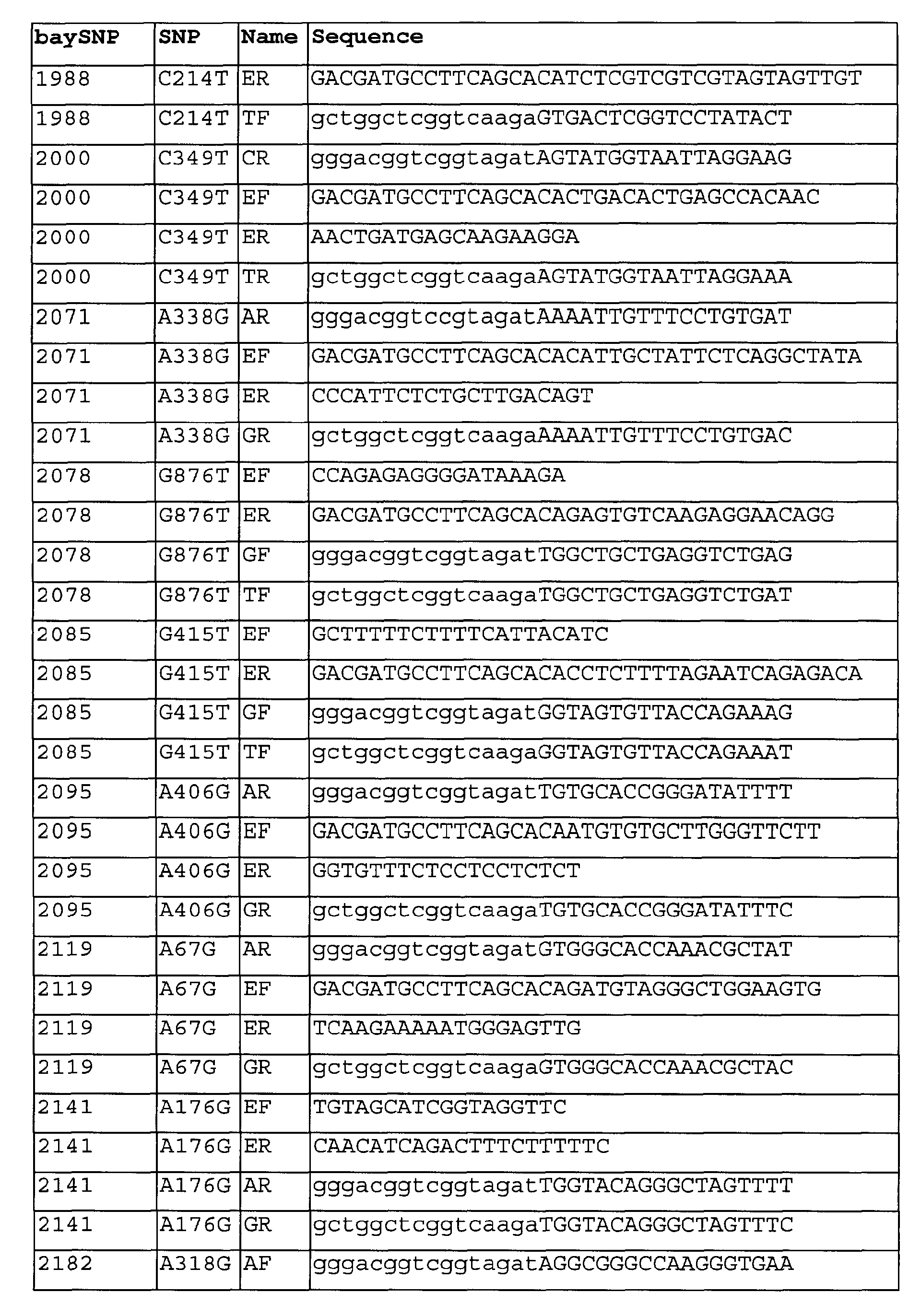

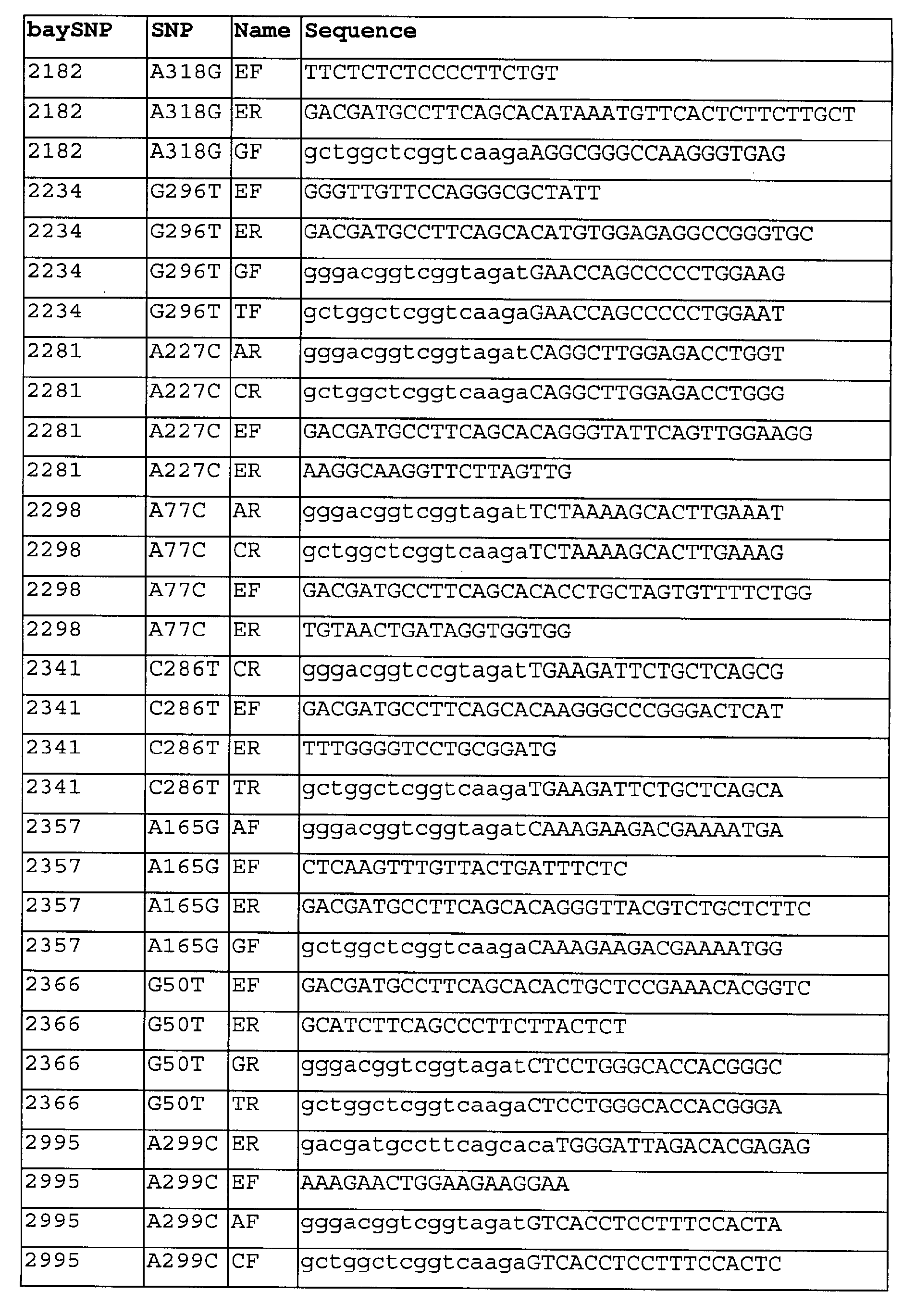

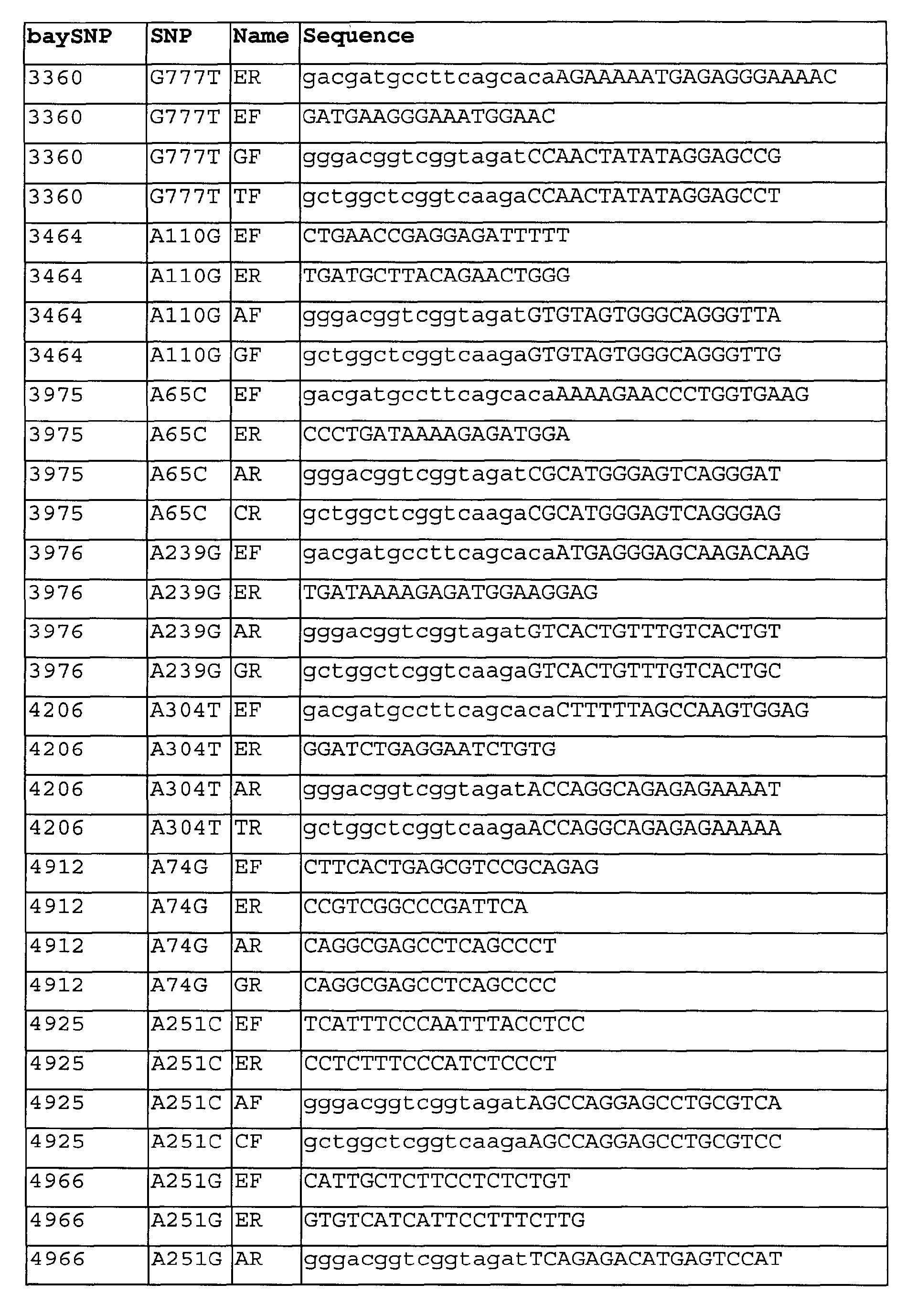

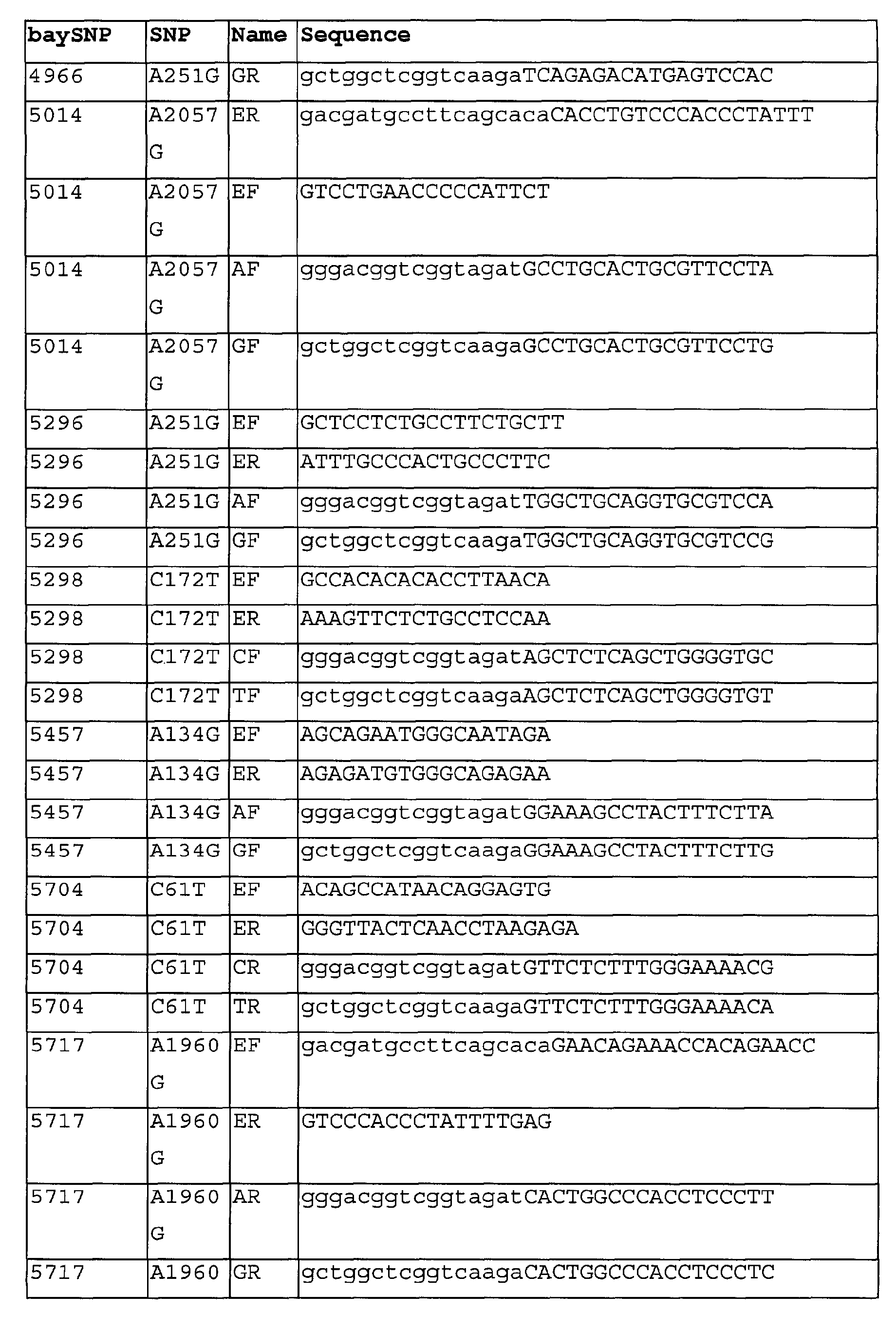

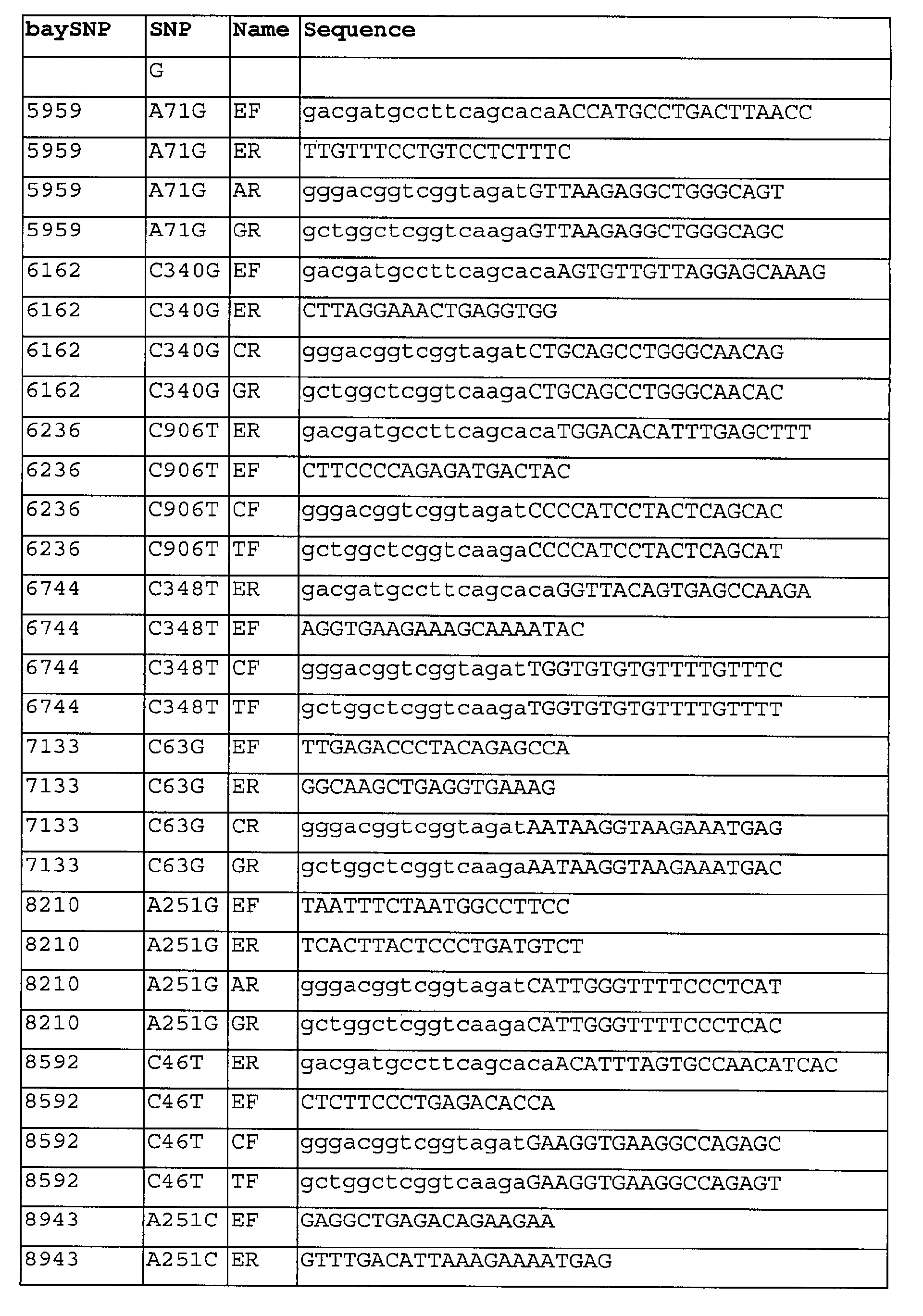

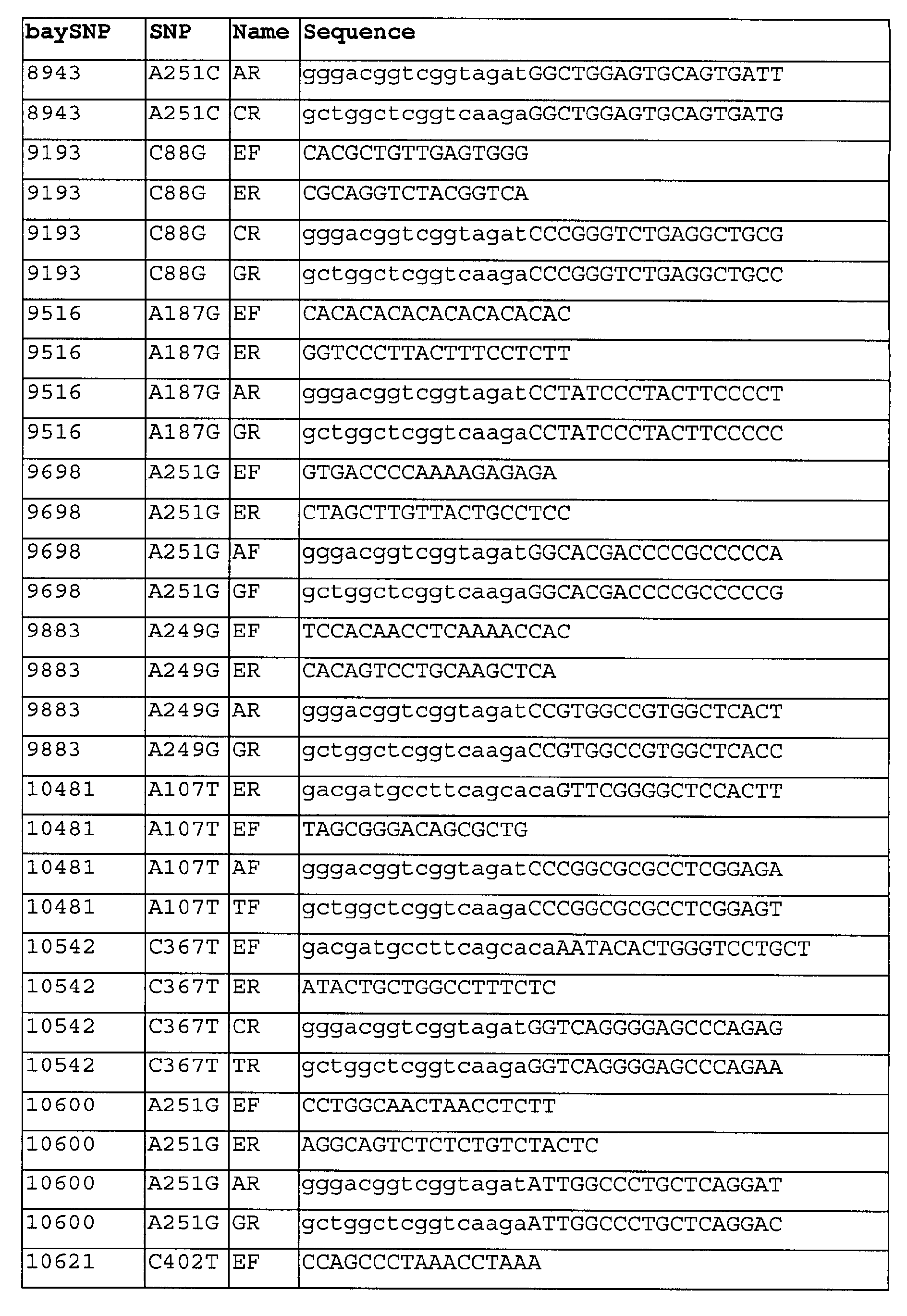

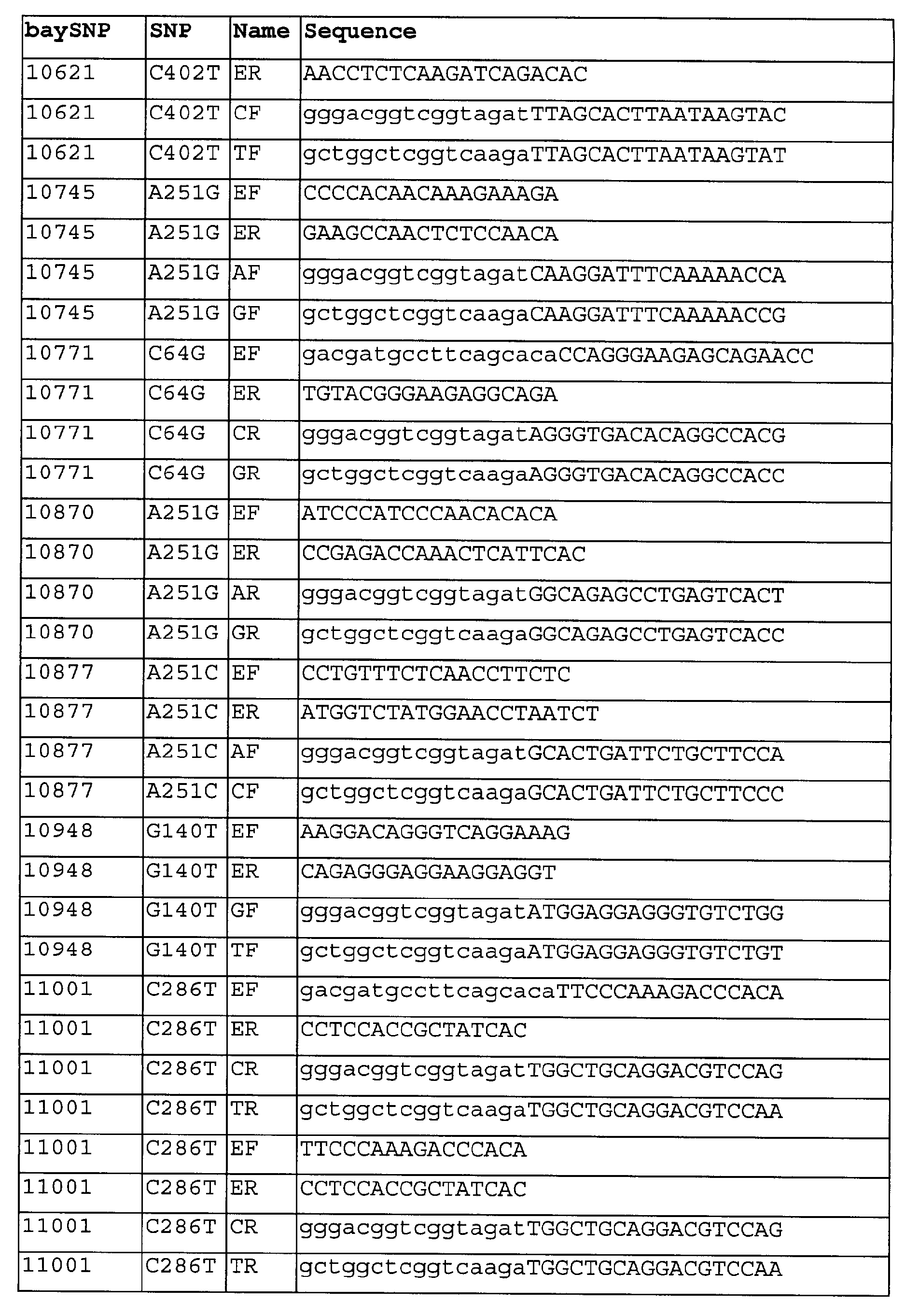

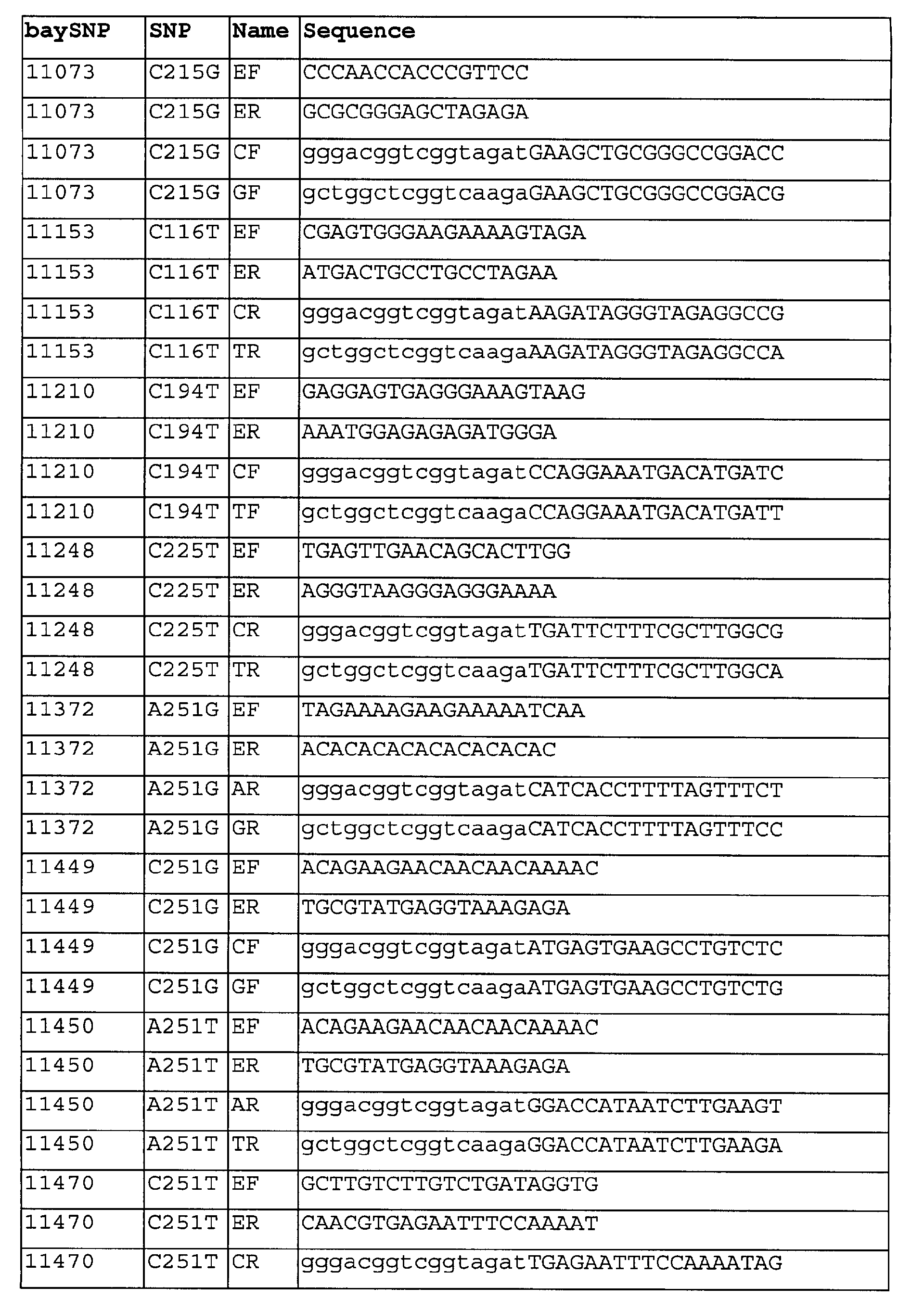

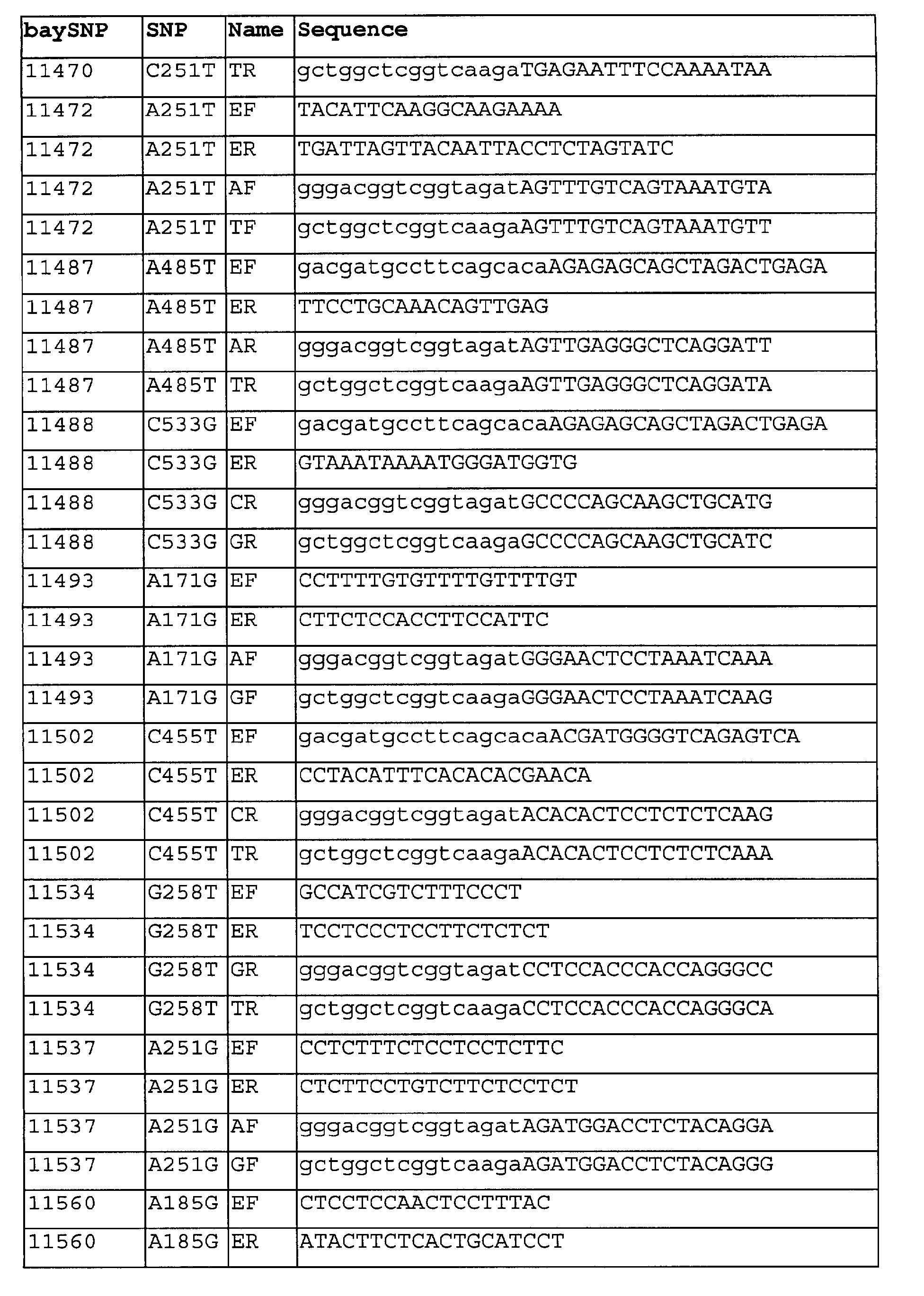

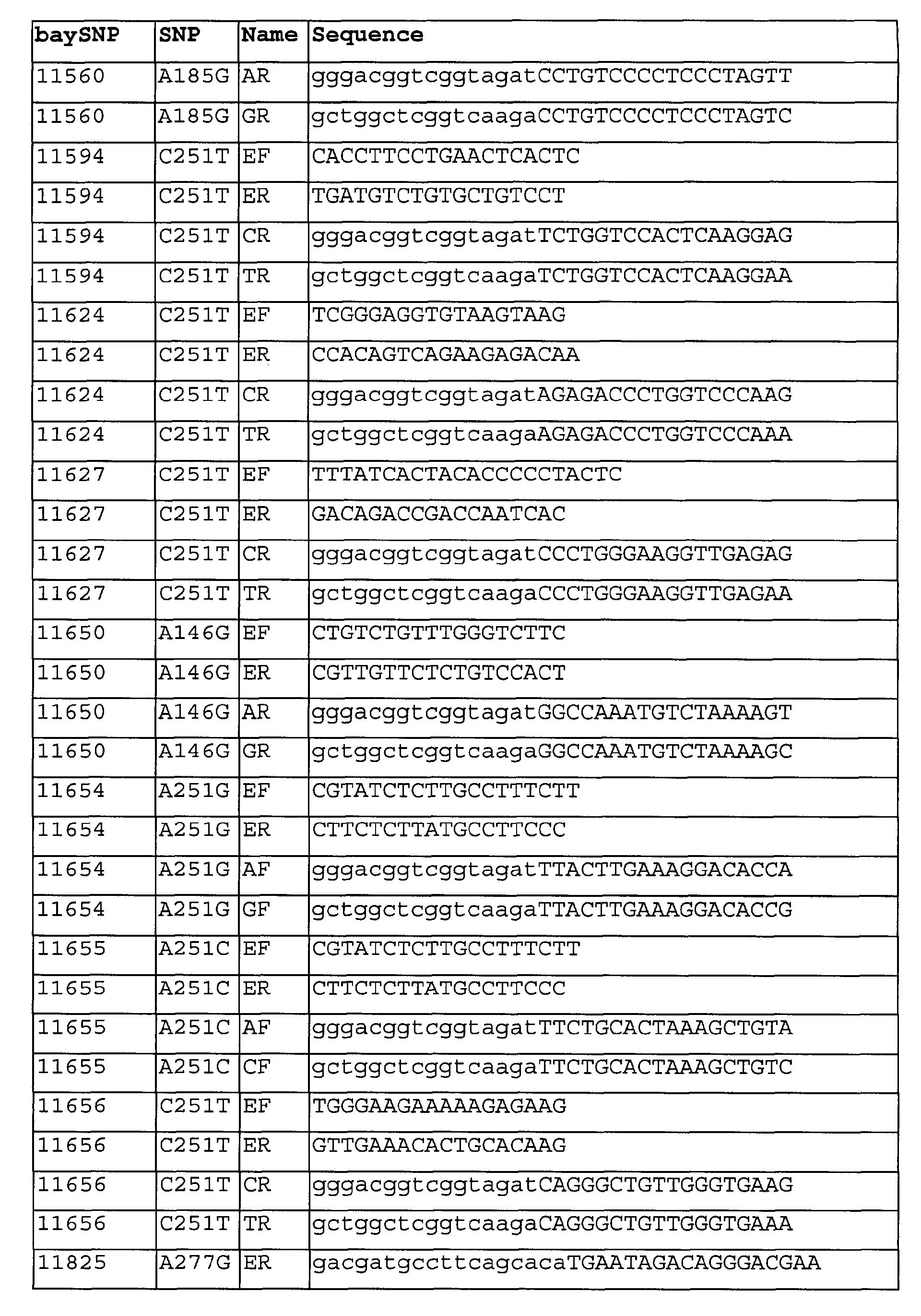

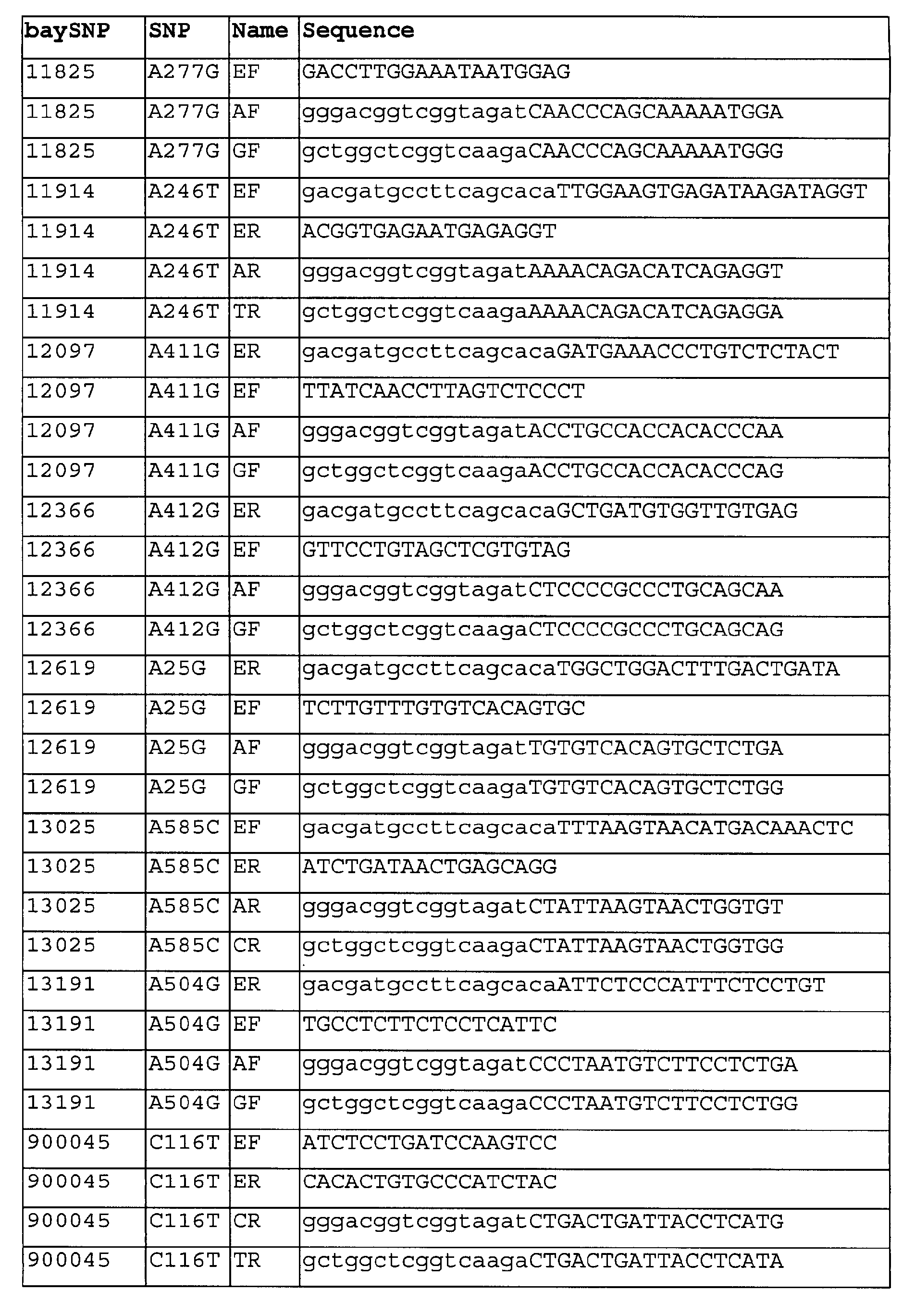

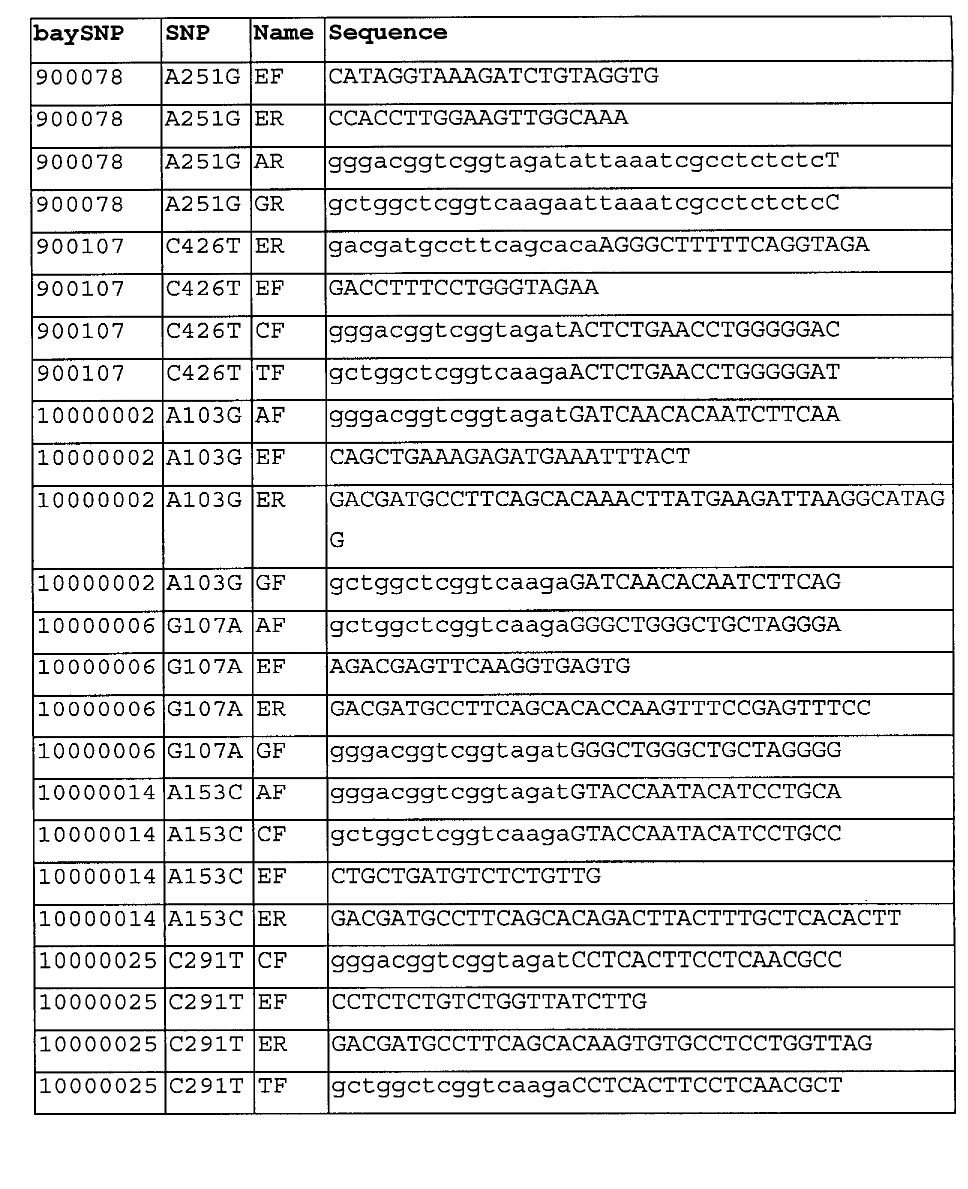

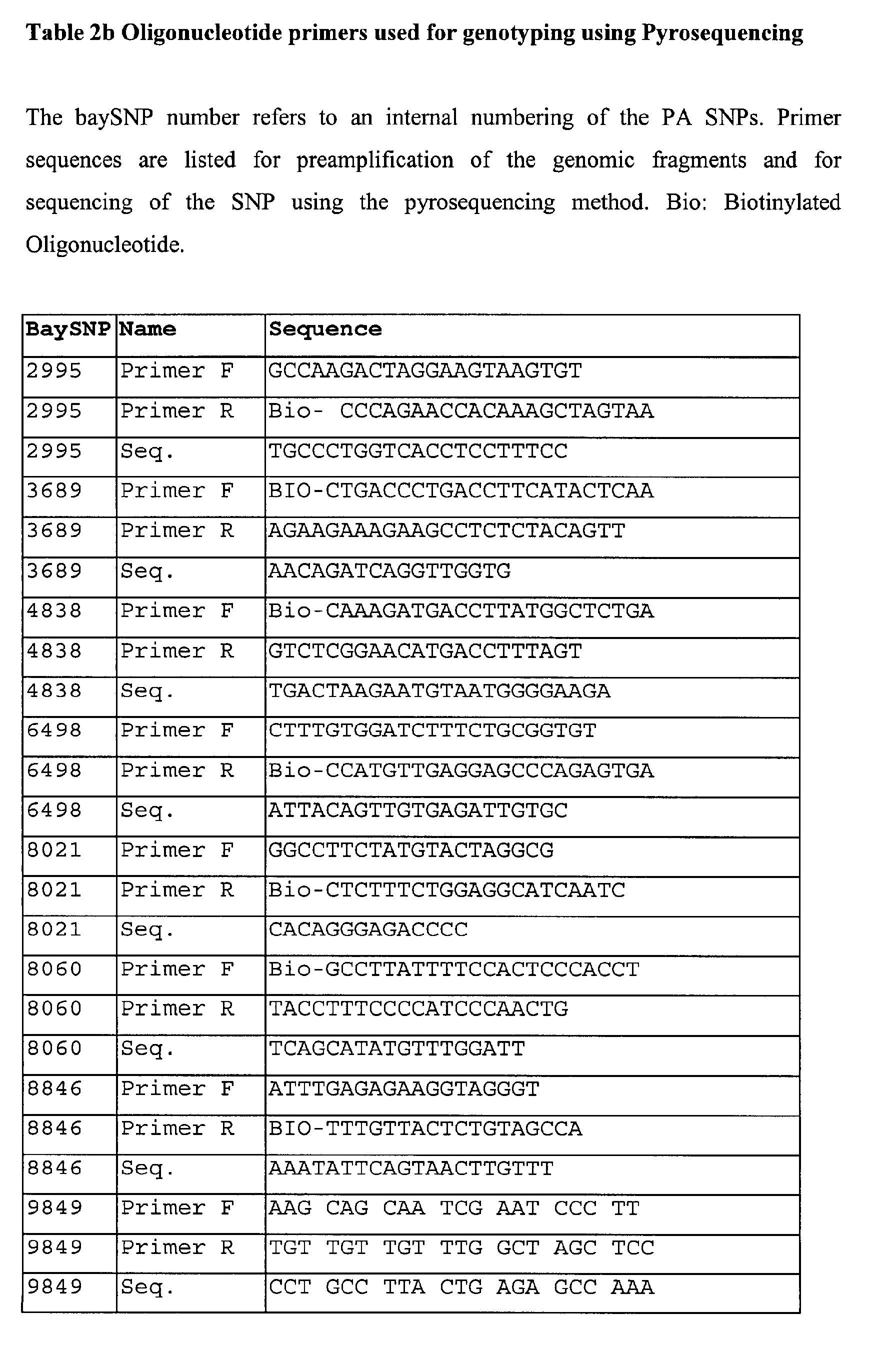

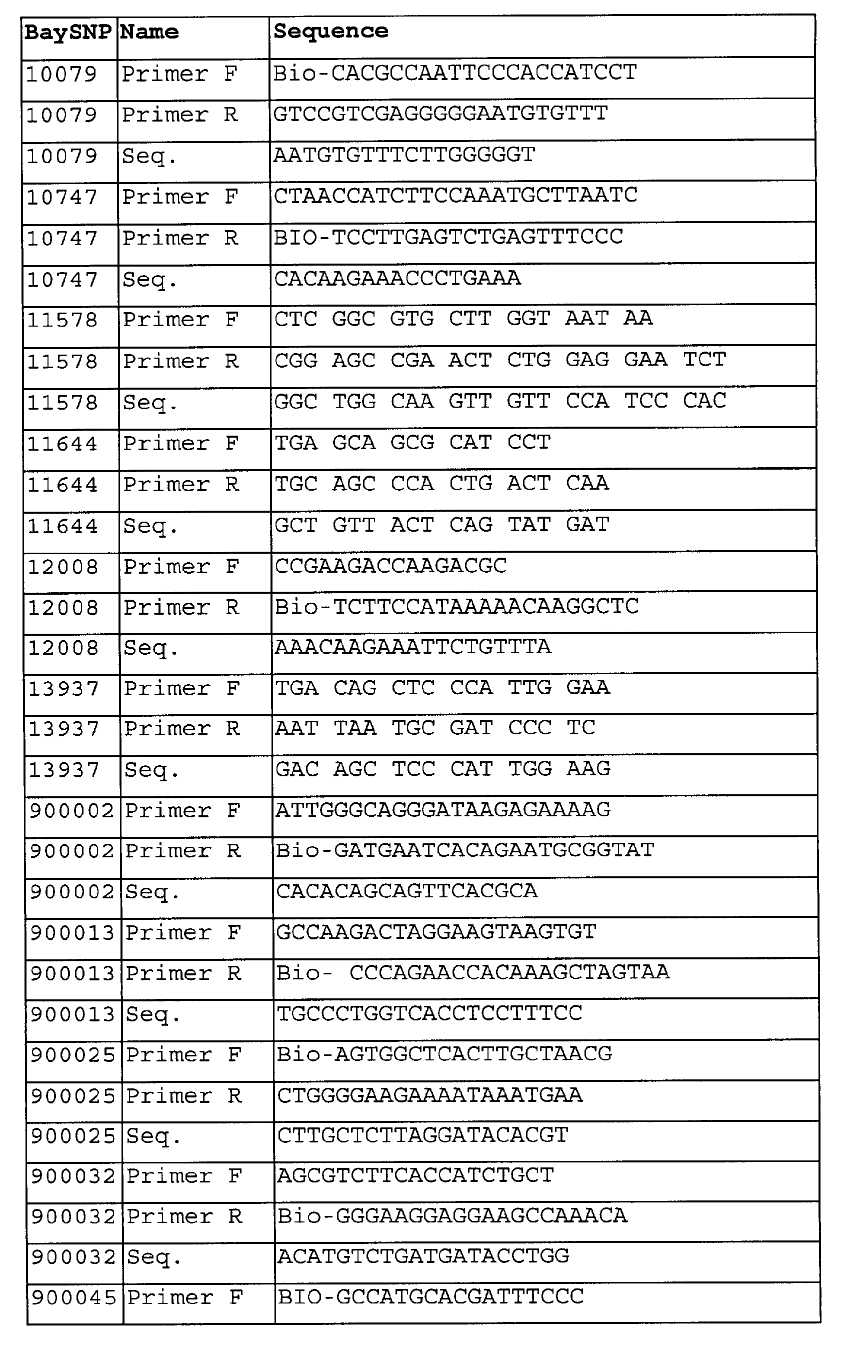

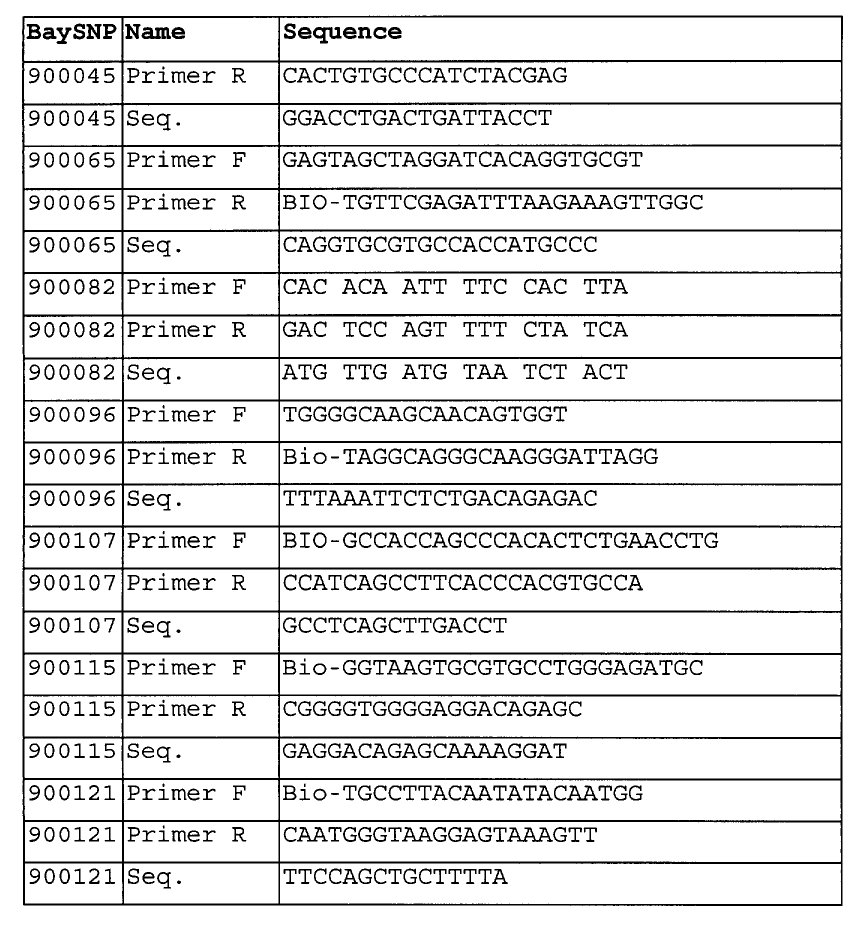

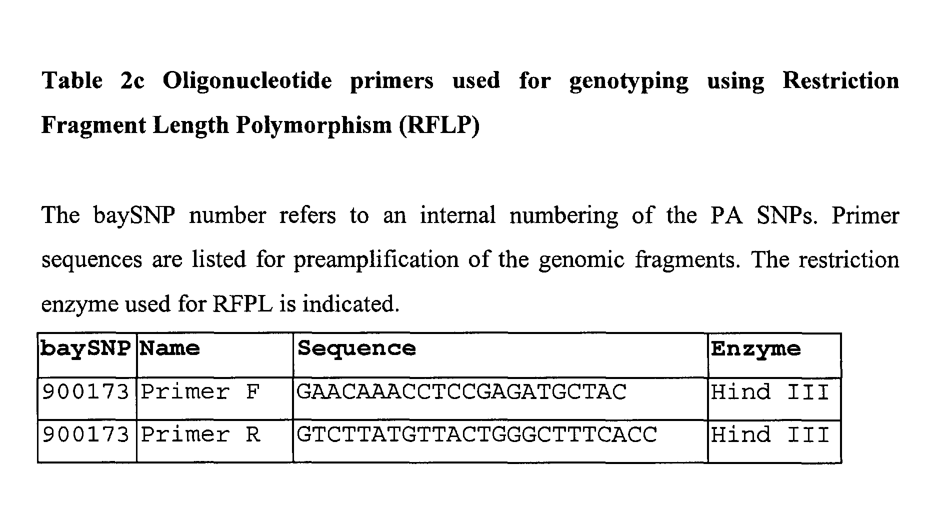

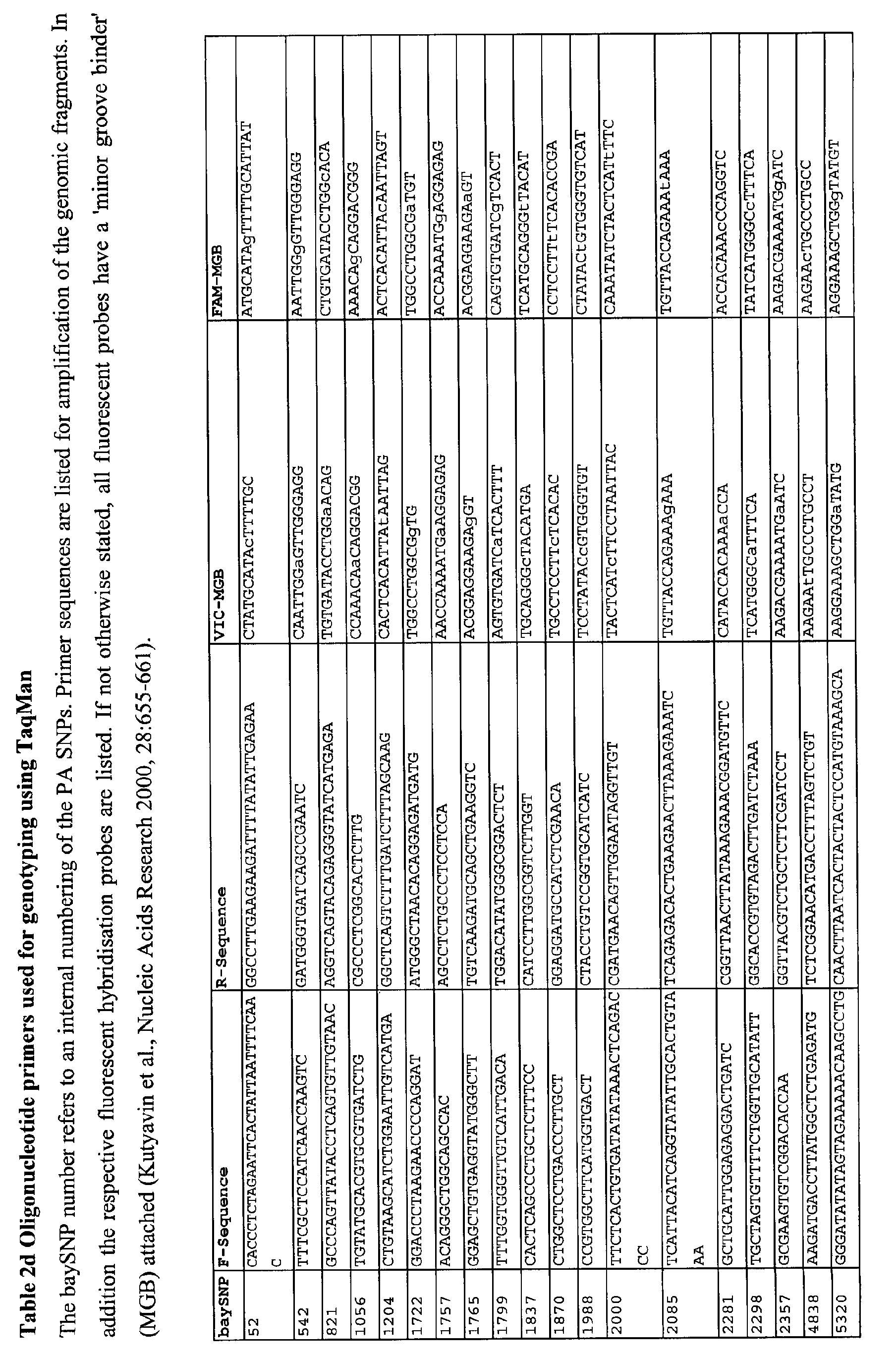

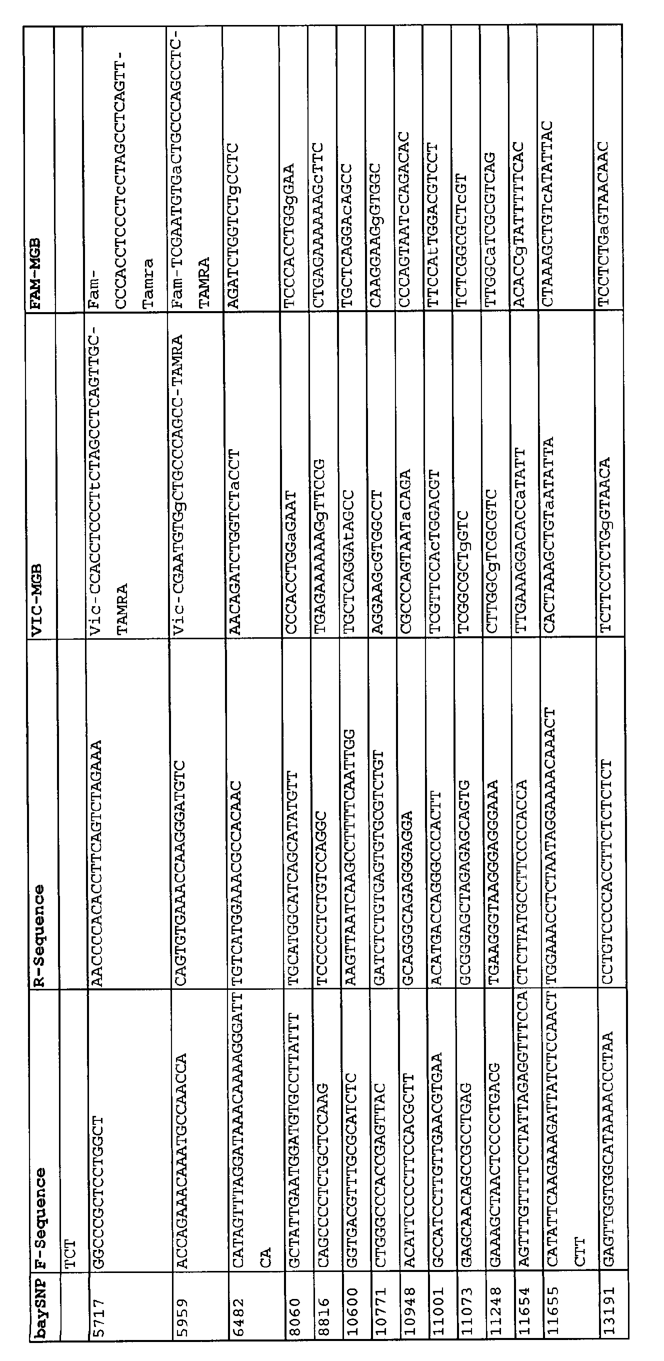

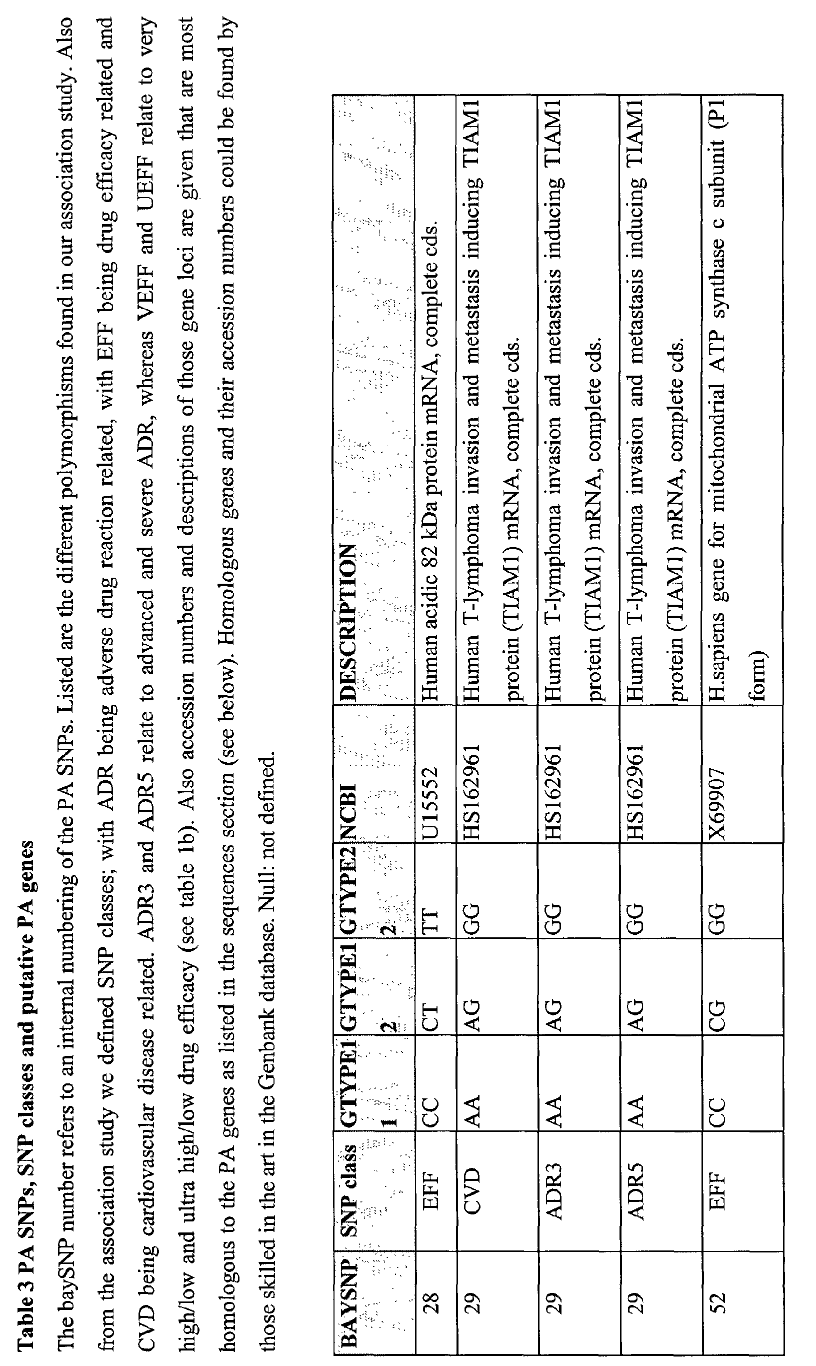

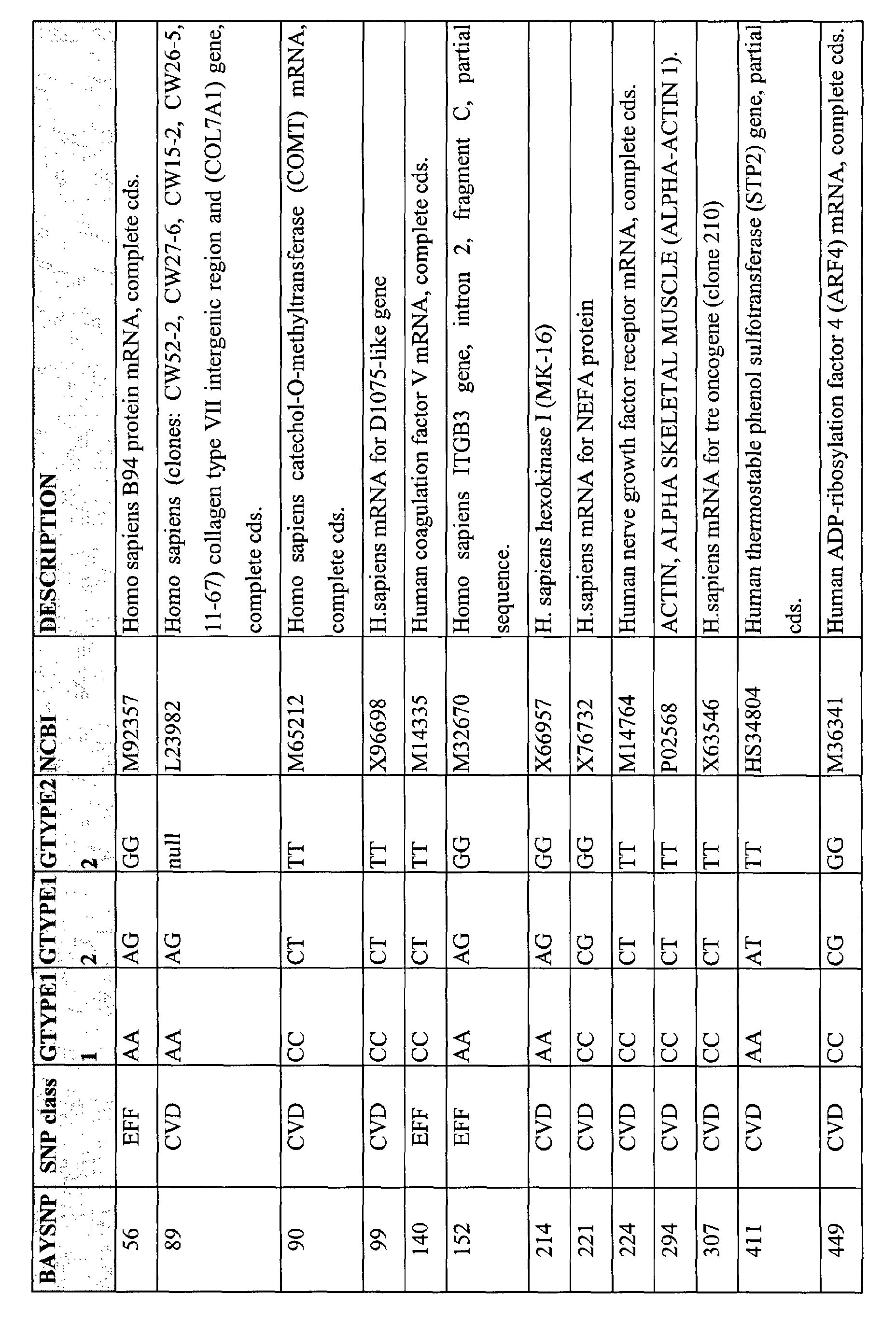

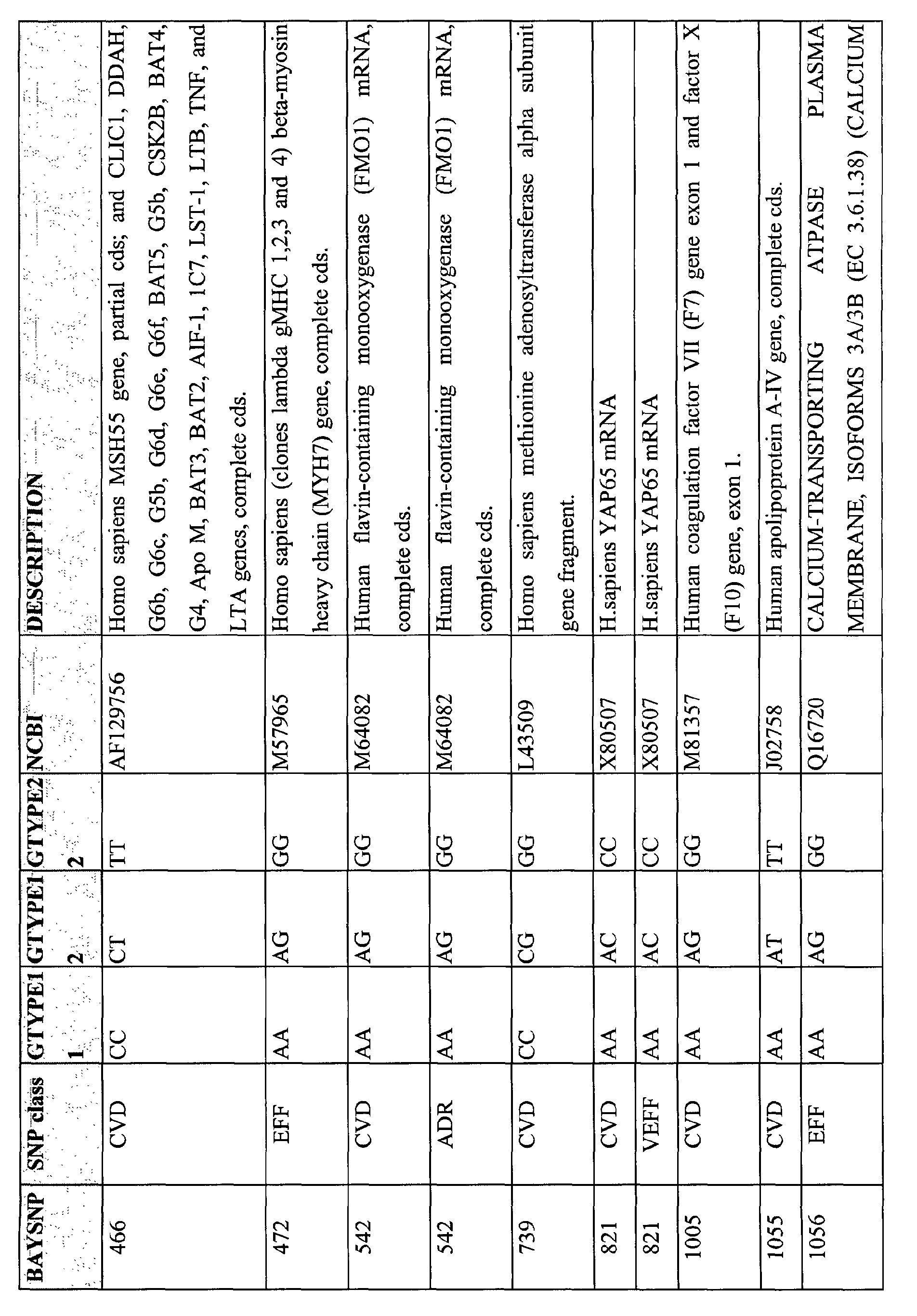

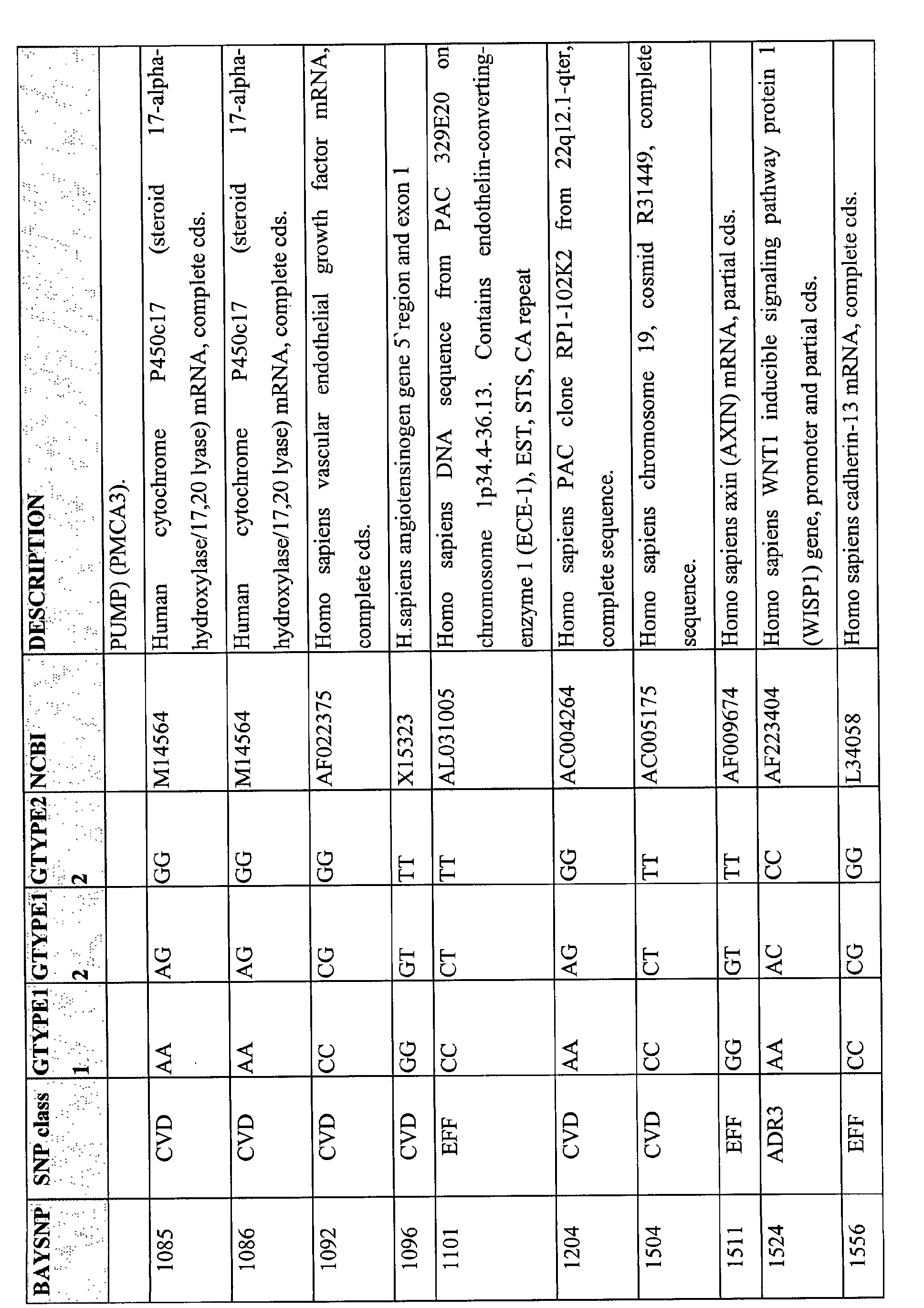

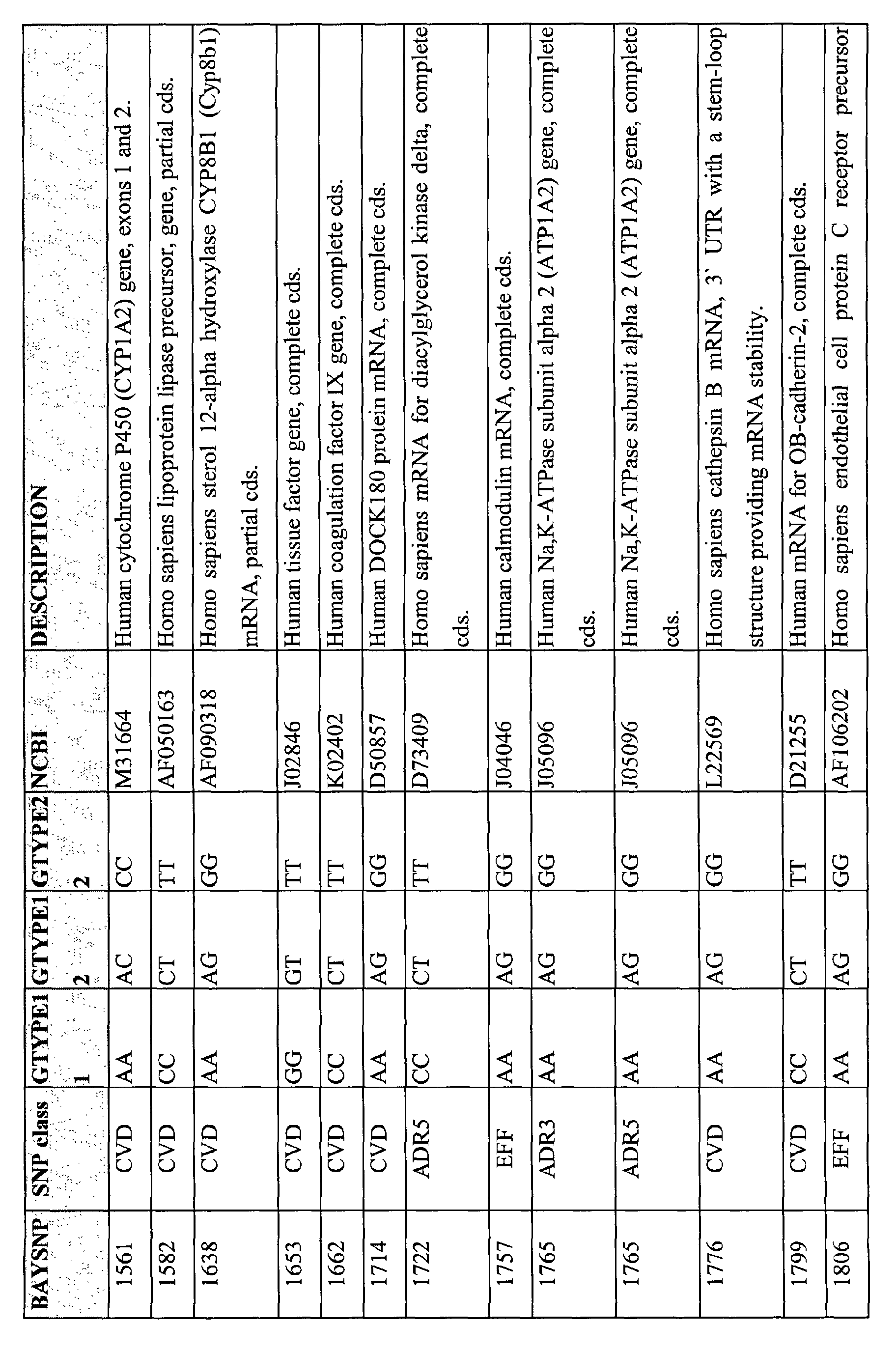

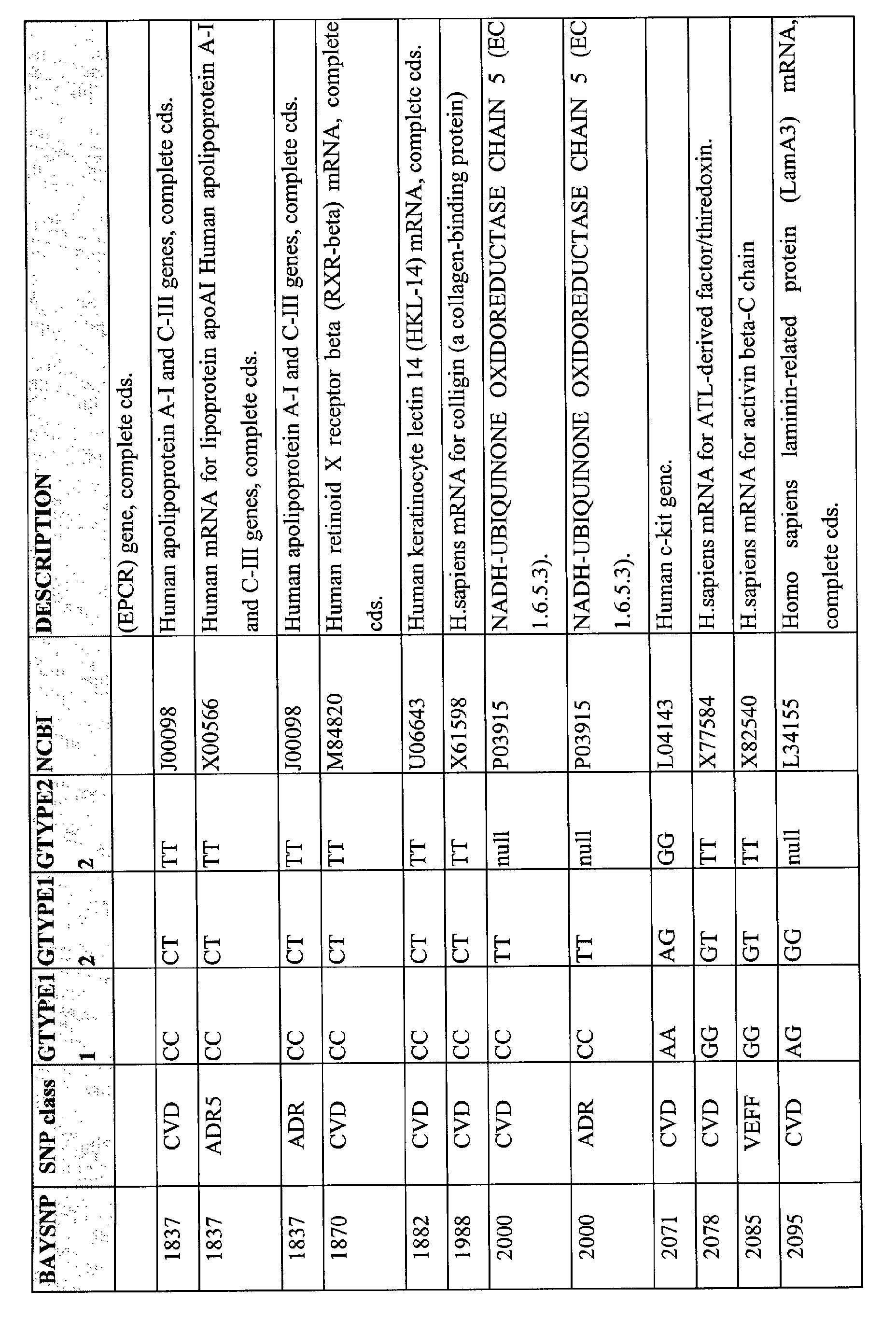

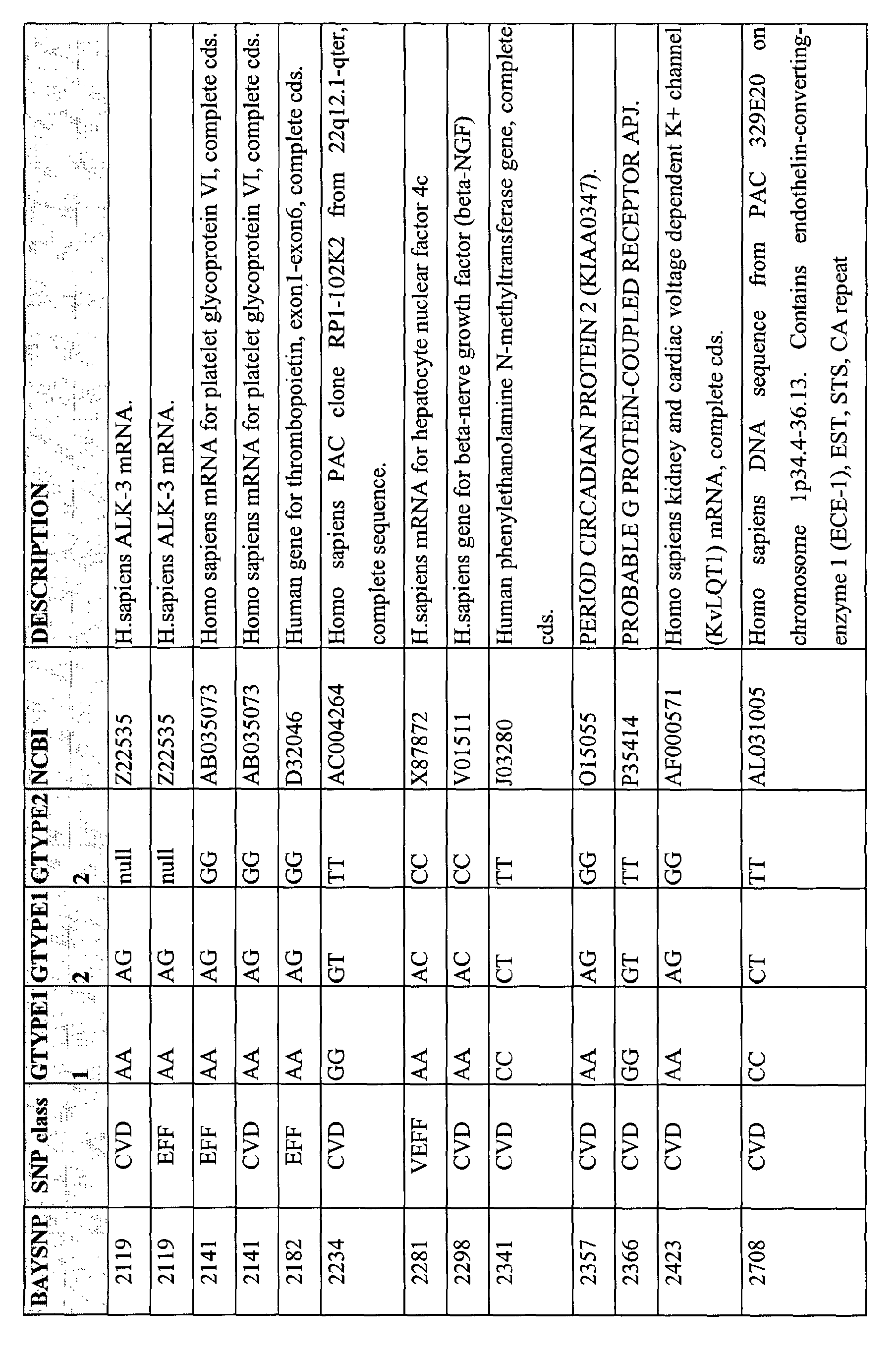

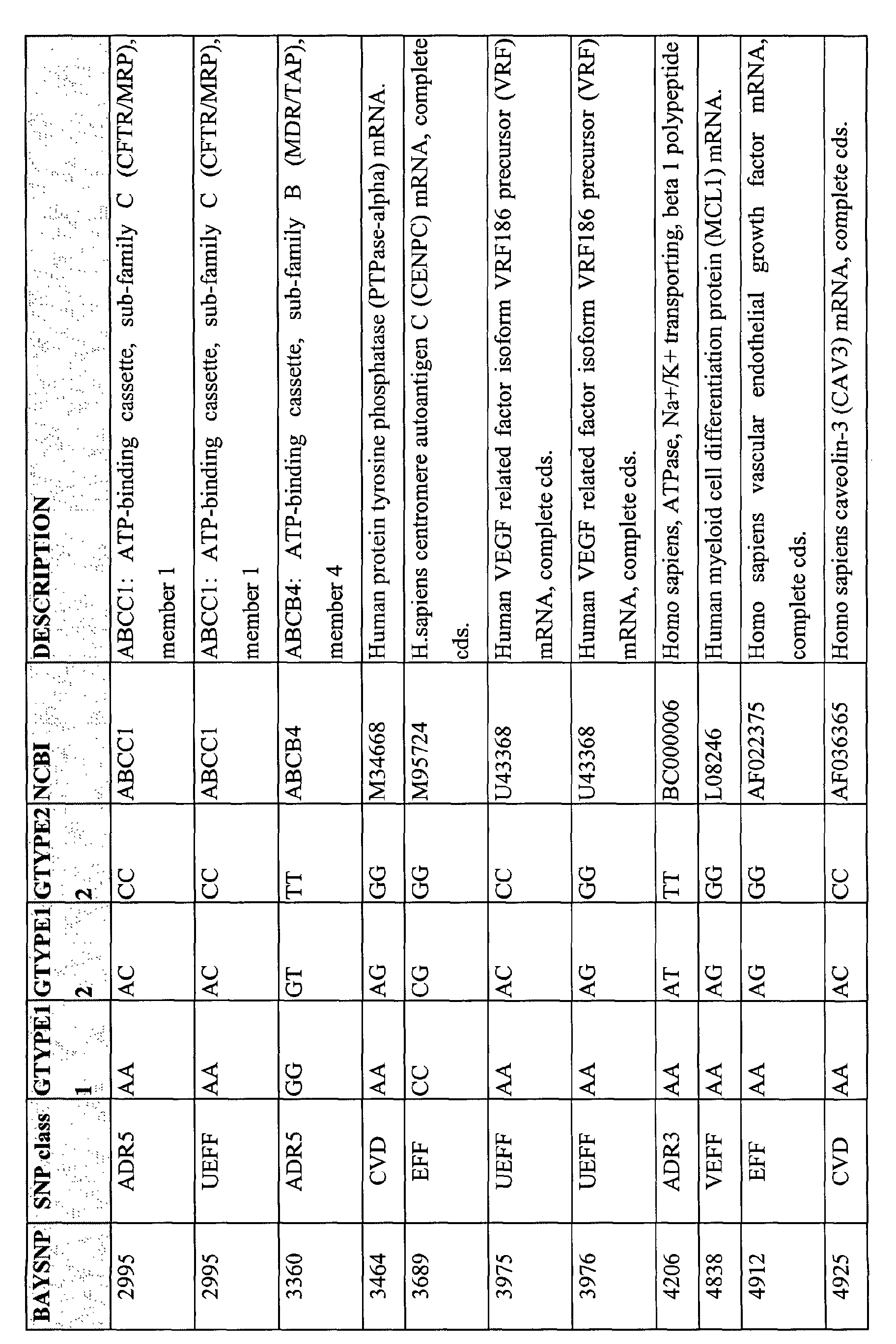

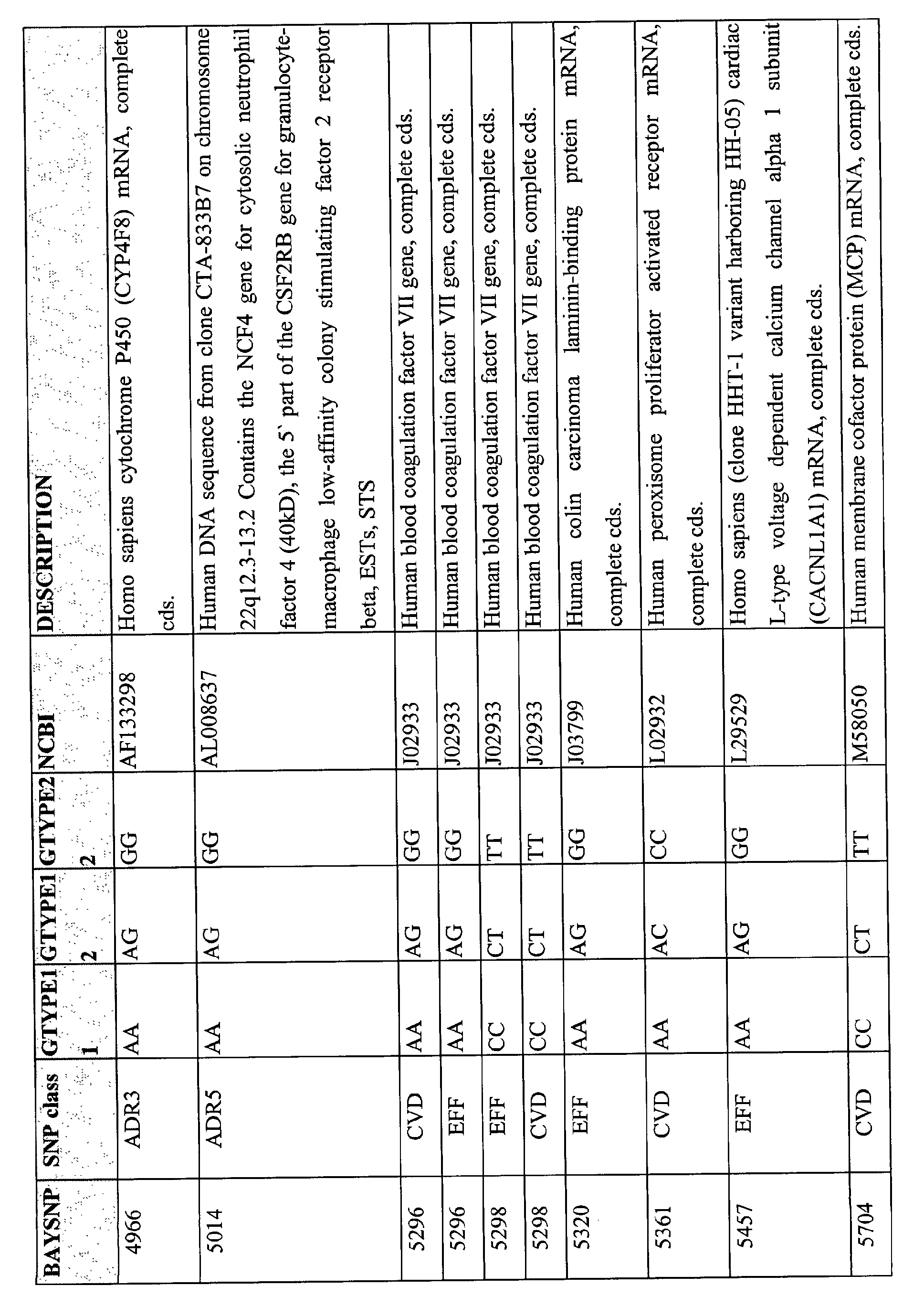

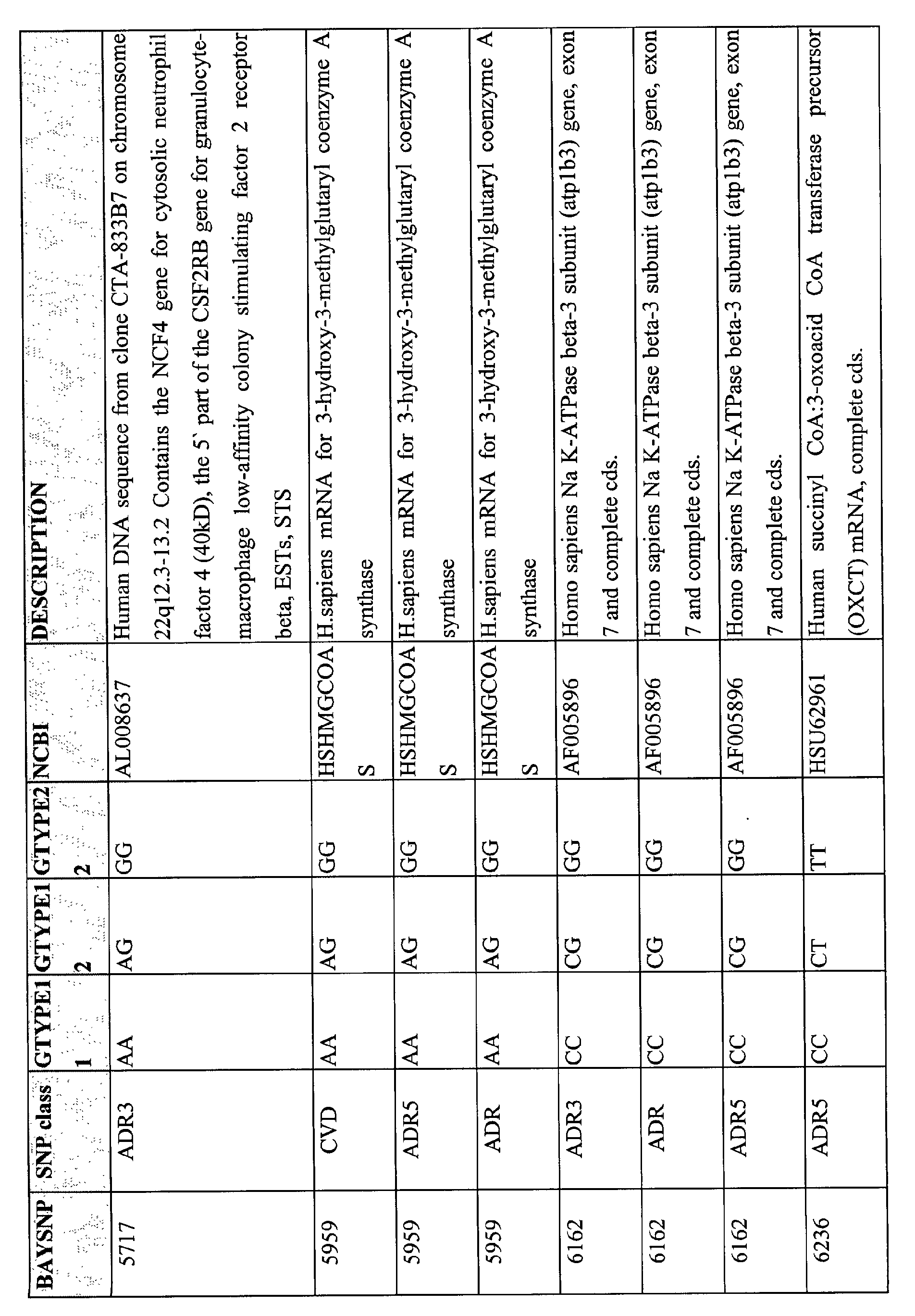

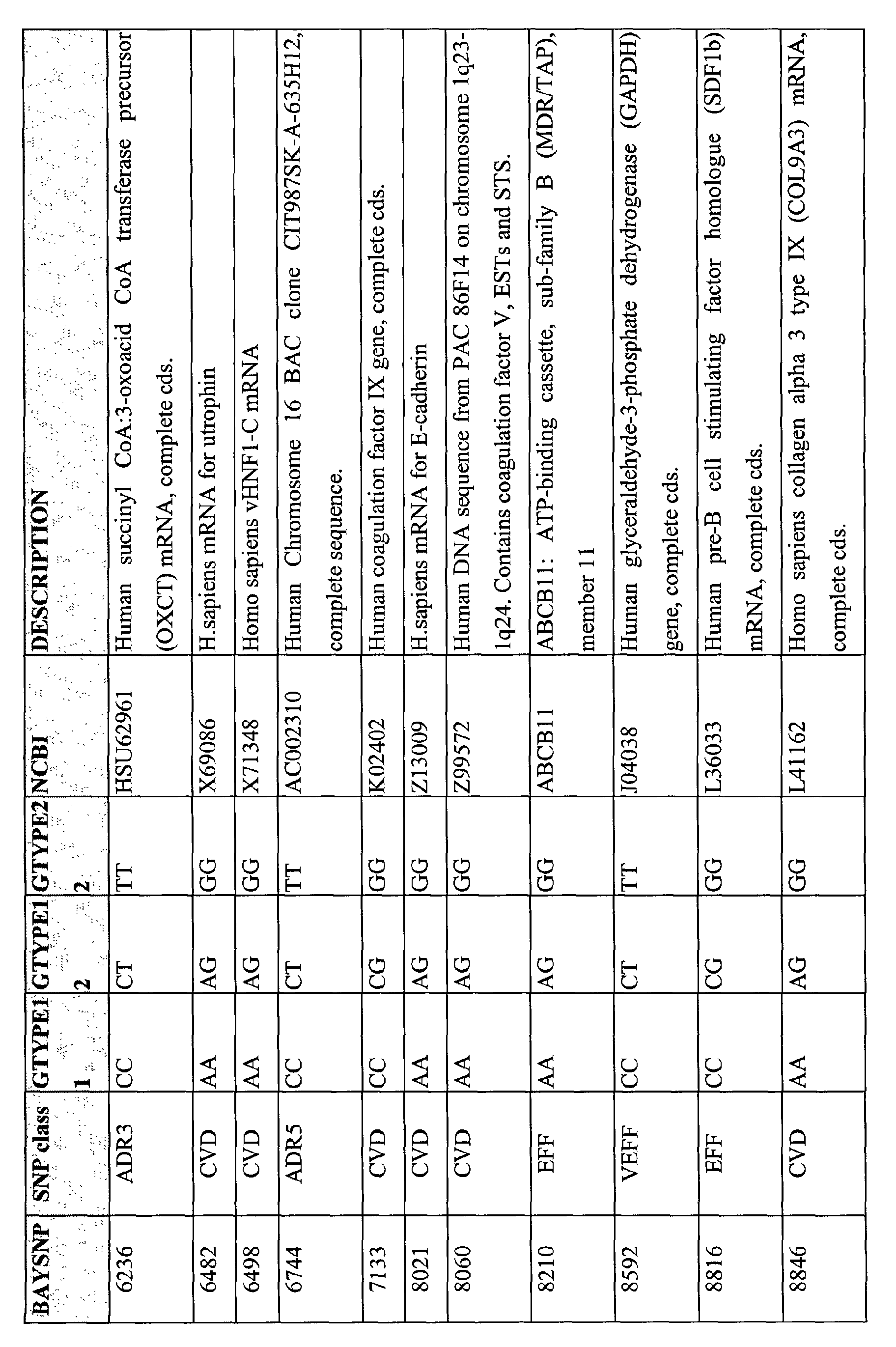

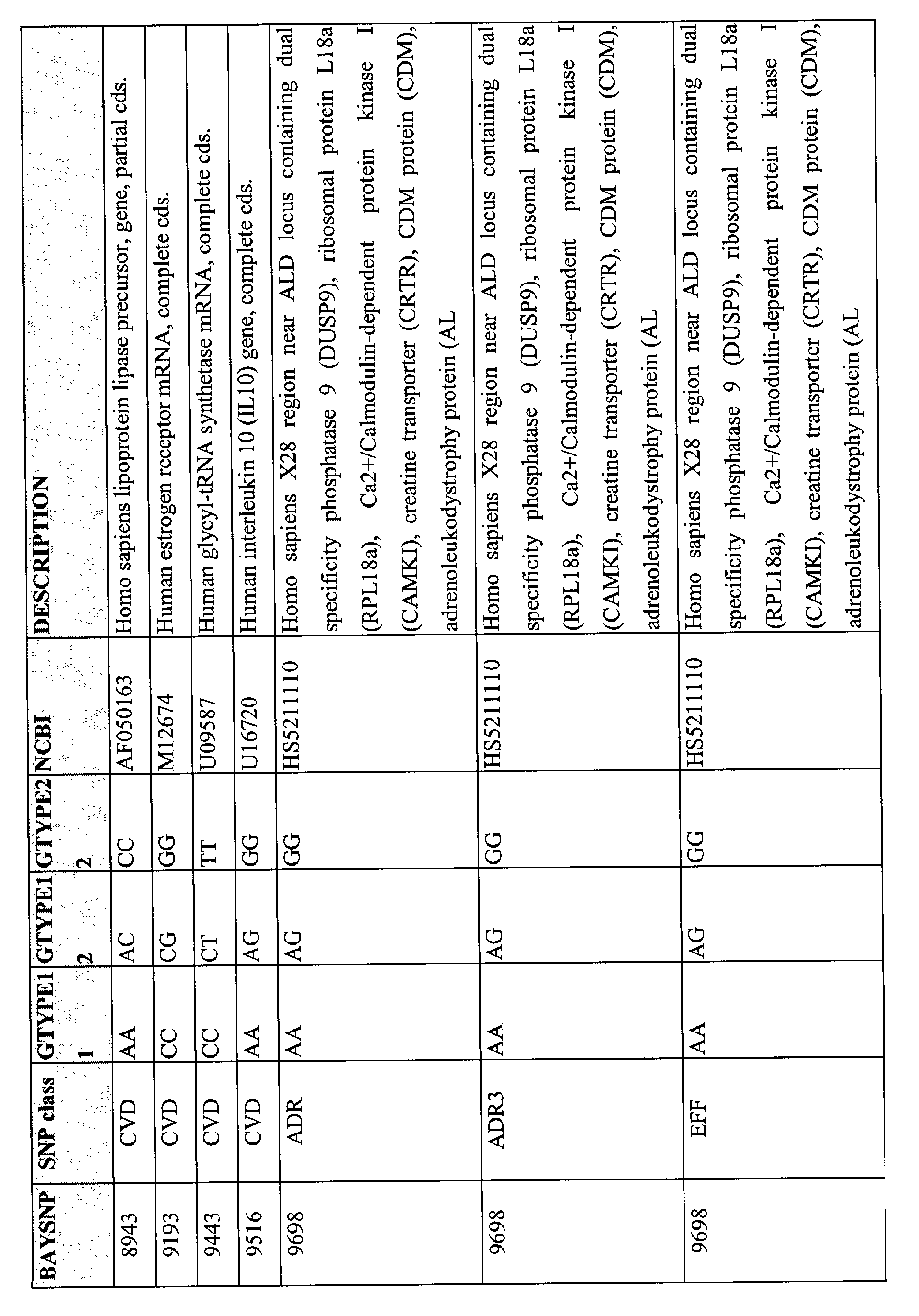

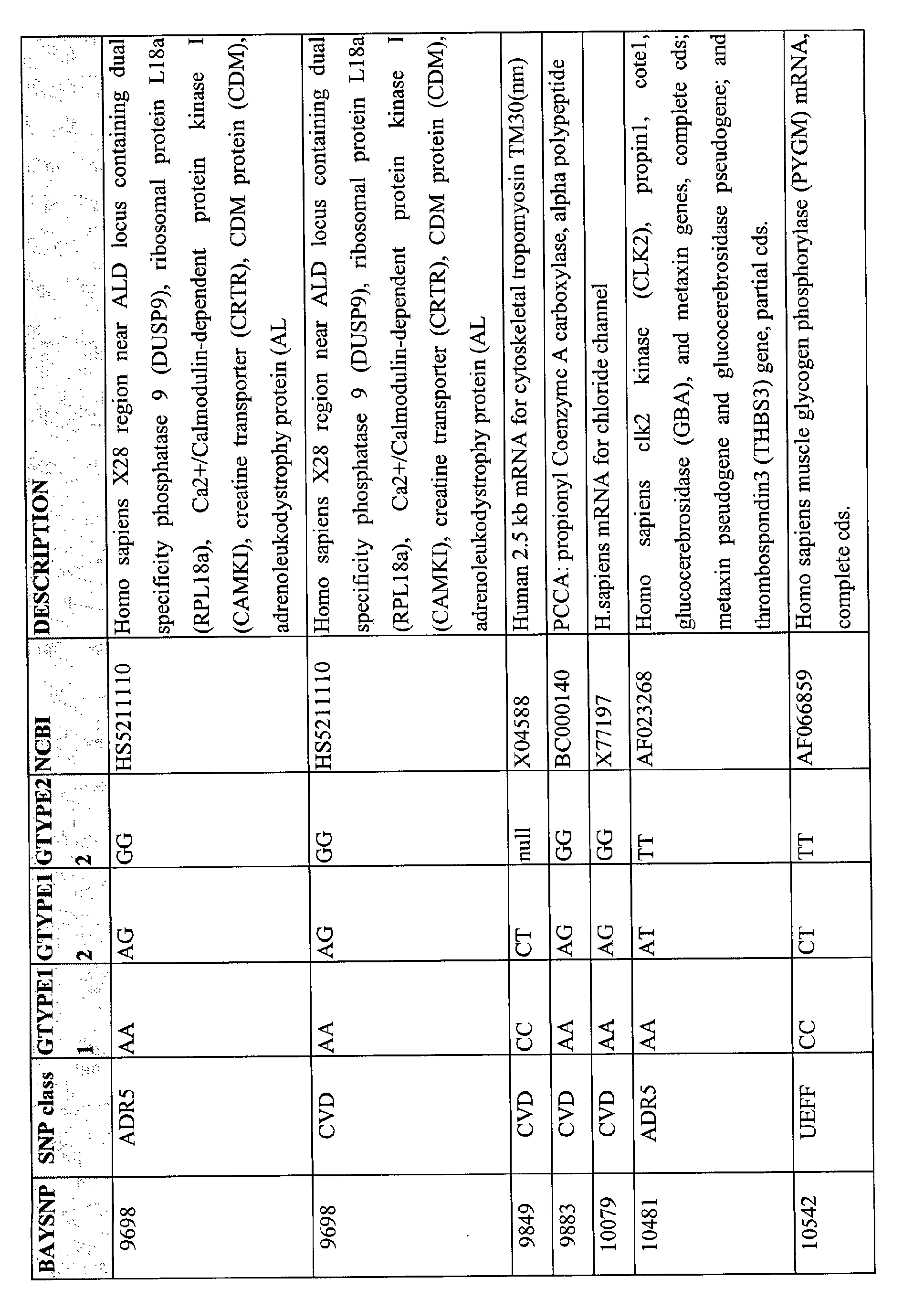

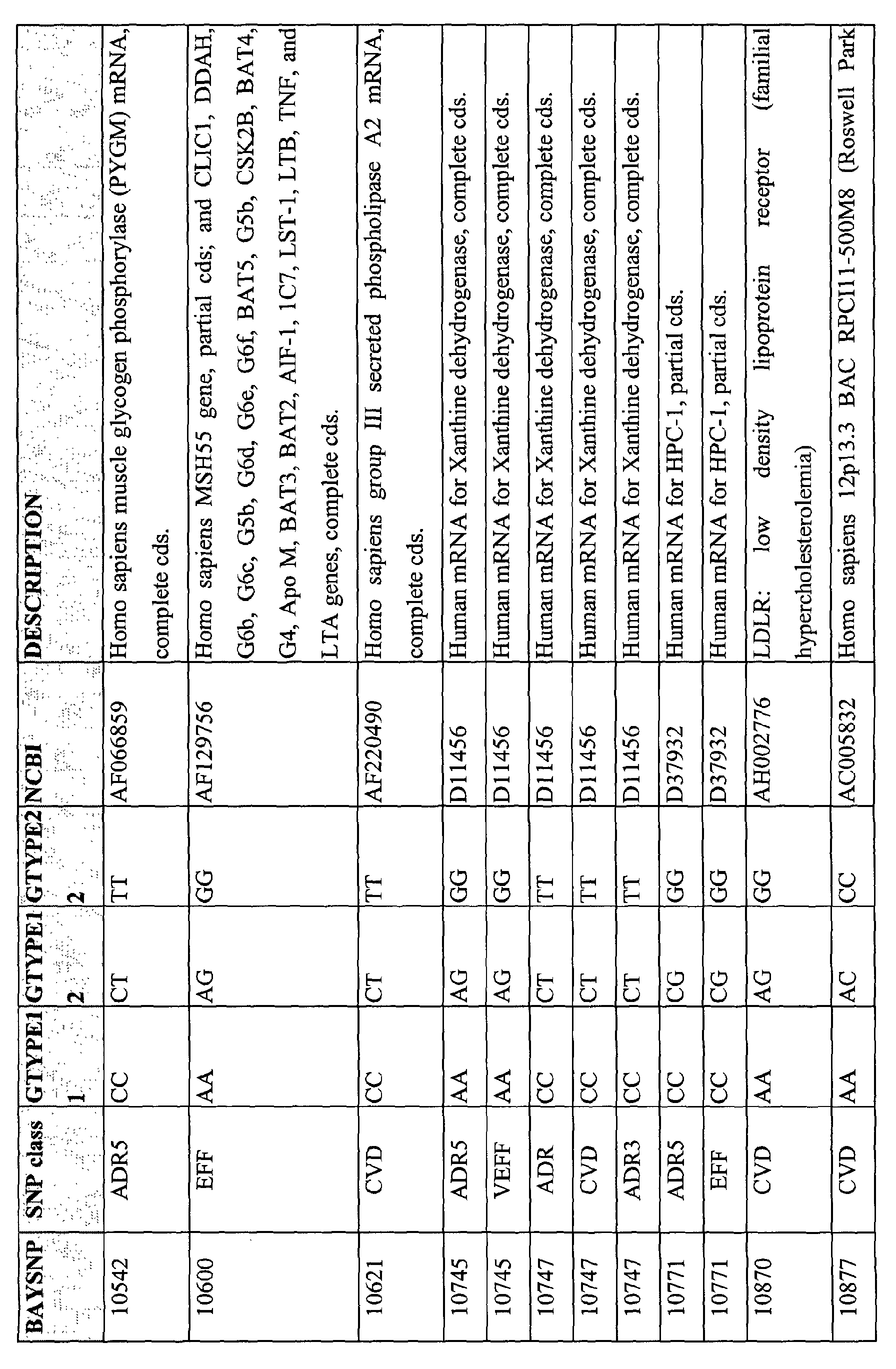

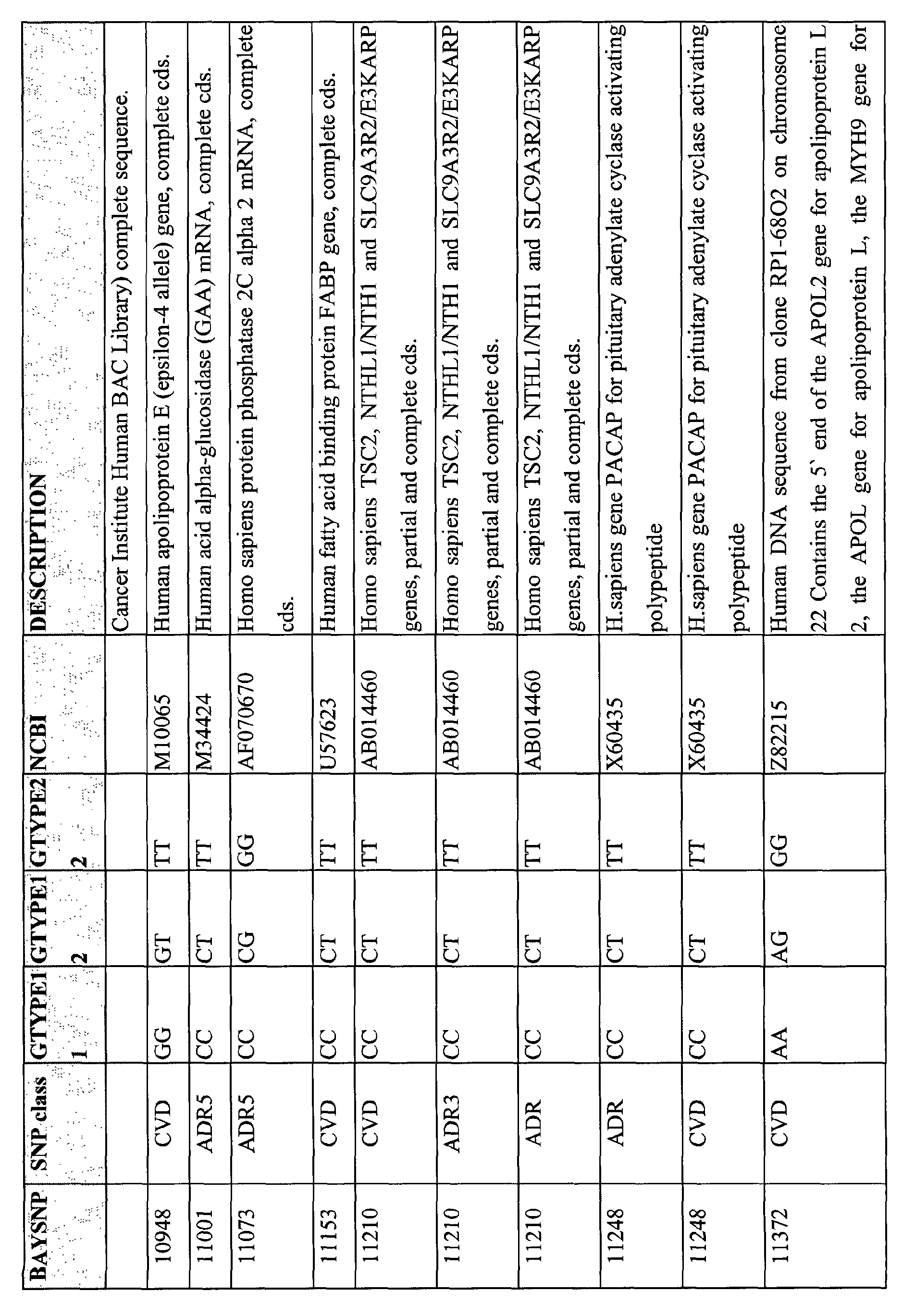

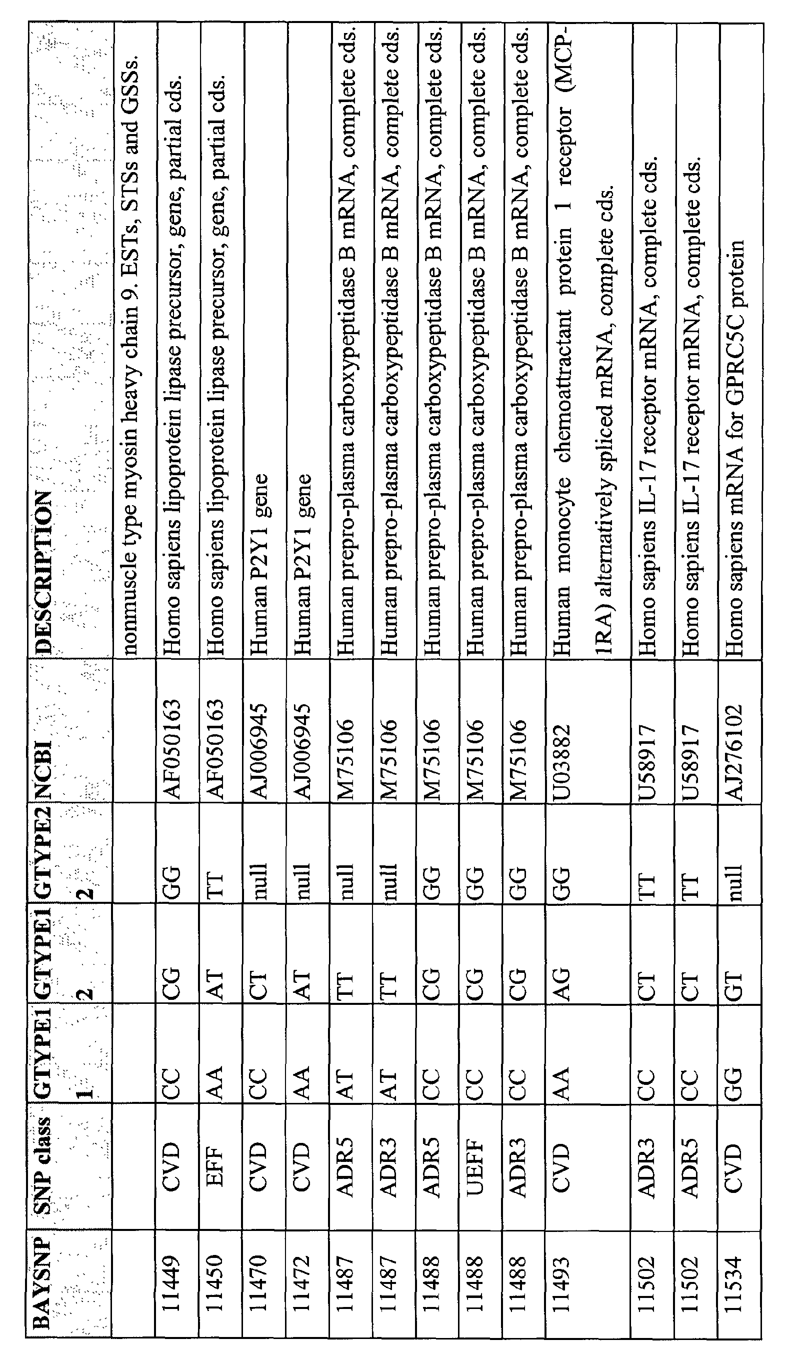

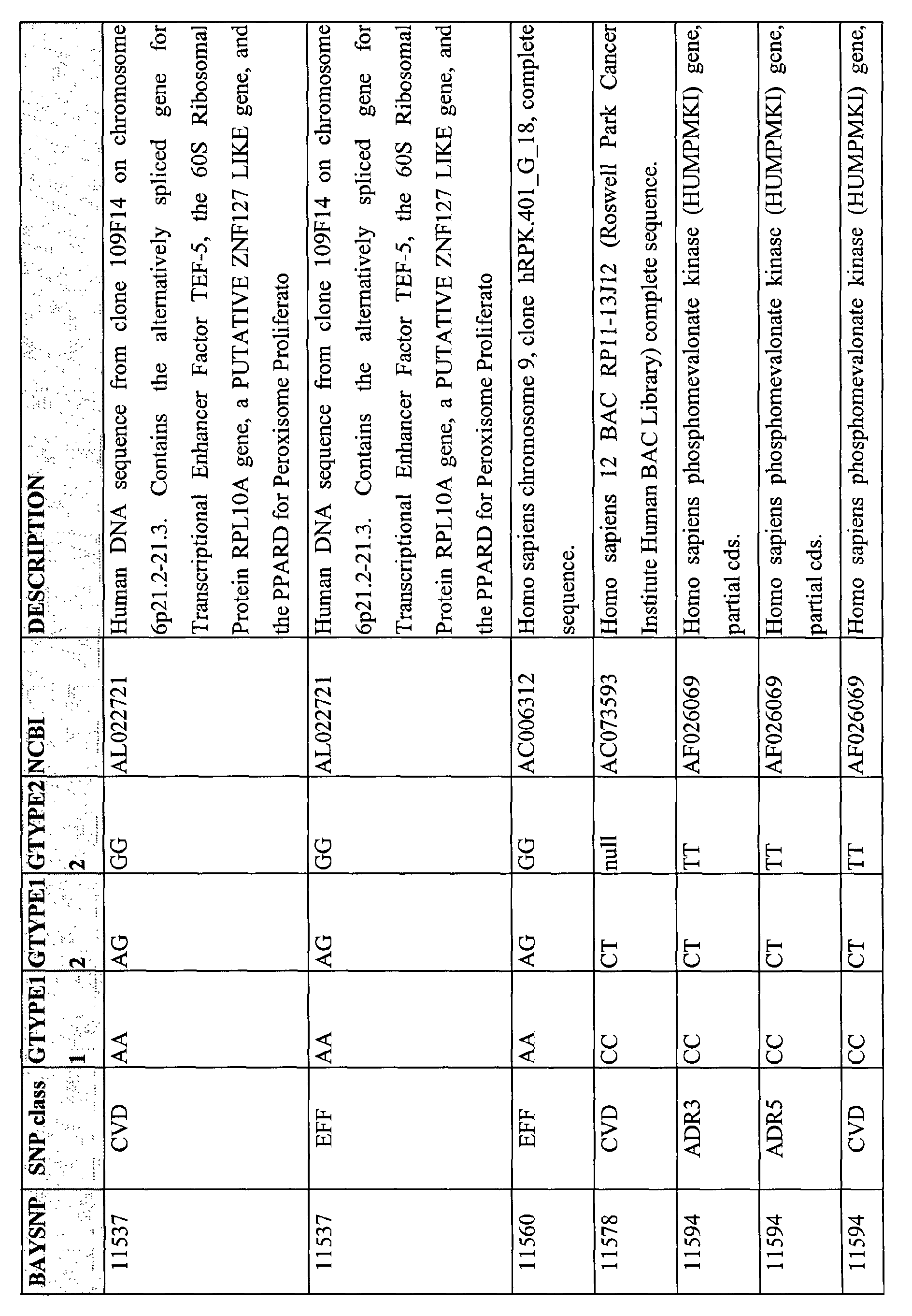

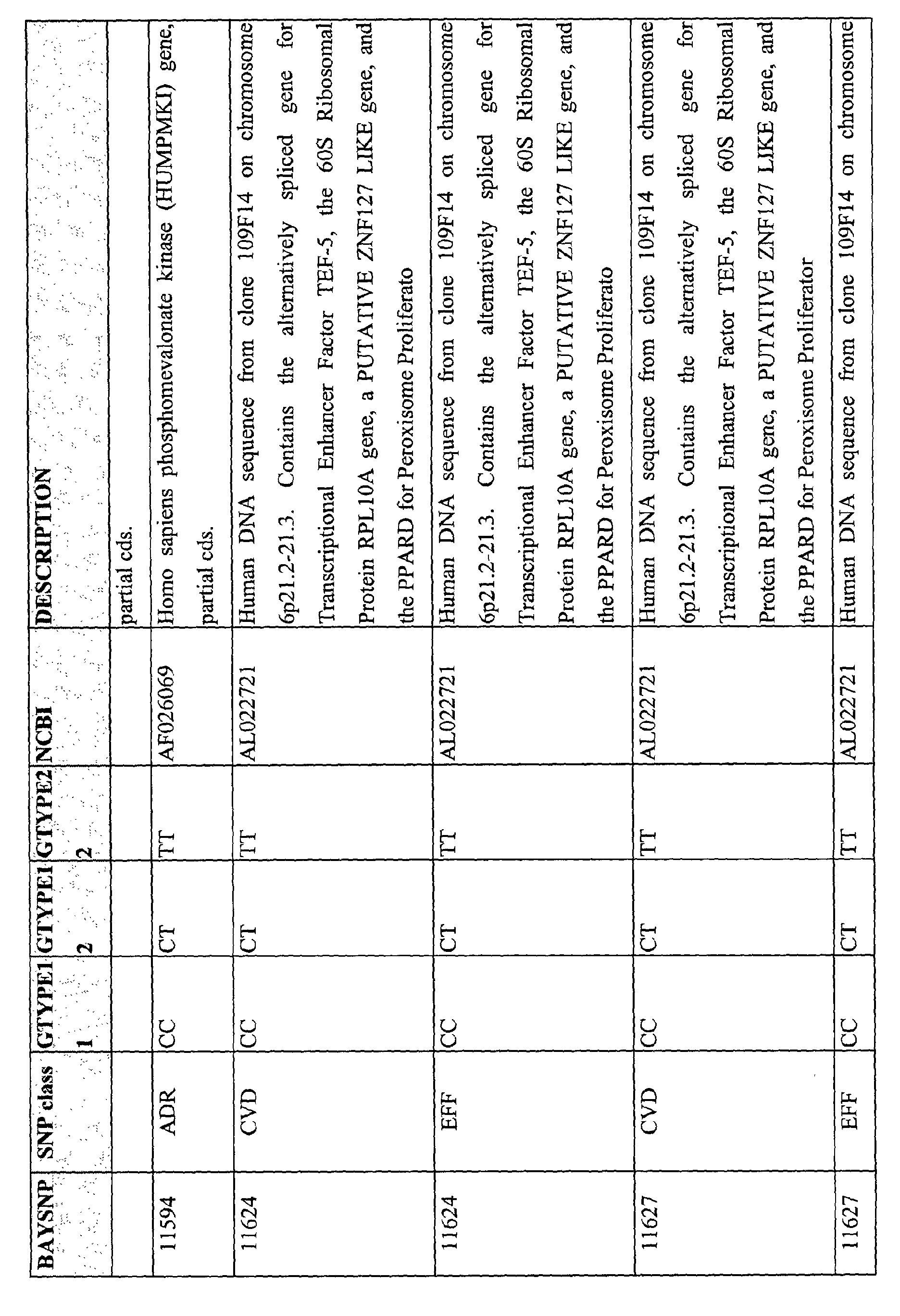

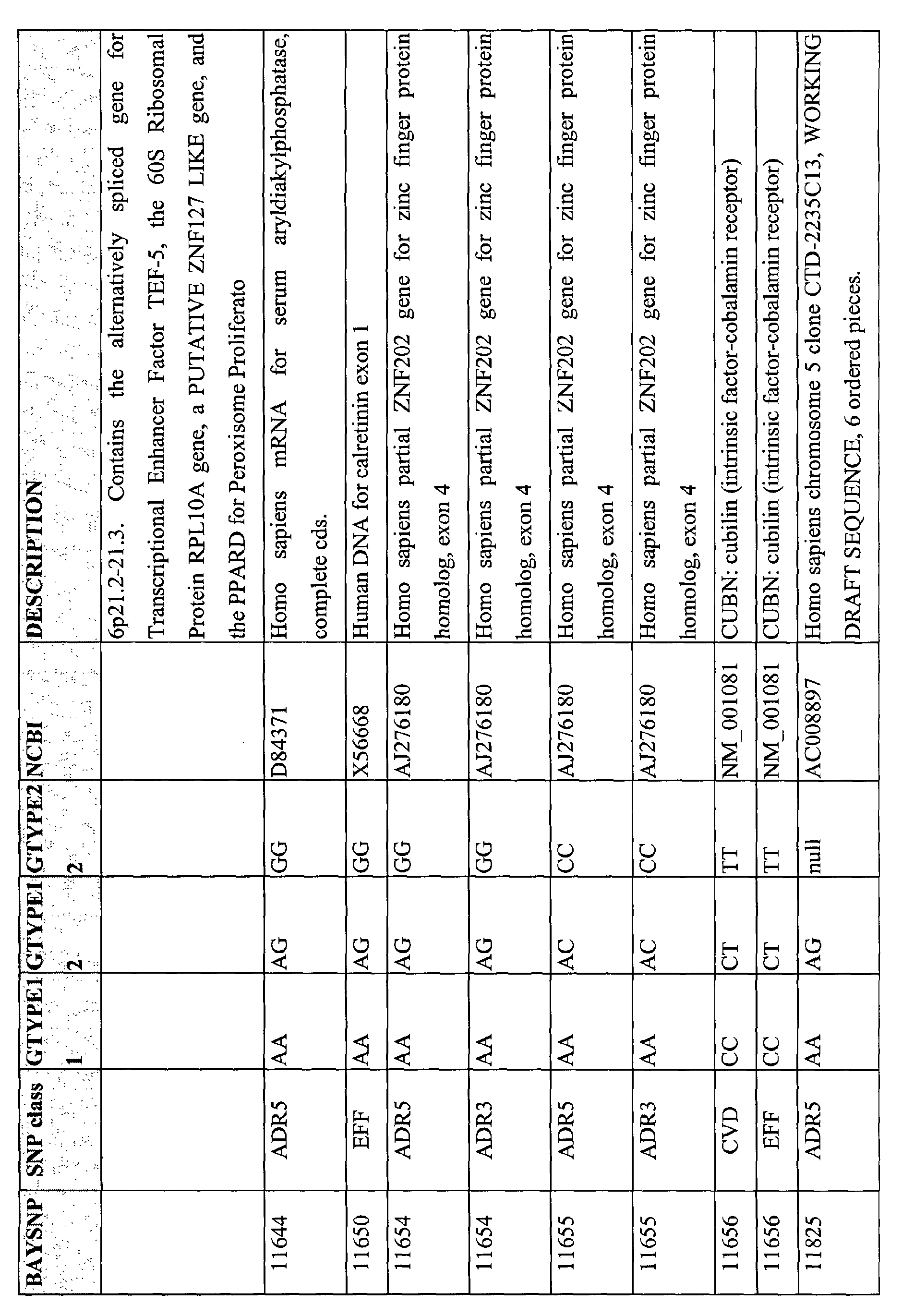

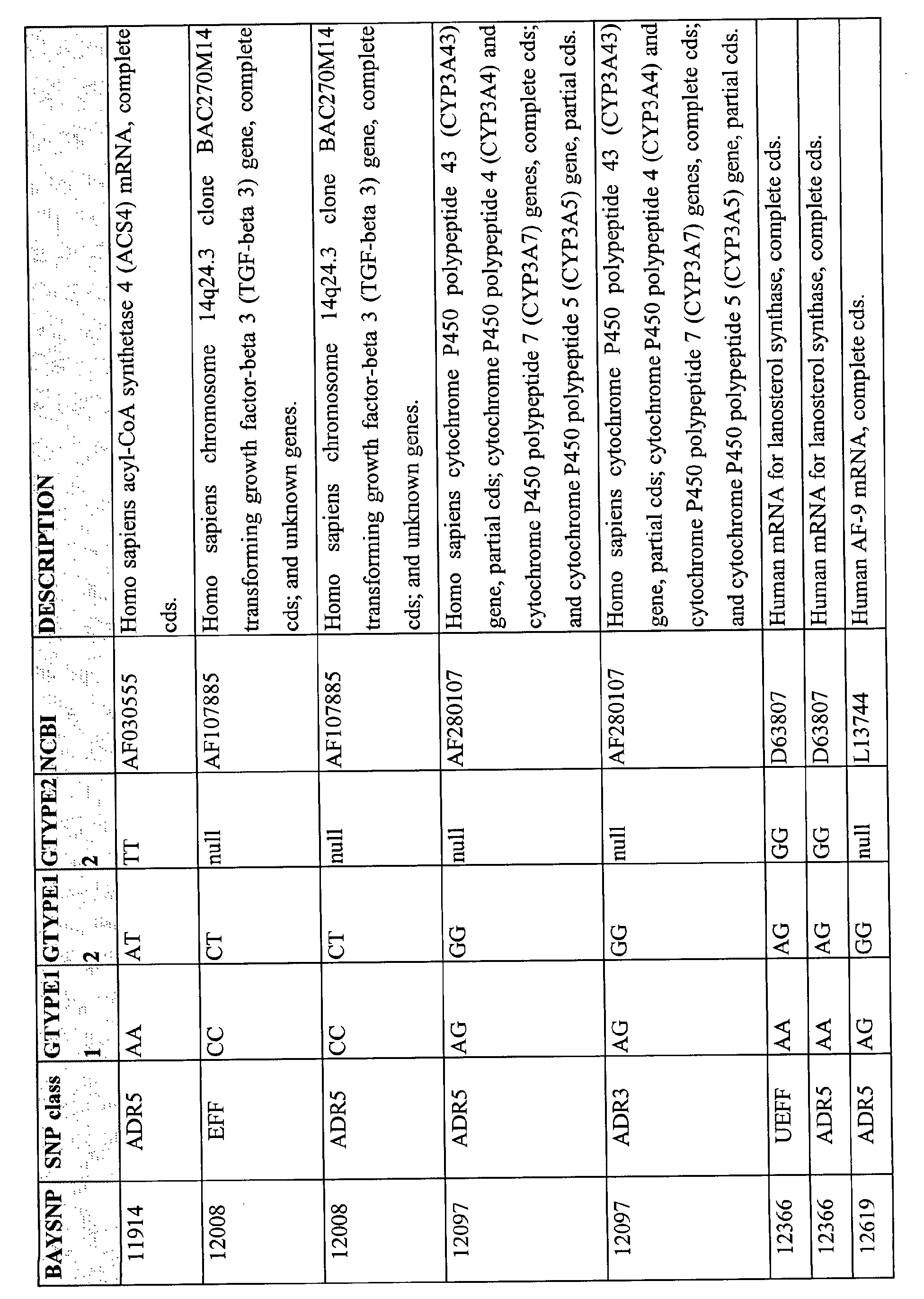

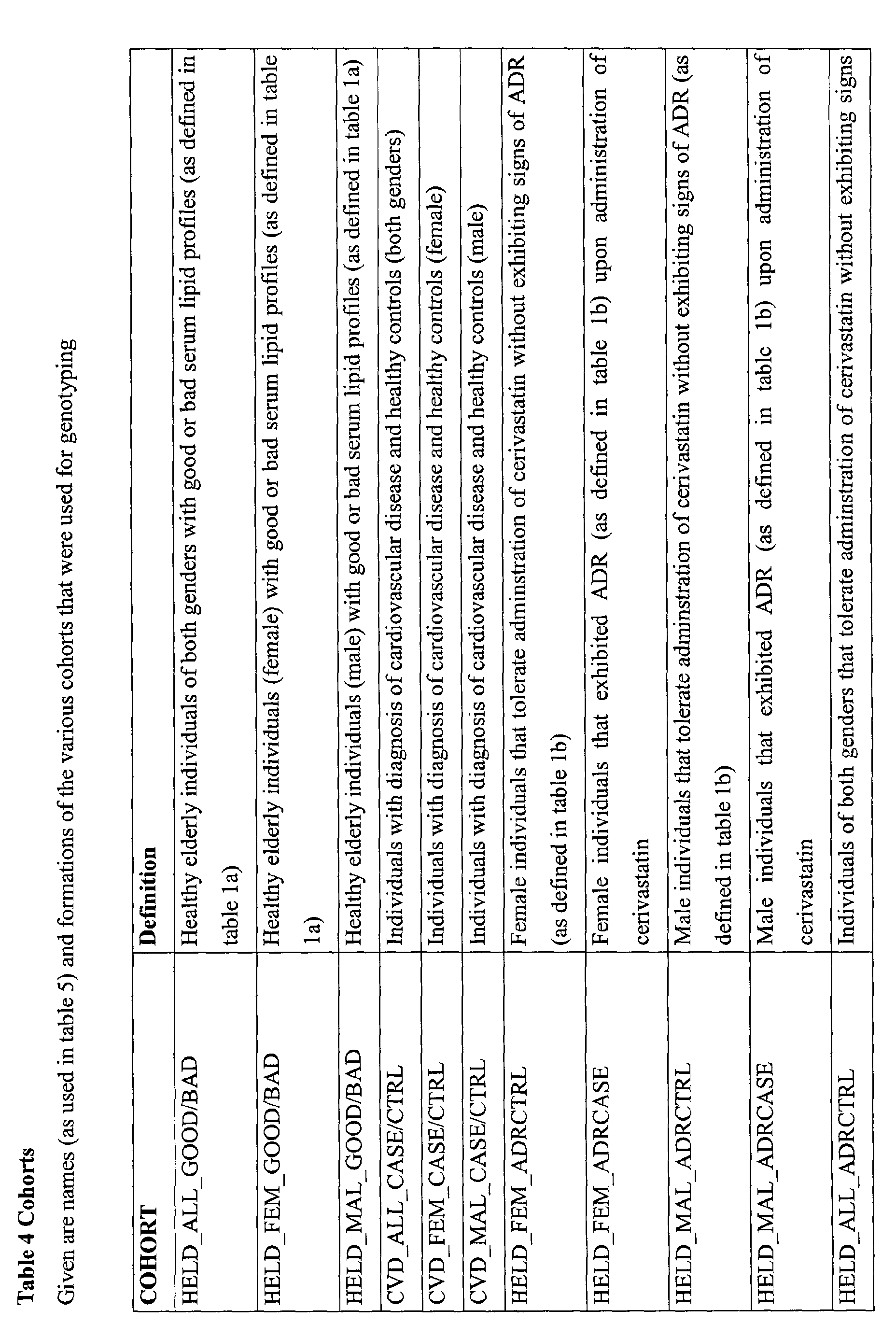

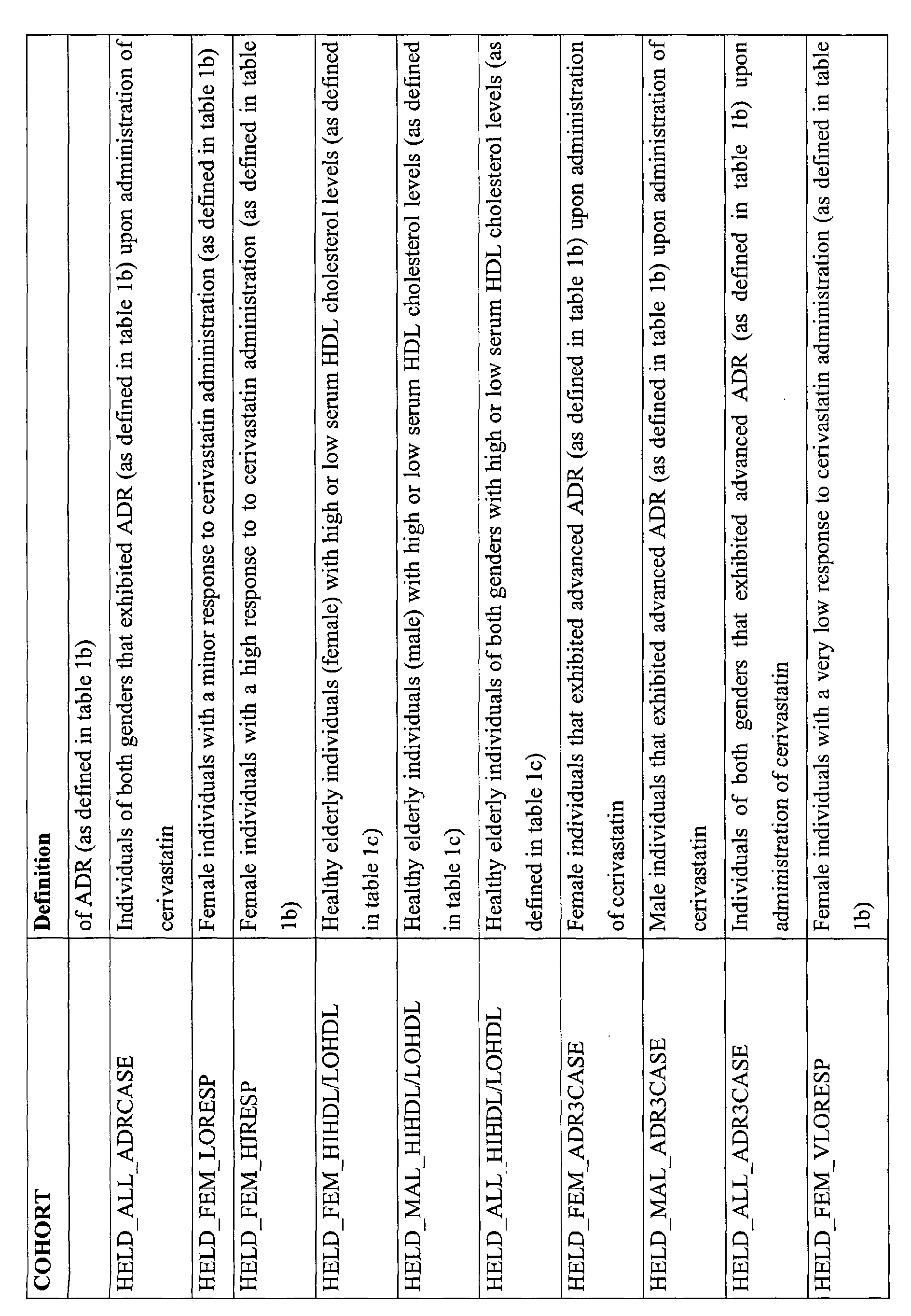

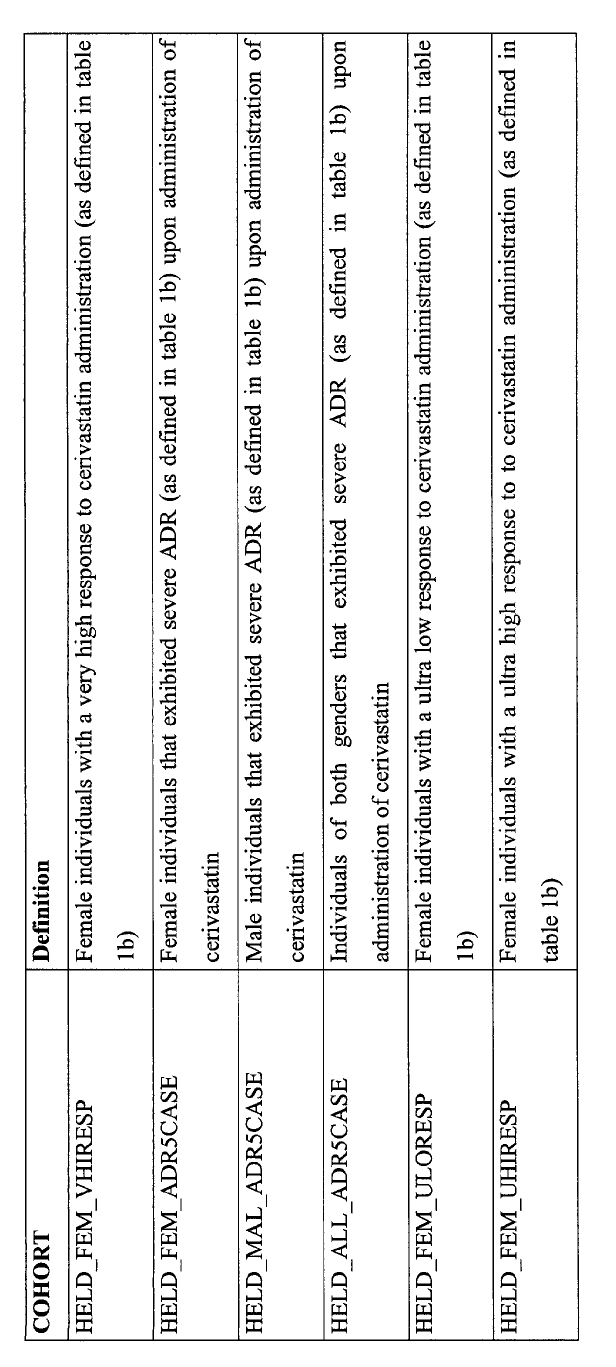

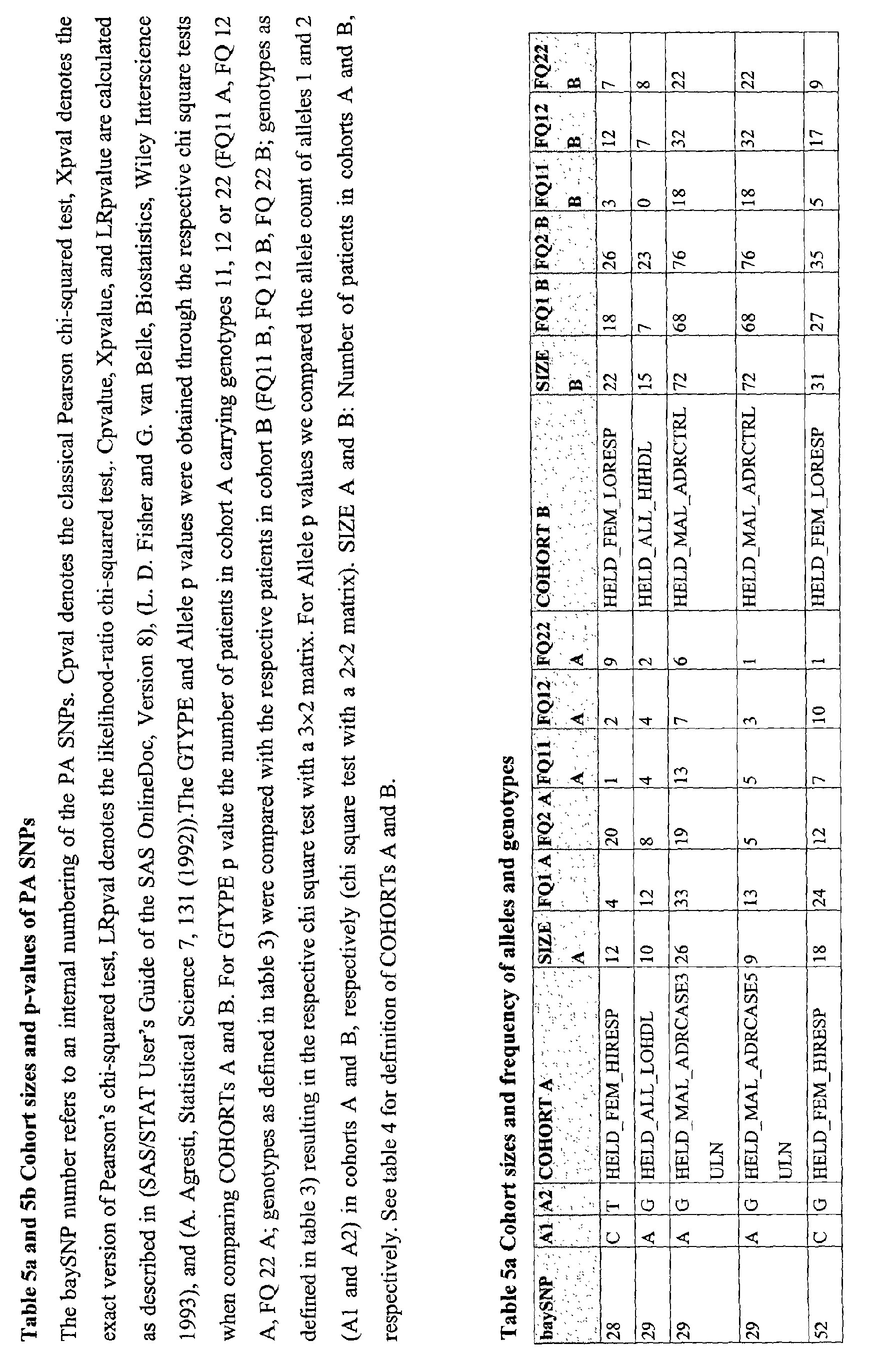

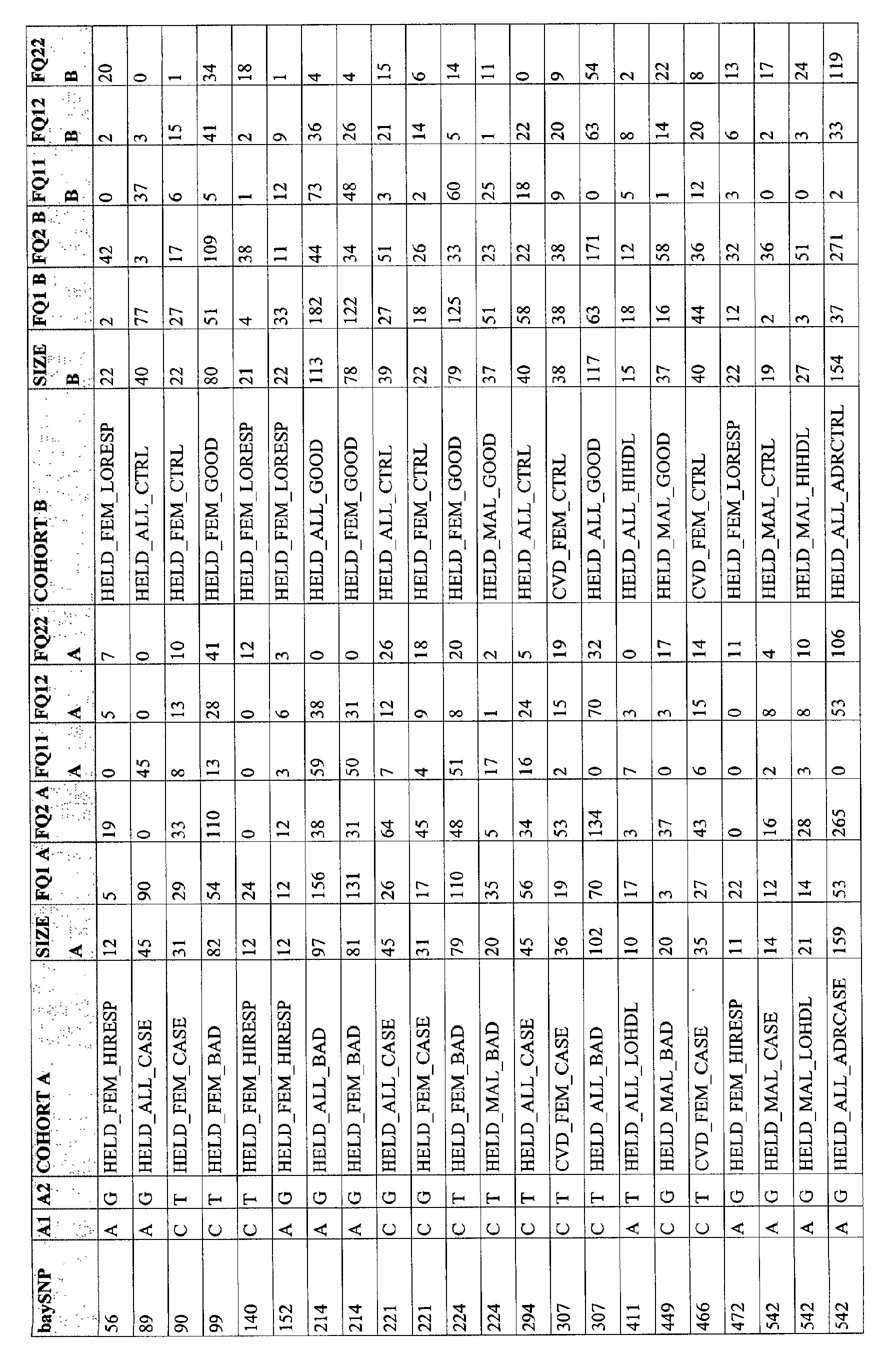

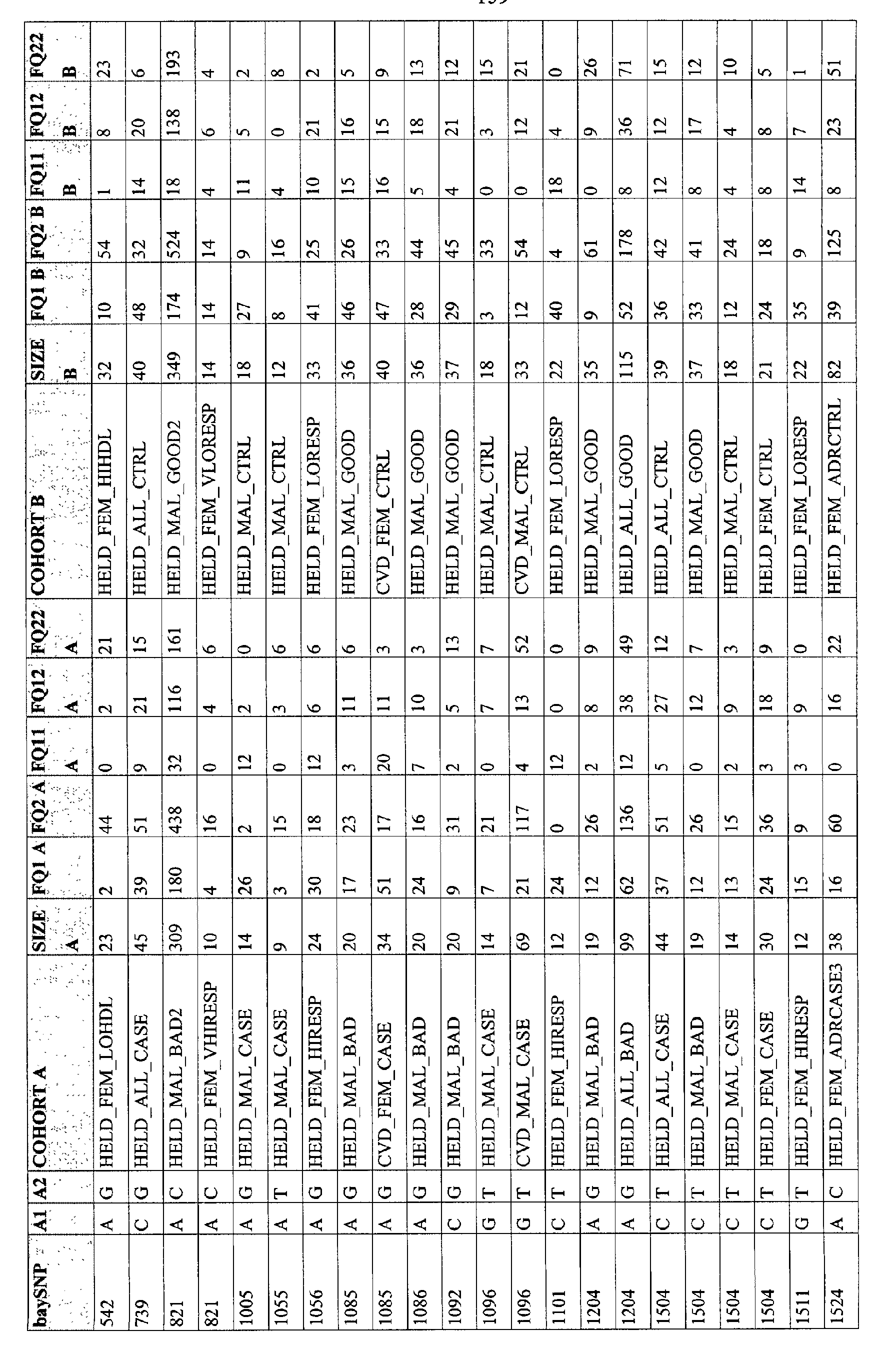

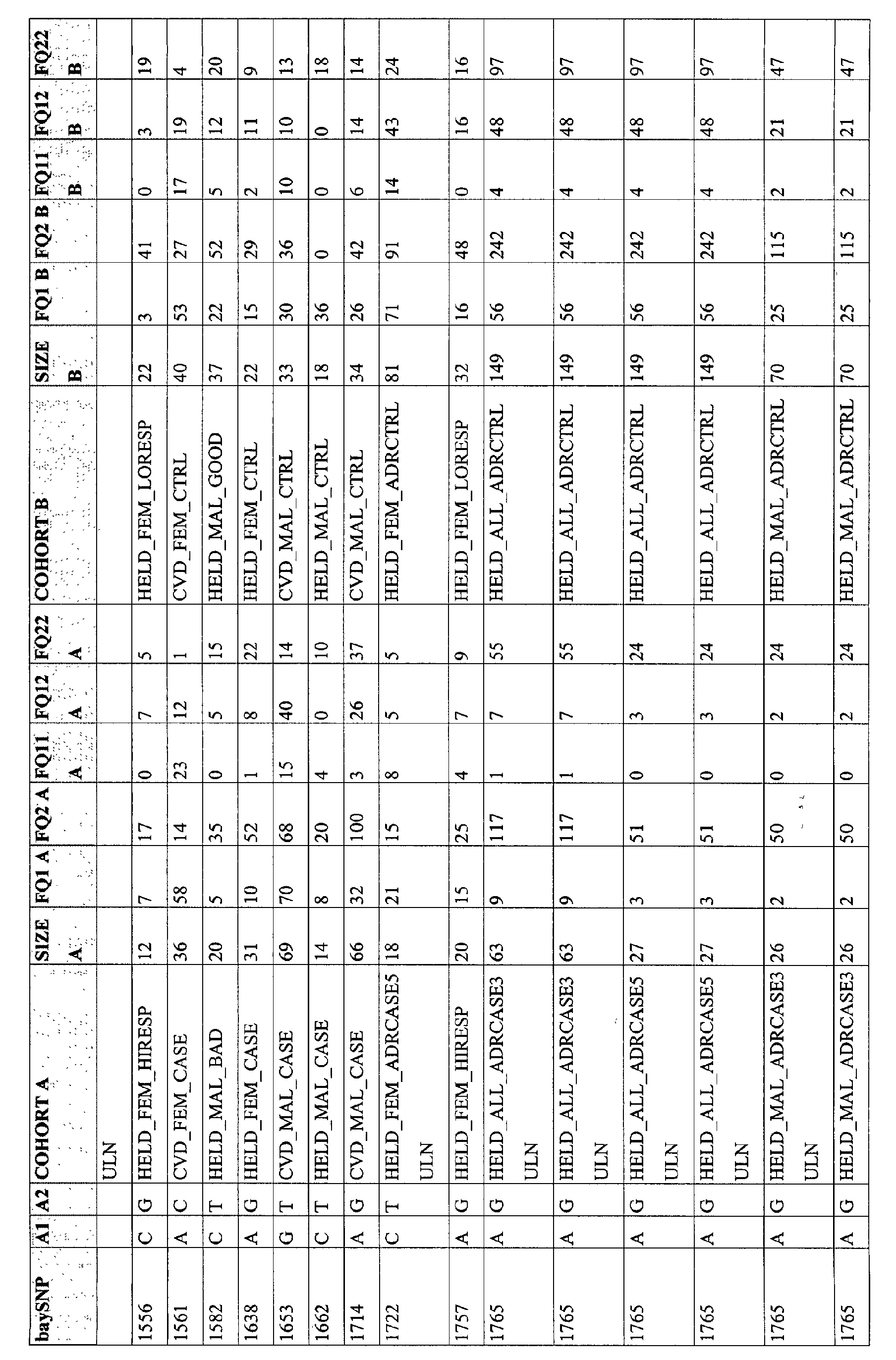

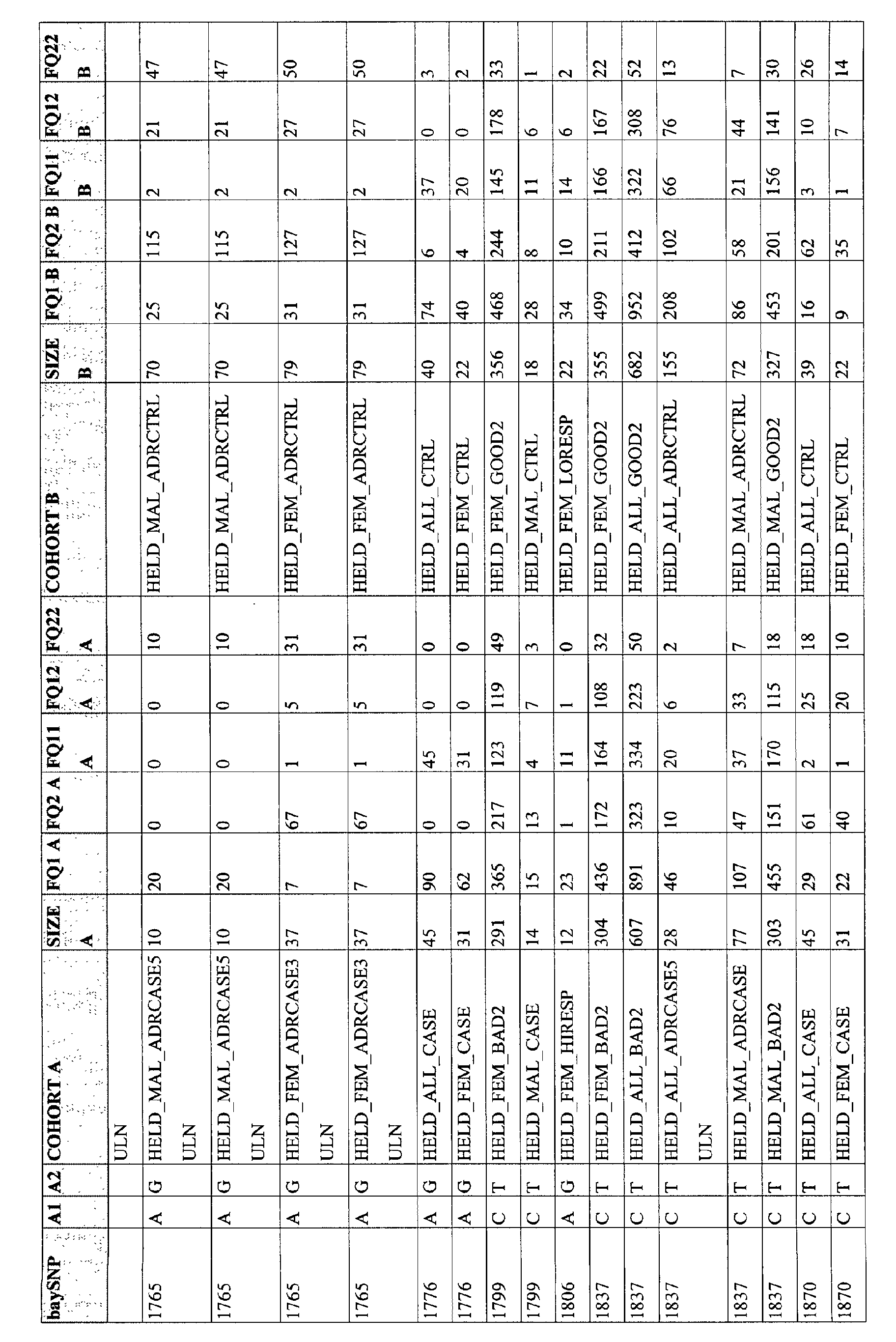

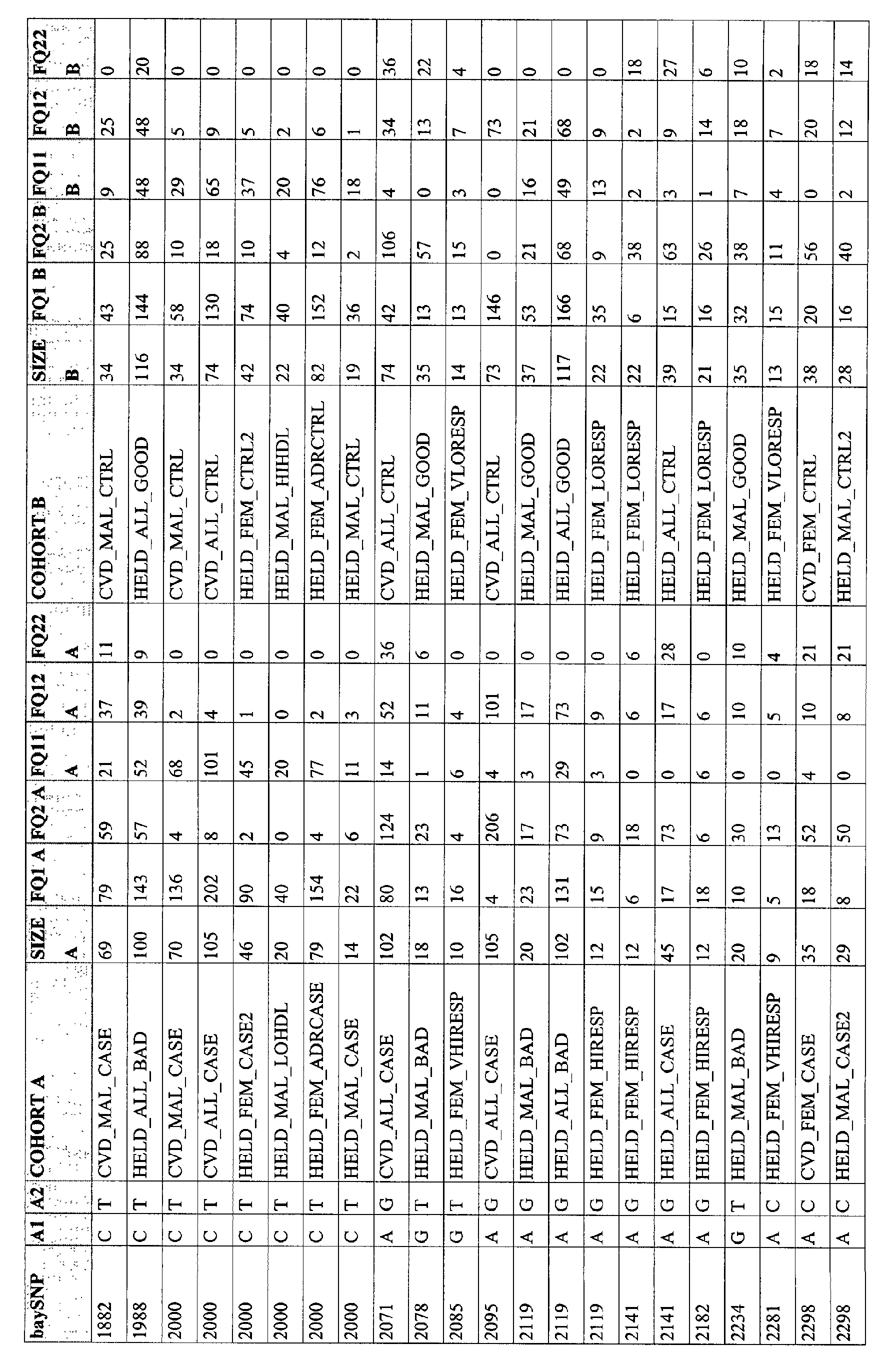

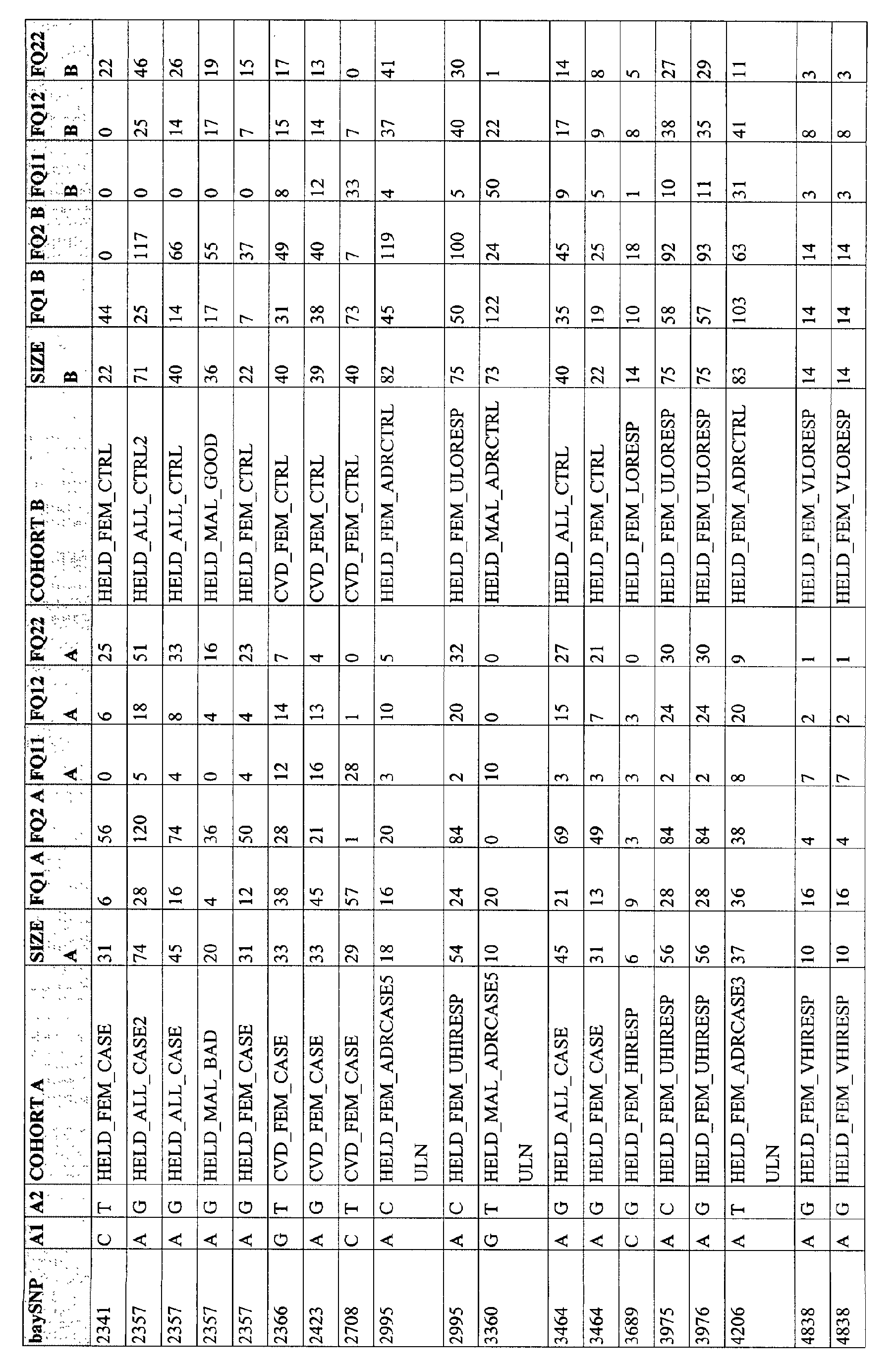

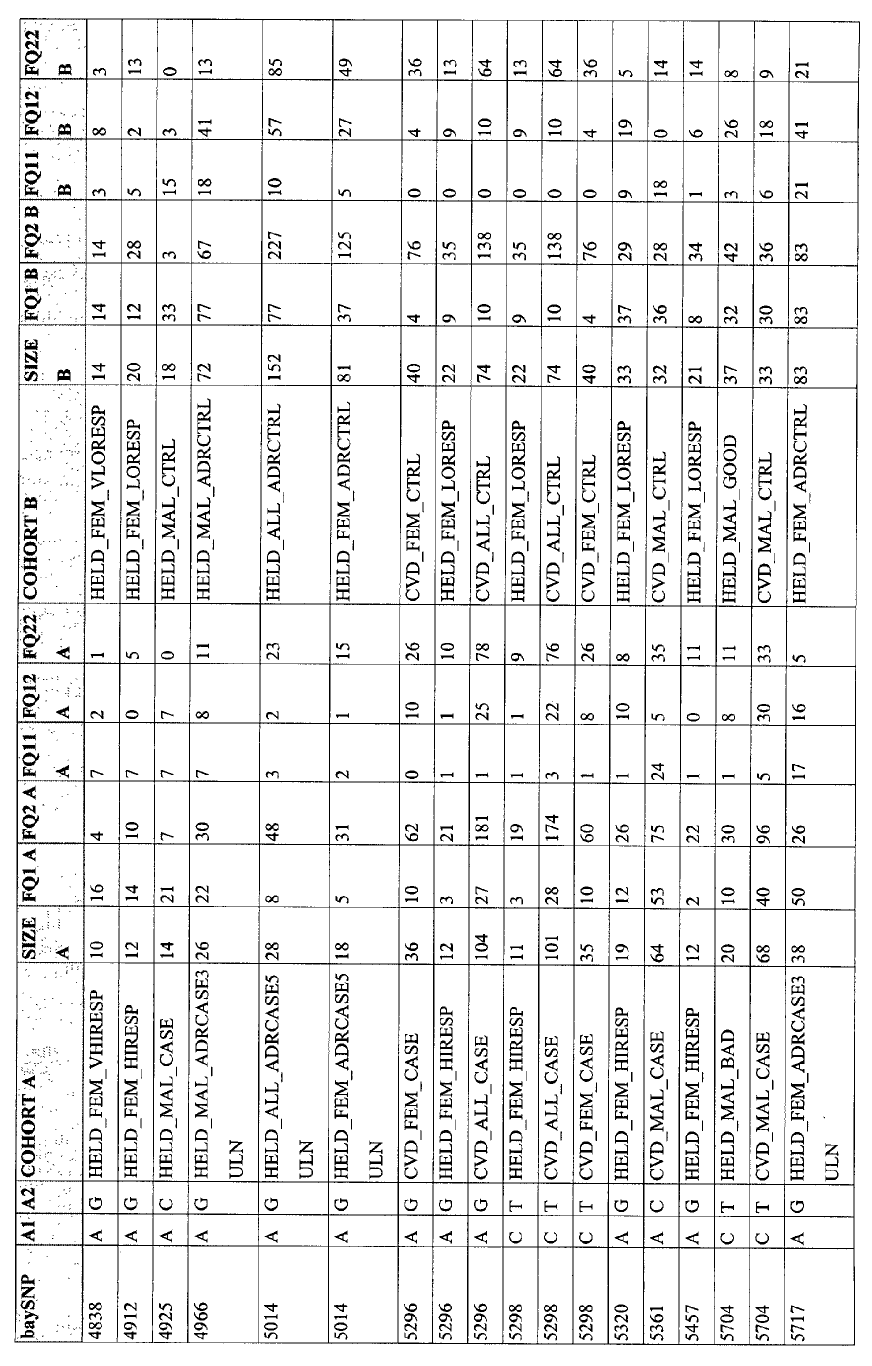

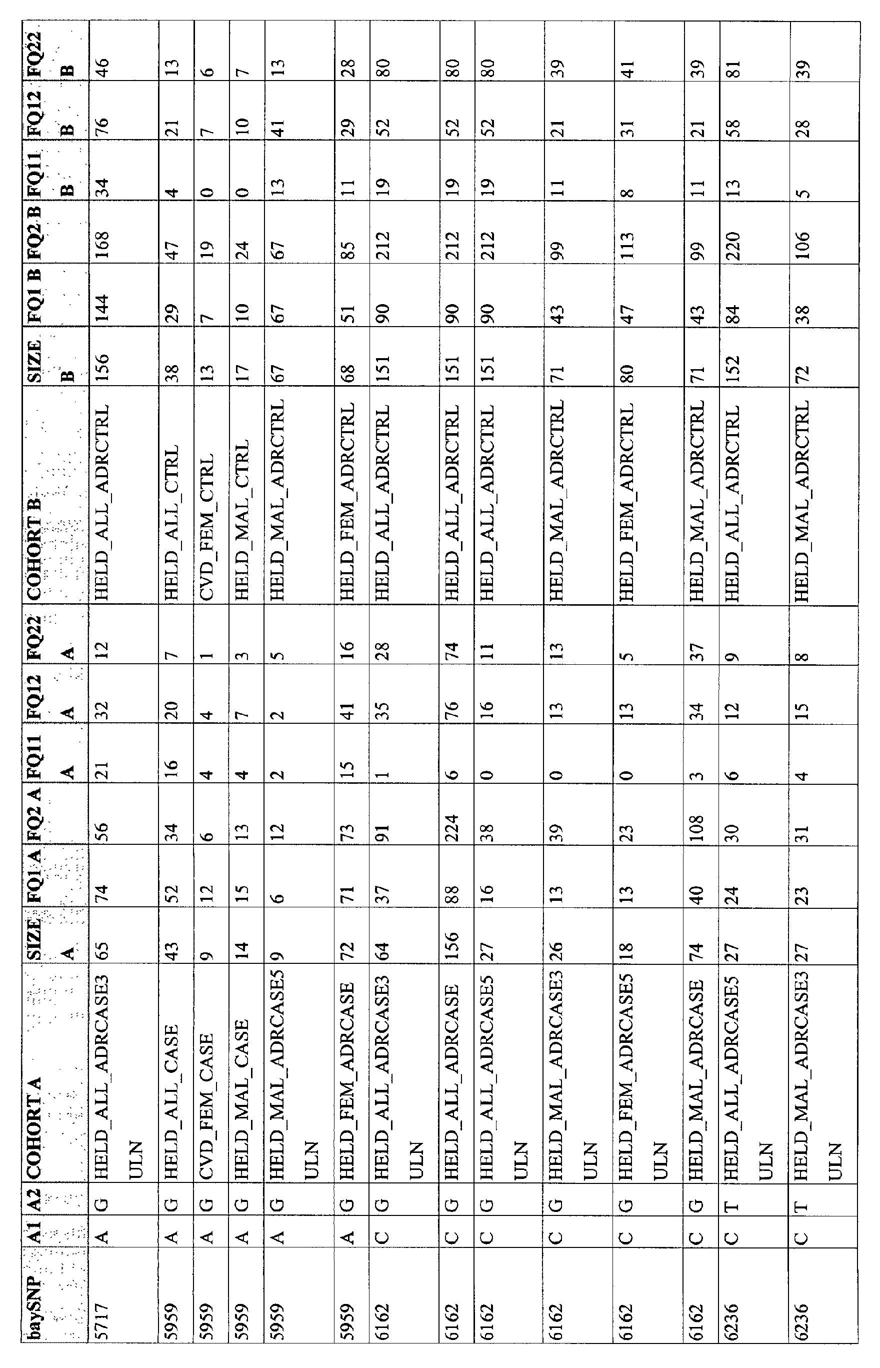

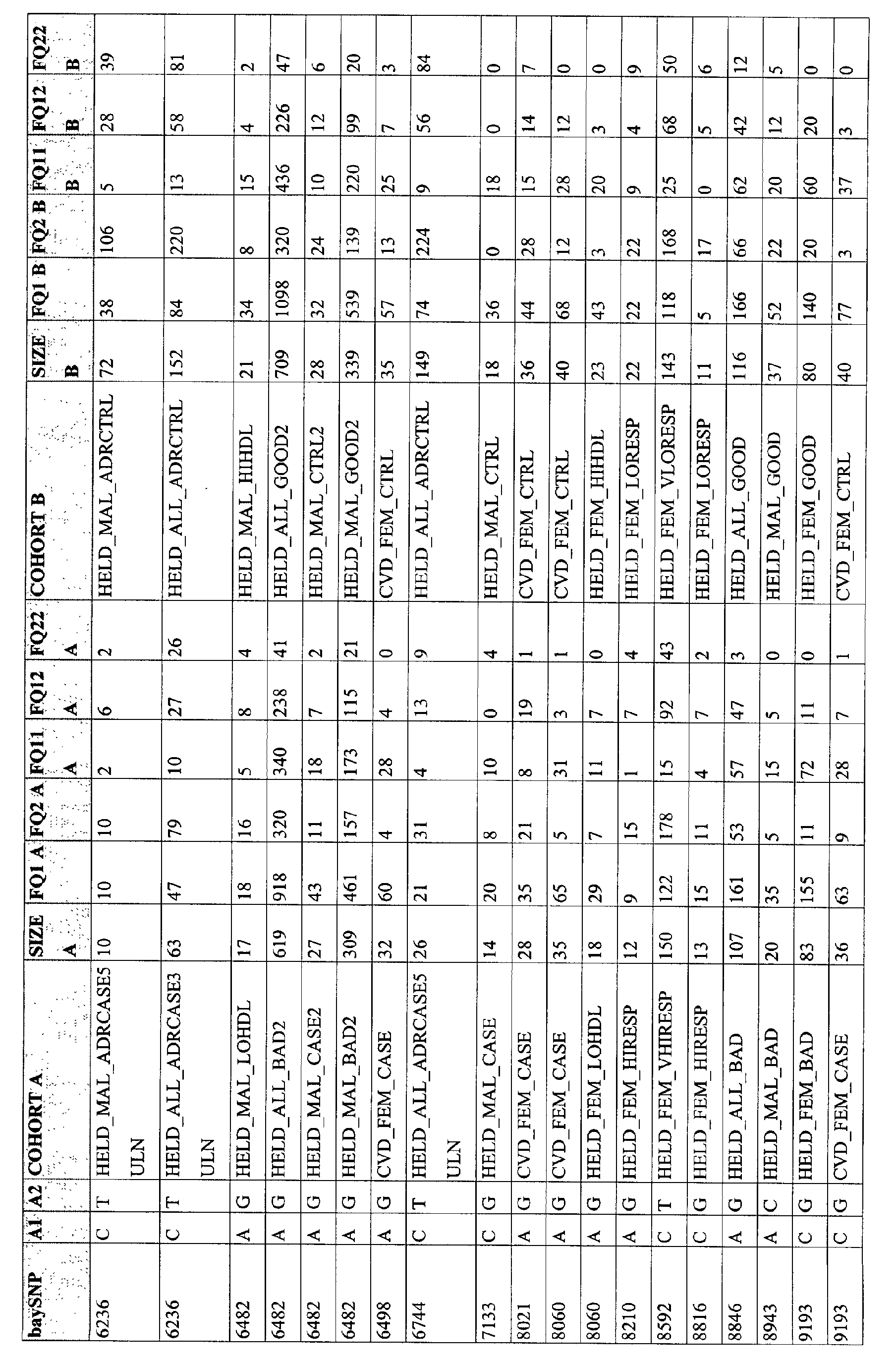

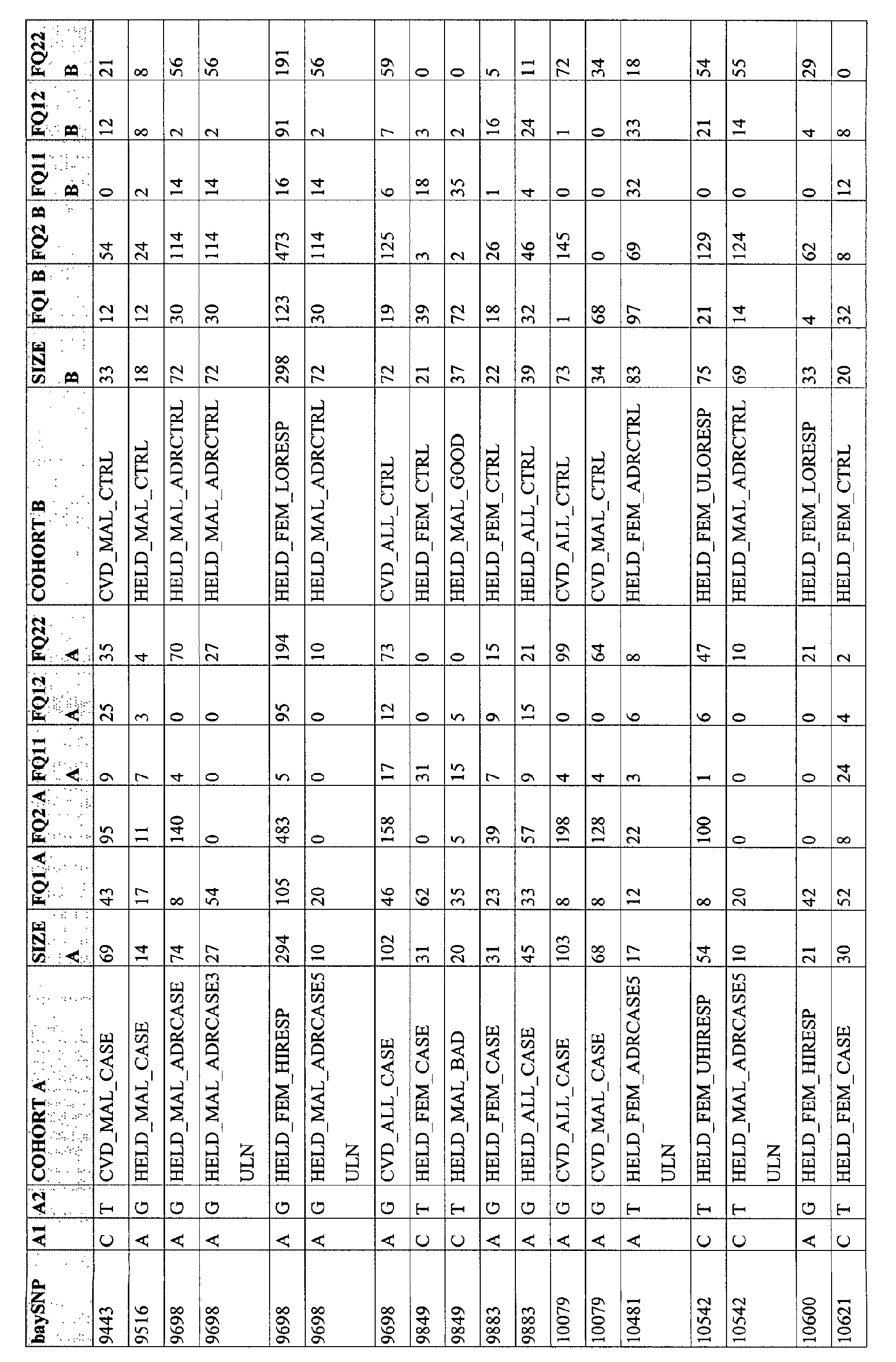

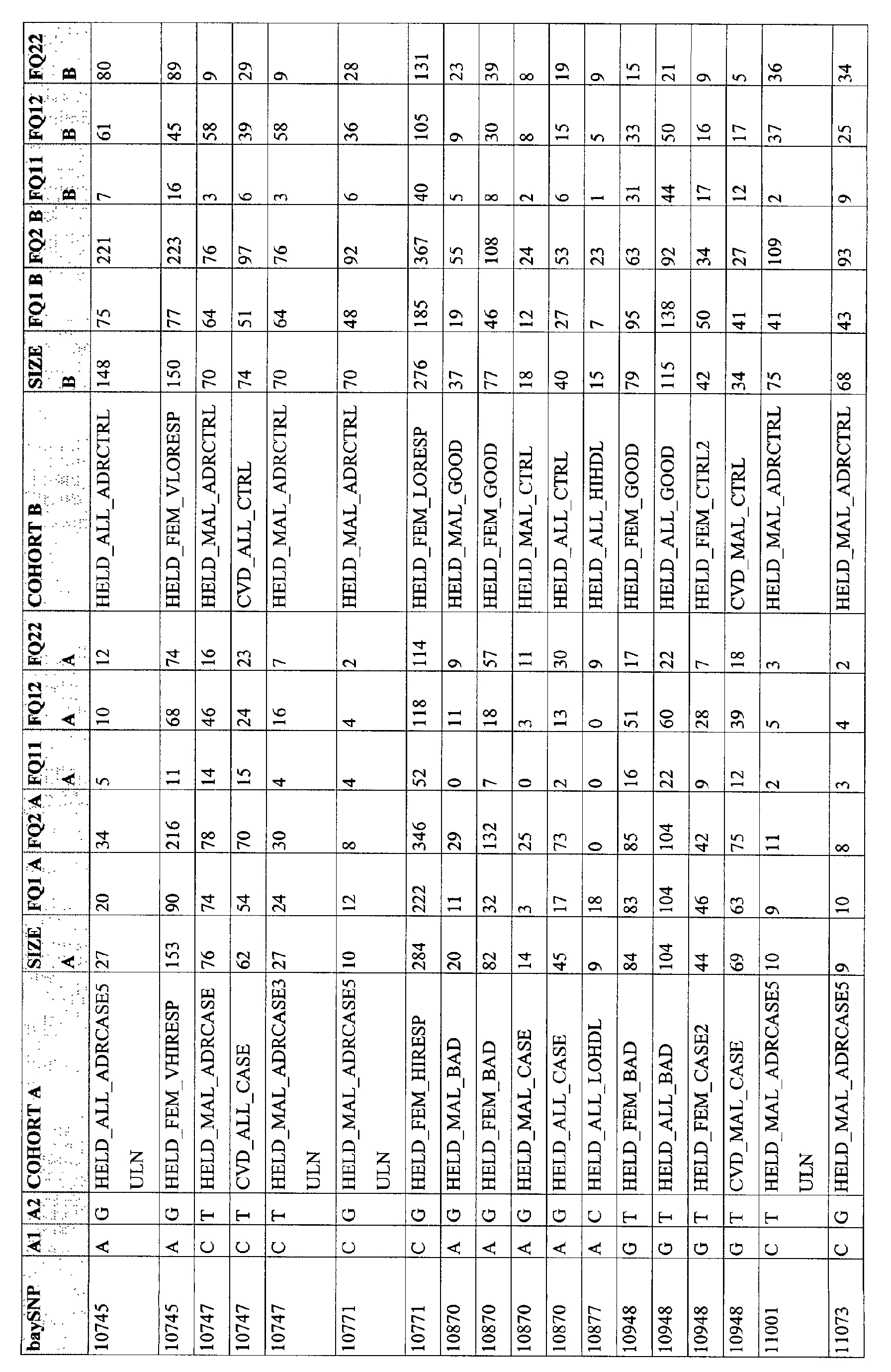

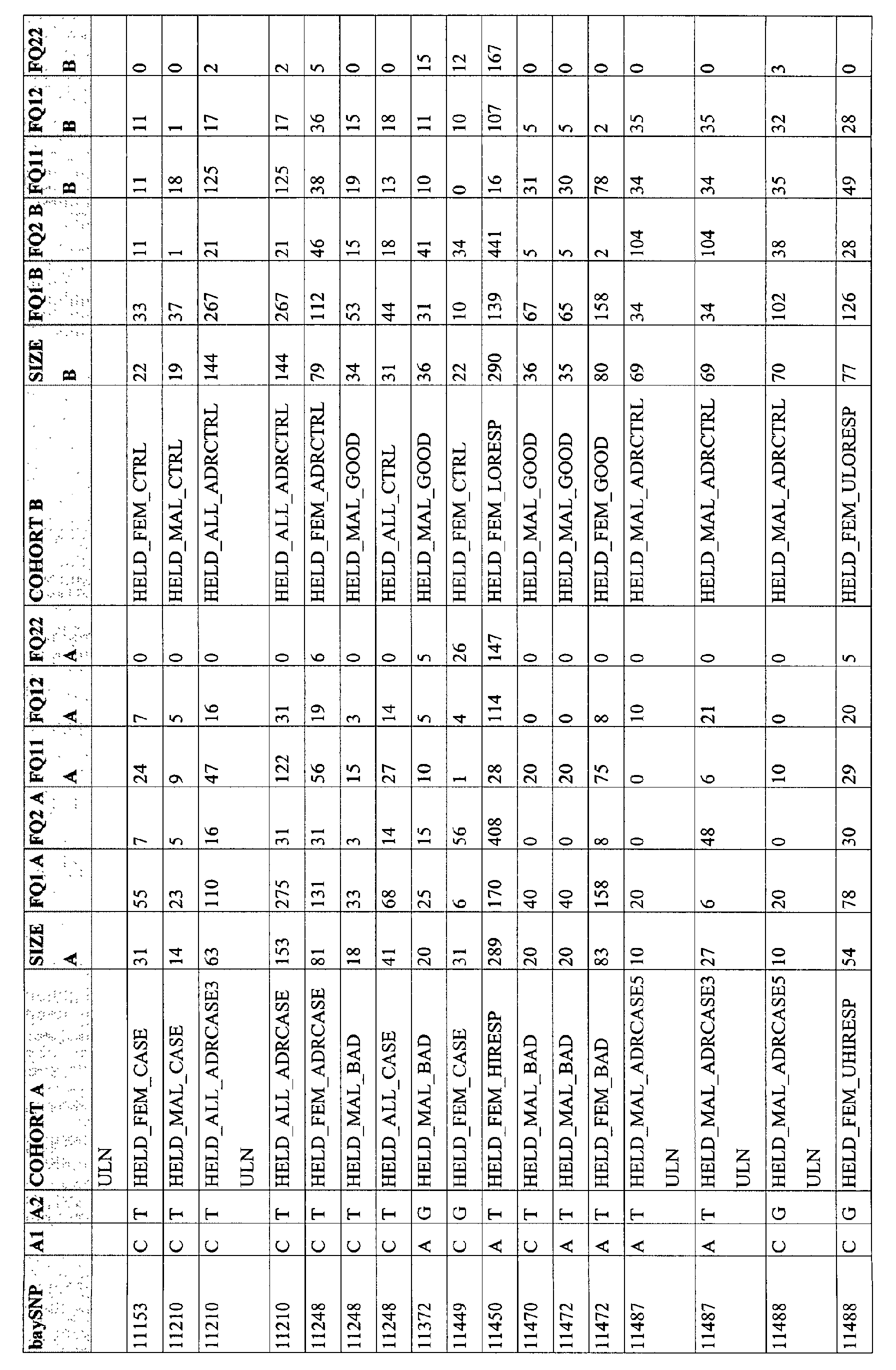

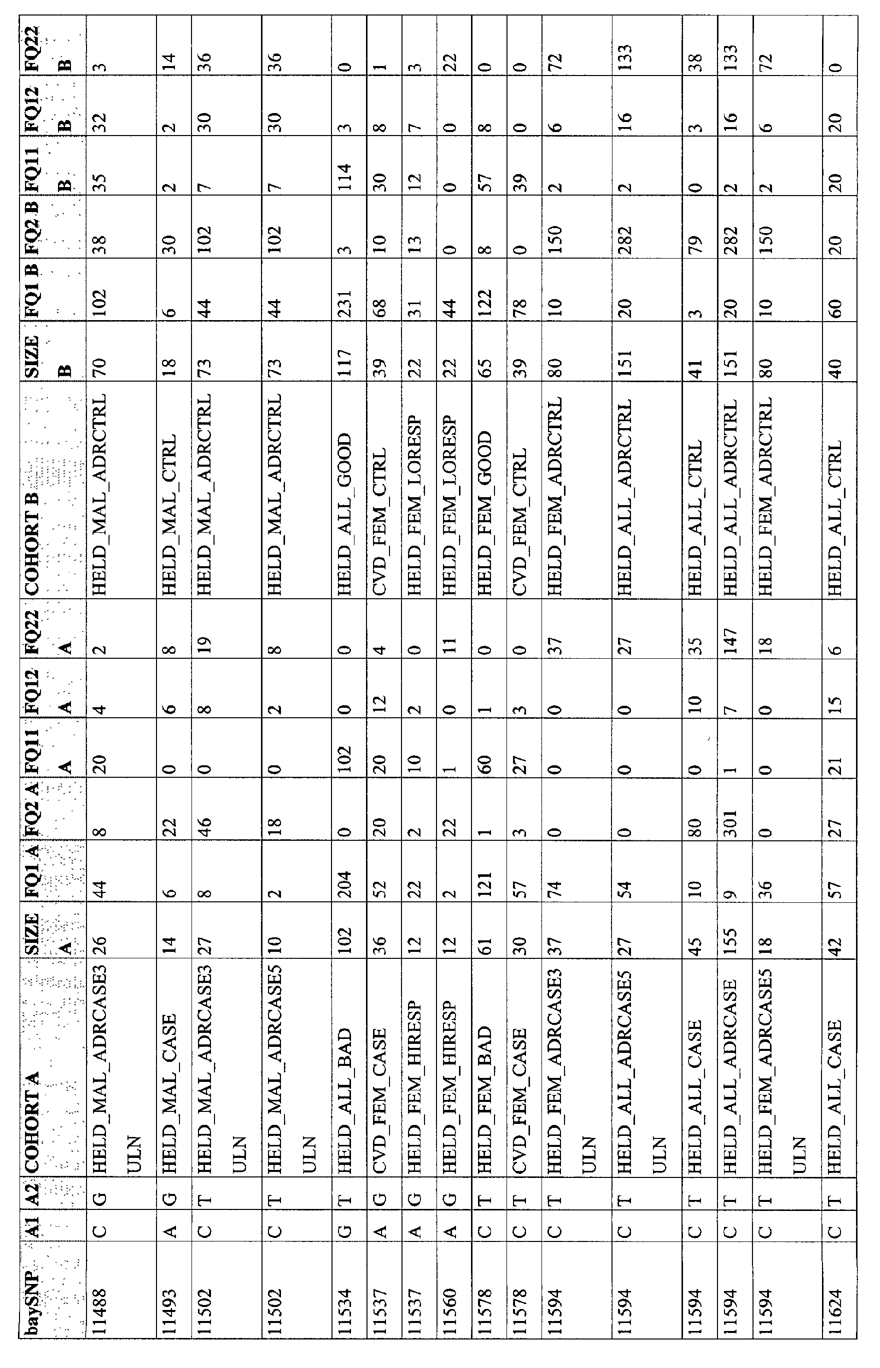

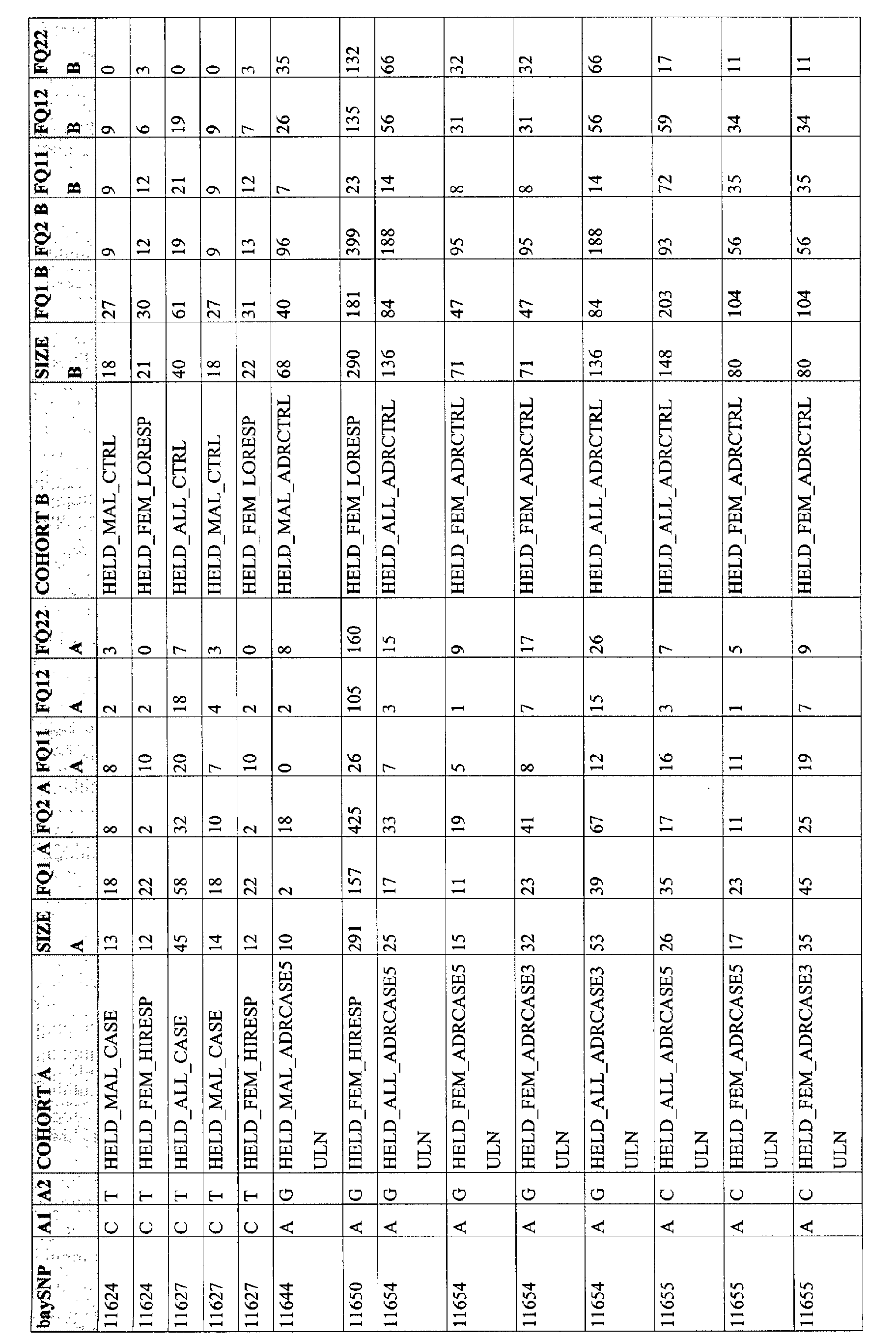

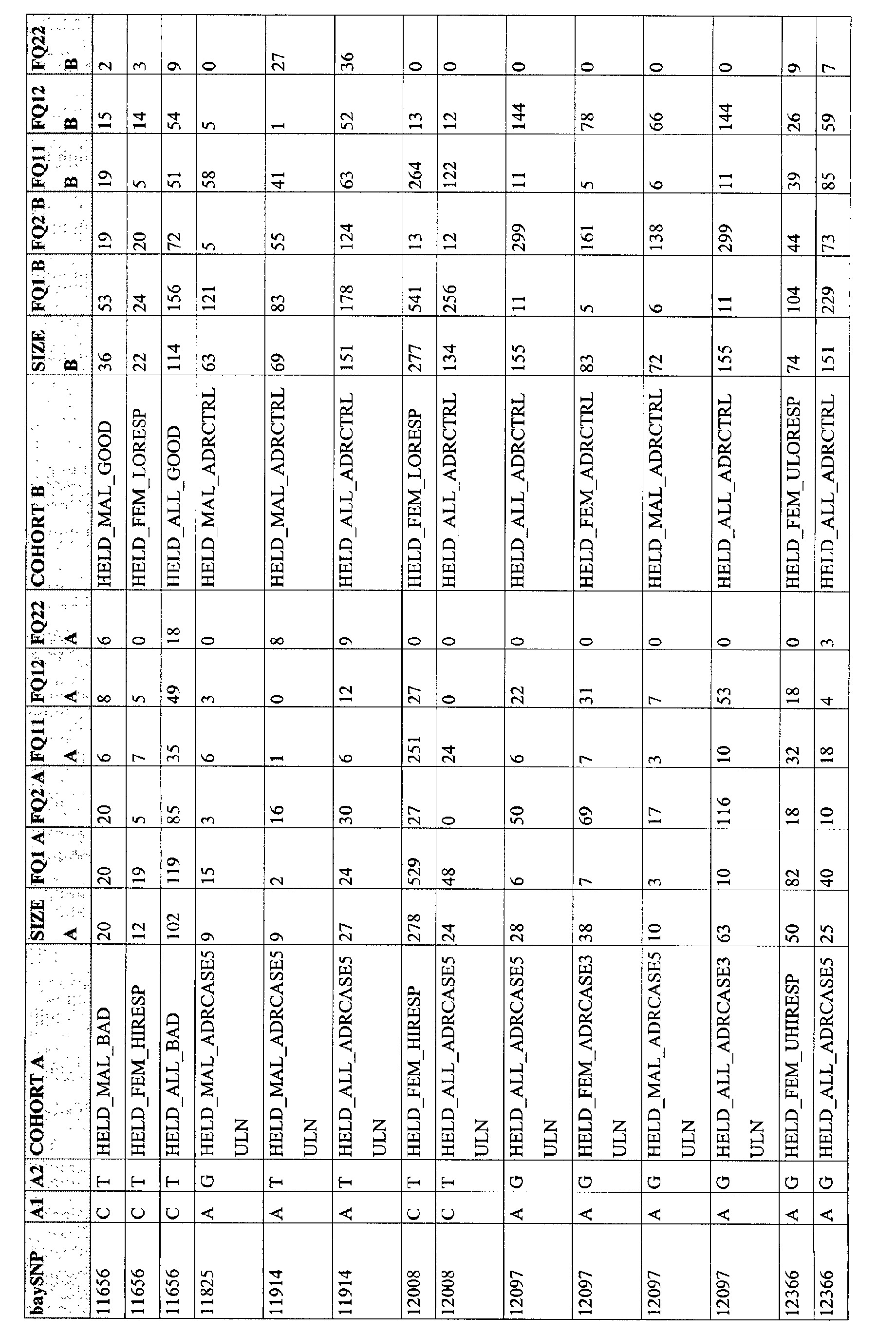

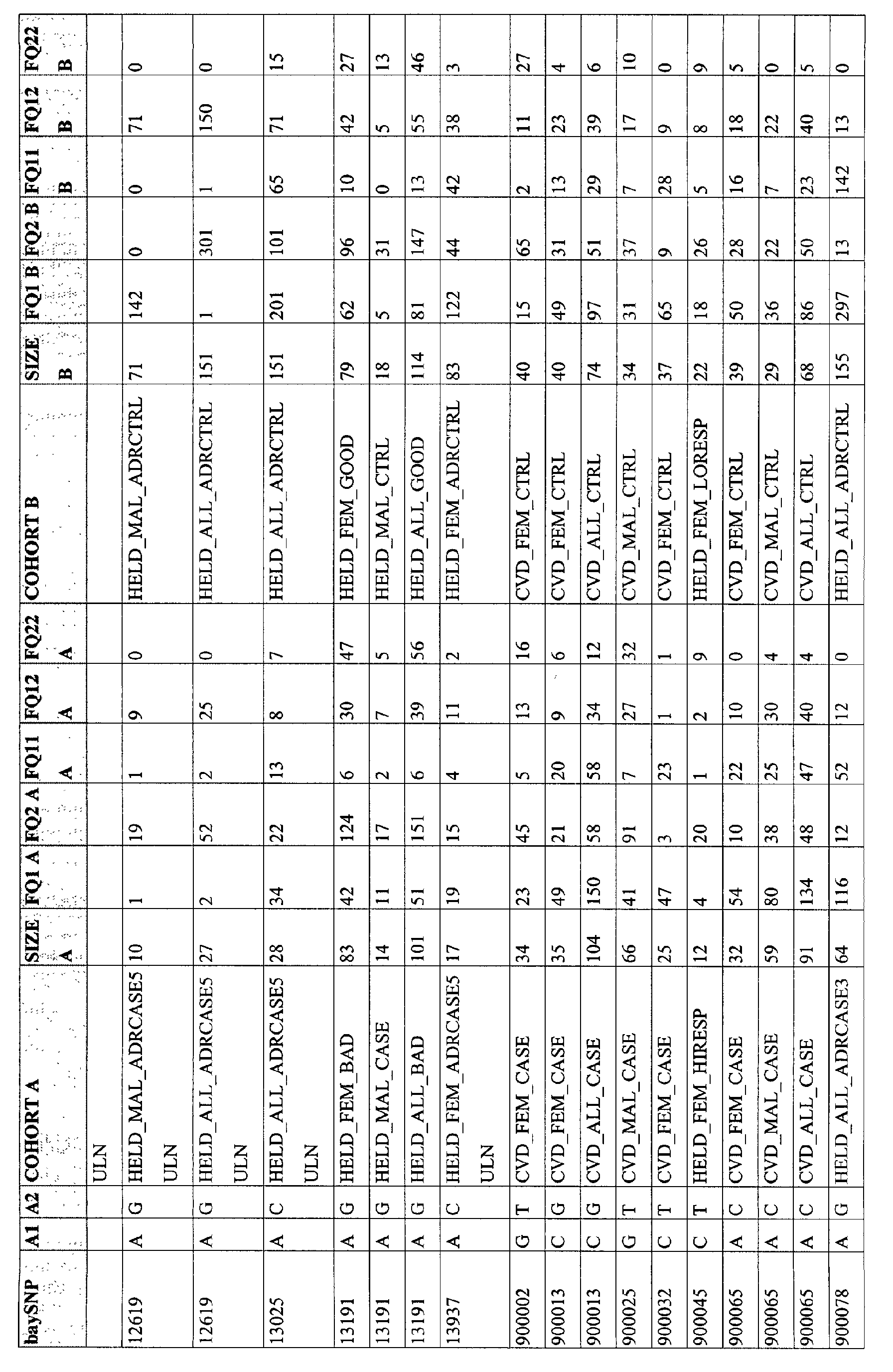

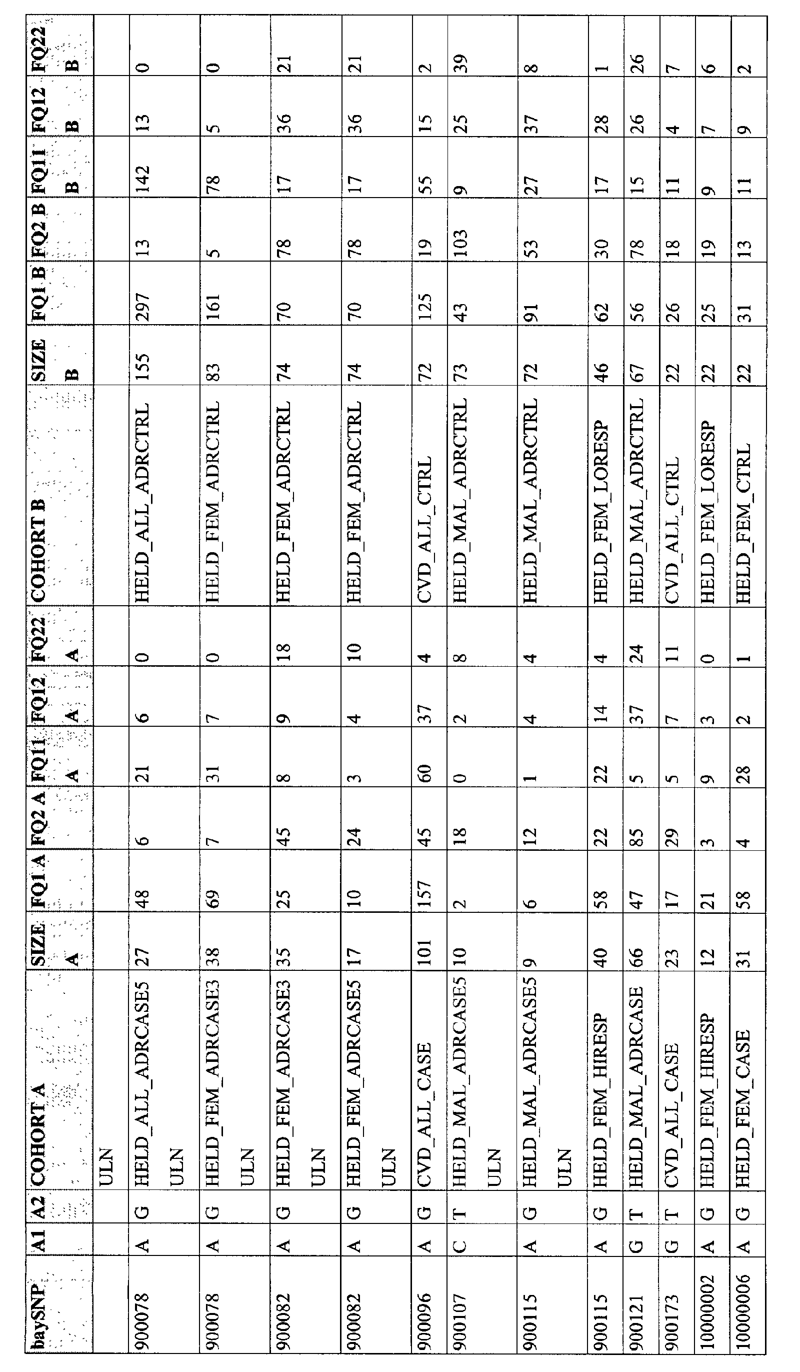

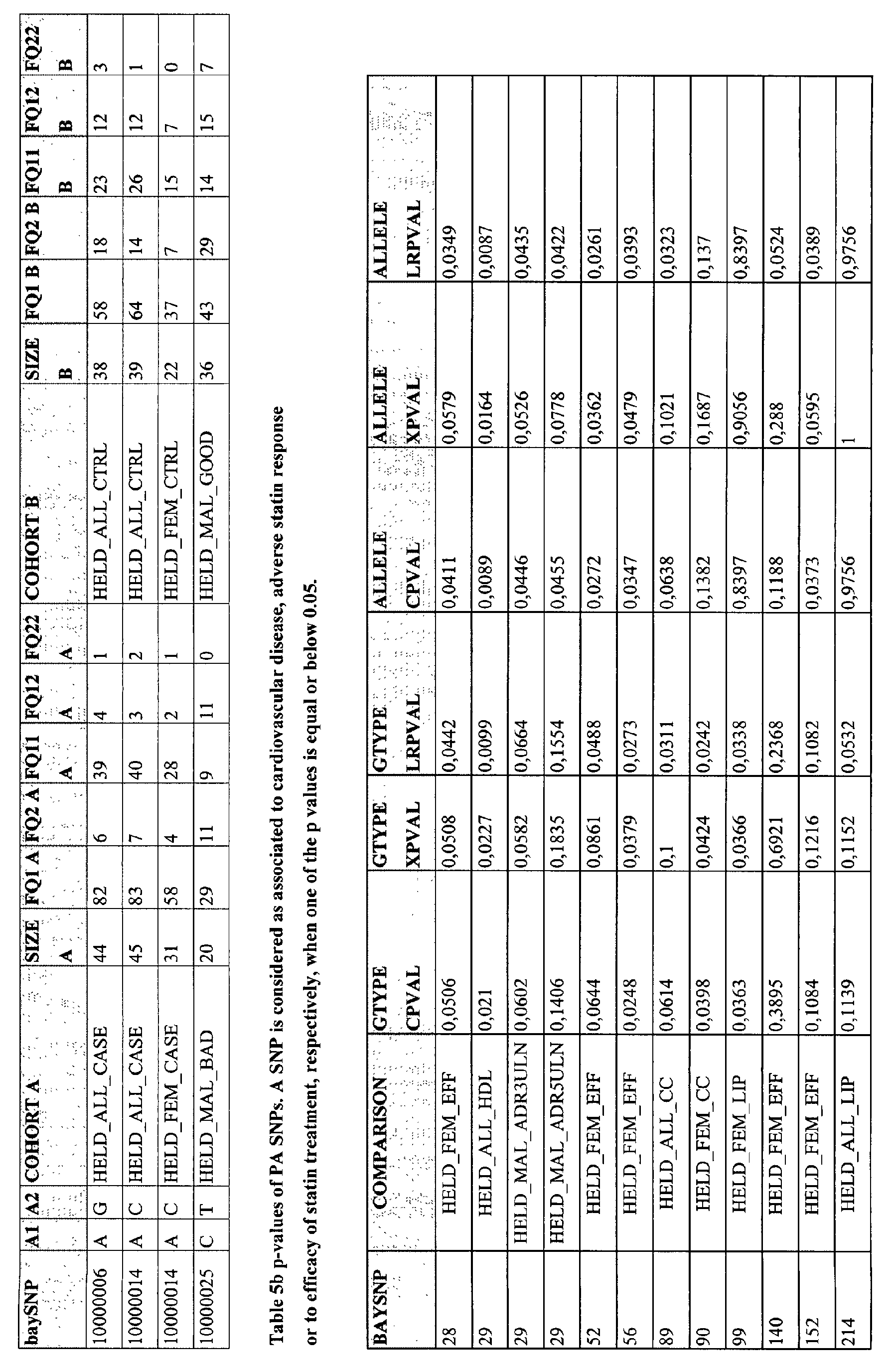

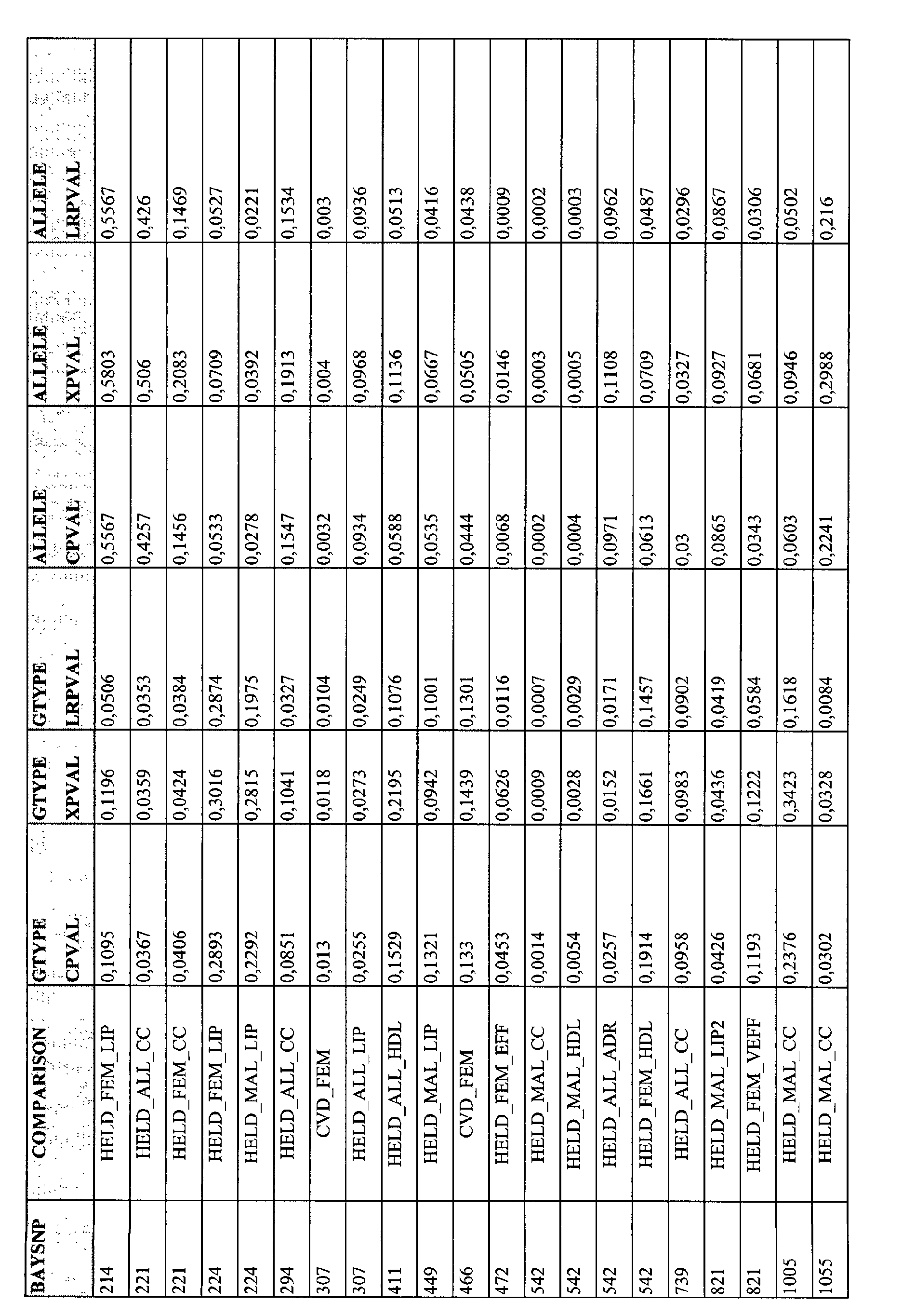

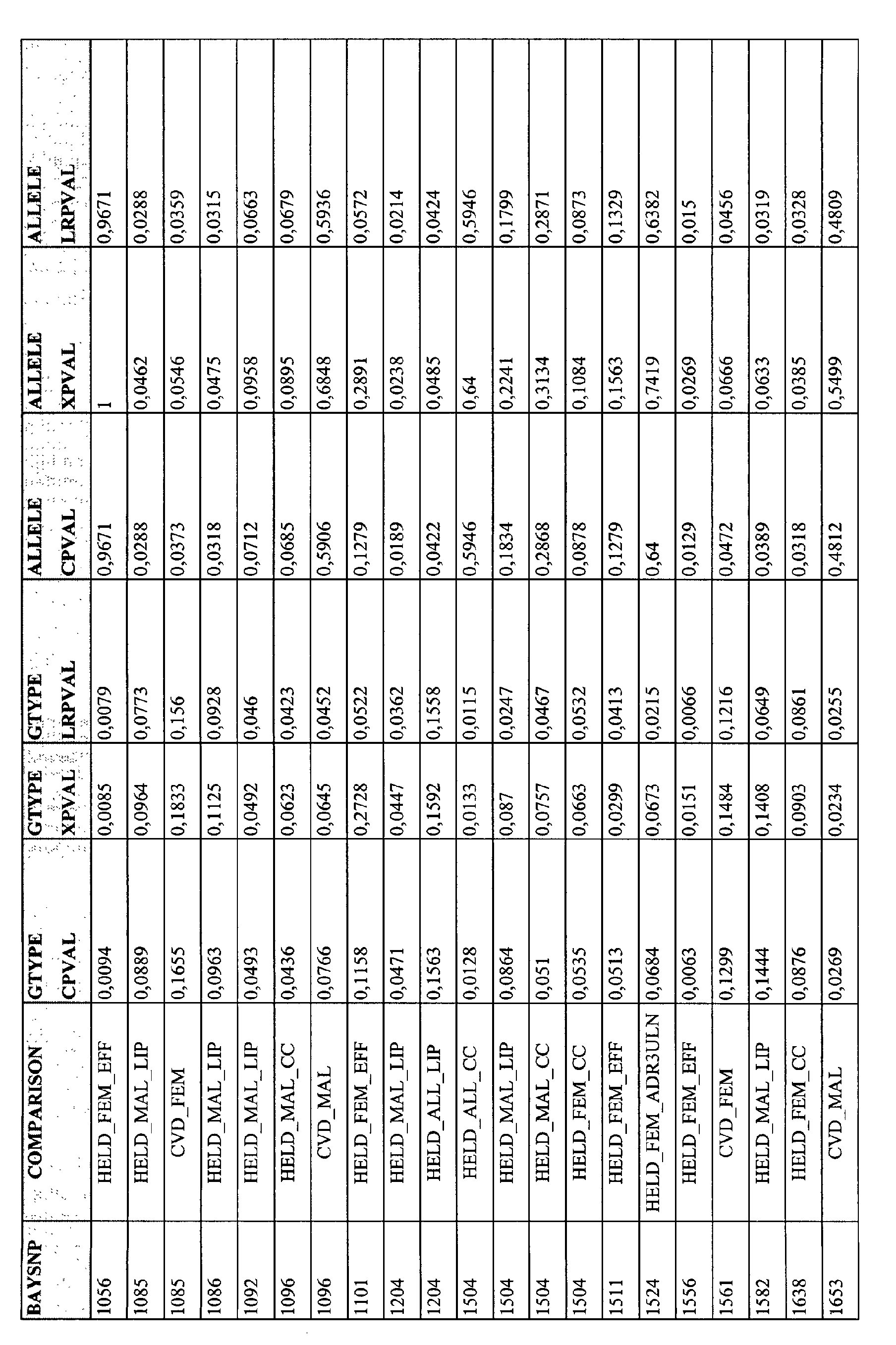

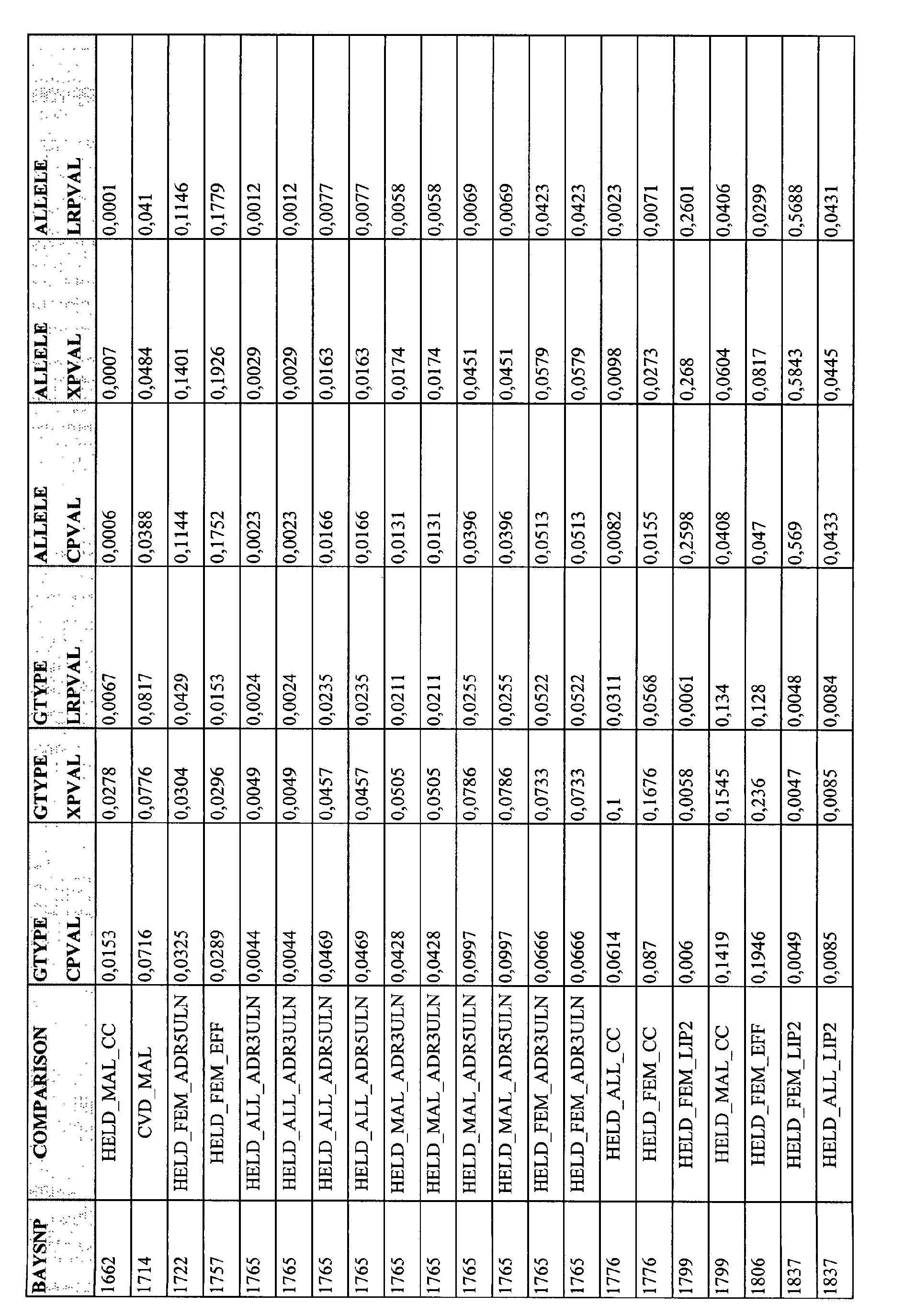

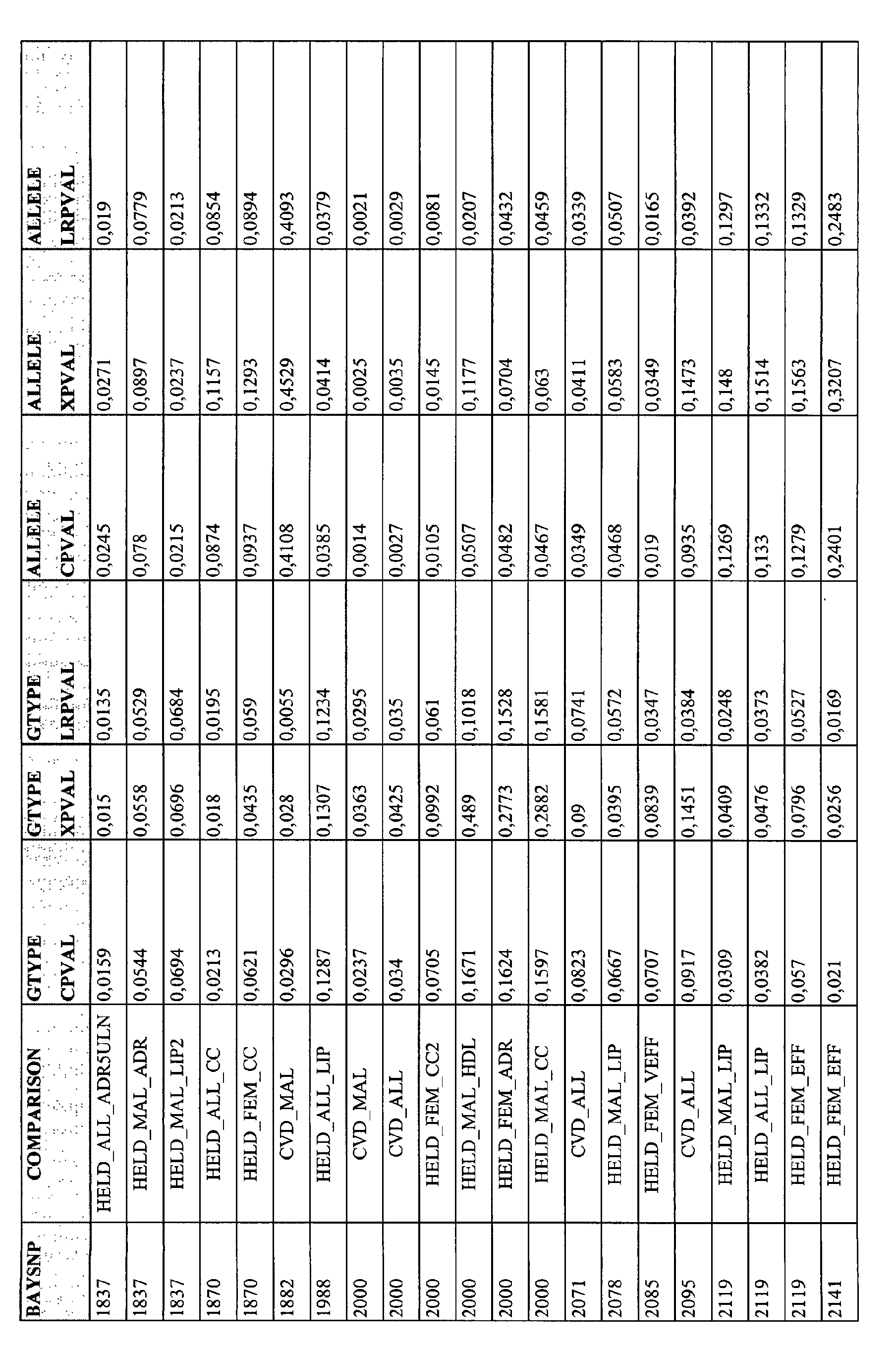

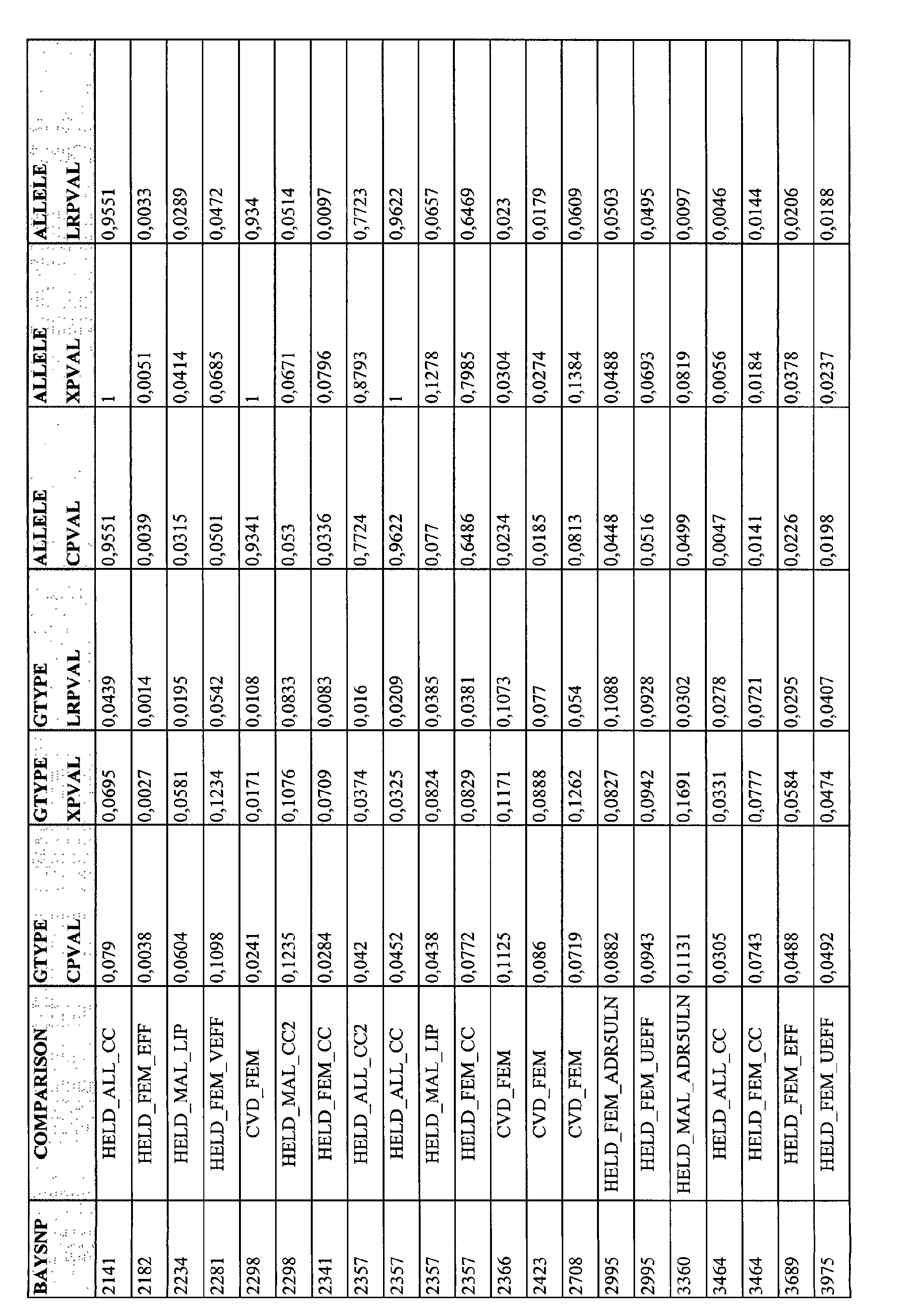

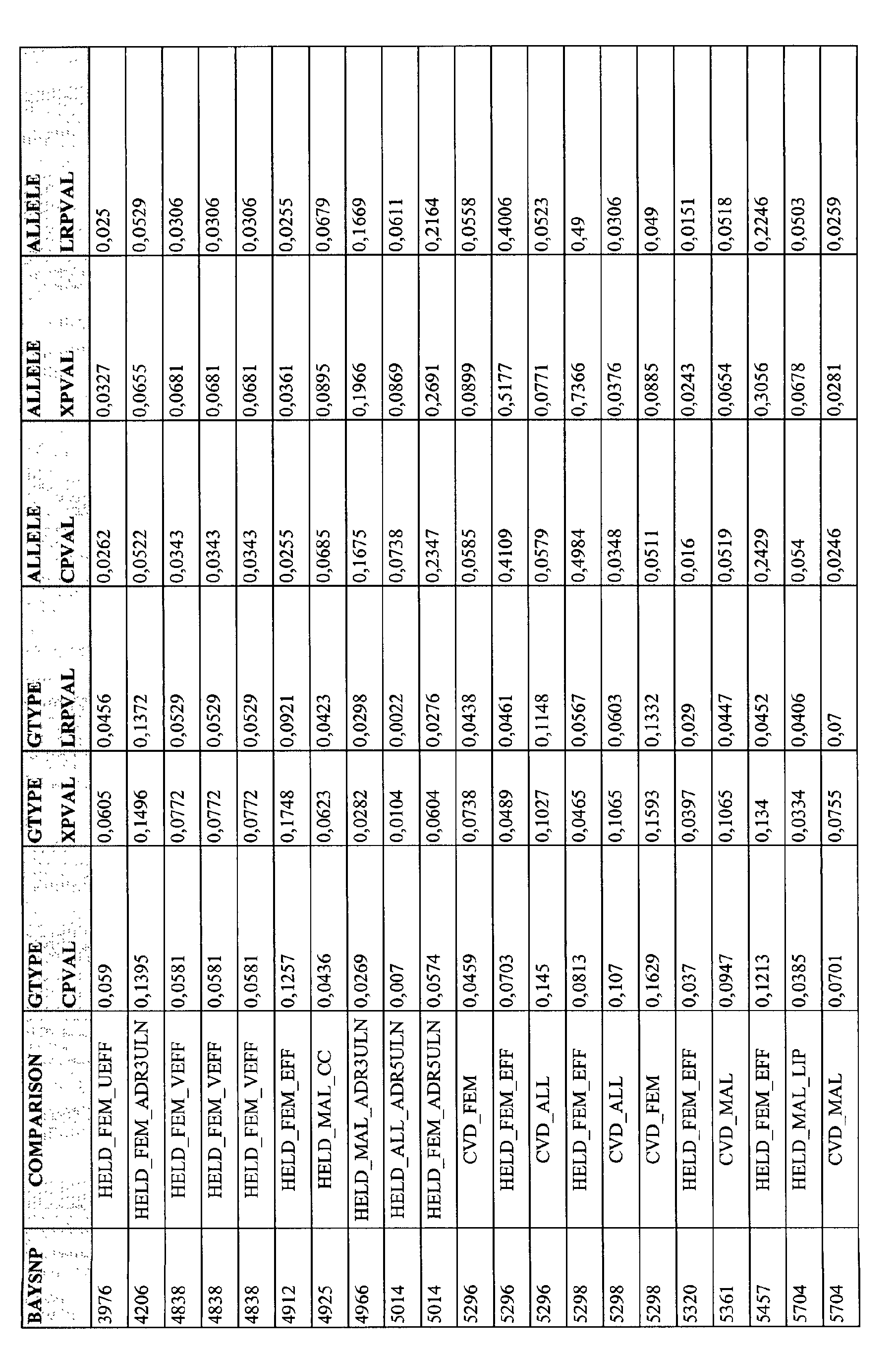

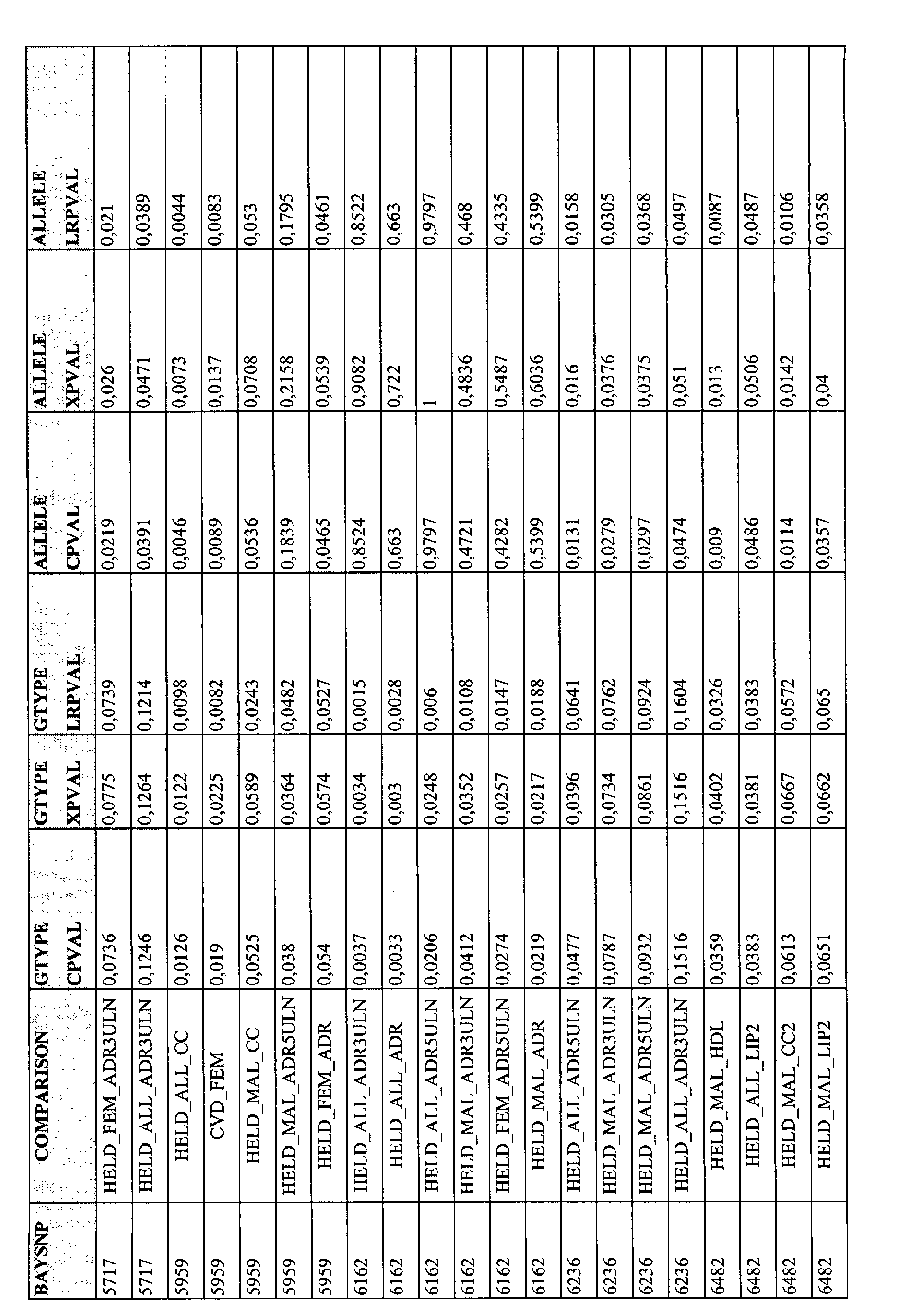

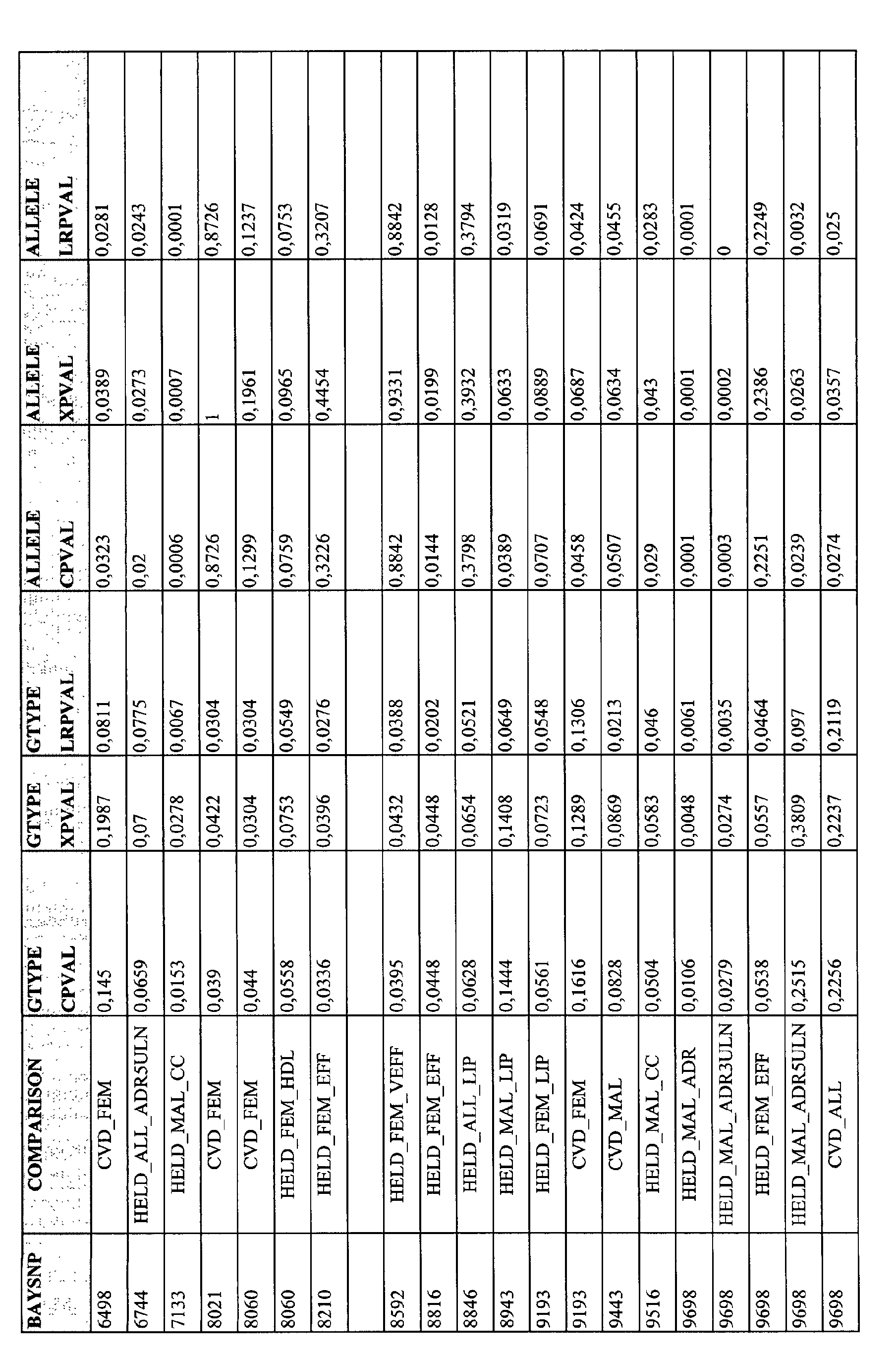

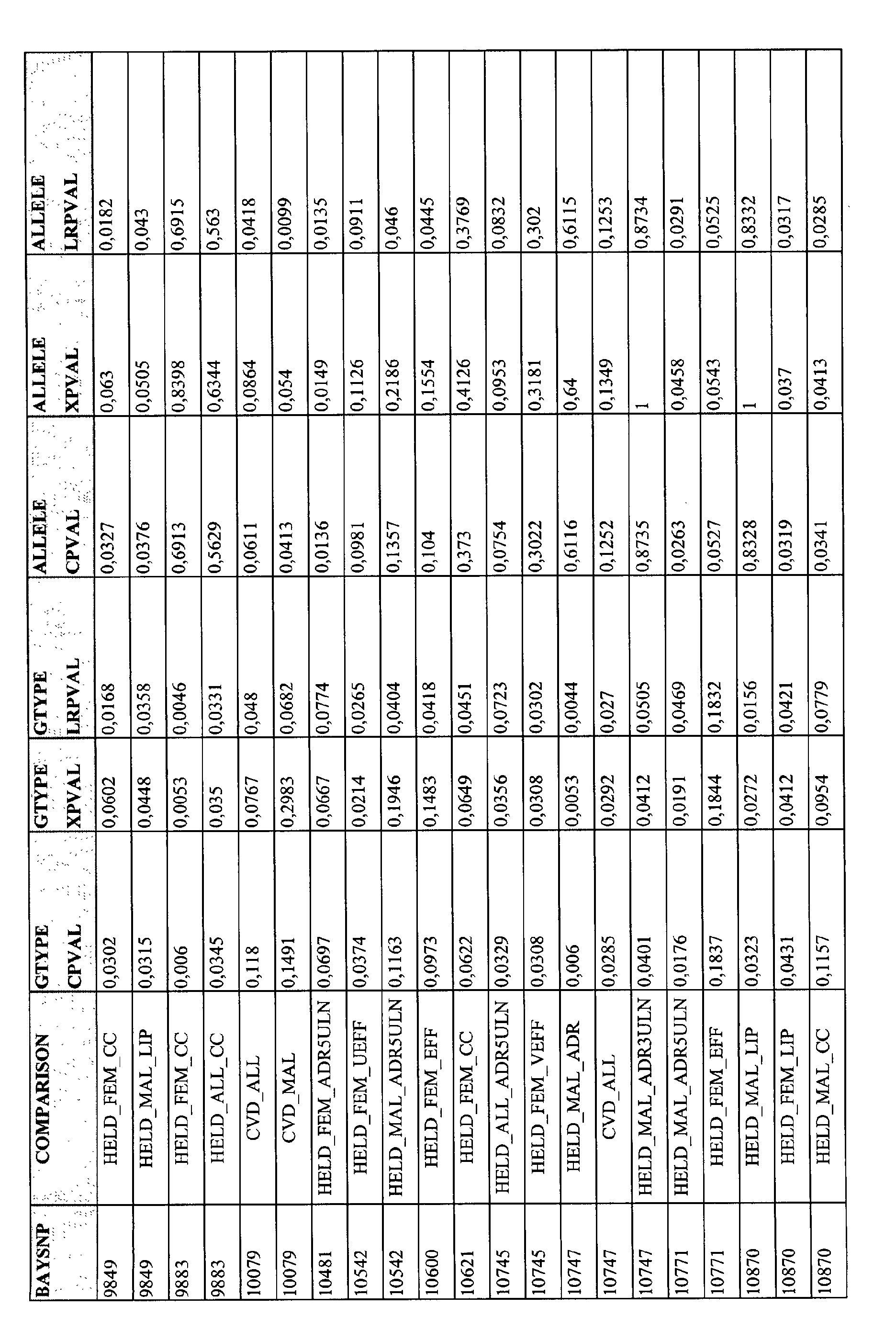

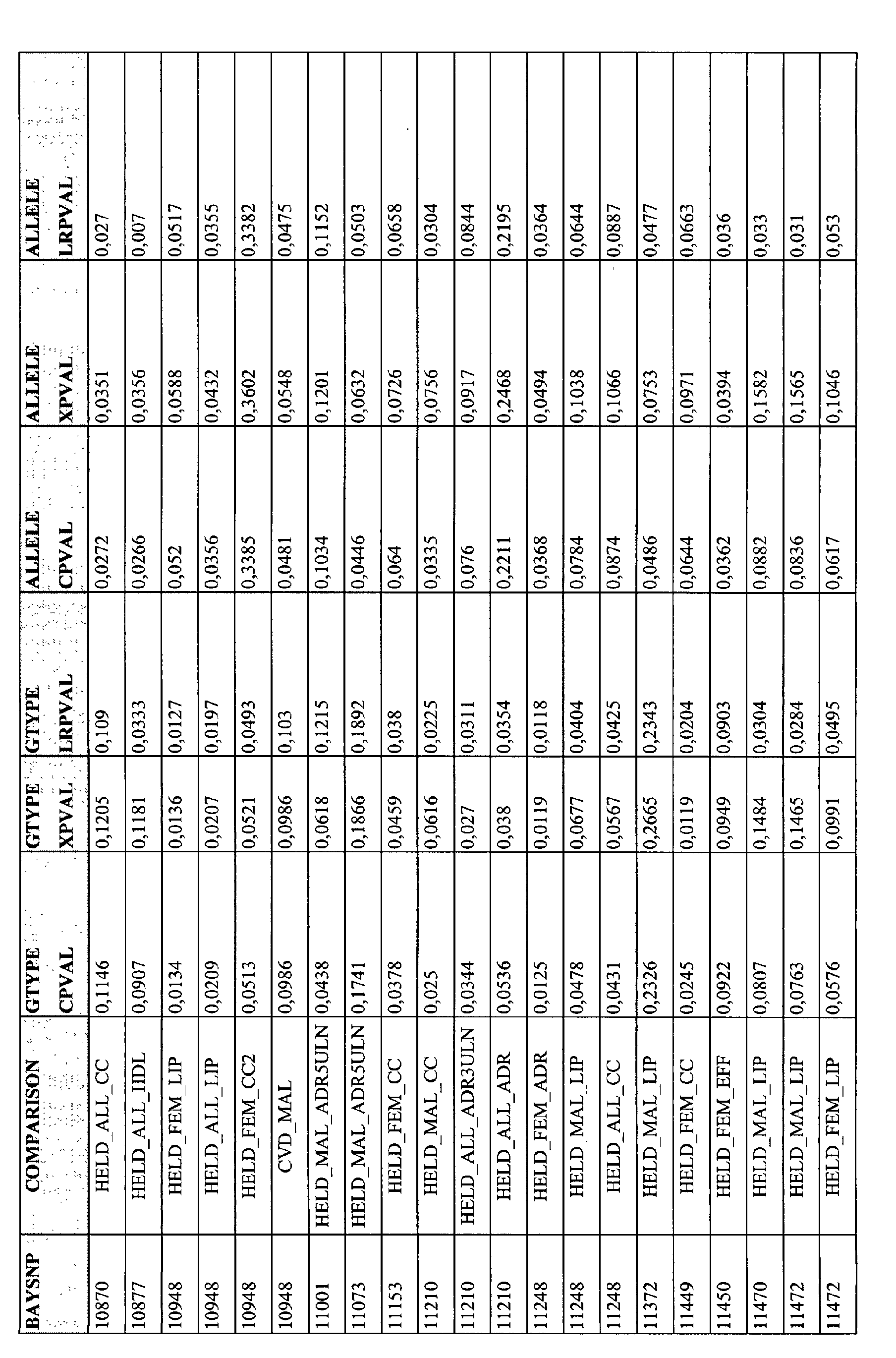

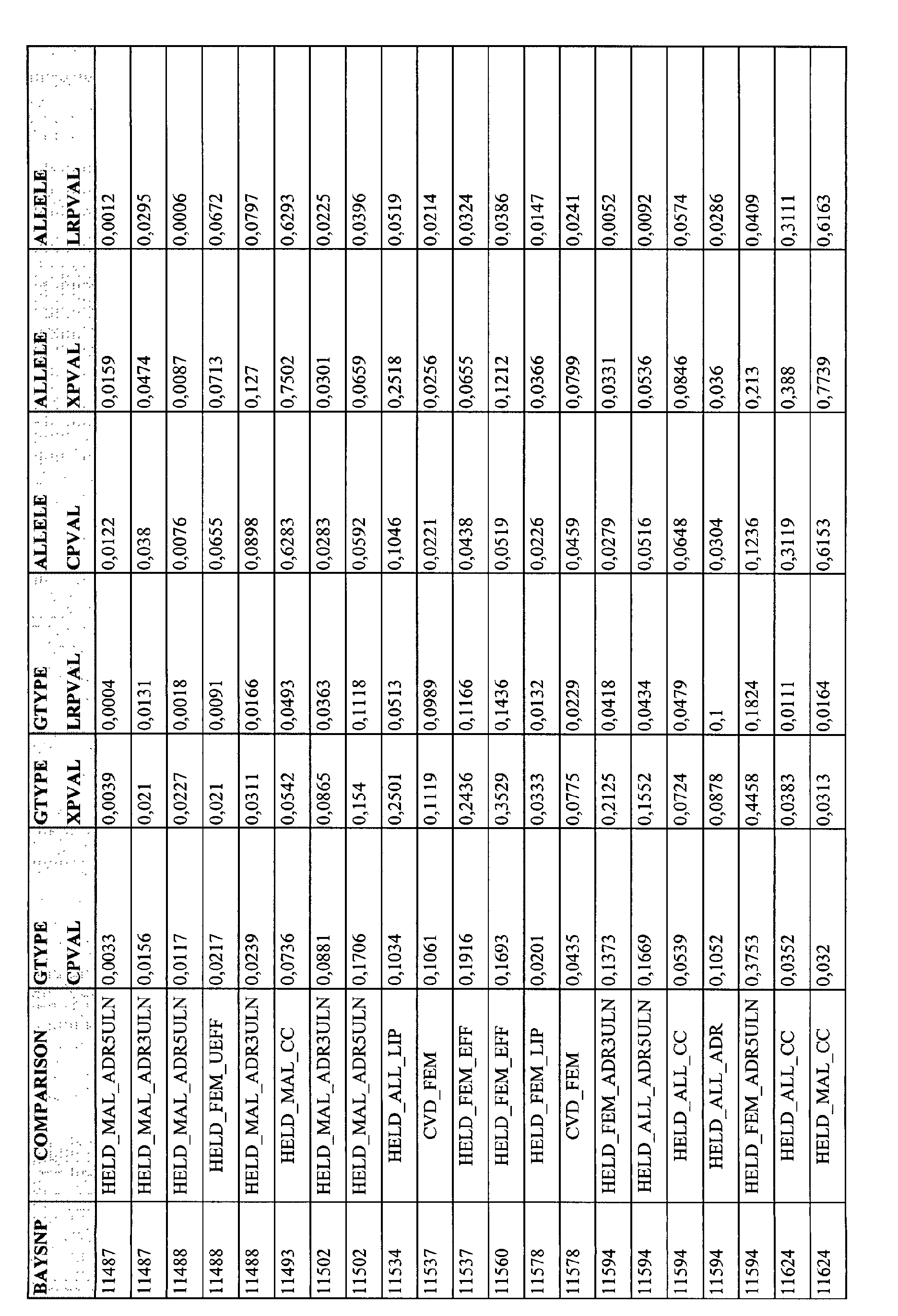

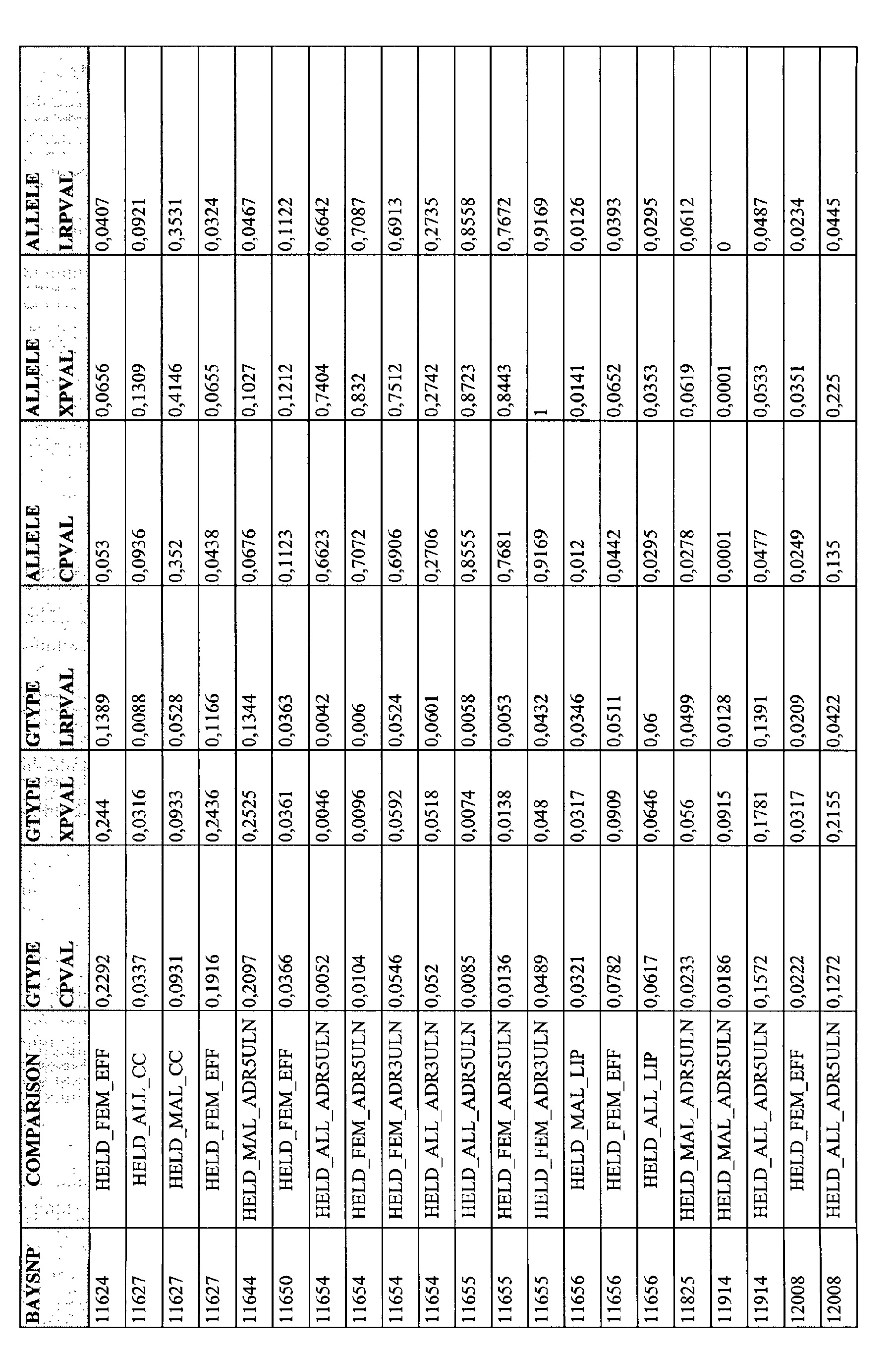

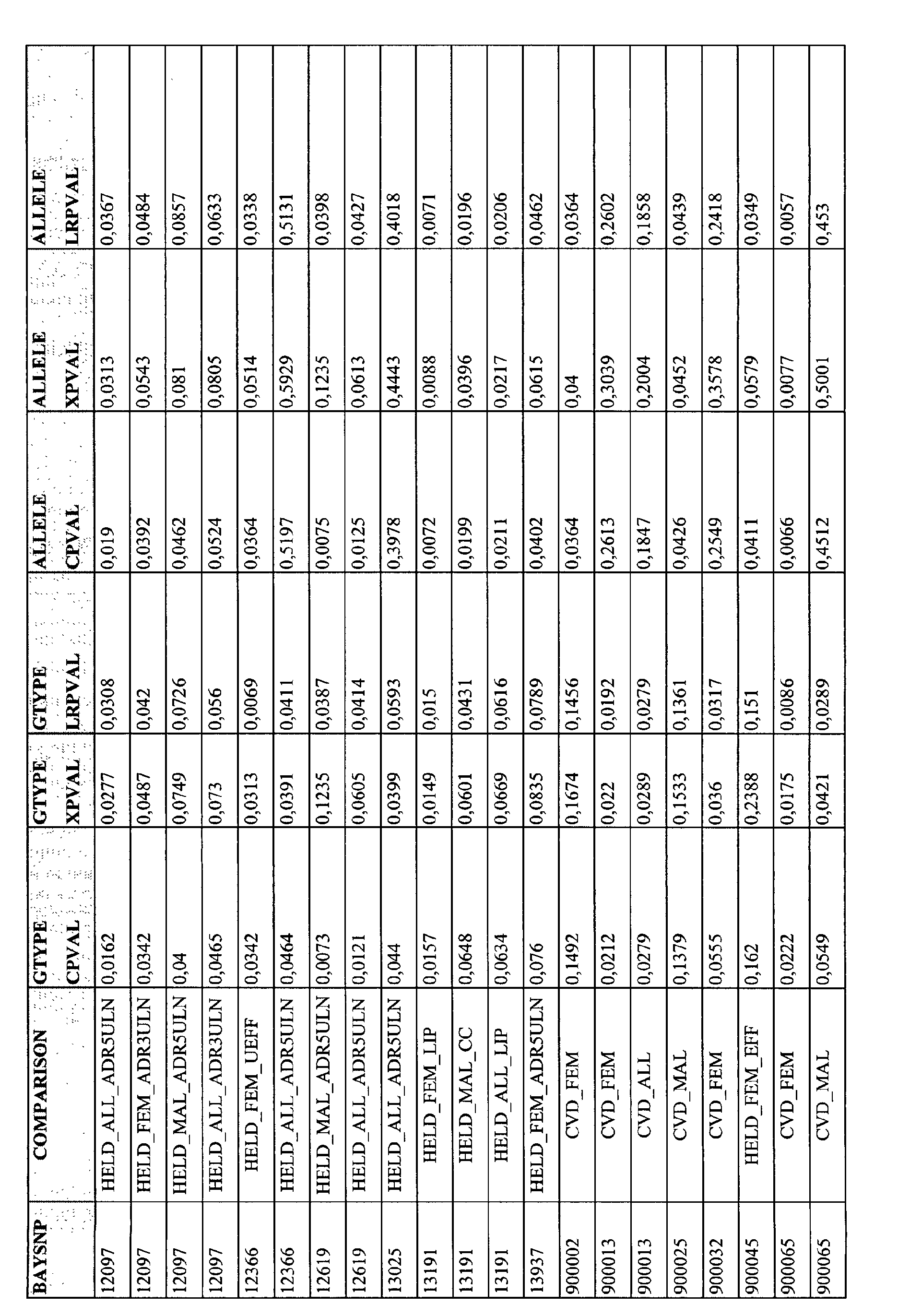

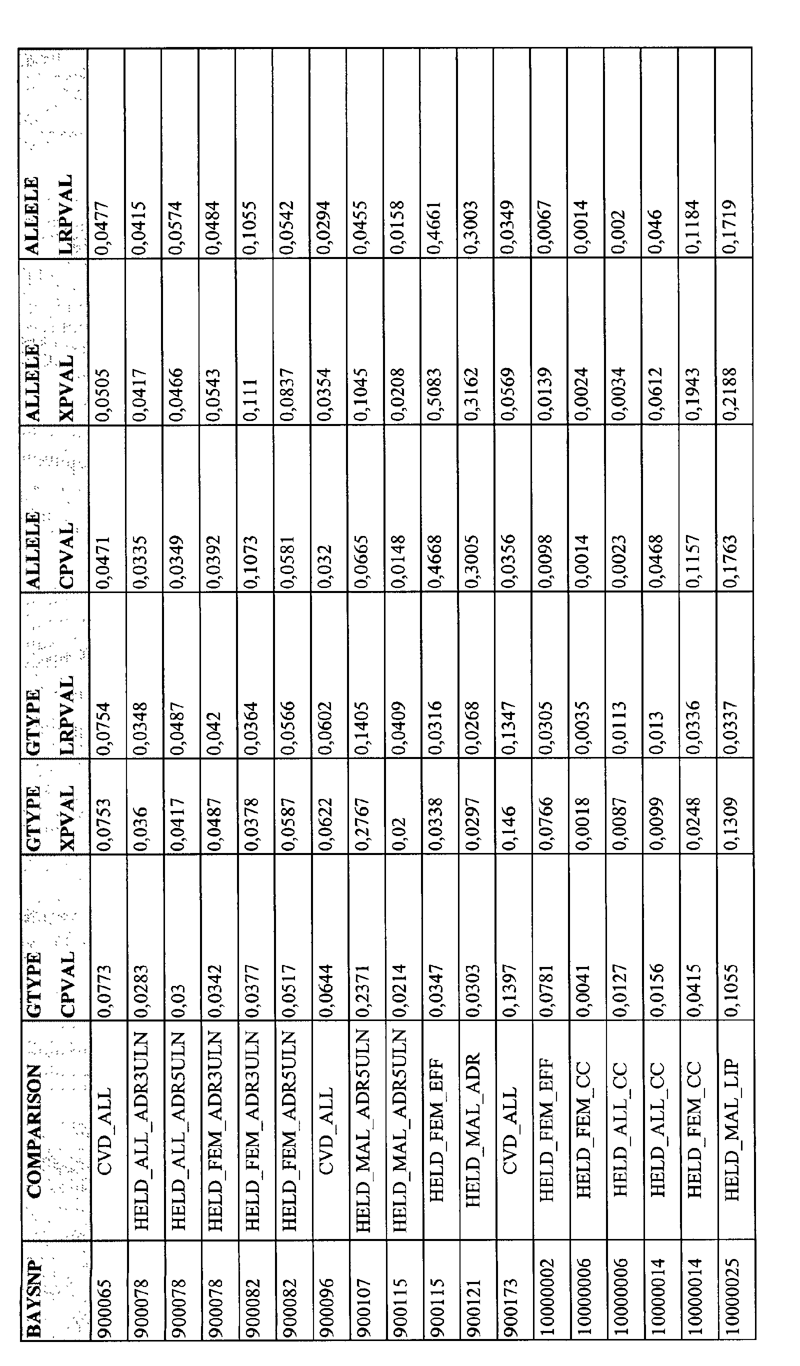

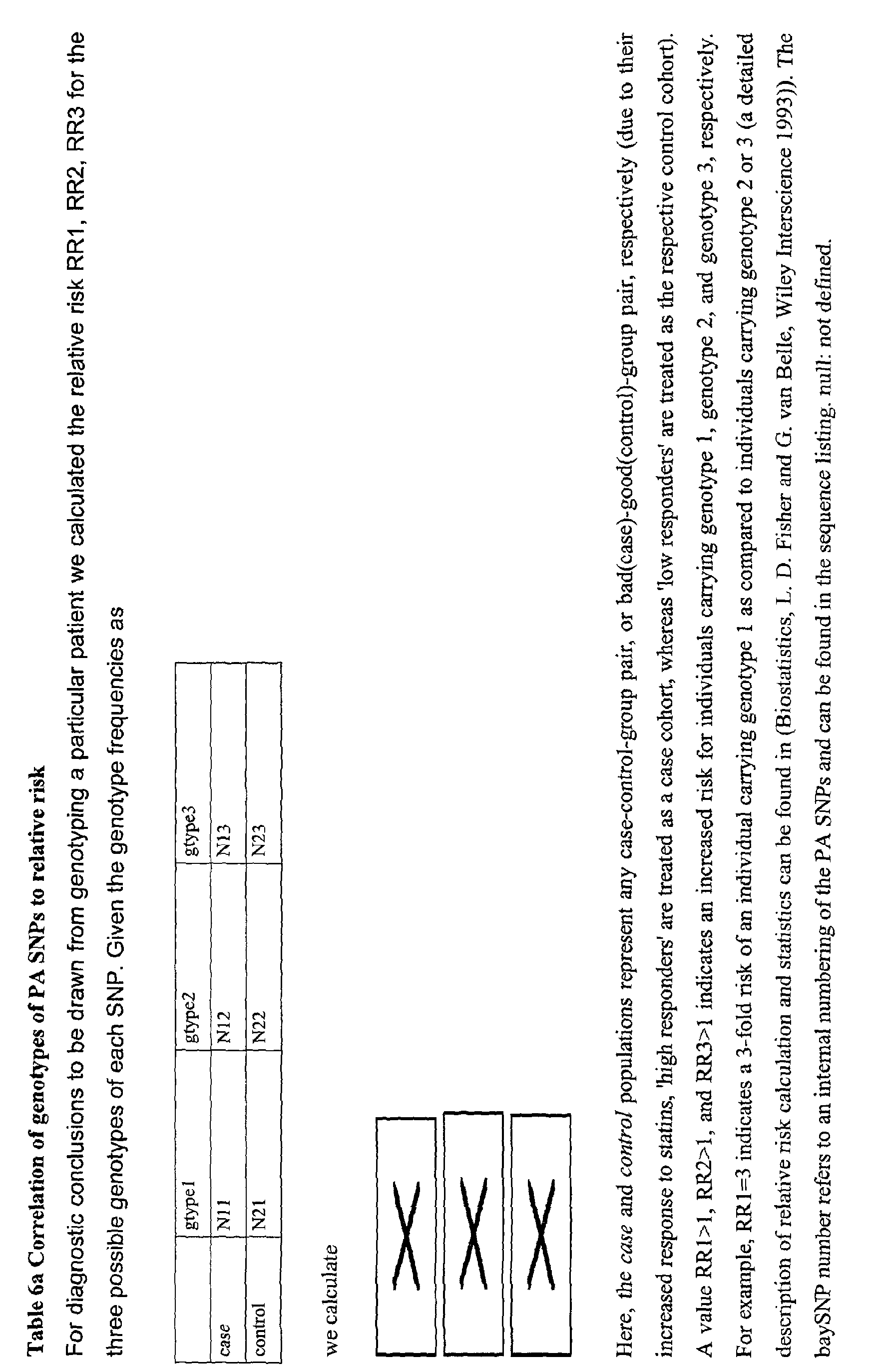

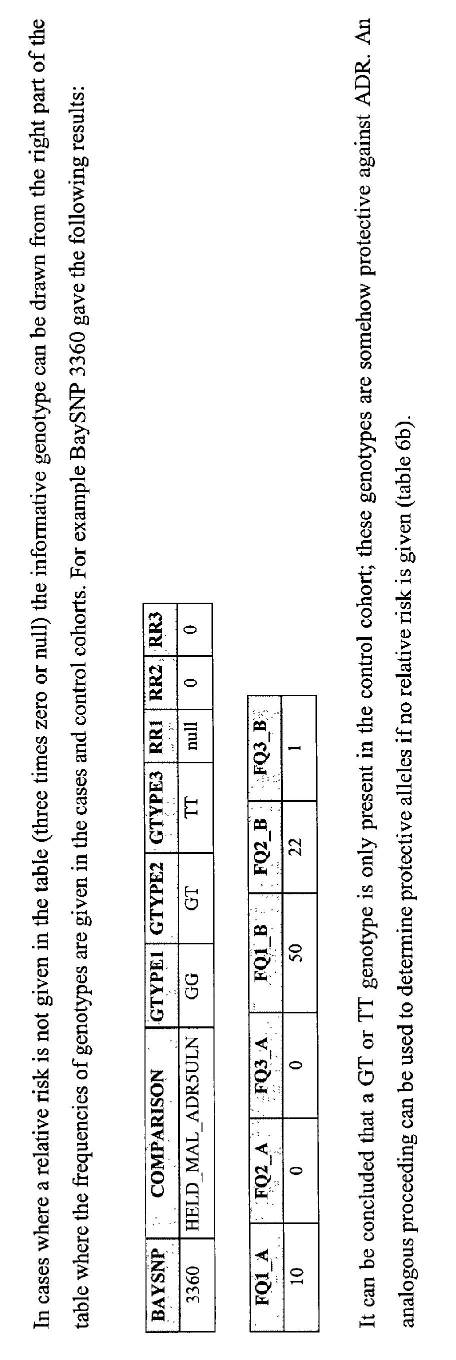

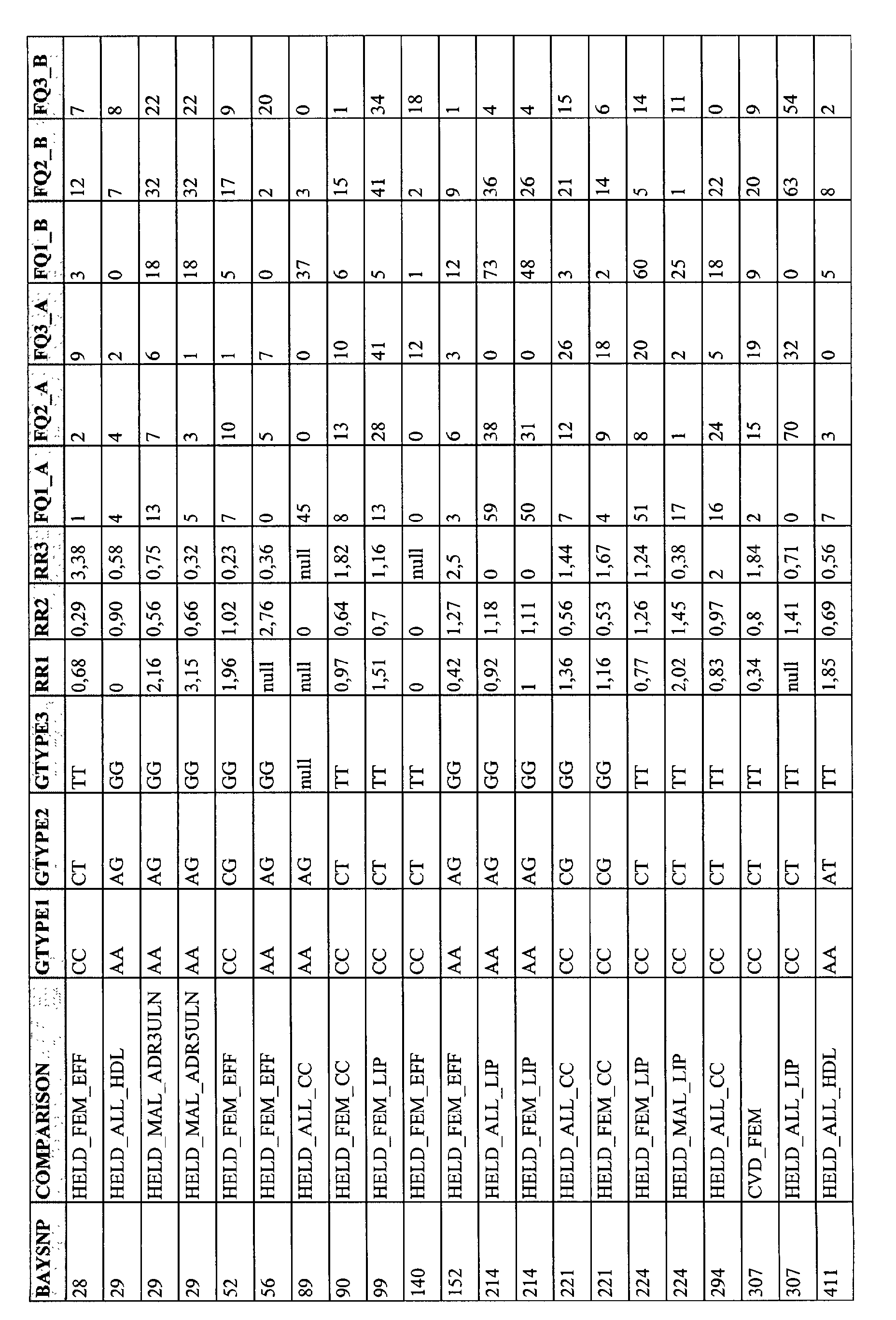

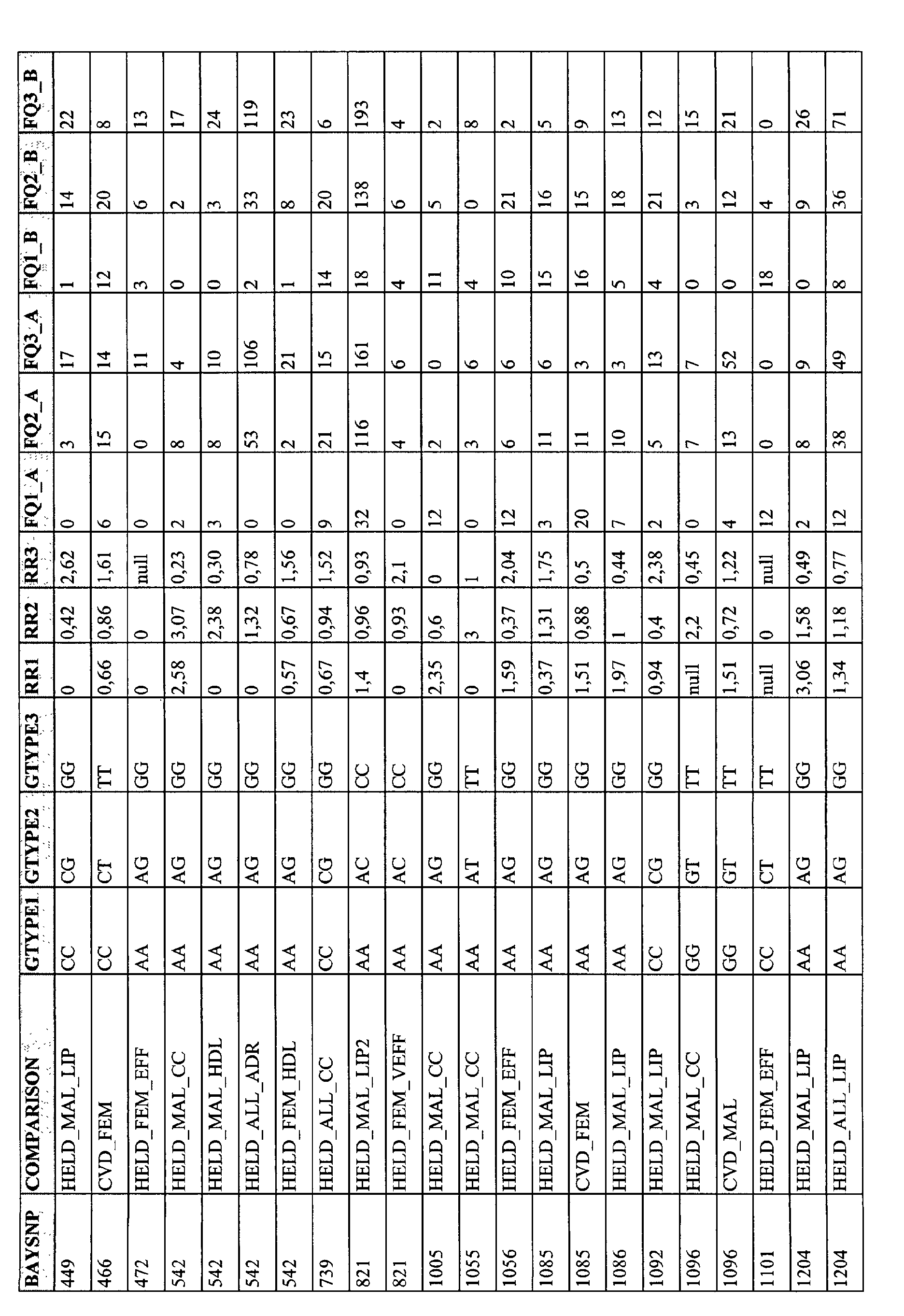

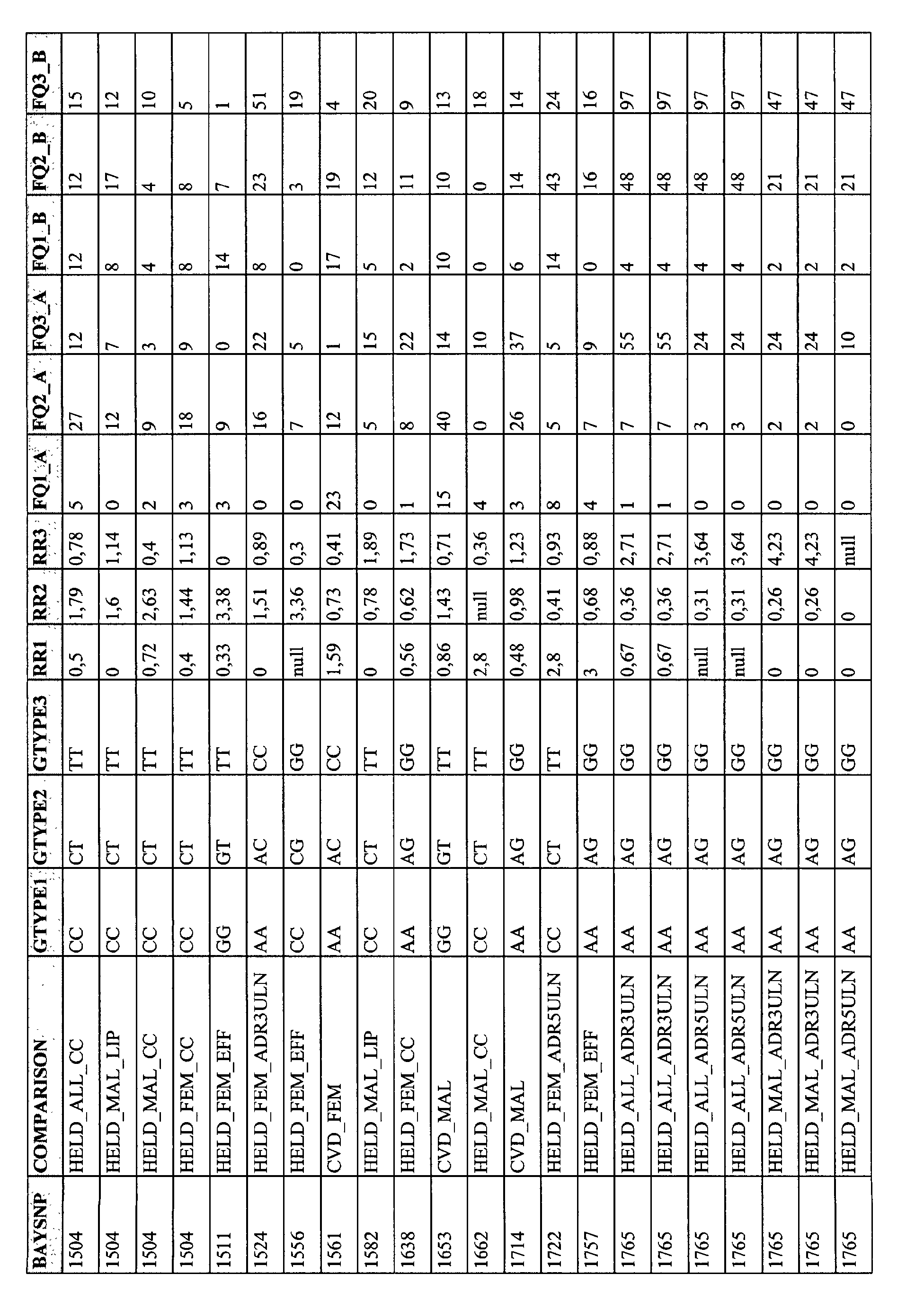

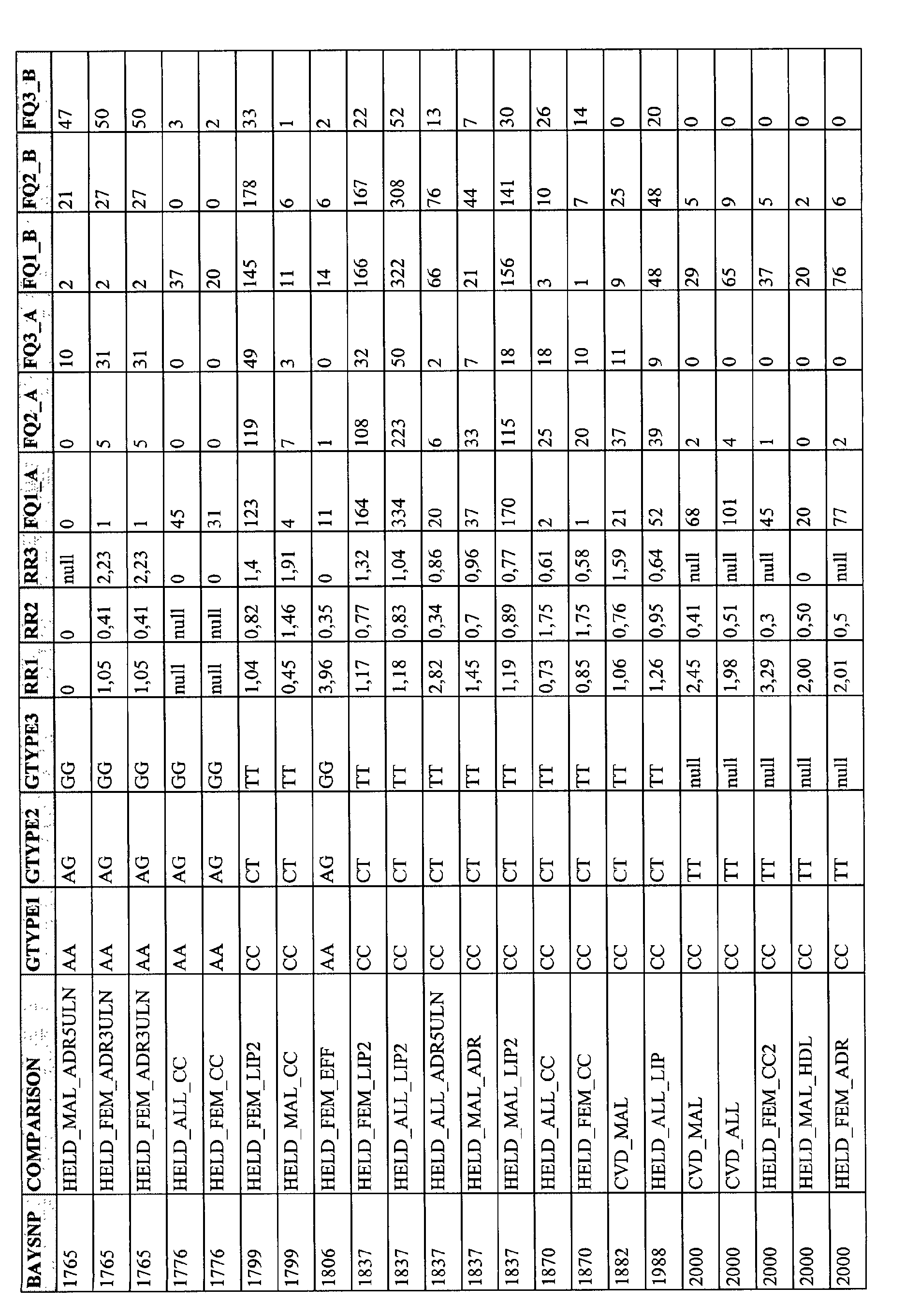

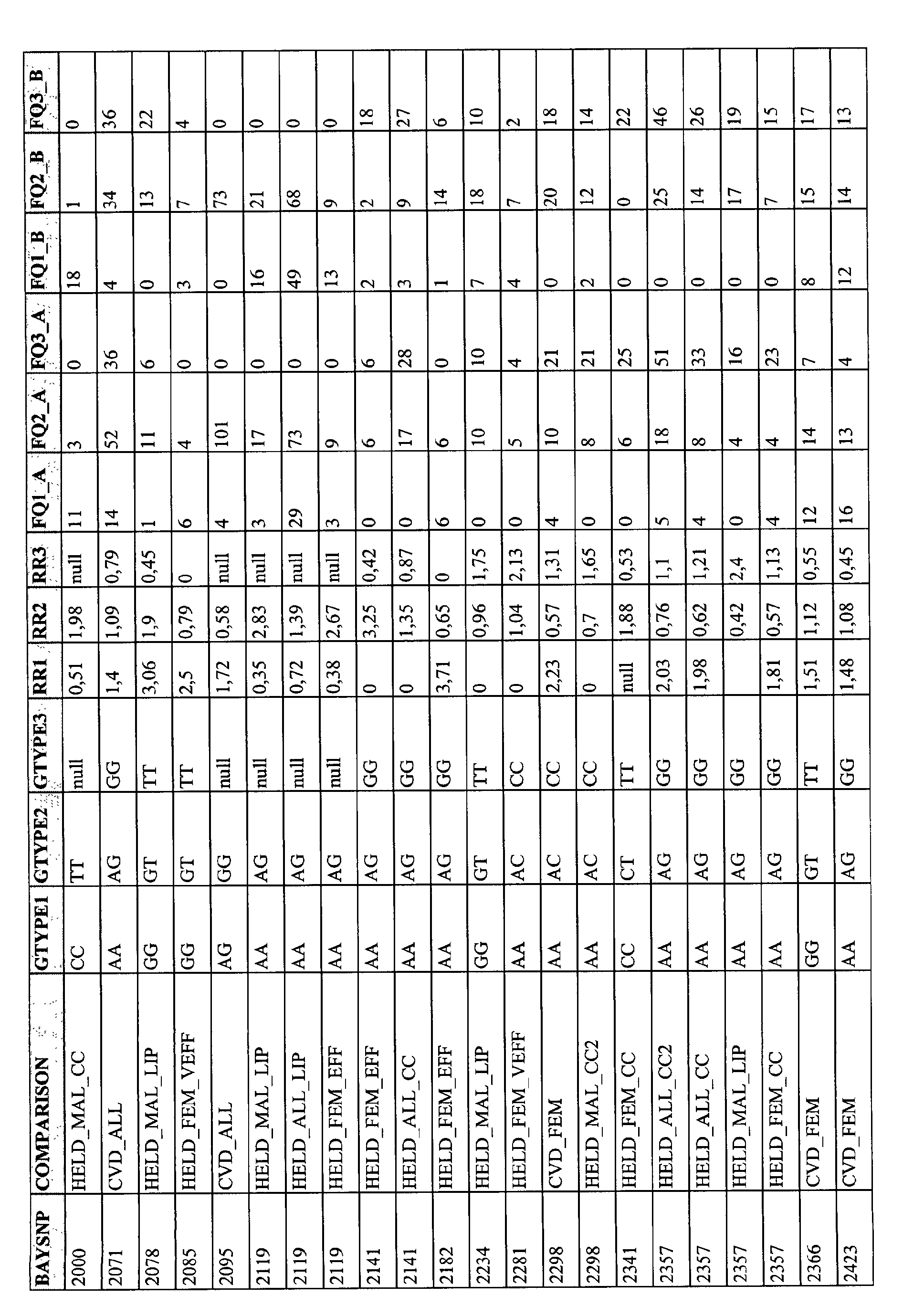

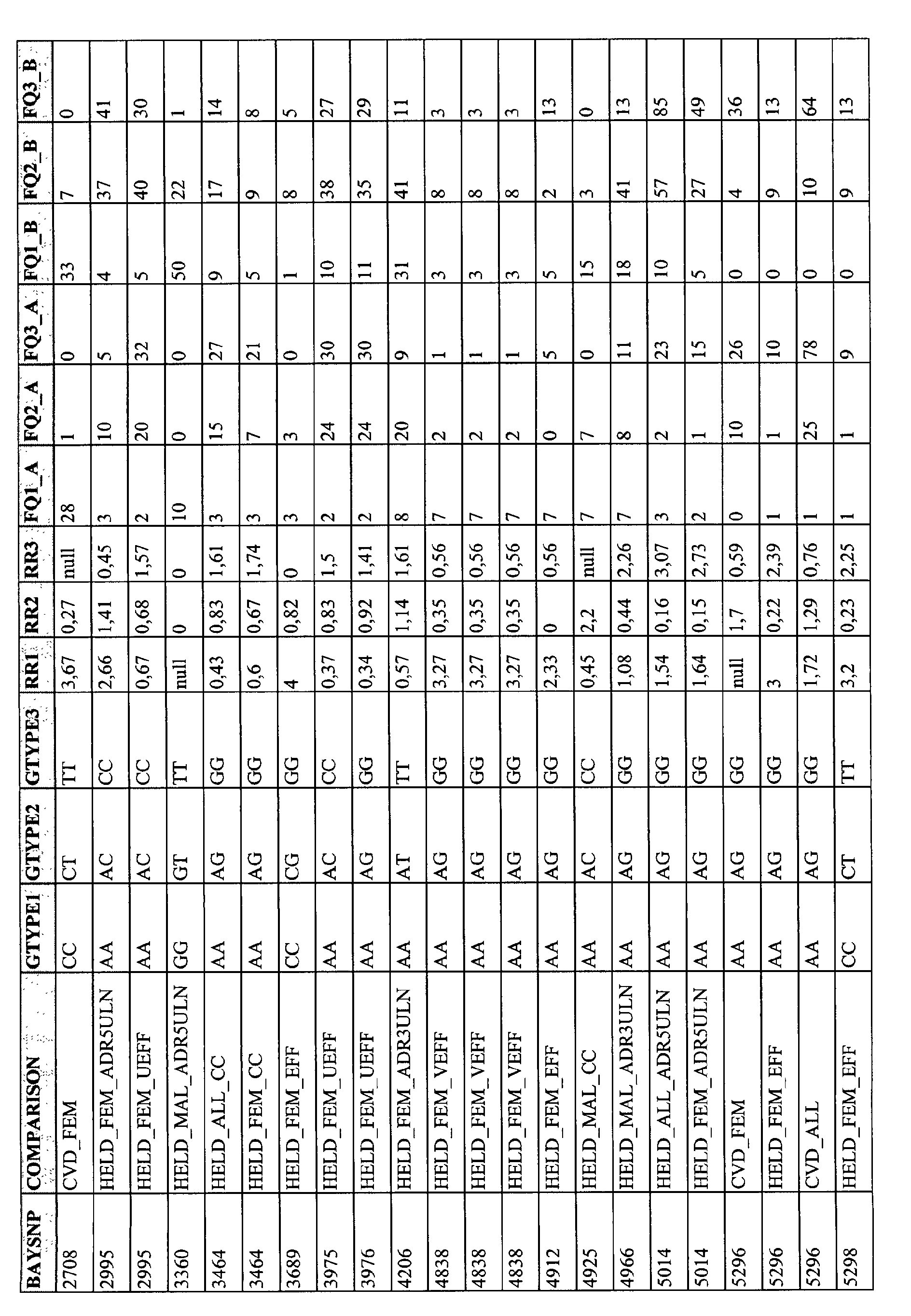

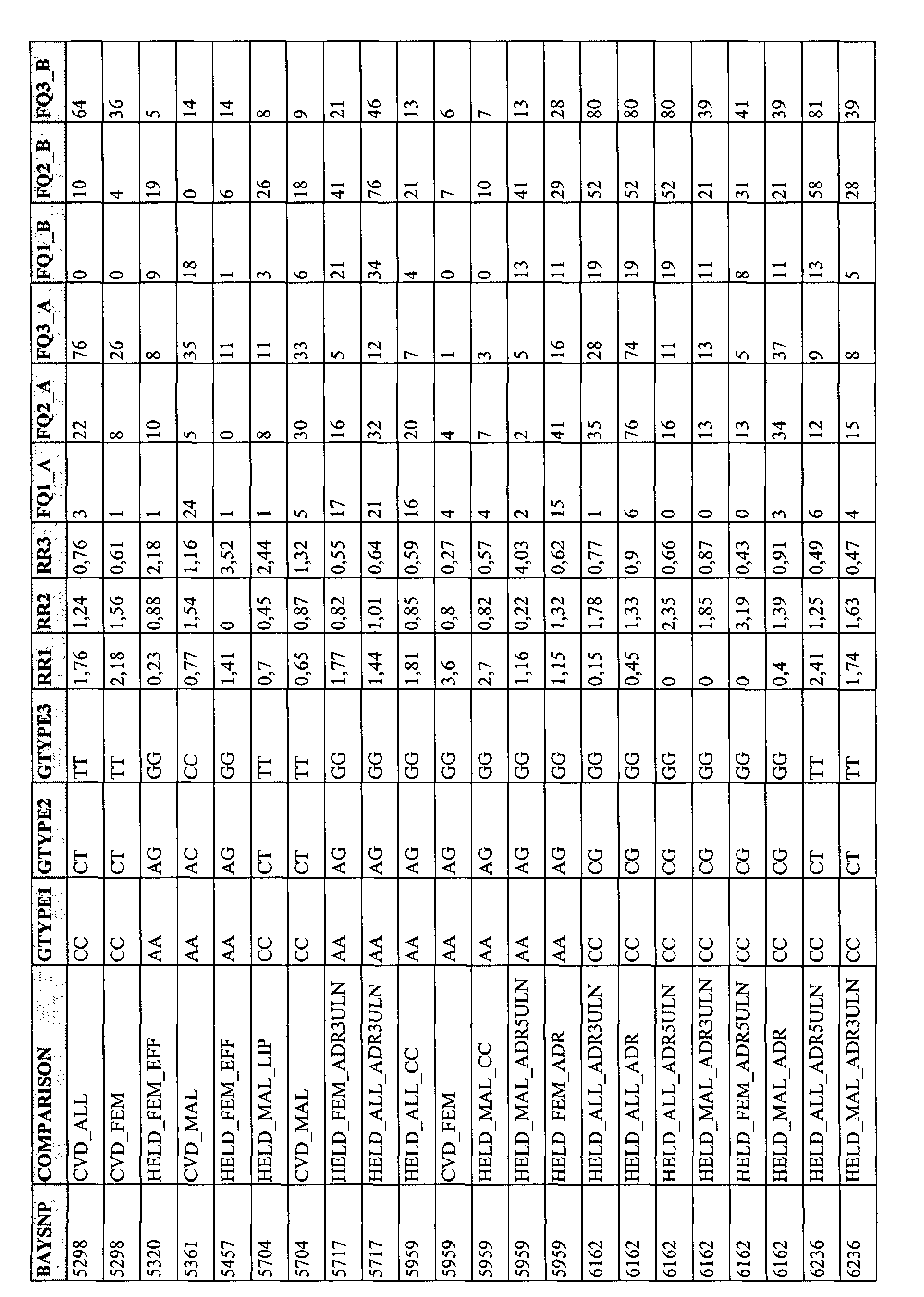

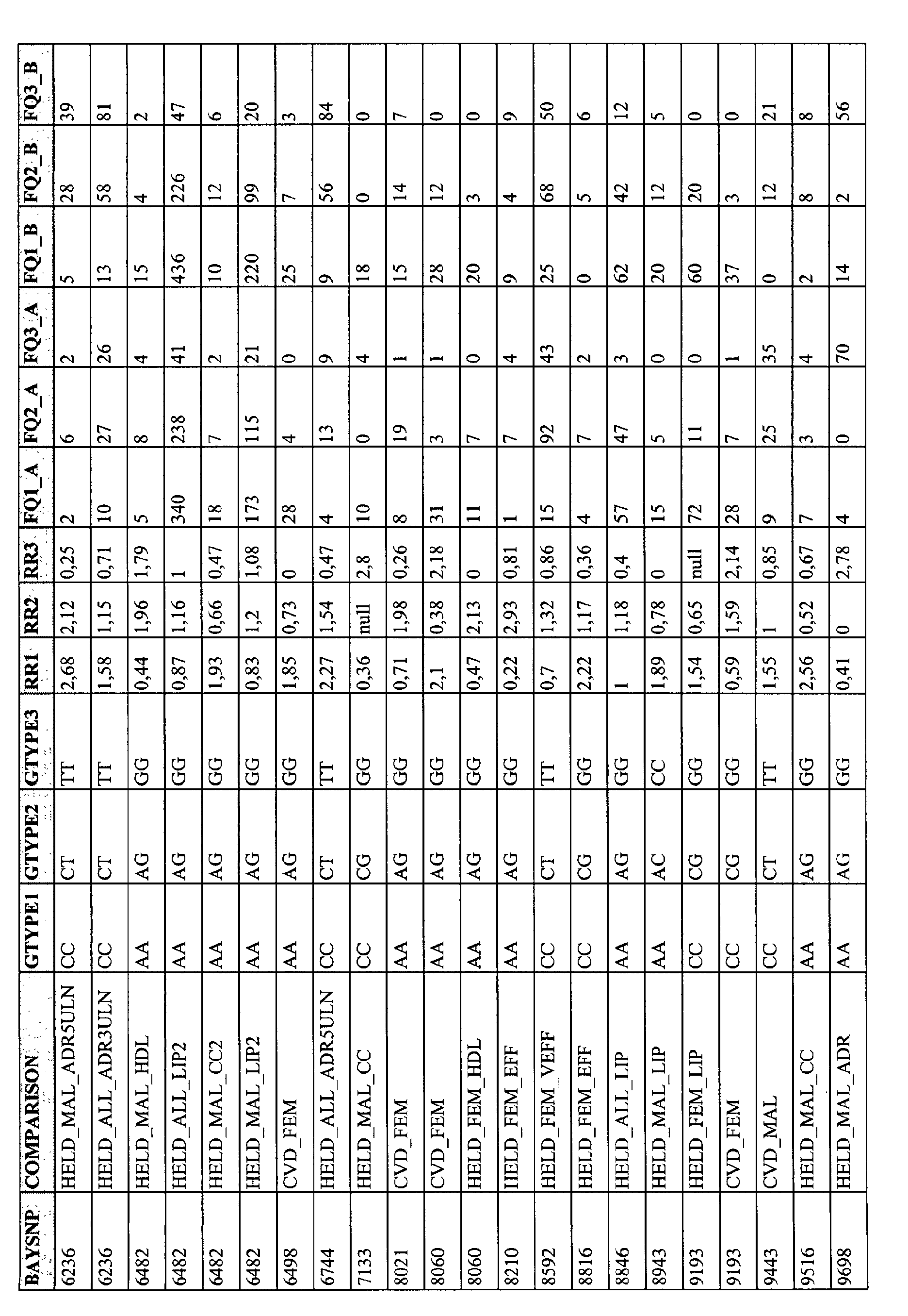

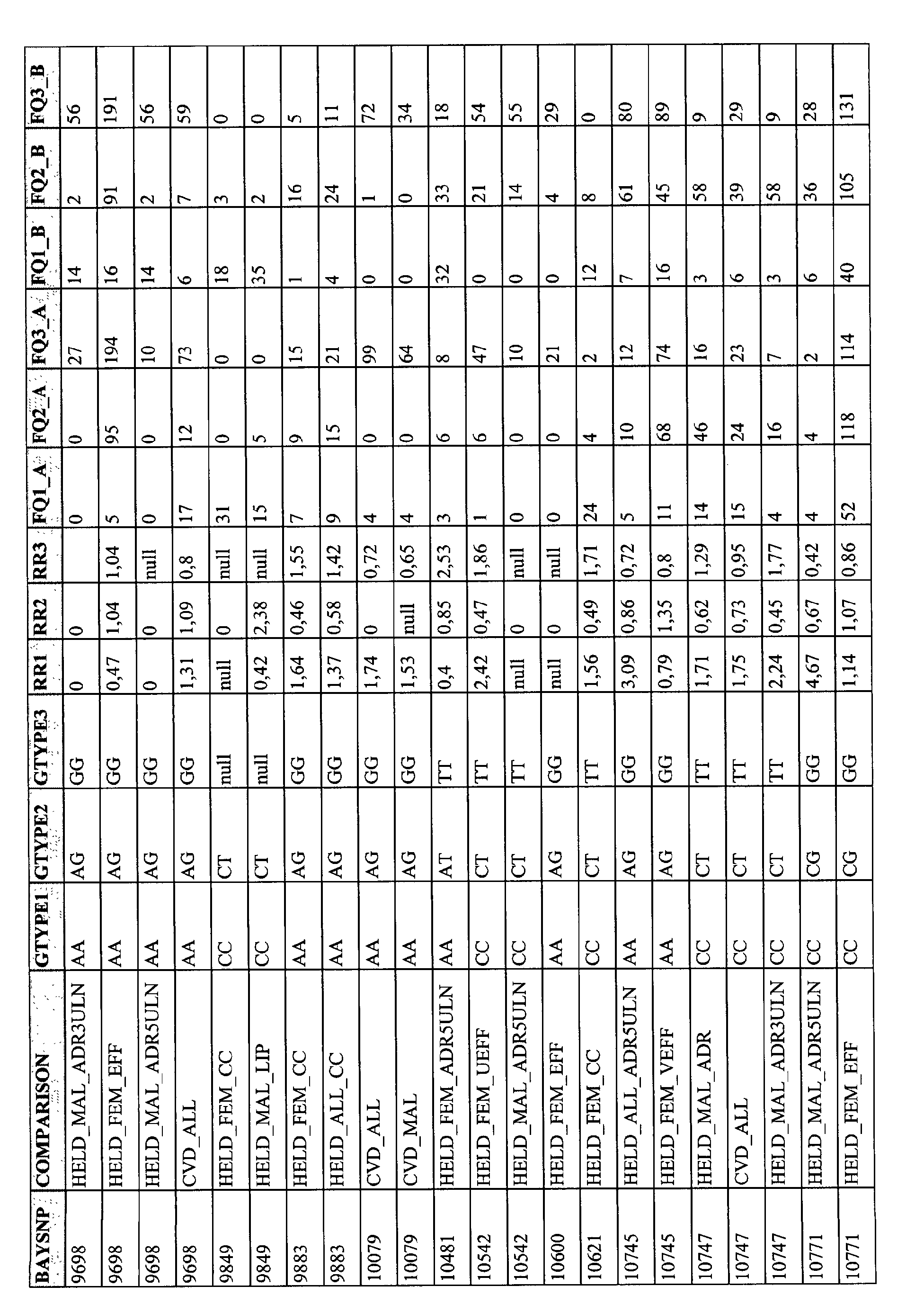

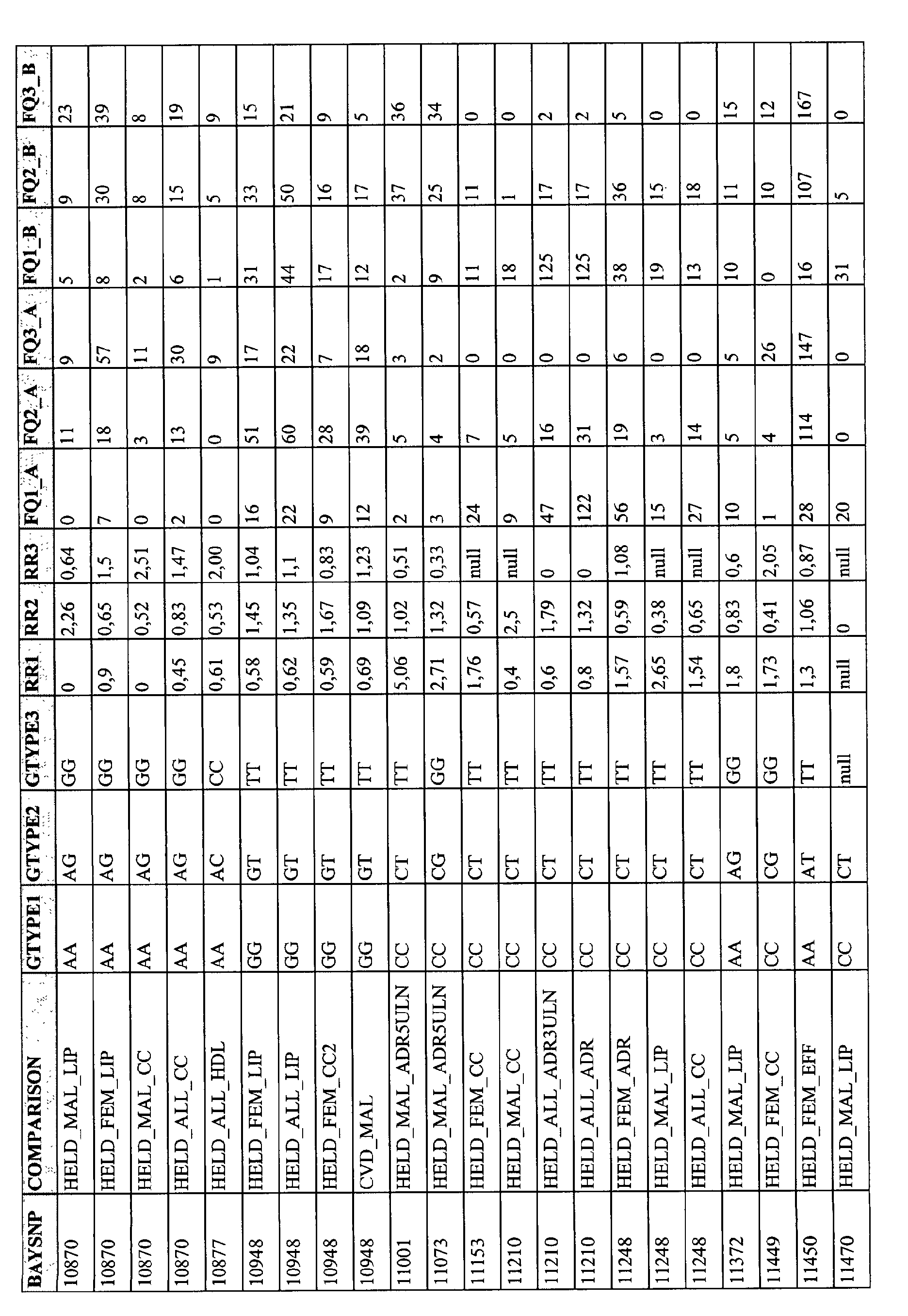

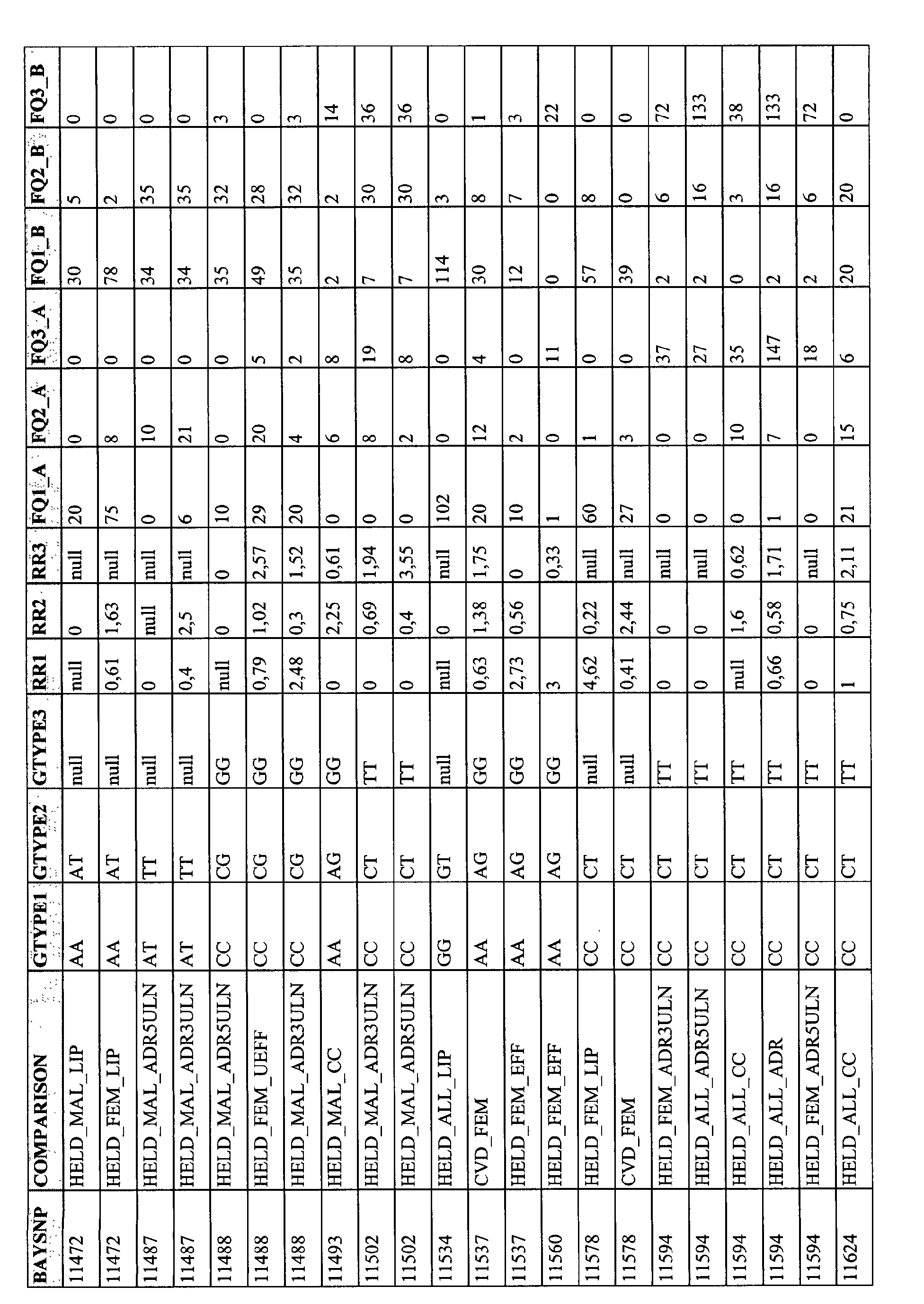

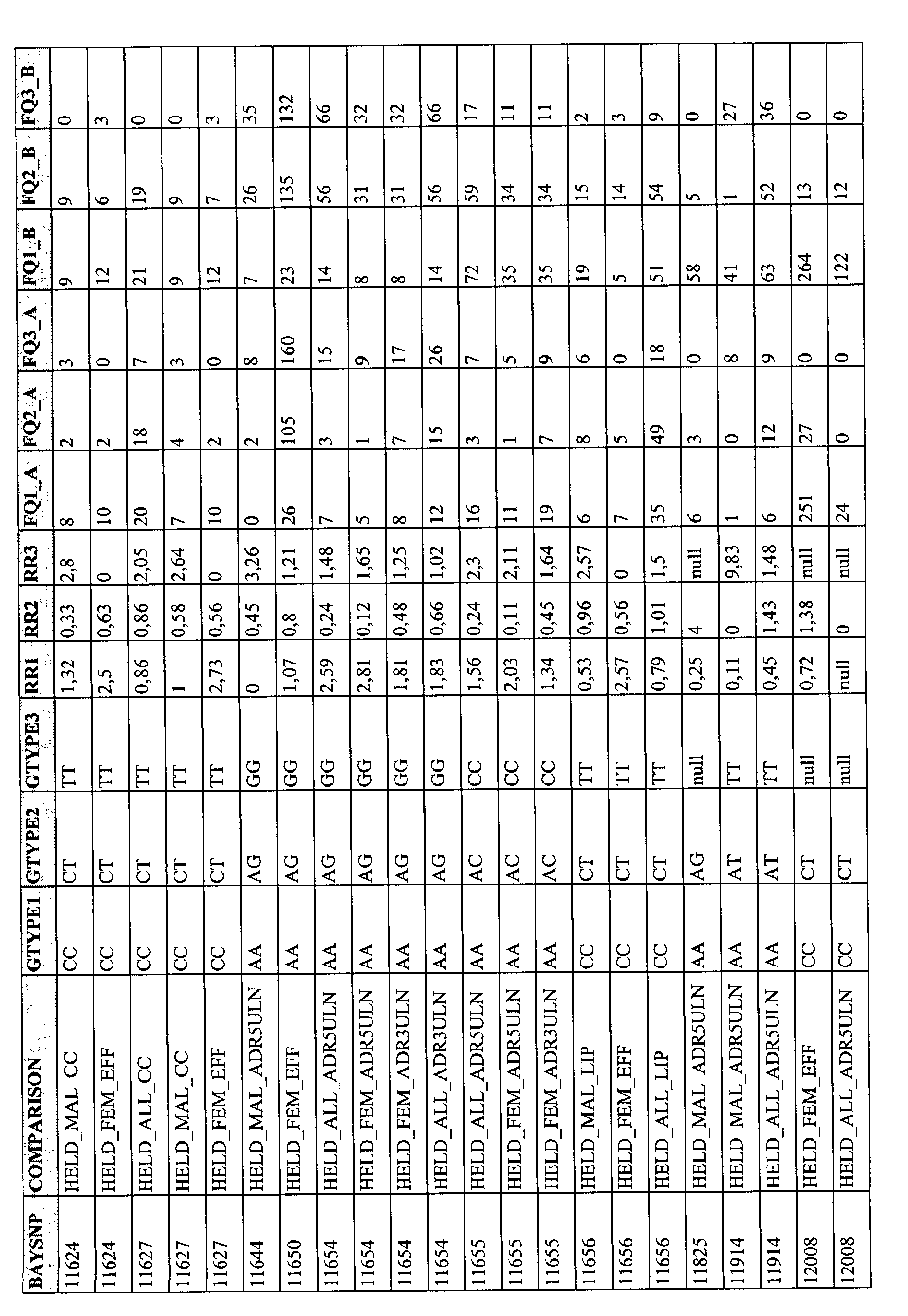

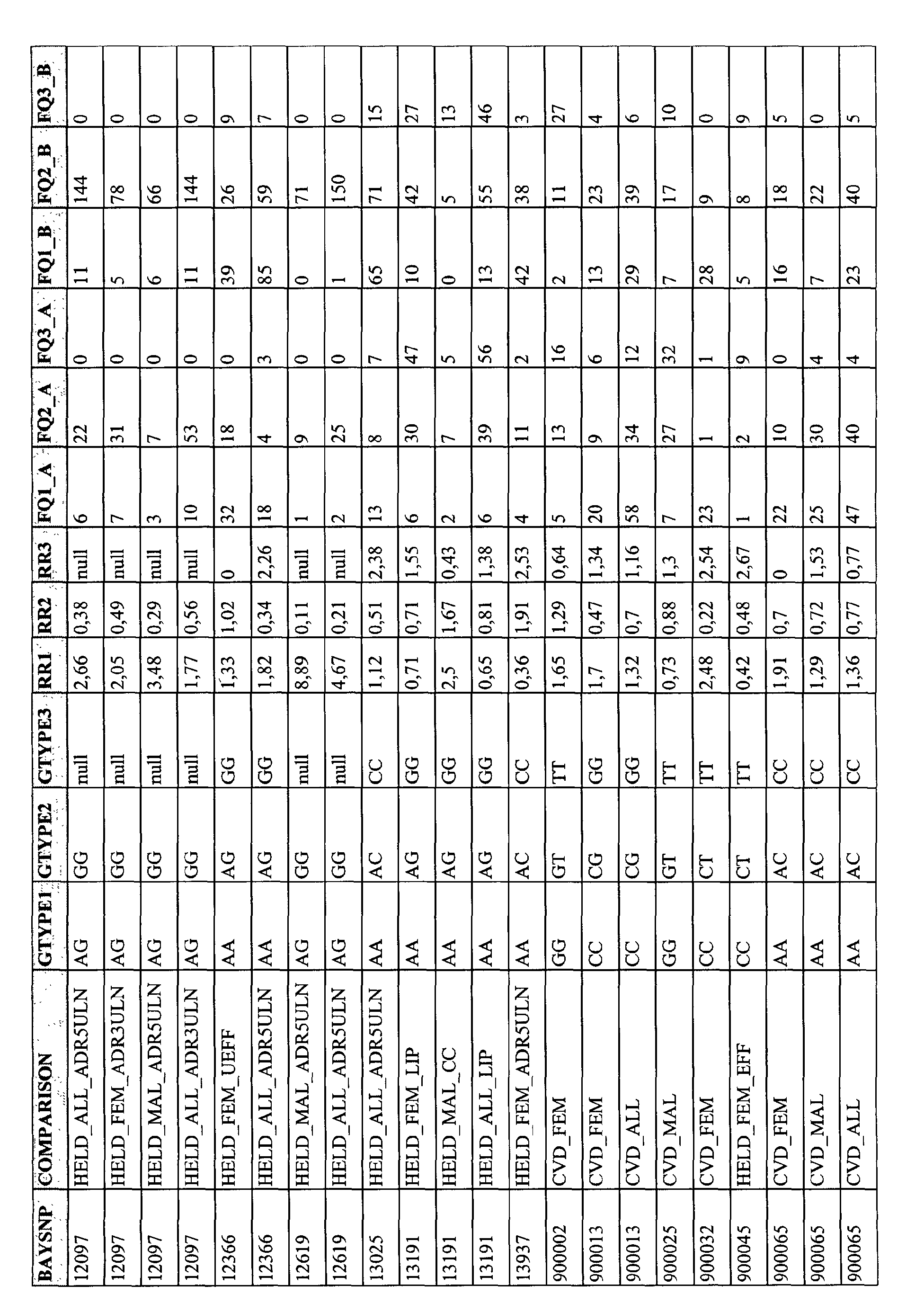

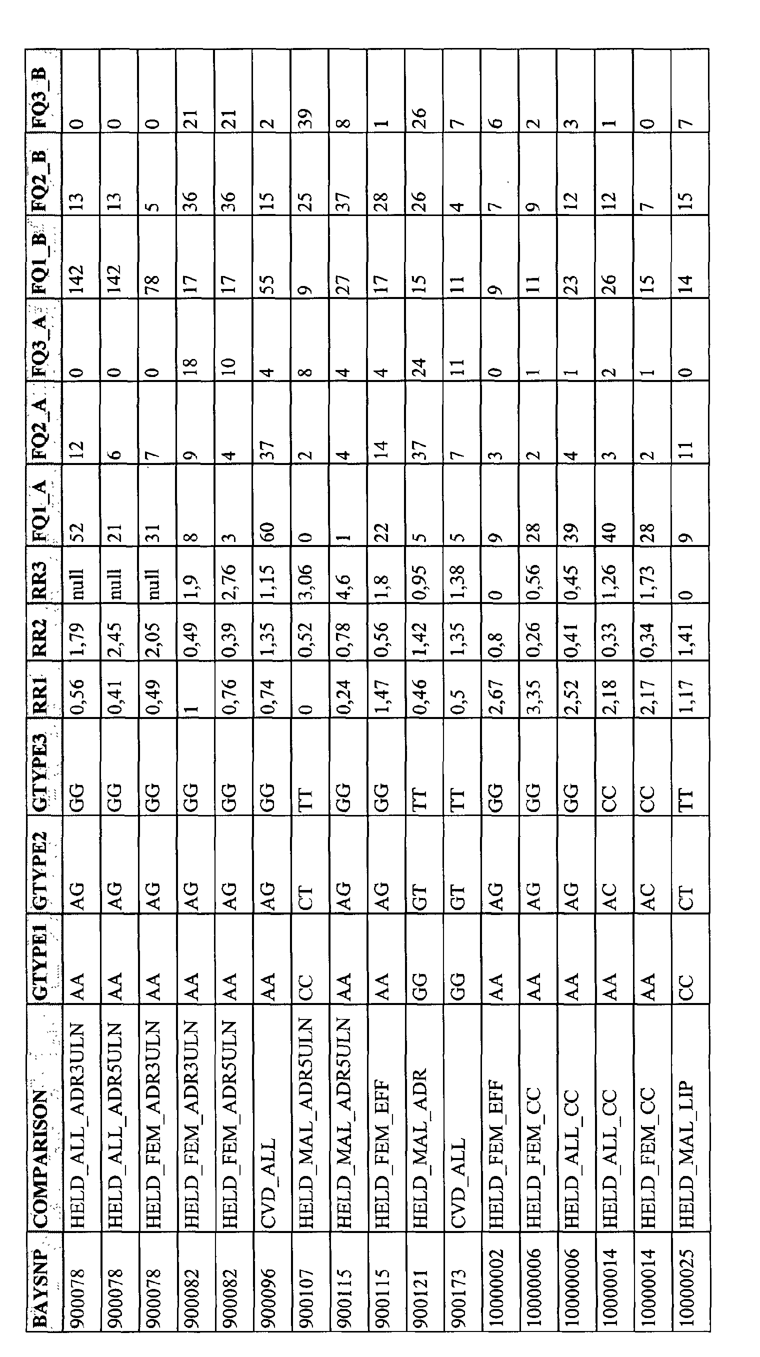

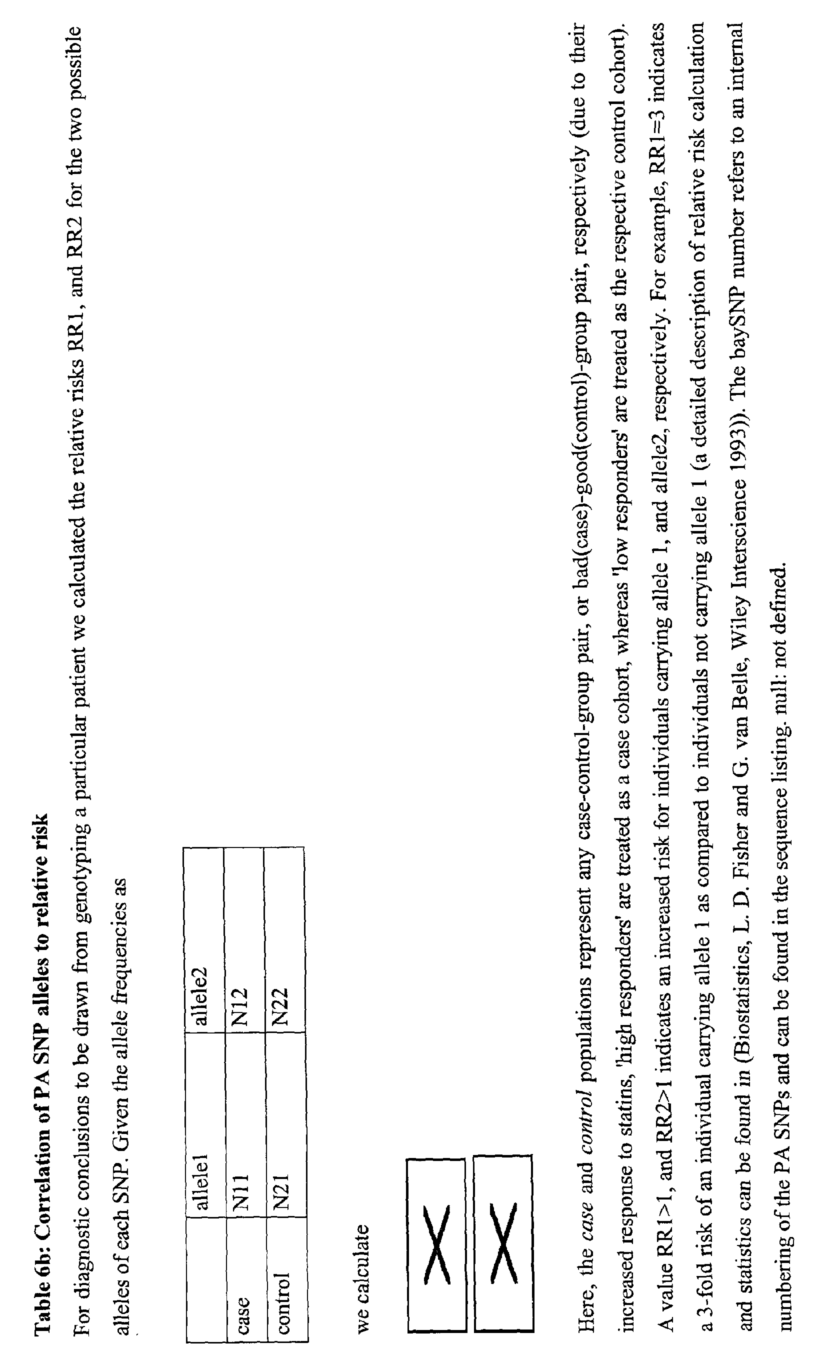

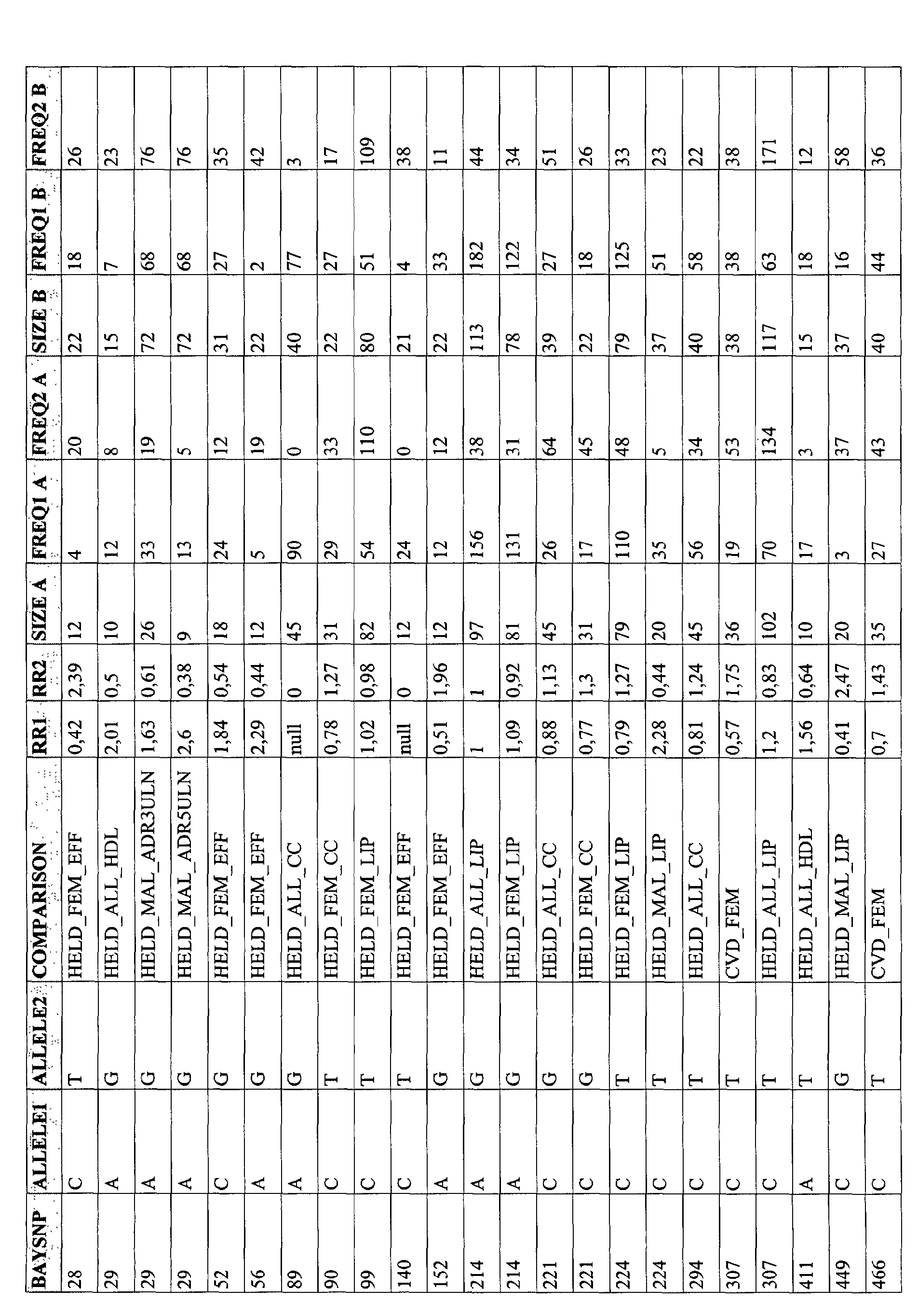

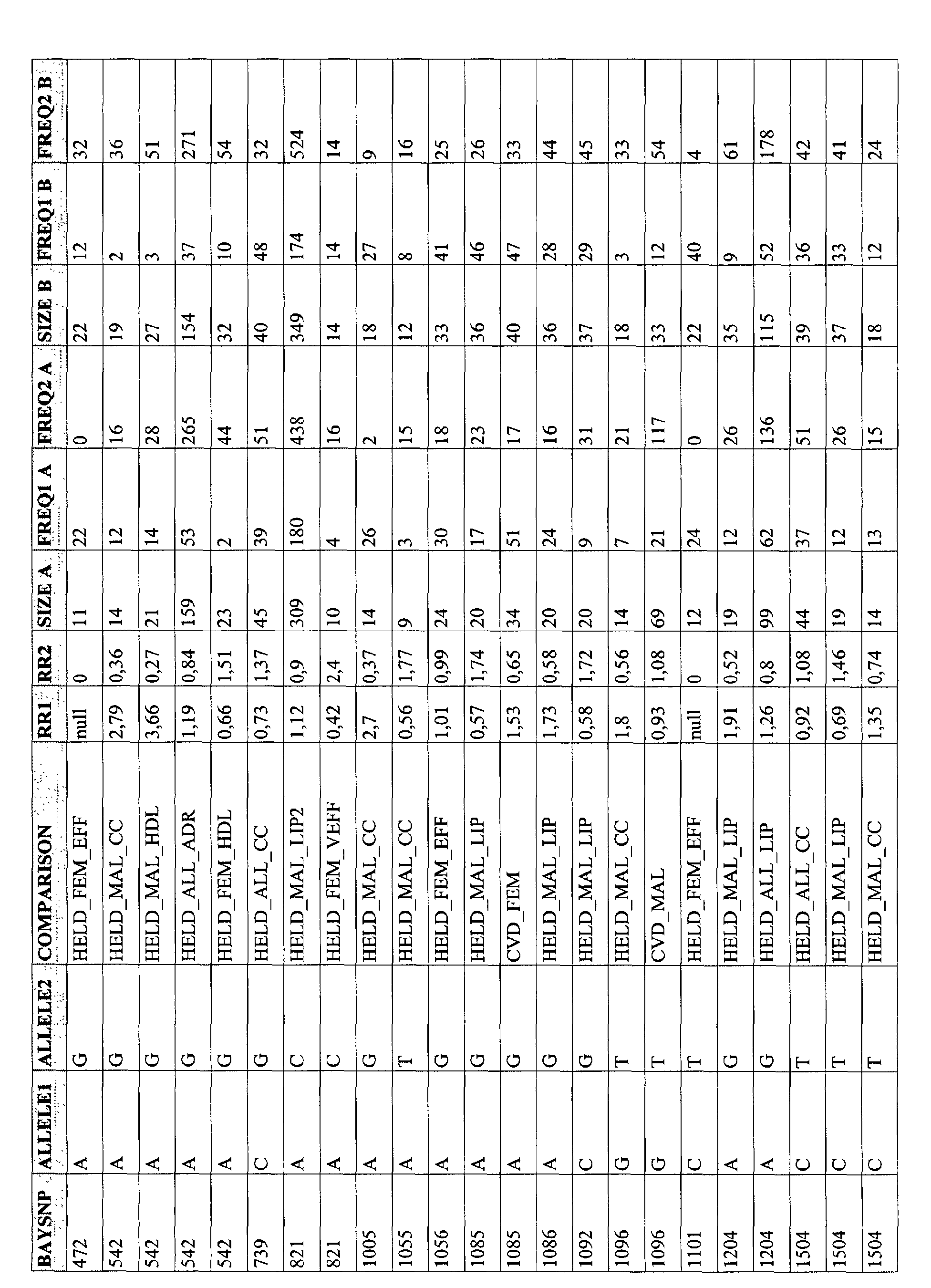

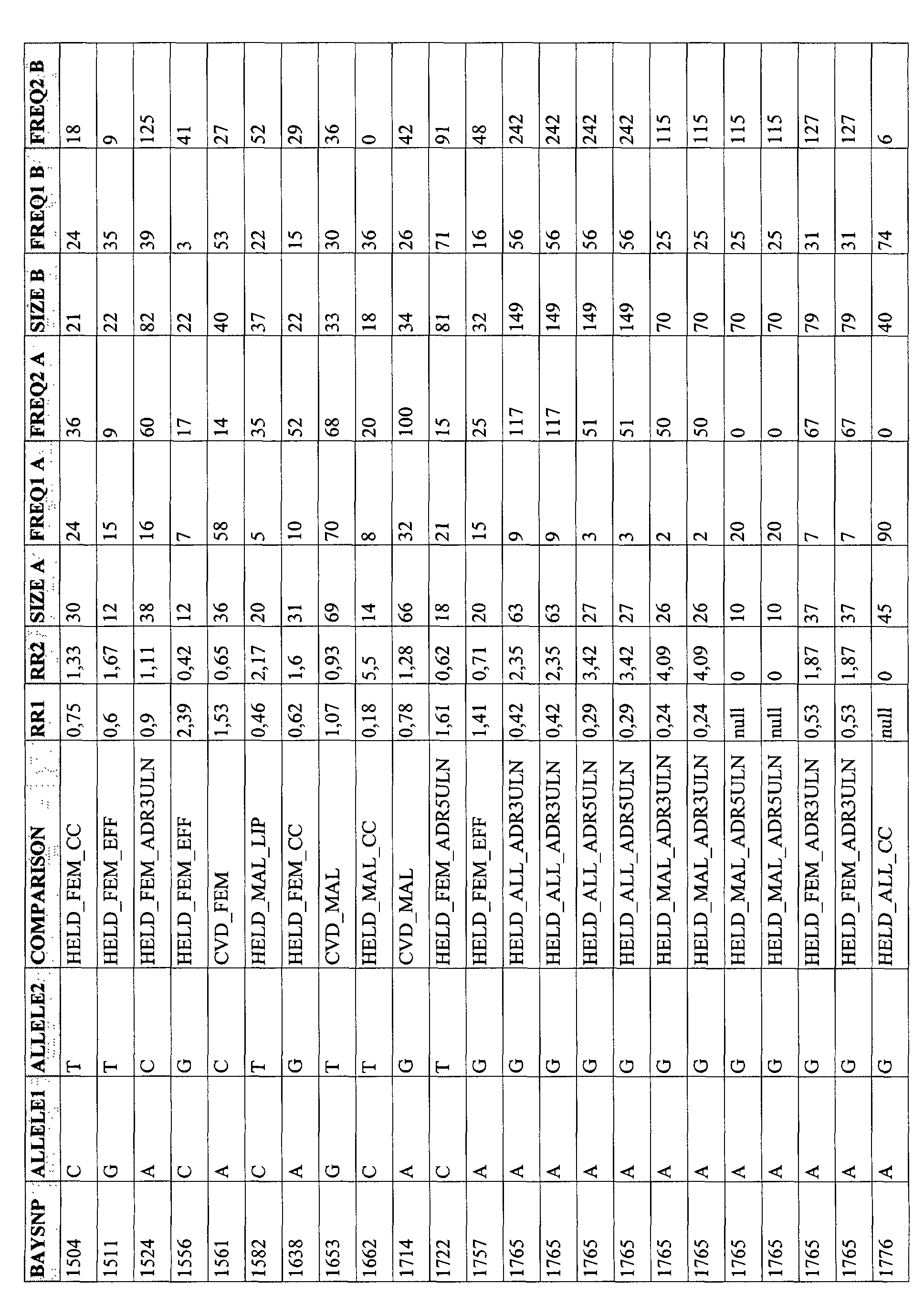

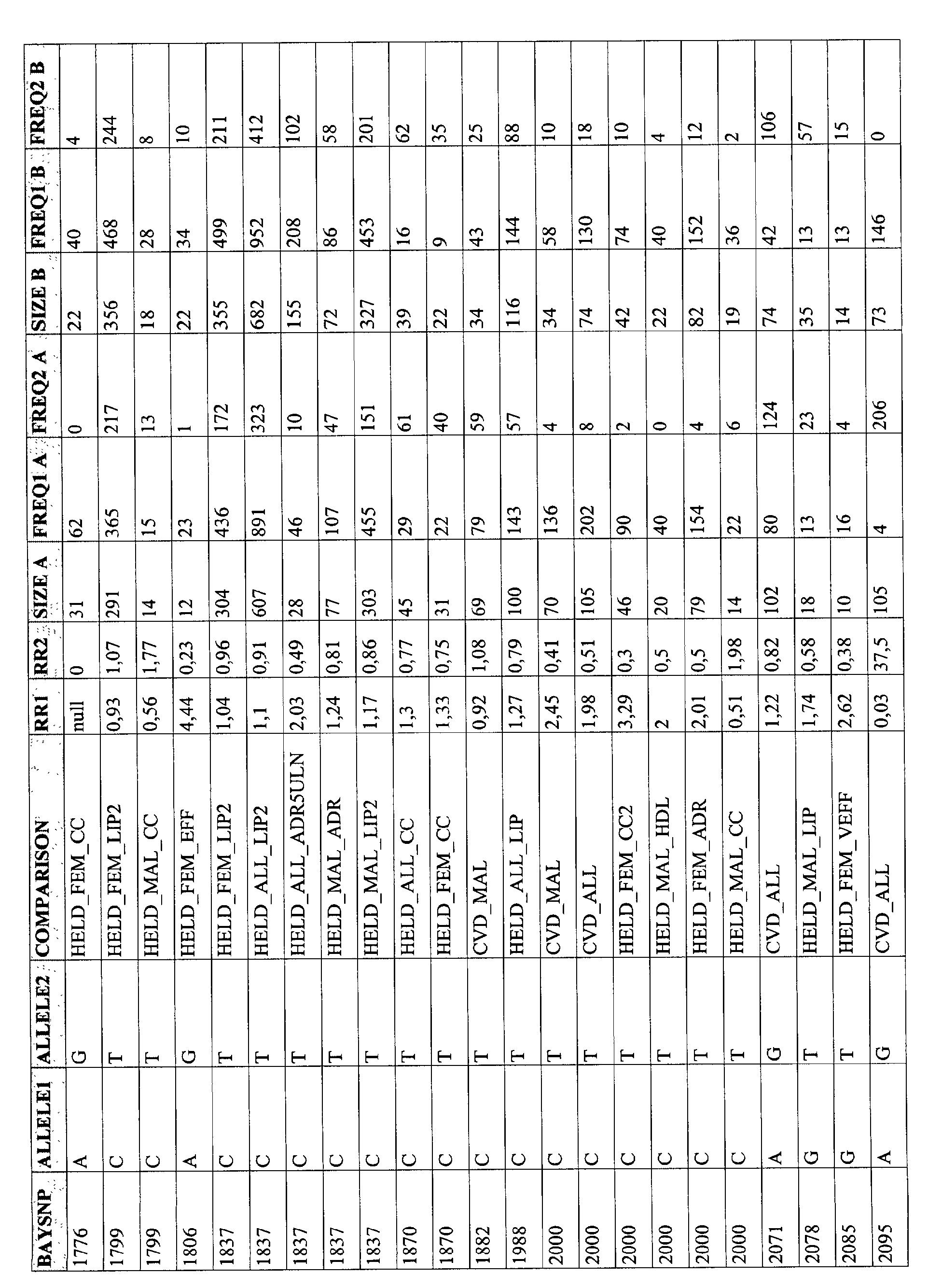

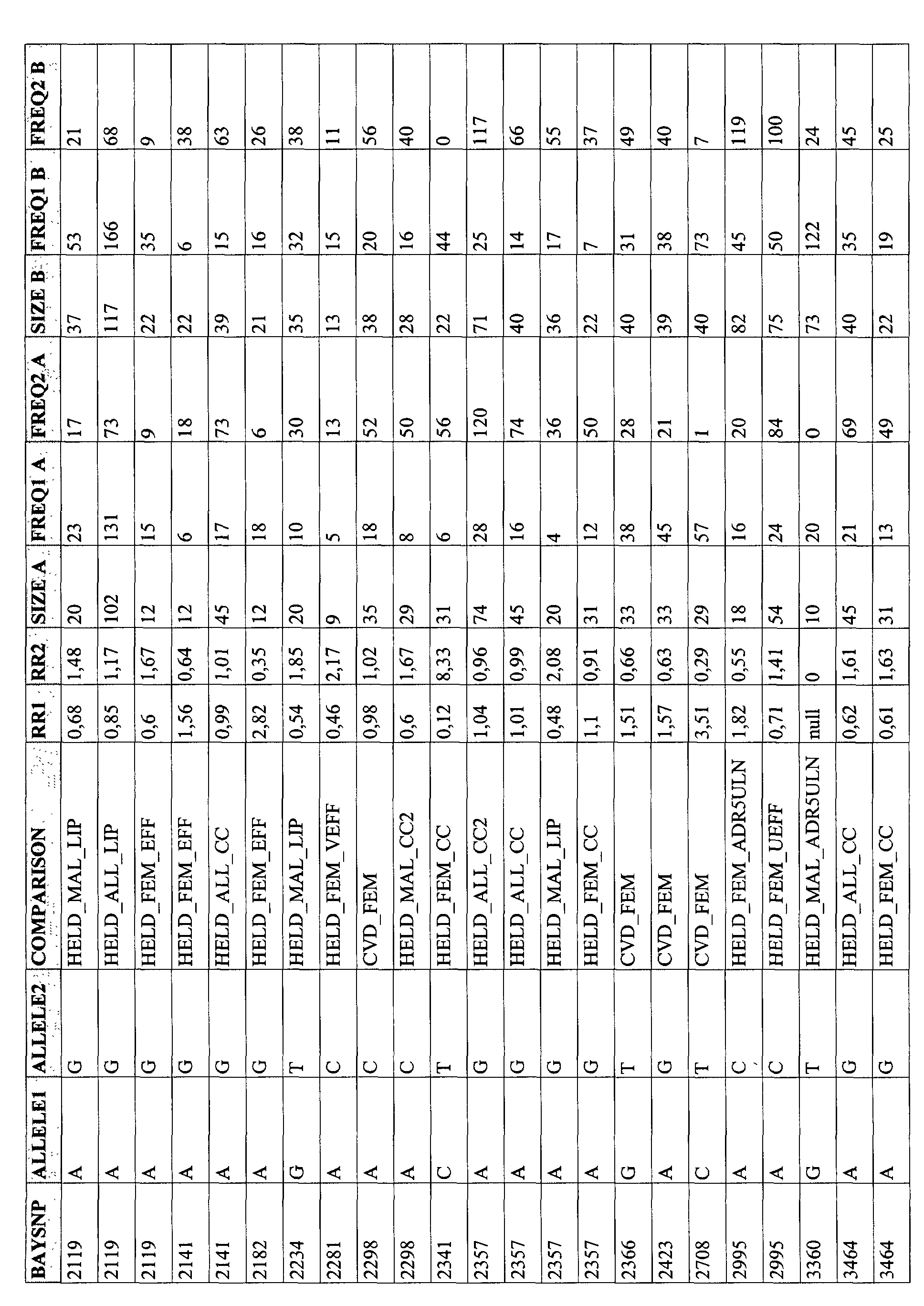

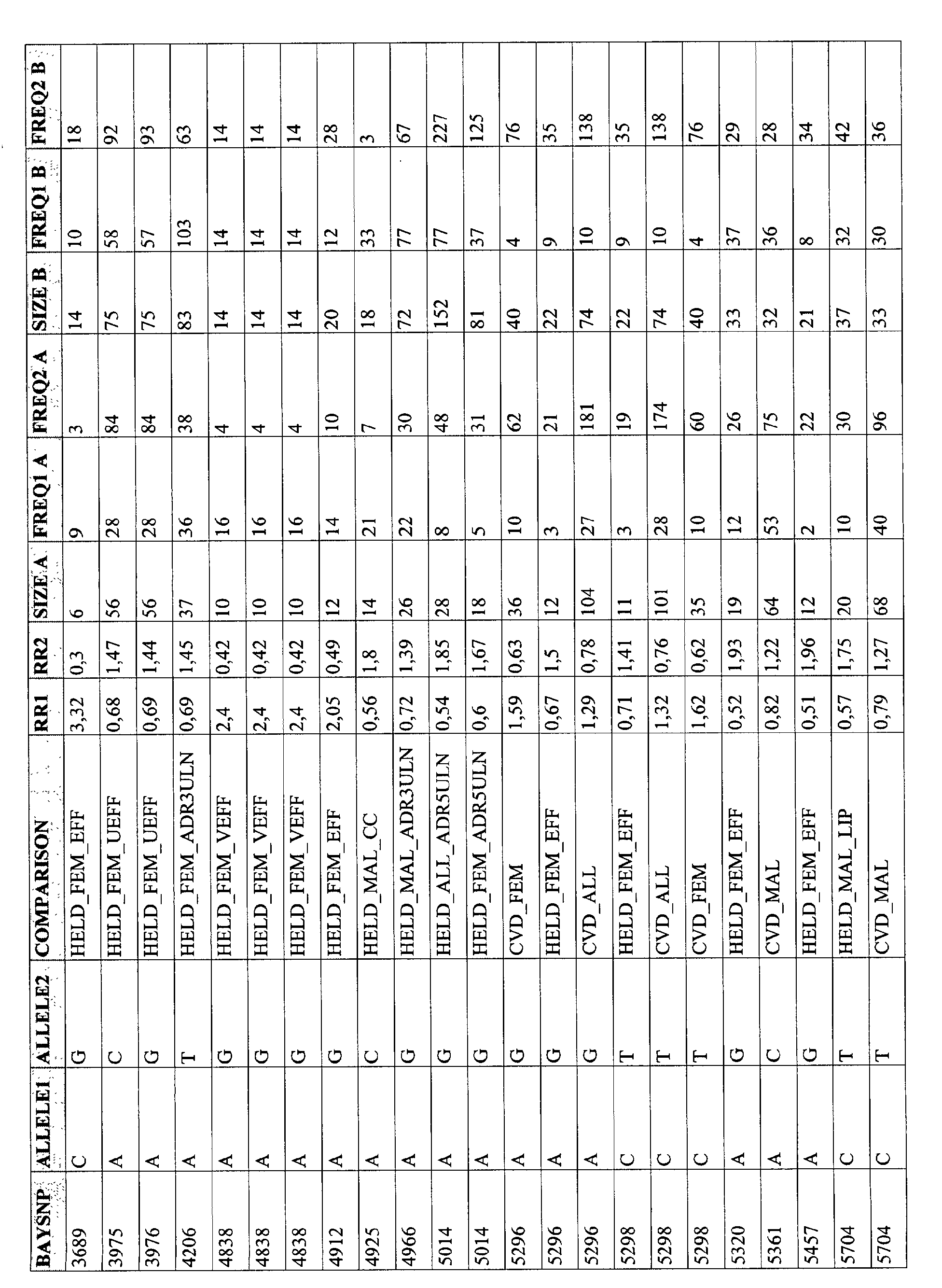

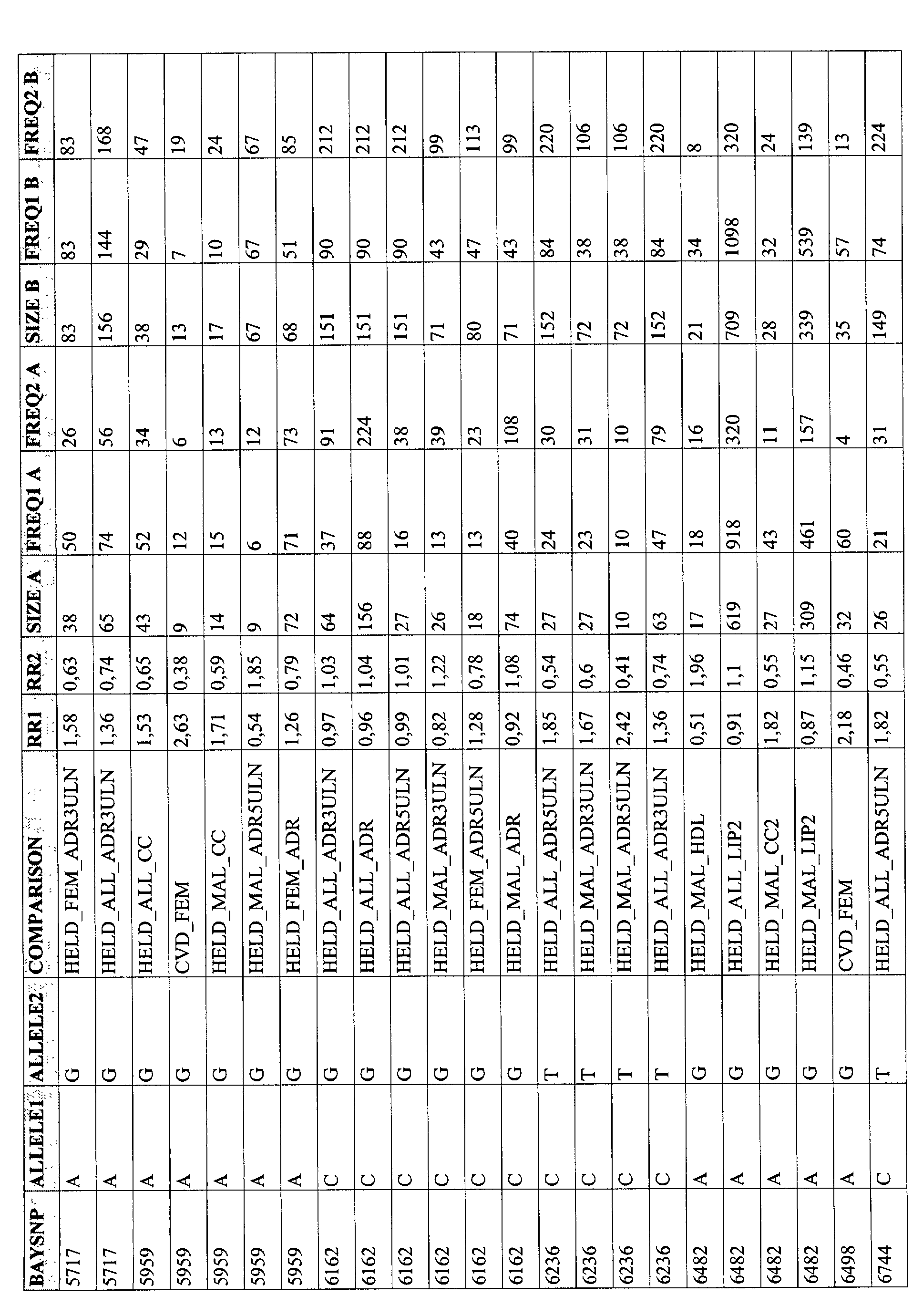

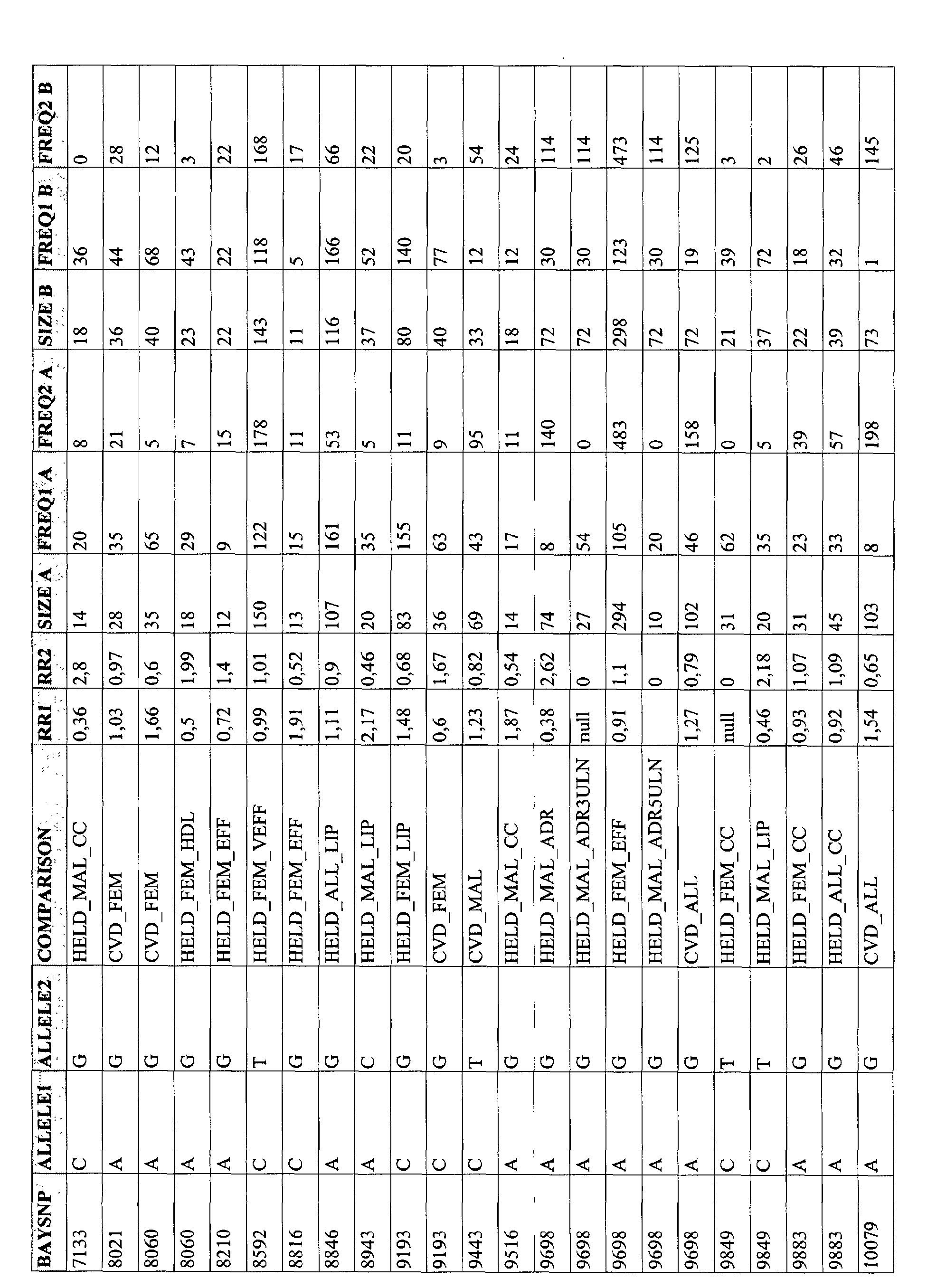

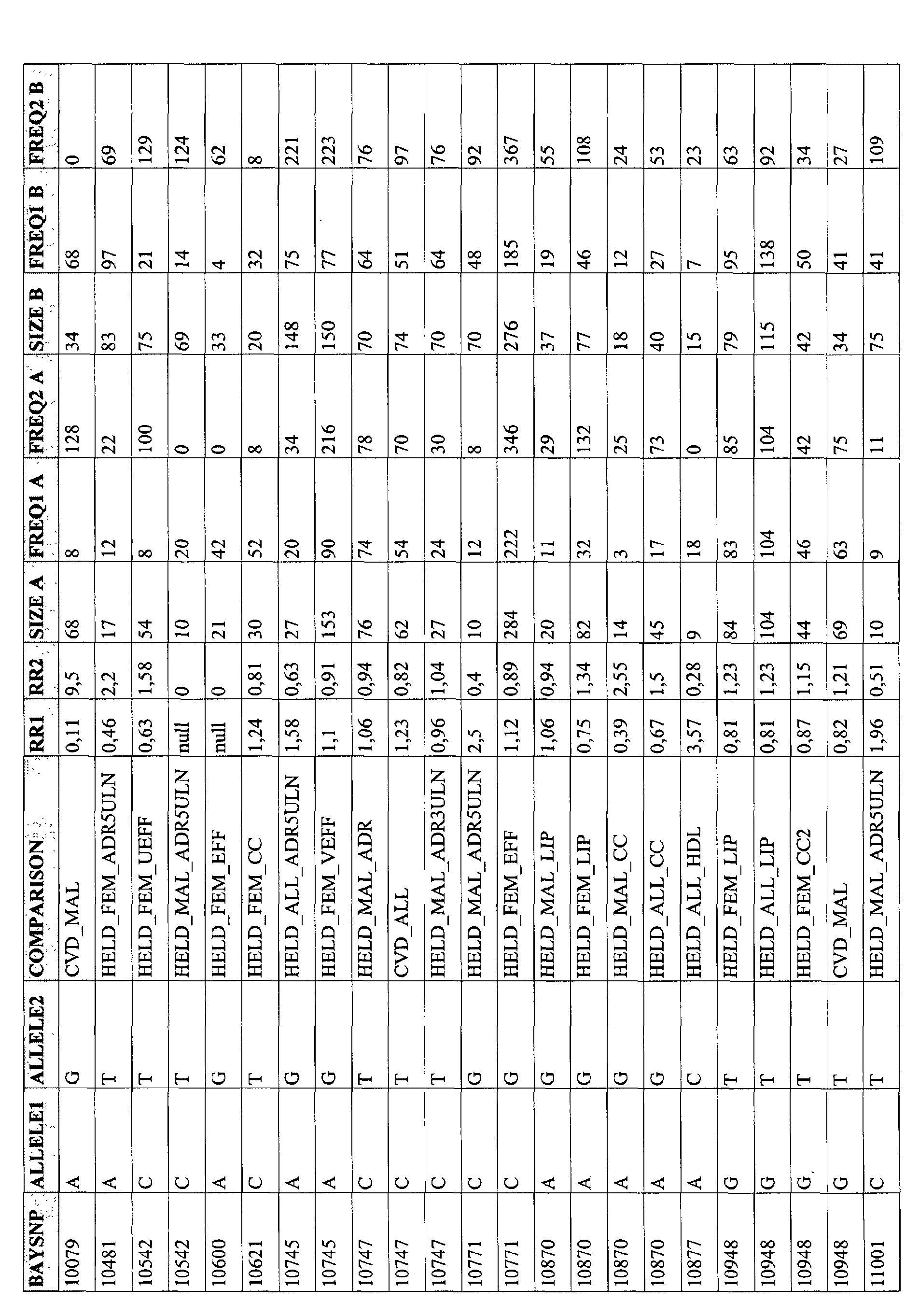

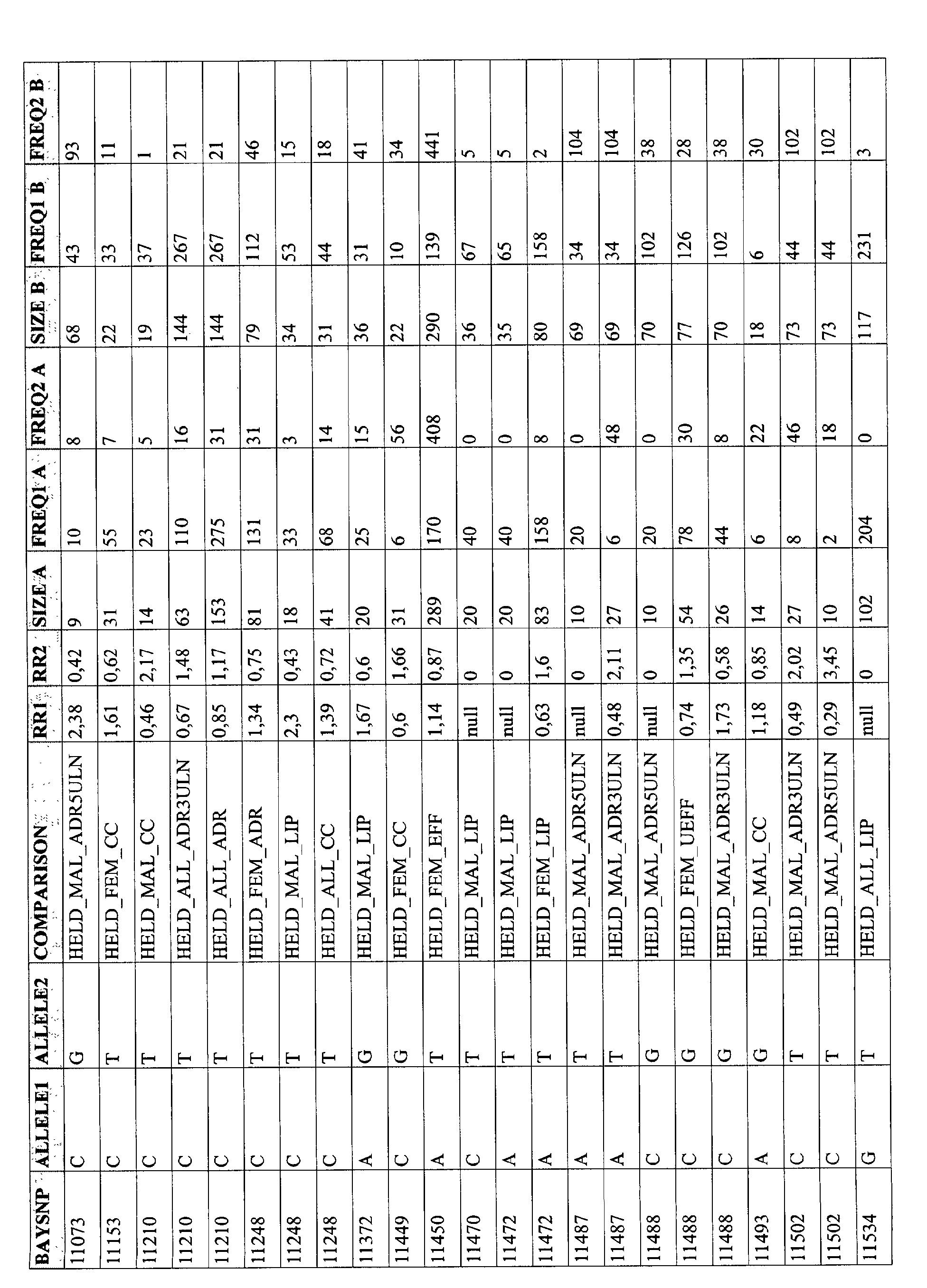

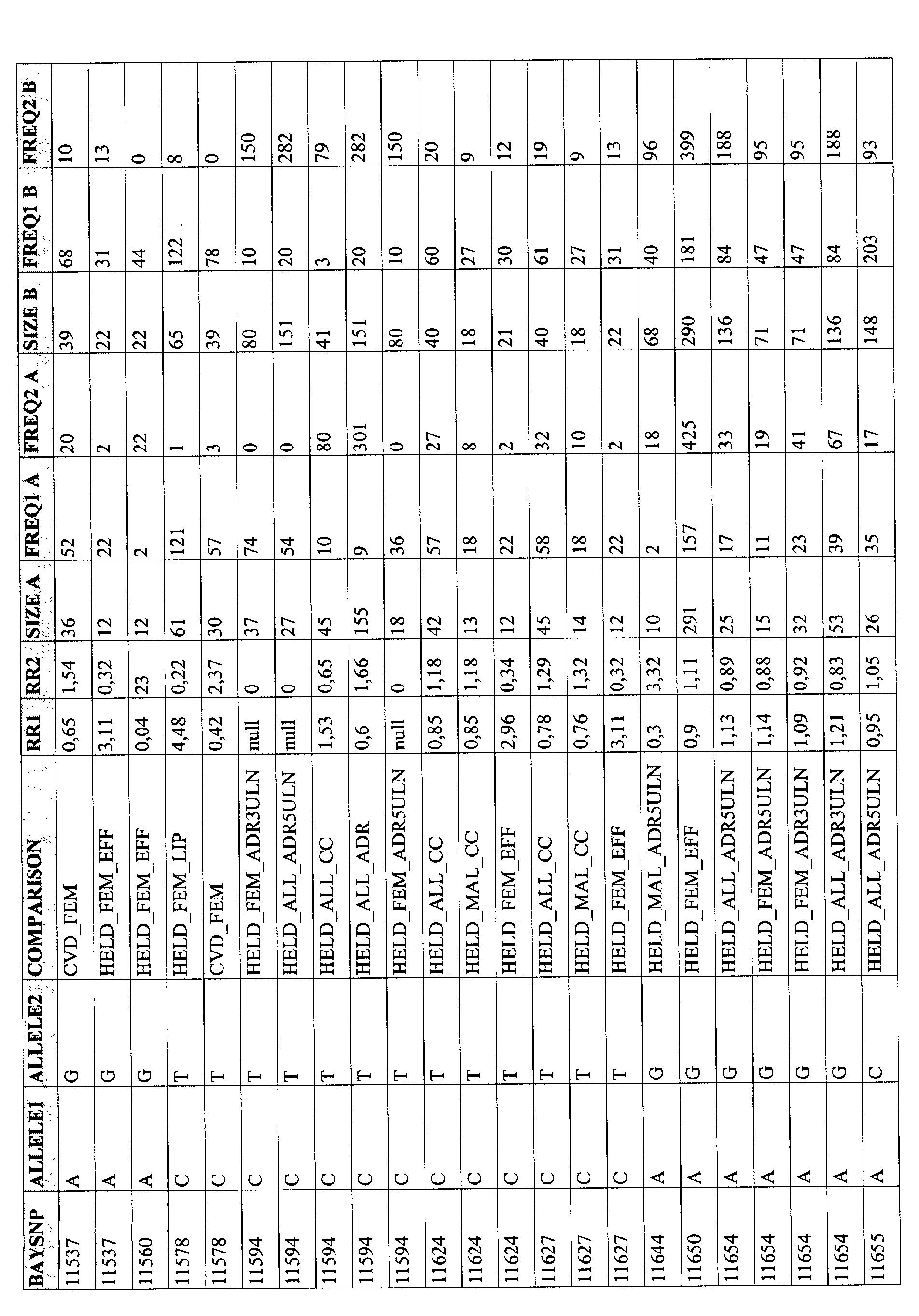

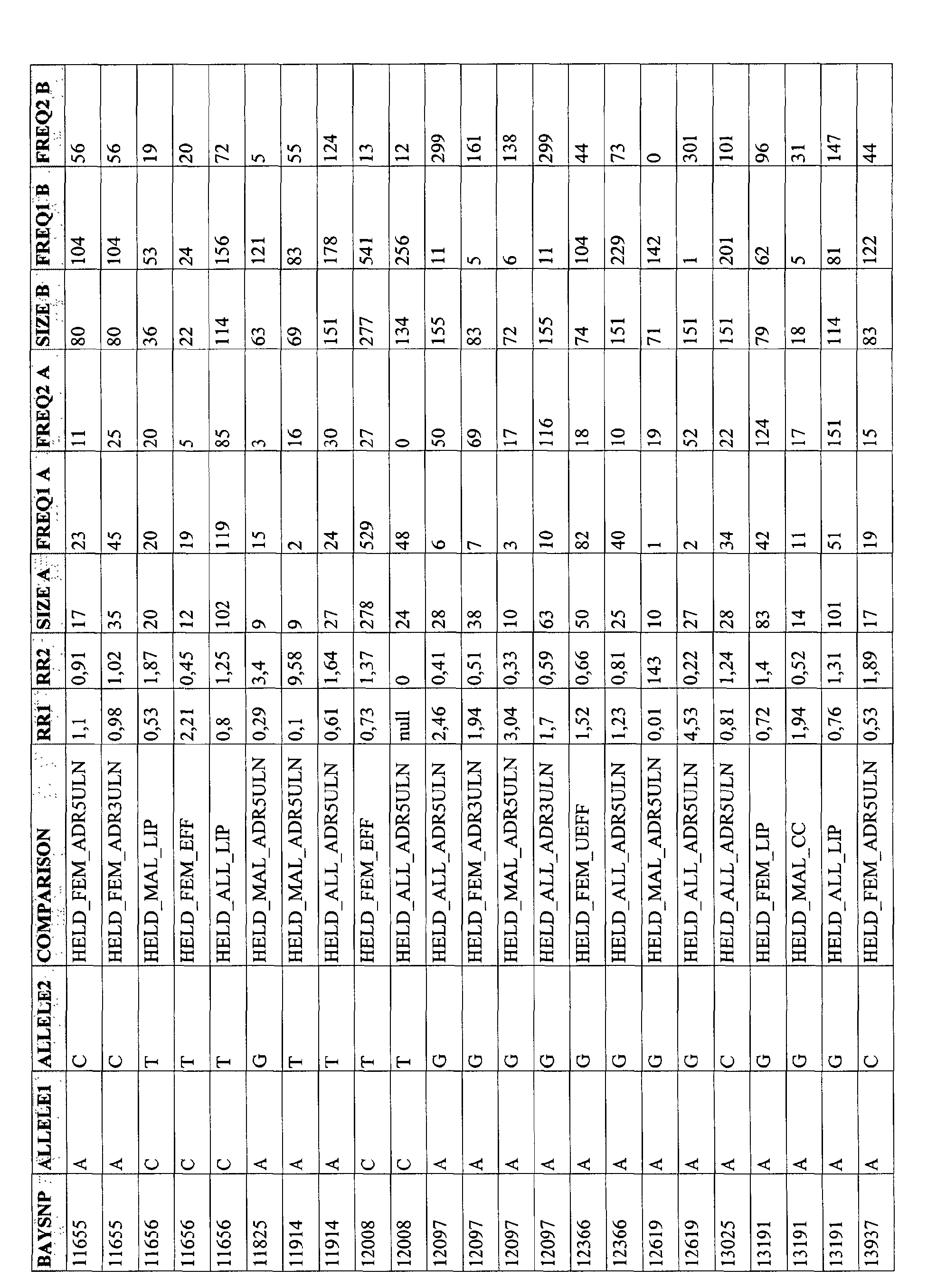

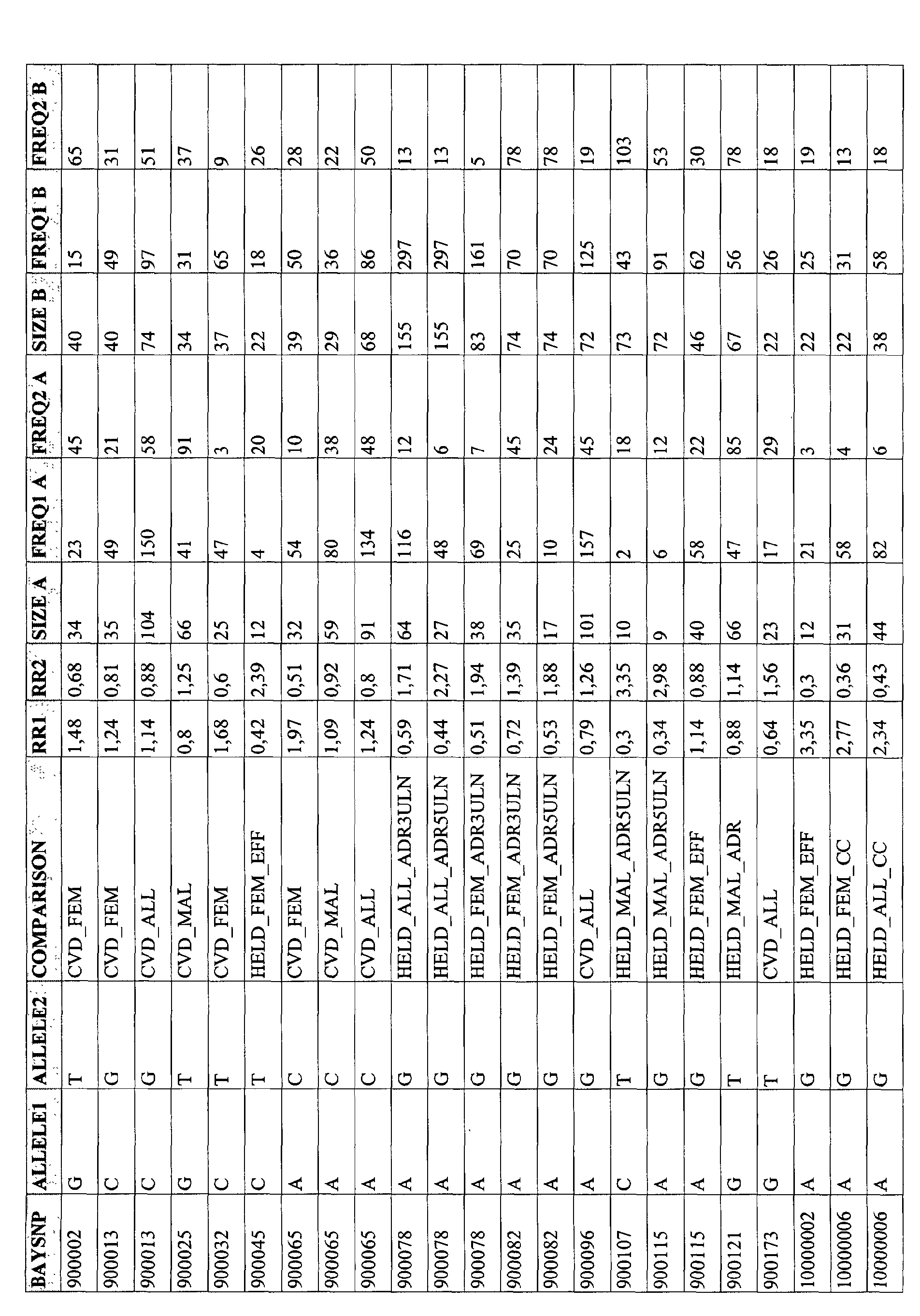

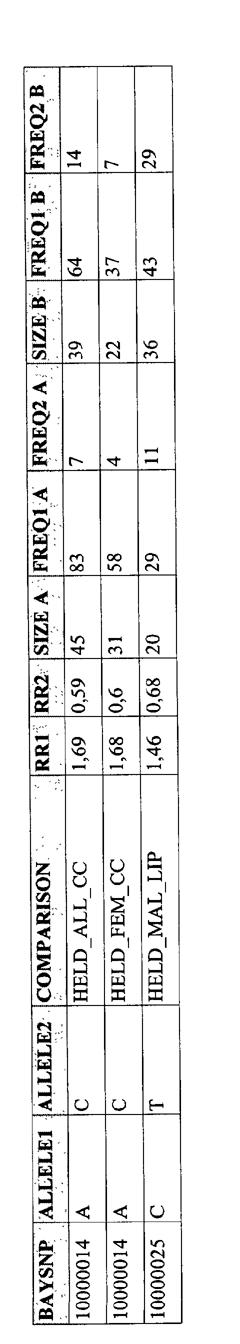

## Claims

1. An isolated polynucleotide encoded by a phenotype associated (PA) gene; the polynucleotide is selected from the group comprising
SEQ ID 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168 with allelic variation as indicated in the sequences section contained in a functional surrounding like full length cDNA for PA gene polypeptide and with or without the PA gene promoter sequence.

2. An expression vector containing one or more of the polynucleotides of claim 1.

3. A host cell containing the expression vector of claim 2.

4. A substantially purified PA gene polypeptide encoded by a polynucleotide of claim 1.

5. A method for producing a PA gene polypeptide, wherein the method comprises the following steps:
a) culturing the host cell of claim 3 under conditions suitable for the expression of the PA gene polypeptide; and
b) recovering the PA gene polypeptide from the host cell culture.

6. A method for the detection of a polynucleotide of claim 1 or a PA gene polypeptide of claim 4 comprising the steps of:
contacting a biological sample with a reagent which specifically interacts with the polynucleotide or the PA gene polypeptide.

7. A method of screening for agents which regulate the activity of a PA gene comprising the steps of:
contacting a test compound with a PA gene polypeptide encoded by any polynucleotide of claim 1; and detecting PA gene activity of the polypeptide, wherein a test compound which increases the PA gene polypeptide activity is identified as a potential therapeutic agent for increasing the activity of the PA gene polypeptide and wherein a test compound which decreases the PA activity of the polypeptide is identified as a potential therapeutic agent for decreasing the activity of the PA gene polypeptide.

8. A reagent that modulates the activity of a PA polypeptide or a polynucleotide wherein said reagent is identified by the method of the claim 7.

9. A pharmaceutical composition, comprising:
the expression vector of claim 2 or the reagent of claim 8 and a pharmaceutically acceptable carrier.

10. Use of the reagent according to claim 8 for the preparation of a medicament.

11. A method for determining whether a human subject has, or is at risk of developing a cardiovascular disease, comprising determining the identity of nucleotide variations as indicated in the sequences section of SEQ ID 1-168 of the PA gene locus of the subject and where the SNP class of the SNP is "CVD" as can be seen from table 3; whereas a "risk" genotype has a risk ratio of greater than 1 as can be seen from table 6.

12. A method for determining a patient's individual response to statin therapy, including drug efficacy and adverse drug reactions, comprising determining the identity of nucleotide variations as indicated in the sequences section of SEQ ID 1-168 of the PA gene locus of the subject and where the SNP class of the SNP is "ADR", "EFF" or both as can be seen from table 3; whereas the probability for such response can be seen from table 6.

13. Use of the method according to claim 12 for the preparation of a medicament tailored to suit a patient's individual response to statin therapy.

14. A kit for assessing cardiovascular status or statin response, said kit comprising
a) sequence determination primers and
b) sequence determination reagents
wherein said primers are selected from the group comprising primers that hybridize to polymorphic positions in human PA genes according to claim 1; and primers that hybridize immediately adjacent to polymorphic positions in human PA genes according to claim 1.

15. A kit as defined in claims 12 detecting a combination of two or more, up to all, polymorphic sites selected from the groups of sequences as defined in claim 1.

16. A kit for assessing cardiovascular status or statin response, said kit comprising one or more antibodies specific for a polymorphic position defined in claim 1 within the human PA gene polypeptides and combinations of any of the foregoing.
